(19) Europäisches Patentamt
European Patent Office
Office européen des brevets

(11) EP 4 043 449 A1

(12) **EUROPEAN PATENT APPLICATION**
published in accordance with Art. 153(4) EPC

(43) Date of publication:
**17.08.2022 Bulletin 2022/33**

(21) Application number: **20874267.6**

(22) Date of filing: **09.10.2020**

(51) International Patent Classification (IPC):
***C07D 333/76*** *(2006.01)* ***C09K 11/06*** *(2006.01)*
***C07D 307/91*** *(2006.01)* ***H01L 51/50*** *(2006.01)*

(52) Cooperative Patent Classification (CPC):
**C07D 307/91; C07D 333/76; C09K 11/06; H01L 51/50**

(86) International application number:
**PCT/JP2020/038398**

(87) International publication number:
**WO 2021/070963 (15.04.2021 Gazette 2021/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.10.2019 JP 2019187783**
**22.11.2019 JP 2019211818**
**05.06.2020 JP 2020098420**
**05.06.2020 JP 2020098426**

(71) Applicant: **Idemitsu Kosan Co., Ltd**
**Tokyo 100-8321 (JP)**

(72) Inventors:
- **HAKETA, Tasuku**
  **Sodegaura-shi, Chiba 299-0293 (JP)**
- **ITO, Hirokatsu**
  **Sodegaura-shi, Chiba 299-0293 (JP)**
- **KUDO, Yu**
  **Sodegaura-shi, Chiba 299-0293 (JP)**
- **TAKAHASHI, Yusuke**
  **Sodegaura-shi, Chiba 299-0293 (JP)**
- **TANAKA, Shota**
  **Sodegaura-shi, Chiba 299-0293 (JP)**

(74) Representative: **Vossius & Partner Patentanwälte Rechtsanwälte mbB**
**Siebertstraße 3**
**81675 München (DE)**

(54) **COMPOUND, MATERIAL FOR ORGANIC ELECTROLUMINESCENT ELEMENT, ORGANIC ELECTROLUMINESCENT ELEMENT, AND ELECTRONIC DEVICE**

(57) Provided are a compound that further improves the capability of an organic EL device, an organic electroluminescent device having a further improved device capability, and an electronic device including the organic electroluminescent device. Precisely provided are: a compound represented by the following formula (1) (wherein N*, Ar, L, X, *a, $*b_1$, $*b_2$,$*b_3$, m, n, $R^{11}$ to $R^{14}$, $R^{15}$ to $R^{22}$, $R^{31}$ to $R^{40}$, and $R^{41}$ to $R^{48}$ are as defined in the description), an organic electroluminescent device containing the compound, and an electronic device including the organic electroluminescent device.

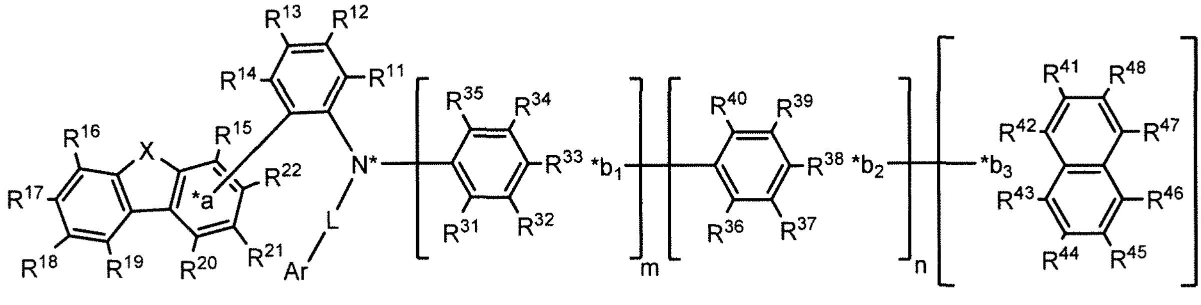

(1)



Processed by Luminess, 75001 PARIS (FR)

## Description

### Technical Field

The present invention relates to a compound, a material for organic electroluminescent devices, an organic electroluminescent device, and an electronic device including the organic luminescent device.

### Background Art

In general, an organic electroluminescent device (which may be hereinafter referred to as an "organic EL device") is constituted by an anode, a cathode, and an organic layer intervening between the anode and the cathode. In application of a voltage between both the electrodes, electrons from the cathode side and holes from the anode side are injected into a light emitting region, and the injected electrons and holes are recombined in the light emitting region to generate an excited state, which then returns to the ground state to emit light. Accordingly, development of a material that efficiently transports electrons or holes into the light emitting region, and promotes recombination of the electrons and holes is important for providing a high-performance organic EL device.

PTLs 1 to 4 describe compounds used for materials for organic electroluminescent devices.

### Citation List

### Patent Literatures

PTL 1: KR2018-138333
PTL 2: WO2007/125714A1
PTL 3: US2019/0006590A1
PTL 4: US2017/0018710A1

### Technical Problem

Heretofore, various compounds for organic EL devices have been reported, but a compound that further enhances the capability of an organic EL device has been still demanded.

The present invention has been made for solving the problem, and an object thereof is to provide a compound that further improves the capability of an organic EL device, an organic EL device having a further improved device capability, and an electronic device including the organic EL device.

### Solution to Problem

As a result of the continued investigations by the present inventors on the capabilities of organic EL devices containing the compounds described in PTLs 1 to 4, it has been found that a monoamine compound having a dibenzofuran or dibenzothiophene skeleton bonding to the central nitrogen atom via an orthophenylene linking group, having a naphthalene skeleton bonding thereto via a single bond or via a phenylene group or a biphenyldiyl group, and having an aryl group or a heterocyclic group bonding thereto via a single bond or via a specific linking group, can provide an organic EL device having a further improved capability.

In one embodiment, the present invention provides a compound represented by the following formula (1):

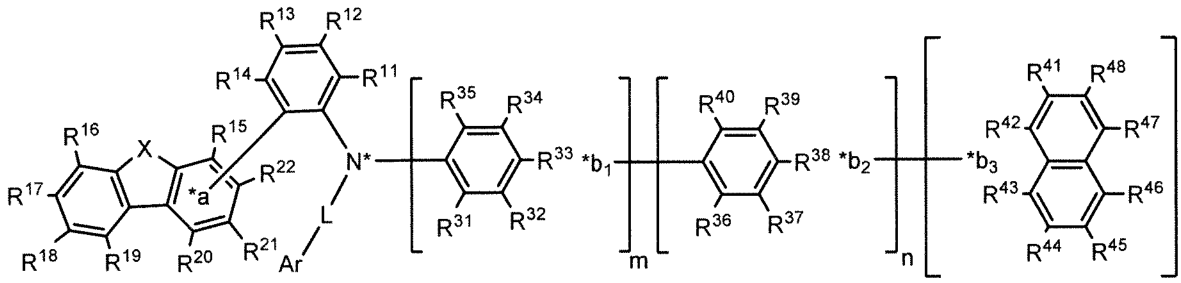

(1)

wherein

N* is a central nitrogen atom;
X represents an oxygen atom or a sulfur atom;
m represents 0 or 1, n represents 0 or 1:

Ar represents a substituted or unsubstituted aryl group having 6 to 50 ring carbon atoms, or a substituted or unsubstituted heterocyclic group having 5 to 50 ring atoms,
provided that when Ar has a substituent, the substituent is each independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group;
L is selected from a single bond, a substituted or unsubstituted arylene group having 6 to 50 ring carbon atoms, and a substituted or unsubstituted 2-valent heterocyclic group having 5 to 50 ring atoms;
$R^{11}$ to $R^{14}$ and $R^{41}$ to $R^{48}$ each are independently selected from a hydrogen atom, and a substituted or unsubstituted alkyl group having 1 to 50 carbon atoms,
provided that one selected from $R^{41}$ to $R^{48}$ is a single bond bonding to $*b_3$;
$R^{15}$ to $R^{16}$, and $R^{18}$ to $R^{22}$ each are independently selected from a hydrogen atom, a substituted or unsubstituted aryl group having 6 to 12 ring carbon atoms, and a substituted or unsubstituted alkyl group having 6 to 18 carbon atoms,
$R^{31}$ to $R^{40}$ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a group represented by $-Si(R_{901})(R_{902})(R_{903})$, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group,
$R^{17}$ is each independently selected from a hydrogen atom, an unsubstituted aryl group having 6 to 12 ring carbon atoms, and a substituted or unsubstituted alkyl group having 6 to 18 carbon atoms,
provided that at least one of $R^{16}$ to $R^{19}$ is a hydrogen atom,
one selected from $R^{15}$, and $R^{20}$ to $R^{22}$ is a single bond bonding to *a, one selected from $R^{31}$ to $R^{35}$ is a single bond bonding to $*b_1$, one selected from $R^{36}$ to $R^{40}$ is a single bond bonding to $*b_2$;
$R_{901}$, $R_{902}$, and $R_{903}$ each are independently selected from a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, and a substituted or unsubstituted cycloalkyl group having 3 to 50 ring carbon atoms; and
adjacent two selected from $R^{11}$ to $R^{14}$, $R^{15}$ to $R^{22}$, $R^{31}$ to $R^{40}$ and $R^{41}$ to $R^{48}$ do not bond to each other to form a ring.

In another embodiment, the present invention provides a material for an organic EL device containing the

compound represented by the formula (1).

In still another embodiment, the present invention provides an organic electroluminescent device including an anode, a cathode, and organic layers intervening between the anode and the cathode, the organic layers including a light emitting layer, at least one layer of the organic layers containing the compound represented by the formula (1).

In a further embodiment, the present invention provides an electronic device including the organic electroluminescent device.

Advantageous Effects of Invention

An organic EL device containing the compound represented by the formula (1) shows an improved device capability.

Brief Description of Drawings

Fig. 1 is a schematic illustration showing an example of the layer configuration of the organic EL device according to one embodiment of the present invention.

Fig. 2 is a schematic illustration showing another example of the layer configuration of the organic EL device according to one embodiment of the present invention.

Description of Embodiment

[Definitions]

In the description herein, the hydrogen atom encompasses isotopes thereof having different numbers of neutrons, i.e., a light hydrogen atom (protium), a heavy hydrogen atom (deuterium), and tritium.

In the description herein, the bonding site where the symbol, such as "R", or "D" representing a deuterium atom is not shown is assumed to have a hydrogen atom, i.e., a protium atom, a deuterium atom, or a tritium atom, bonded thereto.

In the description herein, the number of ring carbon atoms shows the number of carbon atoms among the atoms constituting the ring itself of a compound having a structure including atoms bonded to form a ring (such as a monocyclic compound, a condensed ring compound, a bridged compound, a carbocyclic compound, and a heterocyclic compound). In the case where the ring is substituted by a substituent, the carbon atom contained in the substituent is not included in the number of ring carbon atoms. The same definition is applied to the "number of ring carbon atoms" described hereinafter unless otherwise indicated. For example, a benzene ring has 6 ring carbon atoms, a naphthalene ring has 10 ring carbon atoms, a pyridine ring has 5 ring carbon atoms, and a furan ring has 4 ring carbon atoms. For example, 9,9-diphenylfluorenyl group has 13 ring carbon atoms, and 9,9'-spirobifluorenyl group has 25 ring carbon atoms.

In the case where a benzene ring has, for example, an alkyl group substituted thereon as a substituent, the number of carbon atoms of the alkyl group is not included in the number of ring carbon atoms of the benzene ring. Accordingly, a benzene ring having an alkyl group substituted thereon has 6 ring carbon atoms. In the case where a naphthalene ring has, for example, an alkyl group substituted thereon as a substituent, the number of carbon atoms of the alkyl group is not included in the number of ring carbon atoms of the naphthalene ring. Accordingly, a naphthalene ring having an alkyl group substituted thereon has 10 ring carbon atoms.

In the description herein, the number of ring atoms shows the number of atoms constituting the ring itself of a compound having a structure including atoms bonded to form a ring (such as a monocyclic ring, a condensed ring, and a set of rings) (such as a monocyclic compound, a condensed ring compound, a bridged compound, a carbocyclic compound, and a heterocyclic compound). The atom that does not constitute the ring (such as a hydrogen atom terminating the bond of the atom constituting the ring) and, in the case where the ring is substituted by a substituent, the atom contained in the substituent are not included in the number of ring atoms. The same definition is applied to the "number of ring atoms" described hereinafter unless otherwise indicated. For example, a pyridine ring has 6 ring atoms, a quinazoline ring has 10 ring atoms, and a furan ring has 5 ring atoms. For example, the number of hydrogen atoms bonded to a pyridine ring or atoms constituting a substituent is not included in the number of ring atoms of the pyridine ring. Accordingly, a pyridine ring having a hydrogen atom or a substituent bonded thereto has 6 ring atoms. For example, the number of hydrogen atoms bonded to carbon atoms of a quinazoline ring or atoms constituting a substituent is not included in the number of ring atoms of the quinazoline ring. Accordingly, a quinazoline ring having a hydrogen atom or a substituent bonded thereto has 10 ring atoms.

In the description herein, the expression "having XX to YY carbon atoms" in the expression "substituted or unsubstituted ZZ group having XX to YY carbon atoms" means the number of carbon atoms of the unsubstituted ZZ

group, and, in the case where the ZZ group is substituted, the number of carbon atoms of the substituent is not included. Herein, "YY" is larger than "XX", "XX" represents an integer of 1 or more, and "YY" represents an integer of 2 or more.

In the description herein, the expression "having XX to YY atoms" in the expression "substituted or unsubstituted ZZ group having XX to YY atoms" means the number of atoms of the unsubstituted ZZ group, and, in the case where the ZZ group is substituted, the number of atoms of the substituent is not included. Herein, "YY" is larger than "XX", "XX" represents an integer of 1 or more, and "YY" represents an integer of 2 or more.

In the description herein, an unsubstituted ZZ group means the case where the "substituted or unsubstituted ZZ group" is an "unsubstituted ZZ group", and a substituted ZZ group means the case where the "substituted or unsubstituted ZZ group" is a "substituted ZZ group".

In the description herein, the expression "unsubstituted" in the expression "substituted or unsubstituted ZZ group" means that hydrogen atoms in the ZZ group are not substituted by a substituent. The hydrogen atoms in the "unsubstituted ZZ group" each are a protium atom, a deuterium atom, or a tritium atom.

In the description herein, the expression "substituted" in the expression "substituted or unsubstituted ZZ group" means that one or more hydrogen atom in the ZZ group is substituted by a substituent. The expression "substituted" in the expression "BB group substituted by an AA group" similarly means that one or more hydrogen atom in the BB group is substituted by the AA group.

Substituents in Description

The substituents described in the description herein will be explained.

In the description herein, the number of ring carbon atoms of the "unsubstituted aryl group" is 6 to 50, preferably 6 to 30, and more preferably 6 to 18, unless otherwise indicated in the description.

In the description herein, the number of ring atoms of the "unsubstituted heterocyclic group" is 5 to 50, preferably 5 to 30, and more preferably 5 to 18, unless otherwise indicated in the description.

In the description herein, the number of carbon atoms of the "unsubstituted alkyl group" is 1 to 50, preferably 1 to 20, and more preferably 1 to 6, unless otherwise indicated in the description.

In the description herein, the number of carbon atoms of the "unsubstituted alkenyl group" is 2 to 50, preferably 2 to 20, and more preferably 2 to 6, unless otherwise indicated in the description.

In the description herein, the number of carbon atoms of the "unsubstituted alkynyl group" is 2 to 50, preferably 2 to 20, and more preferably 2 to 6, unless otherwise indicated in the description.

In the description herein, the number of ring carbon atoms of the "unsubstituted cycloalkyl group" is 3 to 50, preferably 3 to 20, and more preferably 3 to 6, unless otherwise indicated in the description.

In the description herein, the number of ring carbon atoms of the "unsubstituted arylene group" is 6 to 50, preferably 6 to 30, and more preferably 6 to 18, unless otherwise indicated in the description.

In the description herein, the number of ring atoms of the "unsubstituted divalent heterocyclic group" is 5 to 50, preferably 5 to 30, and more preferably 5 to 18, unless otherwise indicated in the description.

In the description herein, the number of carbon atoms of the "unsubstituted alkylene group" is 1 to 50, preferably 1 to 20, and more preferably 1 to 6, unless otherwise indicated in the description.

Substituted or Unsubstituted Aryl Group

In the description herein, specific examples (set of specific examples G1) of the "substituted or unsubstituted aryl group" include the unsubstituted aryl groups (set of specific examples G1A) and the substituted aryl groups (set of specific examples G1B) shown below. (Herein, the unsubstituted aryl group means the case where the "substituted or unsubstituted aryl group" is an "unsubstituted aryl group", and the substituted aryl group means the case where the "substituted or unsubstituted aryl group" is a "substituted aryl group".) In the description herein, the simple expression "aryl group" encompasses both the "unsubstituted aryl group" and the "substituted aryl group".

The "substituted aryl group" means a group formed by substituting one or more hydrogen atom of the "unsubstituted aryl group" by a substituent. Examples of the "substituted aryl group" include groups formed by one or more hydrogen atom of each of the "unsubstituted aryl groups" in the set of specific examples G1A by a substituent, and the examples of the substituted aryl groups in the set of specific examples G1B. The examples of the "unsubstituted aryl group" and the examples of the "substituted aryl group" enumerated herein are mere examples, and the "substituted aryl group" in the description herein encompasses groups formed by substituting a hydrogen atom bonded to the carbon atom of the aryl group itself of each of the "substituted aryl groups" in the set of specific examples G1B by a substituent, and groups formed by substituting a hydrogen atom of the substituent of each of the "substituted aryl groups" in the set of specific examples G1B by a substituent.

Unsubstituted Aryl Group (Set of Specific Examples G1A):

a phenyl group,
a p-biphenyl group,
a m-biphenyl group,
an o-biphenyl group,
a p-terphenyl-4-yl group,
a p-terphenyl-3-yl group,
a p-terphenyl-2-yl group,
a m-terphenyl-4-yl group,
a m-terphenyl-3-yl group,
a m-terphenyl-2-yl group,
an o-terphenyl-4-yl group,
an o-terphenyl-3-yl group,
an o-terphenyl-2-yl group,
a 1-naphthyl group,
a 2-naphthyl group,
an anthryl group,
a benzanthryl group,
a phenanthryl group,
a benzophenanthryl group,
a phenarenyl group,
a pyrenyl group,
a chrysenyl group,
a benzochrysenyl group,
a triphenylenyl group,
a benzotriphenylenyl group,
a tetracenyl group,
a pentacenyl group,
a fluorenyl group,
a 9,9'-spirobifluorenyl group,
a benzofluorenyl group,
a dibenzofluorenyl group,
a fluoranthenyl group,
a benzofluoranthenyl group,
a perylenyl group, and
monovalent aryl groups derived by removing one hydrogen atom from each of the ring structures represented by the following general formulae (TEMP-1) to (TEMP-15):

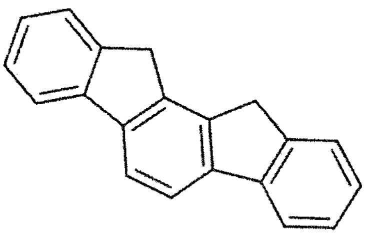
(TEMP-1)

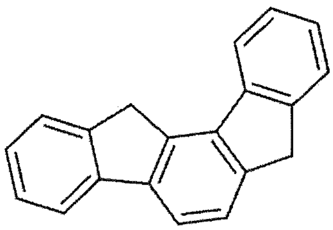
(TEMP-2)

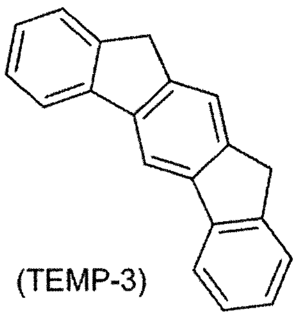
(TEMP-3)

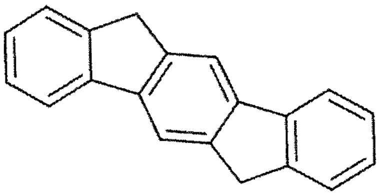

(TEMP-4)

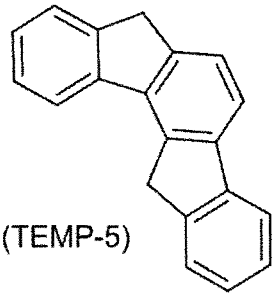

(TEMP-5)

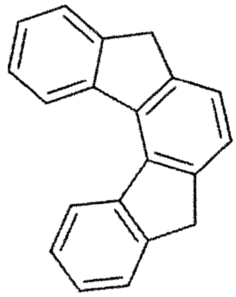

(TEMP-6)

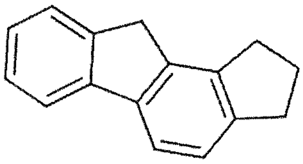

(TEMP-7)

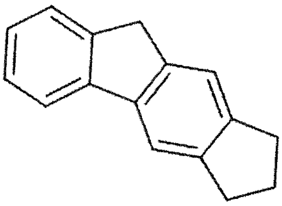

(TEMP-8)

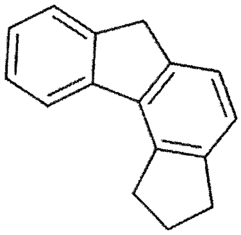

(TEMP-9)

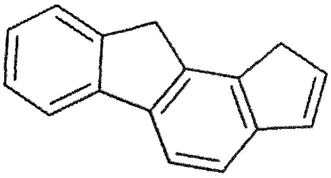

(TEMP-10)

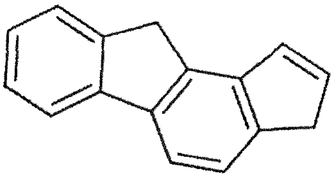

(TEMP-11)

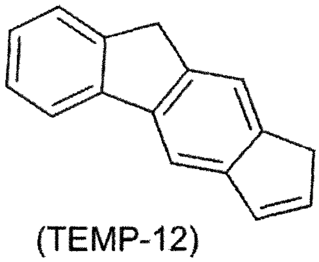

(TEMP-12)

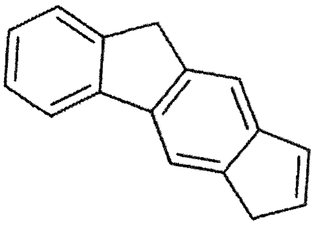

(TEMP-13)

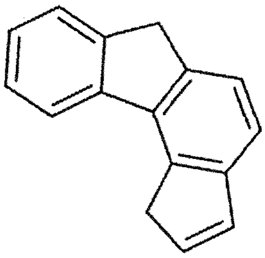

(TEMP-14)

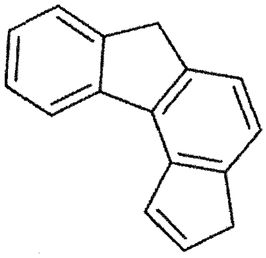

(TEMP-15)

Substituted Aryl Group (Set of Specific Examples G1B):

an o-tolyl group,
a m-tolyl group,
a p-tolyl group,
a p-xylyl group,
a m-xylyl group,
an o-xylyl group,
a p-isopropylphenyl group,
a m-isopropylphenyl group,

an o-isopropylphenyl group,
a p-t-butylphenyl group,
a m-t-butylphenyl group,
a o-t-butylphenyl group,
a 3,4,5-trimethylphenyl group,
a 9,9-dimethylfluorenyl group,
a 9,9-diphenylfluorenyl group,
a 9,9-bis(4-methylphenyl)fluorenyl group,
a 9,9-bis(4-isopropylphenyl)fluorenyl group,
a 9,9-bis(4-t-butylphenyl)fluorenyl group,
a cyanophenyl group,
a triphenylsilylphenyl group,
a trimethylsilylphenyl group,
a phenylnaphthyl group,
a naphthylphenyl group, and
groups formed by substituting one or more hydrogen atom of each of monovalent aryl groups derived from the ring structures represented by the general formulae (TEMP-1) to (TEMP-15) by a substituent.

Substituted or Unsubstituted Heterocyclic Group

In the description herein, the "heterocyclic group" means a cyclic group containing at least one hetero atom in the ring atoms. Specific examples of the hetero atom include a nitrogen atom, an oxygen atom, a sulfur atom, a silicon atom, a phosphorus atom, and a boron atom.

In the description herein, the "heterocyclic group" is a monocyclic group or a condensed ring group.

In the description herein, the "heterocyclic group" is an aromatic heterocyclic group or a non-aromatic heterocyclic group.

In the description herein, specific examples (set of specific examples G2) of the "substituted or unsubstituted heterocyclic group" include the unsubstituted heterocyclic groups (set of specific examples G2A) and the substituted heterocyclic groups (set of specific examples G2B) shown below. (Herein, the unsubstituted heterocyclic group means the case where the "substituted or unsubstituted heterocyclic group" is an "unsubstituted heterocyclic group", and the substituted heterocyclic group means the case where the "substituted or unsubstituted heterocyclic group" is a "substituted heterocyclic group".) In the description herein, the simple expression "heterocyclic group" encompasses both the "unsubstituted heterocyclic group" and the "substituted heterocyclic group".

The "substituted heterocyclic group" means a group formed by substituting one or more hydrogen atom of the "unsubstituted heterocyclic group" by a substituent. Specific examples of the "substituted heterocyclic group" include groups formed by substituting a hydrogen atom of each of the "unsubstituted heterocyclic groups" in the set of specific examples G2A by a substituent, and the examples of the substituted heterocyclic groups in the set of specific examples G2B. The examples of the "unsubstituted heterocyclic group" and the examples of the "substituted heterocyclic group" enumerated herein are mere examples, and the "substituted heterocyclic group" in the description herein encompasses groups formed by substituting a hydrogen atom bonded to the ring atom of the heterocyclic group itself of each of the "substituted heterocyclic groups" in the set of specific examples G2B by a substituent, and groups formed by substituting a hydrogen atom of the substituent of each of the "substituted heterocyclic groups" in the set of specific examples G2B by a substituent.

The set of specific examples G2A includes, for example, the unsubstituted heterocyclic group containing a nitrogen atom (set of specific examples G2A1), the unsubstituted heterocyclic group containing an oxygen atom (set of specific examples G2A2), the unsubstituted heterocyclic group containing a sulfur atom (set of specific examples G2A3), and monovalent heterocyclic groups derived by removing one hydrogen atom from each of the ring structures represented by the following general formulae (TEMP-16) to (TEMP-33) (set of specific examples G2A4).

The set of specific examples G2B includes, for example, the substituted heterocyclic groups containing a nitrogen atom (set of specific examples G2B1), the substituted heterocyclic groups containing an oxygen atom (set of specific examples G2B2), the substituted heterocyclic groups containing a sulfur atom (set of specific examples G2B3), and groups formed by substituting one or more hydrogen atom of each of monovalent heterocyclic groups derived from the ring structures represented by the following general formulae (TEMP-16) to (TEMP-33) by a substituent (set of specific examples G2B4).

Unsubstituted Heterocyclic Group containing Nitrogen Atom (Set of Specific Examples G2A1):

a pyrrolyl group,
an imidazolyl group,

a pyrazolyl group,
a triazolyl group,
a tetrazolyl group,
an oxazolyl group,
an isoxazolyl group,
an oxadiazolyl group,
a thiazolyl group,
an isothiazolyl group,
a thiadiazolyl group,
a pyridyl group,
a pyridazinyl group,
a pyrimidinyl group,
a pyrazinyl group,
a triazinyl group,
an indolyl group,
an isoindolyl group,
an indolizinyl group,
a quinolizinyl group,
a quinolyl group,
an isoquinolyl group,
a cinnolinyl group,
a phthalazinyl group,
a quinazolinyl group,
a quinoxalinyl group,
a benzimidazolyl group,
an indazolyl group,
a phenanthrolinyl group,
a phenanthridinyl group,
an acridinyl group,
a phenazinyl group,
a carbazolyl group,
a benzocarbazolyl group,
a morpholino group,
a phenoxazinyl group,
a phenothiazinyl group,
an azacarbazolyl group, and
a diazacarbazolyl group.

Unsubstituted Heterocyclic Group containing Oxygen Atom (Set of Specific Examples G2A2):

a furyl group,
an oxazolyl group,
an isoxazolyl group,
an oxadiazolyl group,
a xanthenyl group,
a benzofuranyl group,
an isobenzofuranyl group,
a dibenzofuranyl group,
a naphthobenzofuranyl group,
a benzoxazolyl group,
a benzisoxazolyl group,
a phenoxazinyl group,
a morpholino group,
a dinaphthofuranyl group,
an azadibenzofuranyl group,
a diazadibenzofuranyl group,
an azanaphthobenzofuranyl group, and
a diazanaphthobenzofuranyl group.

Unsubstituted Heterocyclic Group containing Sulfur Atom (Set of Specific Examples G2A3):

a thienyl group,
a thiazolyl group,
an isothiazolyl group,
a thiadiazolyl group,
a benzothiophenyl group (benzothienyl group),
an isobenzothiophenyl group (isobenzothienyl group),
a dibenzothiophenyl group (dibenzothienyl group),
a naphthobenzothiophenyl group (naphthobenzothienyl group),
a benzothiazolyl group,
a benzisothiazolyl group,
a phenothiazinyl group,
a dinaphthothiophenyl group (dinaphthothienyl group),
an azadibenzothiophenyl group (azadibenzothienyl group),
a diazadibenzothiophenyl group (diazadibenzothienyl group),
an azanaphthobenzothiophenyl group (azanaphthobenzothienyl group), and
a diazanaphthobenzothiophenyl group (diazanaphthobenzothienyl group).

Monovalent Heterocyclic Group derived by removing One Hydrogen Atom from Ring Structures represented by General Formulae (TEMP-16) to (TEMP-33) (Set of Specific Examples G2A4)

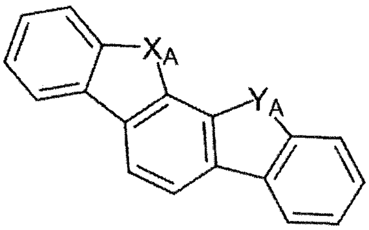
(TEMP-16)

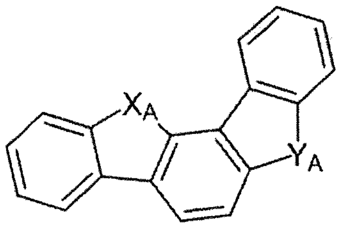
(TEMP-17)

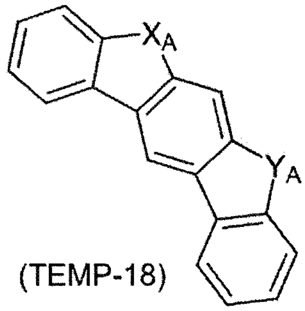
(TEMP-18)

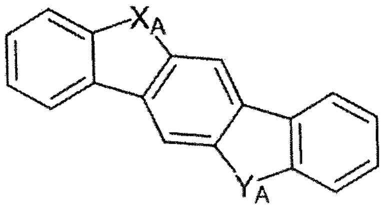
(TEMP-19)

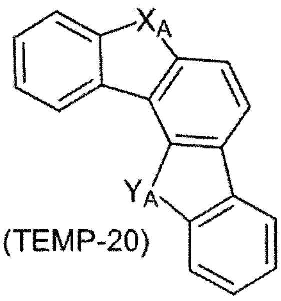
(TEMP-20)

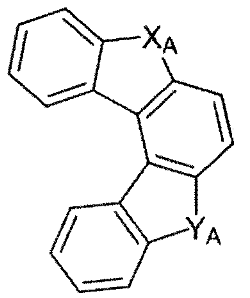
(TEMP-21)

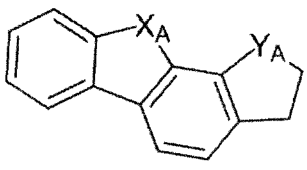
(TEMP-22)

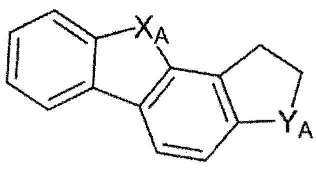
(TEMP-23)

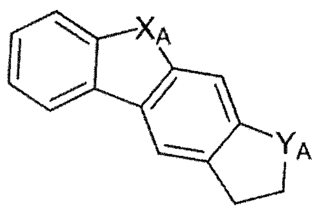
(TEMP-24)

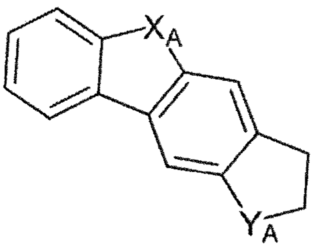

(TEMP-25)

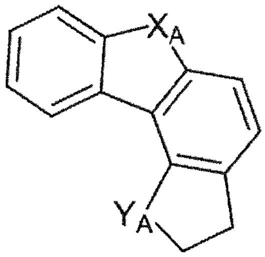

(TEMP-26)

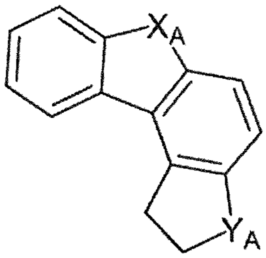

(TEMP-27)

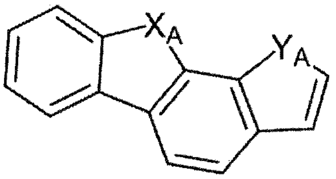

(TEMP-28)

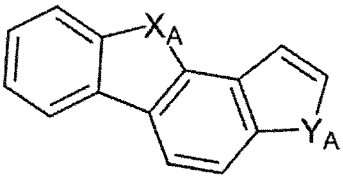

(TEMP-29)

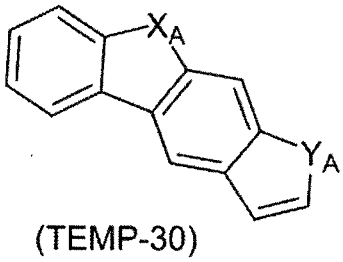

(TEMP-30)

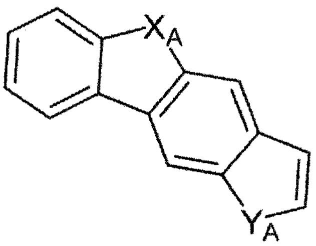

(TEMP-31)

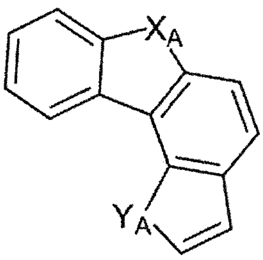

(TEMP-32)

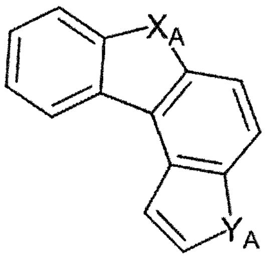

(TEMP-33)

In the general formulae (TEMP-16) to (TEMP-33), $X_A$ and $Y_A$ each independently represent an oxygen atom, a sulfur atom, NH, or $CH_2$, provided that at least one of $X_A$ and $Y_A$ represents an oxygen atom, a sulfur atom, or NH.

In the general formulae (TEMP-16) to (TEMP-33), in the case where at least one of $X_A$ and $Y_A$ represents NH or $CH_2$, the monovalent heterocyclic groups derived from the ring structures represented by the general formulae (TEMP-16) to (TEMP-33) include monovalent groups formed by removing one hydrogen atom from the NH or $CH_2$.

Substituted Heterocyclic Group containing Nitrogen Atom (Set of Specific Examples G2B1):

a (9-phenyl)carbazolyl group,
a (9-biphenylyl)carbazolyl group,
a (9-phenyl)phenylcarbazolyl group,
a (9-naphthyl)carbazolyl group,
a diphenylcarbazol-9-yl group,
a phenylcarbazol-9-yl group,
a methylbenzimidazolyl group,
an ethylbenzimidazolyl group,
a phenyltriazinyl group,
a biphenyltriazinyl group,
a diphenyltriazinyl group,
a phenylquinazolinyl group, and
a biphenylquinazolinyl group.

Substituted Heterocyclic Group containing Oxygen Atom (Set of Specific Examples G2B2):

a phenyldibenzofuranyl group,
a methyldibenzofuranyl group,
a t-butyldibenzofuranyl group, and
a monovalent residual group of spiro[9H-xanthene-9,9'-[9H]fluorene].

Substituted Heterocyclic Group containing Sulfur Atom (Set of Specific Examples G2B3):

a phenyldibenzothiophenyl group,
a methyldibenzothiophenyl group,
a t-butyldibenzothiophenyl group, and
a monovalent residual group of spiro[9H-thioxanthene-9,9'-[9H]fluorene].

Group formed by substituting one or more Hydrogen Atom of Monovalent Heterocyclic Group derived from Ring Structures represented by General Formulae (TEMP-16) to (TEMP-33) by Substituent (Set of Specific Examples G2B4)

The "one or more hydrogen atom of the monovalent heterocyclic group" means one or more hydrogen atom selected from the hydrogen atom bonded to the ring carbon atom of the monovalent heterocyclic group, the hydrogen atom bonded to the nitrogen atom in the case where at least one of $X_A$ and $Y_A$ represents NH, and the hydrogen atom of the methylene group in the case where one of $X_A$ and $Y_A$ represents $CH_2$.

Substituted or Unsubstituted Alkyl Group

In the description herein, specific examples (set of specific examples G3) of the "substituted or unsubstituted alkyl group" include the unsubstituted alkyl groups (set of specific examples G3A) and the substituted alkyl groups (set of specific examples G3B) shown below. (Herein, the unsubstituted alkyl group means the case where the "substituted or unsubstituted alkyl group" is an "unsubstituted alkyl group", and the substituted alkyl group means the case where the "substituted or unsubstituted alkyl group" is a "substituted alkyl group".) In the description herein, the simple expression "alkyl group" encompasses both the "unsubstituted alkyl group" and the "substituted alkyl group".

The "substituted alkyl group" means a group formed by substituting one or more hydrogen atom of the "unsubstituted alkyl group" by a substituent. Specific examples of the "substituted alkyl group" include groups formed by substituting one or more hydrogen atom of each of the "unsubstituted alkyl groups" (set of specific examples G3A) by a substituent, and the examples of the substituted alkyl groups (set of specific examples G3B). In the description herein, the alkyl group in the "unsubstituted alkyl group" means a chain-like alkyl group. Accordingly, the "unsubstituted alkyl group" encompasses an "unsubstituted linear alkyl group" and an "unsubstituted branched alkyl group". The examples of the "unsubstituted alkyl group" and the examples of the "substituted alkyl group" enumerated herein are mere examples, and the "substituted alkyl group" in the description herein encompasses groups formed by substituting a hydrogen atom of the alkyl group itself of each of the "substituted alkyl groups" in the set of specific examples G3B by a substituent, and groups formed by substituting a hydrogen atom of the substituent of each of the "substituted alkyl groups" in the set of specific examples G3B by a substituent.

Unsubstituted Alkyl Group (Set of Specific Examples G3A):

a methyl group,
an ethyl group,
a n-propyl group,
an isopropyl group,
a n-butyl group,
an isobutyl group,
a s-butyl group, and
a t-butyl group.

Substituted Alkyl Group (Set of Specific Examples G3B):

a heptafluoropropyl group (including isomers),
a pentafluoroethyl group,
a 2,2,2-trifluoroethyl group, and
a trifluoromethyl group.

Substituted or Unsubstituted Alkenyl Group

In the description herein, specific examples (set of specific examples G4) of the "substituted or unsubstituted alkenyl group" include the unsubstituted alkenyl groups (set of specific examples G4A) and the substituted alkenyl groups (set of specific examples G4B) shown below. (Herein, the unsubstituted alkenyl group means the case where the "substituted or unsubstituted alkenyl group" is an "unsubstituted alkenyl group", and the substituted alkenyl group means the case where the "substituted or unsubstituted alkenyl group" is a "substituted alkenyl group".) In the description herein, the simple expression "alkenyl group" encompasses both the "unsubstituted alkenyl group" and the "substituted alkenyl group".

The "substituted alkenyl group" means a group formed by substituting one or more hydrogen atom of the "unsubstituted alkenyl group" by a substituent. Specific examples of the "substituted alkenyl group" include the "unsubstituted alkenyl groups" (set of specific examples G4A) that each have a substituent, and the examples of the substituted alkenyl groups (set of specific examples G4B). The examples of the "unsubstituted alkenyl group" and the examples of the "substituted alkenyl group" enumerated herein are mere examples, and the "substituted alkenyl group" in the description herein encompasses groups formed by substituting a hydrogen atom of the alkenyl group itself of each of the "substituted alkenyl groups" in the set of specific examples G4B by a substituent, and groups formed by substituting a hydrogen atom of the substituent of each of the "substituted alkenyl groups" in the set of specific examples G4B by a substituent.

Unsubstituted Alkenyl Group (Set of Specific Examples G4A):

a vinyl group,
an allyl group,
a 1-butenyl group,
a 2-butenyl group, and
a 3-butenyl group.

Substituted Alkenyl Group (Set of Specific Examples G4B):

a 1,3-butanedienyl group,
a 1-methylvinyl group,
a 1-methylallyl group,
a 1,1-dimethylallyl group,
a 2-methylallyl group, and
a 1,2-dimethylallyl group.

Substituted or Unsubstituted Alkynyl Group

In the description herein, specific examples (set of specific examples G5) of the "substituted or unsubstituted alkynyl group" include the unsubstituted alkynyl group (set of specific examples G5A) shown below. (Herein, the unsubstituted alkynyl group means the case where the "substituted or unsubstituted alkynyl group" is an "unsubstituted alkynyl group".) In the description herein, the simple expression "alkynyl group" encompasses both the "unsubstituted alkynyl group" and the "substituted alkynyl group".

The "substituted alkynyl group" means a group formed by substituting one or more hydrogen atom of the "unsubstituted alkynyl group" by a substituent. Specific examples of the "substituted alkenyl group" include groups formed by substituting one or more hydrogen atom of the "unsubstituted alkynyl group" (set of specific examples G5A) by a substituent.

Unsubstituted Alkynyl Group (Set of Specific Examples G5A):
an ethynyl group.

Substituted or Unsubstituted Cycloalkyl Group

In the description herein, specific examples (set of specific examples G6) of the "substituted or unsubstituted cycloalkyl group" include the unsubstituted cycloalkyl groups (set of specific examples G6A) and the substituted cycloalkyl group (set of specific examples G6B) shown below. (Herein, the unsubstituted cycloalkyl group means the case where the "substituted or unsubstituted cycloalkyl group" is an "unsubstituted cycloalkyl group", and the substituted cycloalkyl group means the case where the "substituted or unsubstituted cycloalkyl group" is a "substituted cycloalkyl group".) In the description herein, the simple expression "cycloalkyl group" encompasses both the "unsubstituted cycloalkyl group" and the "substituted cycloalkyl group".

The "substituted cycloalkyl group" means a group formed by substituting one or more hydrogen atom of the "unsubstituted cycloalkyl group" by a substituent. Specific examples of the "substituted cycloalkyl group" include groups formed by substituting one or more hydrogen atom of each of the "unsubstituted cycloalkyl groups" (set of specific examples G6A) by a substituent, and the example of the substituted cycloalkyl group (set of specific examples G6B). The examples of the "unsubstituted cycloalkyl group" and the examples of the "substituted cycloalkyl group" enumerated herein are mere examples, and the "substituted cycloalkyl group" in the description herein encompasses groups formed by substituting one or more hydrogen atom bonded to the carbon atoms of the cycloalkyl group itself of the "substituted cycloalkyl group" in the set of specific examples G6B by a substituent, and groups formed by substituting a hydrogen atom of the substituent of the "substituted cycloalkyl group" in the set of specific examples G6B by a substituent.

Unsubstituted Cycloalkyl Group (Set of Specific Examples G6A):

a cyclopropyl group,
a cyclobutyl group,
a cyclopentyl group,
a cyclohexyl group,
a 1-adamantyl group,
a 2-adamantyl group,
a 1-norbornyl group, and
a 2-norbornyl group.

Substituted Cycloalkyl Group (Set of Specific Examples G6B):
a 4-methylcyclohexyl group.

Group represented by $-Si(R_{901})(R_{902})(R_{903})$

In the description herein, specific examples (set of specific examples G7) of the group represented by $-Si(R_{901})(R_{902})(R_{903})$ include:

-Si(G1)(G1)(G1),
-Si(G1)(G2)(G2),
-Si(G1)(G1)(G2),
-Si(G2)(G2)(G2),
-Si(G3)(G3)(G3), and
-Si(G6)(G6)(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.
Plural groups represented by G1 in -Si(G1)(G1)(G1) are the same as or different from each other.
Plural groups represented by G2 in -Si(G1)(G2)(G2) are the same as or different from each other.
Plural groups represented by G1 in -Si(G1)(G1)(G2) are the same as or different from each other.
Plural groups represented by G2 in -Si(G2)(G2)(G2) are the same as or different from each other.
Plural groups represented by G3 in -Si(G3)(G3)(G3) are the same as or different from each other.
Plural groups represented by G6 in -Si(G6)(G6)(G6) are the same as or different from each other.

Group represented by $-O-(R_{904})$

In the description herein, specific examples (set of specific examples G8) of the group represented by $-O-(R_{904})$ include:

-O(G1),
-O(G2),
-O(G3), and
-O(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Group represented by $-S-(R_{905})$

In the description herein, specific examples (set of specific examples G9) of the group represented by $-S-(R_{905})$ include:

-S(G1),
-S(G2),
-S(G3), and
-S(G6).

Herein,

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.

Group represented by $-N(R_{906})(R_{907})$

In the description herein, specific examples (set of specific examples G10) of the group represented by $-N(P_{906})(P_{907})$ include:

-N(G1)(G1),
- N(G2)(G2),
- N(G1)(G2),
- N(G3)(G3), and
- N(G6)(G6).

G1 represents the "substituted or unsubstituted aryl group" described in the set of specific examples G1,
G2 represents the "substituted or unsubstituted heterocyclic group" described in the set of specific examples G2,
G3 represents the "substituted or unsubstituted alkyl group" described in the set of specific examples G3, and
G6 represents the "substituted or unsubstituted cycloalkyl group" described in the set of specific examples G6.
Plural groups represented by G1 in -N(G1)(G1) are the same as or different from each other.
Plural groups represented by G2 in -N(G2)(G2) are the same as or different from each other.
Plural groups represented by G3 in -N(G3)(G3) are the same as or different from each other.
Plural groups represented by G6 in -N(G6)(G6) are the same as or different from each other.

Halogen Atom

In the description herein, specific examples (set of specific examples G11) of the "halogen atom" include a fluorine atom, a chlorine atom, a bromine atom, and an iodine atom.

Substituted or Unsubstituted Fluoroalkyl Group

In the description herein, the "substituted or unsubstituted fluoroalkyl group" means a group formed by substituting at least one hydrogen atom bonded to the carbon atom constituting the alkyl group in the "substituted or unsubstituted alkyl group" by a fluorine atom, and encompasses a group formed by substituting all the hydrogen atoms bonded to the carbon atoms constituting the alkyl group in the "substituted or unsubstituted alkyl group" by fluorine atoms (i.e., a perfluoroalkyl group). The number of carbon atoms of the "unsubstituted fluoroalkyl group" is 1 to 50, preferably 1 to 30, and more preferably 1 to 18, unless otherwise indicated in the description. The "substituted fluoroalkyl group" means a group formed by substituting one or more hydrogen atom of the "fluoroalkyl group" by a substituent. In the description

herein, the "substituted fluoroalkyl group" encompasses a group formed by substituting one or more hydrogen atom bonded to the carbon atom of the alkyl chain in the "substituted fluoroalkyl group" by a substituent, and a group formed by substituting one or more hydrogen atom of the substituent in the "substituted fluoroalkyl group" by a substituent. Specific examples of the "unsubstituted fluoroalkyl group" include examples of groups formed by substituting one or more hydrogen atom in each of the "alkyl group" (set of specific examples G3) by a fluorine atom.

Substituted or Unsubstituted Haloalkyl Group

In the description herein, the "substituted or unsubstituted haloalkyl group" means a group formed by substituting at least one hydrogen atom bonded to the carbon atom constituting the alkyl group in the "substituted or unsubstituted alkyl group" by a halogen atom, and encompasses a group formed by substituting all the hydrogen atoms bonded to the carbon atoms constituting the alkyl group in the "substituted or unsubstituted alkyl group" by halogen atoms. The number of carbon atoms of the "unsubstituted haloalkyl group" is 1 to 50, preferably 1 to 30, and more preferably 1 to 18, unless otherwise indicated in the description. The "substituted haloalkyl group" means a group formed by substituting one or more hydrogen atom of the "haloalkyl group" by a substituent. In the description herein, the "substituted haloalkyl group" encompasses a group formed by substituting one or more hydrogen atom bonded to the carbon atom of the alkyl chain in the "substituted haloalkyl group" by a substituent, and a group formed by substituting one or more hydrogen atom of the substituent in the "substituted haloalkyl group" by a substituent. Specific examples of the "unsubstituted haloalkyl group" include examples of groups formed by substituting one or more hydrogen atom in each of the "alkyl group" (set of specific examples G3) by a halogen atom. A haloalkyl group may be referred to as a halogenated alkyl group in some cases.

Substituted or Unsubstituted Alkoxy Group

In the description herein, specific examples of the "substituted or unsubstituted alkoxy group" include a group represented by -O(G3), wherein G3 represents the "substituted or unsubstituted alkyl group" described in the set of specific examples G3. The number of carbon atoms of the "unsubstituted alkoxy group" is 1 to 50, preferably 1 to 30, and more preferably 1 to 18, unless otherwise indicated in the description.

Substituted or Unsubstituted Alkylthio Group

In the description herein, specific examples of the "substituted or unsubstituted alkylthio group" include a group represented by -S(G3), wherein G3 represents the "substituted or unsubstituted alkyl group" described in the set of specific examples G3. The number of carbon atoms of the "unsubstituted alkylthio group" is 1 to 50, preferably 1 to 30, and more preferably 1 to 18, unless otherwise indicated in the description.

Substituted or Unsubstituted Aryloxy Group

In the description herein, specific examples of the "substituted or unsubstituted aryloxy group" include a group represented by -O(G1), wherein G1 represents the "substituted or unsubstituted aryl group" described in the set of specific examples G1. The number of ring carbon atoms of the "unsubstituted aryloxy group" is 6 to 50, preferably 6 to 30, and more preferably 6 to 18, unless otherwise indicated in the description.

Substituted or Unsubstituted Arylthio Group

In the description herein, specific examples of the "substituted or unsubstituted arylthio group" include a group represented by -S(G1), wherein G1 represents the "substituted or unsubstituted aryl group" described in the set of specific examples G1. The number of ring carbon atoms of the "unsubstituted arylthio group" is 6 to 50, preferably 6 to 30, and more preferably 6 to 18, unless otherwise indicated in the description.

Substituted or Unsubstituted Trialkylsilyl Group

In the description herein, specific examples of the "trialkylsilyl group" include a group represented by -Si(G3)(G3)(G3), wherein G3 represents the "substituted or unsubstituted alkyl group" described in the set of specific examples G3. Plural groups represented by G3 in -Si(G3)(G3)(G3) are the same as or different from each other. The number of carbon atoms of each of alkyl groups of the "substituted or unsubstituted trialkylsilyl group" is 1 to 50, preferably 1 to 20, and more preferably 1 to 6, unless otherwise indicated in the description.

Substituted or Unsubstituted Aralkyl Group

In the description herein, specific examples of the "substituted or unsubstituted aralkyl group" include a group represented by -(G3)-(G1), wherein G3 represents the "substituted or unsubstituted alkyl group" described in the set of specific examples G3, and G1 represents the "substituted or unsubstituted aryl group" described in the set of specific examples G1. Accordingly, the "aralkyl group" is a group formed by substituting a hydrogen atom of an "alkyl group" by an "aryl group" as a substituent, and is one embodiment of the "substituted alkyl group". The "unsubstituted aralkyl group" is an "unsubstituted alkyl group" that is substituted by an "unsubstituted aryl group", and the number of carbon atoms of the "unsubstituted aralkyl group" is 7 to 50, preferably 7 to 30, and more preferably 7 to 18, unless otherwise indicated in the description.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, and a 2-β-naphthylisopropyl group.

In the description herein, the substituted or unsubstituted aryl group is preferably a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group, a 9,9-diphenylfluorenyl group, and the like, unless otherwise indicated in the description.

In the description herein, the substituted or unsubstituted heterocyclic group is preferably a pyridyl group, a pyrimidinyl group, a triazinyl group, a quinolyl group, an isoquinolyl group, a quinazolinyl group, a benzimidazolyl group, a phenanthrolinyl group, a carbazolyl group (e.g., a 1-carbazolyl, group, a 2-carbazolyl, group, a 3-carbazolyl, group, a 4-carbazolyl, group, or a 9-carbazolyl, group), a benzocarbazolyl group, an azacarbazolyl group, a diazacarbazolyl group, a dibenzofuranyl group, a naphthobenzofuranly group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (e.g., a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like, unless otherwise indicated in the description.

In the description herein, the carbazolyl group is specifically any one of the following groups unless otherwise indicated in the description.

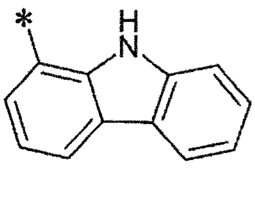

(TEMP-Cz1)

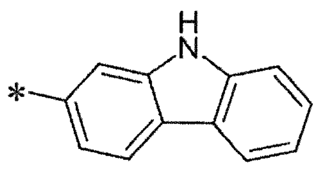

(TEMP-Cz2)

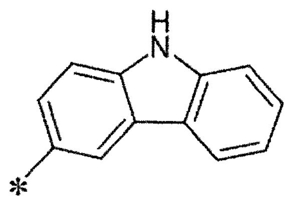

(TEMP-Cz3)

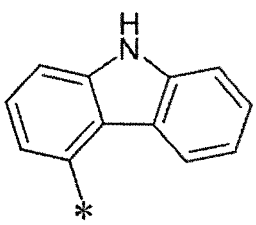

(TEMP-Cz4)

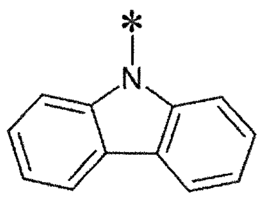

(TEMP-Cz5)

In the description herein, the (9-phenyl)carbazolyl group is specifically any one of the following groups unless otherwise indicated in the description.

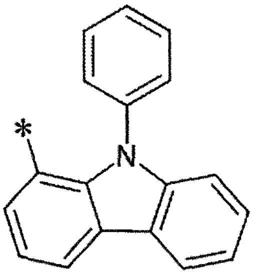

(TEMP-Cz6)

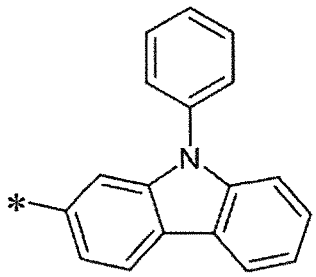

(TEMP-Cz7)

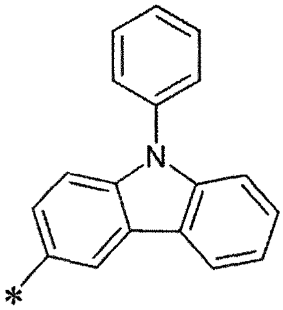

(TEMP-Cz8)

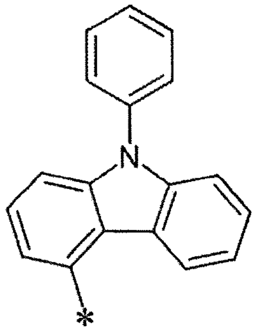

(TEMP-Cz9)

In the general formulae (TEMP-Czl) to (TEMP-Cz9), * represents a bonding site.

In the description herein, the dibenzofuranyl group and the dibenzothiophenyl group are specifically any one of the following groups unless otherwise indicated in the description.

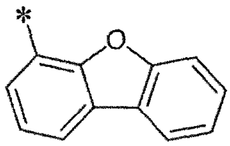

(TEMP-34)

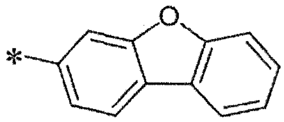

(TEMP-35)

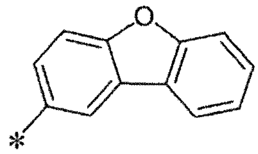

(TEMP-36)

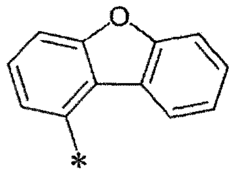

(TEMP-37)

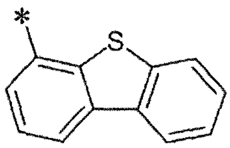

(TEMP-38)

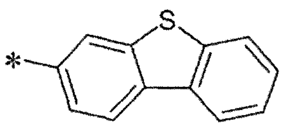

(TEMP-39)

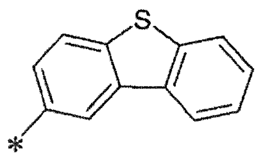

(TEMP-40)

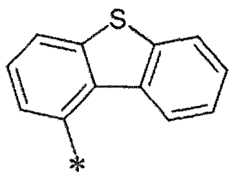

(TEMP-41)

In the general formulae (TEMP-34) to (TEMP-41), * represents a bonding site.

In the description herein, the substituted or unsubstituted alkyl group is preferably a methyl group, an ethyl group, a propyl group, an isopropyl group, a n-butyl group, an isobutyl group, a t-butyl group, or the like unless otherwise indicated in the description.

Substituted or Unsubstituted Arylene Group

In the description herein, the "substituted or unsubstituted arylene group" is a divalent group derived by removing one hydrogen atom on the aryl ring from the "substituted or unsubstituted aryl group" described above unless otherwise indicated in the description. Specific examples (set of specific examples G12) of the "substituted or unsubstituted arylene group" include divalent groups derived by removing one hydrogen atom on the aryl ring from the "substituted or unsubstituted aryl groups" described in the set of specific examples G1.

Substituted or Unsubstituted Divalent Heterocyclic Group

In the description herein, the "substituted or unsubstituted divalent heterocyclic group" is a divalent group derived by removing one hydrogen atom on the heterocyclic ring from the "substituted or unsubstituted heterocyclic group" described above unless otherwise indicated in the description. Specific examples (set of specific examples G13) of the "substituted or unsubstituted divalent heterocyclic group" include divalent groups derived by removing one hydrogen atom on the heterocyclic ring from the "substituted or unsubstituted heterocyclic groups" described in the set of specific examples G2.

Substituted or Unsubstituted Alkylene Group

In the description herein, the "substituted or unsubstituted alkylene group" is a divalent group derived by removing one hydrogen atom on the alkyl chain from the "substituted or unsubstituted alkyl group" described above unless otherwise indicated in the description. Specific examples (set of specific examples G14) of the "substituted or unsubstituted alkylene group" include divalent groups derived by removing one hydrogen atom on the alkyl chain from the "substituted or unsubstituted alkyl groups" described in the set of specific examples G3.

In the description herein, the substituted or unsubstituted arylene group is preferably any one of the groups represented by the following general formulae (TEMP-42) to (TEMP-68) unless otherwise indicated in the description.

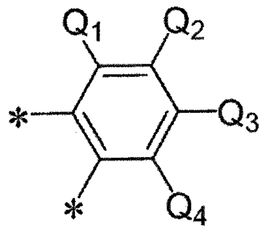

(TEMP-42)

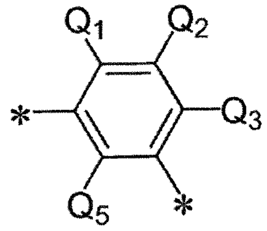

(TEMP-43)

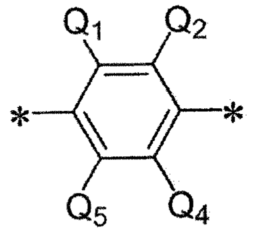

(TEMP-44)

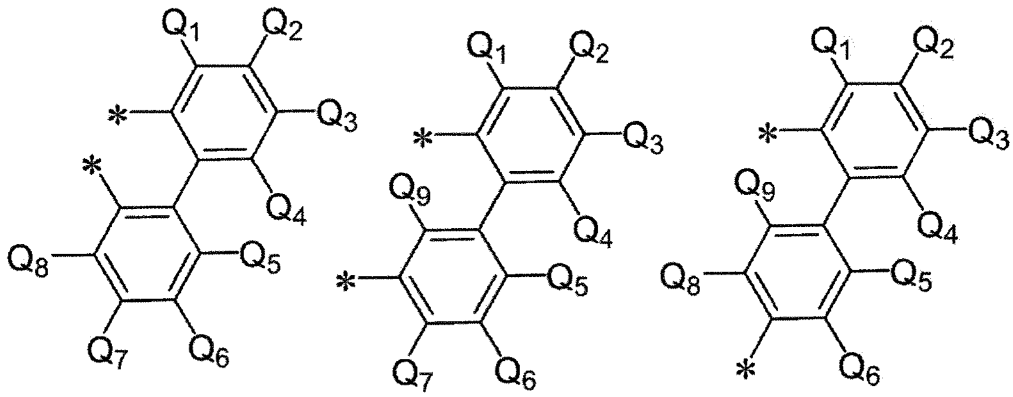

(TEMP-45) (TEMP-46) (TEMP-47)

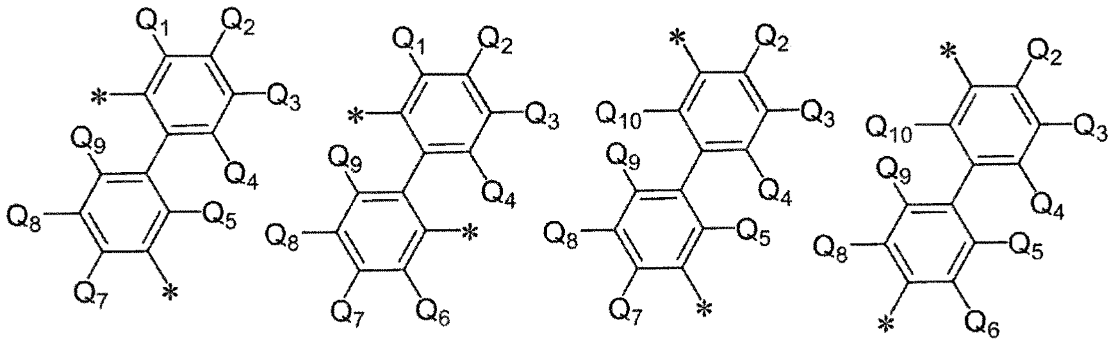

(TEMP-48) (TEMP-49) (TEMP-50) (TEMP-51)

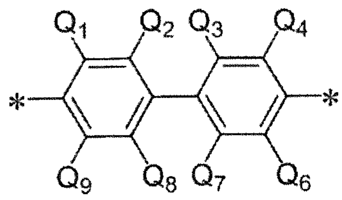

(TEMP-52)

In the general formulae (TEMP-42) to (TEMP-52), $Q_1$ to Qio each independently represent a hydrogen atom or a substituent.

In the general formulae (TEMP-42) to (TEMP-52), * represents a bonding site.

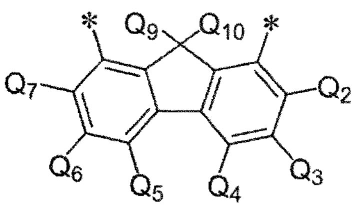

(TEMP-53)

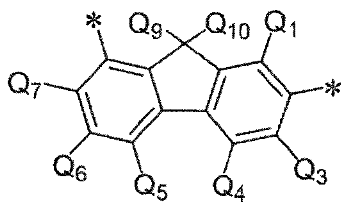

(TEMP-54)

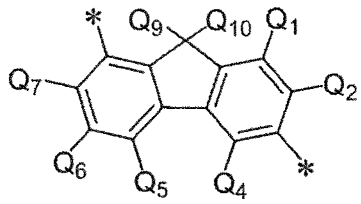

(TEMP-55)

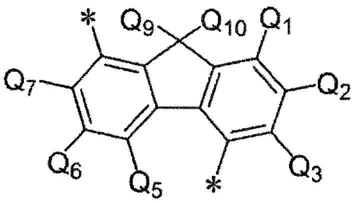

(TEMP-56)

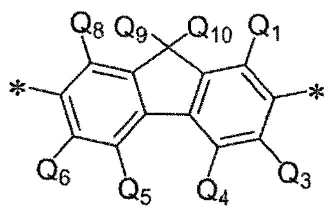

(TEMP-57)

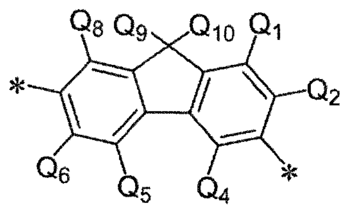

(TEMP-58)

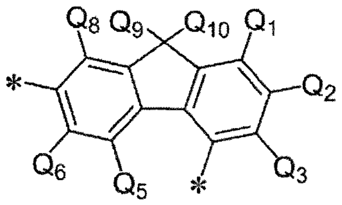

(TEMP-59)

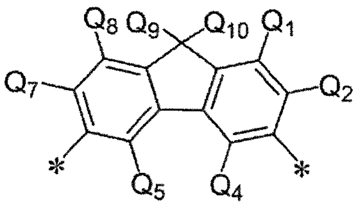

(TEMP-60)

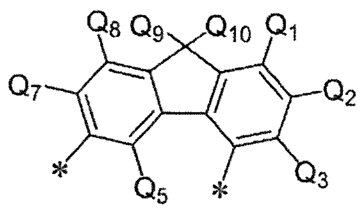

(TEMP-61)

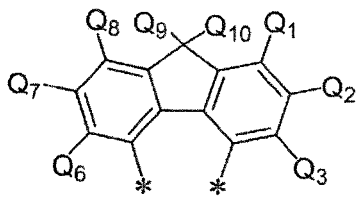

(TEMP-62)

In the general formulae (TEMP-53) to (TEMP-62), $Q_1$ to $Q_{10}$ each independently represent a hydrogen atom or a substituent.

The formulae $Q_9$ and $Q_{10}$ may be bonded to each other to form a ring via a single bond.

In the general formulae (TEMP-53) to (TEMP-62), * represents a bonding site.

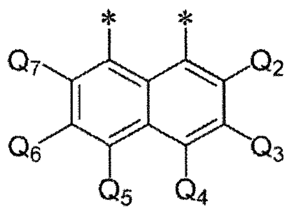
(TEMP-63)

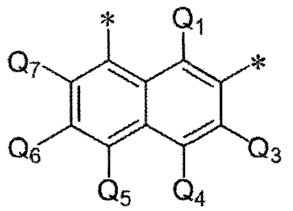
(TEMP-64)

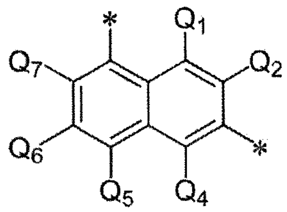
(TEMP-65)

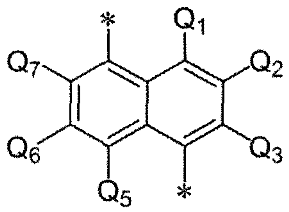
(TEMP-66)

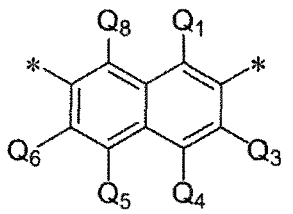
(TEMP-67)

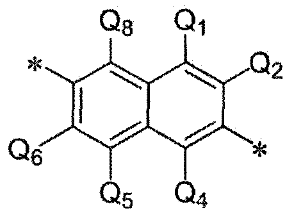
(TEMP-68)

In the general formulae (TEMP-63) to (TEMP-68), $Q_1$ to $Q_8$ each independently represent a hydrogen atom or a substituent.

In the general formulae (TEMP-63) to (TEMP-68), * represents a bonding site.

In the description herein, the substituted or unsubstituted divalent heterocyclic group is preferably the groups represented by the following general formulae (TEMP-69) to (TEMP-102) unless otherwise indicated in the description.

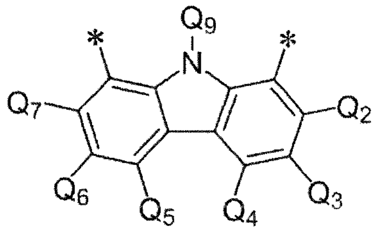
(TEMP-69)

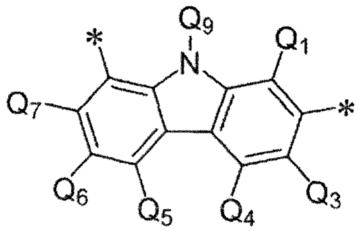
(TEMP-70)

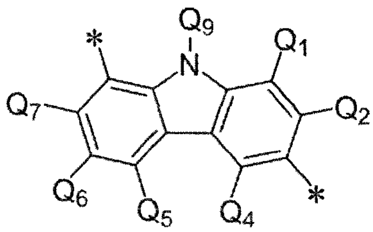
(TEMP-71)

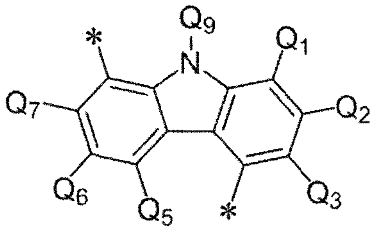
(TEMP-72)

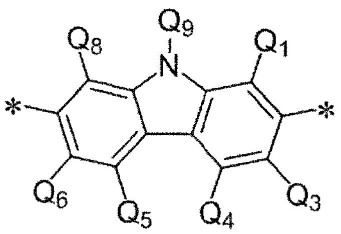
(TEMP-73)

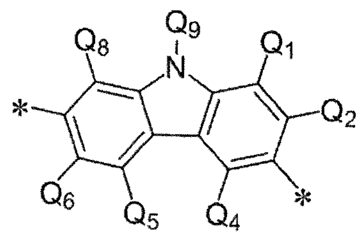
(TEMP-74)

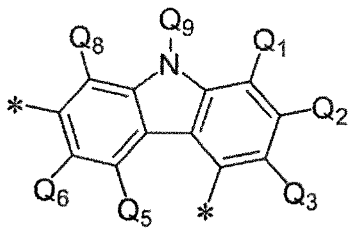
(TEMP-75)

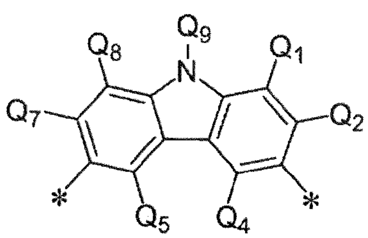
(TEMP-76)

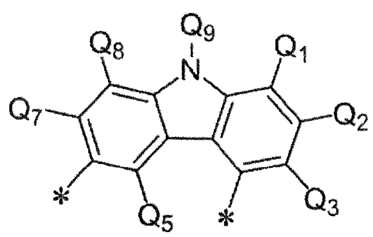
(TEMP-77)

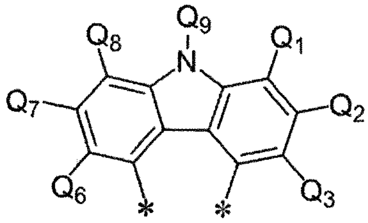

(TEMP-78)

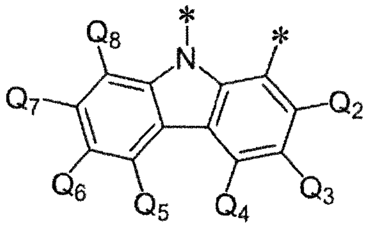

(TEMP-79)

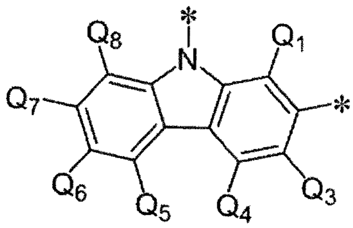

(TEMP-80)

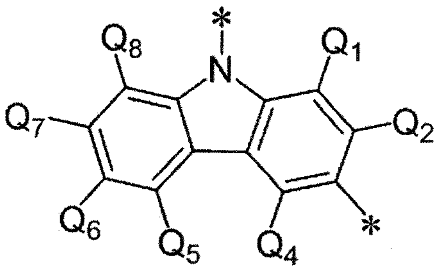

(TEMP-81)

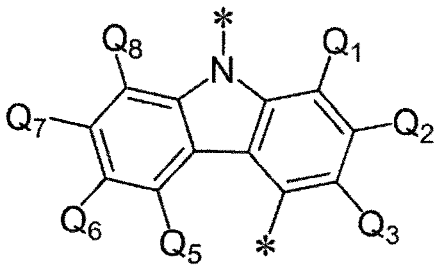

(TEMP-82)

In the general formulae (TEMP-69) to (TEMP-82), $Q_1$ to $Q_9$ each independently represent a hydrogen atom or a substituent.

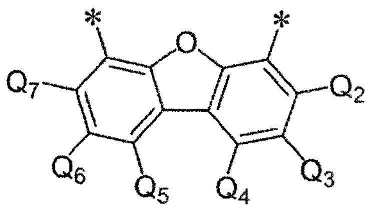

(TEMP-83)

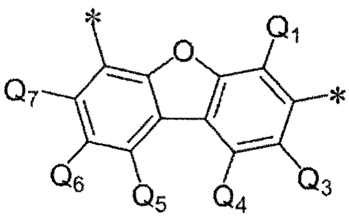

(TEMP-84)

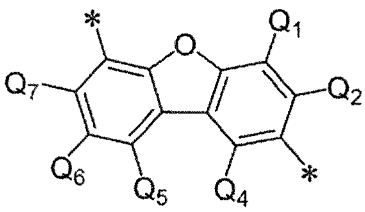

(TEMP-85)

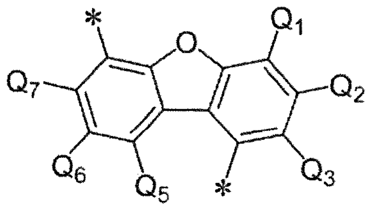

(TEMP-86)

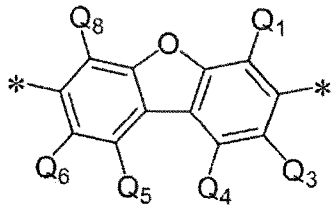

(TEMP-87)

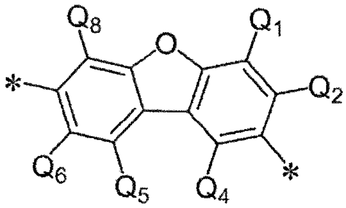

(TEMP-88)

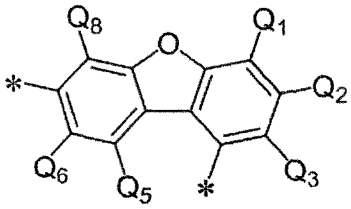

(TEMP-89)

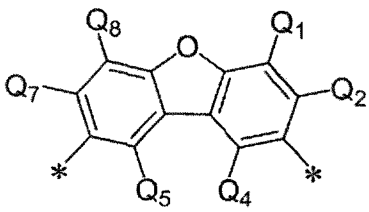

(TEMP-90)

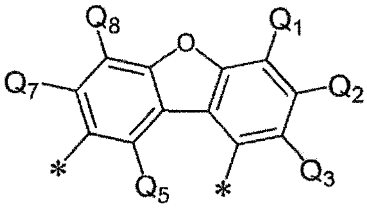

(TEMP-91)

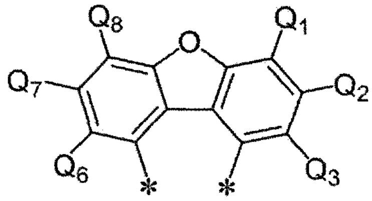

(TEMP-92)

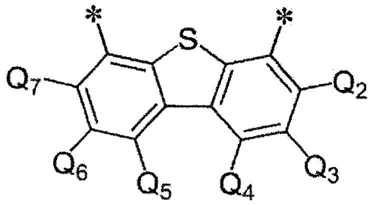

(TEMP-93)

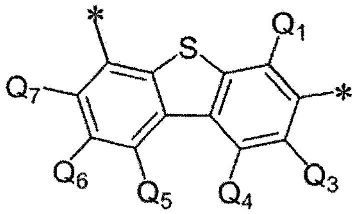

(TEMP-94)

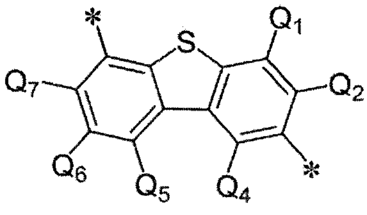

(TEMP-95)

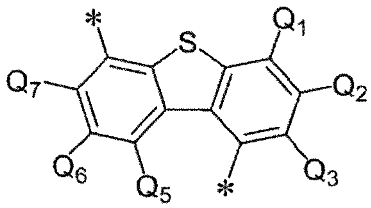

(TEMP-96)

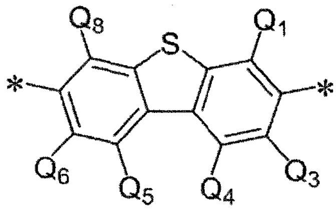

(TEMP-97)

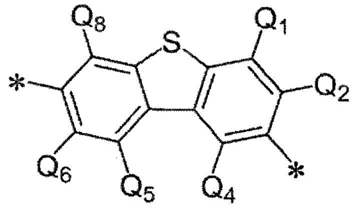

(TEMP-98)

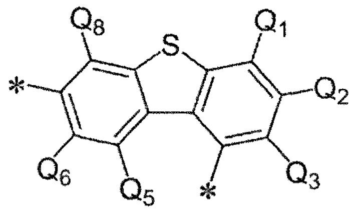

(TEMP-99)

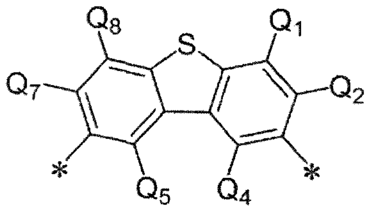

(TEMP-100)

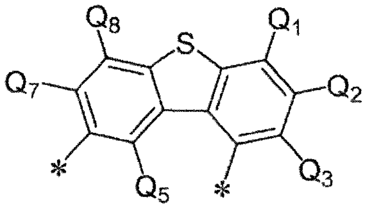

(TEMP-101)

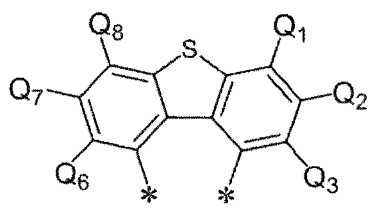

(TEMP-102)

In the general formulae (TEMP-83) to (TEMP-102), $Q_1$ to $Q_8$ each independently represent a hydrogen atom or a substituent.

The above are the explanation of the "substituents in the description herein".

Case forming Ring by bonding

In the description herein, the case where "one or more combinations of combinations each including adjacent two or more each are bonded to each other to form a substituted or unsubstituted monocyclic ring, or each are bonded

to each other to form a substituted or unsubstituted condensed ring, or each are not bonded to each other" means a case where "one or more combinations of combinations each including adjacent two or more each are bonded to each other to form a substituted or unsubstituted monocyclic ring", a case where "one or more combinations of combinations each including adjacent two or more each are bonded to each other to form a substituted or unsubstituted condensed ring", and a case where "one or more combinations of combinations each including adjacent two or more each are not bonded to each other".

In the description herein, the case where "one or more combinations of combinations each including adjacent two or more each are bonded to each other to form a substituted or unsubstituted monocyclic ring" and the case where "one or more combinations of combinations each including adjacent two or more each are bonded to each other to form a substituted or unsubstituted condensed ring" (which may be hereinafter collectively referred to as a "case forming a ring by bonding") will be explained below. The cases will be explained for the anthracene compound represented by the following general formula (TEMP-103) having an anthracene core skeleton as an example.

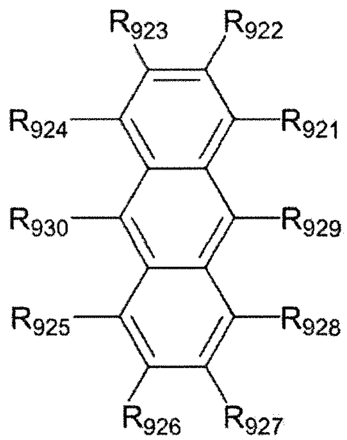

(TEMP-103)

For example, in the case where "one or more combinations of combinations each including adjacent two or more each are bonded to each other to form a ring" among $R_{921}$ to $R_{930}$, the combinations each including adjacent two as one combination include a combination of $R_{921}$ and $R_{922}$, a combination of $R_{922}$ and $R_{923}$, a combination of $R_{923}$ and $R_{924}$, a combination of $R_{924}$ and $R_{930}$, a combination of $R_{930}$ and $R_{925}$, a combination of $R_{925}$ and $R_{926}$, a combination of $R_{926}$ and $R_{927}$, a combination of $R_{927}$ and $R_{928}$, a combination of $R_{928}$ and $R_{929}$, and a combination of $R_{929}$ and $R_{921}$.

The "one or more combinations" mean that two or more combinations each including adjacent two or more may form rings simultaneously. For example, in the case where $R_{921}$ and $R_{922}$ are bonded to each other to form a ring $Q_A$, and simultaneously $R_{925}$ and $R_{926}$ are bonded to each other to form a ring $Q_B$, the anthracene compound represented by the general formula (TEMP-103) is represented by the following general formula (TEMP-104).

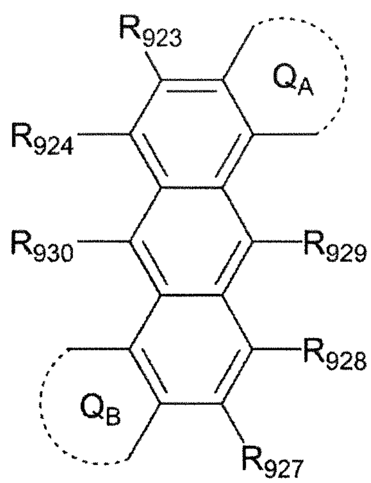

(TEMP-104)

The case where the "combination including adjacent two or more forms rings" encompasses not only the case where adjacent two included in the combination are bonded as in the aforementioned example, but also the case where adjacent three or more included in the combination are bonded. For example, this case means that $R_{921}$ and $R_{922}$ are

bonded to each other to form a ring $Q_A$, $R_{922}$ and $R_{923}$ are bonded to each other to form a ring $Q_C$, and adjacent three ($R_{921}$, $R_{922}$, and $R_{923}$) included in the combination are bonded to each other to form rings, which are condensed to the anthracene core skeleton, and in this case, the anthracene compound represented by the general formula (TEMP-103) is represented by the following general formula (TEMP-105). In the following general formula (TEMP-105), the ring $Q_A$ and the ring Qc share $R_{922}$.

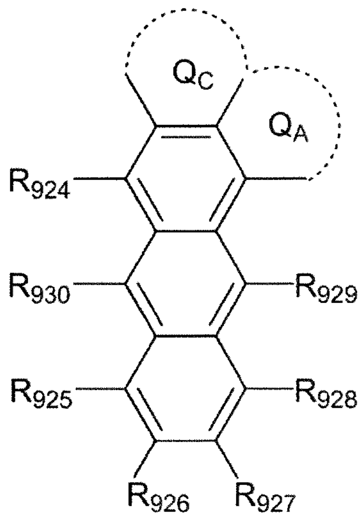

(TEMP-105)

The formed "monocyclic ring" or "condensed ring" may be a saturated ring or an unsaturated ring in terms of structure of the formed ring itself. In the case where the "one combination including adjacent two" forms a "monocyclic ring" or a "condensed ring", the "monocyclic ring" or the "condensed ring" may form a saturated ring or an unsaturated ring. For example, the ring $Q_A$ and the ring $Q_B$ formed in the general formula (TEMP-104) each are a "monocyclic ring" or a "condensed ring". The ring $Q_A$ and the ring Qc formed in the general formula (TEMP-105) each are a "condensed ring". The ring $Q_A$ and the ring Qc in the general formula (TEMP-105) form a condensed ring through condensation of the ring $Q_A$ and the ring Qc. In the case where the ring $Q_A$ in the general formula (TMEP-104) is a benzene ring, the ring $Q_A$ is a monocyclic ring. In the case where the ring $Q_A$ in the general formula (TMEP-104) is a naphthalene ring, the ring $Q_A$ is a condensed ring.

The "unsaturated ring" means an aromatic hydrocarbon ring or an aromatic heterocyclic ring. The "saturated ring" means an aliphatic hydrocarbon ring or a non-aromatic heterocyclic ring.

Specific examples of the aromatic hydrocarbon ring include the structures formed by terminating the groups exemplified as the specific examples in the set of specific examples G1 with a hydrogen atom.

Specific examples of the aromatic heterocyclic ring include the structures formed by terminating the aromatic heterocyclic groups exemplified as the specific examples in the set of specific examples G2 with a hydrogen atom.

Specific examples of the aliphatic hydrocarbon ring include the structures formed by terminating the groups exemplified as the specific examples in the set of specific examples G6 with a hydrogen atom.

The expression "to form a ring" means that the ring is formed only with the plural atoms of the core structure or with the plural atoms of the core structure and one or more arbitrary element. For example, the ring $Q_A$ formed by bonding $R_{921}$ and $R_{922}$ each other shown in the general formula (TEMP-104) means a ring formed with the carbon atom of the anthracene skeleton bonded to $R_{921}$, the carbon atom of the anthracene skeleton bonded to $R_{922}$, and one or more arbitrary element. As a specific example, in the case where the ring $Q_A$ is formed with $R_{921}$ and $R_{922}$, and in the case where a monocyclic unsaturated ring is formed with the carbon atom of the anthracene skeleton bonded to $R_{921}$, the carbon atom of the anthracene skeleton bonded to $R_{922}$, and four carbon atoms, the ring formed with $R_{921}$ and $R_{922}$ is a benzene ring.

Herein, the "arbitrary element" is preferably at least one kind of an element selected from the group consisting of a carbon element, a nitrogen element, an oxygen element, and a sulfur element, unless otherwise indicated in the description. For the arbitrary element (for example, for a carbon element or a nitrogen element), a bond that does not form a ring may be terminated with a hydrogen atom or the like, and may be substituted by an "arbitrary substituent" described later. In the case where an arbitrary element other than a carbon element is contained, the formed ring is a heterocyclic ring.

The number of the "one or more arbitrary element" constituting the monocyclic ring or the condensed ring is preferably 2 or more and 15 or less, more preferably 3 or more and 12 or less, and further preferably 3 or more and 5 or less, unless otherwise indicated in the description.

What is preferred between the "monocyclic ring" and the "condensed ring" is the "monocyclic ring" unless otherwise indicated in the description.

What is preferred between the "saturated ring" and the "unsaturated ring" is the "unsaturated ring" unless otherwise indicated in the description.

The "monocyclic ring" is preferably a benzene ring unless otherwise indicated in the description.

The "unsaturated ring" is preferably a benzene ring unless otherwise indicated in the description.

In the case where the "one or more combinations of combinations each including adjacent two or more" each are "bonded to each other to form a substituted or unsubstituted monocyclic ring", or each are "bonded to each other to form a substituted or unsubstituted condensed ring", it is preferred that the one or more combinations of combinations each including adjacent two or more each are bonded to each other to form a substituted or unsubstituted "unsaturated ring" containing the plural atoms of the core skeleton and 1 or more and 15 or less at least one kind of an element selected from the group consisting of a carbon element, a nitrogen element, an oxygen element, and a sulfur element, unless otherwise indicated in the description.

In the case where the "monocyclic ring" or the "condensed ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

In the case where the "saturated ring" or the "unsaturated ring" has a substituent, the substituent is, for example, an "arbitrary substituent" described later. In the case where the "monocyclic ring" or the "condensed ring" has a substituent, specific examples of the substituent include the substituents explained in the section "Substituents in Description" described above.

The above are the explanation of the case where "one or more combinations of combinations each including adjacent two or more" each are "bonded to each other to form a substituted or unsubstituted monocyclic ring", and the case where "one or more combinations of combinations each including adjacent two or more" each are "bonded to each other to form a substituted or unsubstituted condensed ring" (i.e., the "case forming a ring by bonding").

Substituent for "Substituted or Unsubstituted"

In one embodiment in the description herein, the substituent for the case of "substituted or unsubstituted" (which may be hereinafter referred to as an "arbitrary substituent") is, for example, a group selected from the group consisting of

an unsubstituted alkyl group having 1 to 50 carbon atoms,
an unsubstituted alkenyl group having 2 to 50 carbon atoms,
an unsubstituted alkynyl group having 2 to 50 carbon atoms,
an unsubstituted cycloalkyl group having 3 to 50 ring carbon atoms,

$-Si(R_{901})(R_{902})(R_{903})$,
$-O-(R_{904})$,
$-S-(R_{905})$,
$-N(R_{906})(R_{907})$,

a halogen atom, a cyano group, a nitro group,
an unsubstituted aryl group having 6 to 50 ring carbon atoms, and
an unsubstituted heterocyclic group having 5 to 50 ring atoms,
wherein $R_{901}$ to $R_{907}$ each independently represent
a hydrogen atom,
a substituted or unsubstituted alkyl group having 1 to 50 carbon atoms
a substituted or unsubstituted cycloalkyl group having 3 to 50 ring carbon atoms,
a substituted or unsubstituted aryl group having 6 to 50 ring carbon atoms, or
a substituted or unsubstituted heterocyclic group having 5 to 50 ring atoms.

In the case where two or more groups each represented by $R_{901}$ exist, the two or more groups each represented by $R_{901}$ are the same as or different from each other,

in the case where two or more groups each represented by $R_{902}$ exist, the two or more groups each represented by $R_{902}$ are the same as or different from each other,
in the case where two or more groups each represented by $R_{903}$ exist, the two or more groups each represented by $R_{903}$ are the same as or different from each other,

in the case where two or more groups each represented by $R_{904}$ exist, the two or more groups each represented by $R_{904}$ are the same as or different from each other,
in the case where two or more groups each represented by $R_{905}$ exist, the two or more groups each represented by $R_{905}$ are the same as or different from each other,
in the case where two or more groups each represented by $R_{906}$ exist, the two or more groups each represented by $R_{906}$ are the same as or different from each other, and
in the case where two or more groups each represented by $R_{907}$ exist, the two or more groups each represented by $R_{907}$ are the same as or different from each other.

In one embodiment, the substituent for the case of "substituted or unsubstituted" may be a group selected from the group consisting of

an alkyl group having 1 to 50 carbon atoms,
an aryl group having 6 to 50 ring carbon atoms, and
a heterocyclic group having 5 to 50 ring atoms.

In one embodiment, the substituent for the case of "substituted or unsubstituted" may be a group selected from the group consisting of

an alkyl group having 1 to 18 carbon atoms,
an aryl group having 6 to 18 ring carbon atoms, and
a heterocyclic group having 5 to 18 ring atoms.

The specific examples of the groups for the arbitrary substituent described above are the specific examples of the substituent described in the section "Substituents in Description" described above.

In the description herein, the arbitrary adjacent substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, and more preferably form a benzene ring, unless otherwise indicated.

In the description herein, the arbitrary substituent may further have a substituent unless otherwise indicated in the description. The definition of the substituent that the arbitrary substituent further has may be the same as the arbitrary substituent.

In the description herein, a numerical range shown by "AA to BB" means a range including the numerical value AA as the former of "AA to BB" as the lower limit value and the numerical value BB as the latter of "AA to BB" as the upper limit value.

The compound of the present invention will be described below.

The compound of the present invention is represented by the following formula (1). In the following description, the compounds of the present invention represented by the formula (1) and the subordinate formulae of the formula (1) described later each may be referred simply to as an "inventive compound".

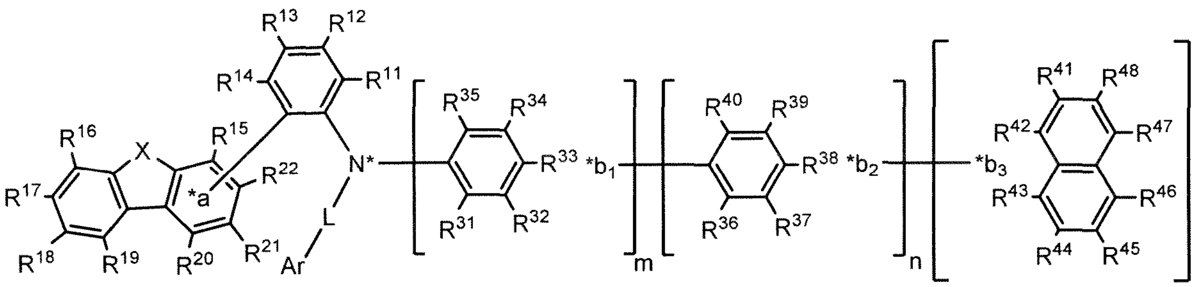

(1)

The formula (1) may be represented by any of the following formula (2) to (5).

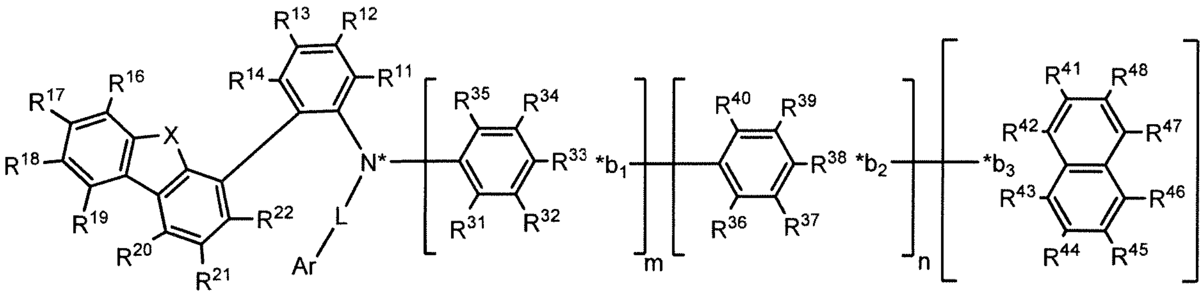

(2)

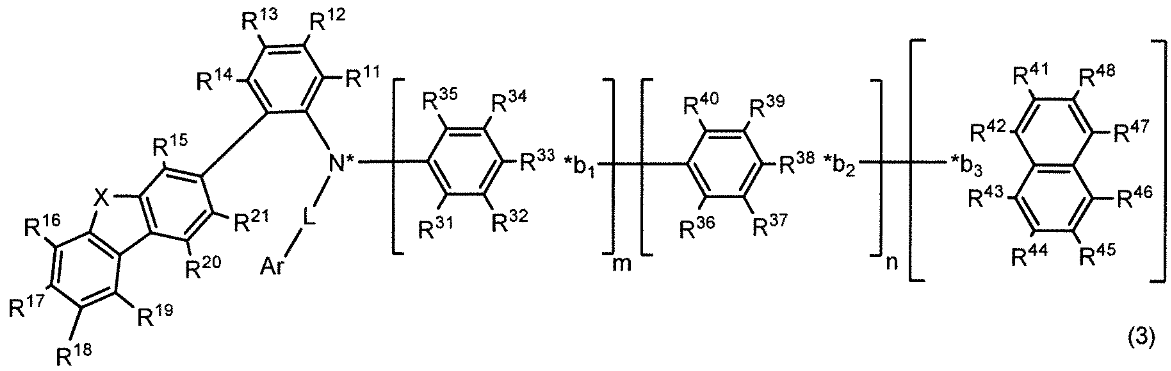

(3)

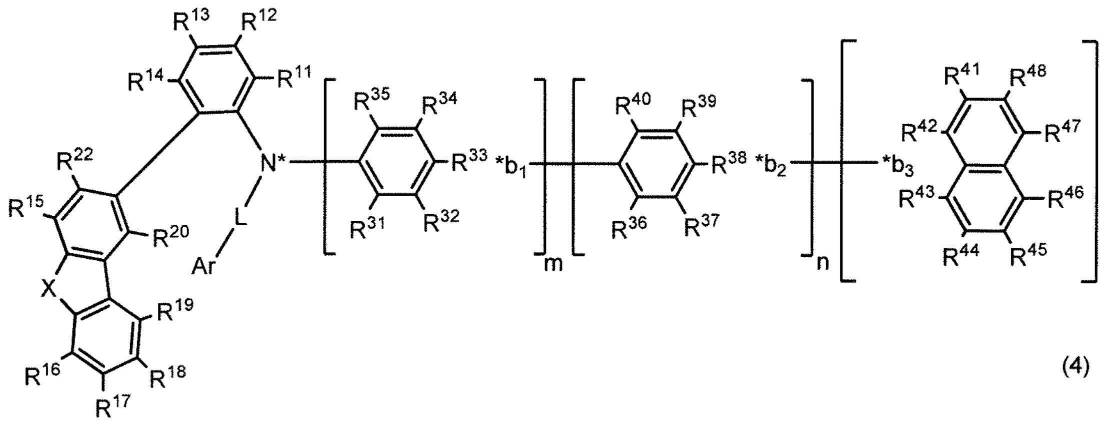

(4)

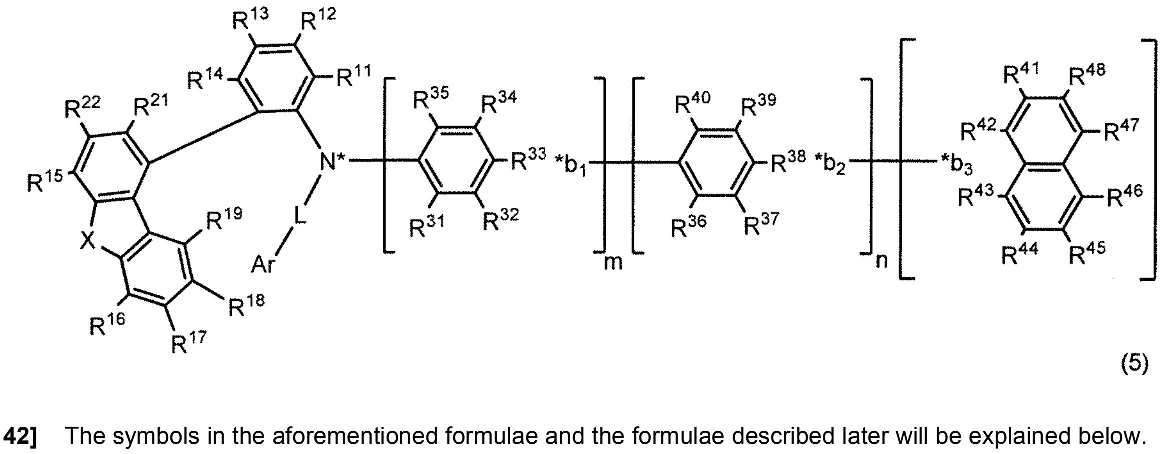

(5)

The symbols in the aforementioned formulae and the formulae described later will be explained below.

N* is a central nitrogen atom.

m is 0 or 1, and n is 0 or 1. In one embodiment of the present invention, preferably, m is 0 and n is 0. In another

embodiment of the present invention, preferably, m is 1 and n is 0. In still another embodiment of the present invention, preferably, m is 1 and n is 1.

Accordingly, the inventive compound includes compounds represented by any of the following formulae (6) to (9).

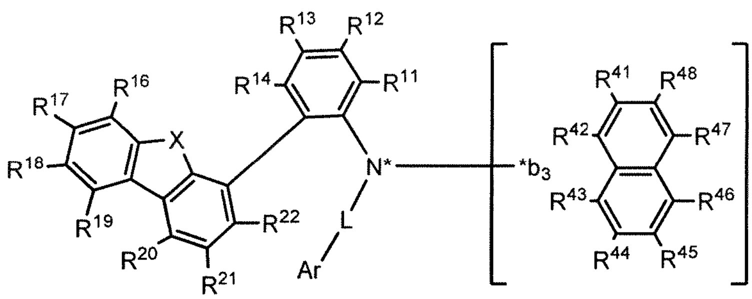

(6)

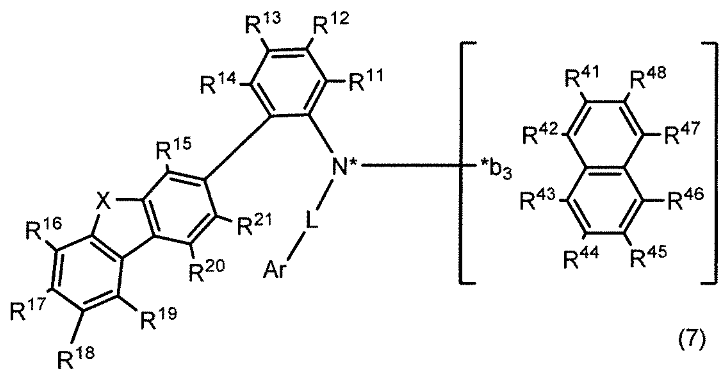

(7)

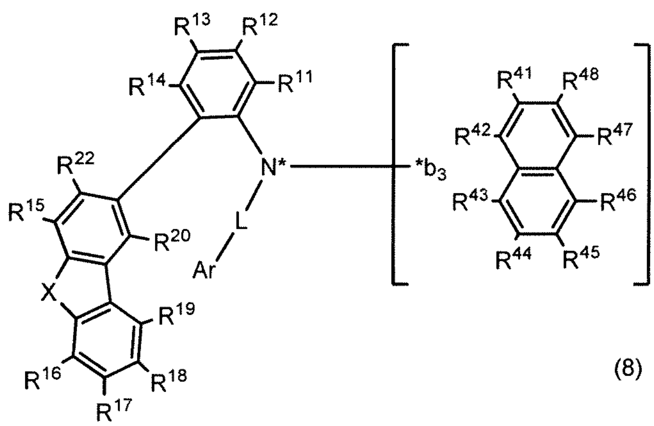

(8)

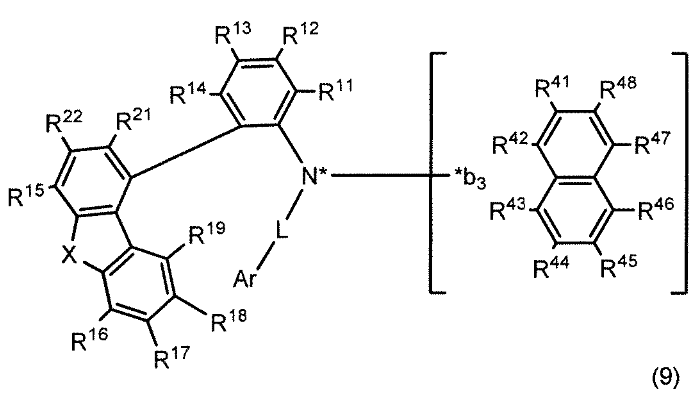

(9)

Also, the inventive compound includes compounds represented by any of the following formulae (10) to (13).

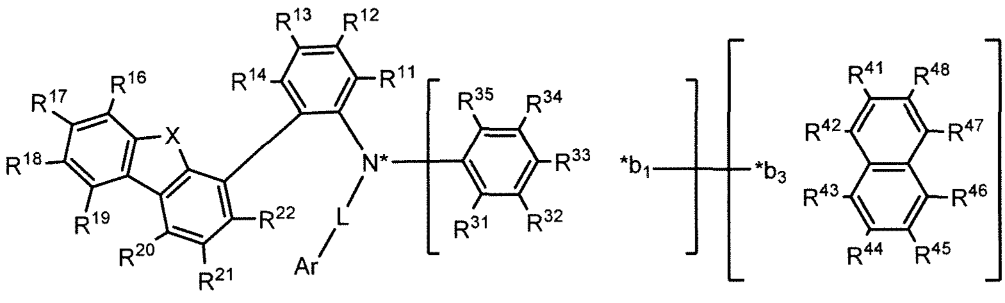

(10)

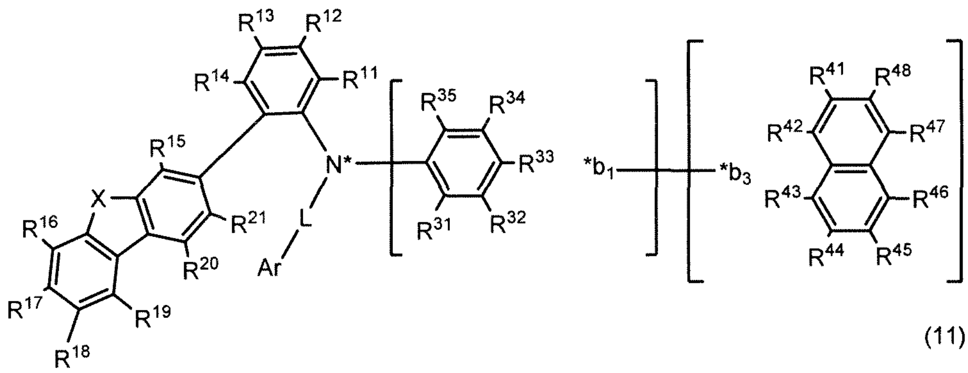

(11)

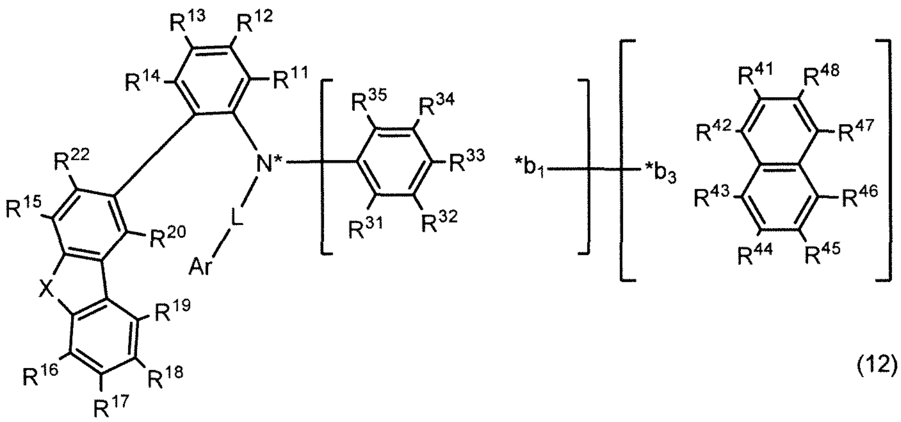

(12)

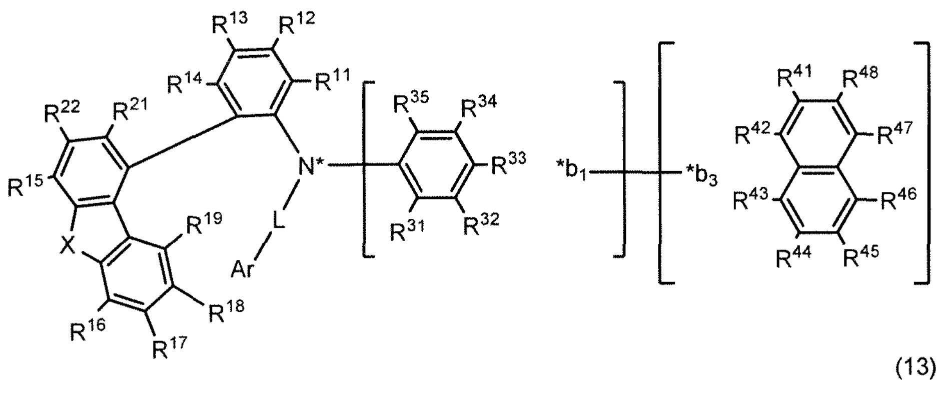

(13)

X is an oxygen atom or a sulfur atom.

In one embodiment of the present invention, preferably, X is an oxygen atom. In another embodiment of the present invention, preferably, X is a sulfur atom.

$R^{15}$ to $R^{16}$, and $R^{18}$ to $R^{22}$ each are independently selected from a hydrogen atom, a substituted or unsubstituted aryl group having 6 to 12 ring carbon atoms, and a substituted or unsubstituted alkyl group having 6 to 18 carbon atoms.

The substituted or unsubstituted aryl group having 6 to 12 ring carbon atoms that $R^{15}$ to $R^{16}$, and $R^{18}$ to $R^{22}$ represent is preferably selected from a phenyl group, a p-biphenyl group, an m-biphenyl group, an o-biphenyl group, a 1-naphthyl group and a 2-naphthyl group.

The substituted or unsubstituted alkyl group having 6 to 18 carbon atoms that $R^{15}$ to $R^{16}$, and $R^{18}$ to $R^{22}$ represent is preferably selected from a methyl group, an ethyl group, an n-propyl group, an isopropyl group, an n-butyl group, an isobutyl group, an s-butyl group, a t-butyl group, a heptafluoropropyl group, a pentafluoroethyl group, a 2,2,2-trifluoroethyl group, and a trifluoromethyl group.

$R^{31}$ to $R^{40}$ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a group represented by $-Si(R_{901})(R_{902})(R_{903})$, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group.

Preferably, they are each independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a group represented by $-Si(R_{901}(R_{902})(R_{903})$, and a cyano group;

more preferably selected from a hydrogen atom, a substituted or unsubstituted aryl group having 6 to 50 ring carbon atoms, a substituted or unsubstituted heterocyclic group having 5 to 50 ring atoms, and a substituted or unsubstituted alkyl group having 1 to 50 carbon atoms.

$R^{17}$ is each independently selected from a hydrogen atom, an unsubstituted aryl group having 6 to 12 ring carbon atoms, and a substituted or unsubstituted alkyl group having 6 to 18 carbon atoms,

The unsubstituted aryl group having 6 to 12 ring carbon atoms that $R^{17}$ represents is preferably selected from a phenyl group, a p-biphenyl group, an m-biphenyl group, an o-biphenyl group, a 1-naphthyl group and a 2-naphthyl group.

The substituted or unsubstituted alkyl group having 6 to 18 carbon atoms that $R^{17}$ represents is preferably selected from a methyl group, an ethyl group, an n-propyl group, an isopropyl group, an n-butyl group, an isobutyl group, an s-butyl group, a t-butyl group, a heptafluoropropyl group, a pentafluoroethyl group, a 2,2,2-trifluoroethyl group, and a trifluoromethyl group.

However, at least one of $R^{16}$ to $R^{19}$ is a hydrogen atom, one selected from $R^{15}$, and $R^{20}$ to $R^{22}$ is a single bond bonding to *a, one selected from $R^{31}$ to $R^{35}$ is a single bond bonding to $*b_1$, and one selected from $R^{36}$ to $R^{40}$ is a single bond bonding to $*b_2$.

Details of the substituted or unsubstituted aryl group having 6 to 50 ring carbon atoms are as described in the section of "Substituents in Description", and are more preferably a phenyl group, a p-biphenyl group, an m-biphenyl group, an o-biphenyl group, a p-terphenyl-4-yl group, a p-terphenyl-3-yl group, a p-terphenyl-2-yl group an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, a fluorenyl group, a 9,9'-spirobifluorenyl group, a 9,9-dimethylfluorenyl group or a 9,9-diphenylfluorenyl group, even more preferably a phenyl group, a p-biphenyl group, an m-biphenyl group, an o-biphenyl group, a 1-naphthyl group, a 2-naphthyl group, a fluorenyl group, a 9,9'-spirobifluorenyl group, a 9,9-dimethylfluorenyl group, or a 9,9-diphenylfluorenyl group.

Details of the substituted or unsubstituted heterocyclic group having 5 to 50 ring atoms are as described in the section of "Substituents in Description", and are more preferably selected from a carbazolyl group, a benzocarbazolyl group, a dibenzofuranyl group, a naphthobenzofuranyl group, a dibenzothiophenyl group, a naphthobenzothiophenyl group, a (9-phenyl)carbazolyl group, a (9-biphenylyl)carbazolyl group, a (9-phenyl)phenylcarbazolyl group, a diphenylcarbazol-9-yl group, a phenylcarbazole-9-yl group, a phenyldibenzofuranyl group, and a phenyldibenzothiophenyl group.

The substituted or unsubstituted alkyl group having 1 to 50 carbon atoms has been described in detail in the section "Substituents in Description", and is preferably a methyl group, an ethyl group, a n-propyl group, an isopropyl group, a n-butyl group, an isobutyl group, an s-butyl group, or a t-butyl group, and more preferably a methyl group, an isopropyl group, or a t-butyl group.

The substituted or unsubstituted cycloalkyl group having 3 to 50 ring carbon atoms has been described in detail in the section "Substituents in Description", and is preferably a cyclopropyl group, a cyclobutyl group, a cyclopentyl

group, or a cyclohexyl group, and more preferably a cyclopropyl group, a cyclopentyl group, or a cyclohexyl group.

$R_{901}$, $R_{902}$ and $R_{903}$ in the group represented by -Si($R_{901}$)($R_{902}$)($R_{903}$) each are independently selected from a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, and a substituted or unsubstituted cycloalkyl group having 3 to 50 ring carbon atoms, and details thereof are as described in the section of "Substituents in Description".

The group represented by -Si($R_{901}$)($R_{902}$)($R_{903}$) is preferably a trimethylsilyl group, a triethylsilyl group, a t-butyldimethylsilyl group, a propyldimethylsilyl group, an isopropyldimethylsilyl group, a triphenylsilyl group, a phenyldimethylsilyl group, a t-butyldiphenylsilyl group or a tritolylsilyl group, more preferably a trimethylsilyl group or a triphenylsilyl group.

Details of the halogen atom are as described in the section of "Substituents in Description", and are preferably a fluorine atom.

Details of the substituted or unsubstituted haloalkyl group having 1 to 50 carbon atoms are as described in the section of "Substituents in Description", and are preferably a substituted or unsubstituted fluoroalkyl group having 1 to 50 carbon atoms, more preferably a trifluoromethyl group, a 2,2,2-trifluoroethyl group, a pentafluoroethyl group, or a heptafluoropropyl group, even more preferably a trifluoromethyl group.

Details of the substituted or unsubstituted alkoxy group having 1 to 50 carbon atoms are as described in the section of "Substituents in Description", and are preferably a methoxy group, an ethoxy group, a propoxy group, or a t-butoxy group, more preferably a methoxy group or an ethoxy group, even more preferably a methoxy group.

Details of the substituted or unsubstituted aryloxy group having 6 to 50 ring carbon atoms are as described in the section of "Substituents in Description", and are preferably a phenoxy group, a biphenyloxy group or a terphenyloxy group, more preferably a phenoxy group or a biphenyloxy group, even more preferably a phenoxy group.

The substituted or unsubstituted haloalkoxy group having 1 to 50, preferably 1 to 30, more preferably 1 to 18 carbon atoms is a group represented by -O(G12), and G12 is a substituted or unsubstituted haloalkyl group described in the section of "Substituents in Description".

The substituted or unsubstituted haloalkoxy group having 1 to 50 carbon atoms is preferably a substituted or unsubstituted fluoroalkoxy group having 1 to 50 carbon atoms, more preferably a trifluoromethoxy group, a 2,2,2-trifluoroethoxy group, a pentafluoroethoxy group, or a heptafluoropropoxy group, even more preferably a trifluoromethoxy group, a 2,2,2-trifluoroethoxy group, or a pentafluoroethoxy group, especially more preferably a trifluoromethoxy group.

Details of the substituted or unsubstituted aralkyl group having 7 to 50 carbon atoms are as described in the section of "Substituents in Description", and are preferably a benzyl group, a phenyl-t-butyl group, an $\alpha$-naphthylmethyl group, a $\beta$-naphthylmethyl group, a 1-$\beta$-naphthylisopropyl group, or a 2-6-naphthylisopropyl group, more preferably a benzyl group, a phenyl-t-butyl group, an $\alpha$-naphthylmethyl group or a $\beta$-naphthylmethyl group.

$R^{11}$ to $R^{14}$ and $R^{11}$ to $R^{48}$ each are independently selected from a hydrogen atom, and a substituted or unsubstituted alkyl group having 1 to 50 carbon atoms.

However, one selected from $R^{41}$ to $R^{48}$ is a single bond bonding to $*b_3$.

Details of the substituted or unsubstituted alkyl group having 1 to 50 carbon atoms are the same as those of the corresponding group that $R^{15}$ to $R^{22}$ and $R^{31}$ to $R^{40}$ mentioned above represent.

Ar represents a substituted or unsubstituted aryl group having 6 to 50 ring carbon atoms, or a substituted or unsubstituted heterocyclic group having 5 to 50 ring atoms.

Details of the substituted or unsubstituted aryl group having 6 to 50 ring carbon atoms are the same as those of the corresponding group that $R^{15}$, $R^{16}$, $R^{18}$, $R^{19}$, $R^{20}$ to $R^{22}$, and $R^{31}$ to $R^{40}$ mentioned above represent.

Details of the substituted or unsubstituted heterocyclic group having 5 to 50 ring atoms are the same as those of the corresponding group that $R^{15}$ to $R^{22}$ and $R^{31}$ to $R^{40}$ mentioned above represent.

Regarding the substituted or unsubstituted aryl group having 6 to 50 ring carbon atoms that Ar represents, the aryl group is preferably selected from a phenyl group, a p-biphenyl group, an m-biphenyl group, an o-biphenyl group, a p-terphenyl-4-yl group, a p-terphenyl-3-yl group, a p-terphenyl-2-yl group an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group and a 9,9-diphenylfluorenyl group, more preferably from a phenyl group, a p-biphenyl group, a 1-naphthyl group, a 2-naphthyl group, a phenanthryl group, a 9,9'-spirobifluorenyl group, a 9,9-dimethylfluorenyl group and a 9,9-diphenylfluorenyl group, even more preferably from a phenyl group, a p-biphenyl group and a 1-naphthyl group.

In one embodiment of the present invention, Ar is more preferably a substituted or unsubstituted phenyl group.

Regarding the substituted or unsubstituted heterocyclic group having 5 to 50 ring atoms that Ar represents, the heterocyclic group is preferably selected from a carbazolyl group, a benzocarbazolyl group, a dibenzofuranyl group, a naphthobenzofuranyl group, a dibenzothiophenyl group, a naphthobenzothiophenyl group, a (9-phenyl)carbazolyl group, a (9-biphenyl)carbazolyl group, a (9-phenyl)phenylcarbazolyl group, a diphenylcarbazol-9-yl group, a phenylcarbazole-9-yl group, a phenyldibenzofuranyl group and a phenyldibenzothiophenyl group, more preferably from a dibenzofuranyl group, a dibenzothiophenyl group, a carbazolyl group and a (9-phenyl)carbazolyl group, even more preferably from a dibenzofuranyl group, a dibenzothiophenyl group and a carbazolyl group.

In one embodiment of the present invention, Ar is preferably a substituted or unsubstituted dibenzofuranyl group.

When Ar has a substituent, the substituent is each independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group.

L is selected from a single bond, a substituted or unsubstituted arylene group having 6 to 50 ring carbon atoms, and a substituted or unsubstituted 2-valent heterocyclic group having 5 to 50 ring atoms.

In one embodiment of the present invention, L is preferably a single bond.

Regarding the substituted or unsubstituted arylene group having 6 to 50 ring carbon atoms that L represents, the arylene group is preferably selected from a phenylene group, a biphenyldiyl group, a naphthylene group, a terphenylene group, a phenalenylene group, and a fluorenylene group, more preferably from a phenylene group, a biphenyldiyl group, a naphthylene group, a terphenylene group and a fluorenylene group, even more preferably from a phenylene group, a biphenyldiyl group and a naphthylene group.

In another embodiment of the present invention, L is more preferably a substituted or unsubstituted phenylene group.

In still another embodiment of the present invention, L is more preferably a substituted or unsubstituted biphenyldiyl group.

Regarding the substituted or unsubstituted divalent heterocyclic group having 5 to 50 ring atoms that L represents, the divalent heterocyclic group is preferably selected from a divalent residue of an aromatic hetero ring of furan, thiophene, indole, carbazole, benzofuran, dibenzofuran, benzothiophene and dibenzothiophene, more preferably from a divalent residue of an aromatic hetero ring of carbazole, dibenzofuran and dibenzothiophene.

In another embodiment of the present invention, L is more preferably a substituted or unsubstituted divalent residue of an aromatic hetero ring of dibenzofuran.

In still another embodiment of the present invention, L is more preferably a substituted or unsubstituted divalent residue of an aromatic hetero ring of dibenzothiophene.

In the case where X is a sulfur atom in the formula (1), one embodiment may be any of the following (p) to (u).

(p) The substituted or unsubstituted aryl group that Ar represents is a substituted or unsubstituted aryl group having 6 to 16 ring carbon atoms, and L is a single bond.

(q) One selected from $R^{20}$ to $R^{22}$ is a single bond bonding to *a.

(r) In the substituted or unsubstituted arylene group having 6 to 50 ring carbon atoms that L represents, the arylene group is a phenylene group, and in the substituted or unsubstituted heterocyclic group having 5 to 50 ring atoms that Ar represent, the heterocyclic group is a dibenzothiophenyl group, and the phenylene group is the general formula (TEMP-43) or (TEMP-44).

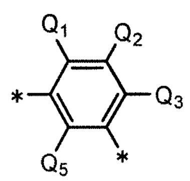

(TEMP-43)

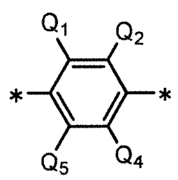

(TENP-44)

(s) In the substituted or unsubstituted aryl group having 6 to 50 ring carbon atoms that Ar represents, the aryl group is a naphthyl group.

(t) In the substituted or unsubstituted arylene group having 6 to 50 ring carbon atoms that L represents, the arylene group is a biphenylene group, and the biphenylene group is selected from any of the general formulae (21-1) to (21-10).

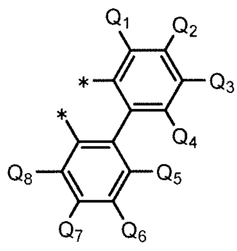

(21-1)

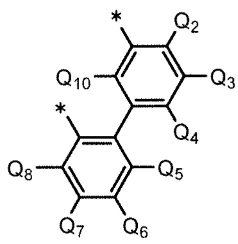

(21-2)

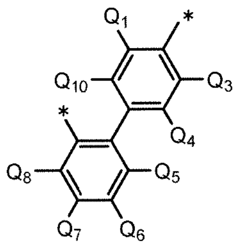

(21-3)

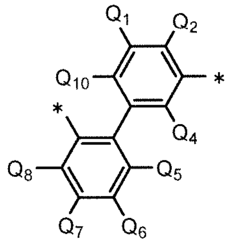

(21-4)

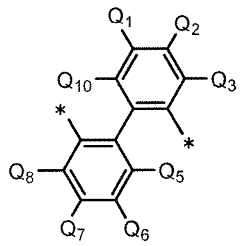

(21-5)

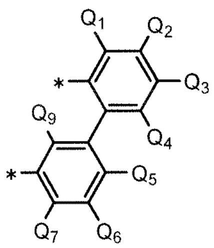

(21-6)

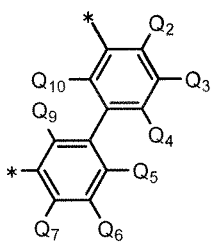

(21-7)

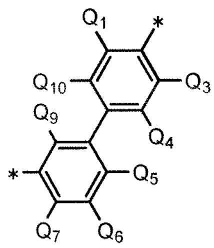

(21-8)

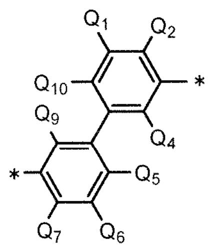

(21-9)

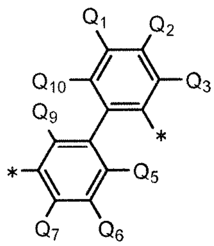

(21-10)

(u) In the substituted or unsubstituted aryl group having 6 to 50 ring carbon atoms that Ar represents, the aryl group is a terphenyl group, and the terphenyl group is any of the general formulae (22-1) to (22-20), and excludes the general formula (22).

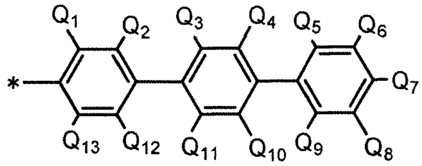

**(22)**

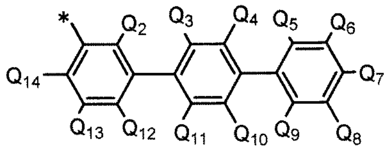

(22-1)

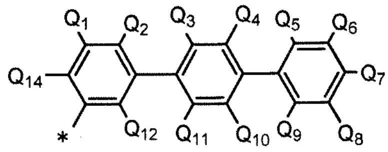

(22-2)

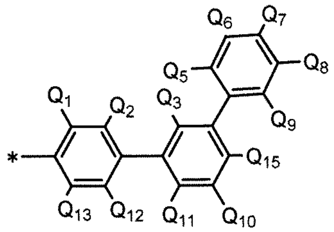

(22-3)

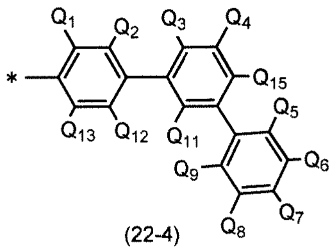

(22-4)

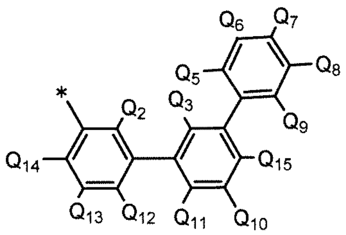

(22-5)

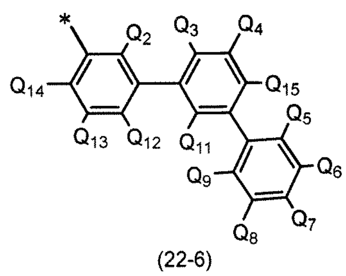

(22-6)

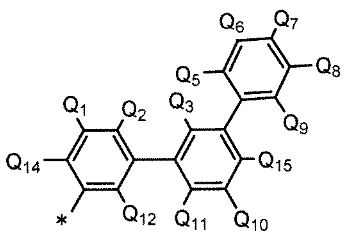

(22-7)

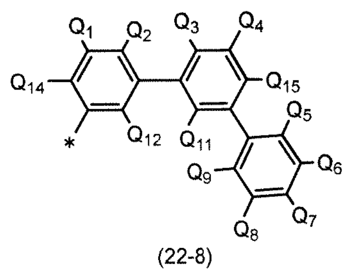

(22-8)

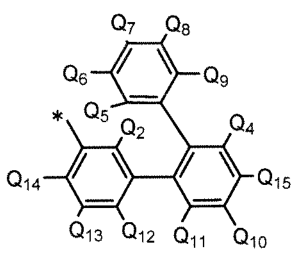

(22-9)

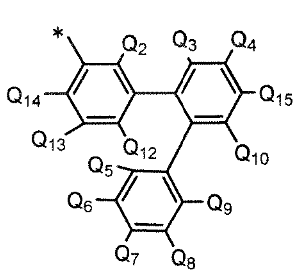

(22-10)

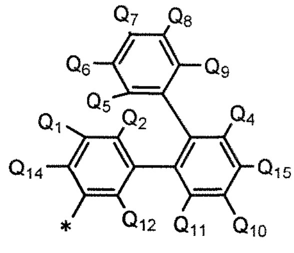

(22-11)

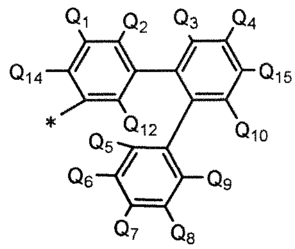

(22-12)

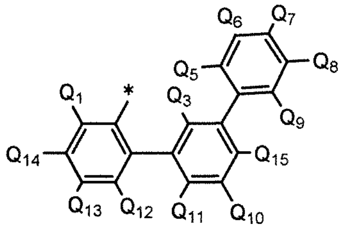

(22-13)

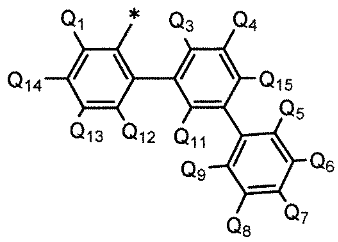

(22-14)

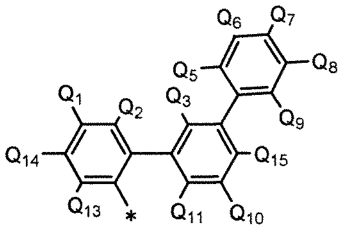

(22-15)

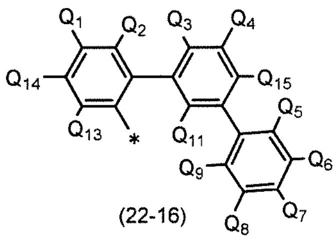

(22-16)

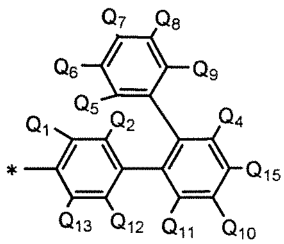

(22-17)

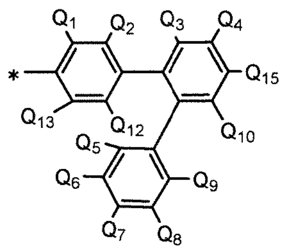

(22-18)

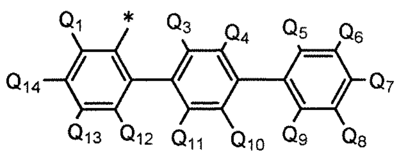

(22-19)

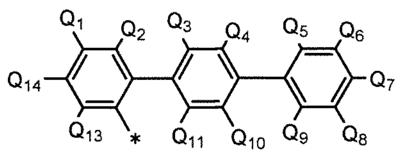

(22-20)

In the general formulae (TEMP-42) to (TEMP-43), (21-1) to (21-10), (22) and (22-1) to (22-20), $Q_1$ to $Q_{15}$ each independently represent a hydrogen atom or a substituent, and * represents a bonding position.

In the case where X is a sulfur atom in one embodiment, the general formulae (23-1), (23-2) and (23-3) are excluded.

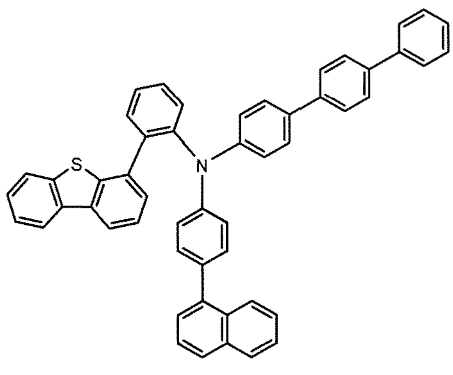
(23-1)

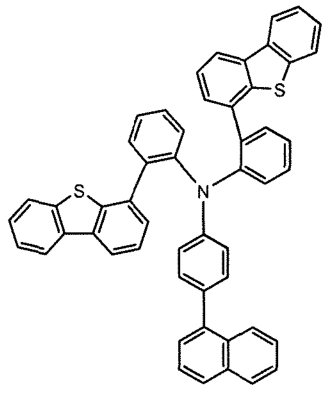
(23-2)

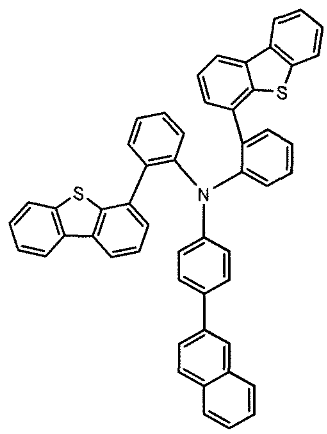
(23-3)

In one embodiment of the present invention,

(1) all $R^{15}$, $R^{20}$, $R^{21}$ and $R^{22}$ that are not a single bond bonding to *a, and all $R^{16}$ to $R^{19}$ may be hydrogen atoms,
(2) all $R^{11}$ to $R^{14}$ may be hydrogen atoms,
(3) all $R^{31}$ to $R^{35}$ that are not a single bond bonding to $*b_1$ may be hydrogen atoms,
(4) all $R^{36}$ to $R^{40}$ that are not a single bond bonding to $*b_2$ may be hydrogen atoms,
(5) all $R^{41}$ to $R^{18}$ that are not a single bond bonding to $*b_3$ may be hydrogen atoms.

The inventive compound may satisfy all the above-mentioned requirements (1) to (5) at the same time, or may satisfy a part of the requirements (1) to (5).

Adjacent two selected from $R^{11}$ to $R^{14}$, $R^{15}$ to $R^{22}$, $R^{31}$ to $R^{40}$ and $R^{41}$ to $R^{48}$ do not bond to each other and do not form a ring.

As described above, the "hydrogen atom" referred in the description herein encompasses a protium atom, a deuterium atom, and tritium atom. Accordingly, the inventive compound may contain a naturally-derived deuterium atom.

A deuterium atom may be intentionally introduced into the inventive compound by using a deuterated compound as a part or the whole of the raw material. Accordingly, in one embodiment of the present invention, the inventive compound contains at least one deuterium atom. That is, the inventive compound may be a compound represented by the formula (1) in which at least one hydrogen atom contained therein is a deuterium atom.

At least one hydrogen atom selected from the following hydrogen atoms may be a deuterium atom:

a hydrogen atom of the substituted or unsubstituted aryl group or heterocyclic group that Ar represents;
a hydrogen atom of the substituted or unsubstituted arylene group or divalent heterocyclic group that L represents;
a hydrogen atom that any of $R^{11}$ to $R^{14}$ and $R^{41}$ to $R^{48}$ represents; a hydrogen atom of the substituted or unsubstituted alkyl group that any of $R^{11}$ to $R^{14}$ and $R^{41}$ to $R^{48}$ represents;
a hydrogen atom that any of $R^{15}$ to $R^{16}$ and $R^{18}$ to $R^{22}$ represents; a hydrogen atom of the substituted or unsubstituted aryl group or alkyl group that any of $R^{15}$ to $R^{16}$ and $R^{18}$ to $R^{22}$ represents;
a hydrogen atom that any of $R^{31}$ to $R^{40}$ represents; a hydrogen atom of the substituted or unsubstituted aryl group, heterocyclic group, alkyl group, cycloalkyl group, group represented by $-Si(R_{901})(R_{902})(R_{903})$, haloalkyl group, alkoxy group, aryloxy group, haloalkoxy group or aralkyl group that any of $R^{31}$ to $R^{40}$ represents;
a hydrogen atom that $R^{17}$ represents; and a hydrogen atom of the unsubstituted aryl group or the substituted or unsubstituted alkyl group that $R^{17}$ represents.

The deuteration rate of the inventive compound depends on the deuteration rate of the raw material compound used. Even when a raw material having a predetermined deuteration rate is used, a naturally-derived protium isotope can be contained in a certain ratio. Accordingly, an embodiment of the deuteration rate of the inventive compound shown below includes the proportion for which a minor amount of a naturally-derived isotope is taken into consideration, relative to the proportion determined by counting the number of the deuterium atoms merely represented by a chemical formula.

The deuteration rate of the inventive compound is preferably 1% or more, more preferably 3% or more, even more preferably 5% or more, further more preferably 10% or more.

The inventive compound may be a mixture of a deuterated compound (i.e., a compound having deuterium atoms intentionally introduced thereto) and a non-deuterated compound, or a mixture of two or more compounds having

different deuteration rates from each other. The deuteration rate of the mixture (i.e., the proportion of the number of deuterium atoms with respect to the number of all hydrogen atoms in the inventive compound contained in the mixture) may be 1% or more, is preferably 3% or more, more preferably 5% or more, and still more preferably 10% or more, and is less than 100%.

In the inventive compound, at least one hydrogen atom selected from the hydrogen atoms of the substituted or unsubstituted aryl group or heterocyclic group that Ar represents may be a deuterium atom. The deuteration rate (i.e., the proportion of the number of deuterium atoms with respect to the number of all hydrogen atoms of Ar) may be 1% or more, is preferably 3% or more, more preferably 5% or more, and still more preferably 10% or more, and is less than 100%.

In the inventive compound, at least one hydrogen atom selected from a hydrogen atom of the substituted or unsubstituted arylene group or divalent heterocyclic group represented by L may be a deuterium atom. The deuteration rate (i.e., the proportion of the number of deuterium atoms with respect to the number of all hydrogen atoms of L) may be 1% or more, is preferably 3% or more, more preferably 5% or more, and still more preferably 10% or more, and is less than 100%.

In the inventive compound, at least one hydrogen atom selected from a hydrogen atom that any of $R^{11}$ to $R^{14}$ and $R^{41}$ to $R^{48}$ represents, and a hydrogen atom of the substituted or unsubstituted alkyl group that any of $R^{11}$ to $R^{14}$ and $R^{41}$ to $R^{48}$ represents may be a deuterium atom. The deuteration rate (i.e., the proportion of the number of deuterium atoms with respect to the number of all hydrogen atoms of $R^{11}$ to $R^{14}$ and $R^{41}$ to $R^{48}$) may be 1% or more, is preferably 3% or more, more preferably 5% or more, and still more preferably 10% or more, and is less than 100%.

In the inventive compound, at least one hydrogen atom selected from a hydrogen atom that any of $R^{15}$ to $R^{16}$, and $R^{18}$ to $R^{22}$ represents, and a hydrogen atom of the substituted or unsubstituted aryl group or alkyl group that any of $R^{15}$, $R^{16}$, $R^{18}$, $R^{19}$ and $R^{20}$ to $R^{22}$ represents may be a deuterium atom. The deuteration rate (i.e., the proportion of the number of deuterium atoms with respect to the number of all hydrogen atoms of $R^{15}$ to $R^{16}$, and $R^{18}$ to $R^{22}$) may be 1% or more, is preferably 3% or more, more preferably 5% or more, and still more preferably 10% or more, and is less than 100%.

In the inventive compound, at least one hydrogen atom selected from a hydrogen atom that any of $R^{31}$ to $R^{40}$ represents, a hydrogen atom of the substituted or unsubstituted aryl group, heterocyclic group, alkyl group, cycloalkyl group, group represented by -Si($R_{901}$)($R_{902}$)($R_{903}$), haloalkyl group, alkoxy group, aryloxy group, haloalkoxy group or aralkyl group that any of $R^{31}$ to $R^{40}$ represents may be a deuterium atom. The deuteration rate (i.e., the proportion of the number of deuterium atoms with respect to the number of all hydrogen atoms of $R^{31}$ to $R^{40}$) may be 1% or more, is preferably 3% or more, more preferably 5% or more, and still more preferably 10% or more, and is less than 100%.

In the inventive compound, at least one hydrogen atom selected from a hydrogen atom that $R^{17}$ represents, and a hydrogen atom of the unsubstituted aryl group or the substituted or unsubstituted alkyl group that $R^{17}$ represents may be a deuterium atom. The deuteration rate (i.e., the proportion of the number of deuterium atoms with respect to the number of all hydrogen atoms of $R^{17}$) may be 1% or more, is preferably 3% or more, more preferably 5% or more, and still more preferably 10% or more, and is less than 100%.

The details of the substituent (arbitrary substituent) in the expression "substituted or unsubstituted" included in the definitions of the aforementioned formulae are the same as in the "substituent in the expression 'substituted or unsubstituted'".

The inventive compound can be readily produced by a person skilled in the art with reference to the following synthesis examples and the known synthesis methods.

Specific examples of the inventive compound will be described below, but the inventive compound is not limited to the following example compounds.

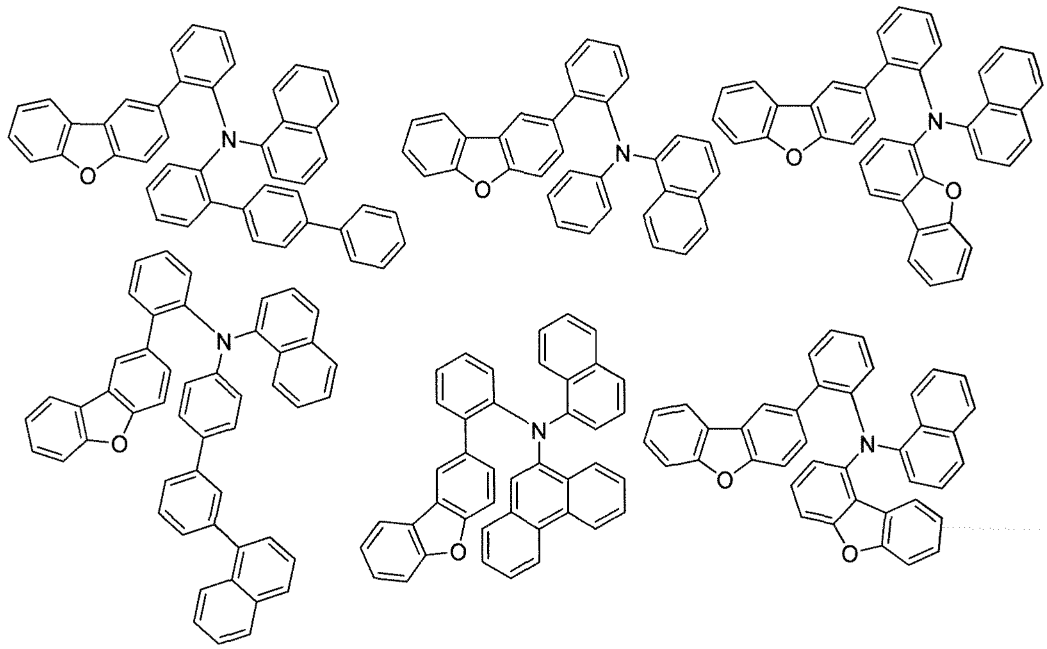

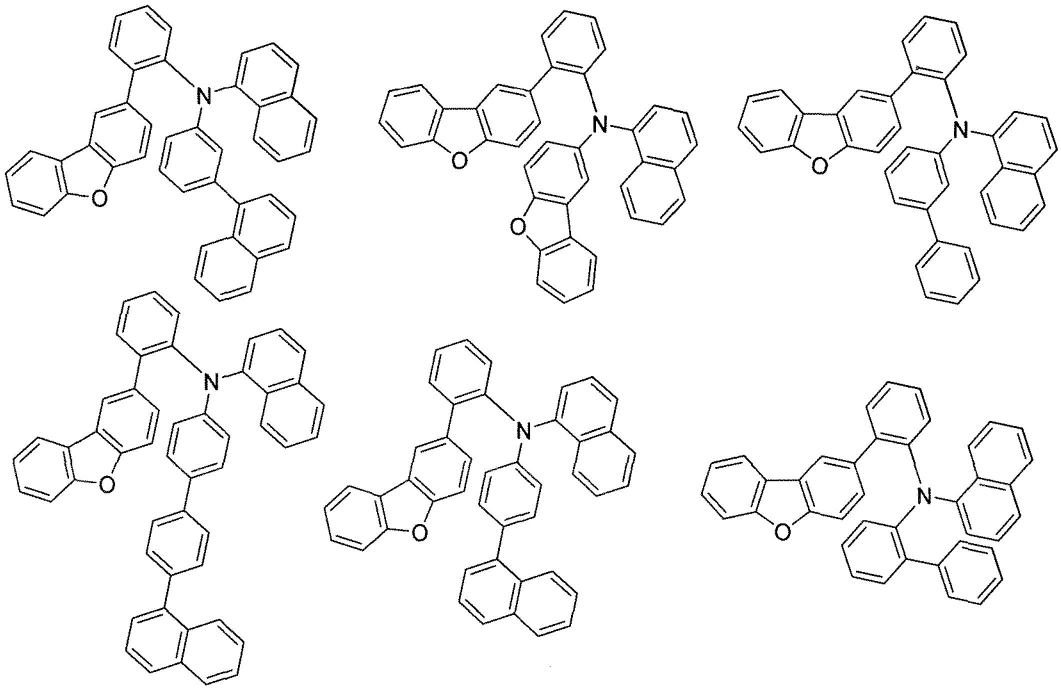

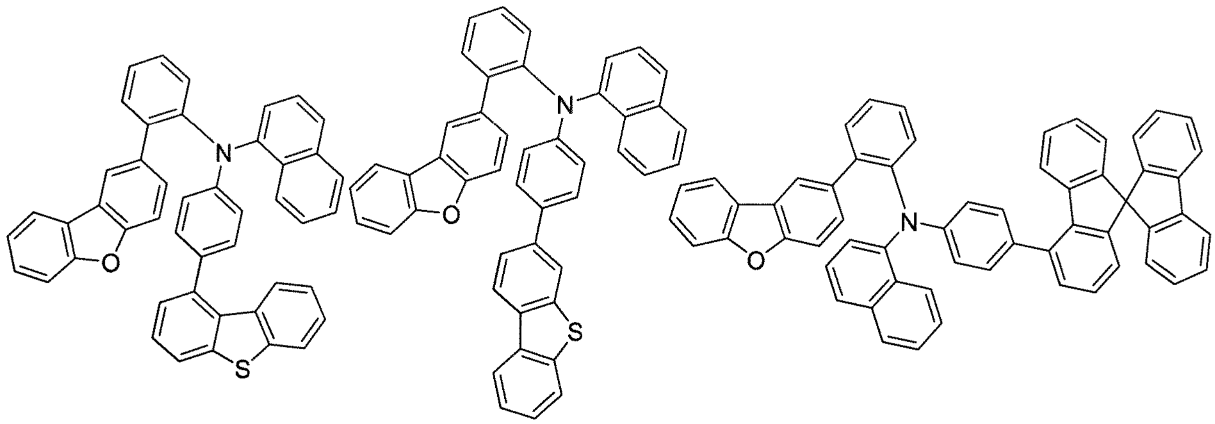

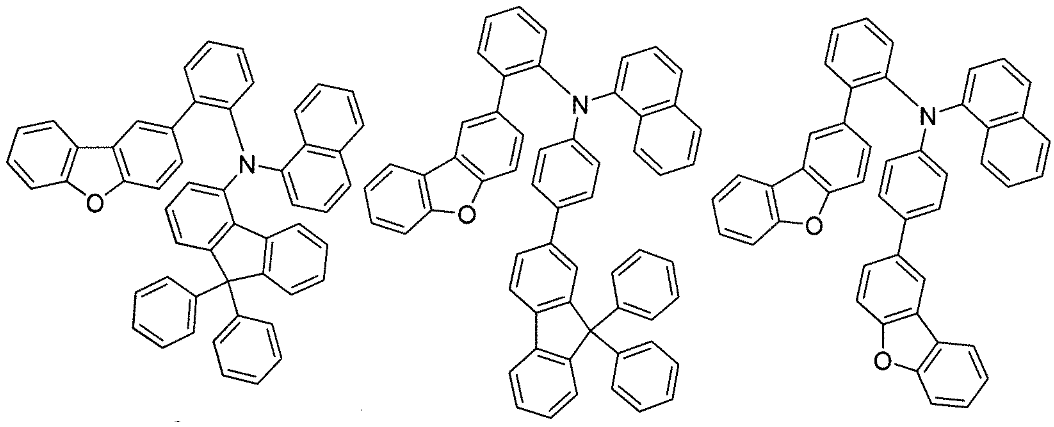

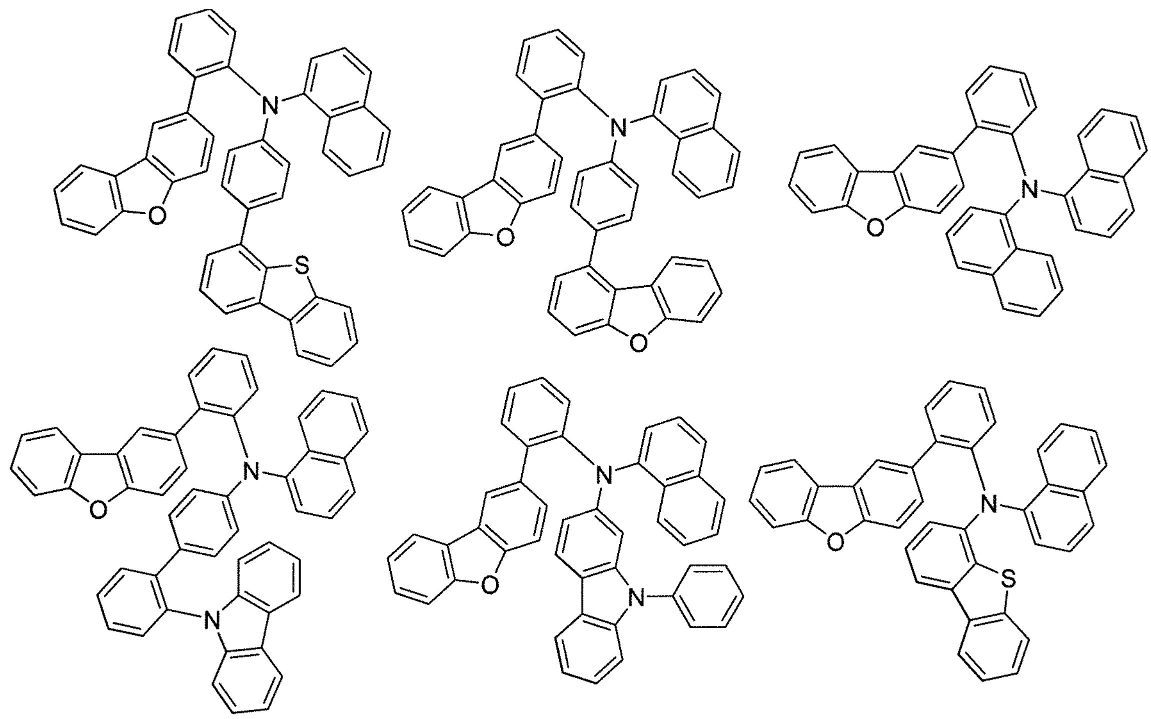

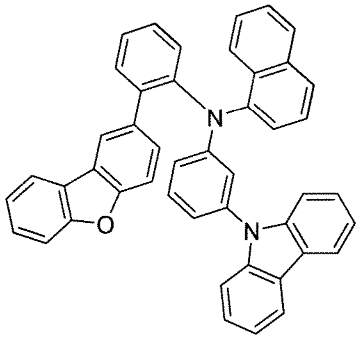
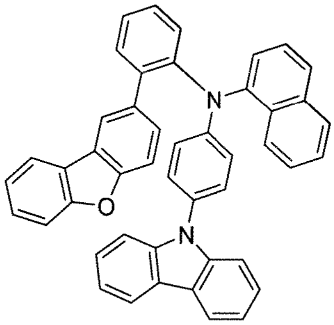
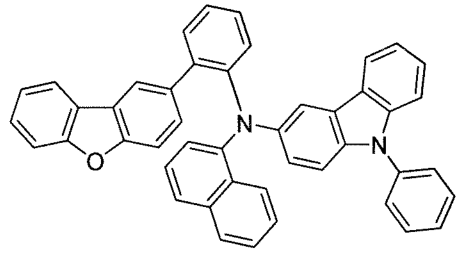

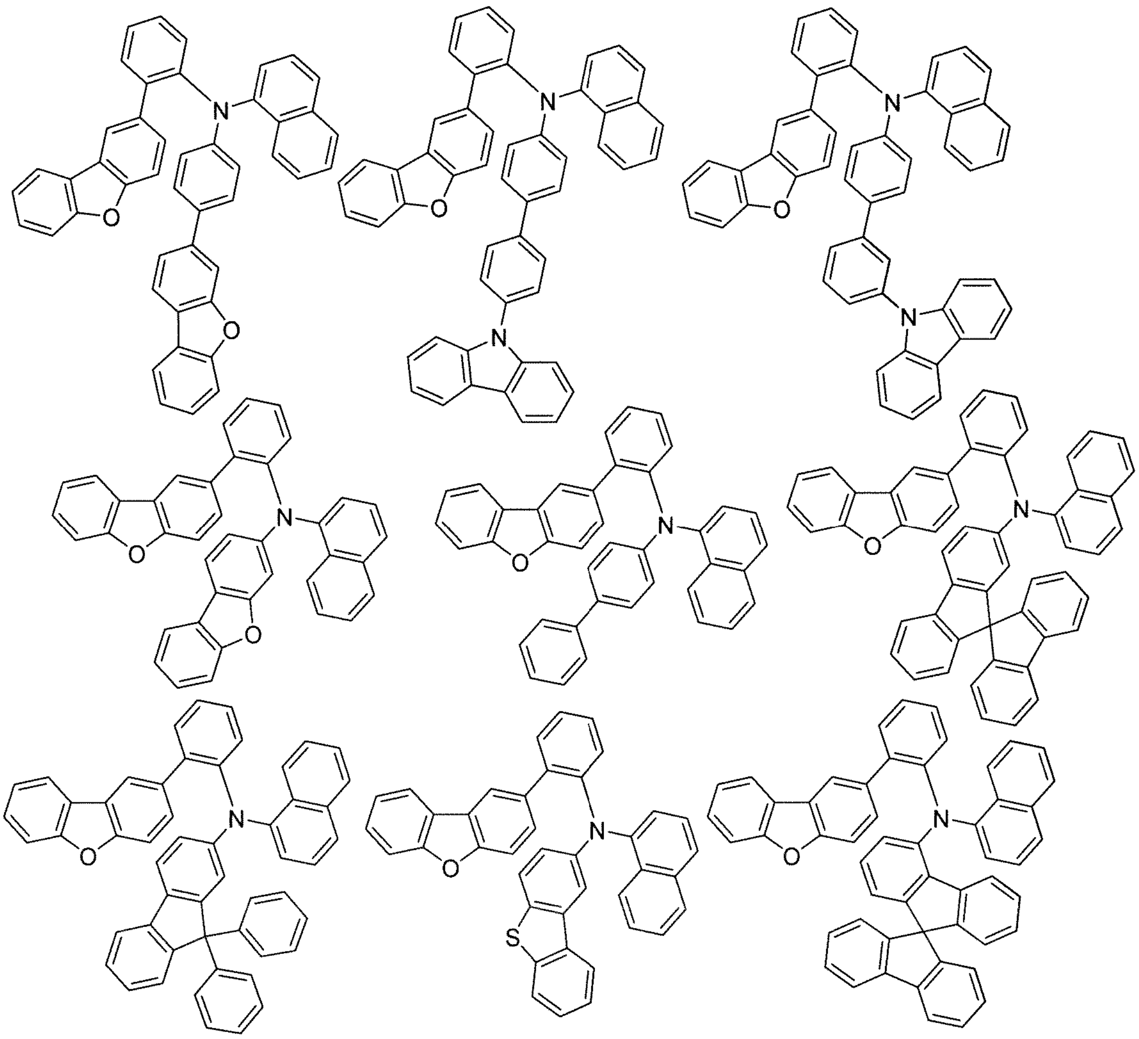

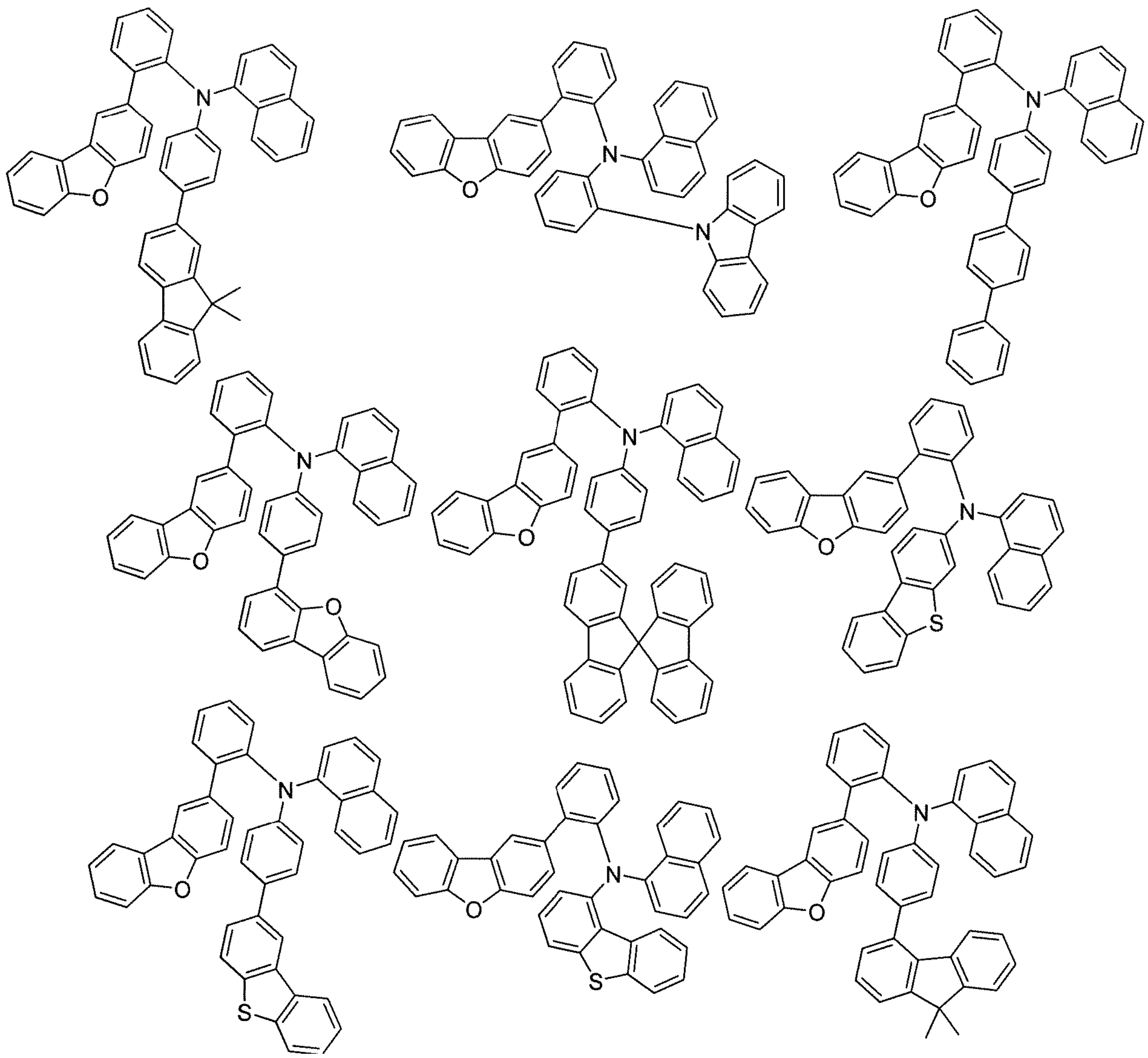

—

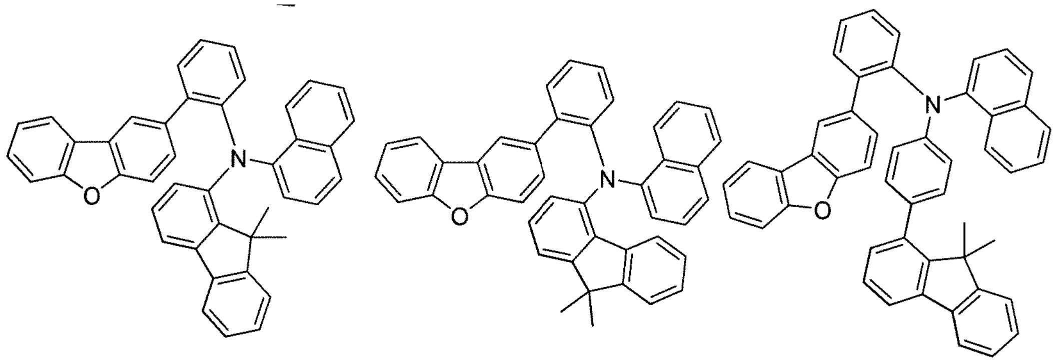

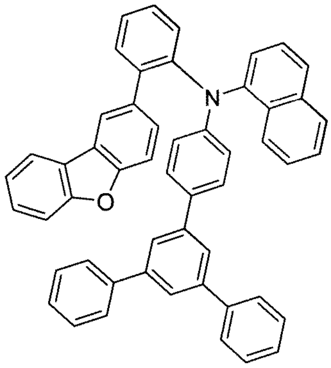
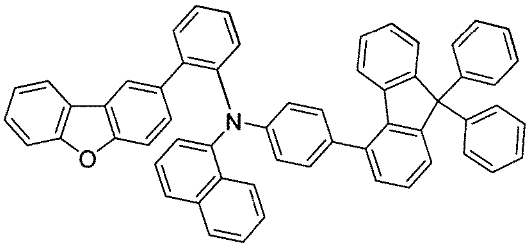
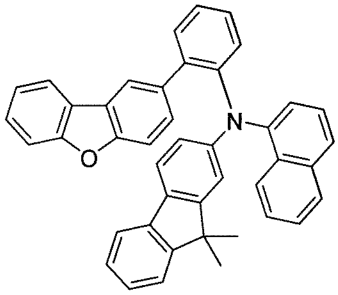
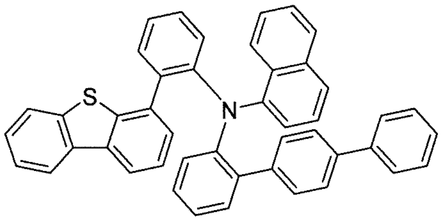
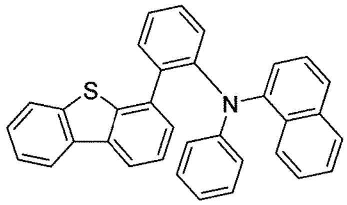
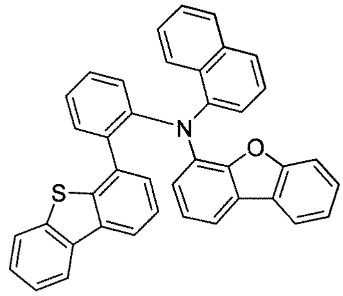
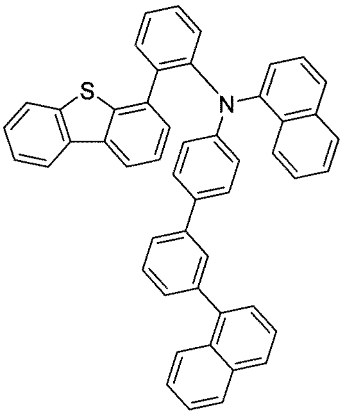
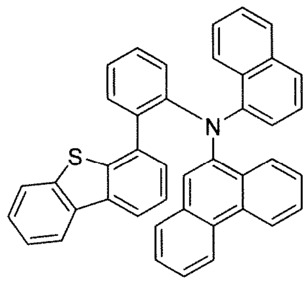
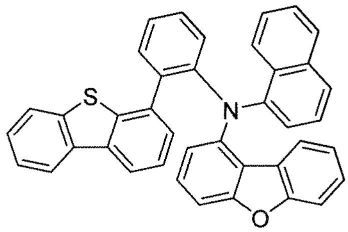
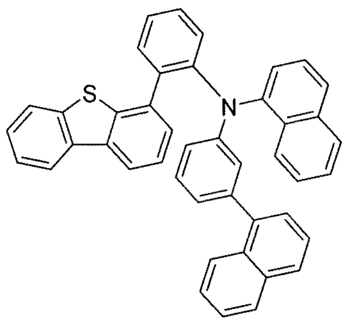
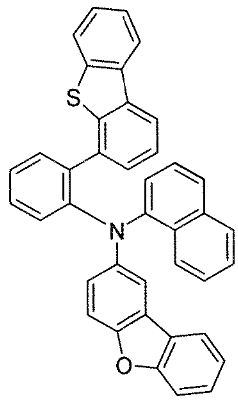
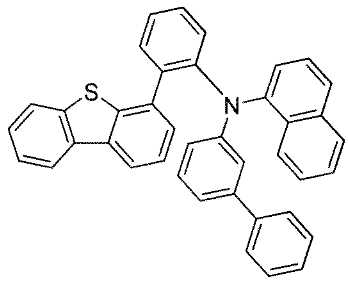

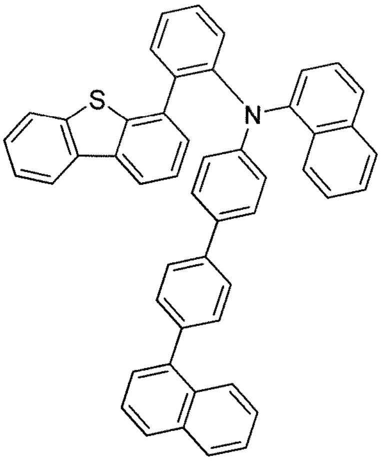
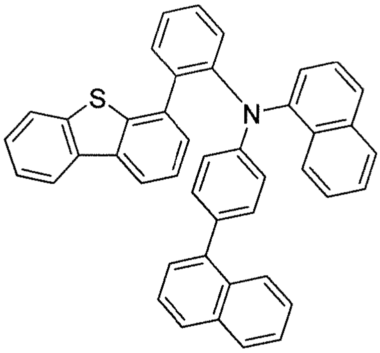
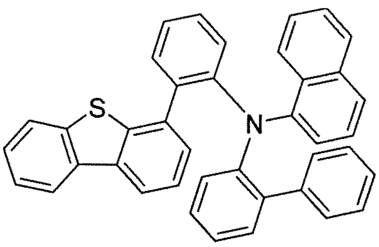
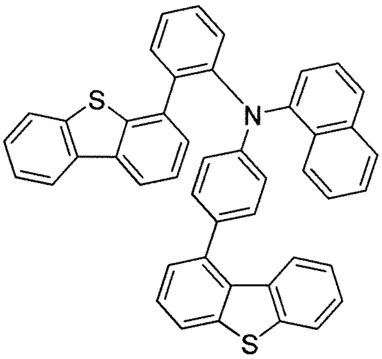
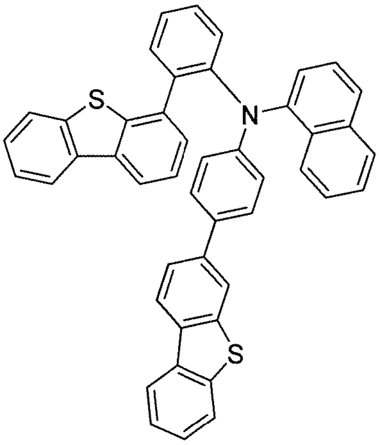
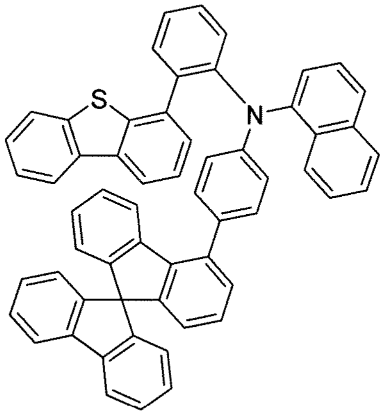
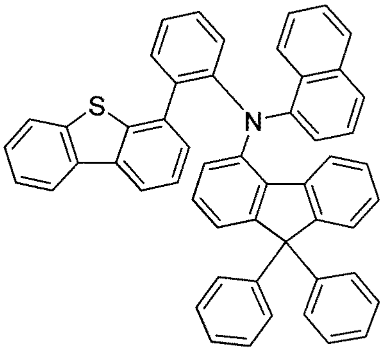
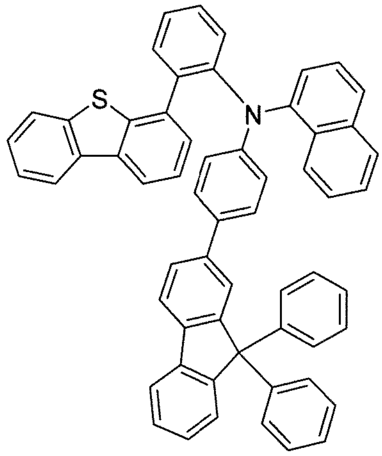
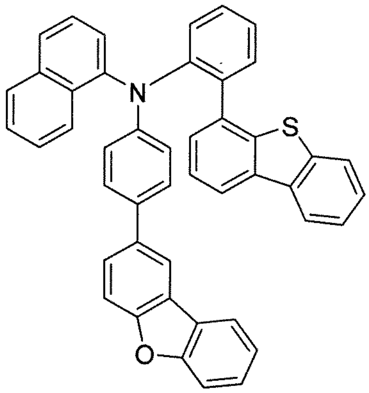
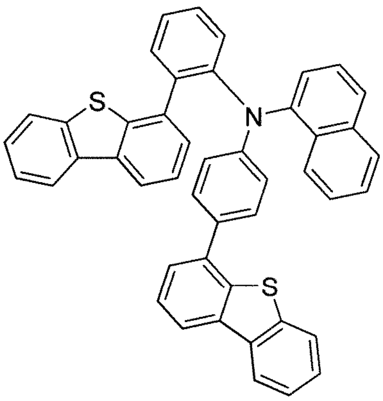
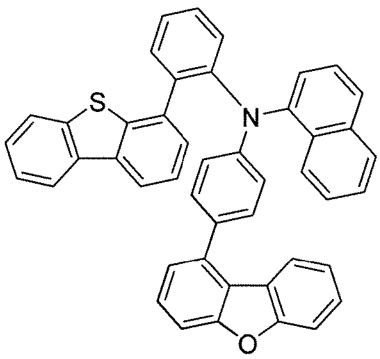
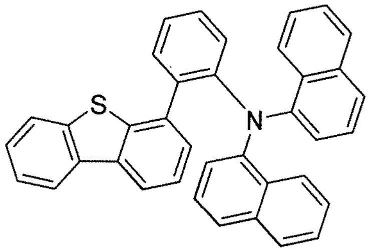

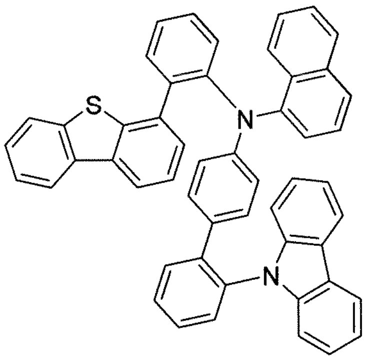

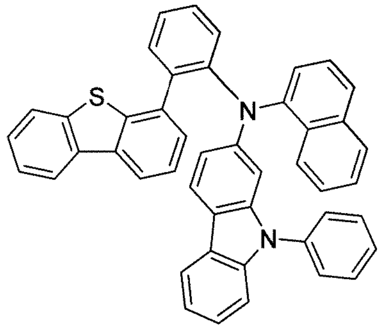

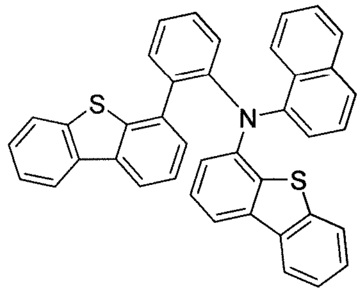

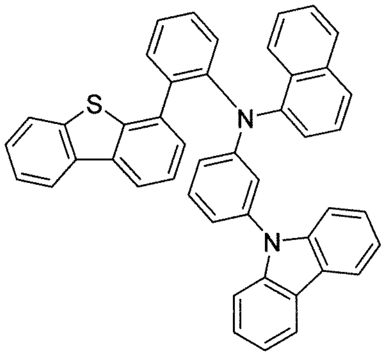

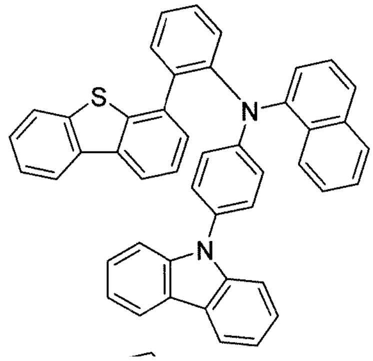

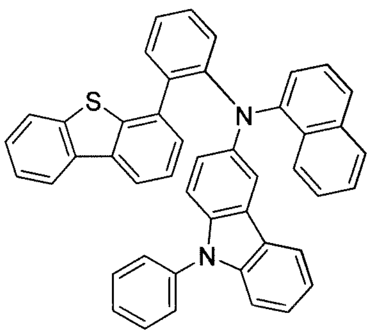

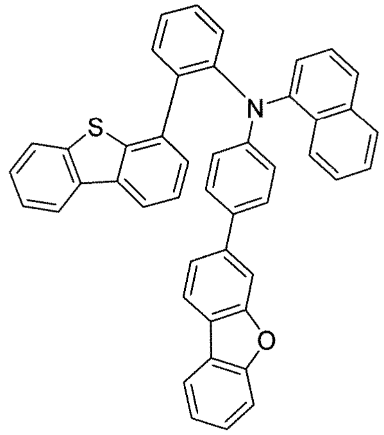

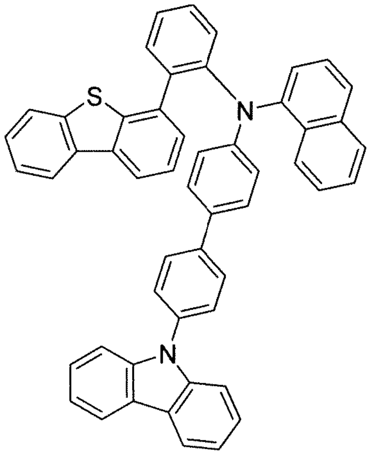

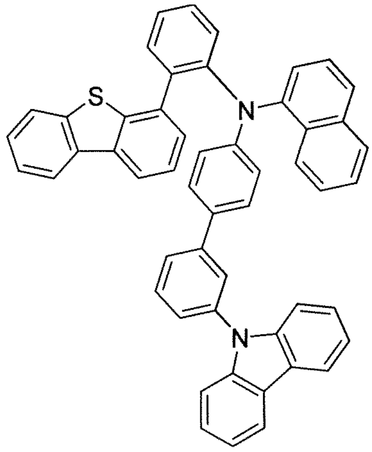

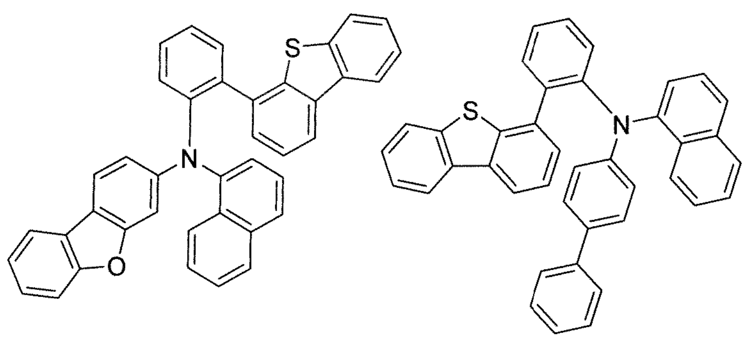

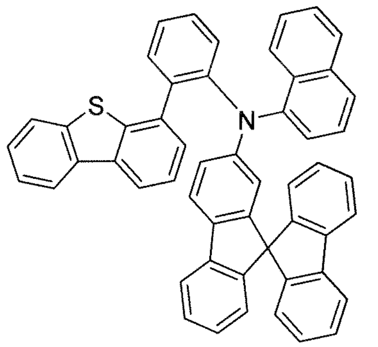

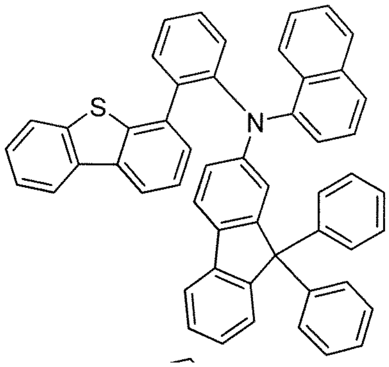
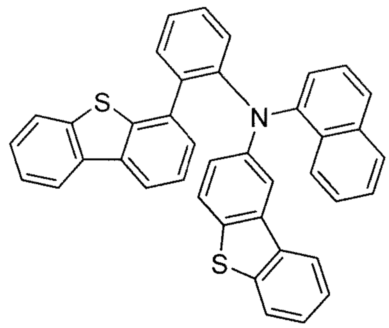
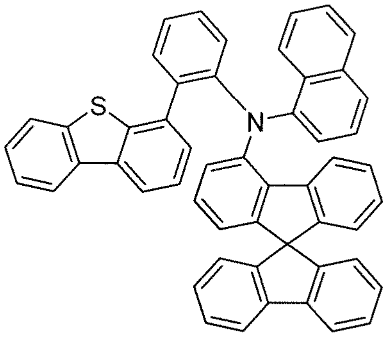

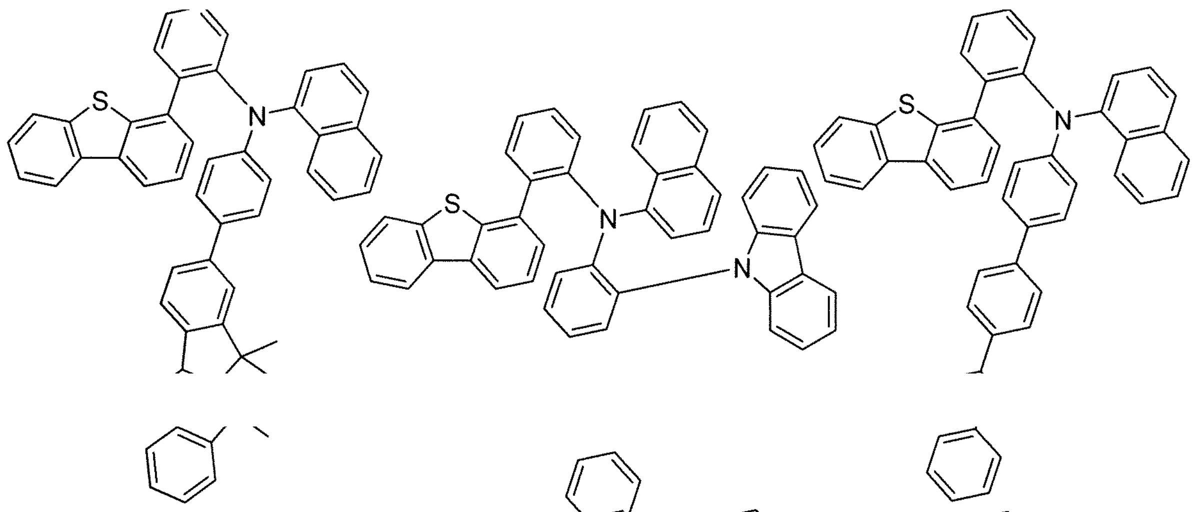

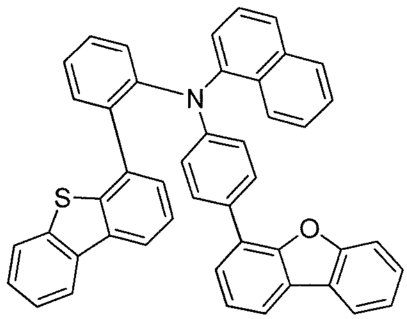
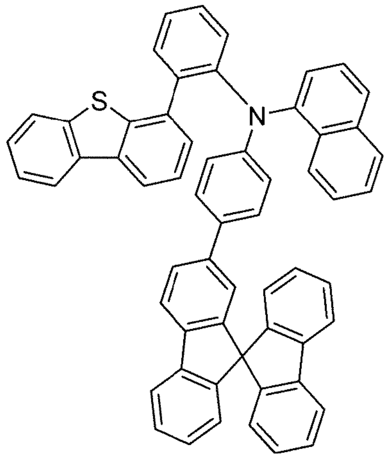
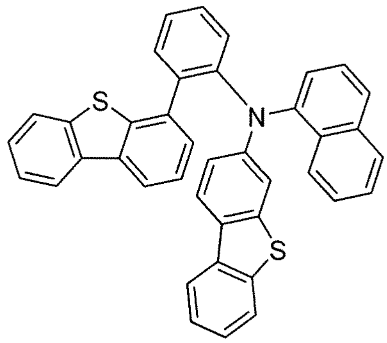

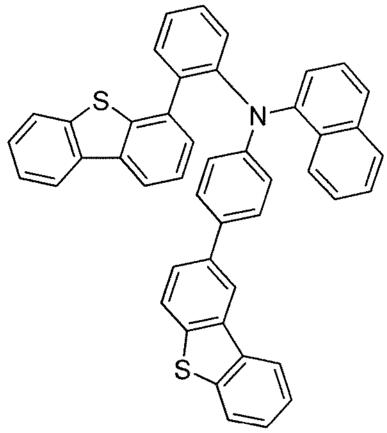
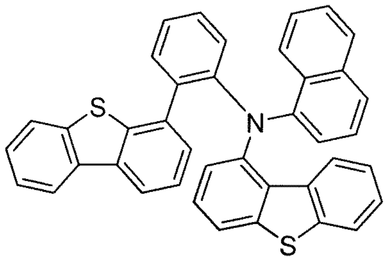
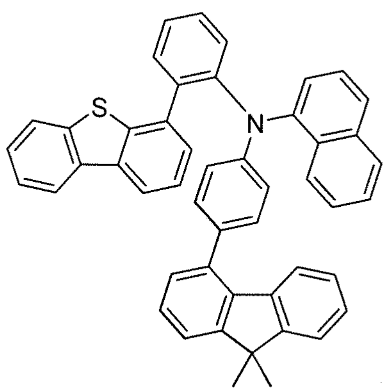

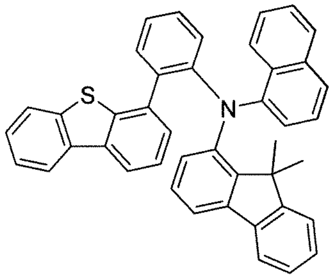
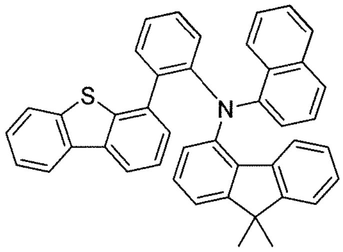
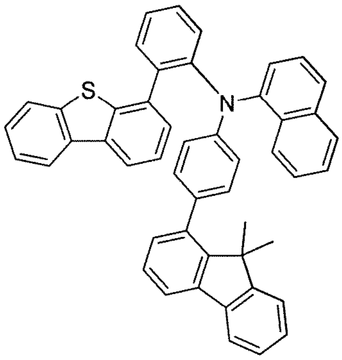
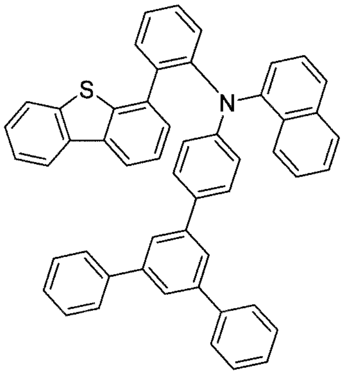
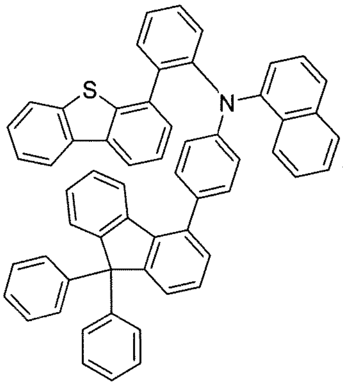
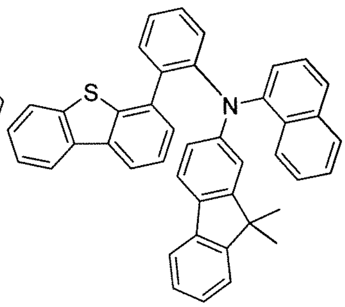
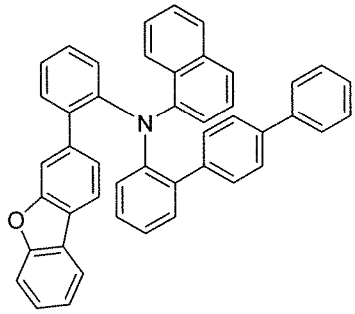
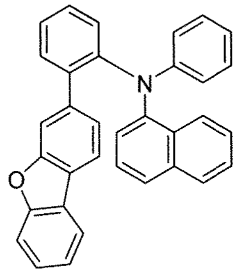
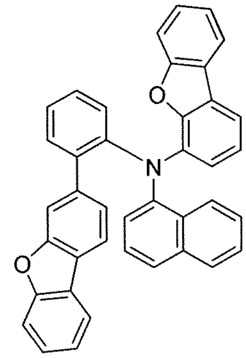
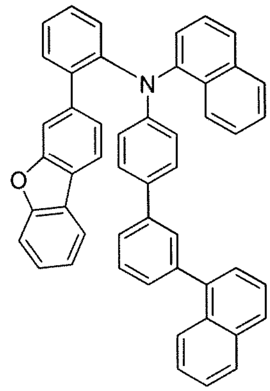
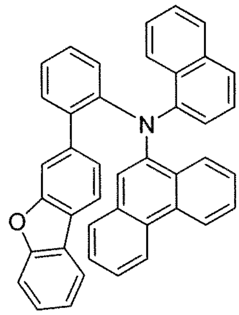
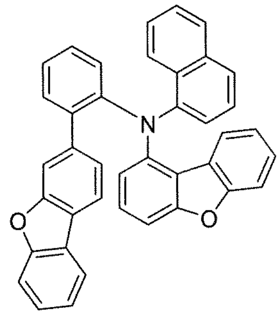
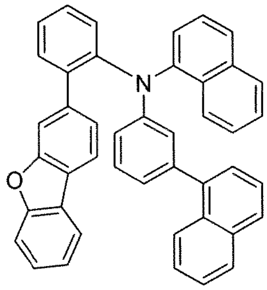
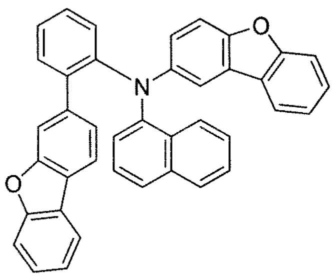

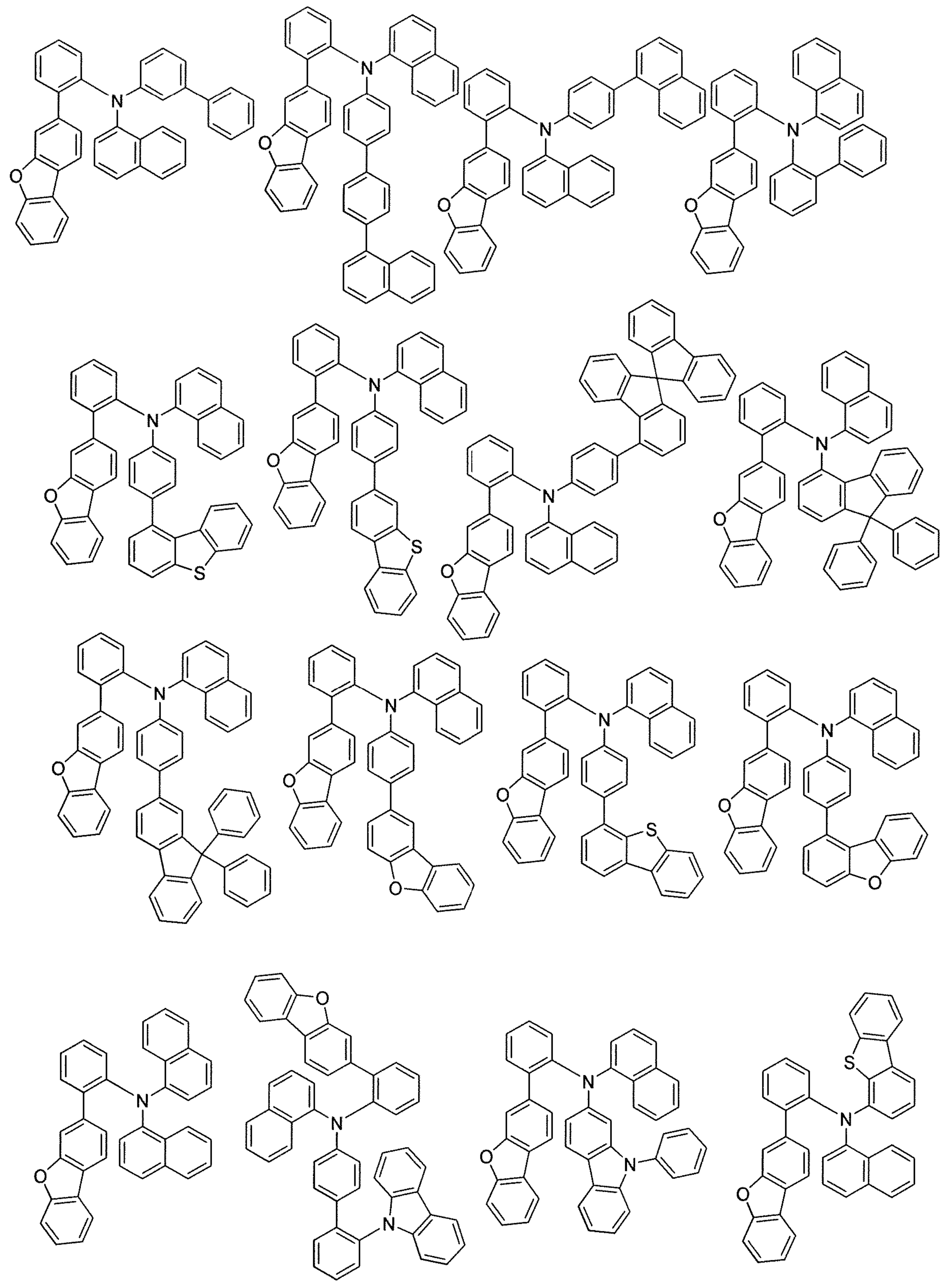

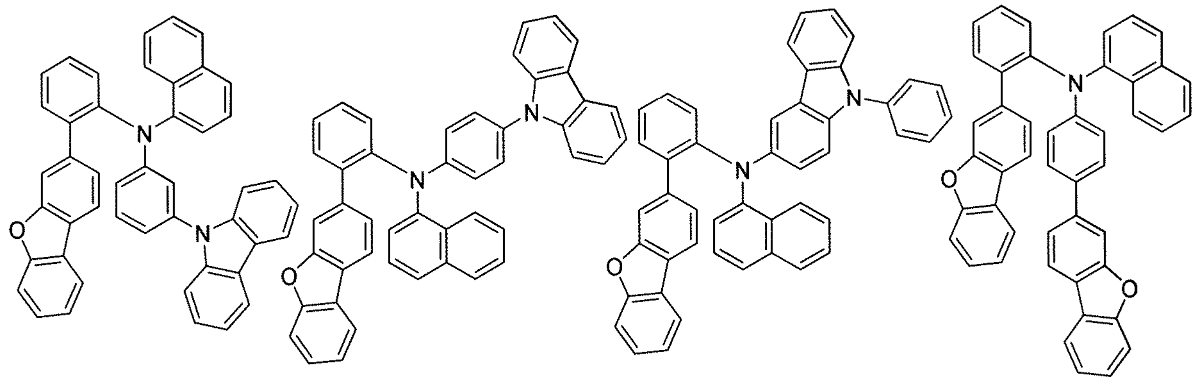

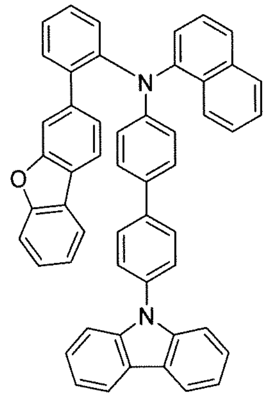

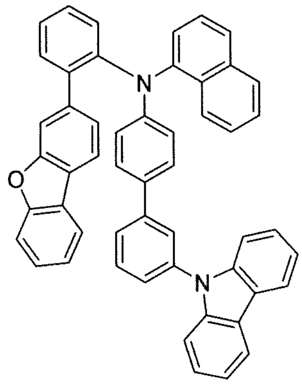

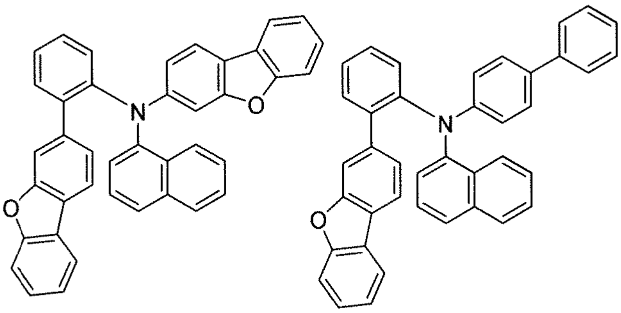

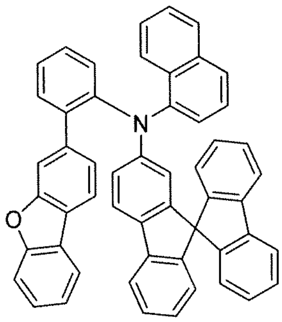

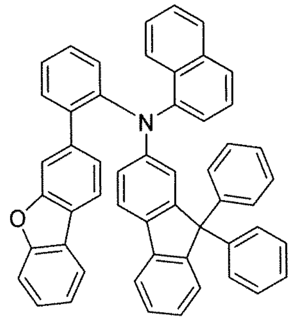

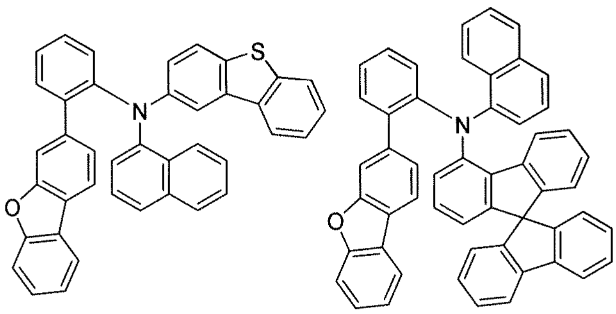

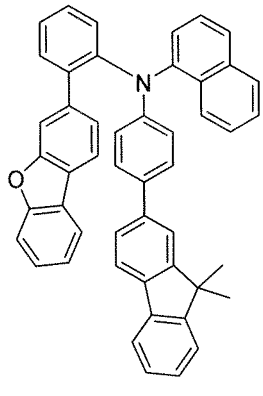

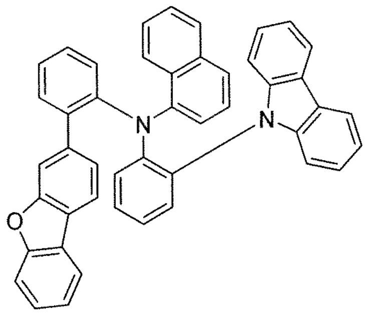

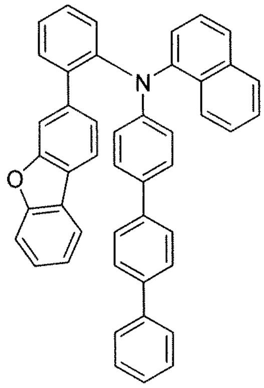

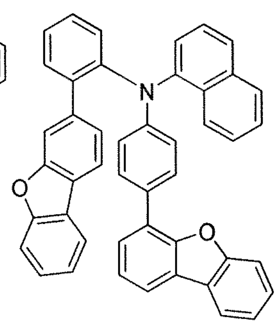

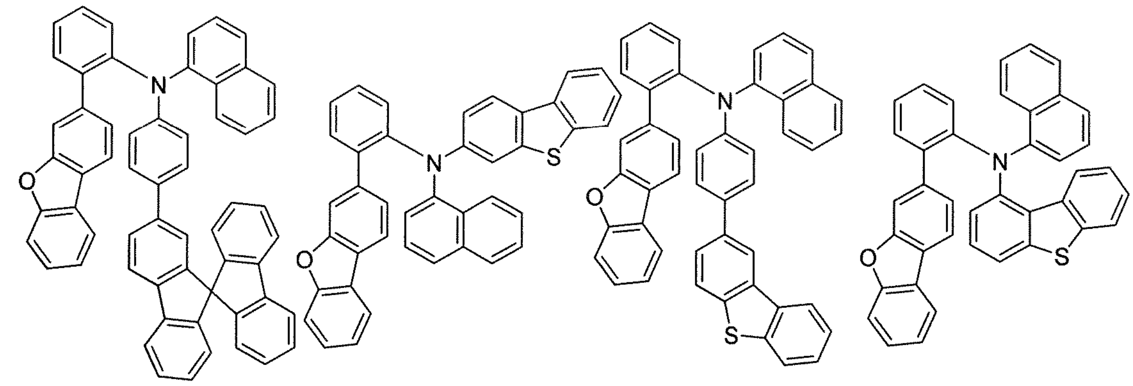

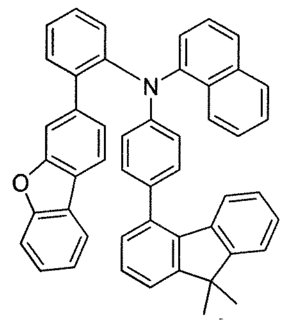

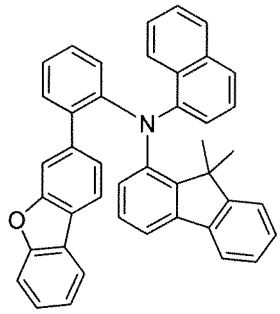

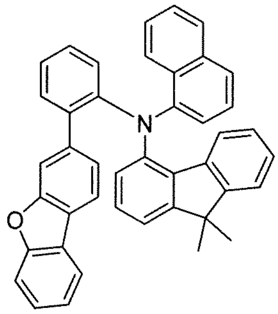

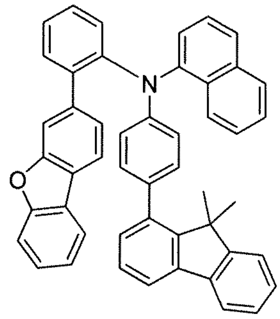

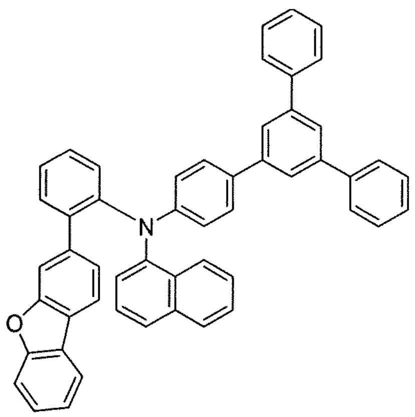

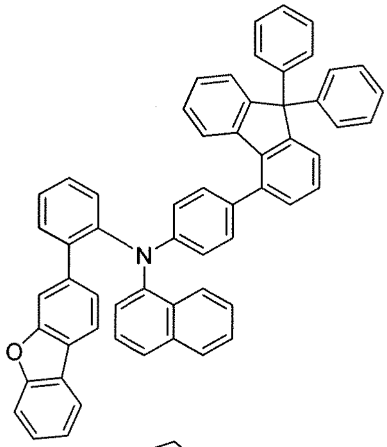

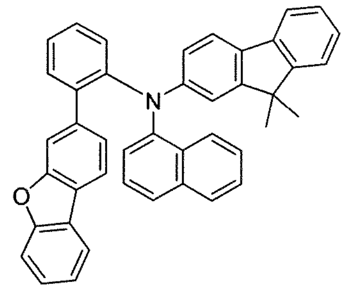

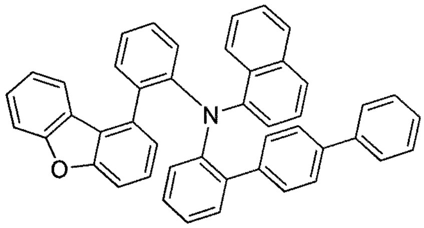

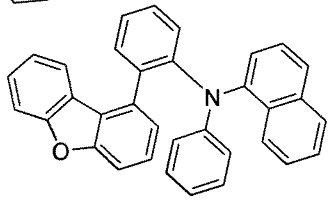

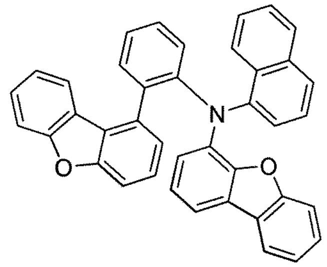

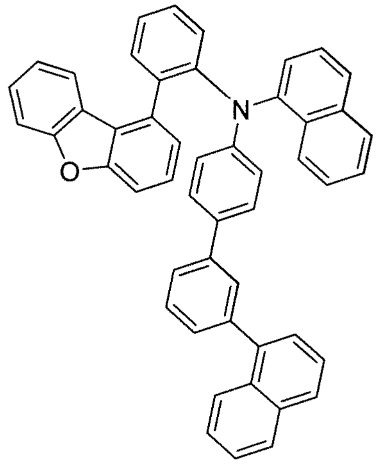
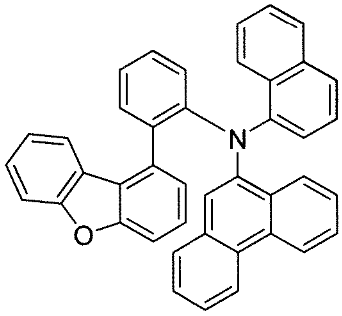
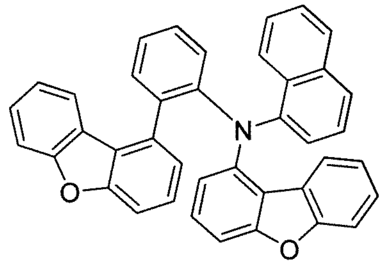
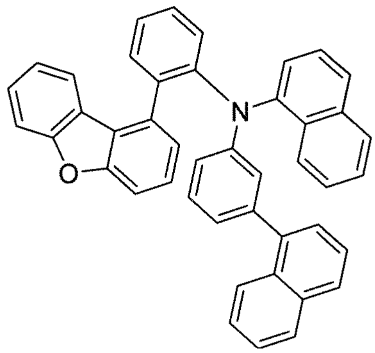
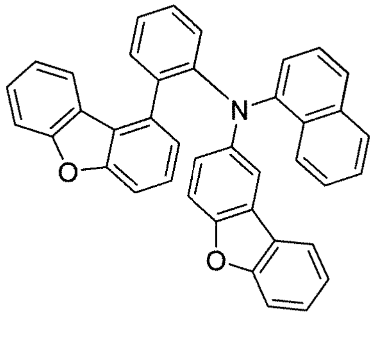
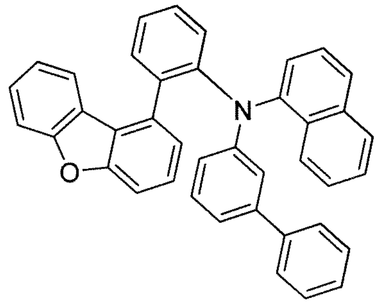
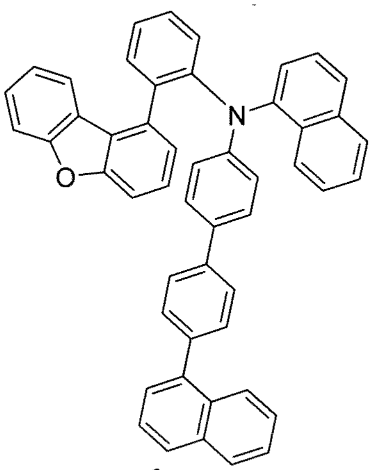
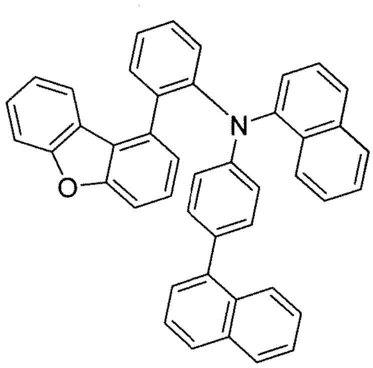
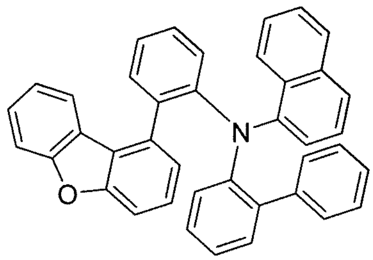
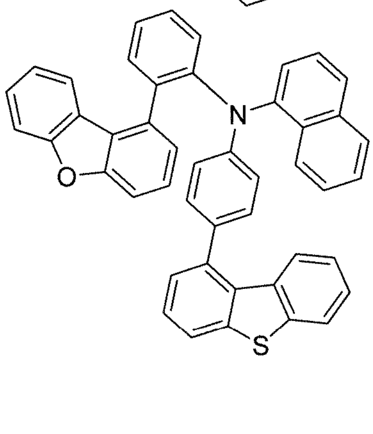
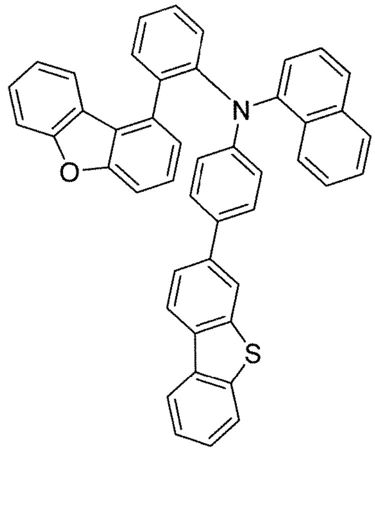
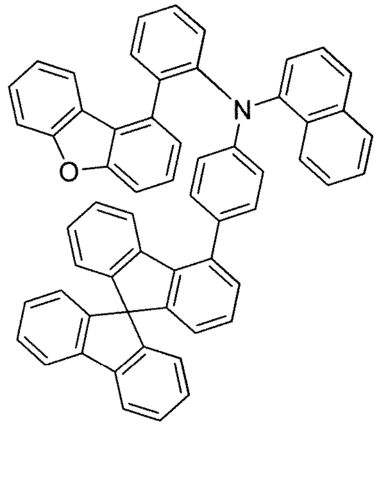

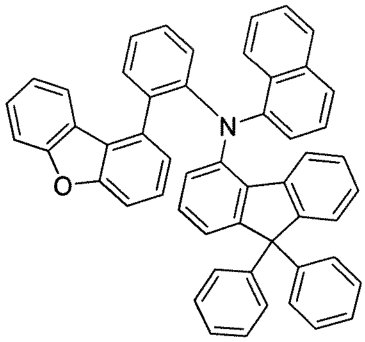

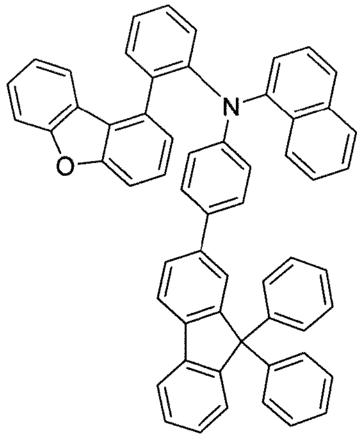

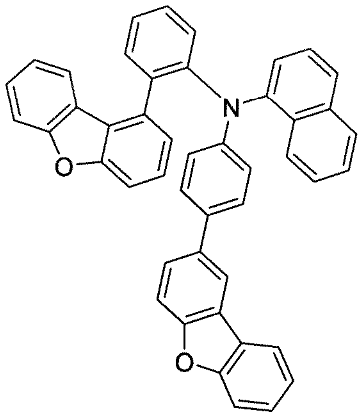

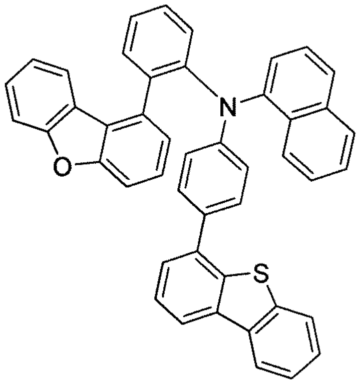

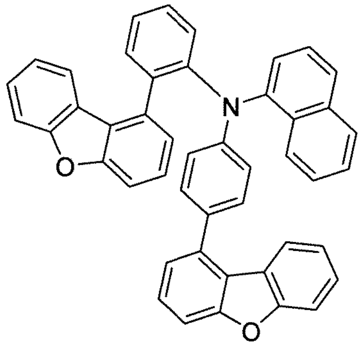

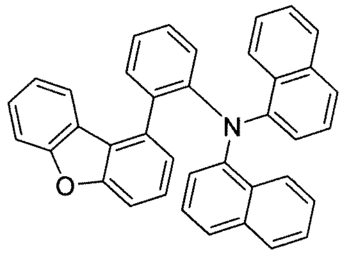

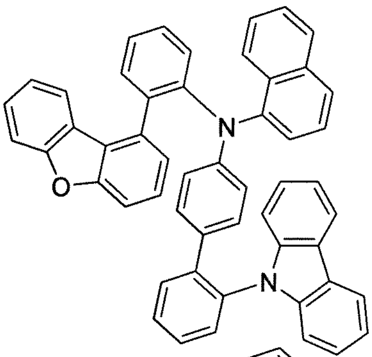

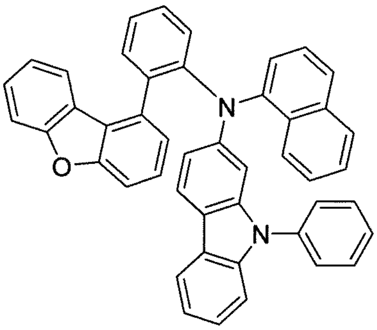

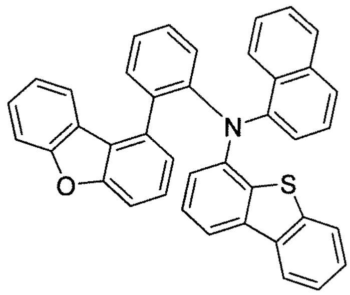

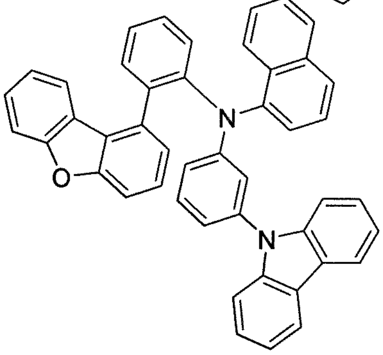

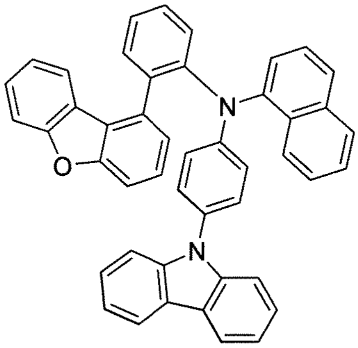

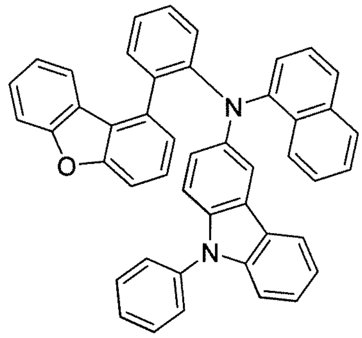

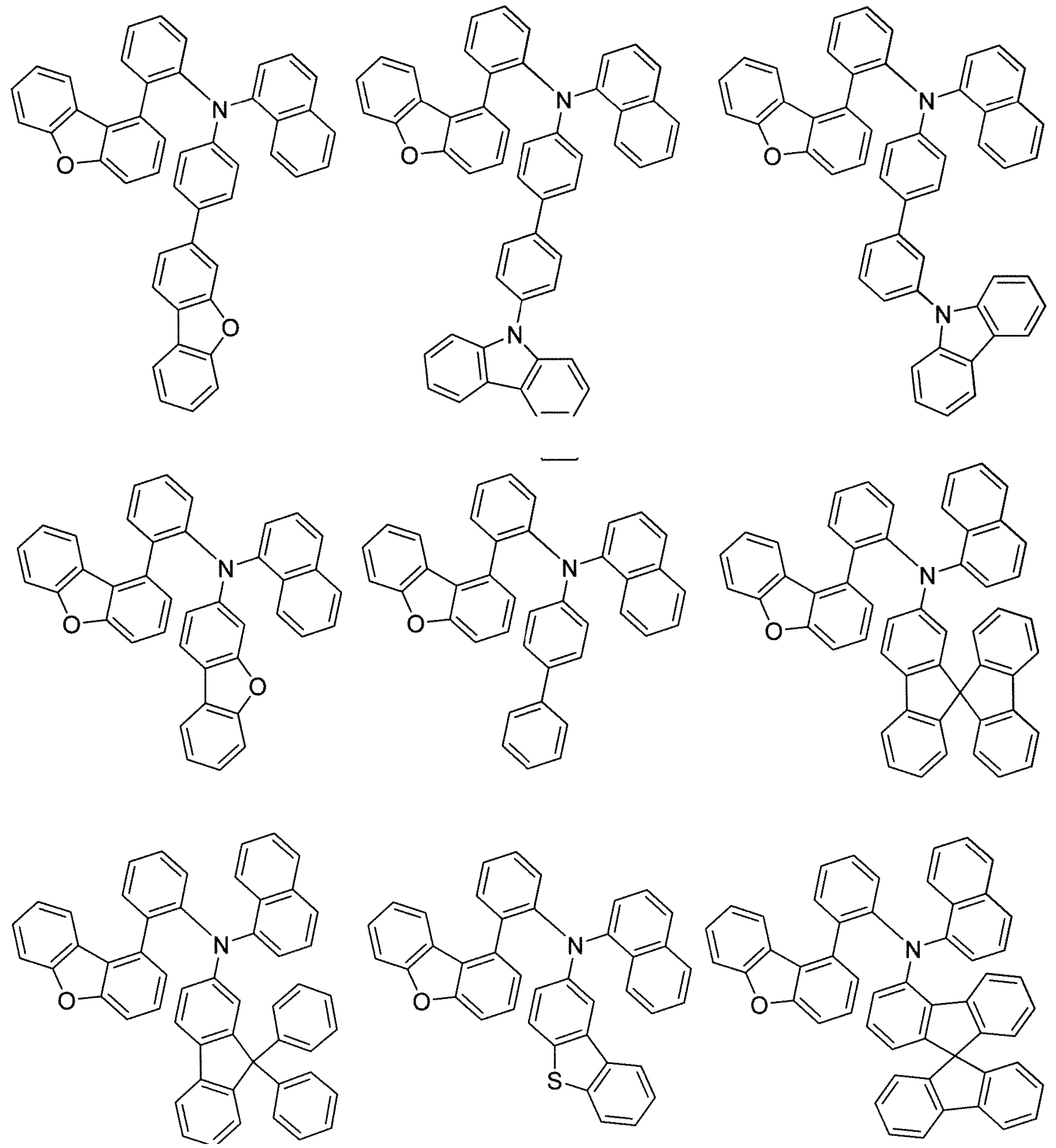

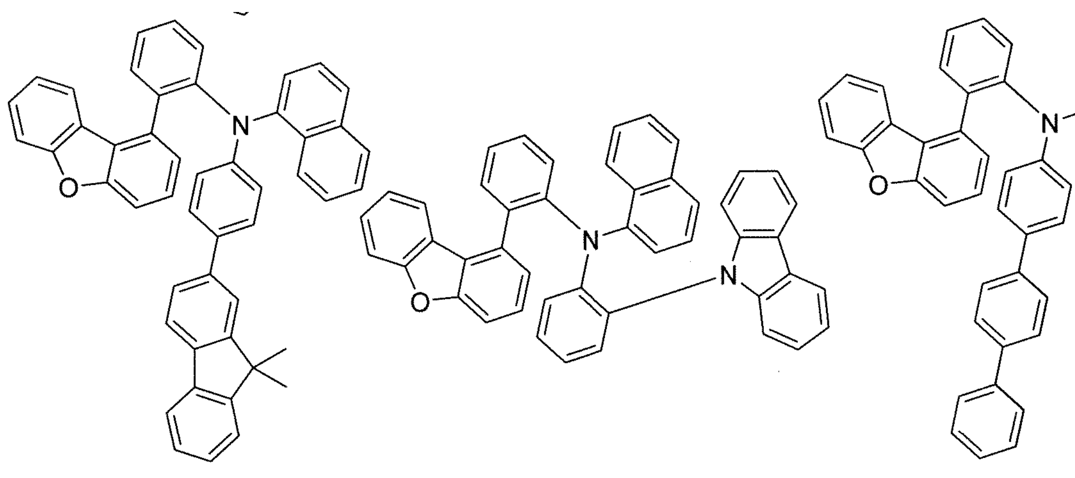

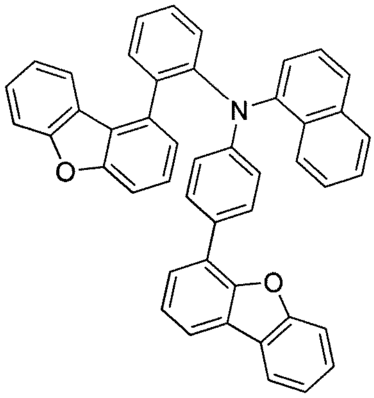

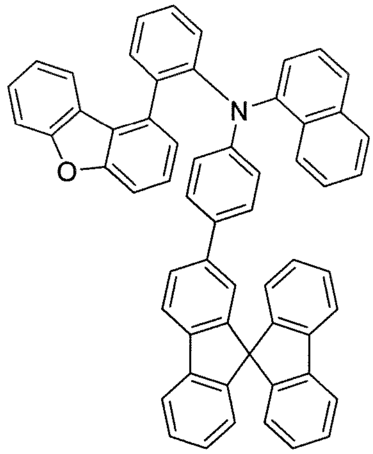

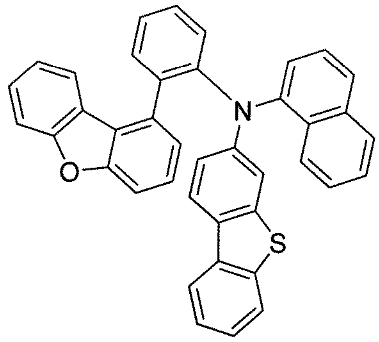

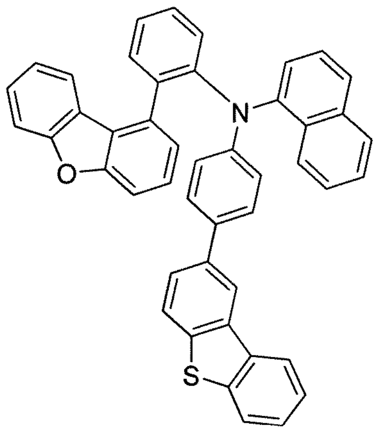

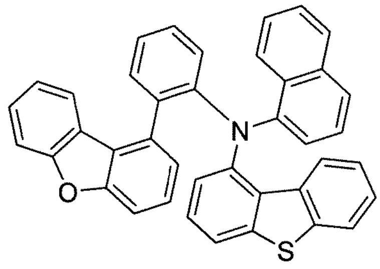

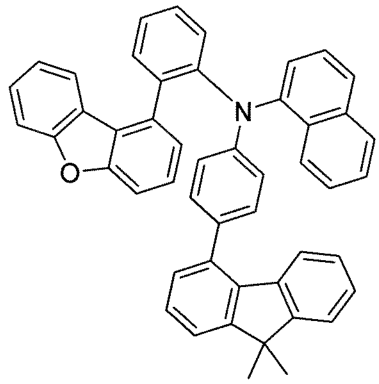

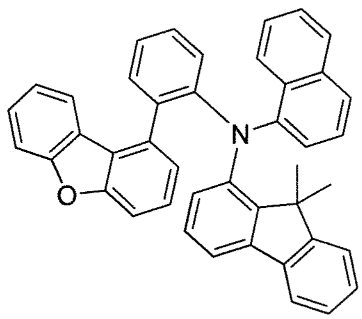

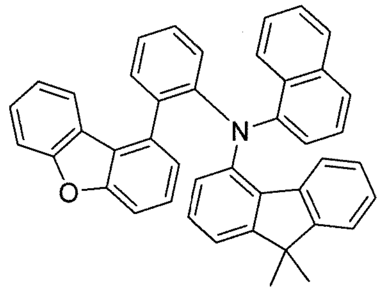

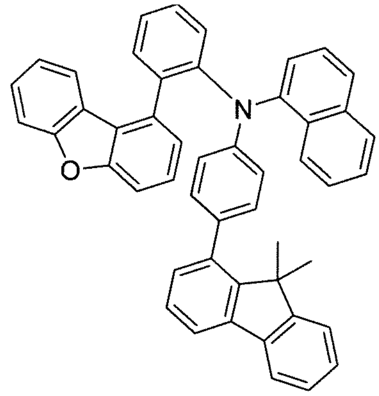

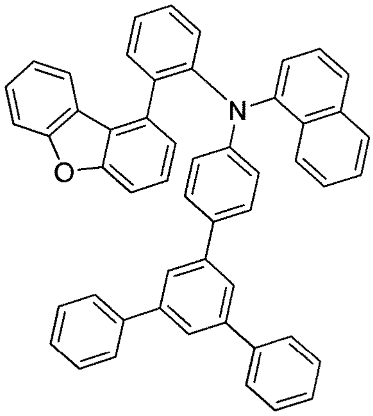
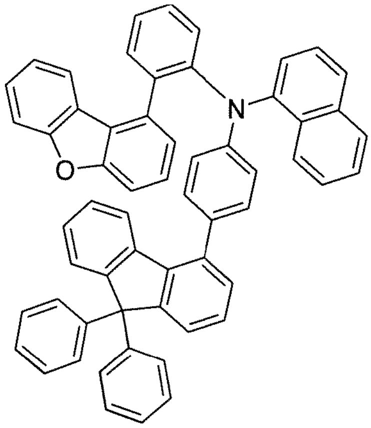
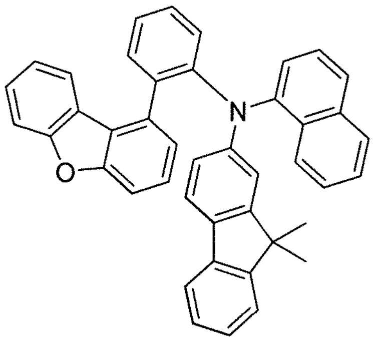

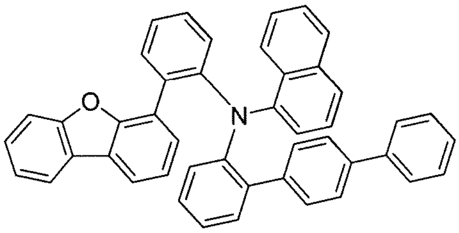
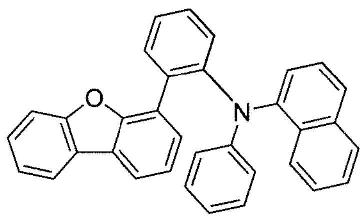
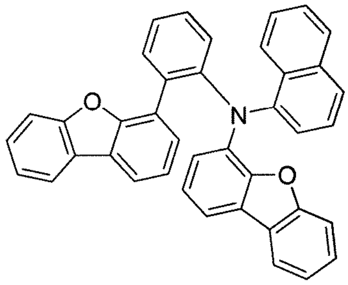

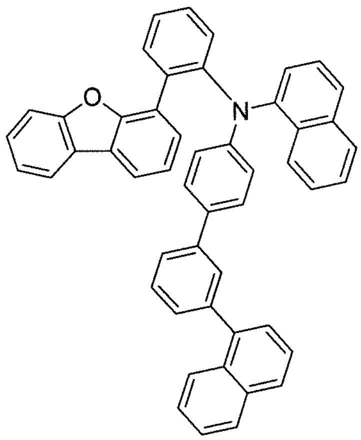
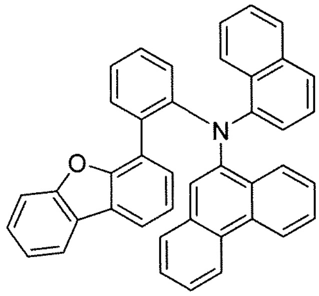
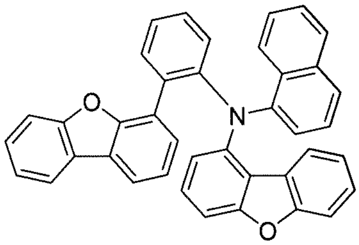

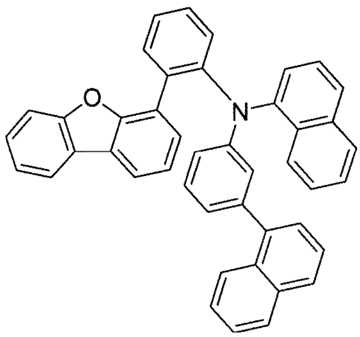
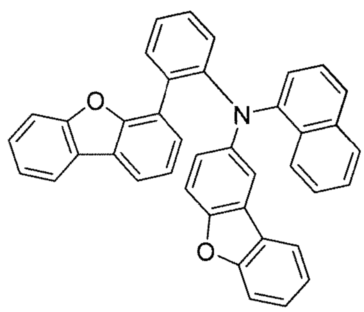
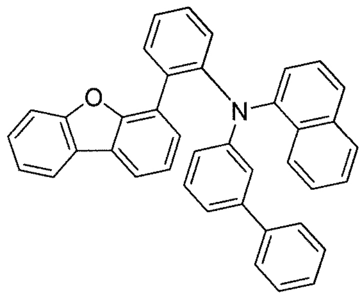

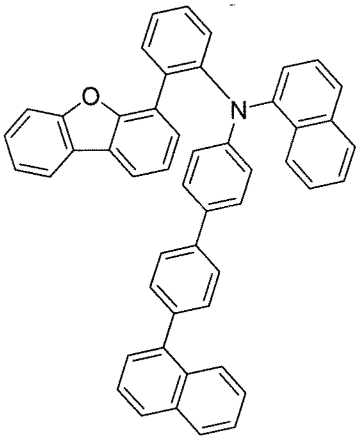
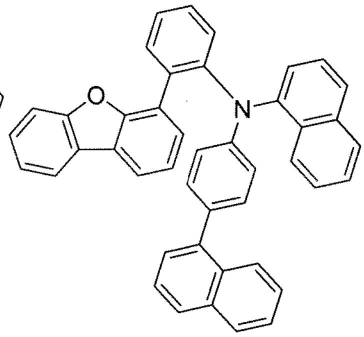
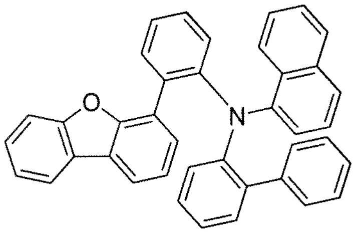

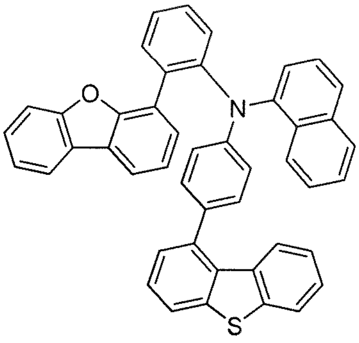
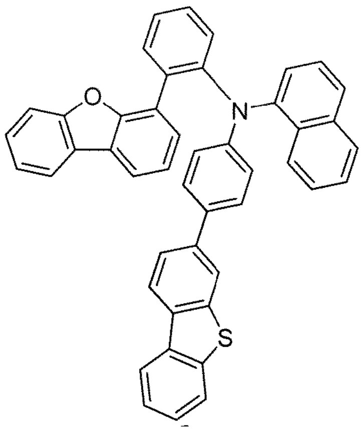
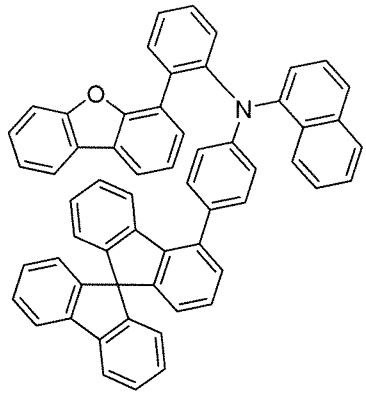

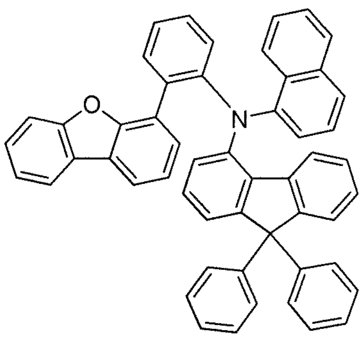
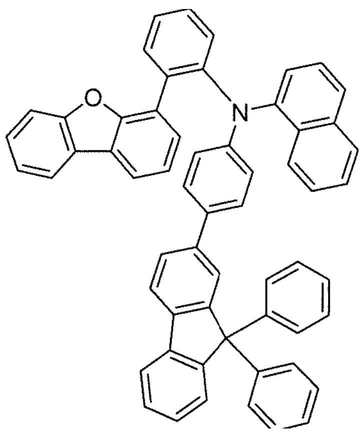
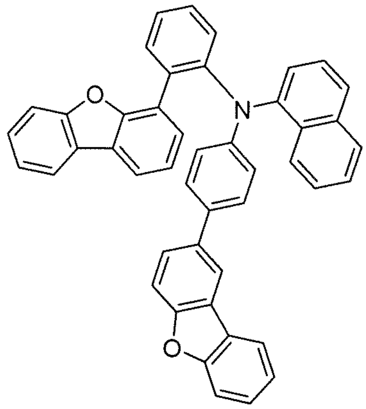

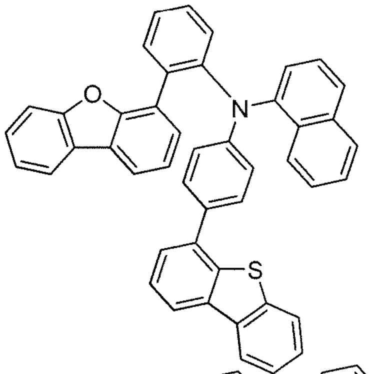
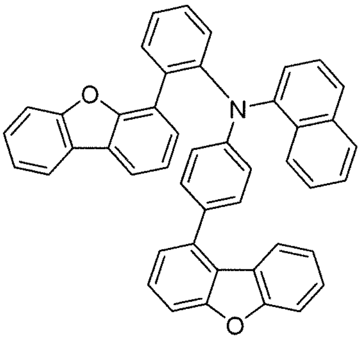
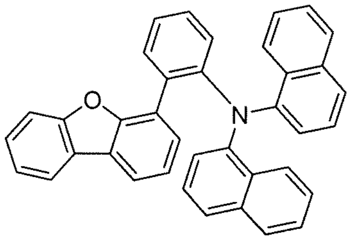
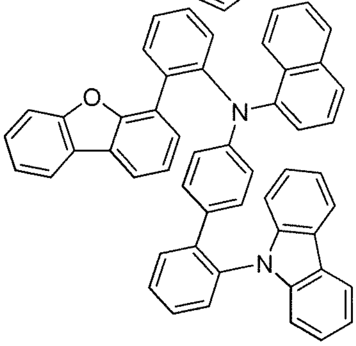
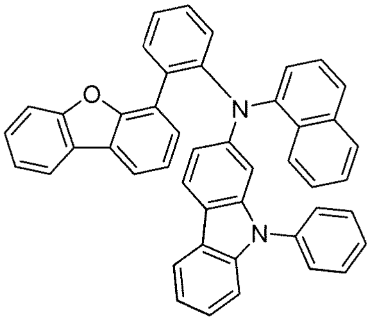
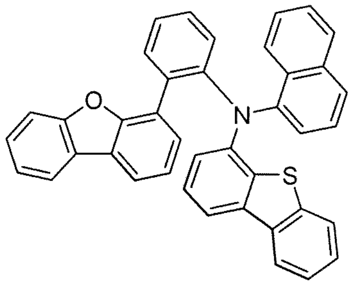
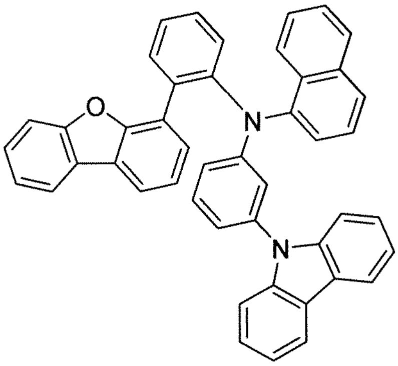
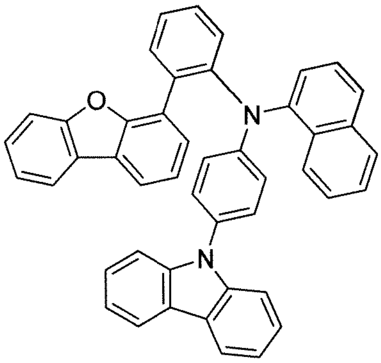
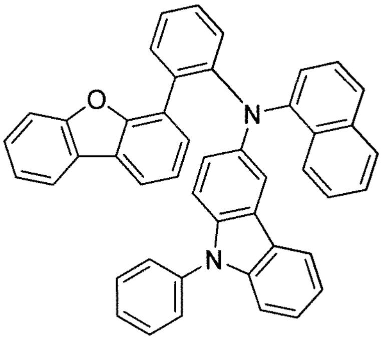
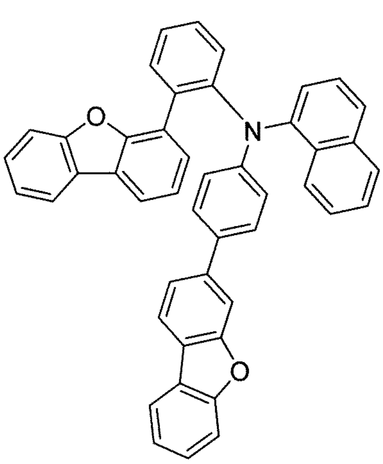
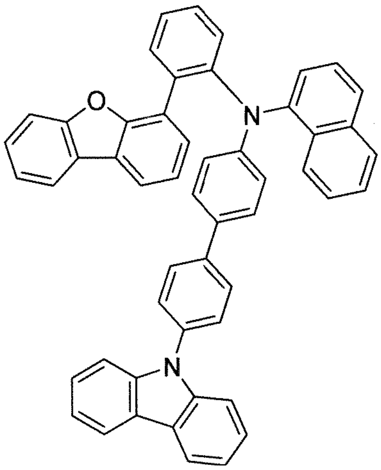
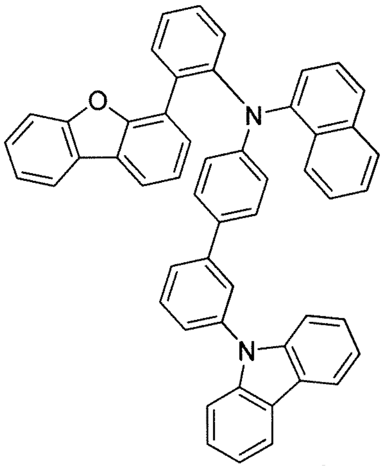

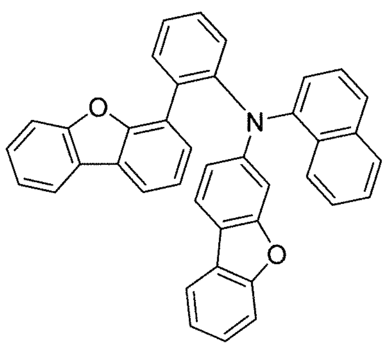
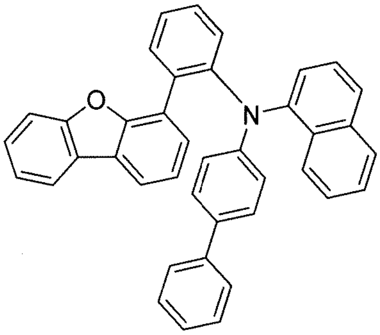
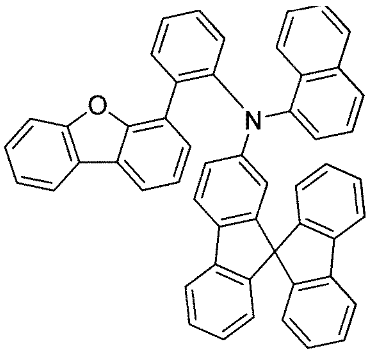

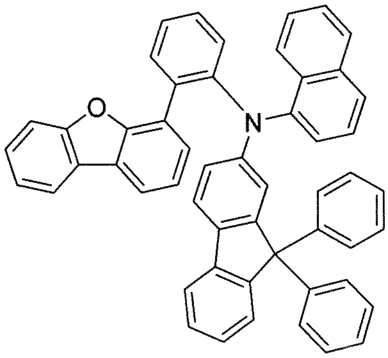
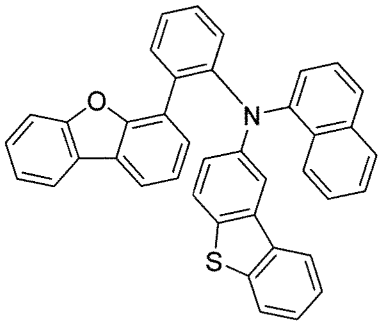
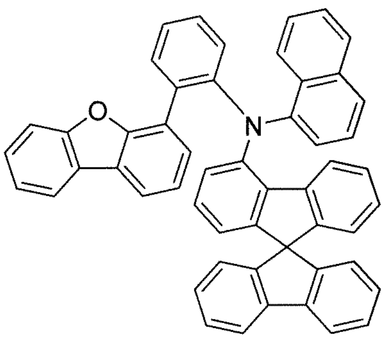

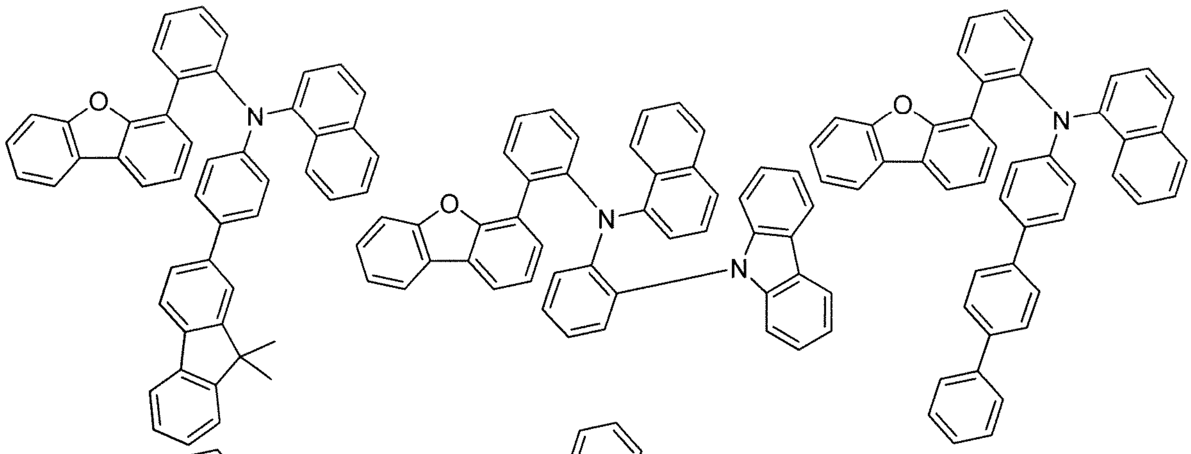

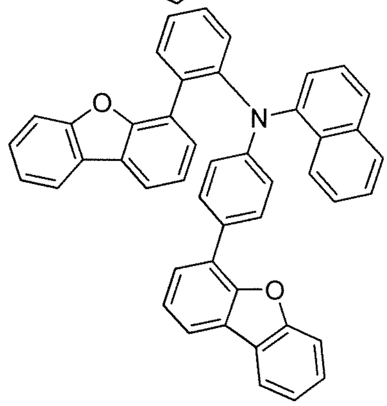

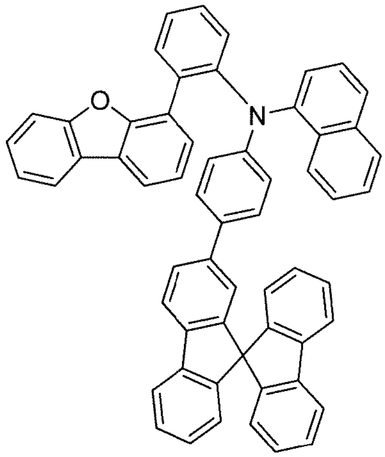

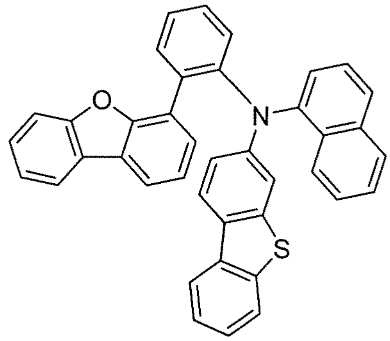

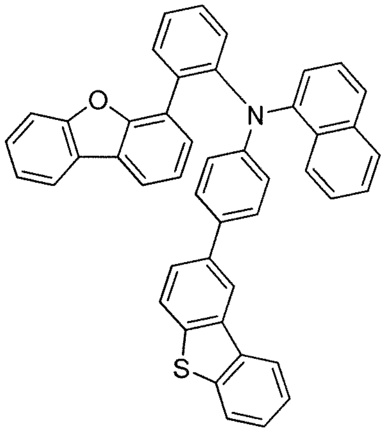

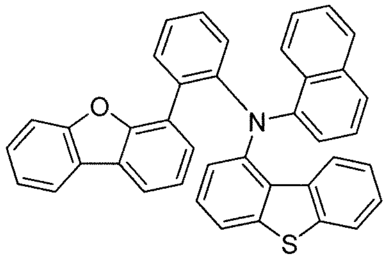

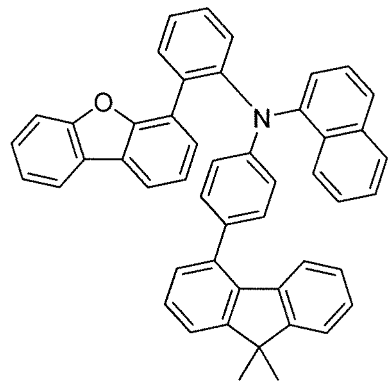

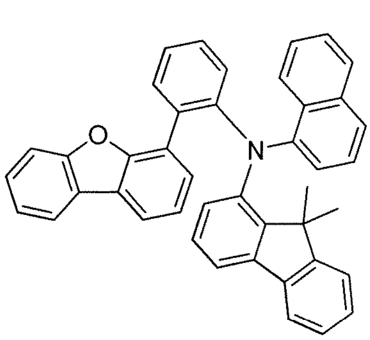

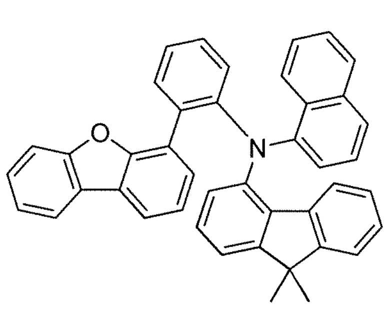

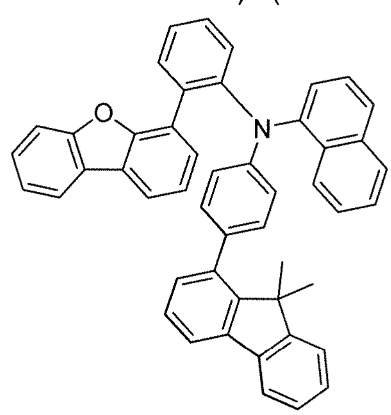

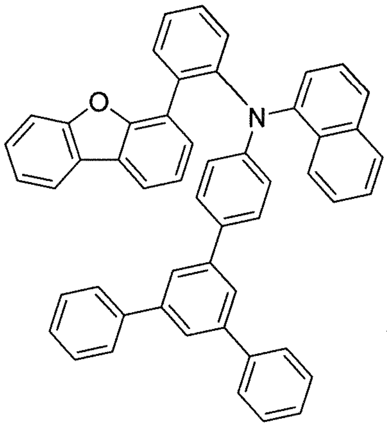

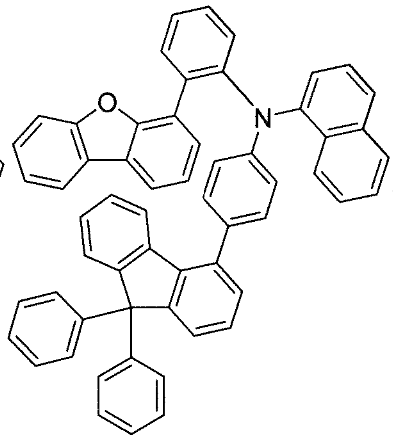

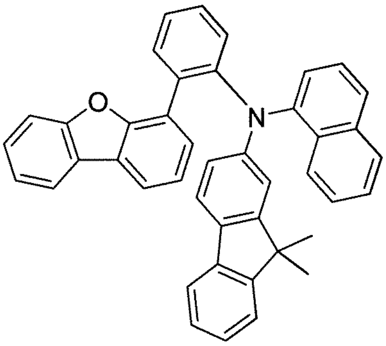

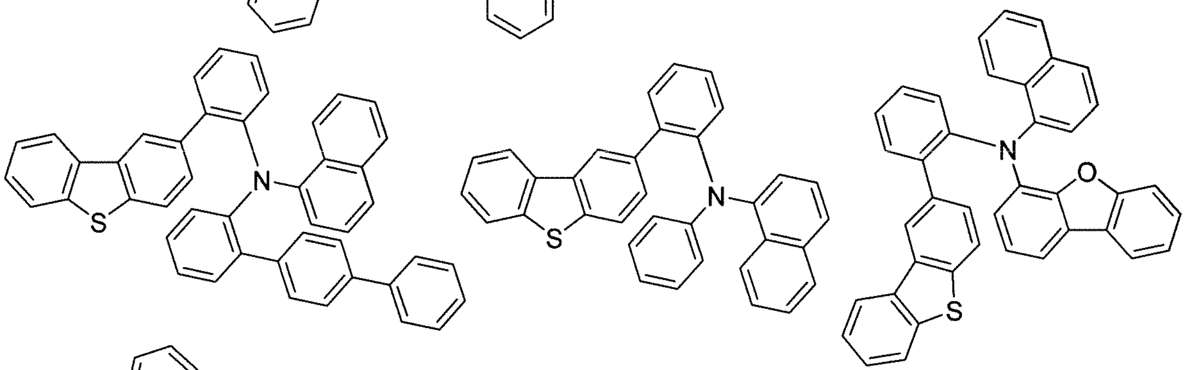

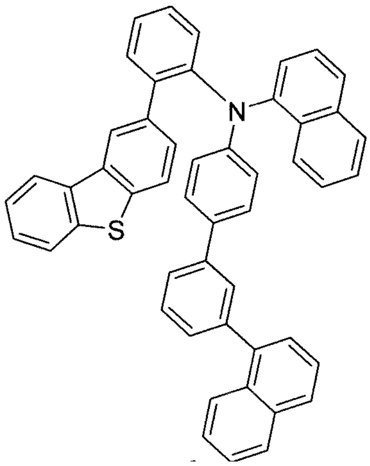

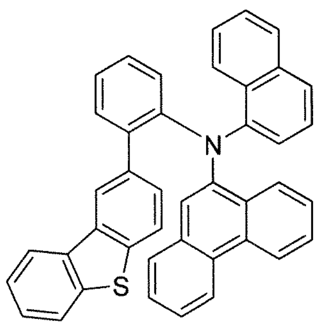

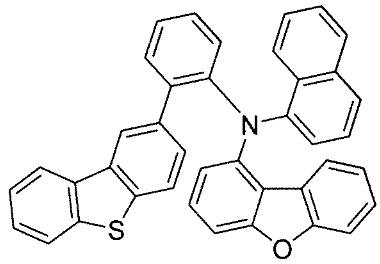

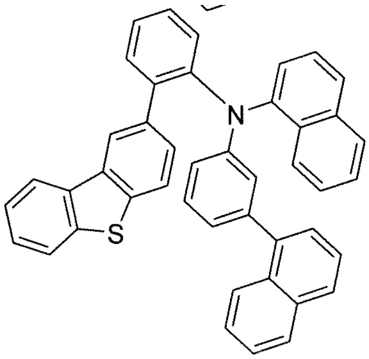

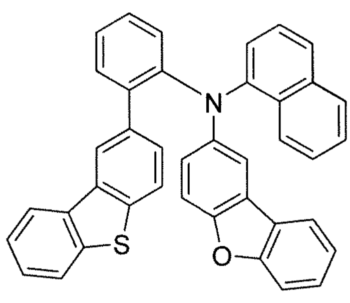

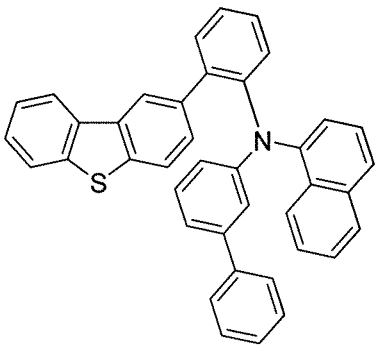

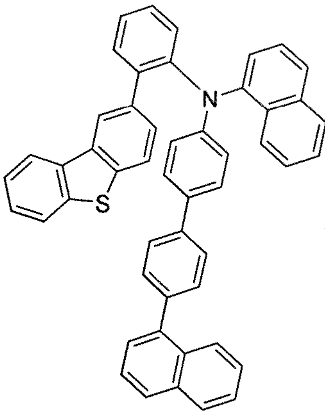

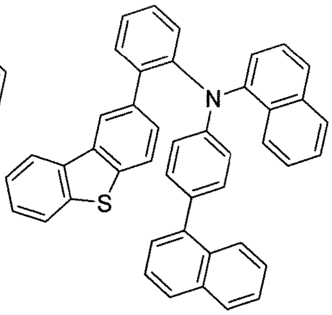

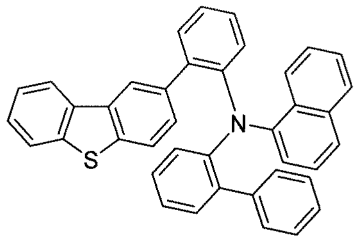

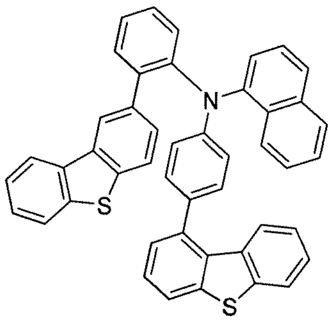

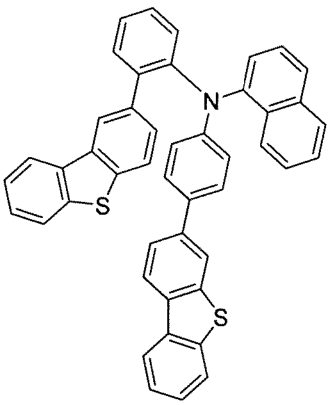

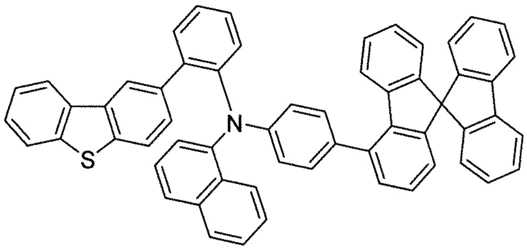

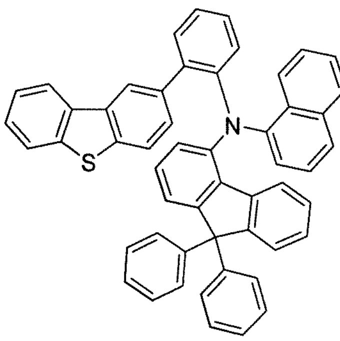

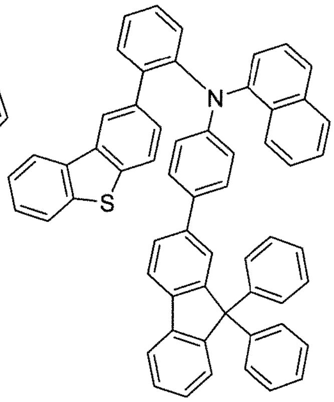

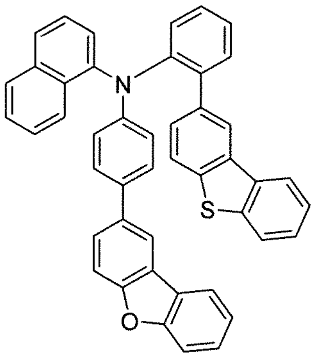

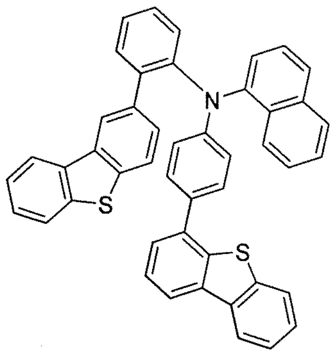

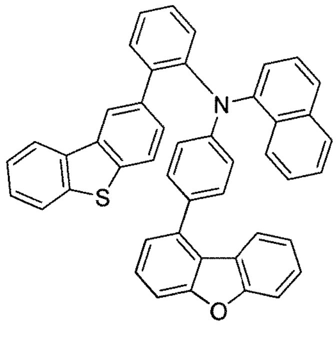

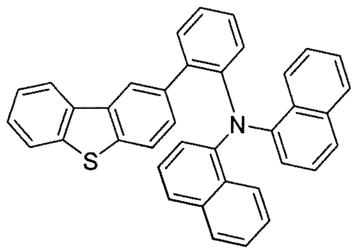

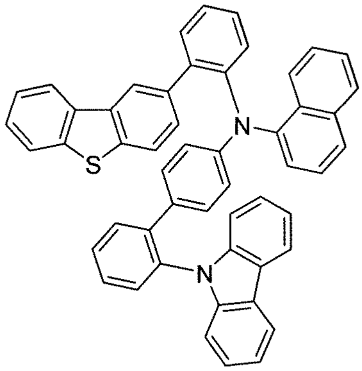

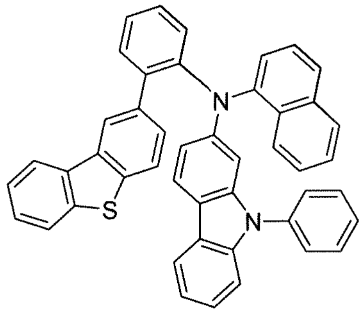

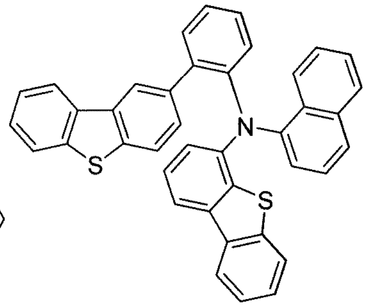

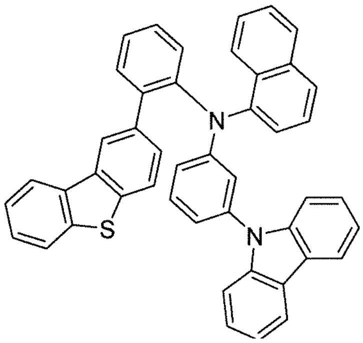

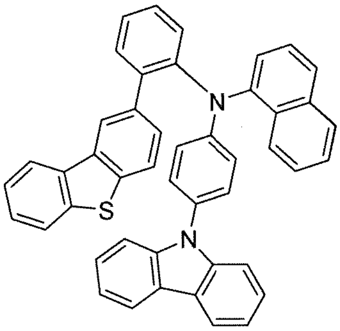

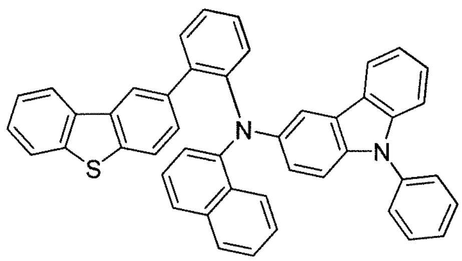

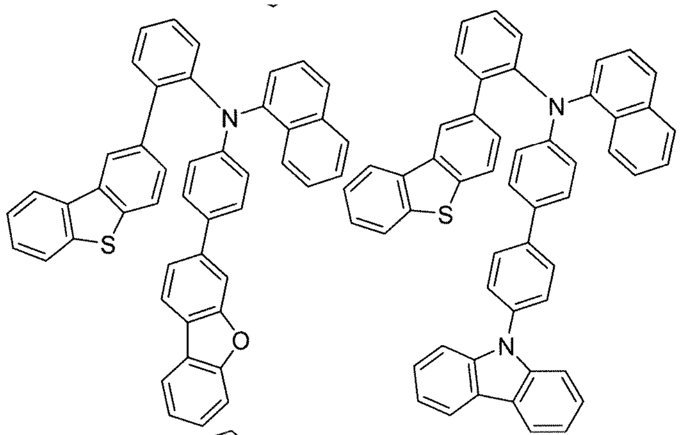

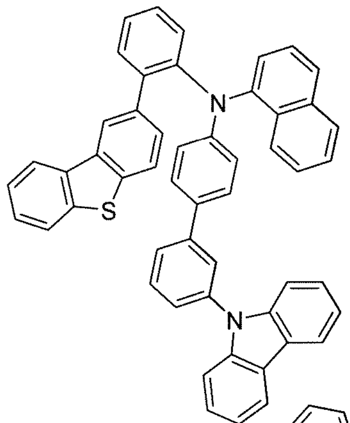

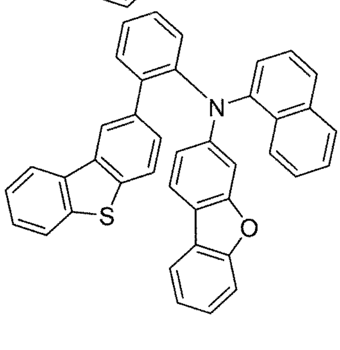

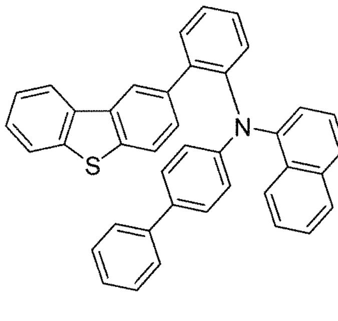

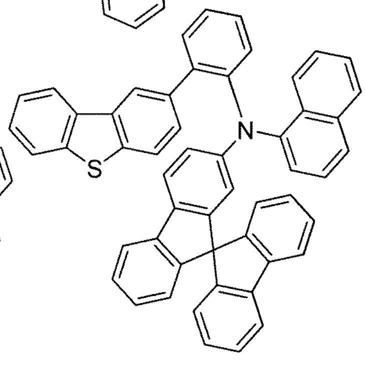

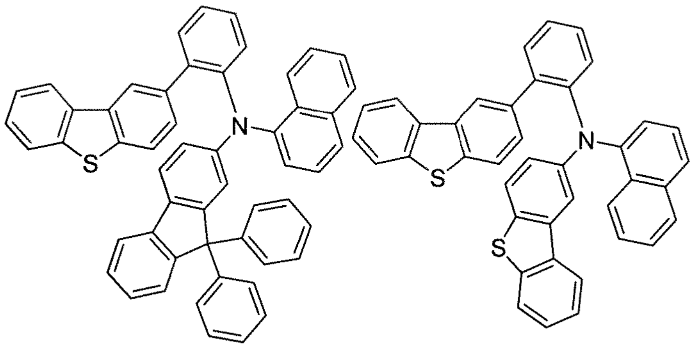
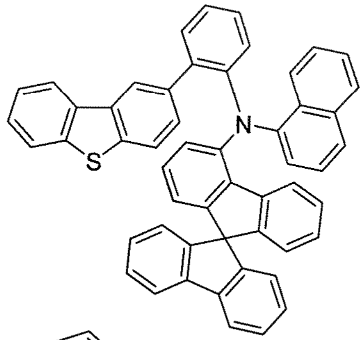
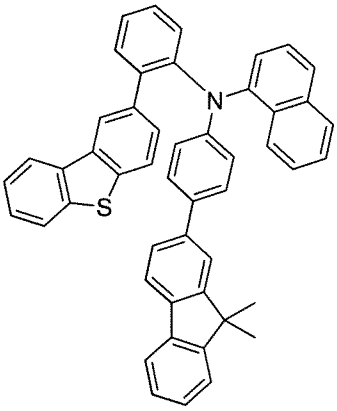
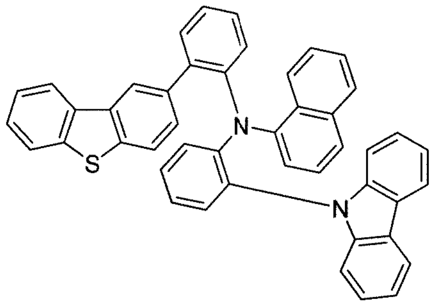
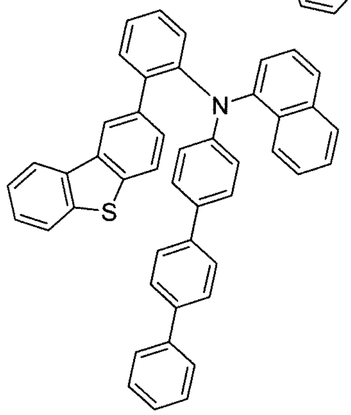
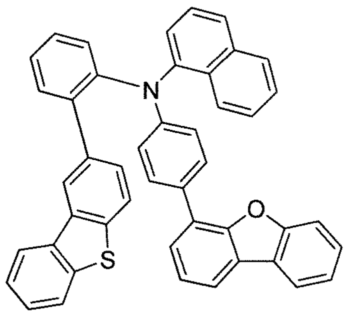
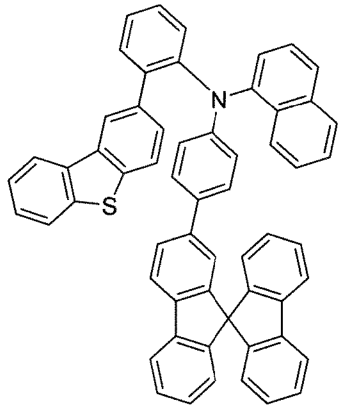
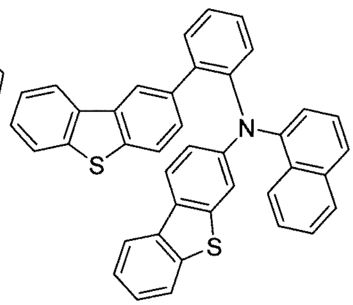
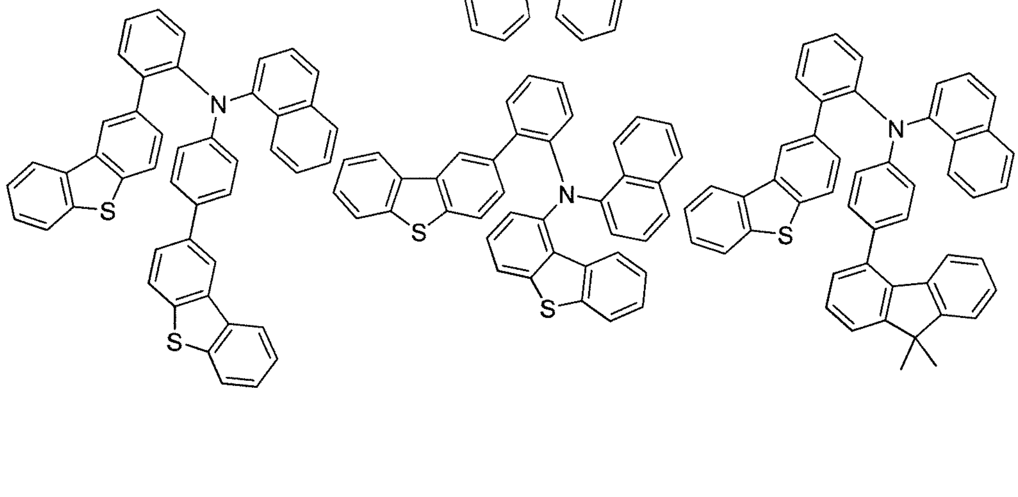

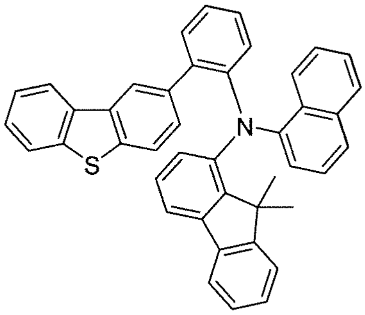
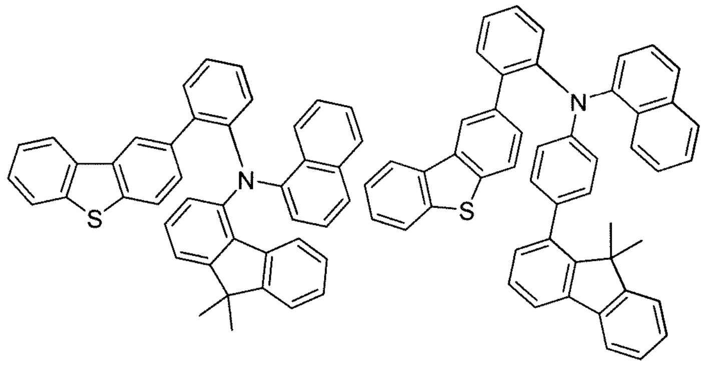

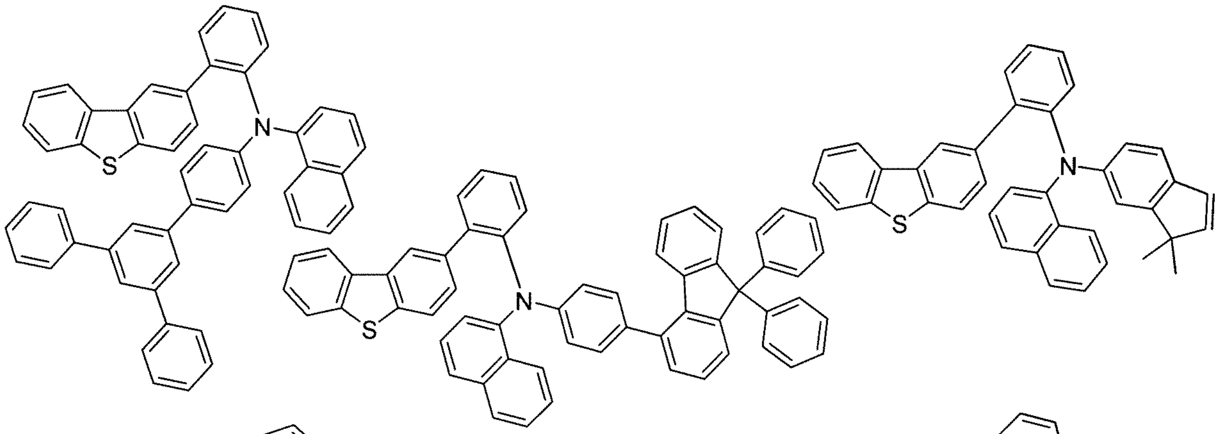

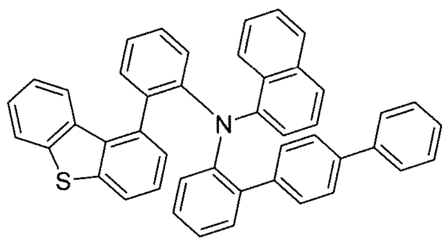
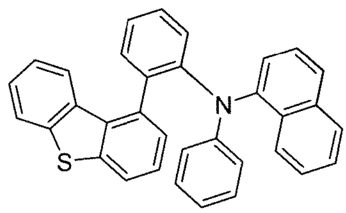
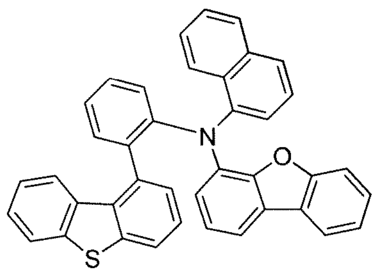

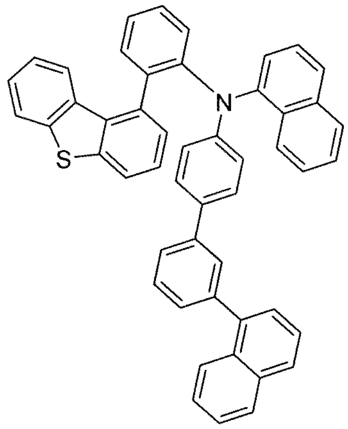
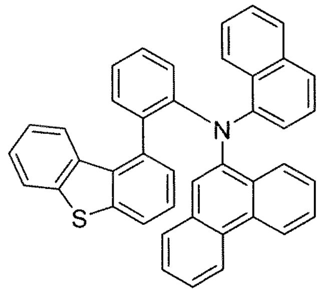
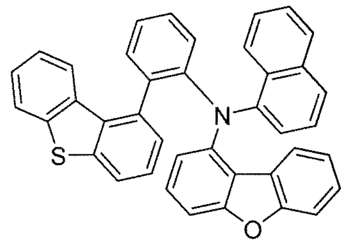

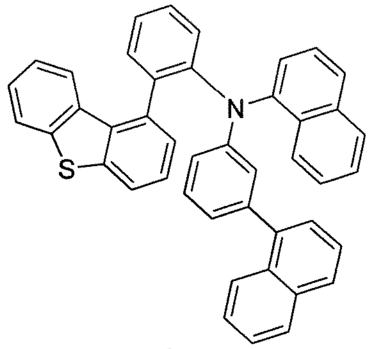
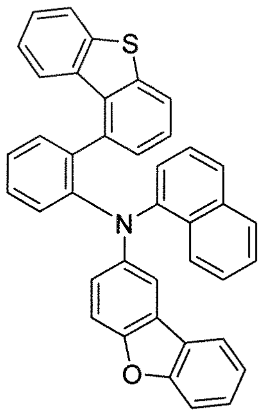
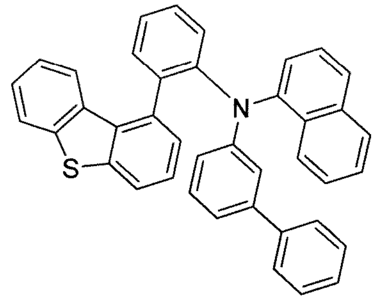
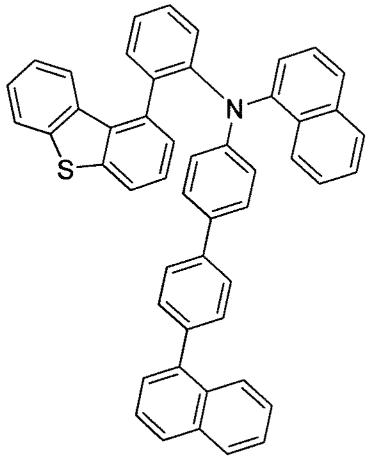
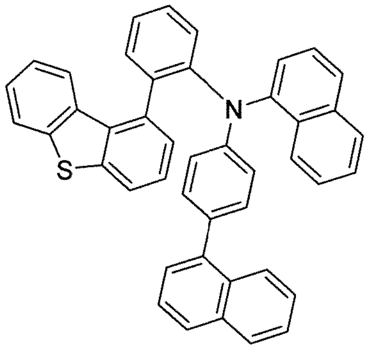
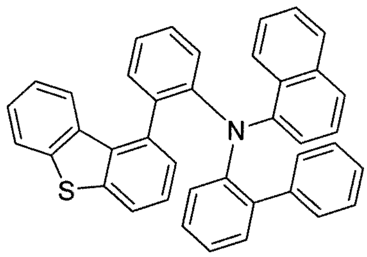
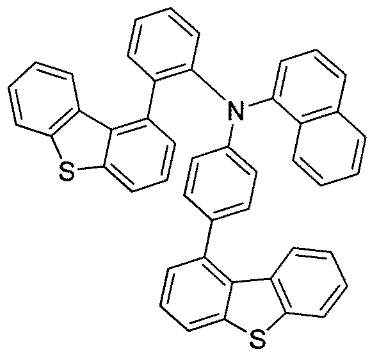
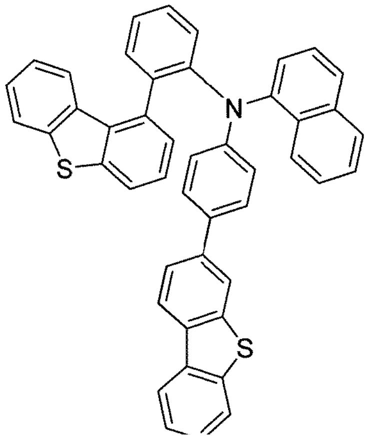
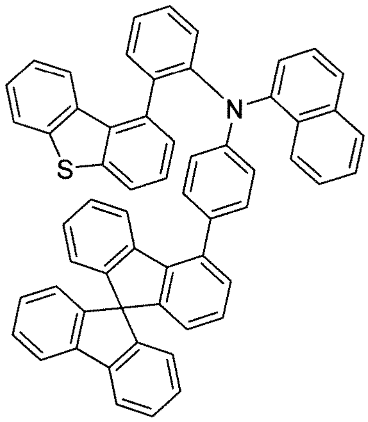
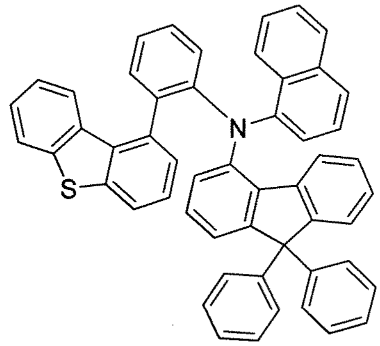
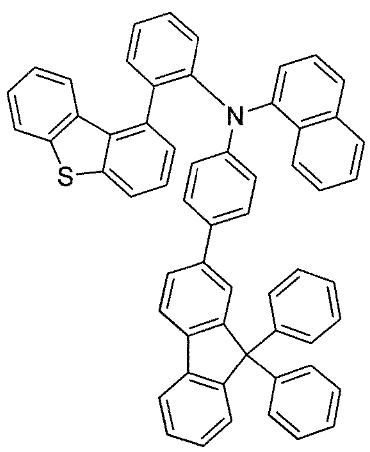
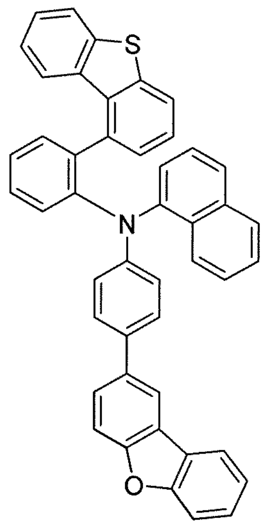

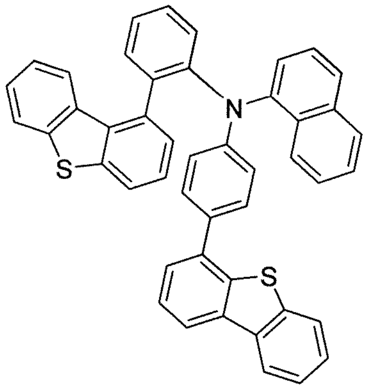
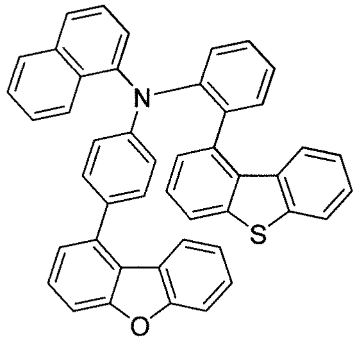
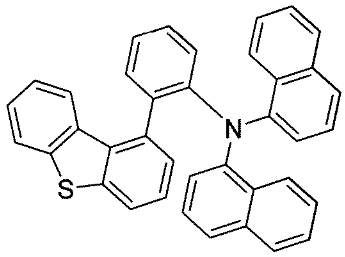

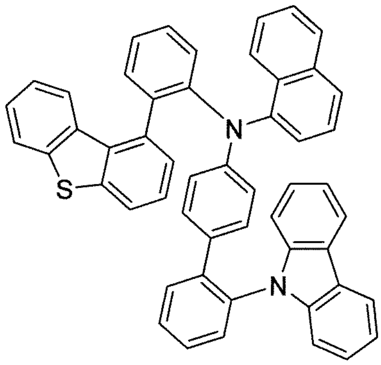
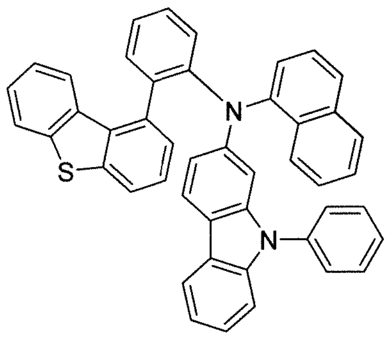
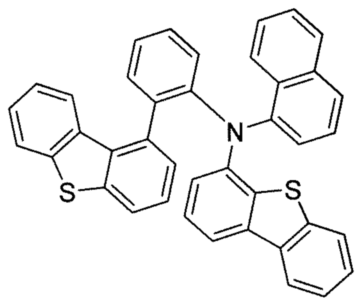

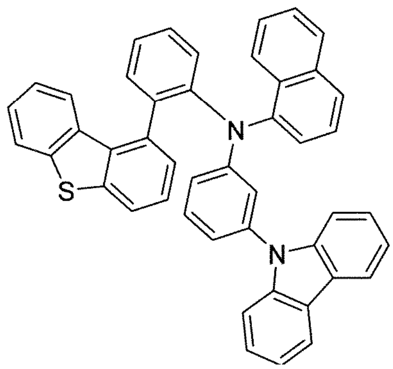
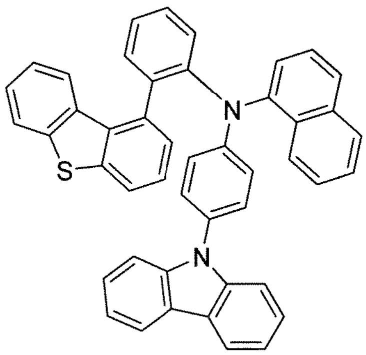
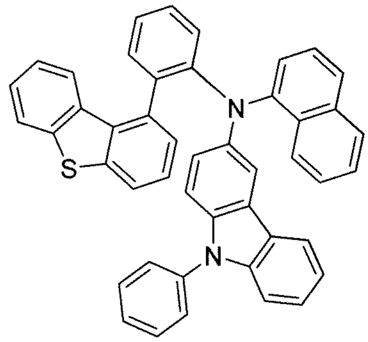

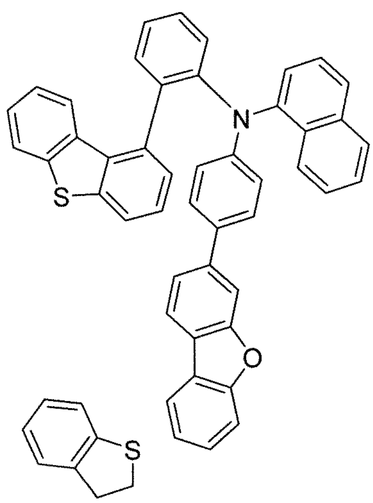
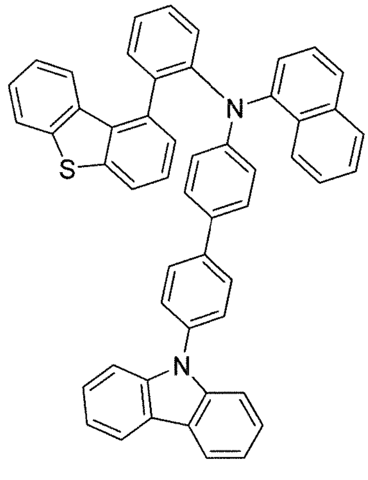
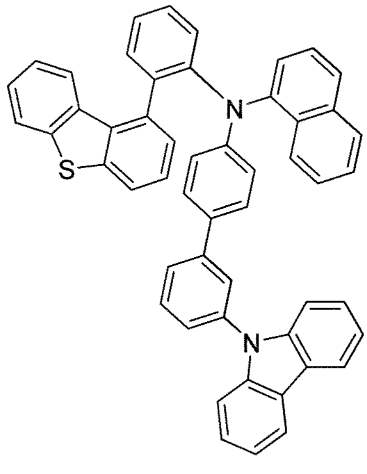

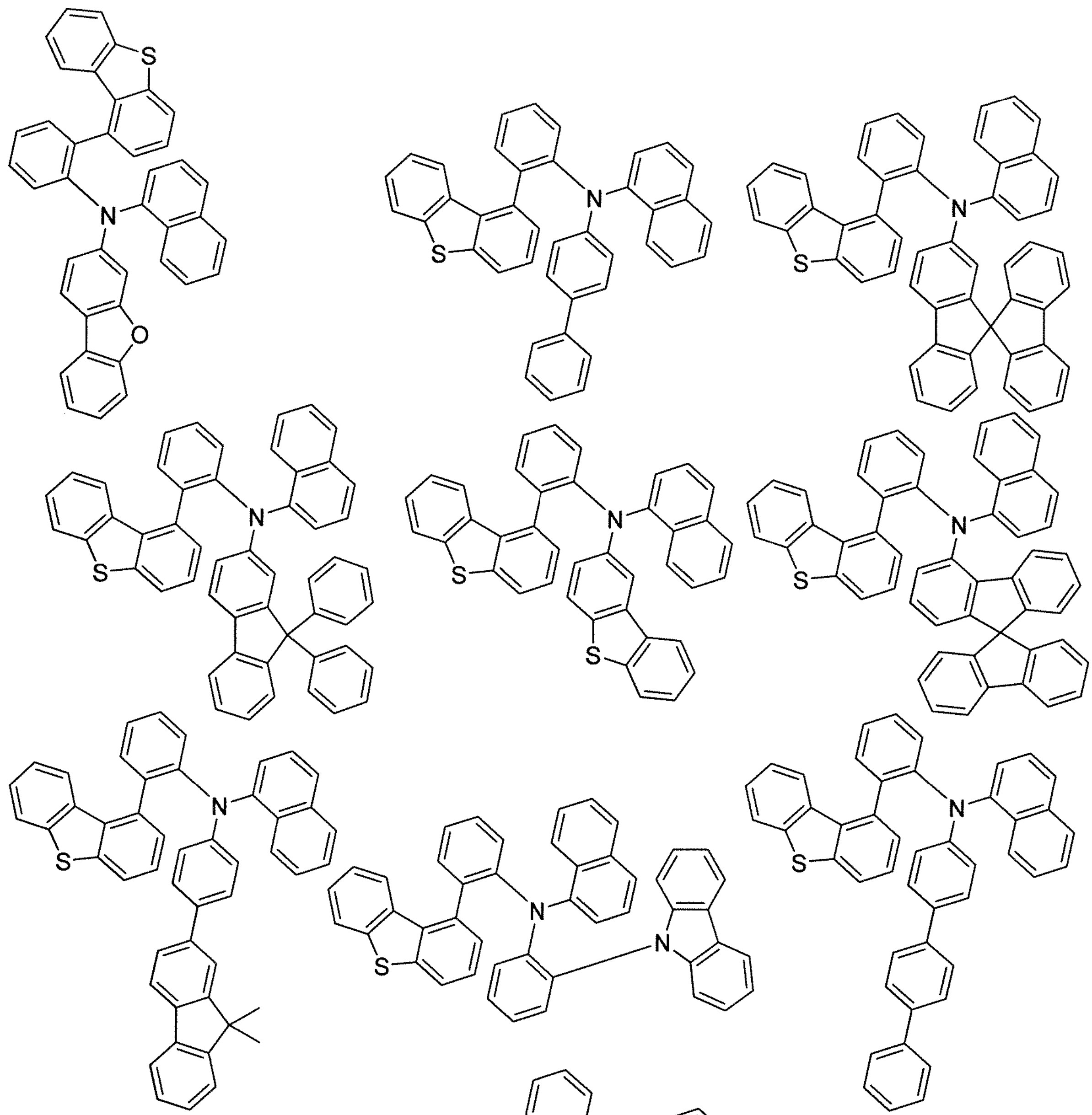

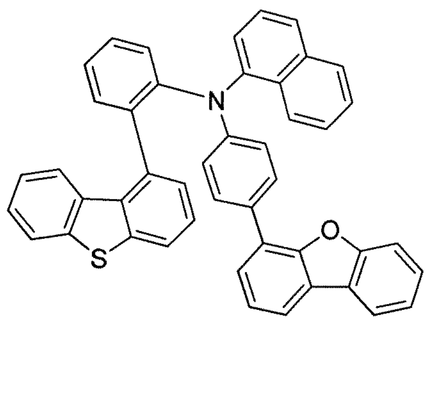

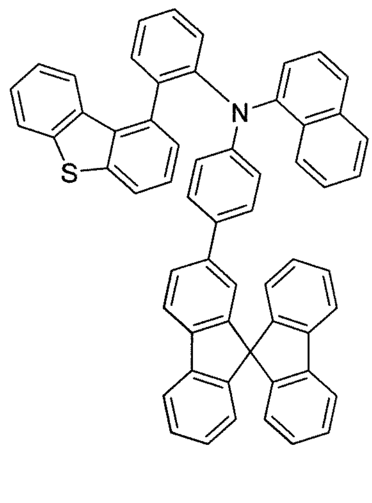

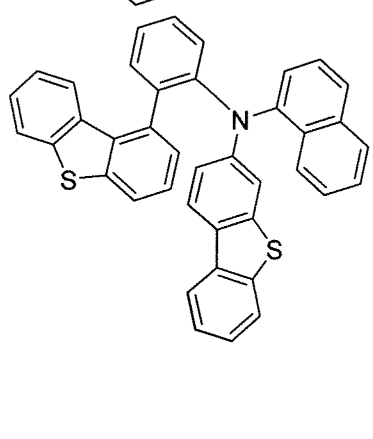

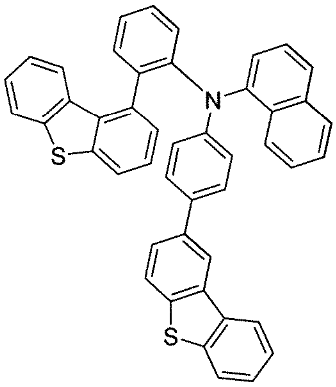
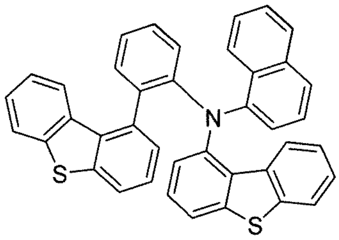
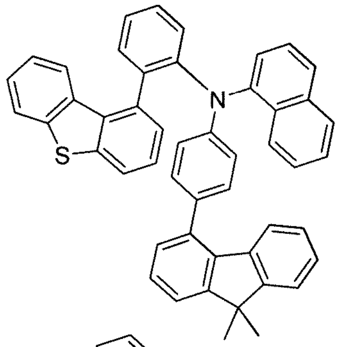
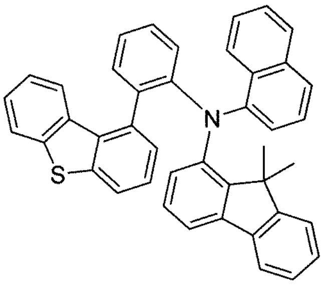
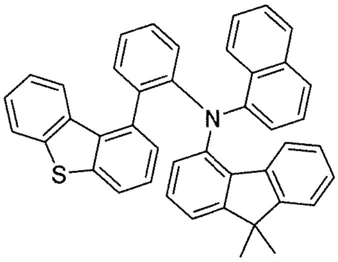
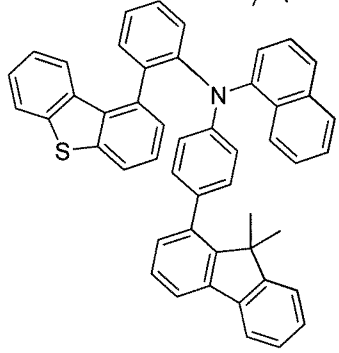
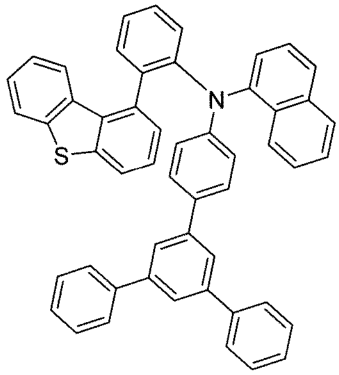
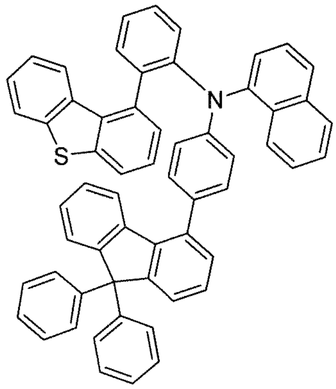
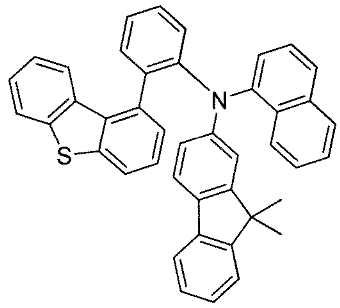
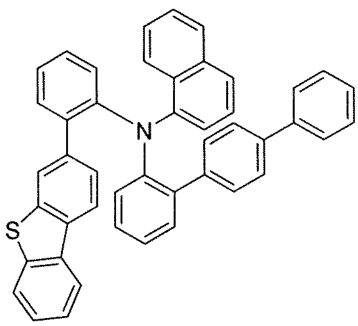
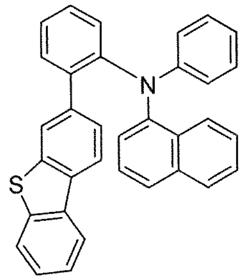
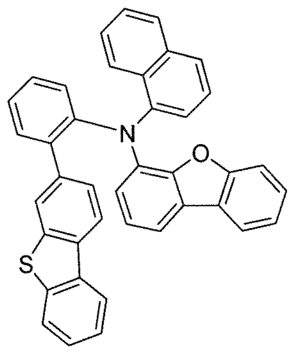
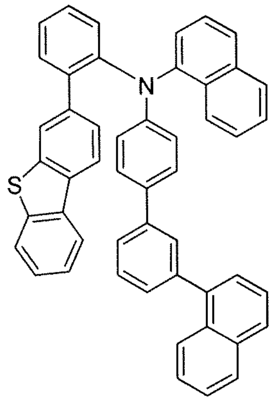
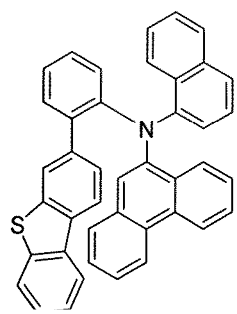
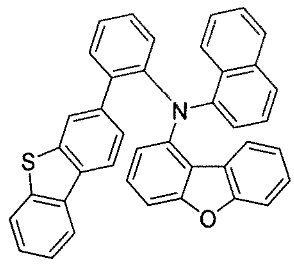
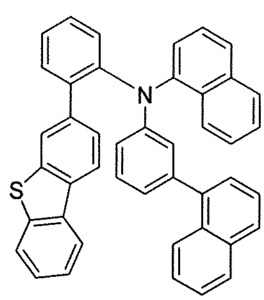
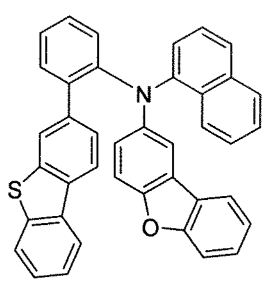

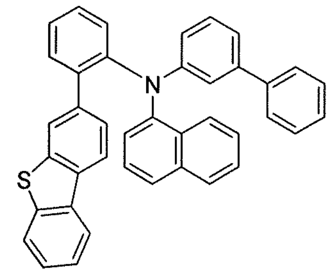

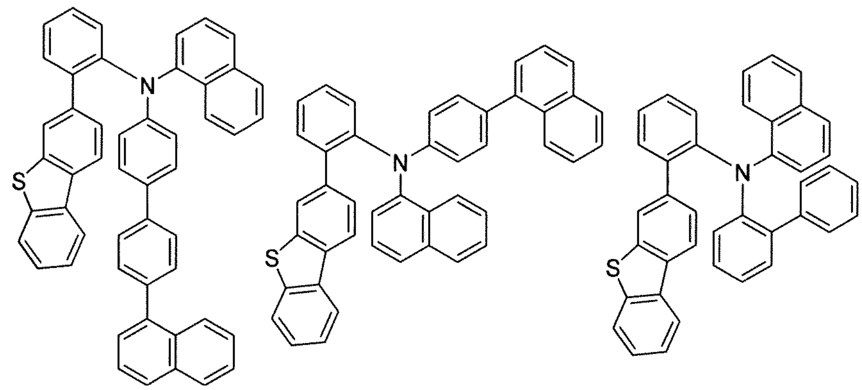

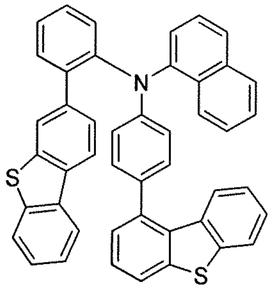

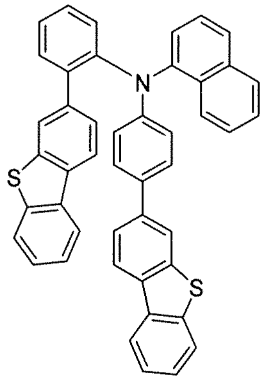

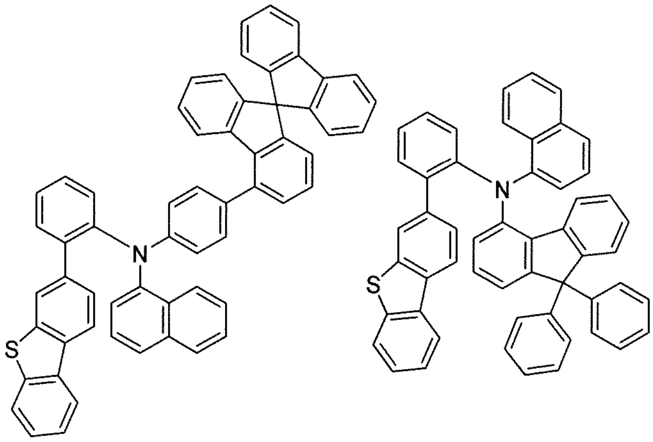

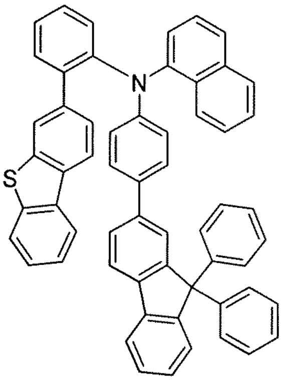

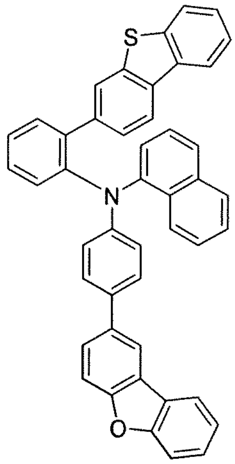

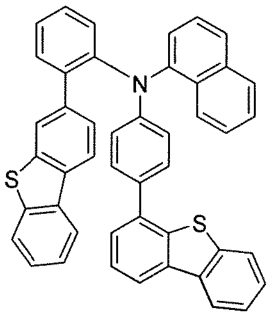

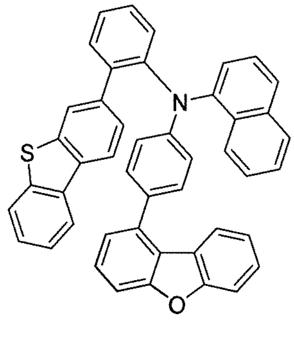

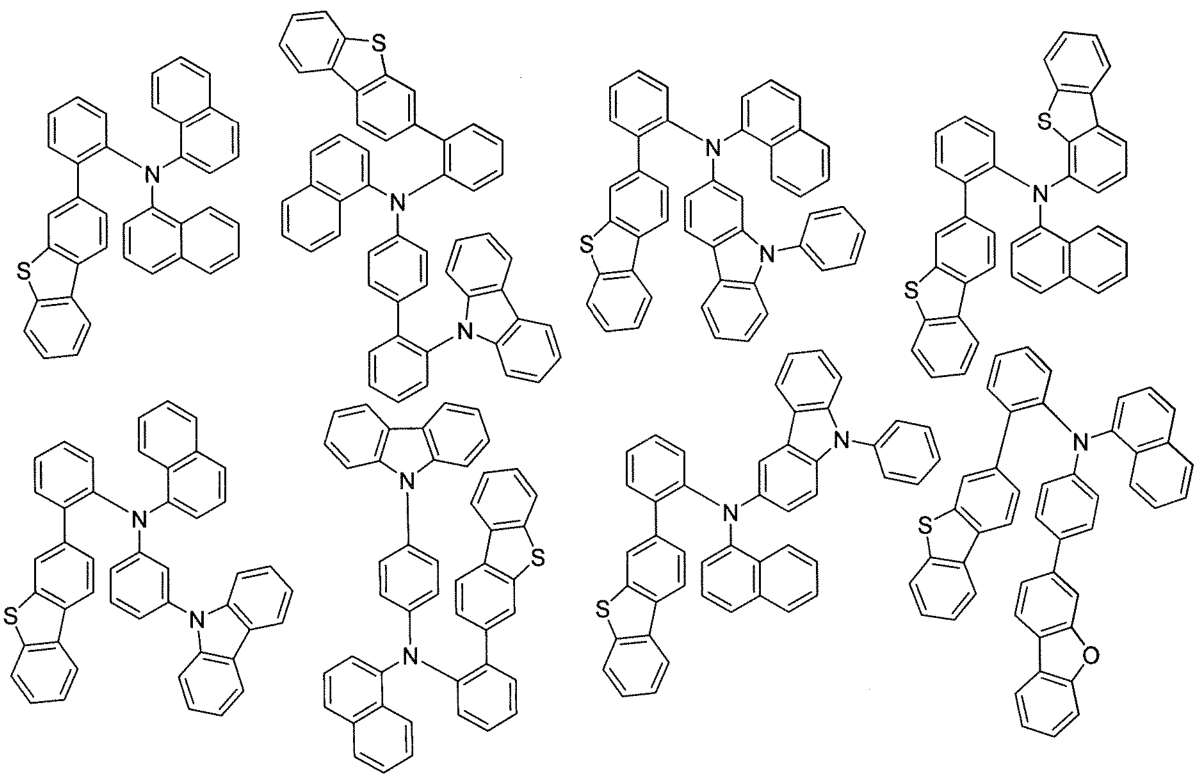

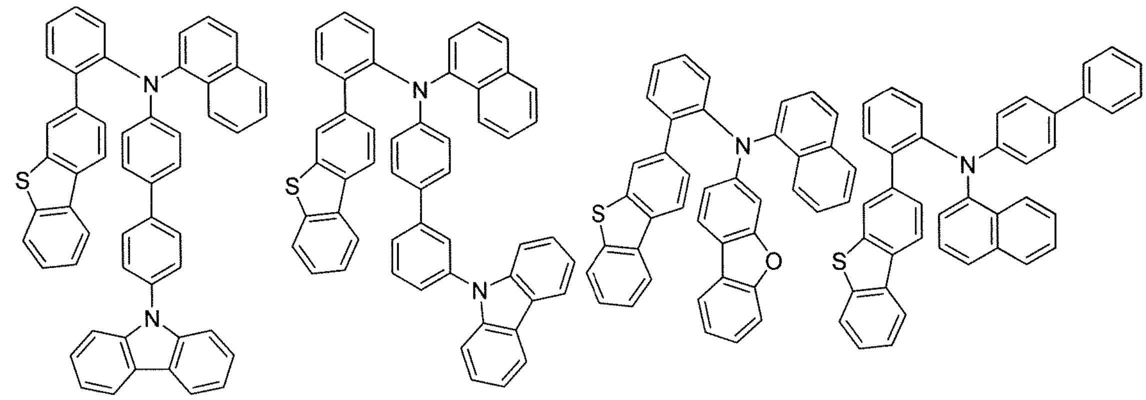

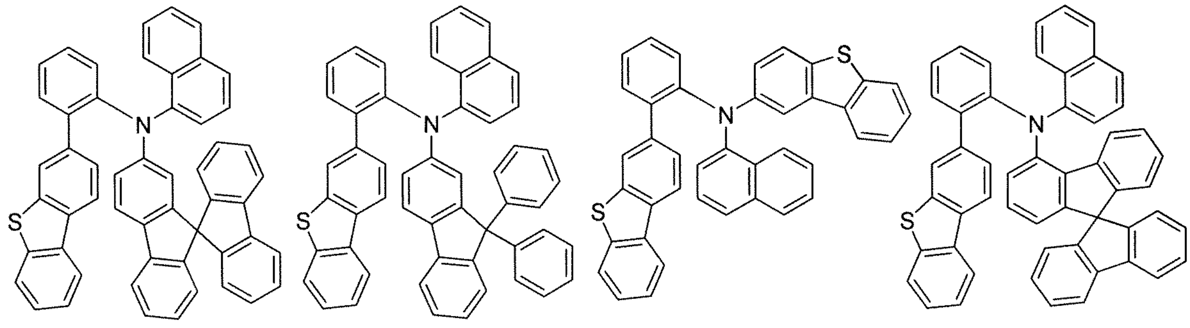

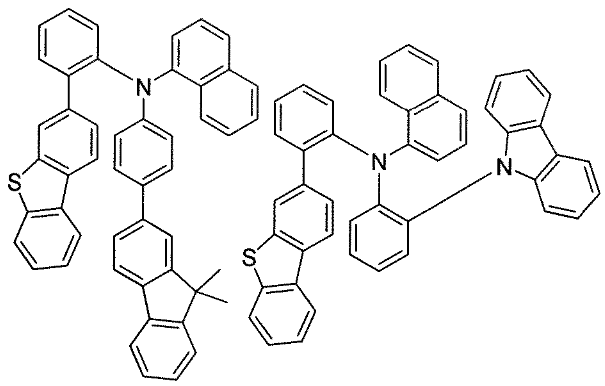
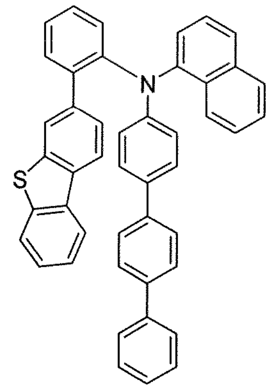
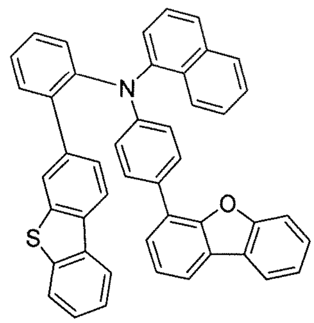

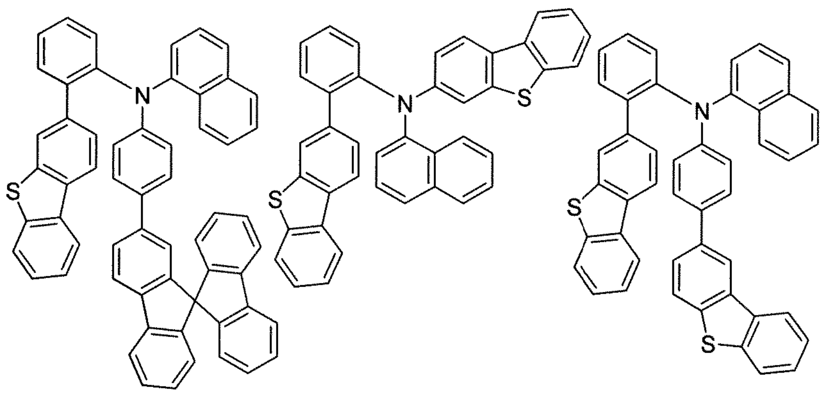
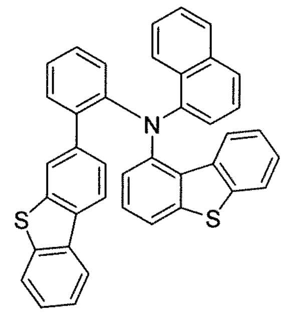

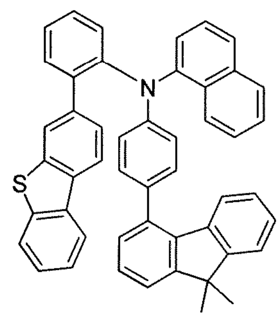
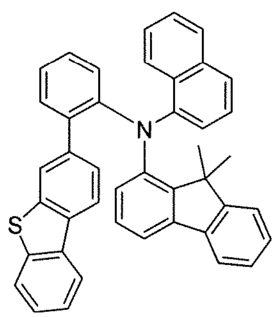
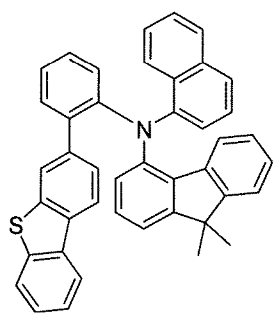
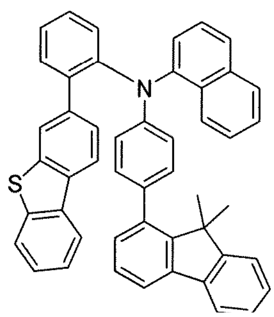

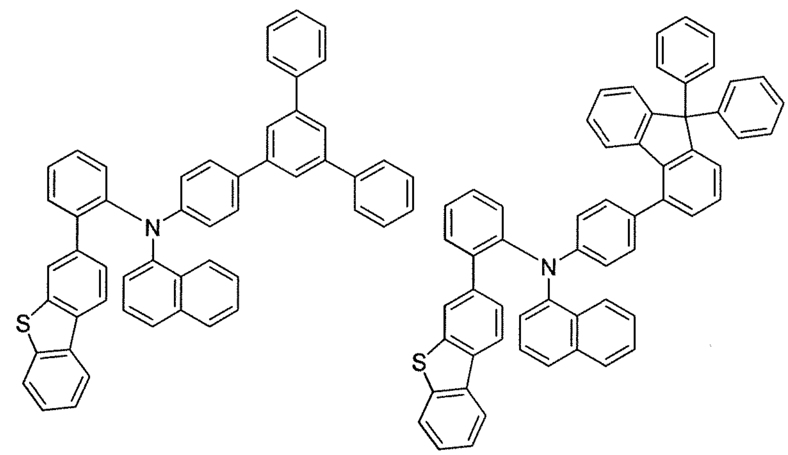
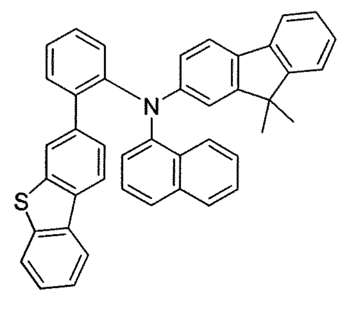

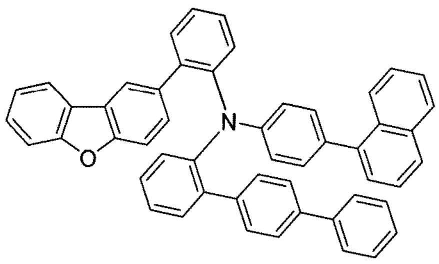

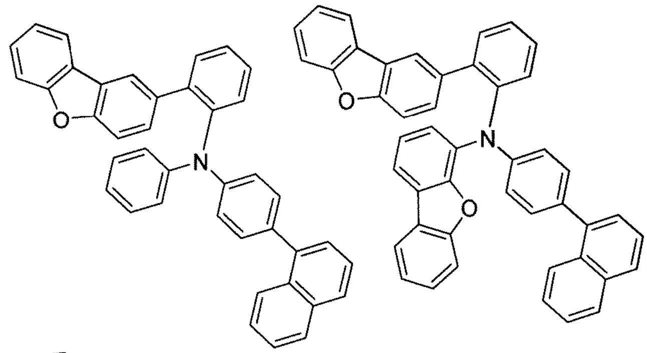

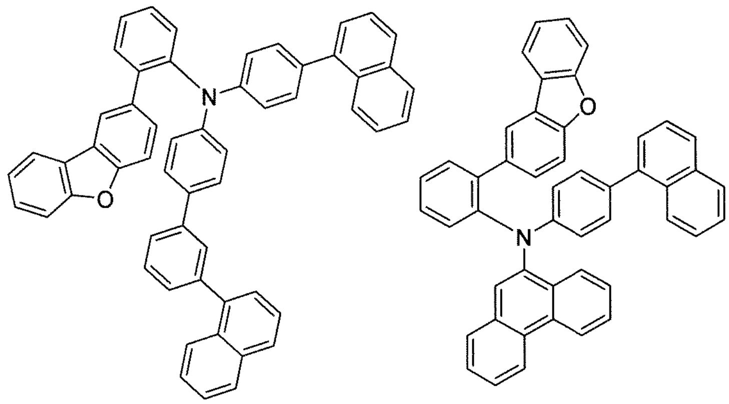

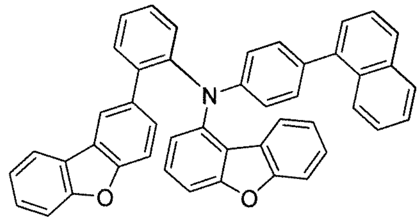

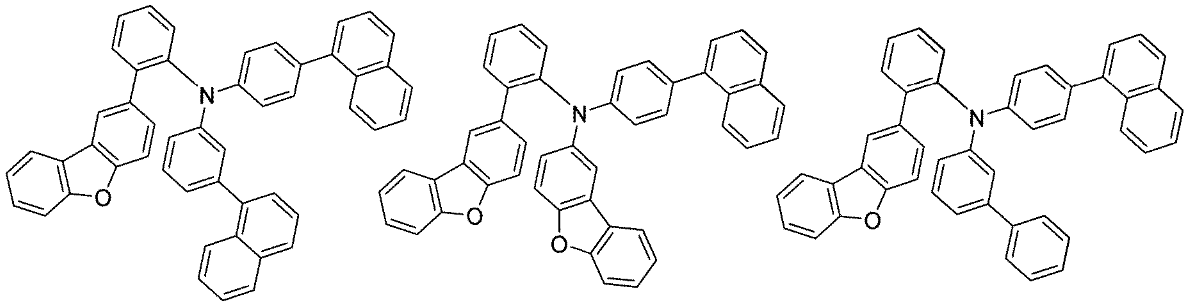

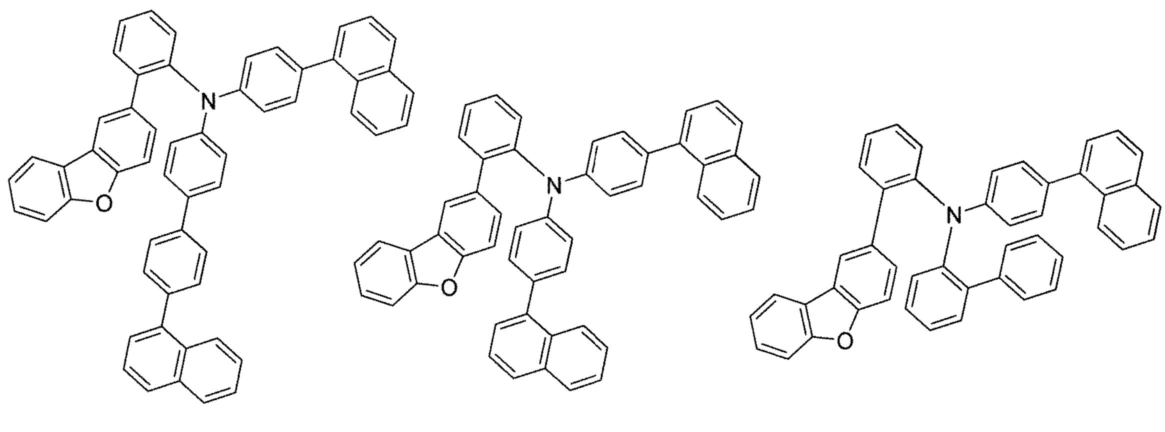

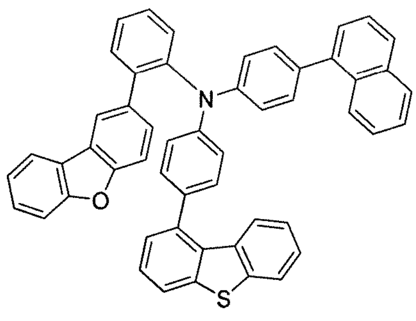

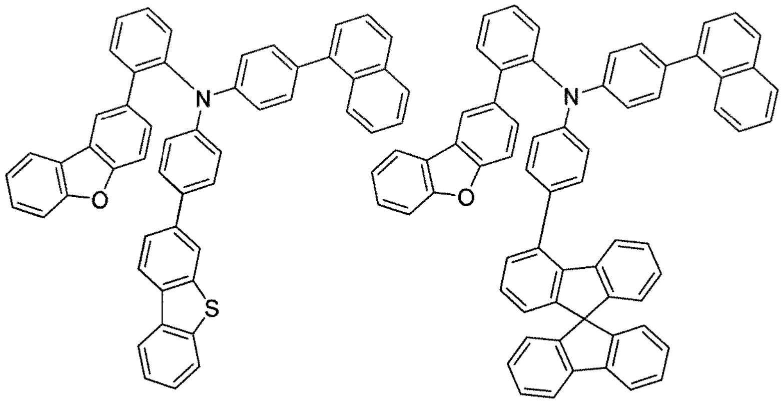

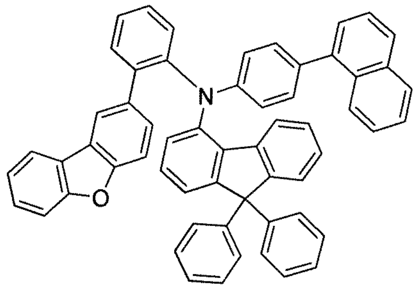

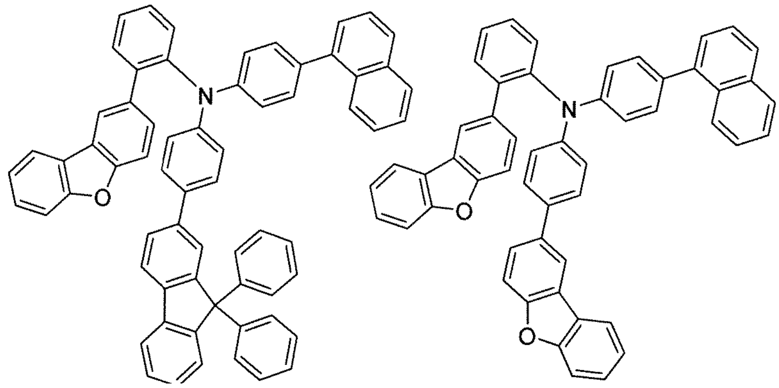

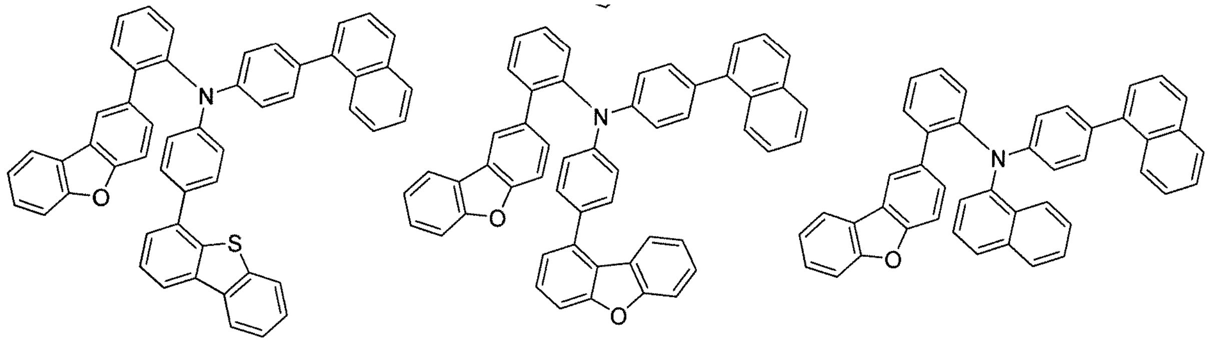

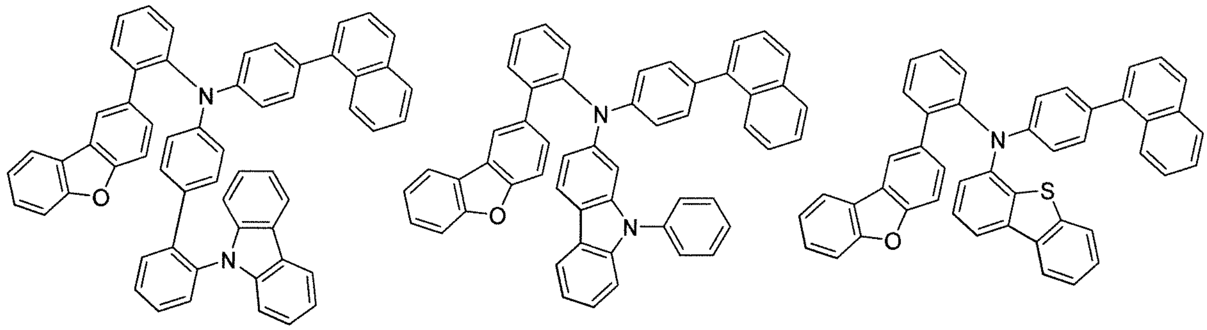

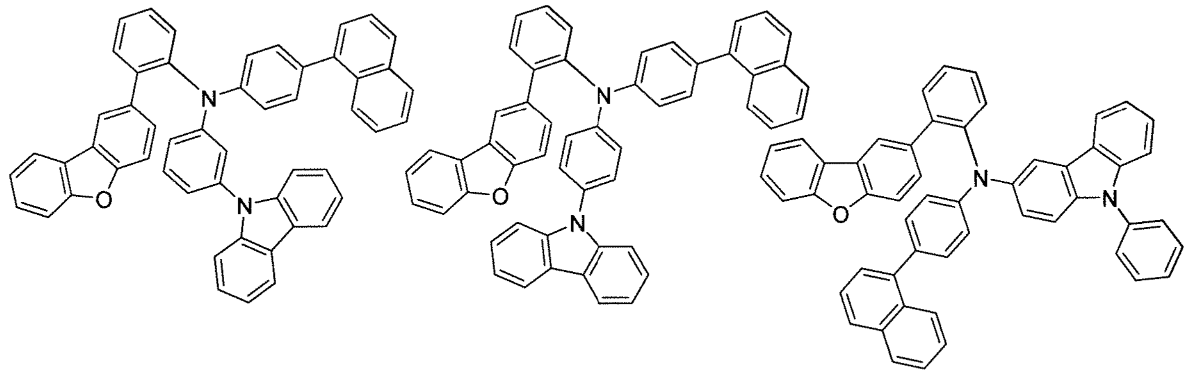

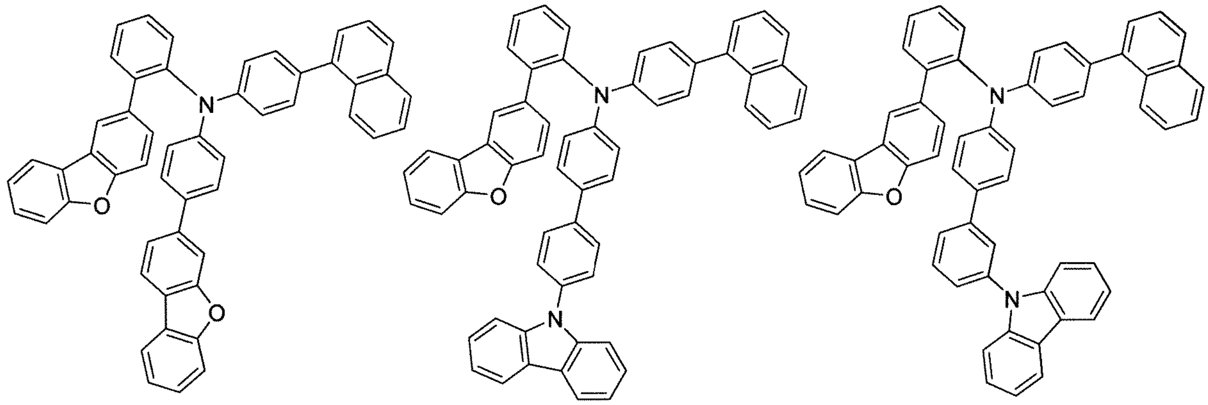

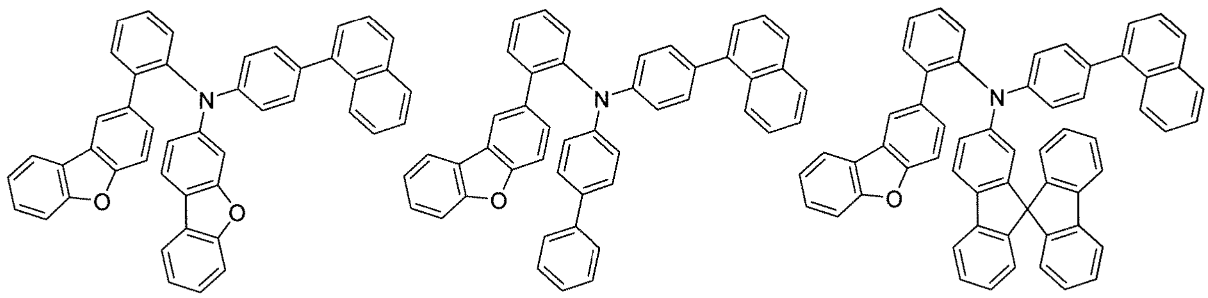

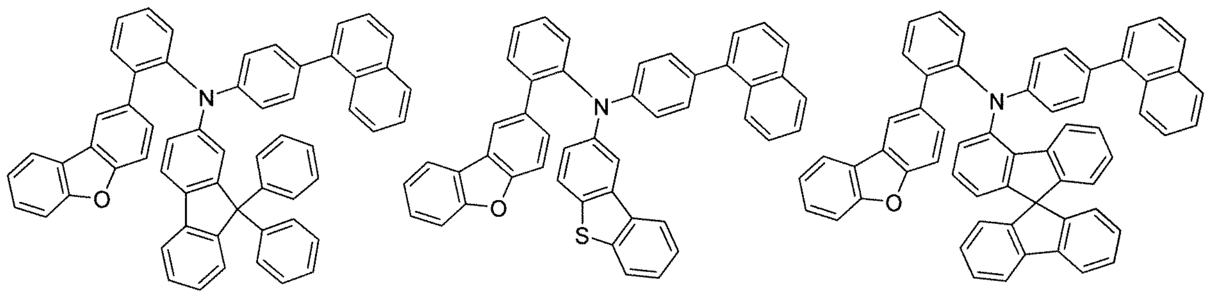

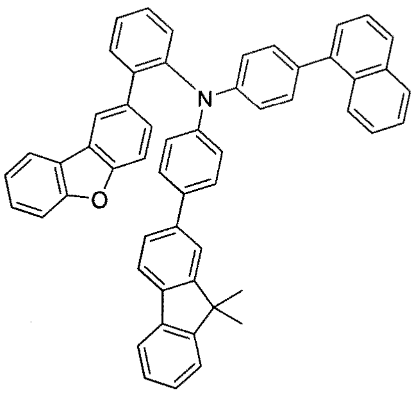

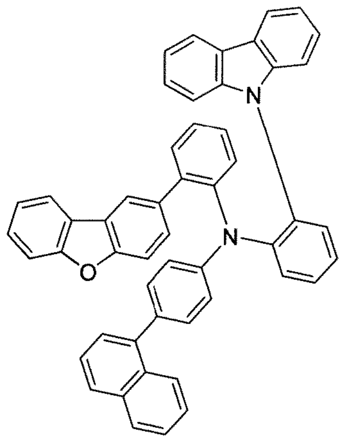

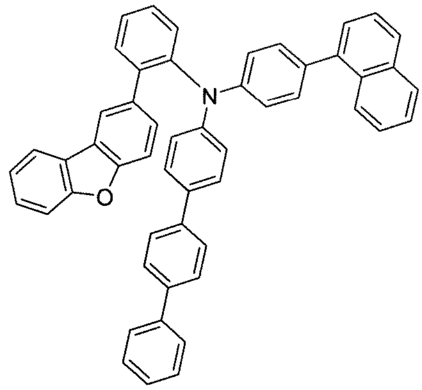

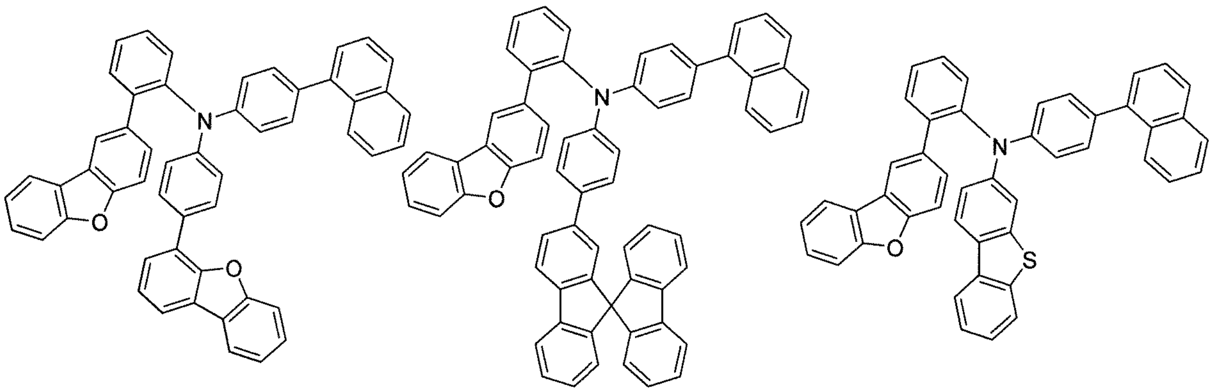

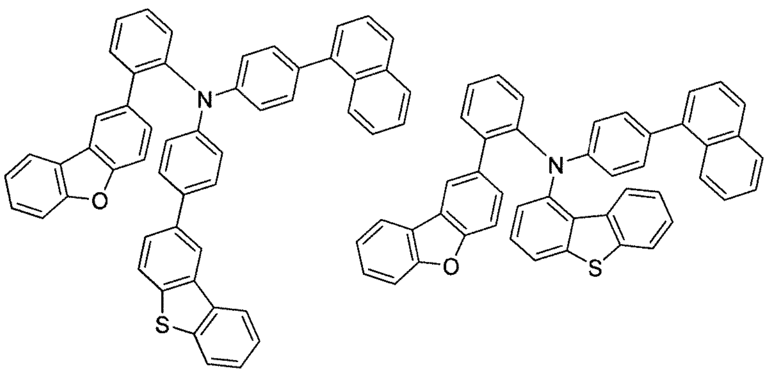

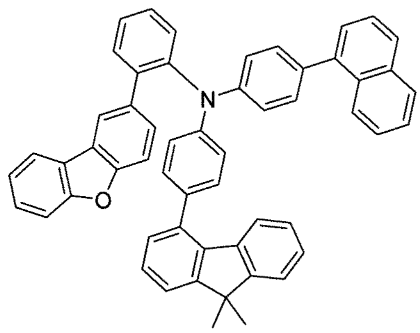

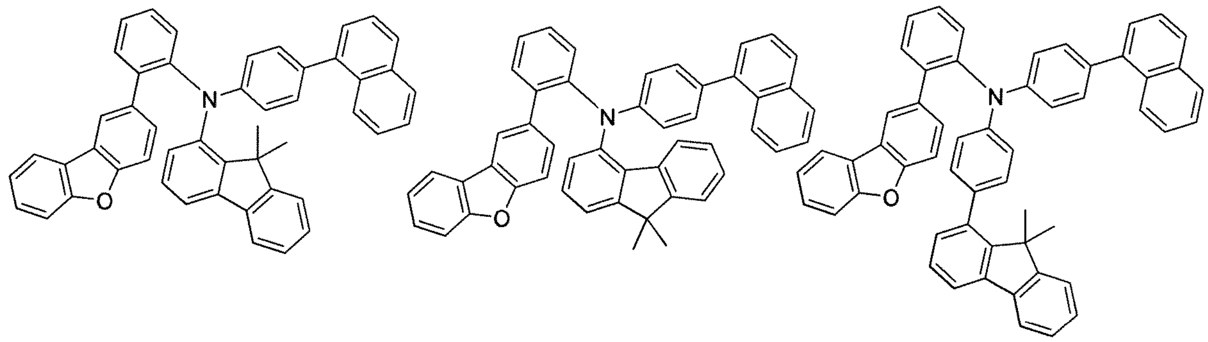

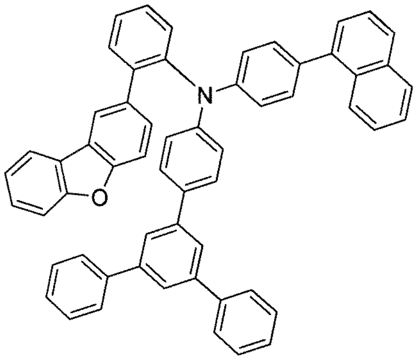

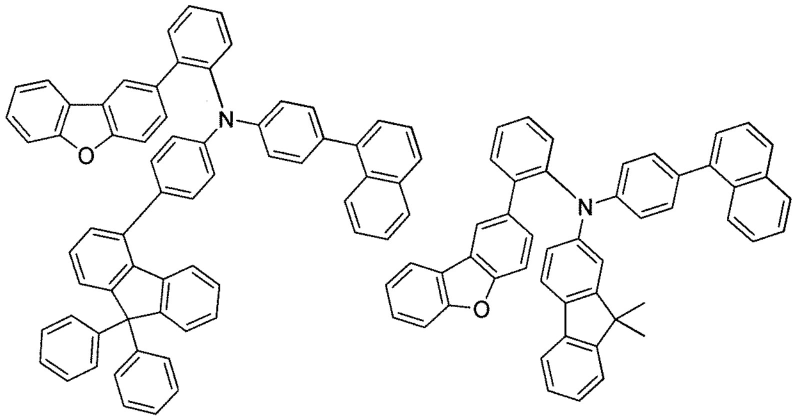

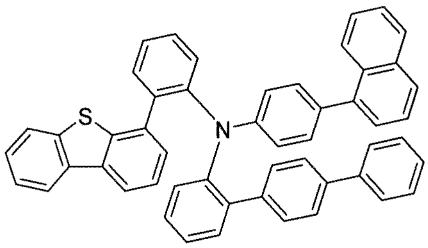

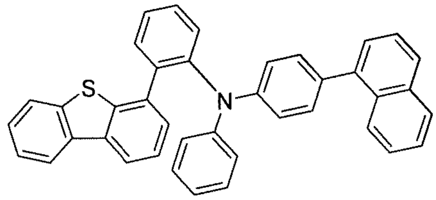

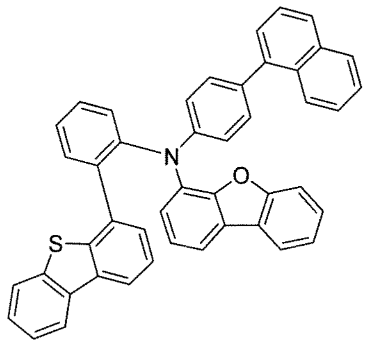

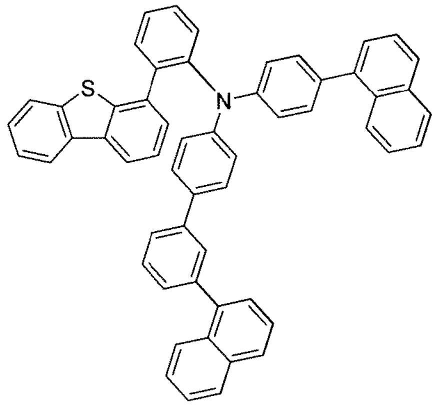

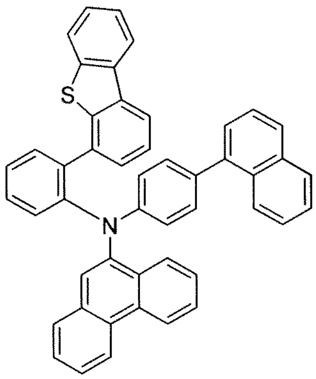

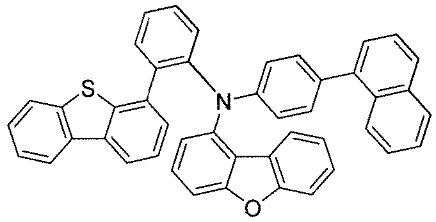

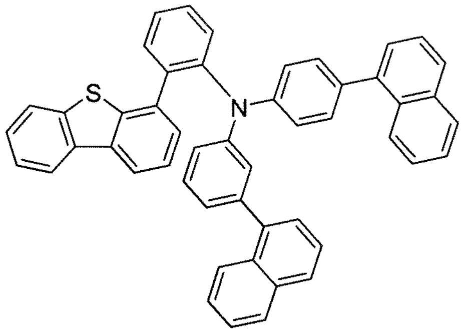

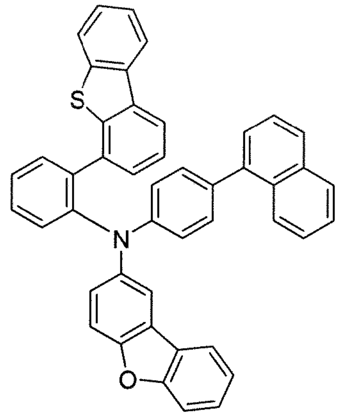

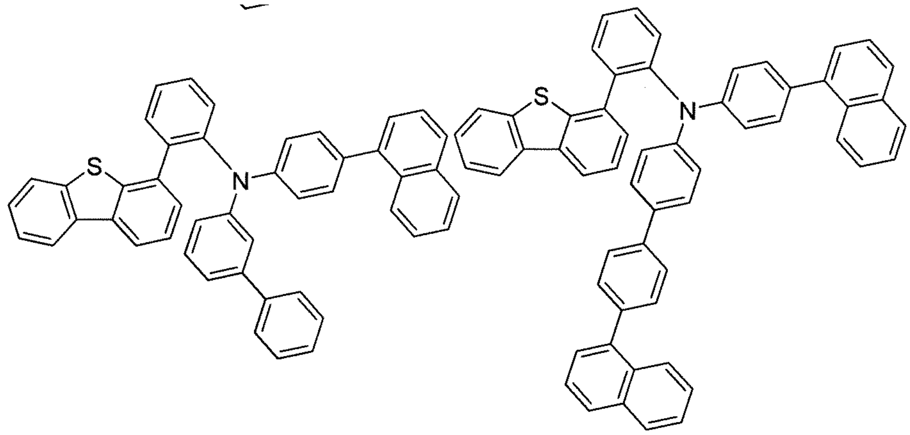

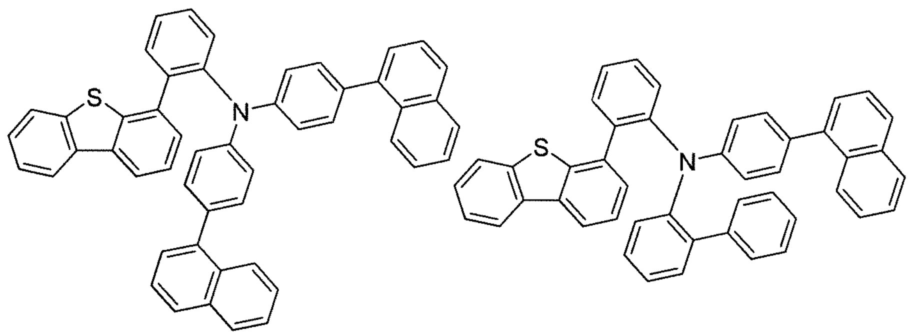

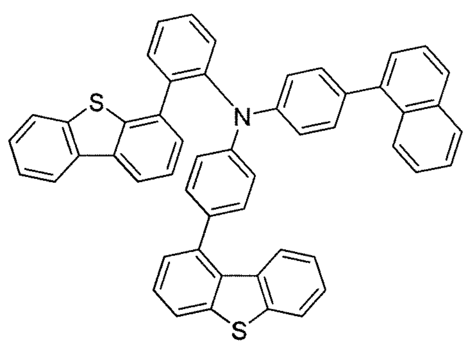

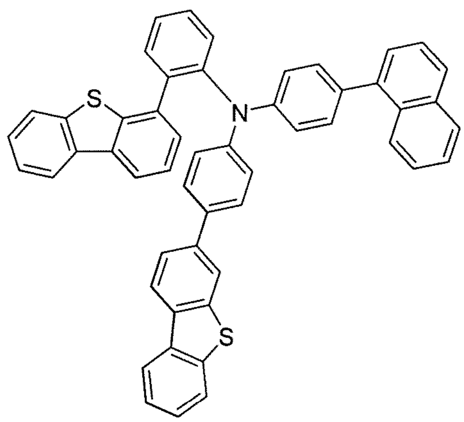

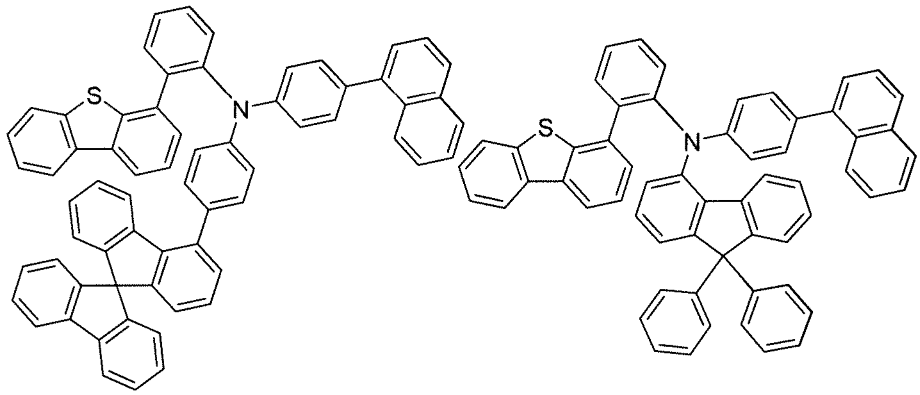

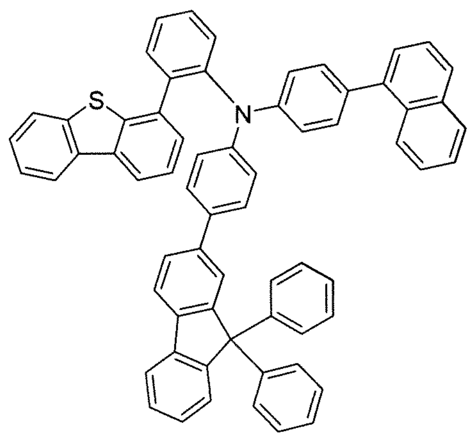

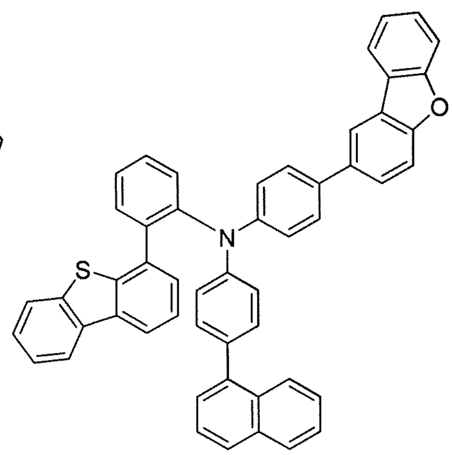

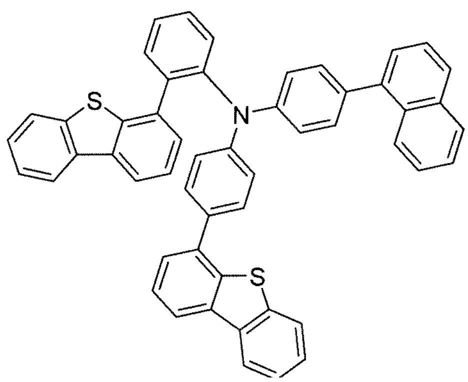

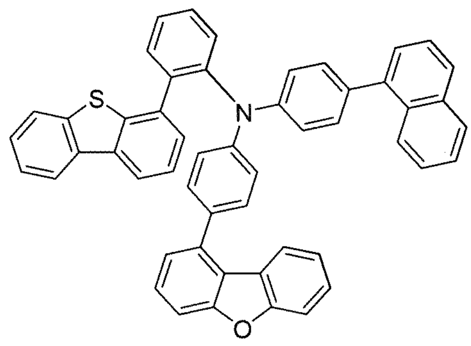

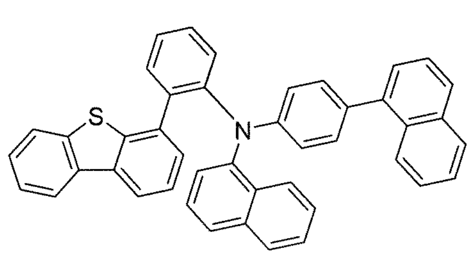

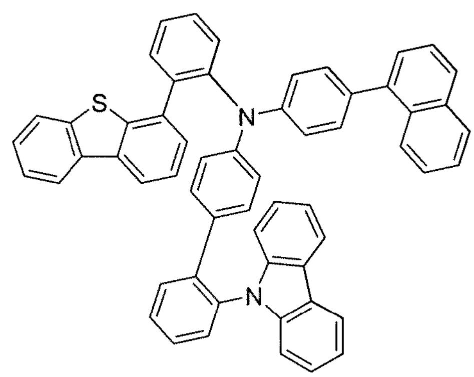

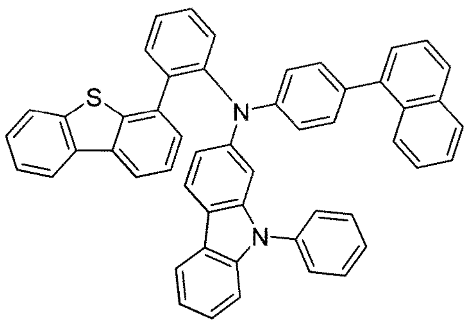

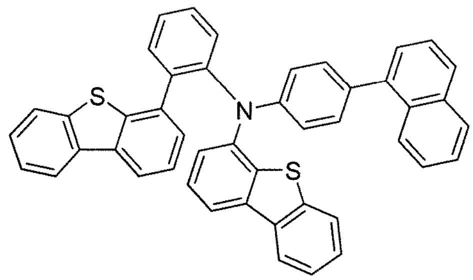

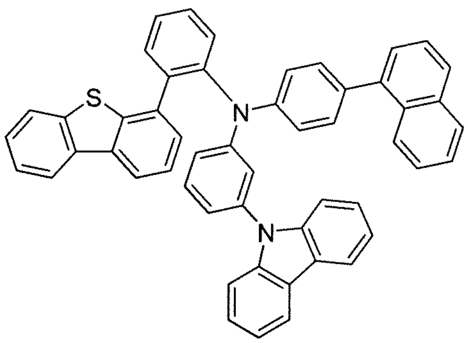

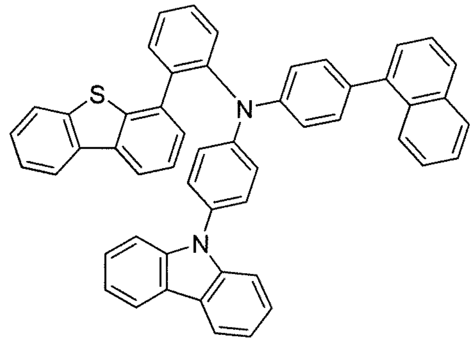

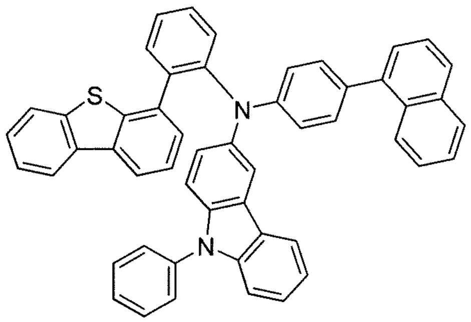

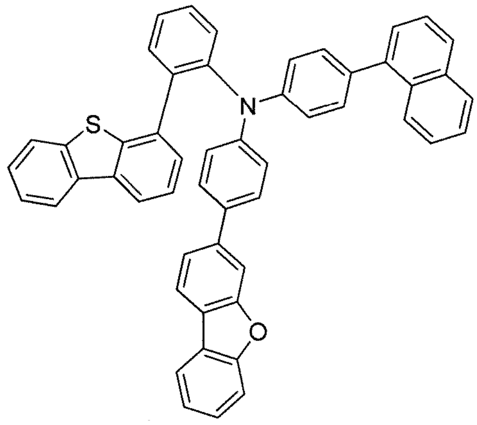

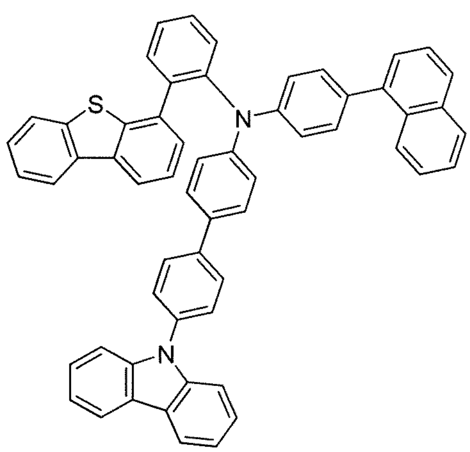

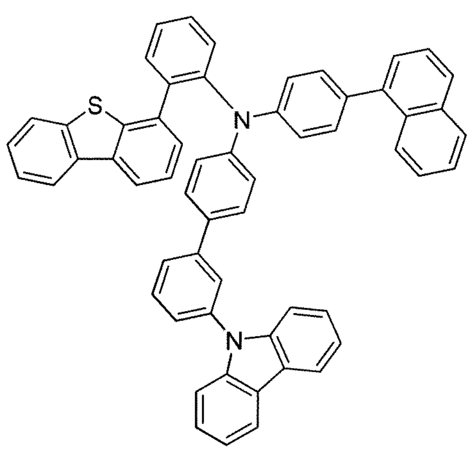

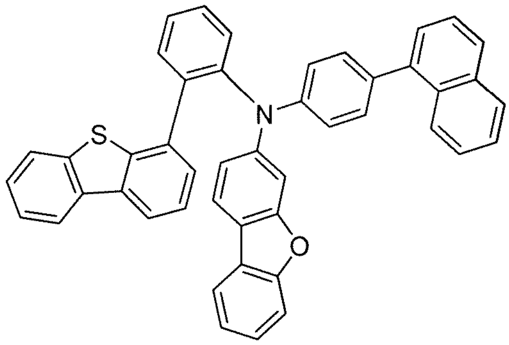
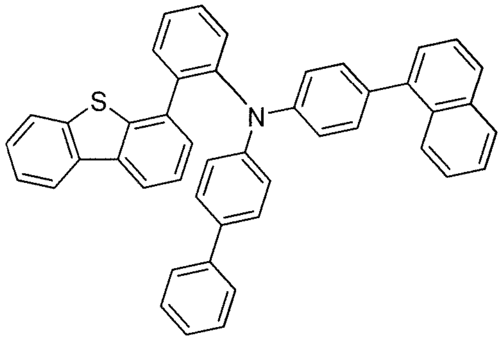

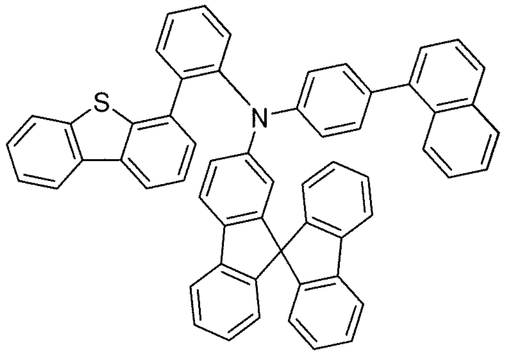
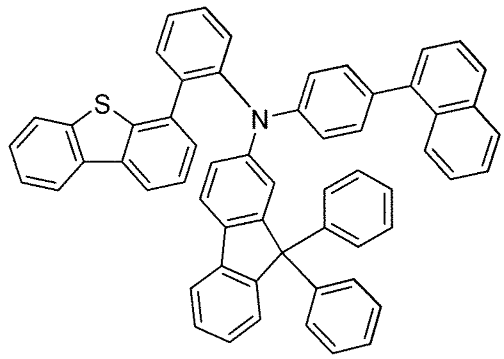

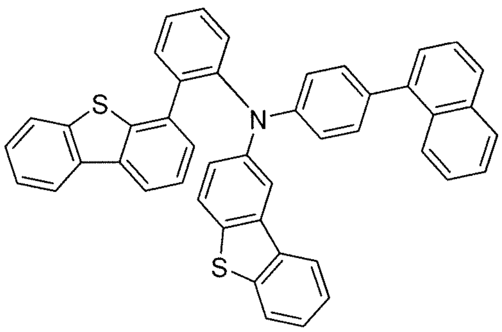
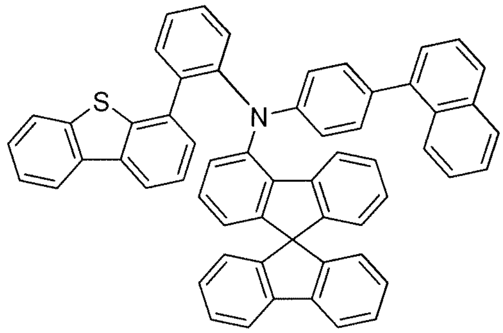

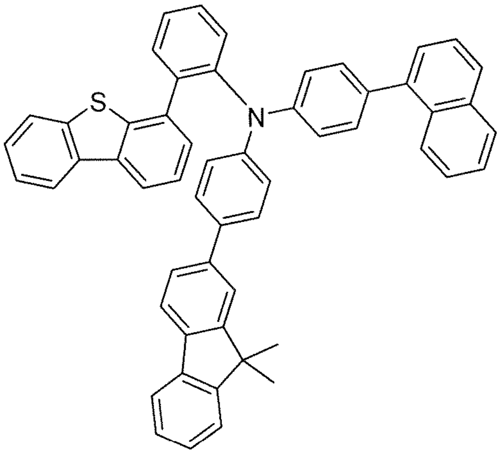
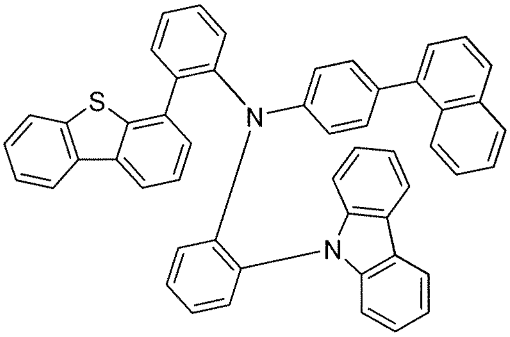

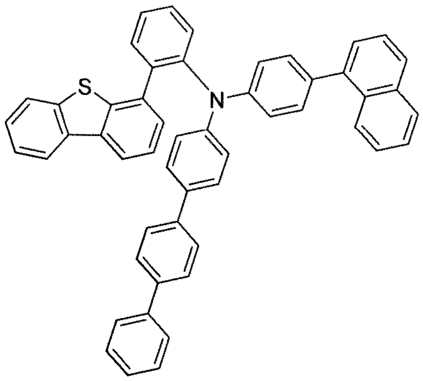
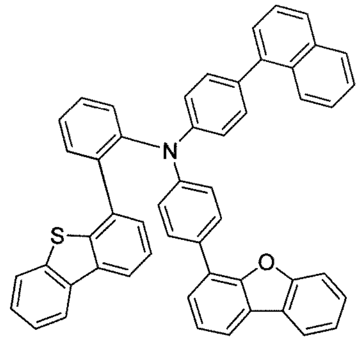

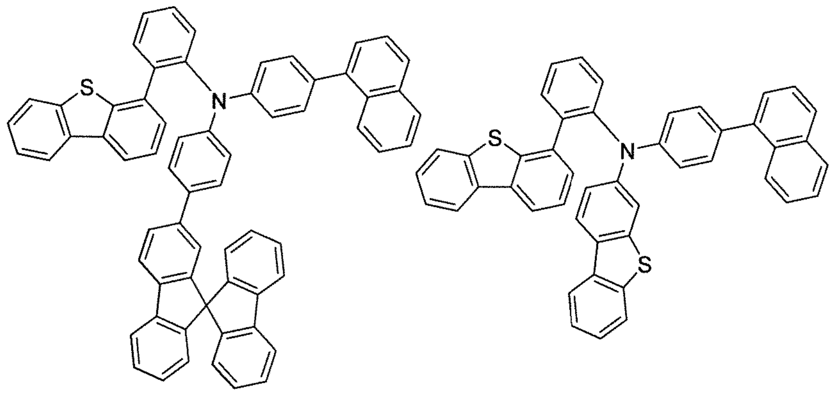

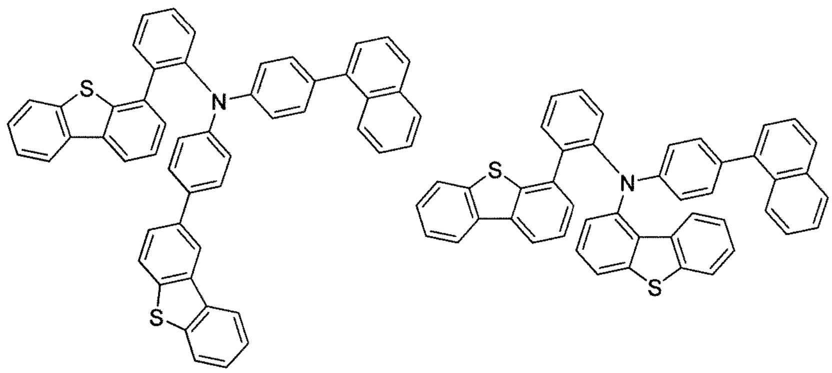

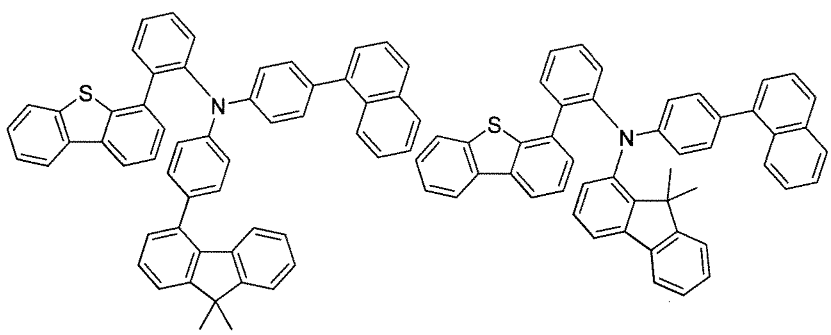

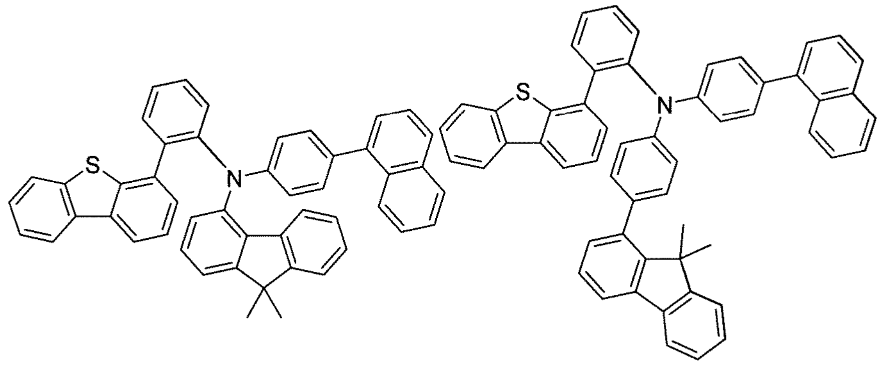

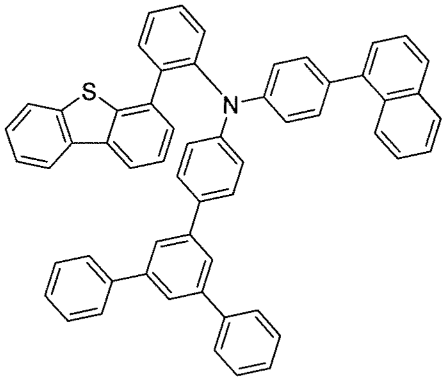

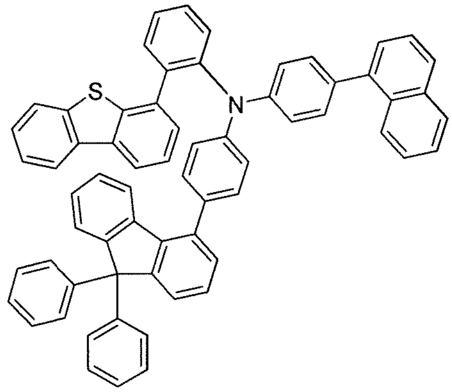

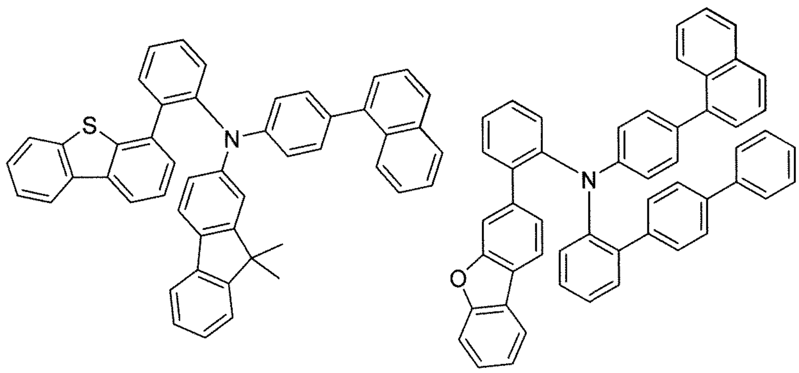

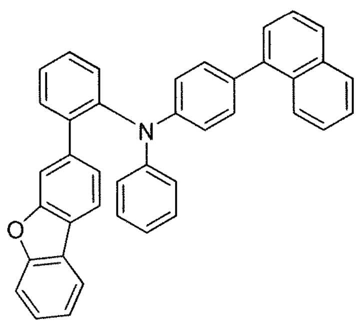

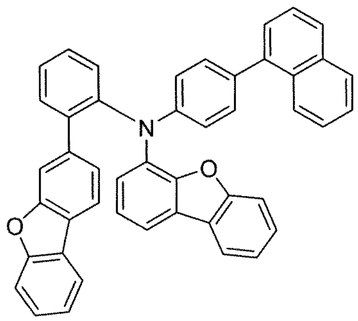

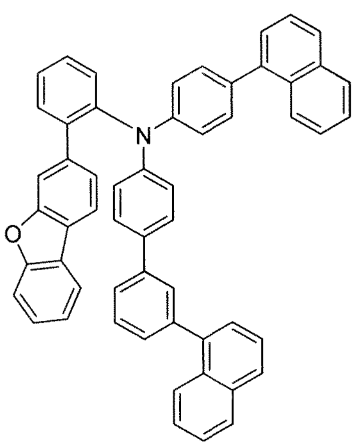

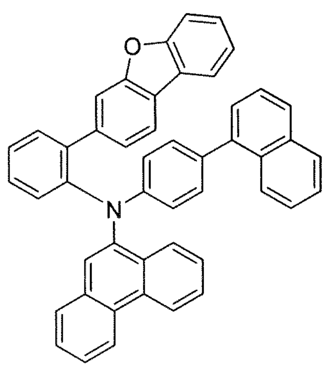

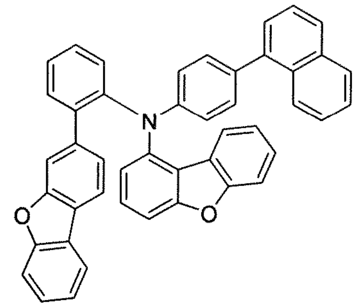 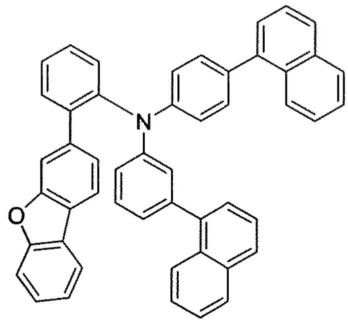 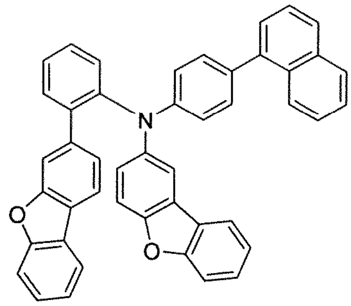

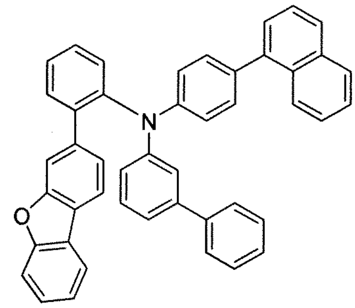 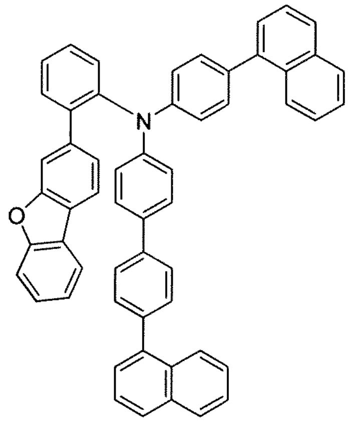 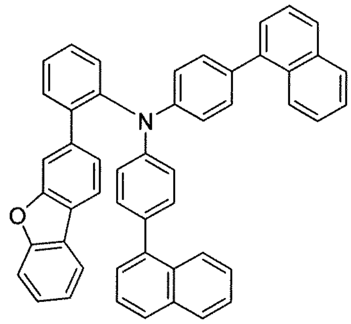

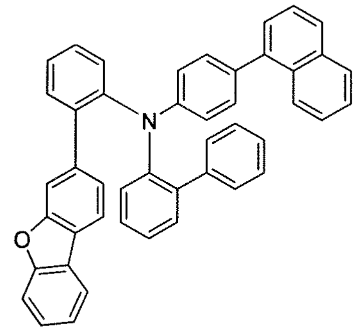 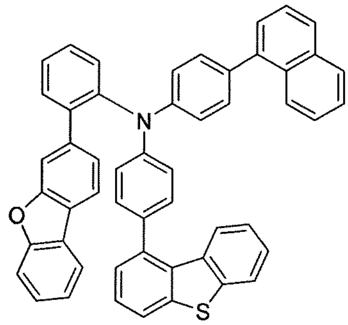 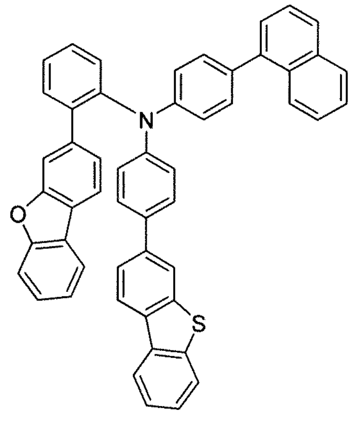

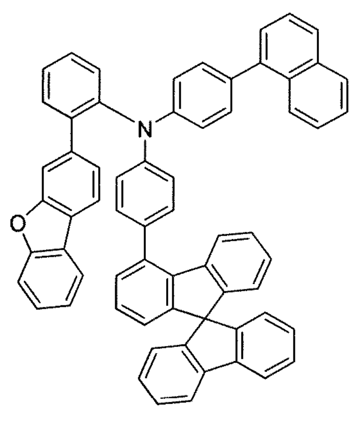 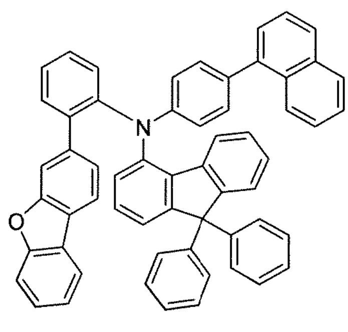 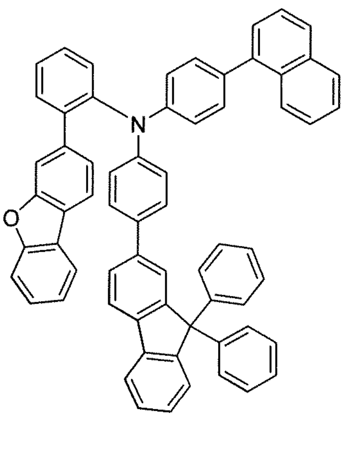

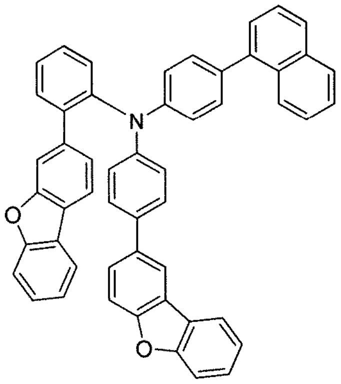
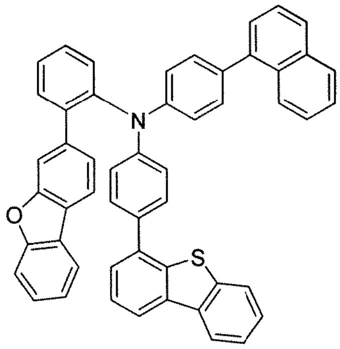
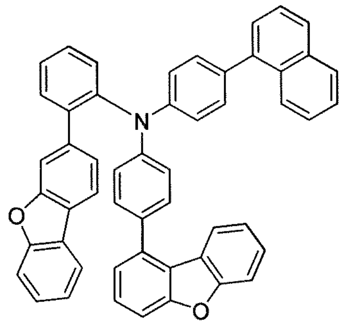

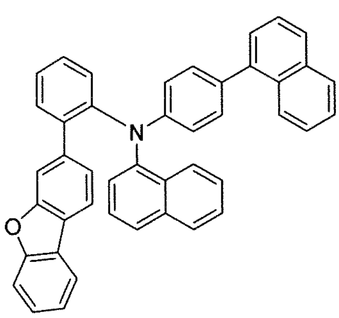
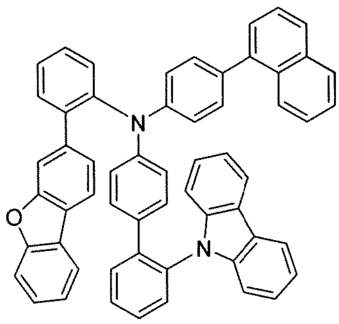
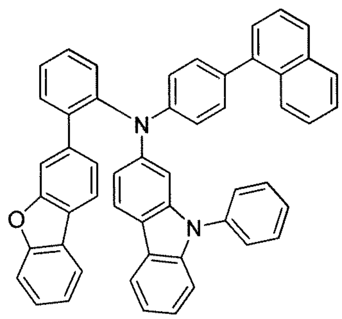

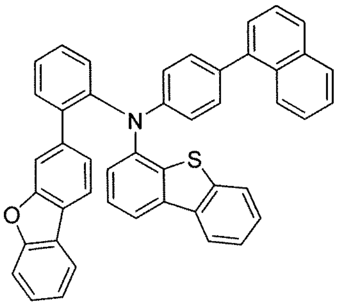
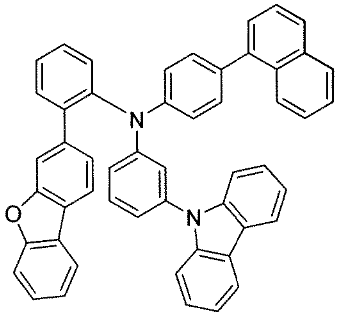
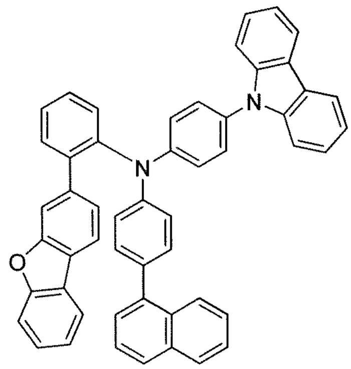

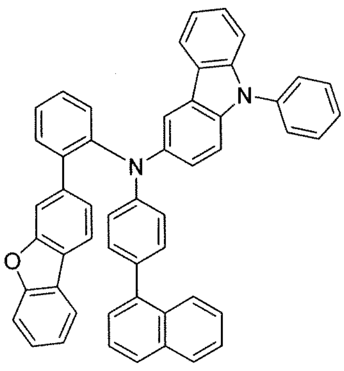
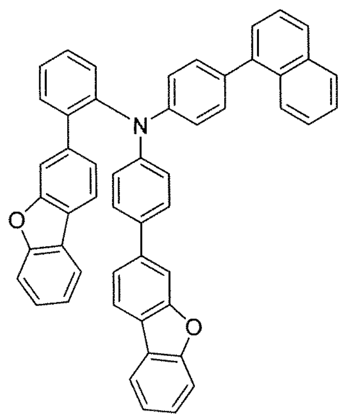
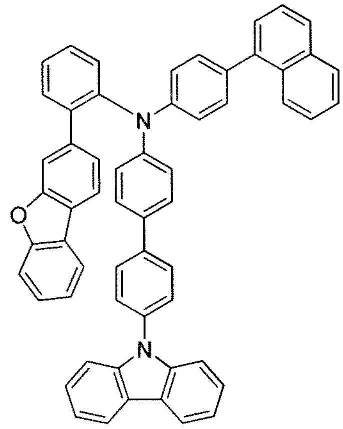

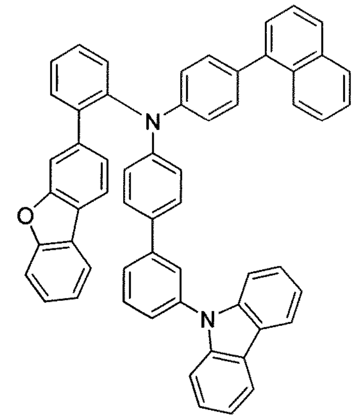
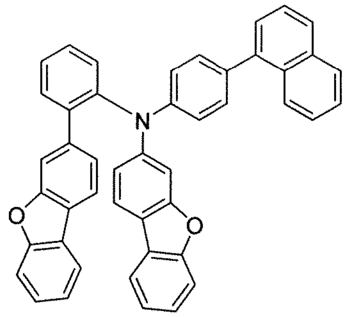
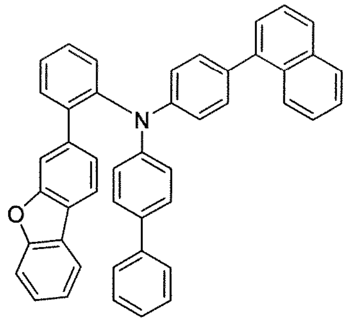
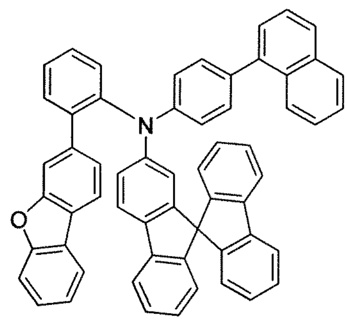
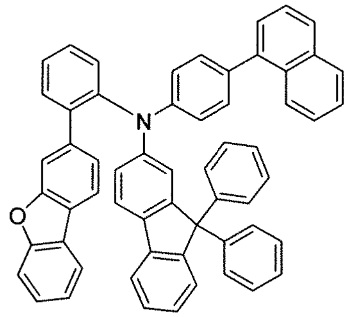
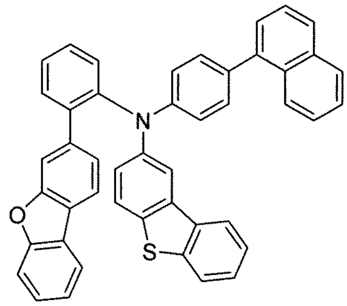
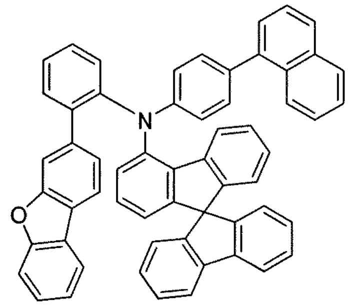
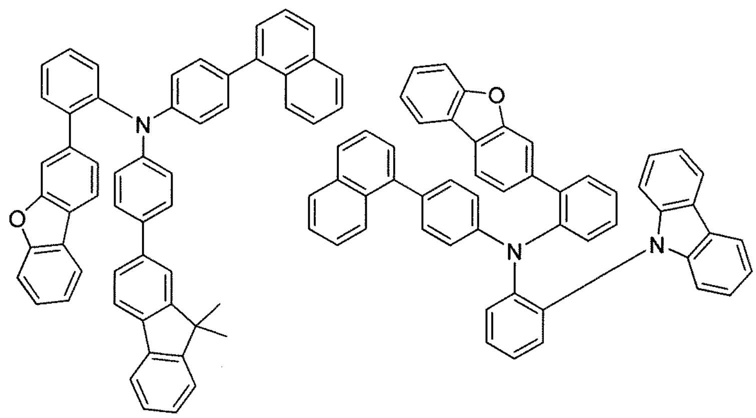
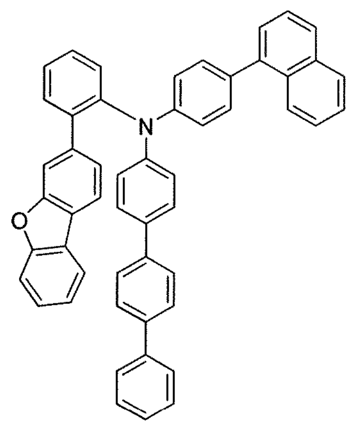
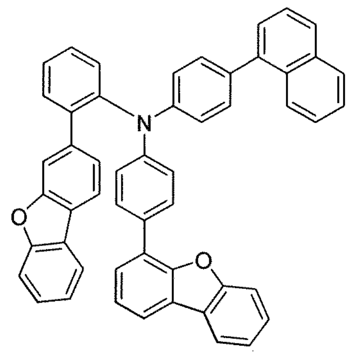
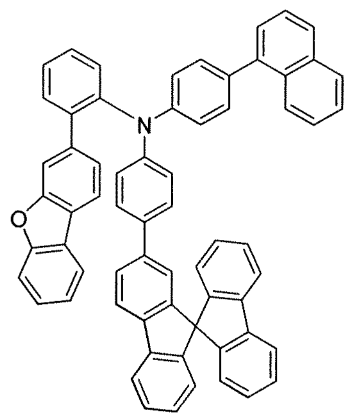

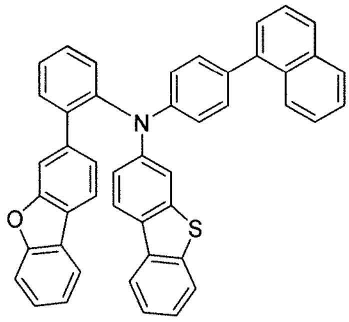
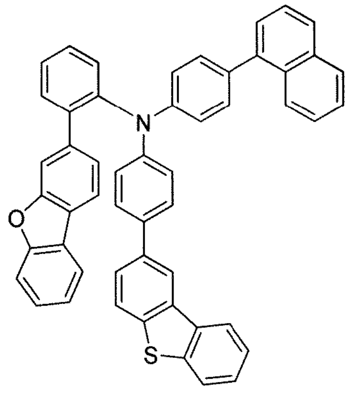
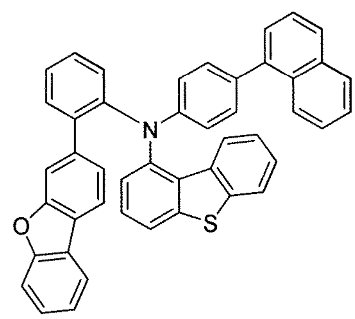

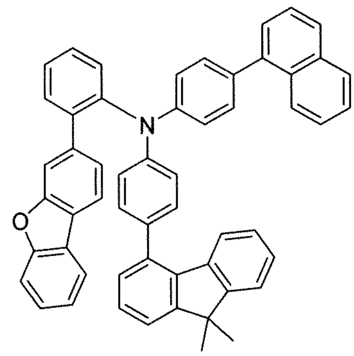
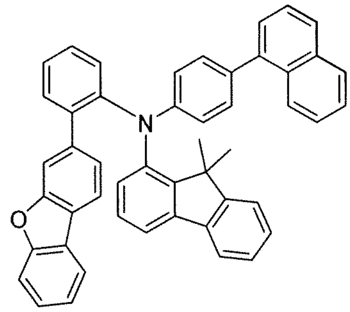
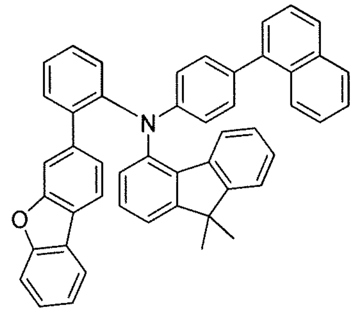

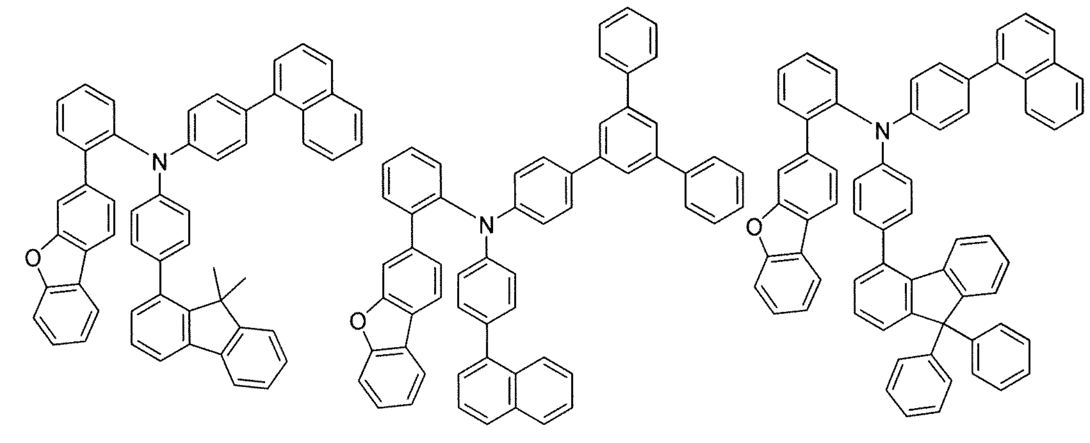

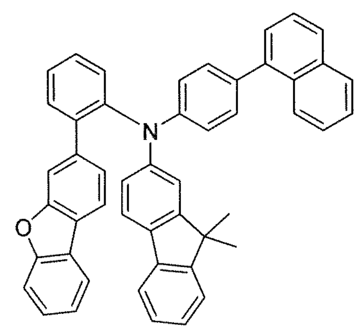
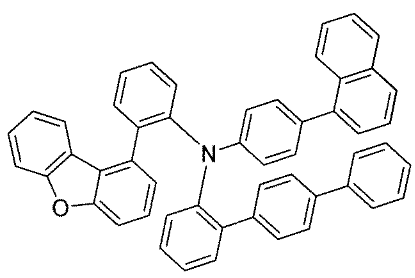
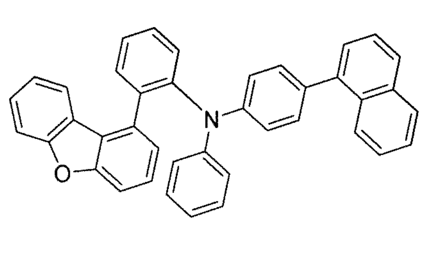

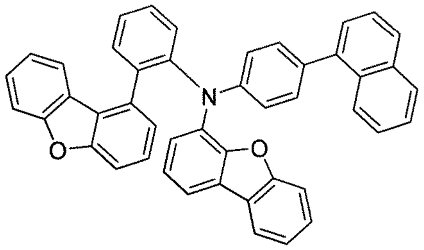

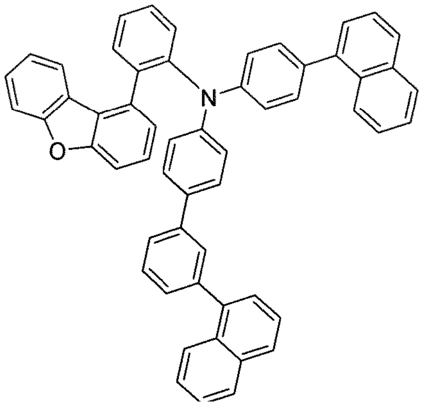

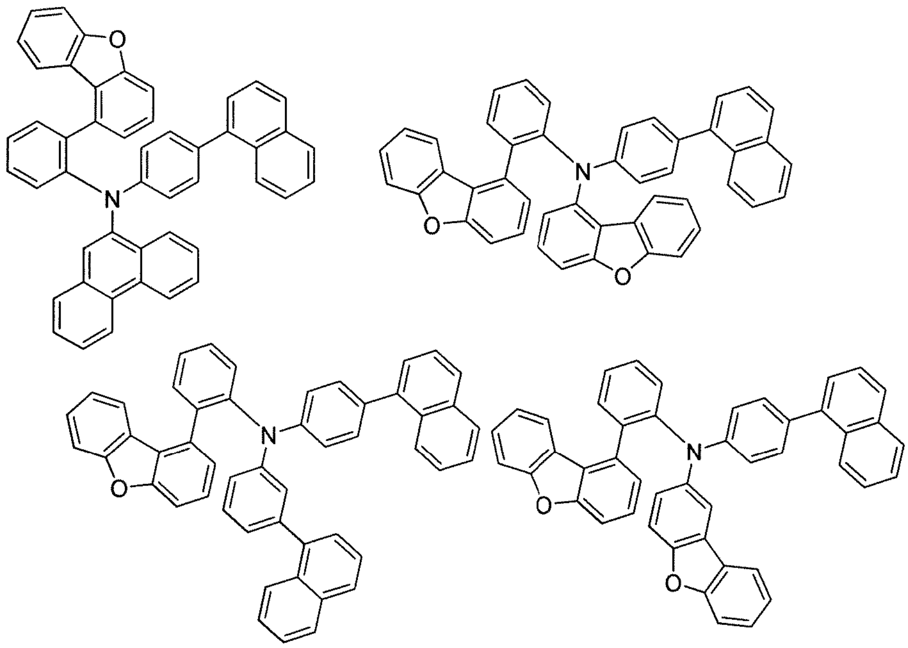

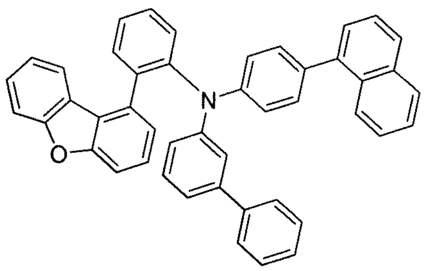

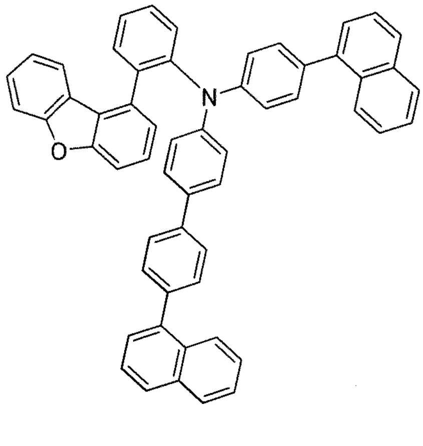

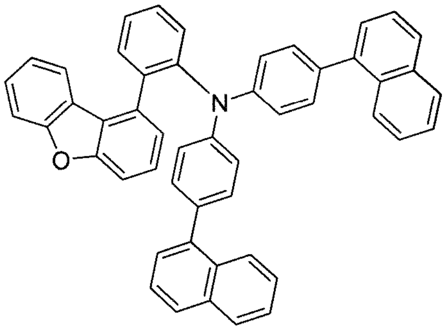

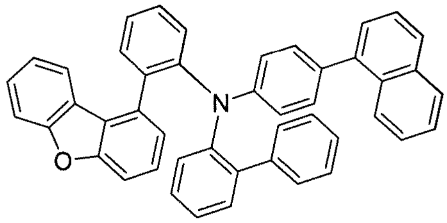

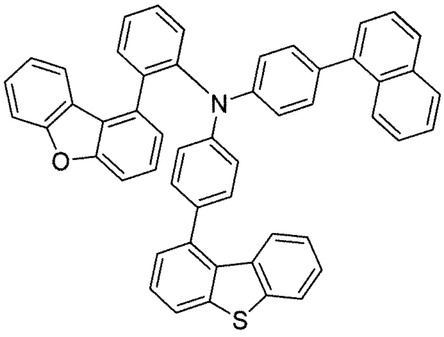

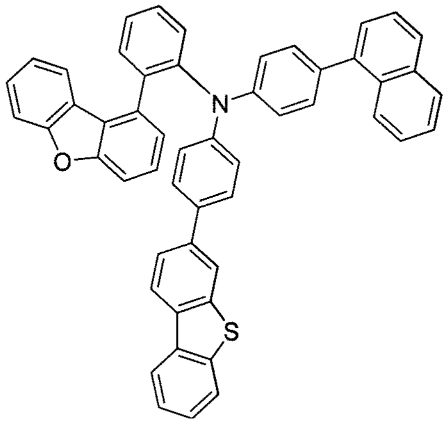

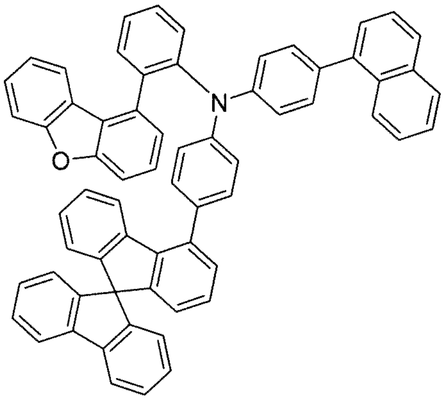

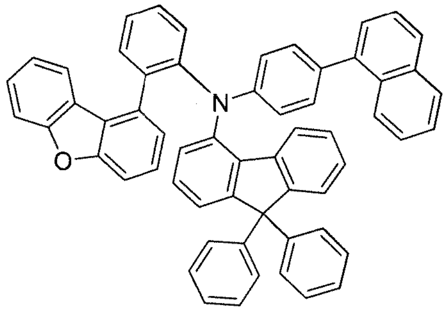

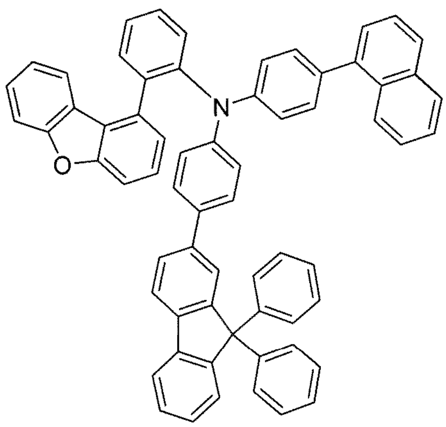

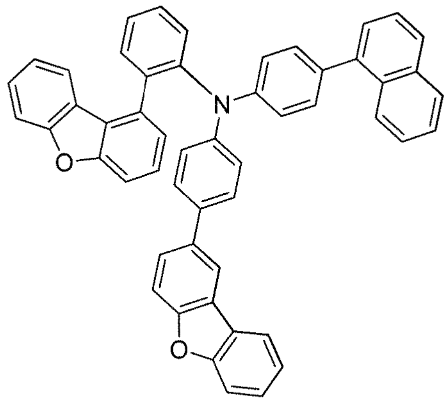

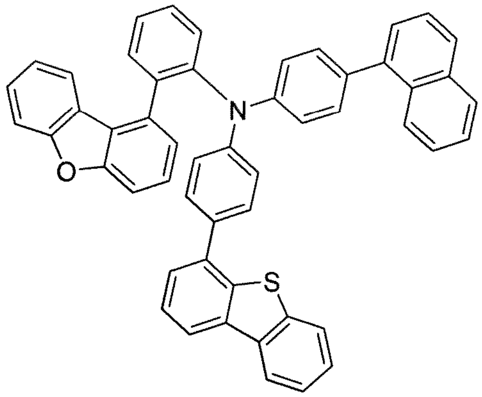

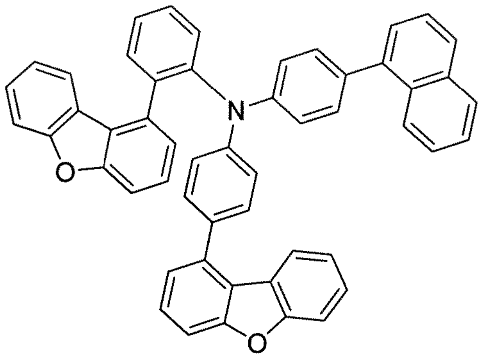

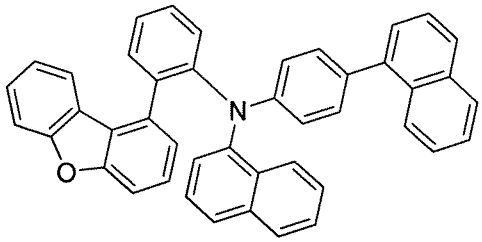

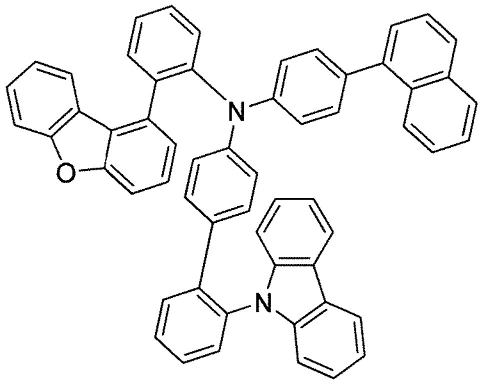

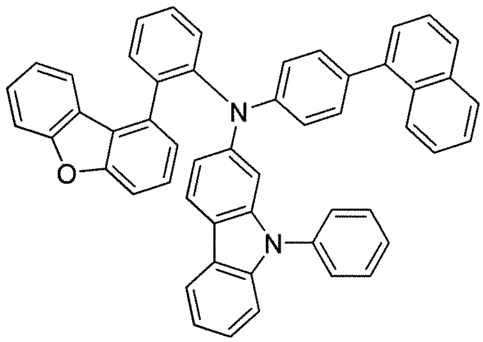

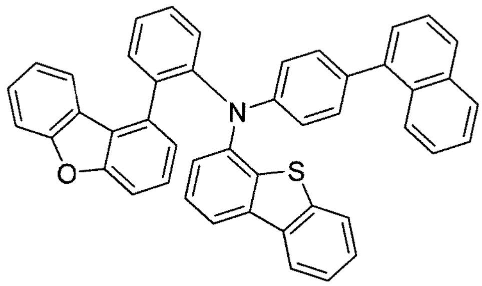

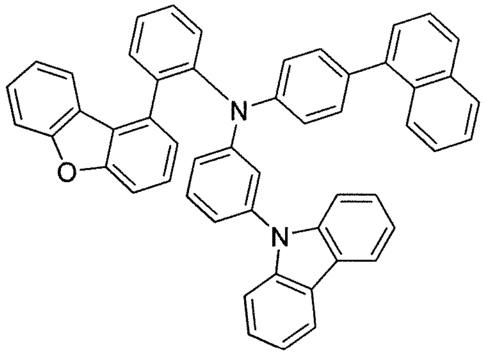

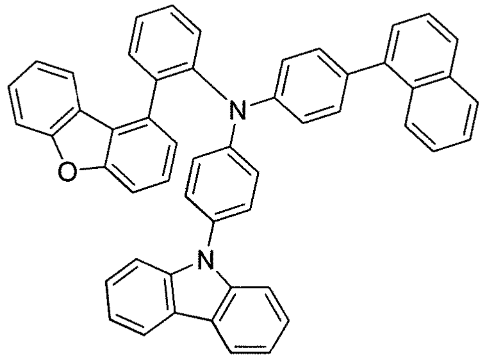

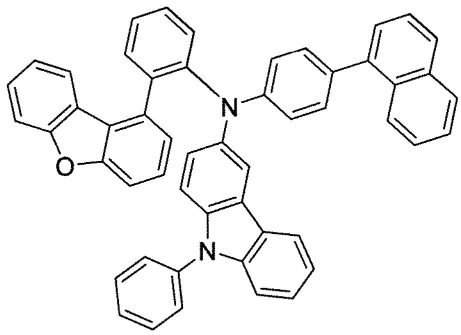

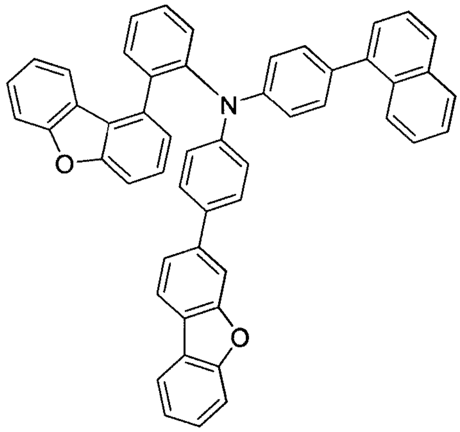

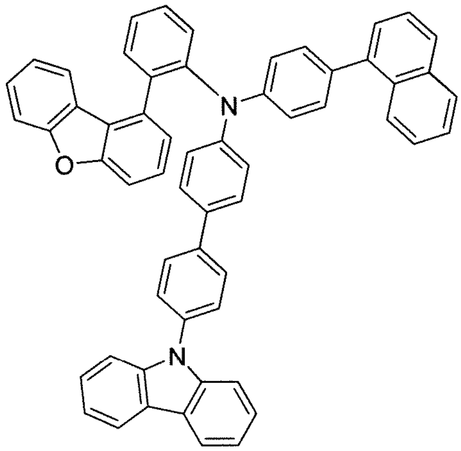

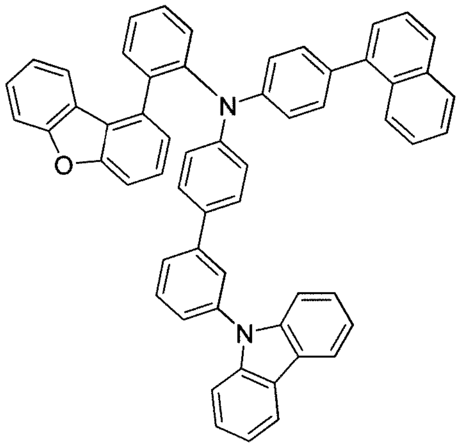

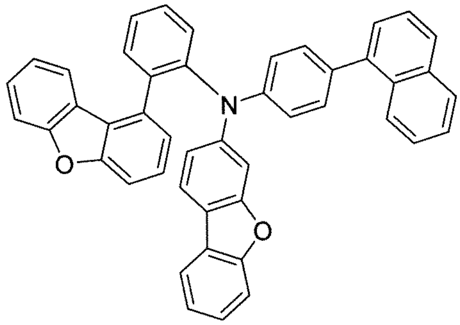

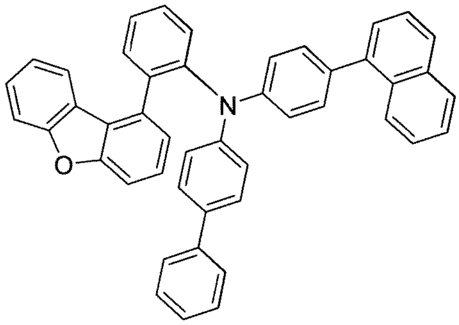

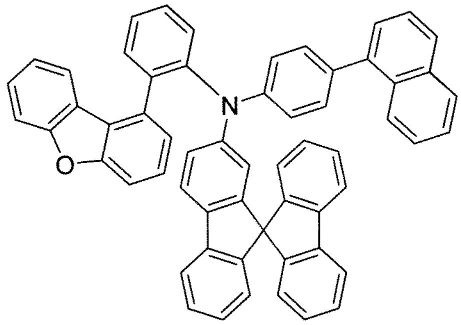

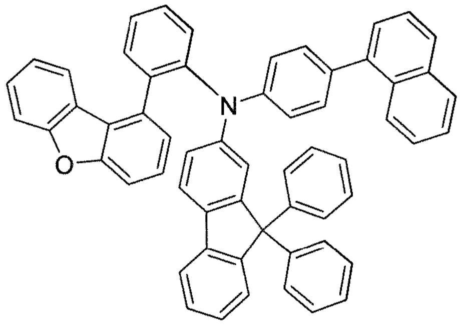

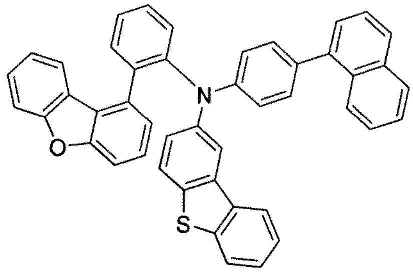
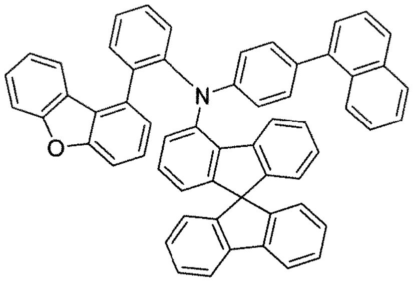
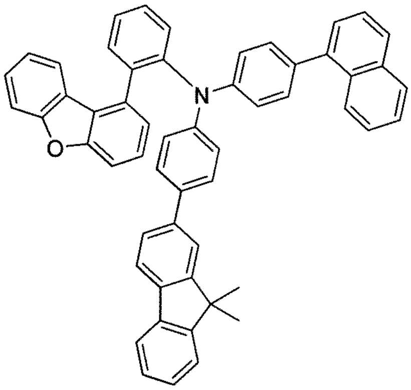
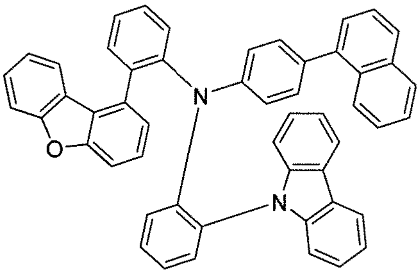
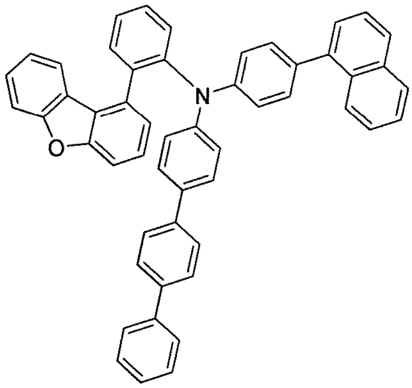
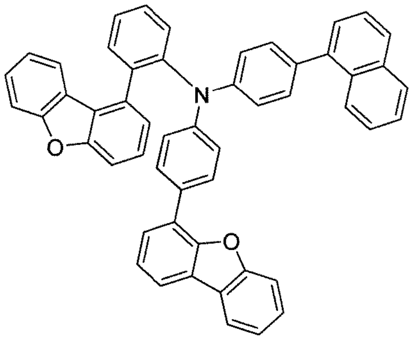
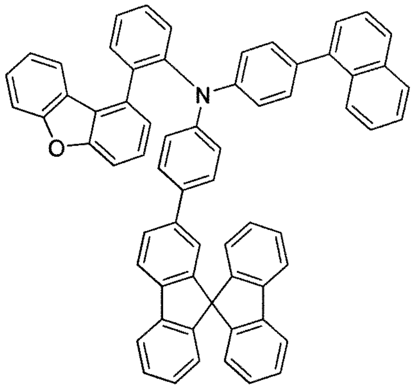
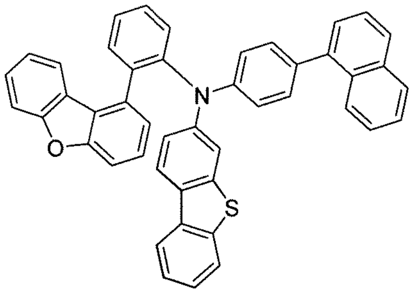

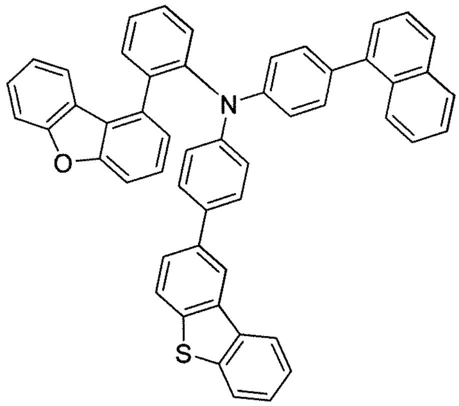

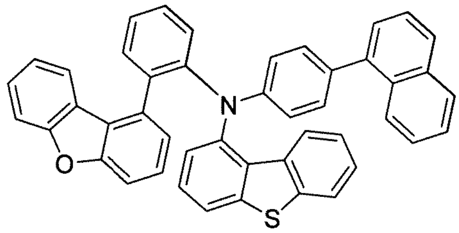

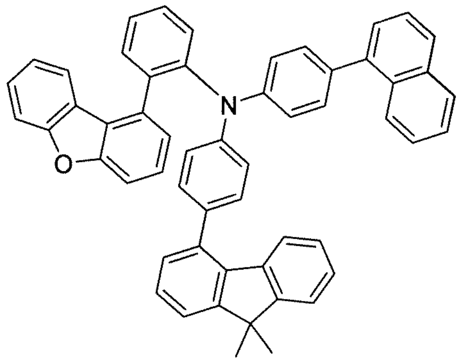

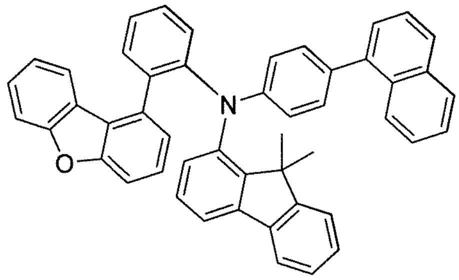

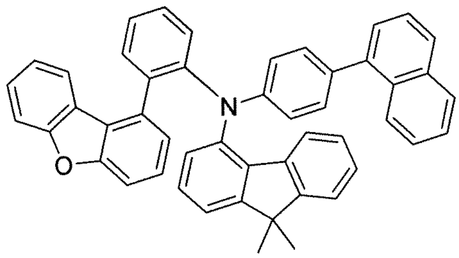

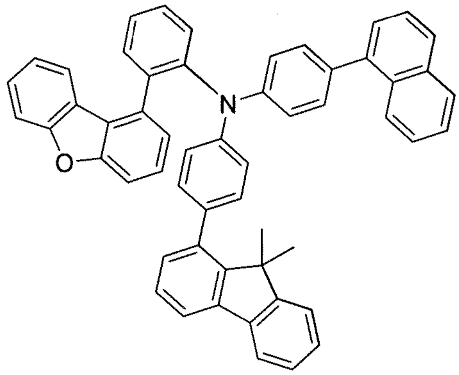

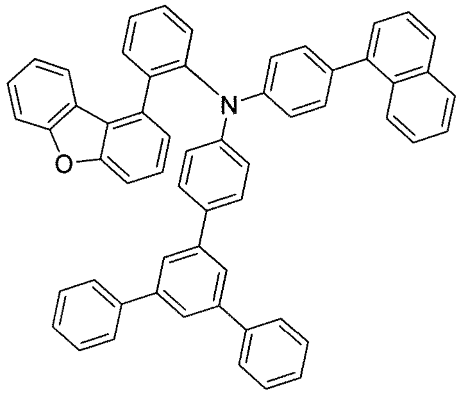

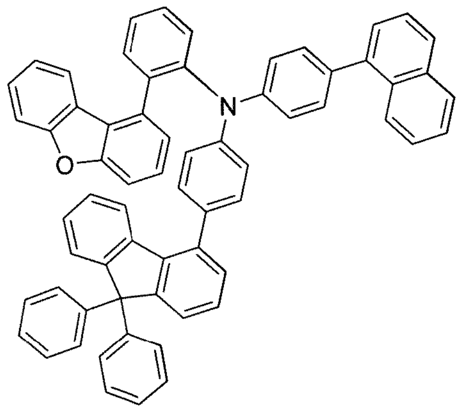

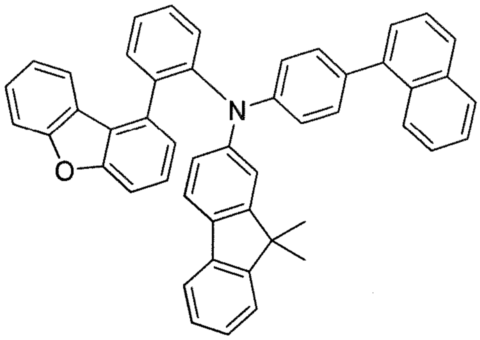

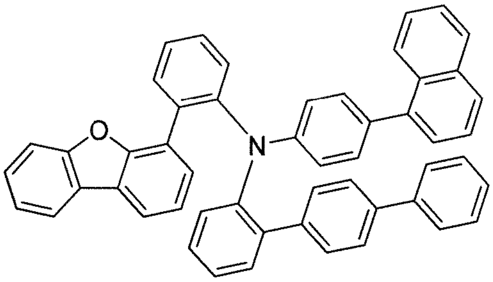

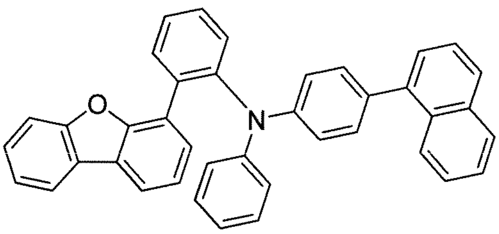

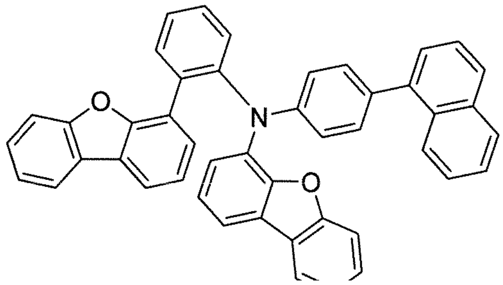

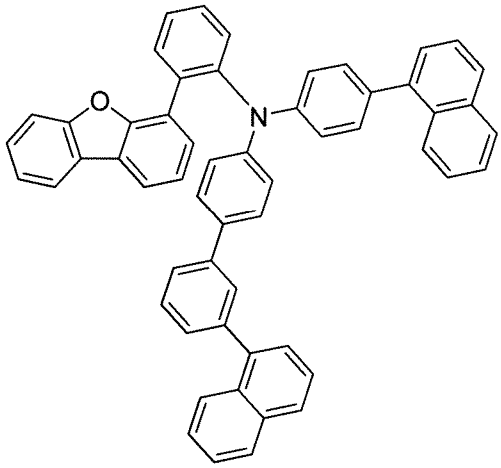

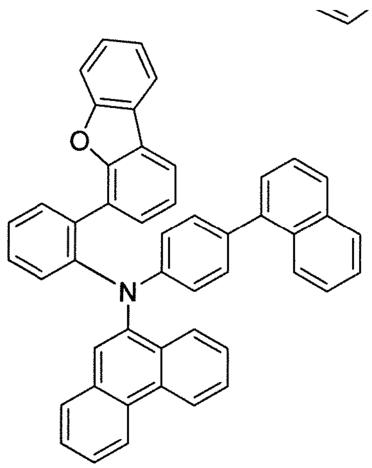

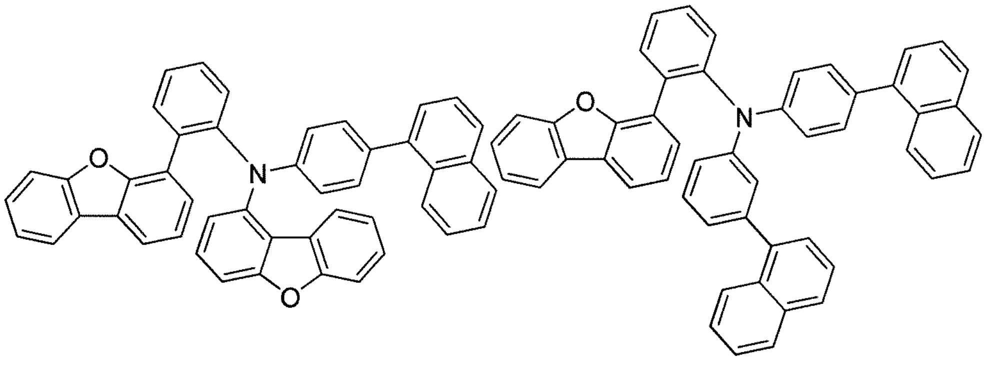

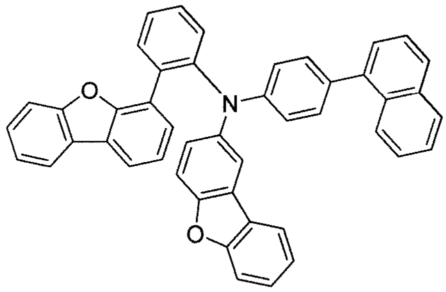
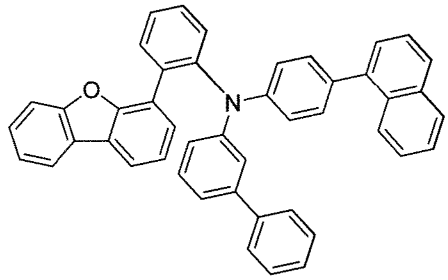

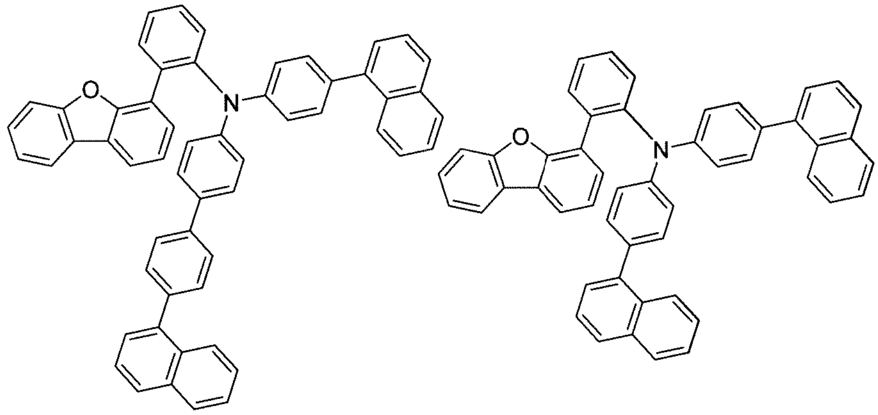

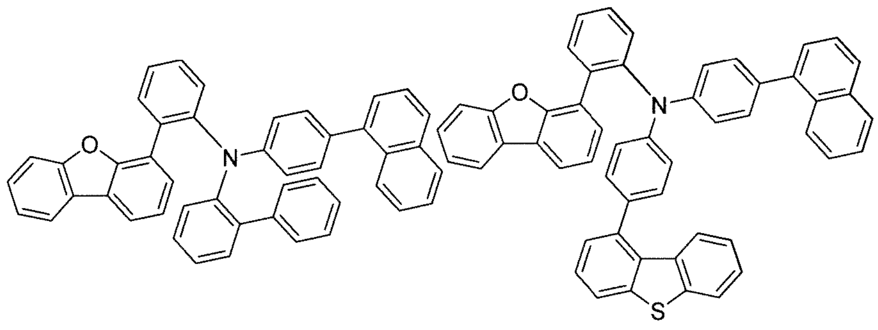

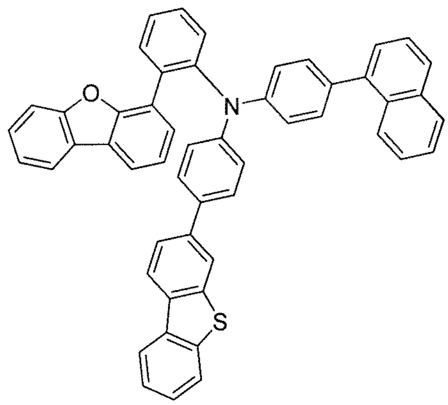
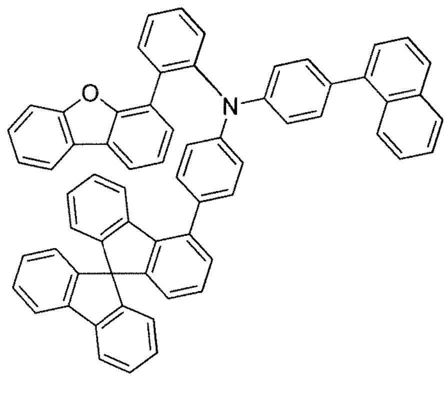

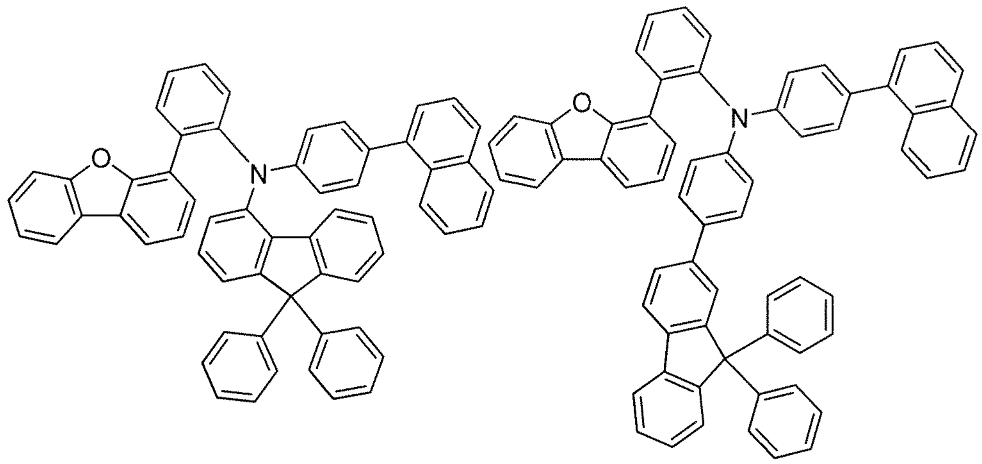

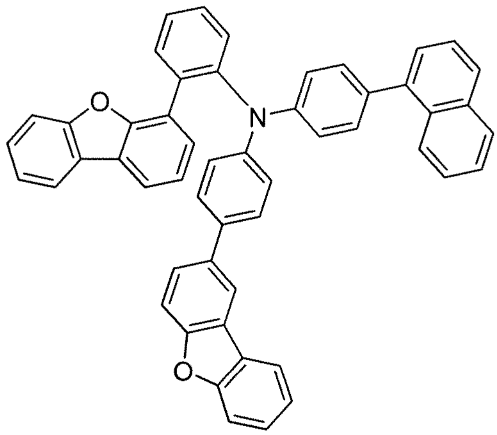

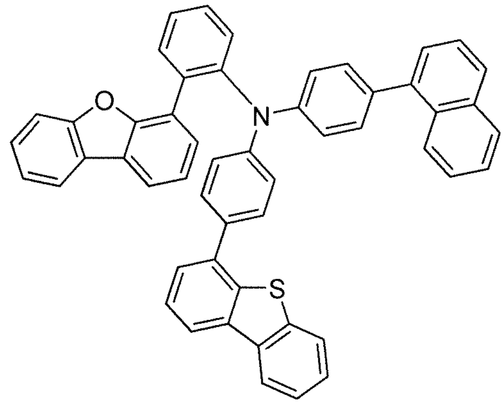

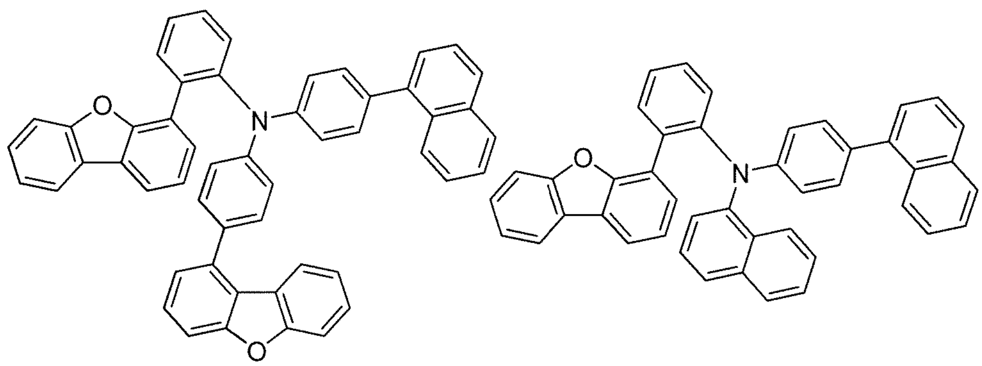

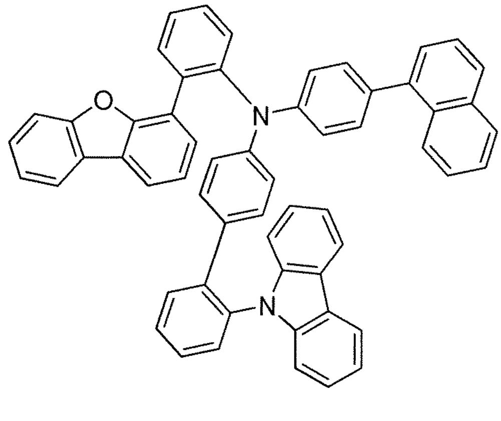

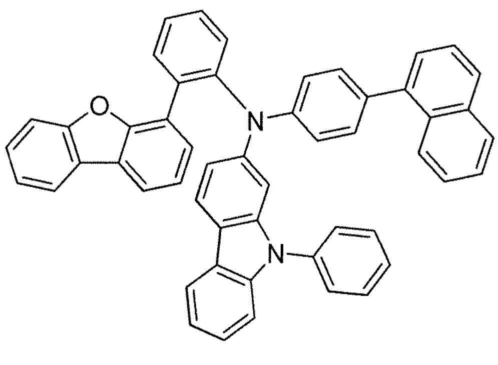

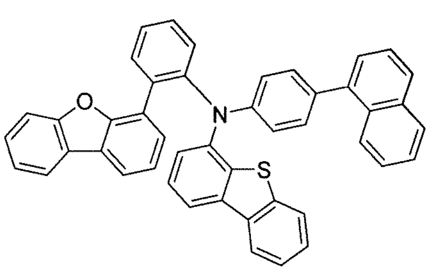

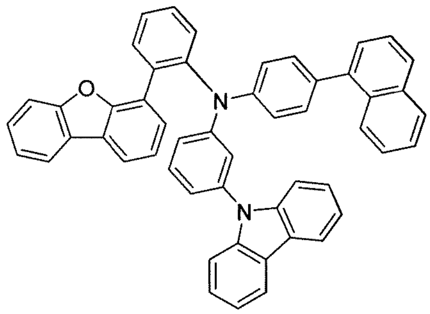

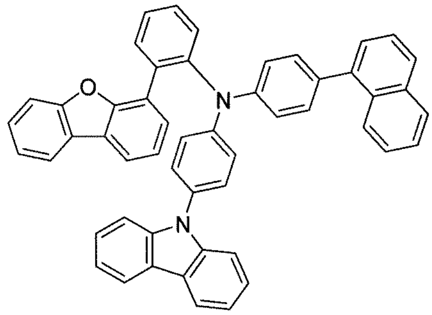

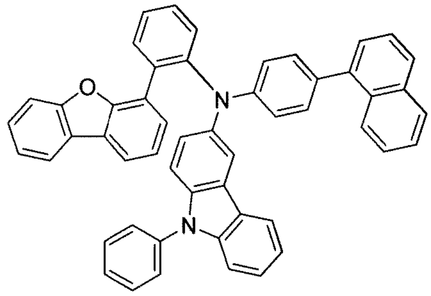

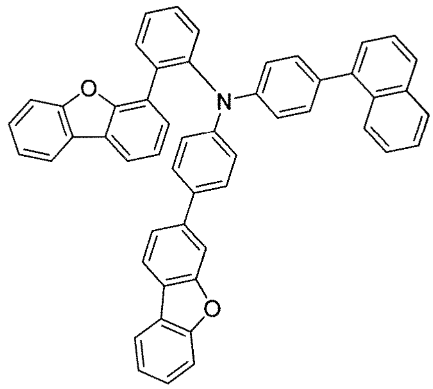

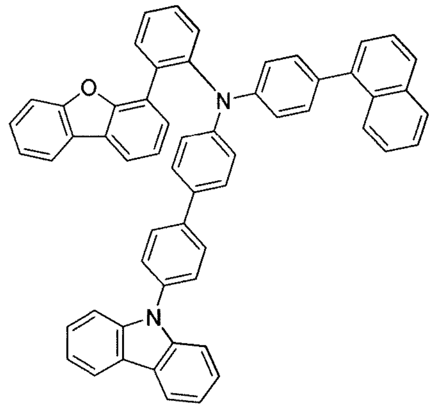

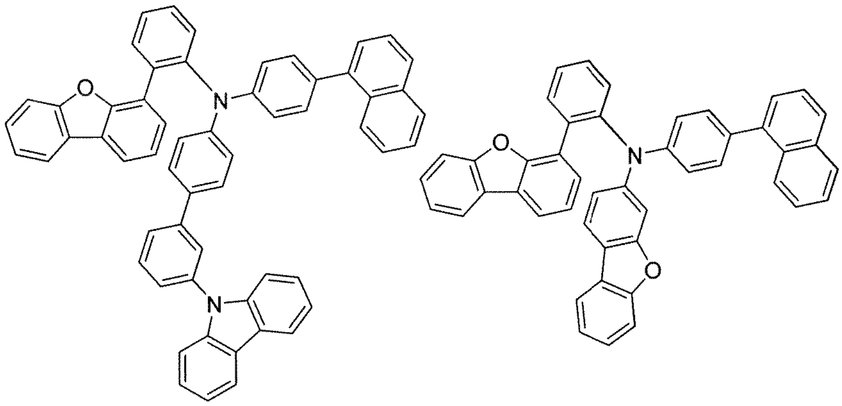

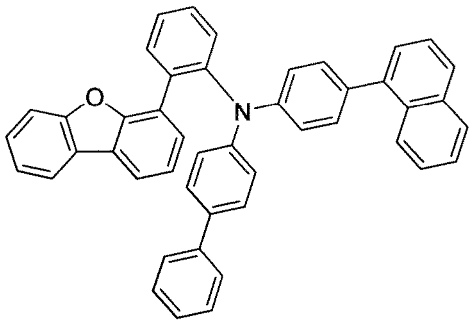

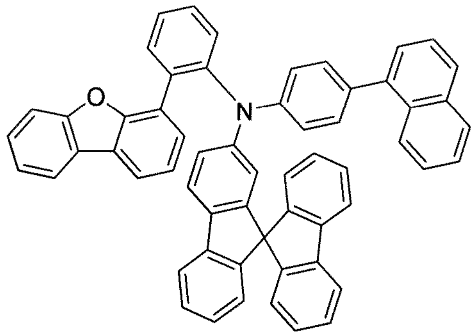

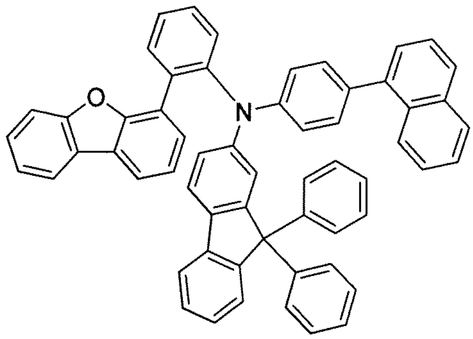

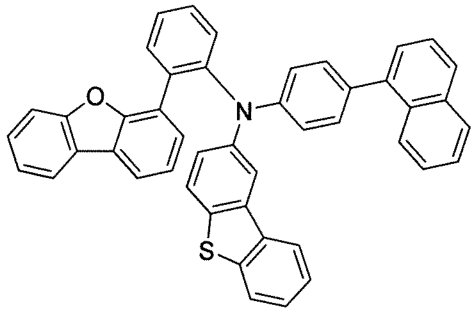

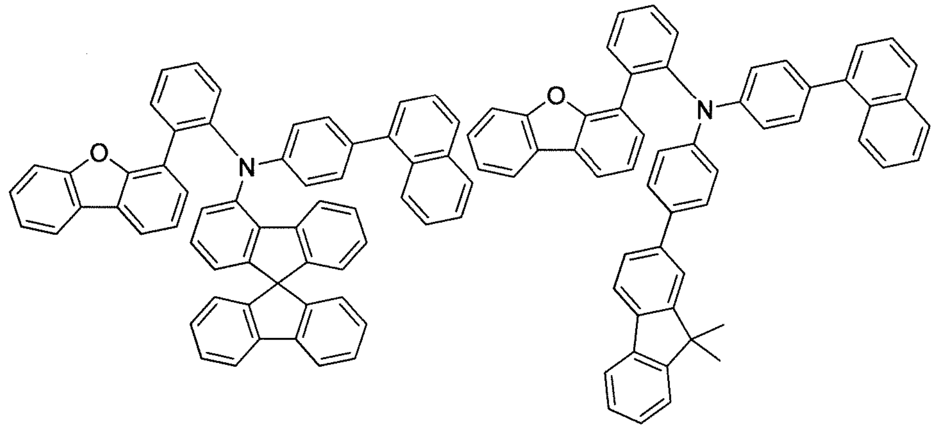

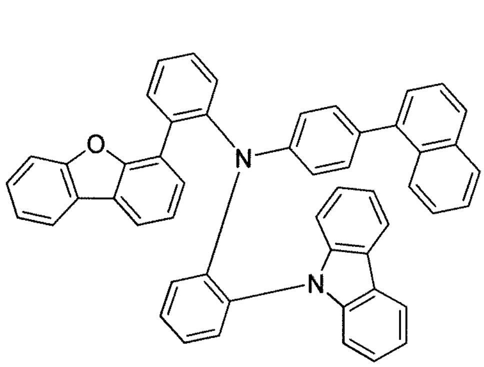

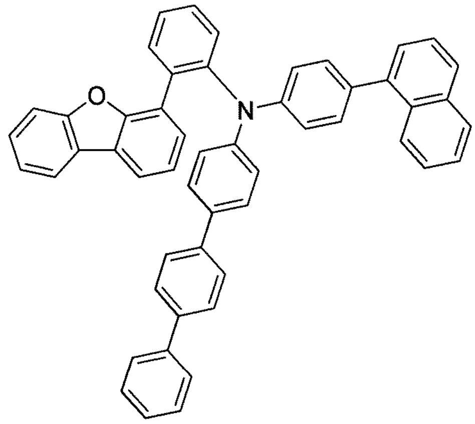

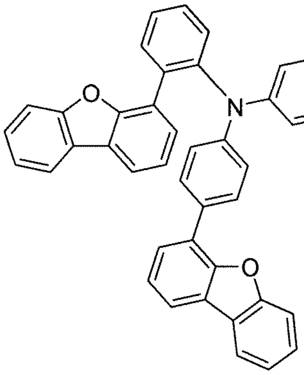

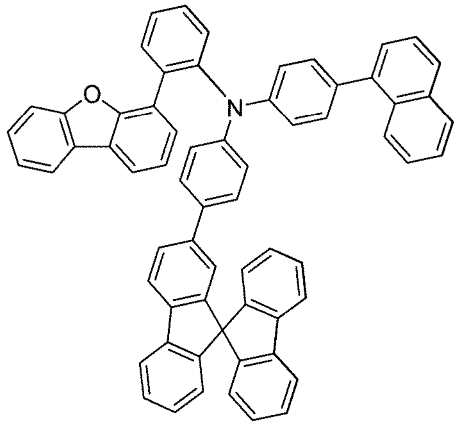

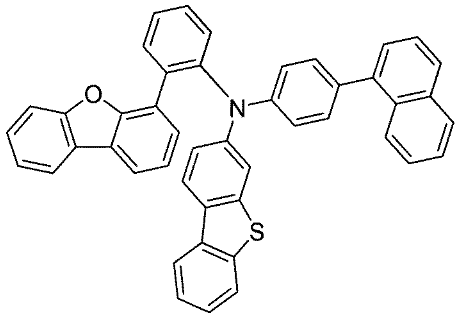

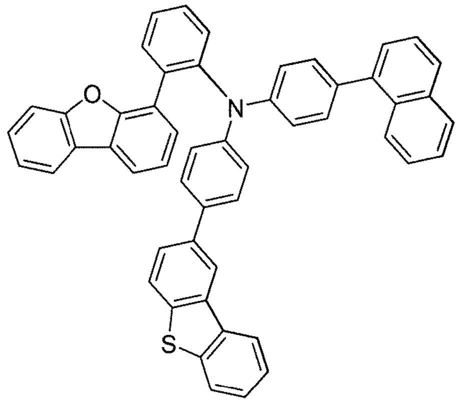

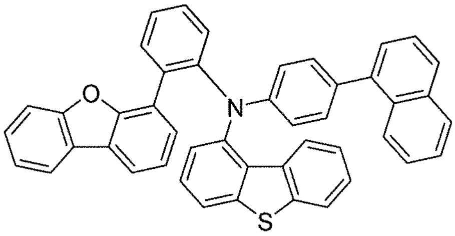

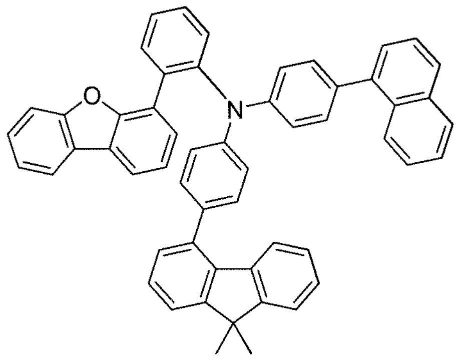

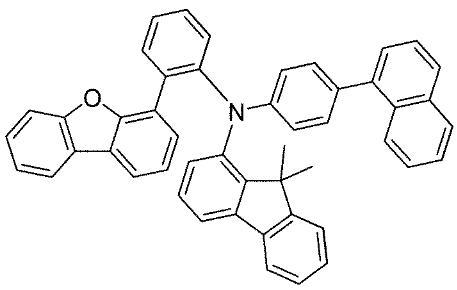

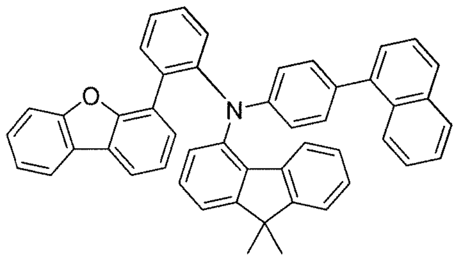

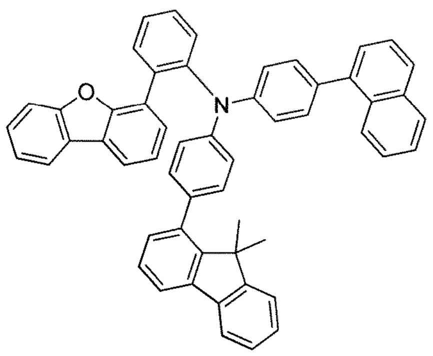

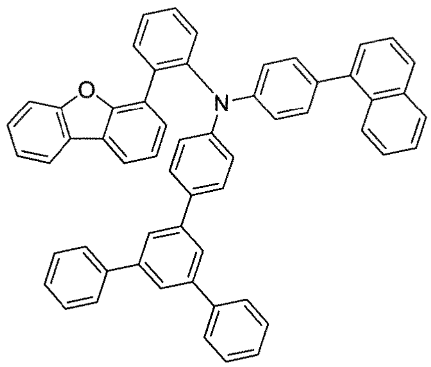

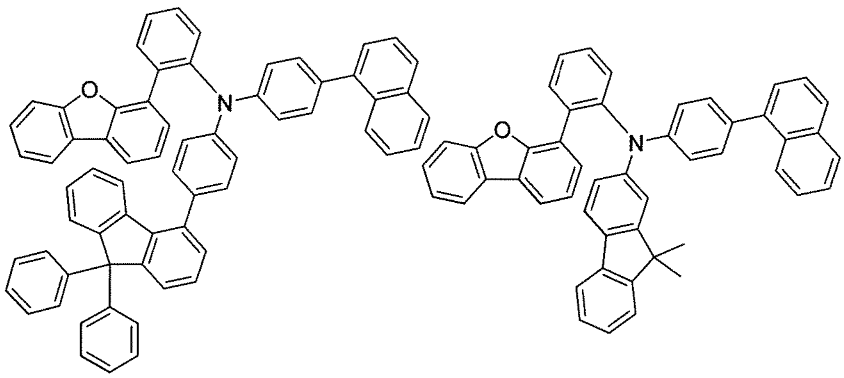

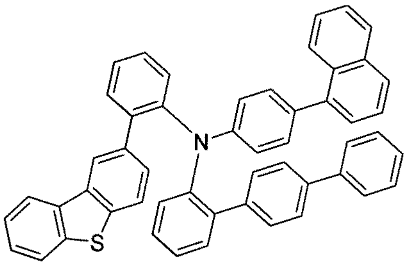

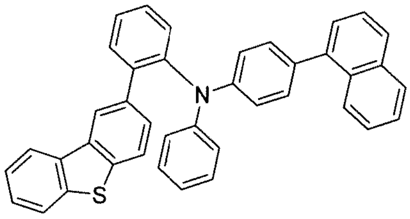

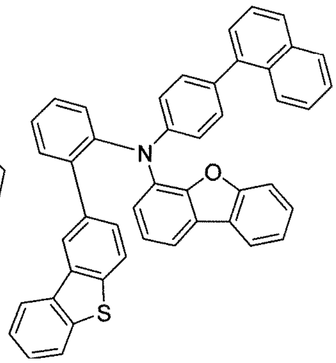

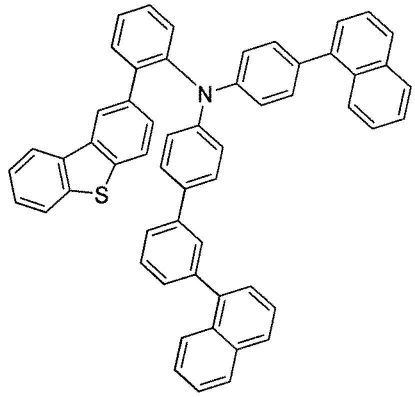

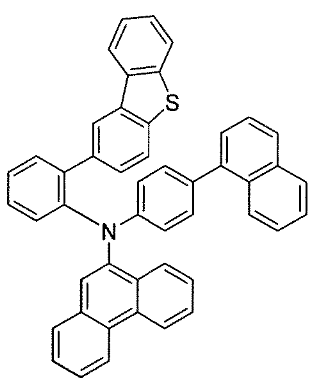

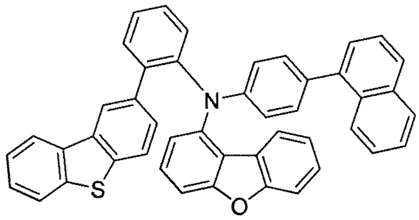

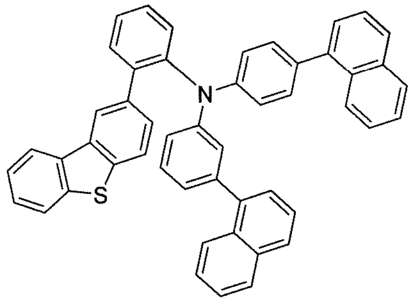

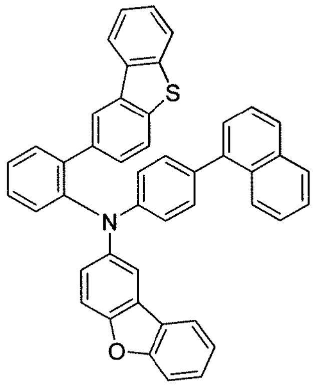

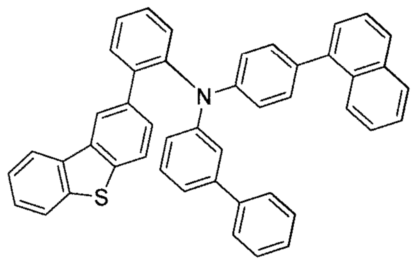

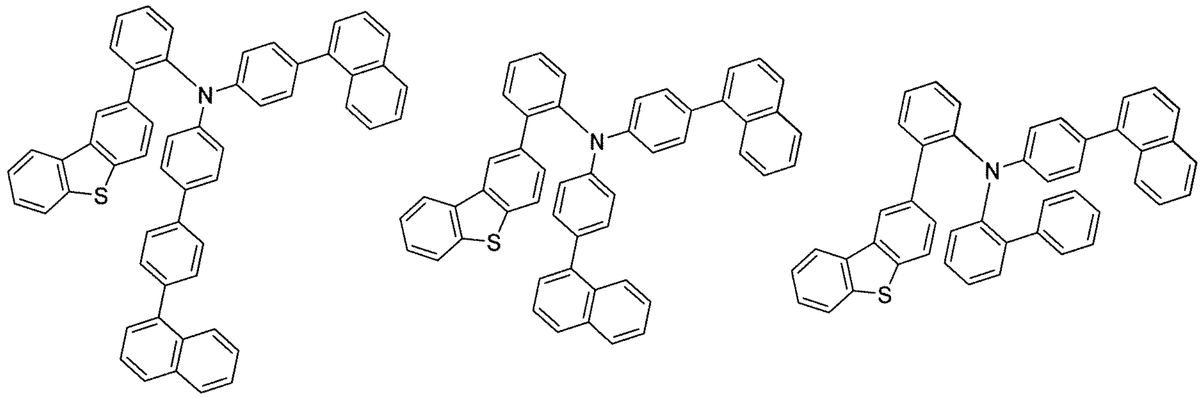

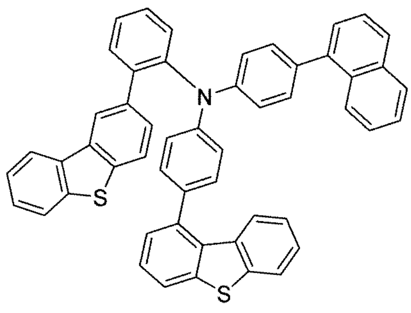

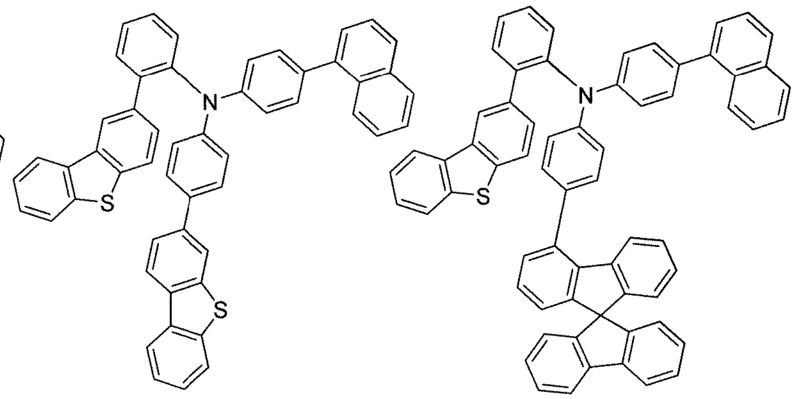

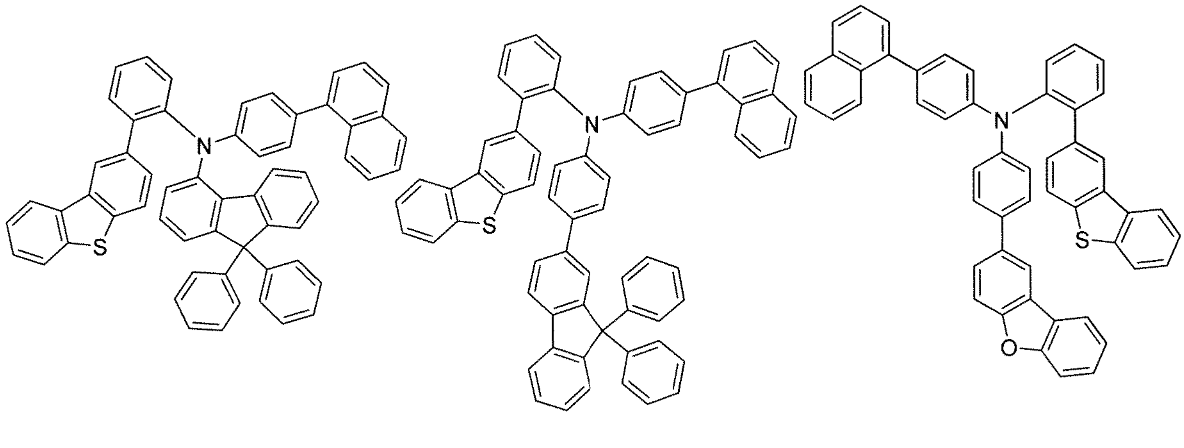

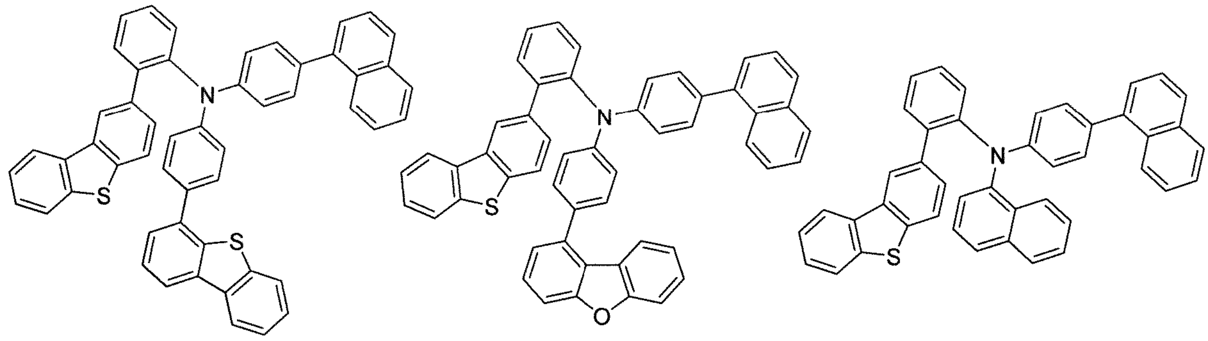

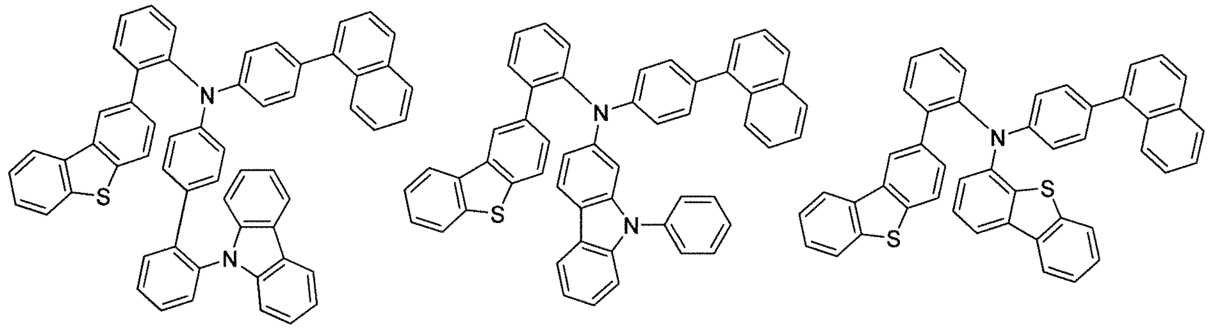

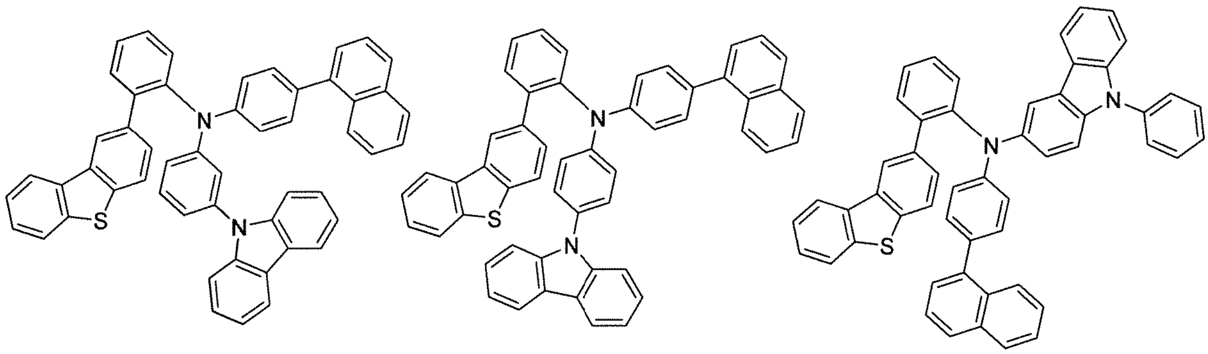

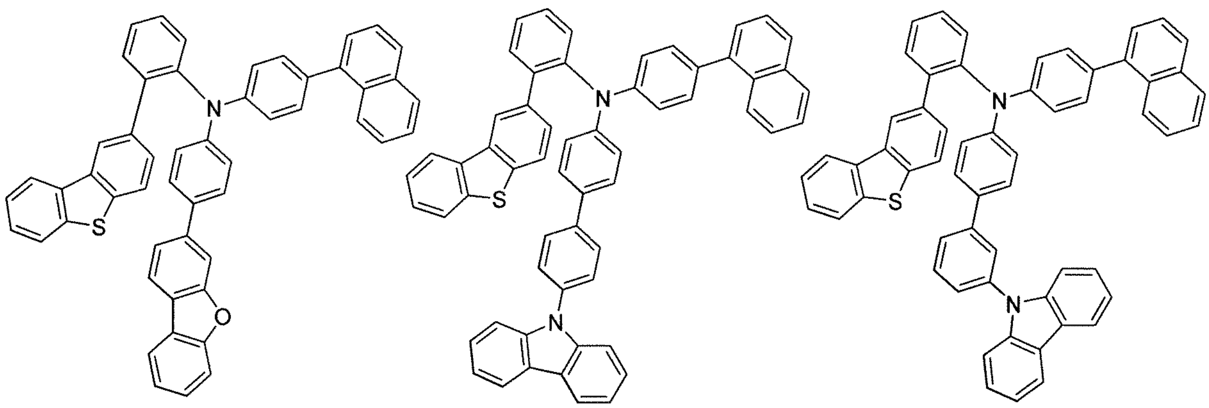

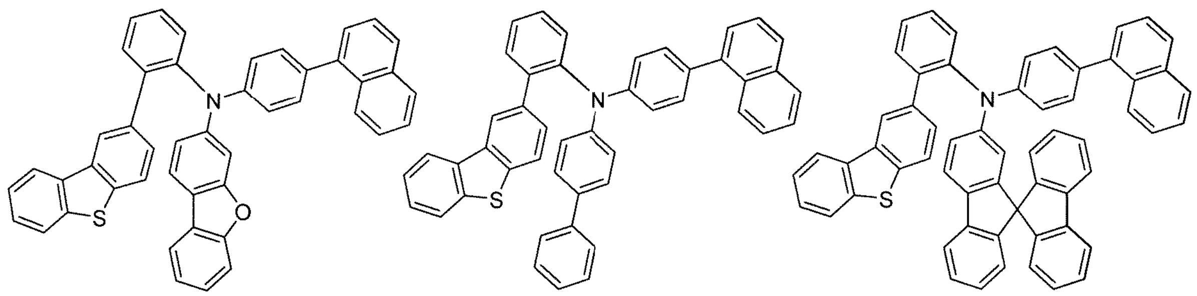

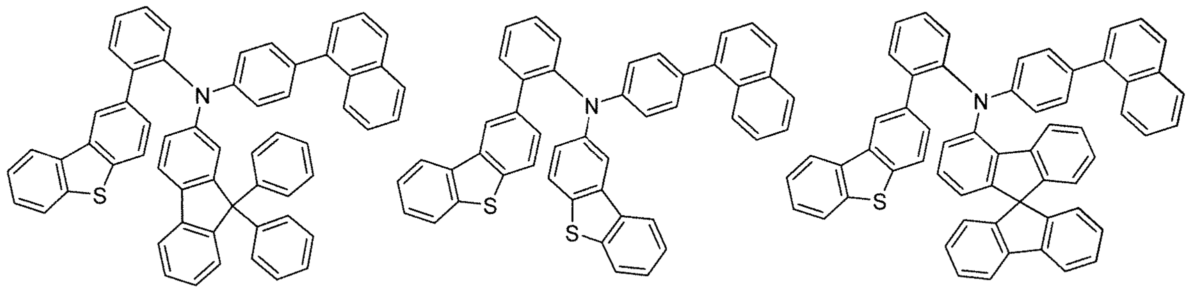

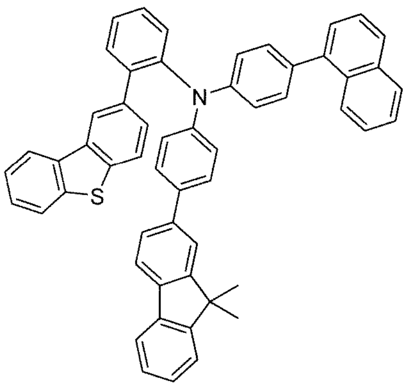

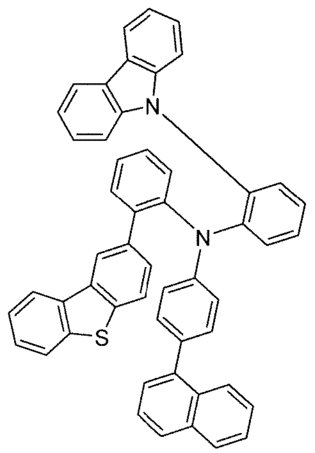

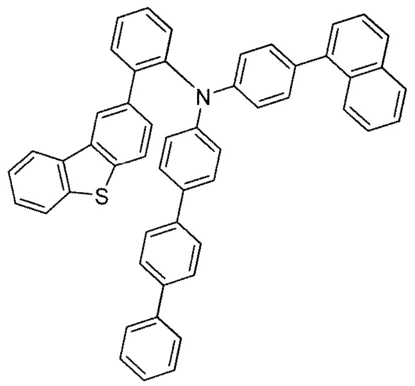

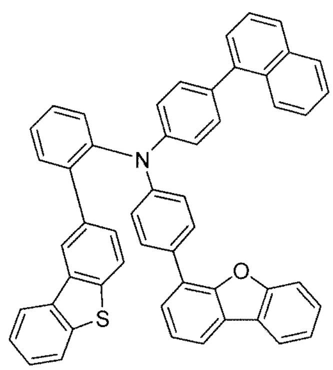

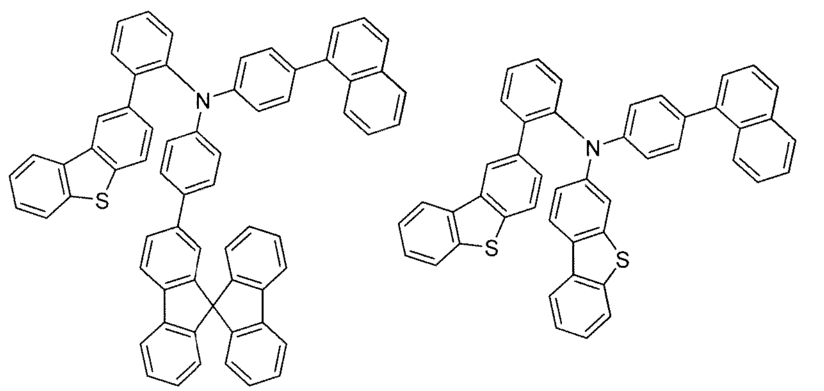

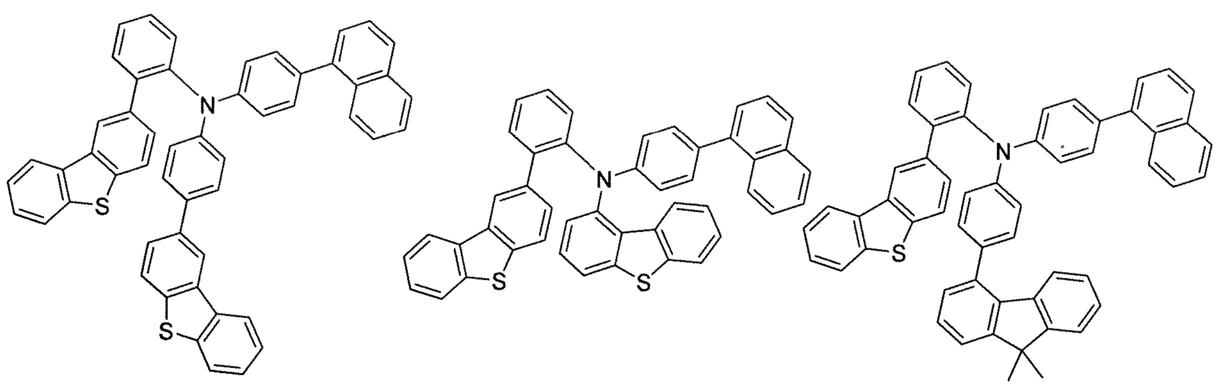

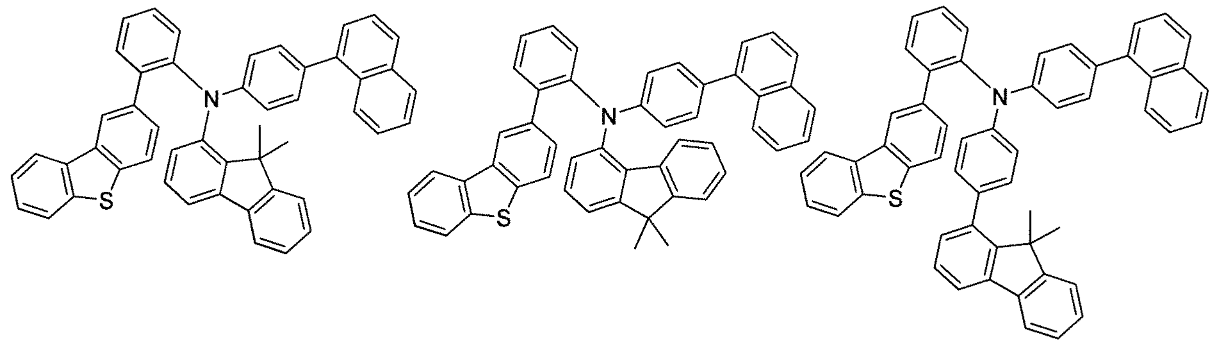

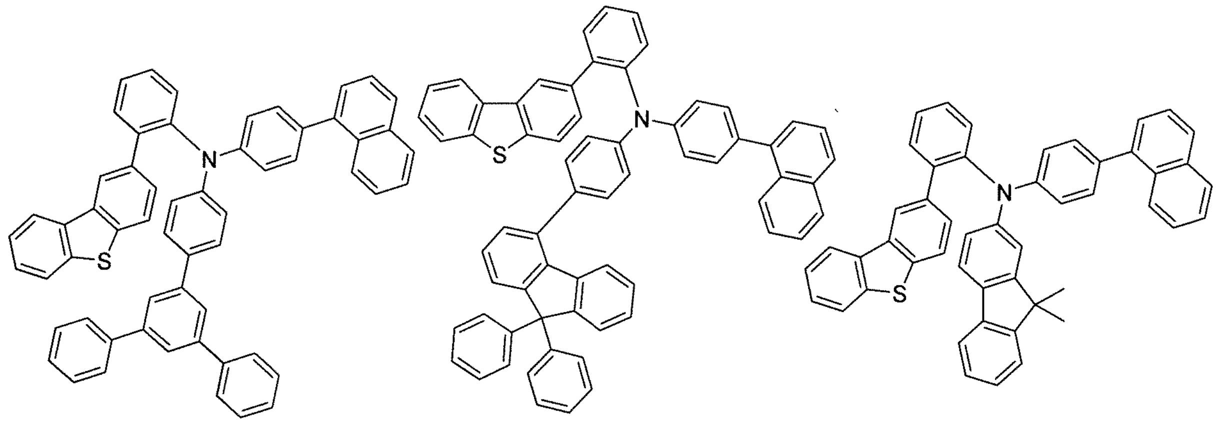

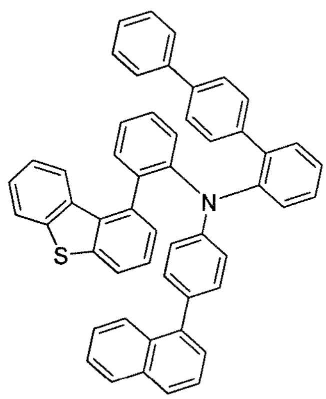

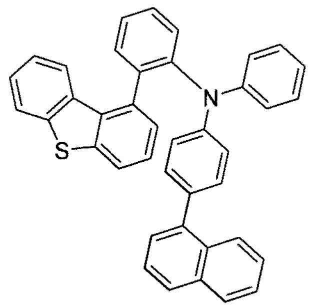

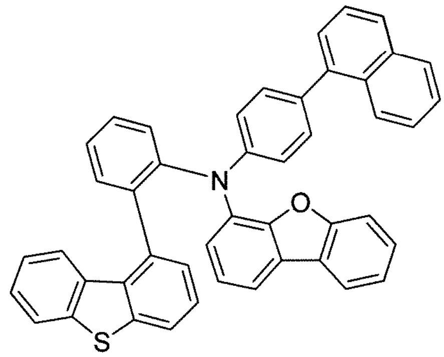

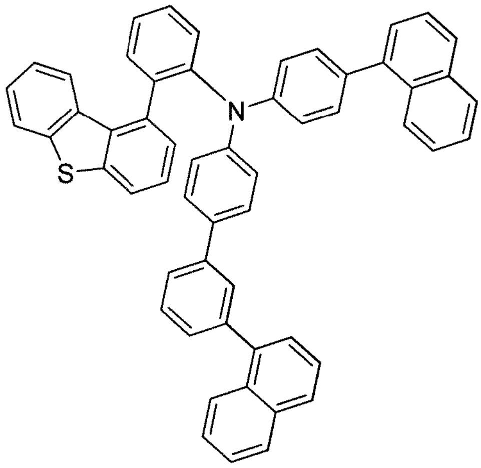

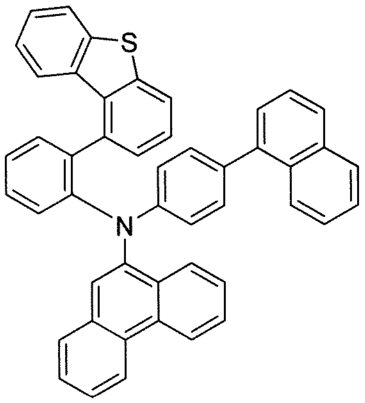

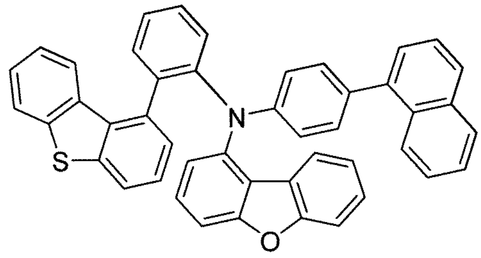

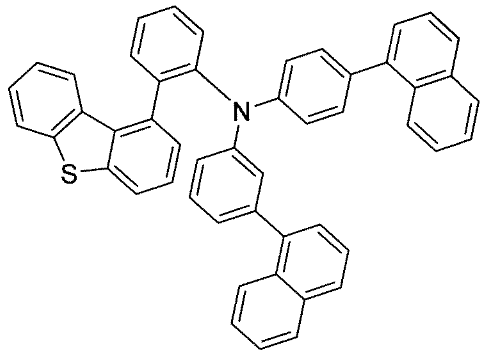

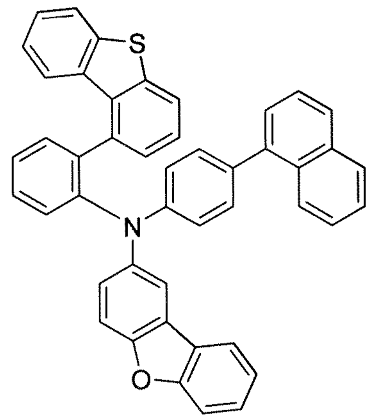

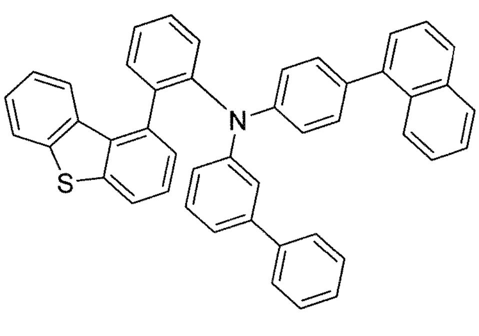

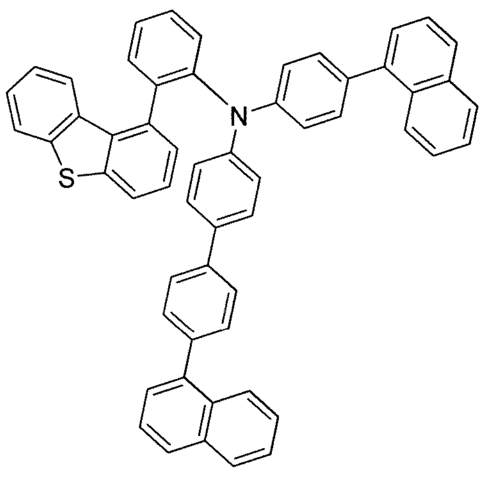

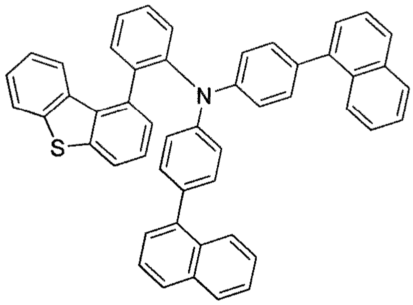
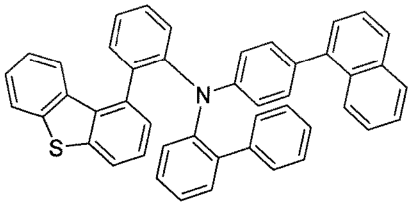

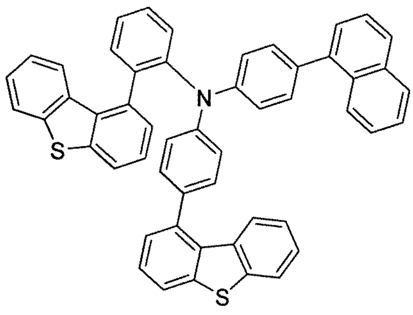
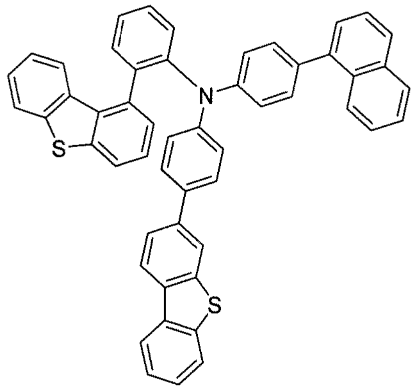

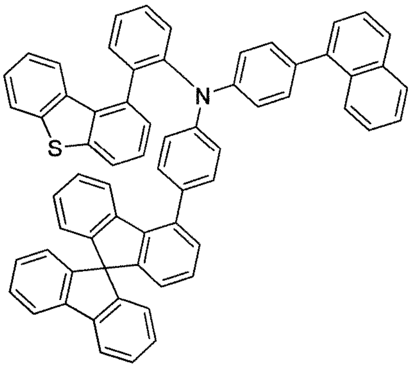
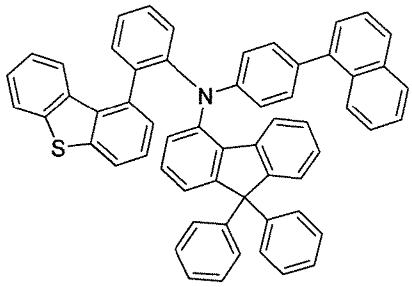

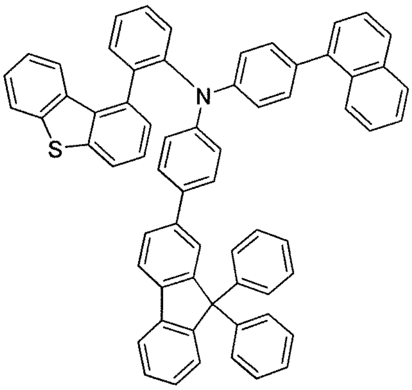
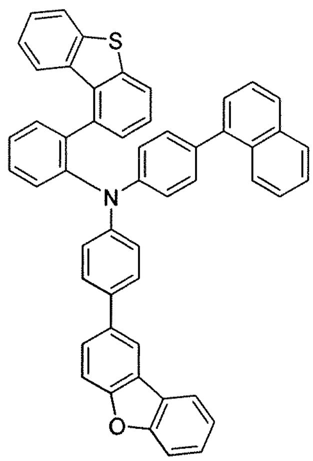

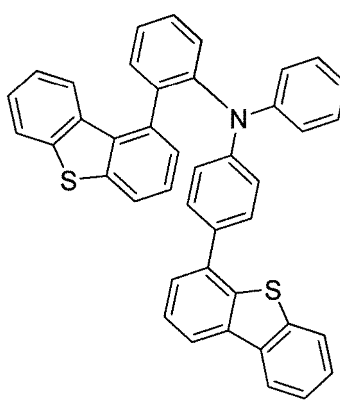

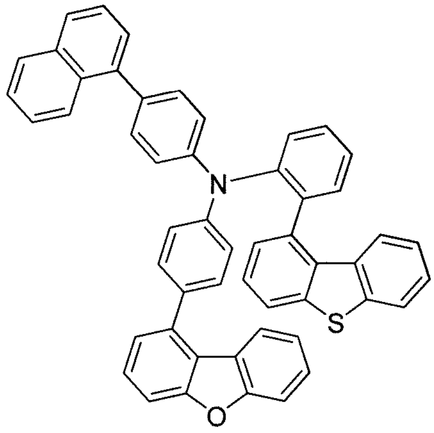

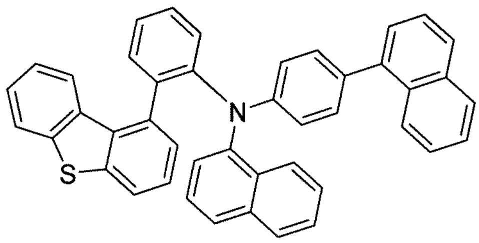

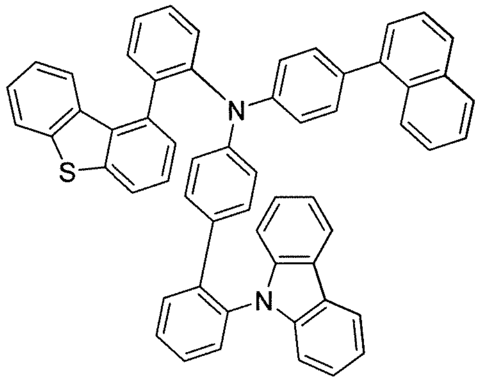

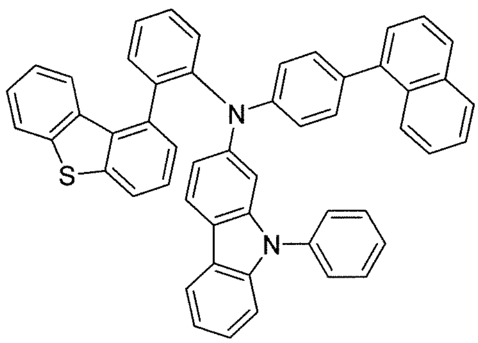

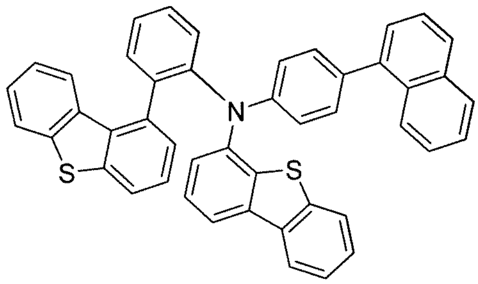

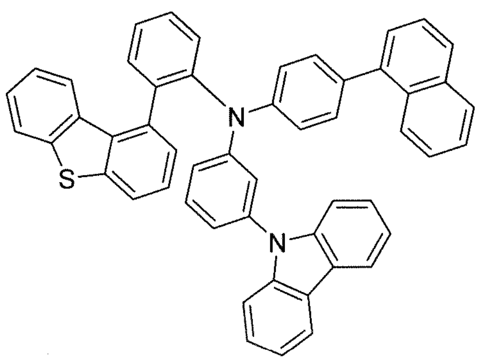

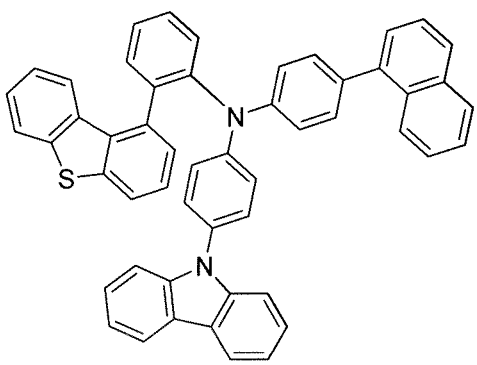

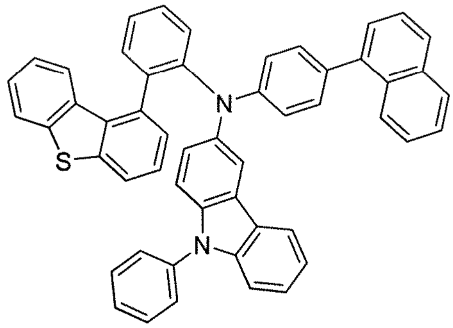

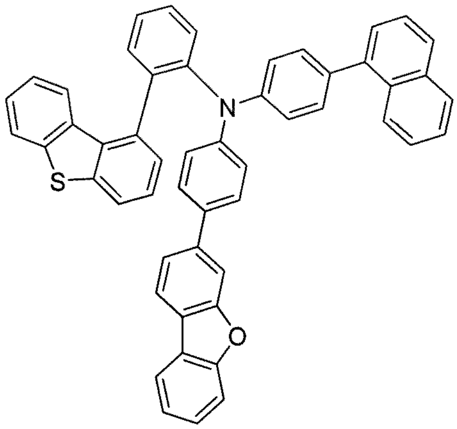

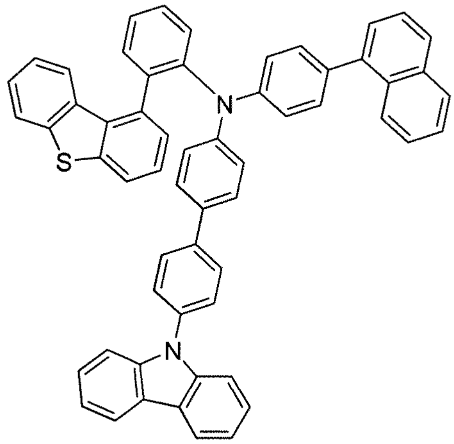

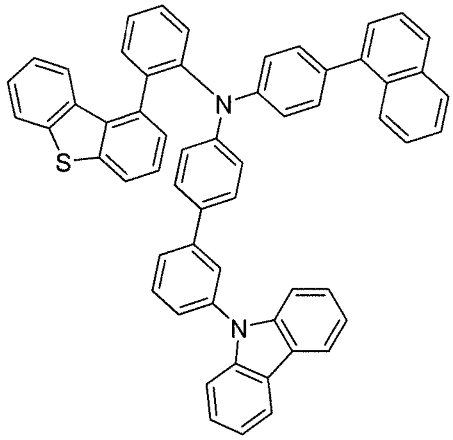

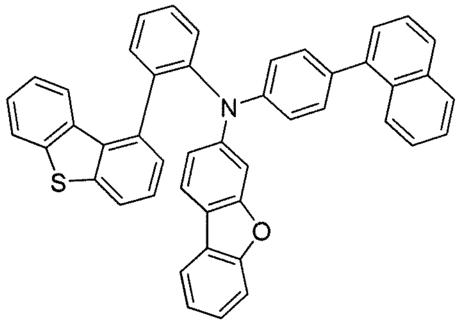

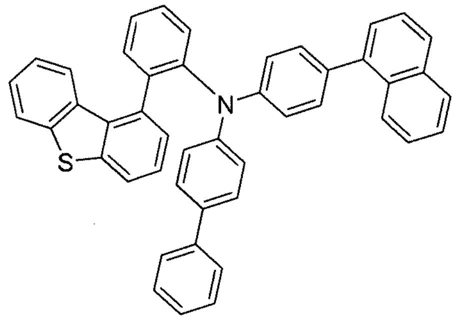

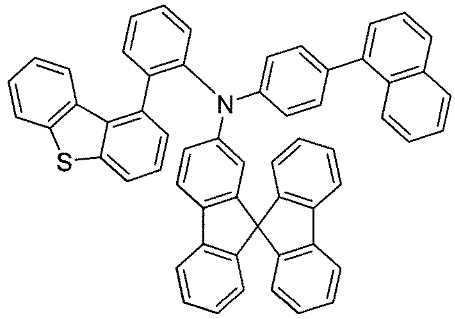

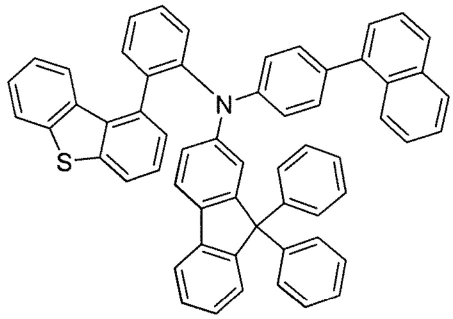

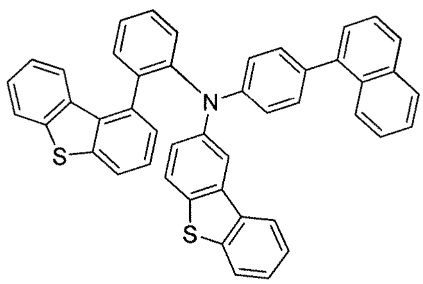

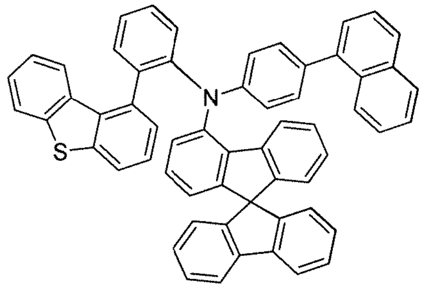

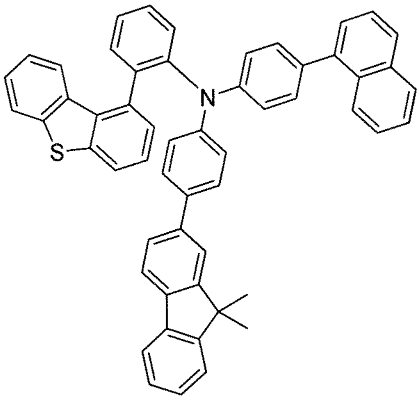

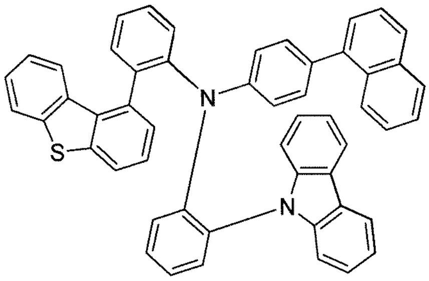

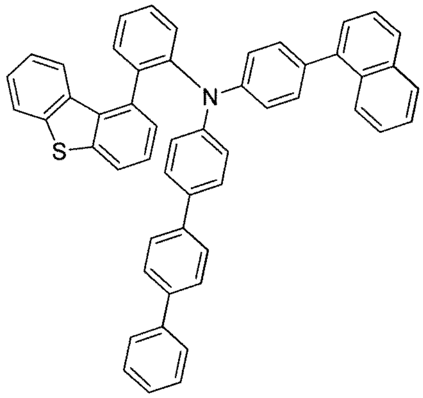

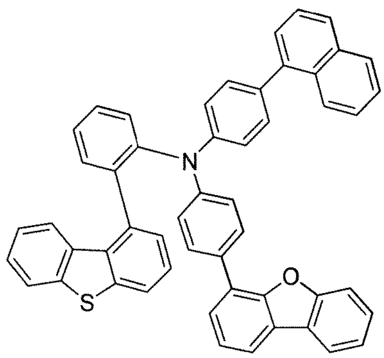

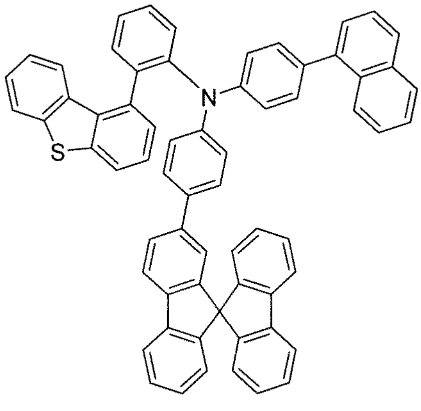

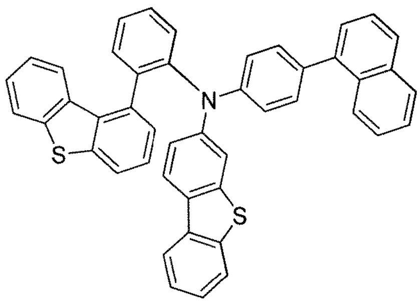

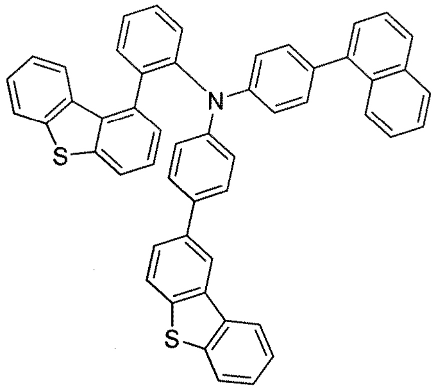

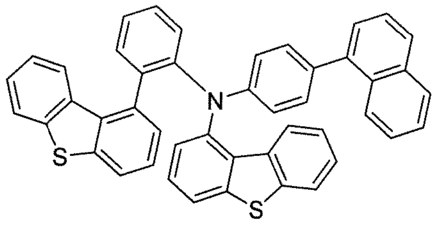

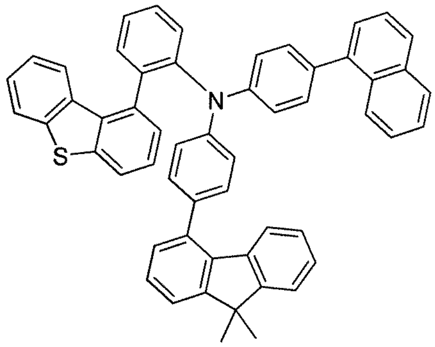

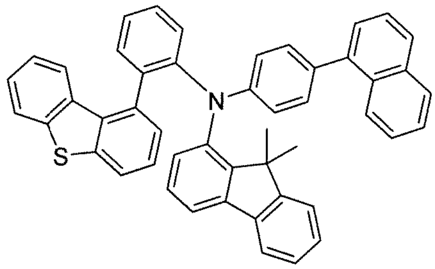

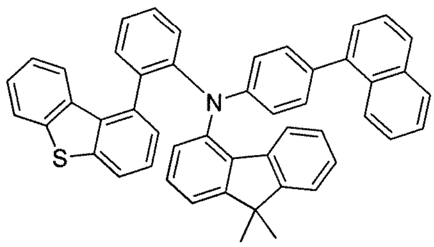

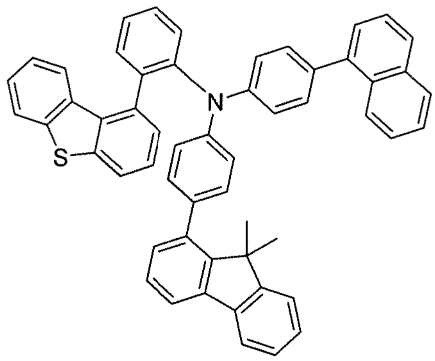

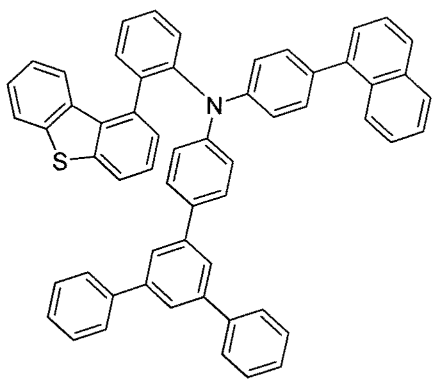

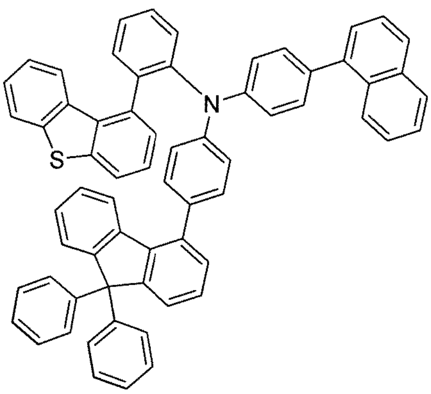

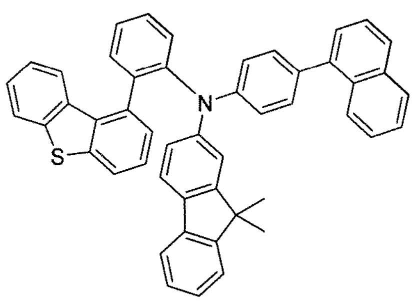

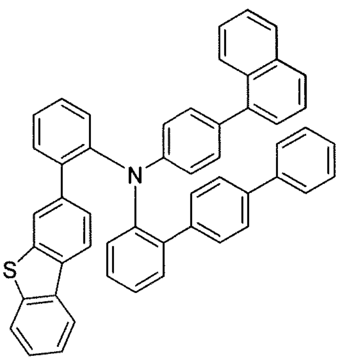

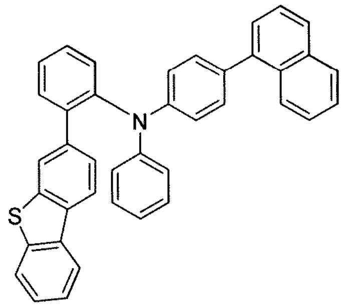

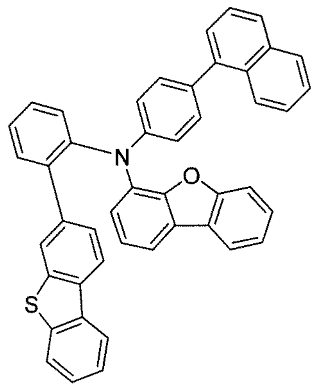

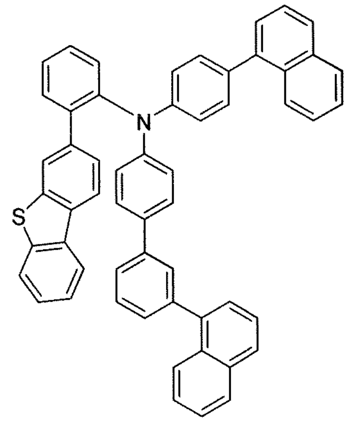

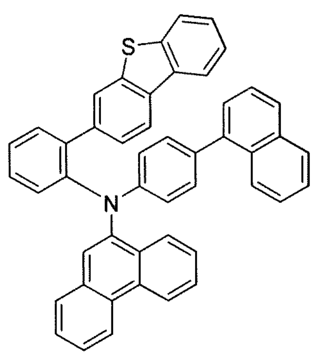

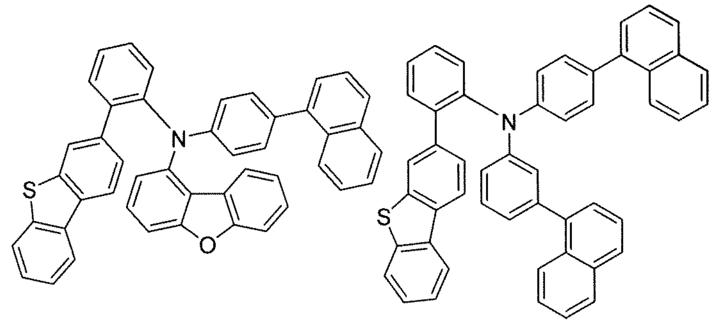

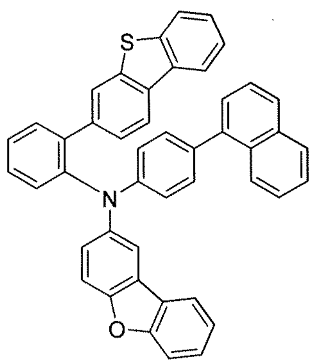

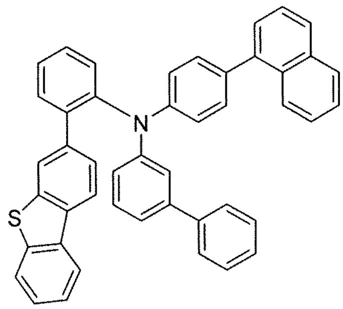

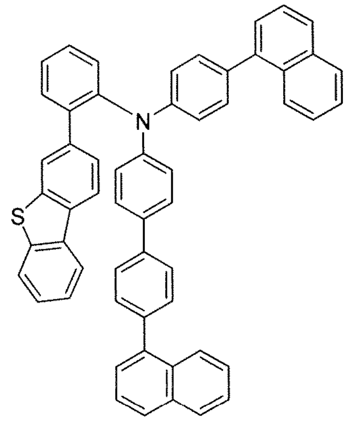

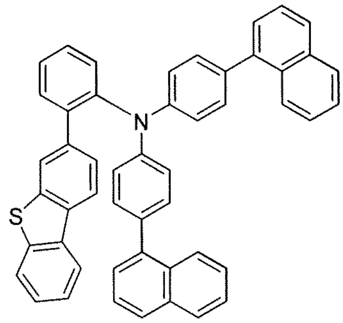

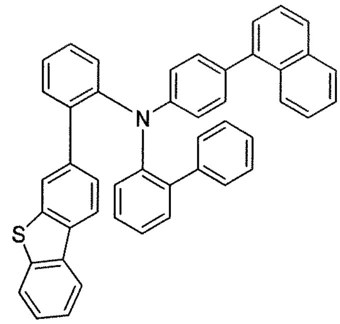

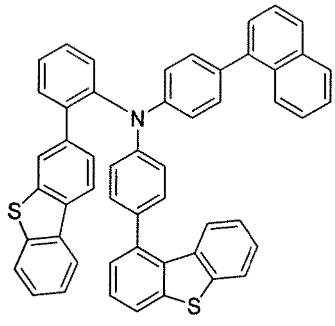

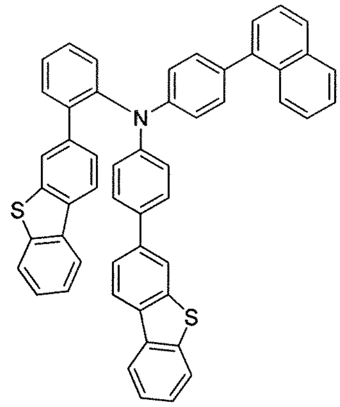

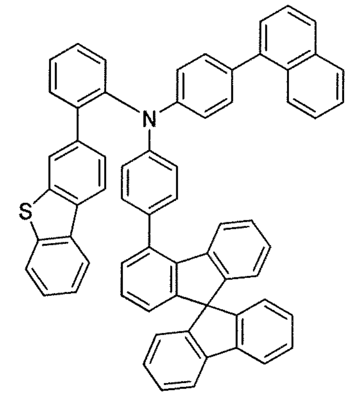

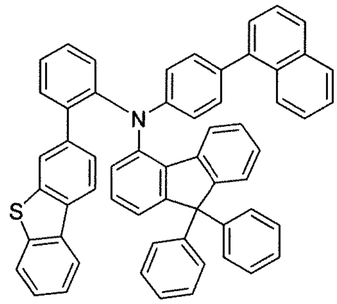

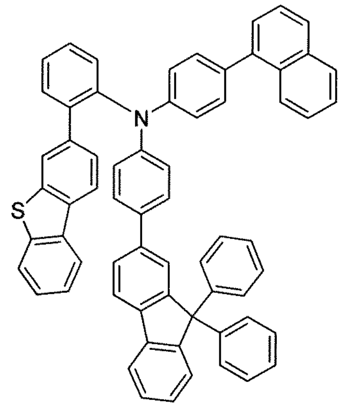

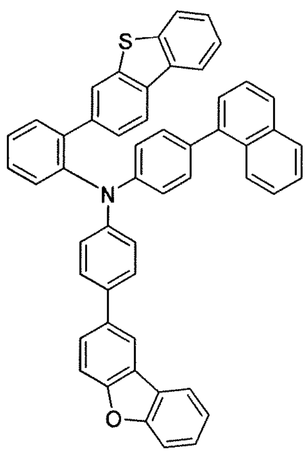

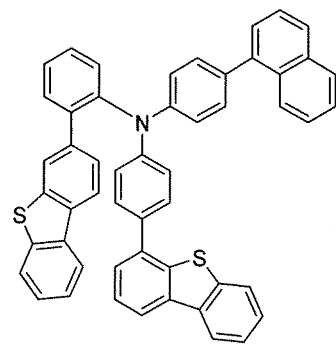

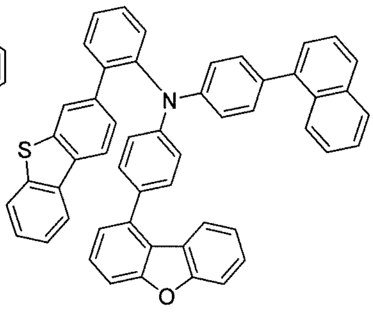

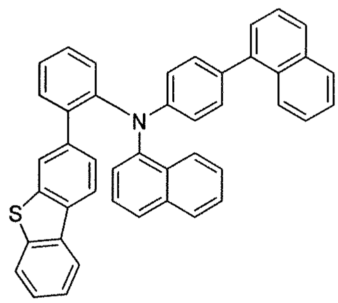

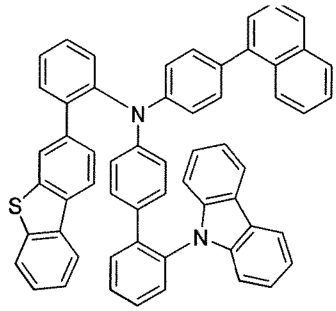

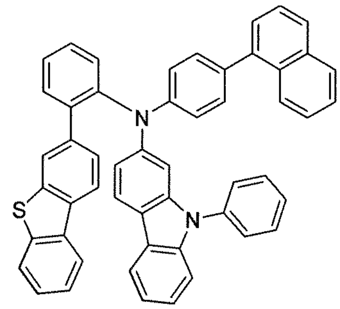

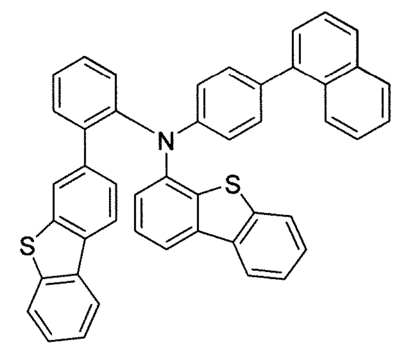

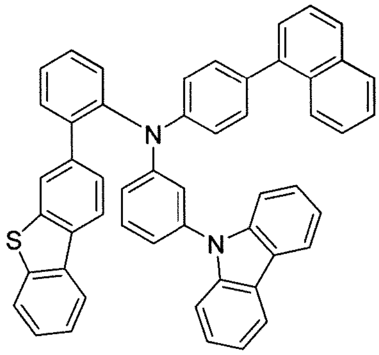

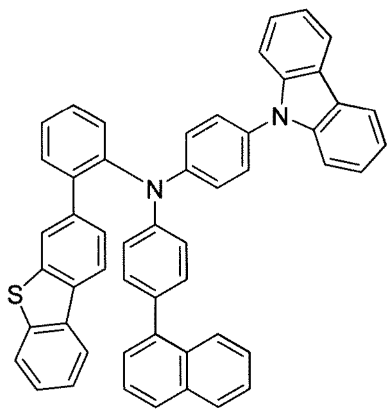

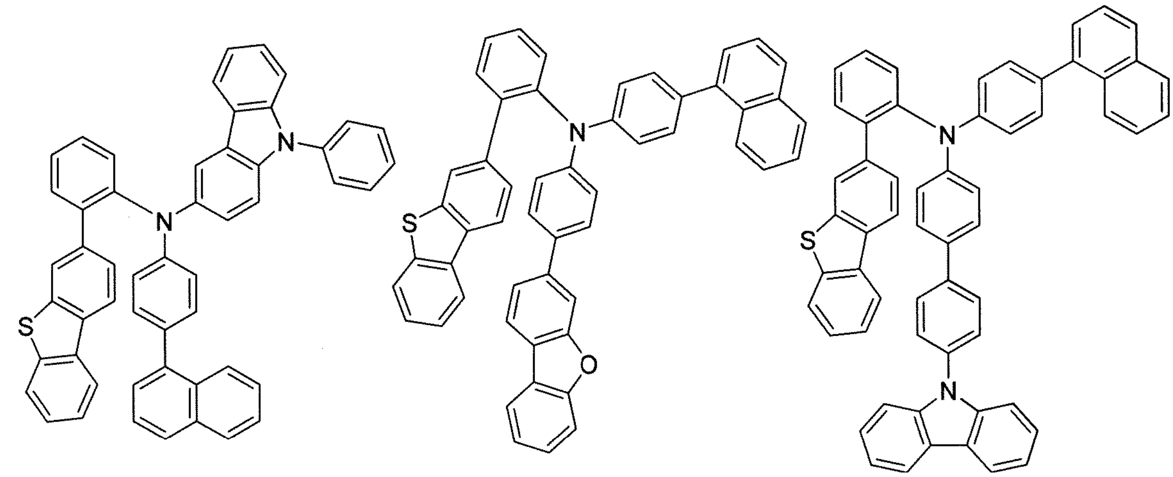

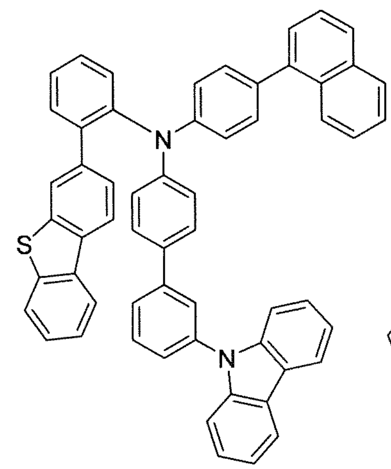

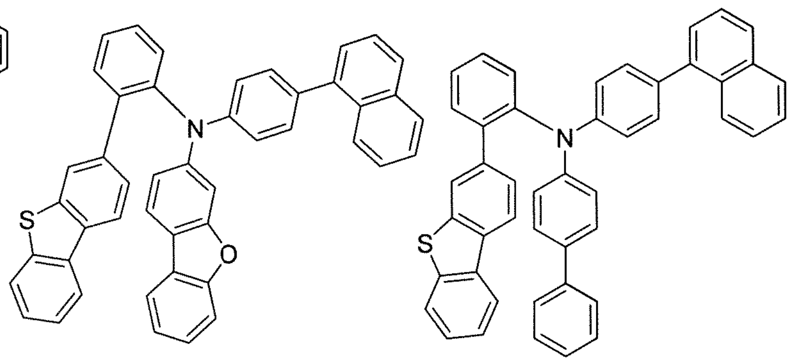

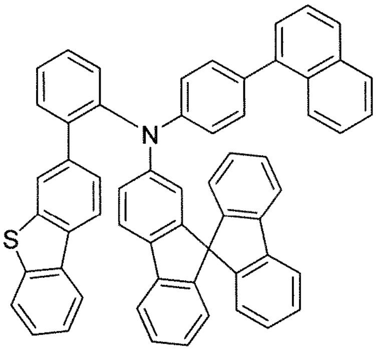

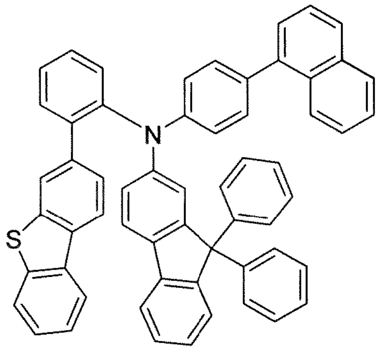

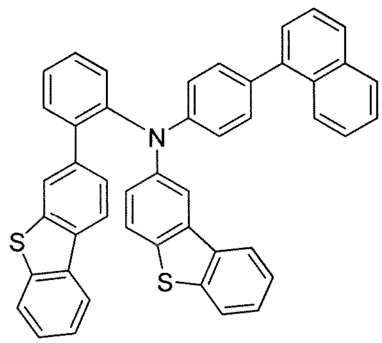

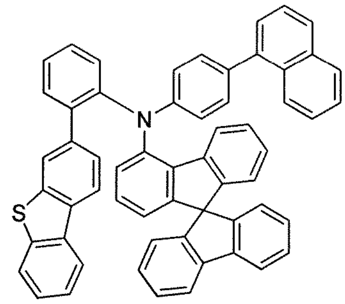

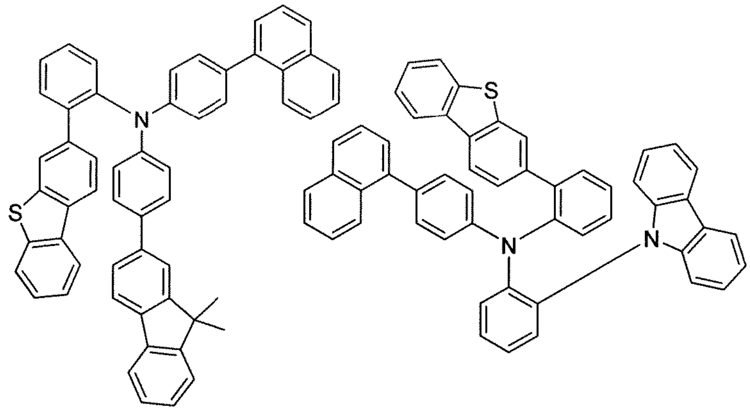

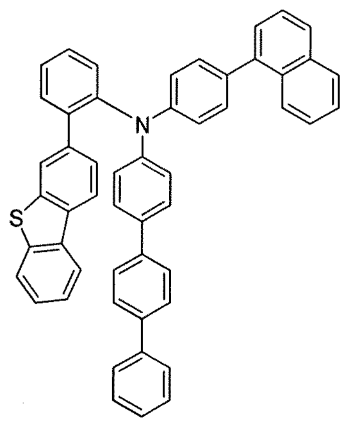

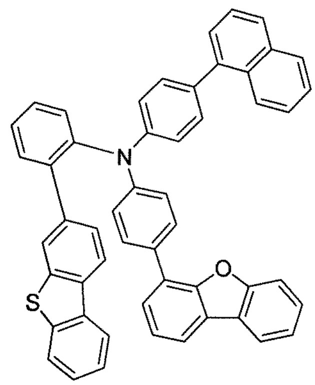

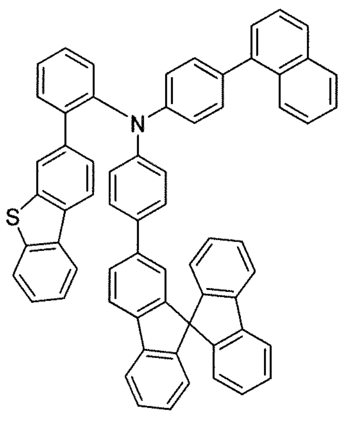

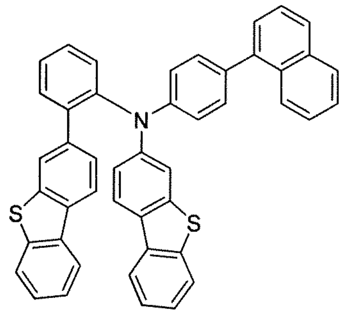

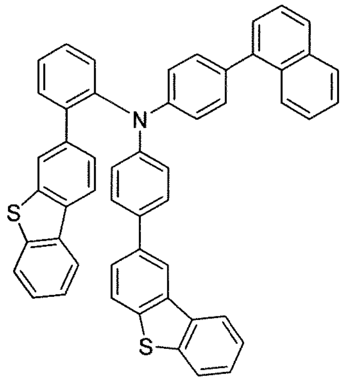

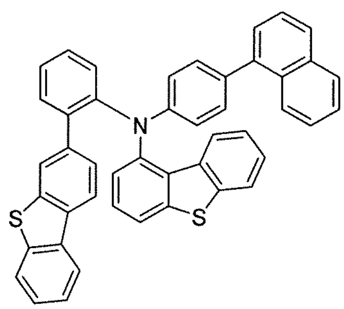

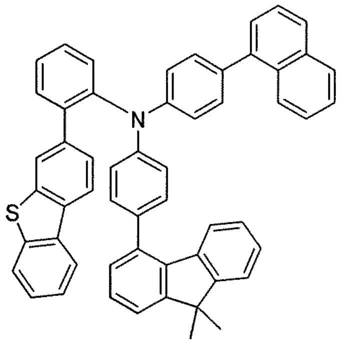
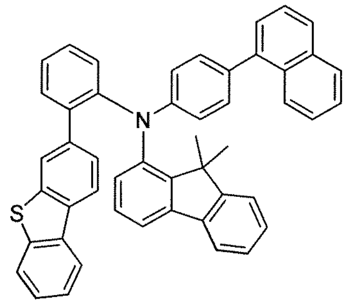
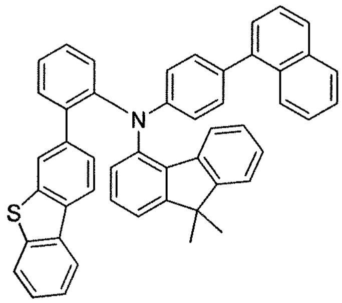

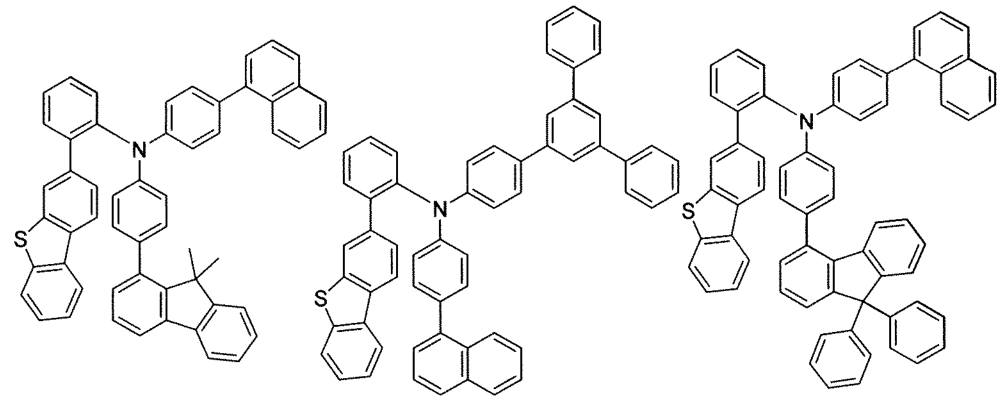

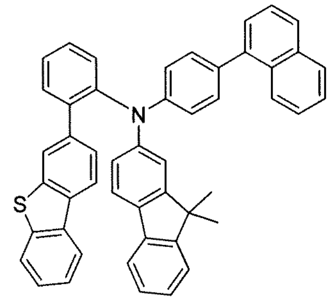

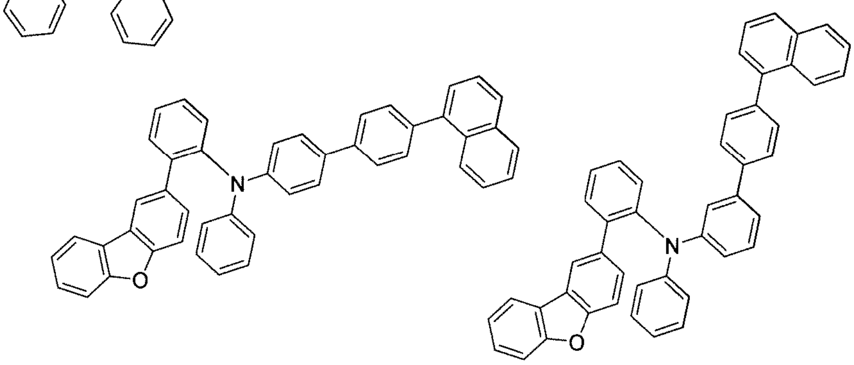

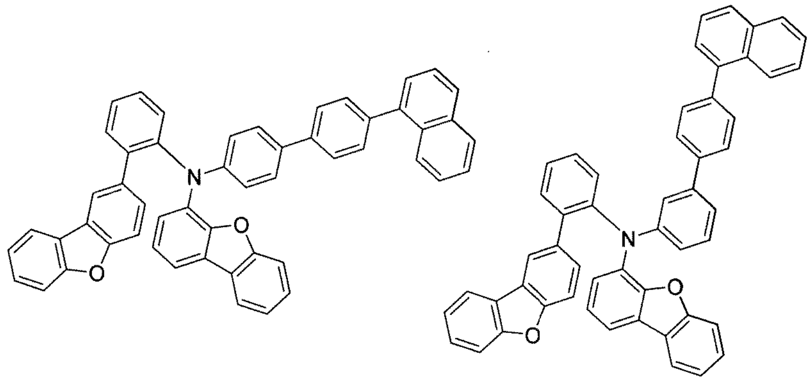

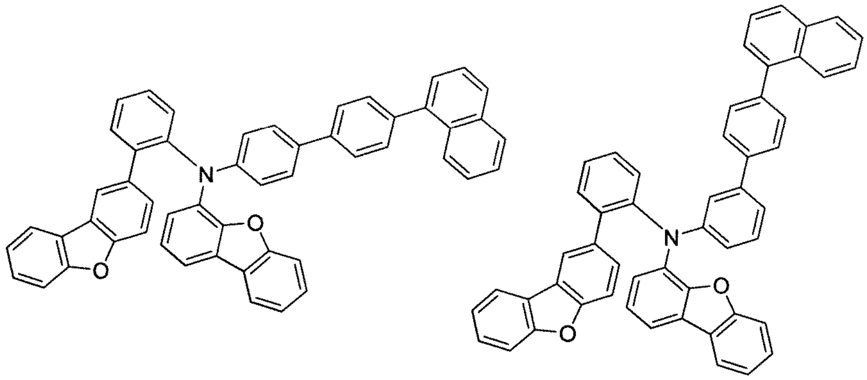

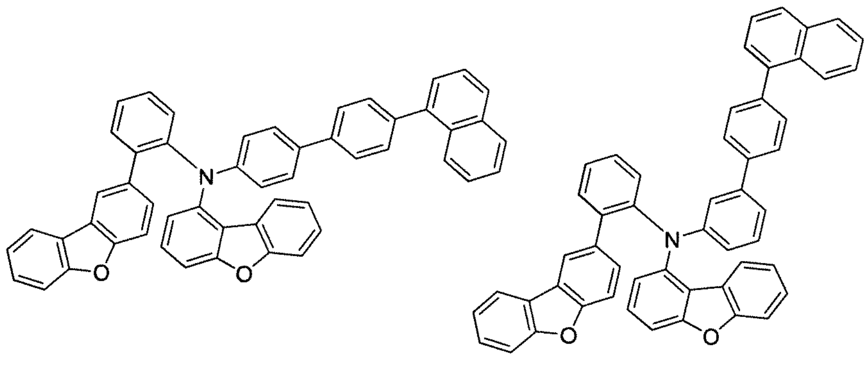

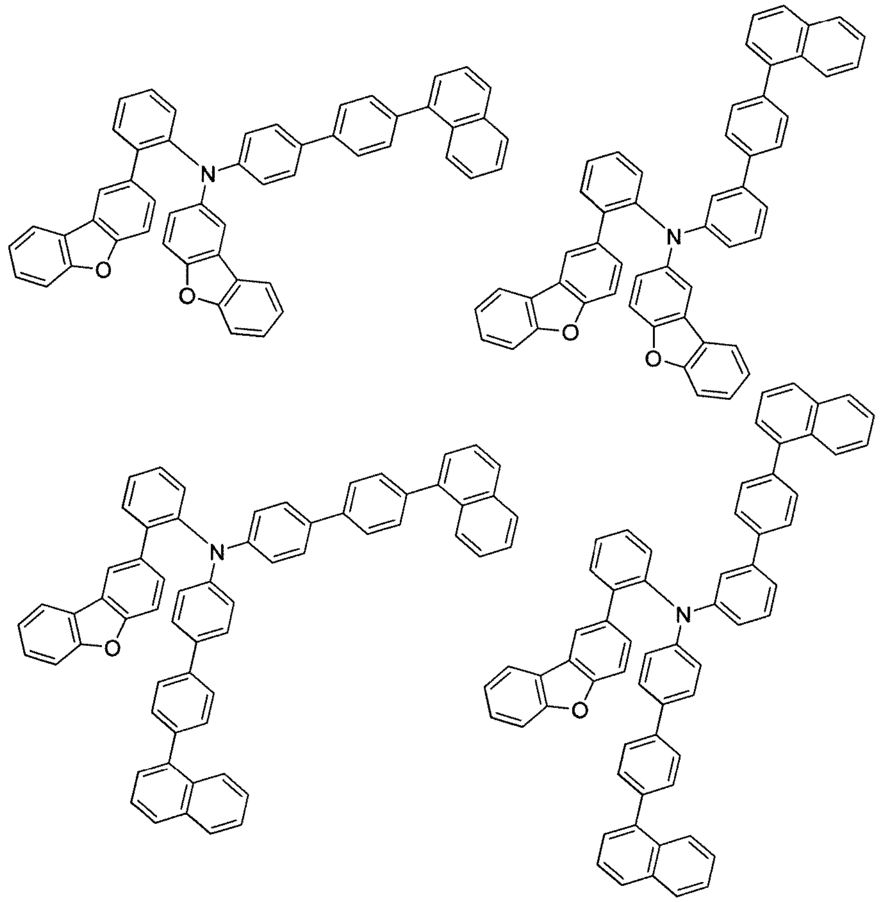

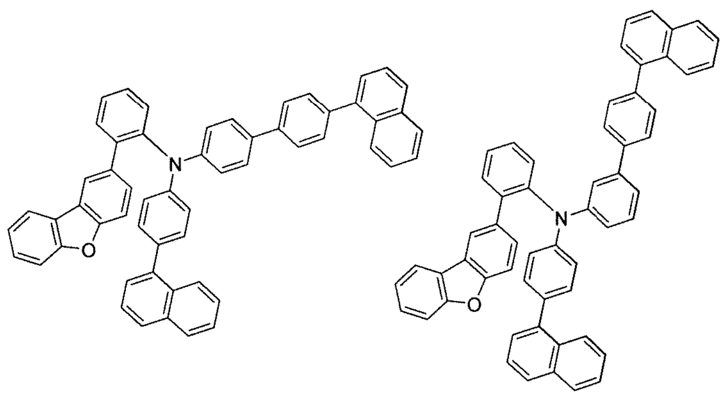

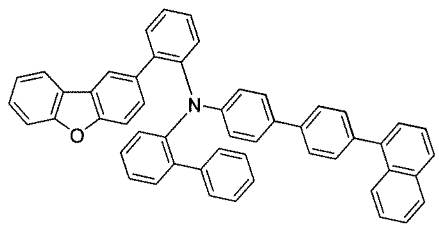

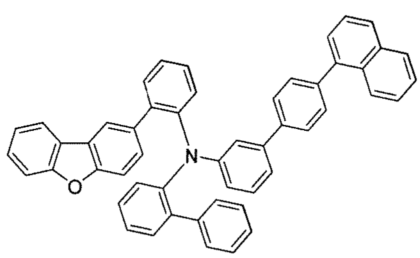

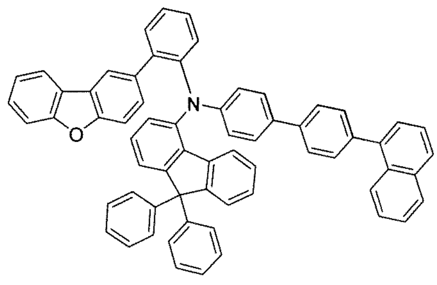

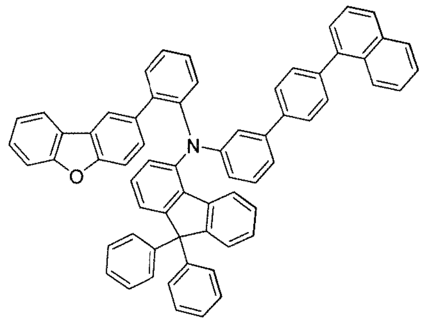

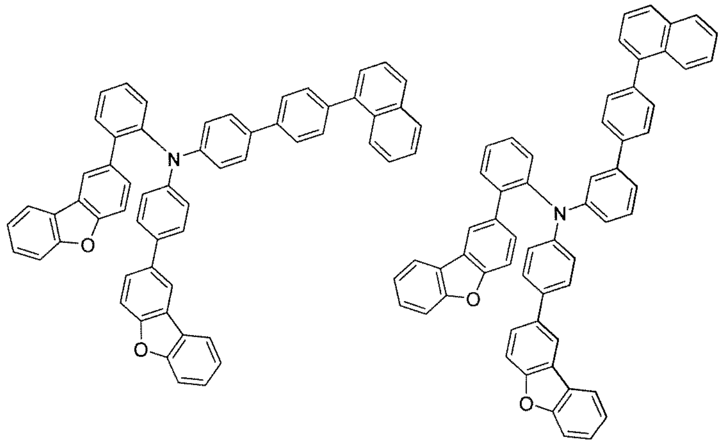

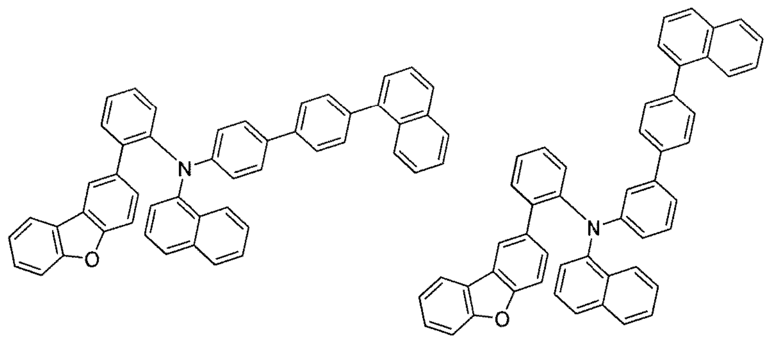

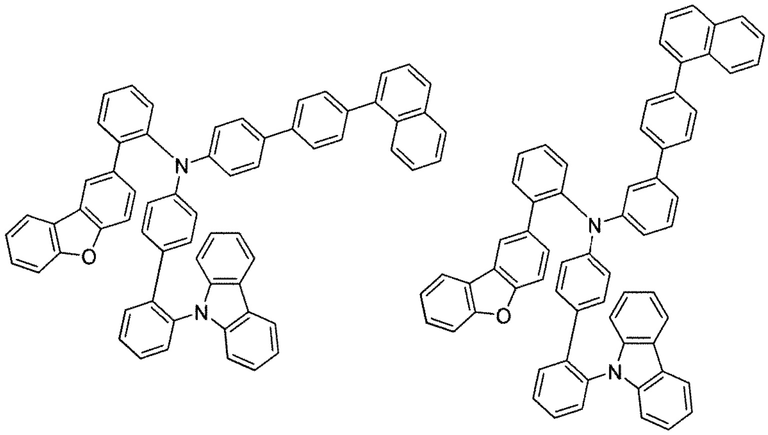

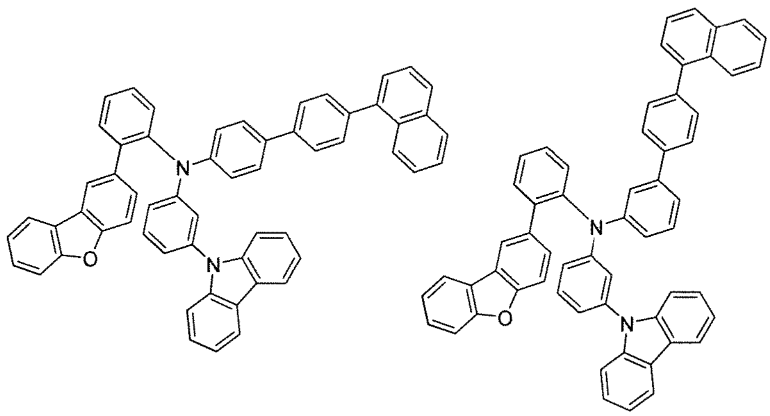

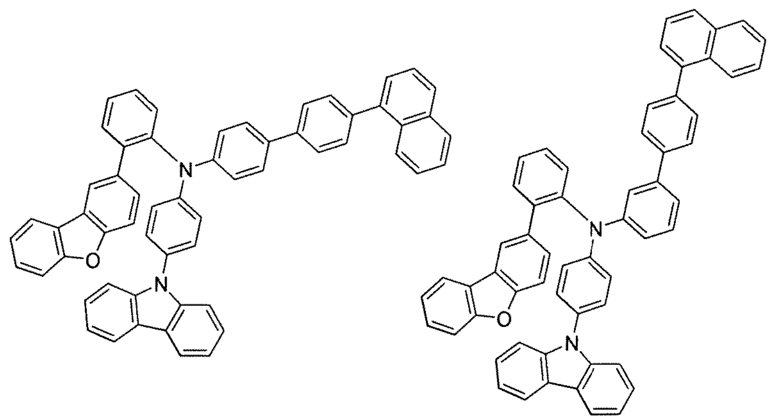

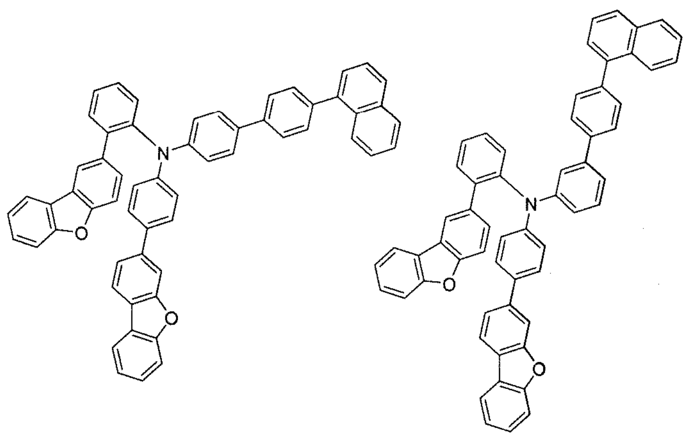

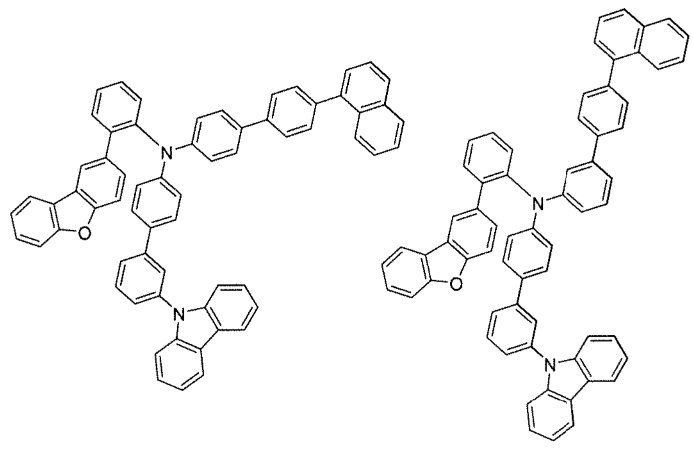

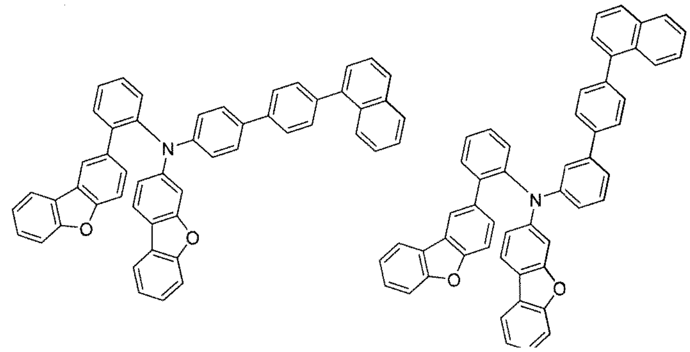

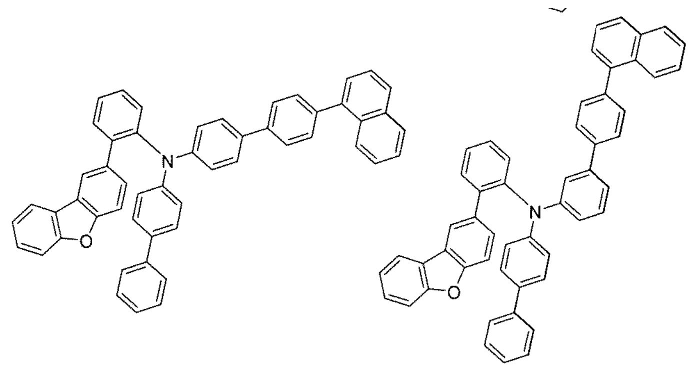

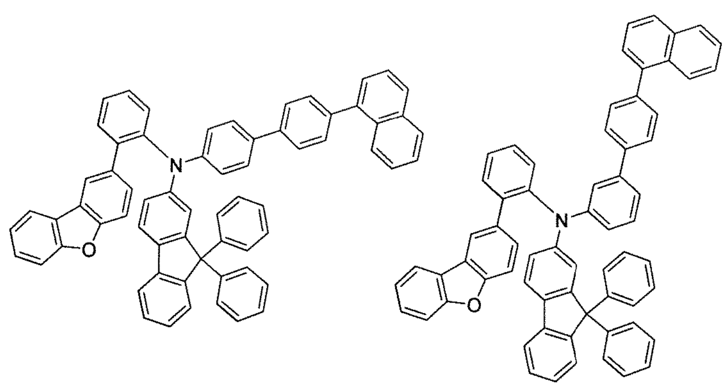

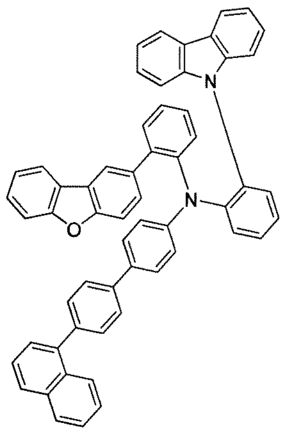

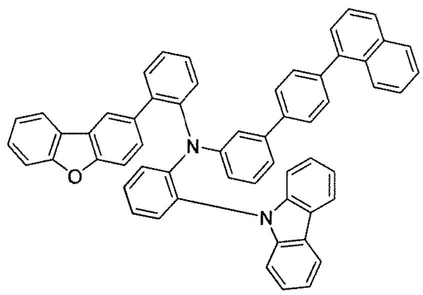

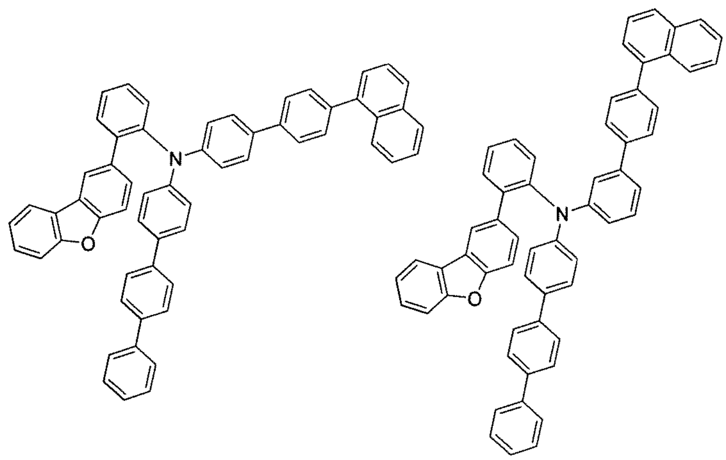

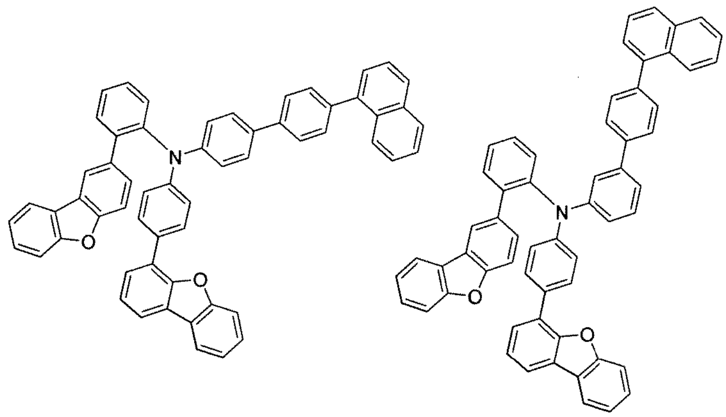

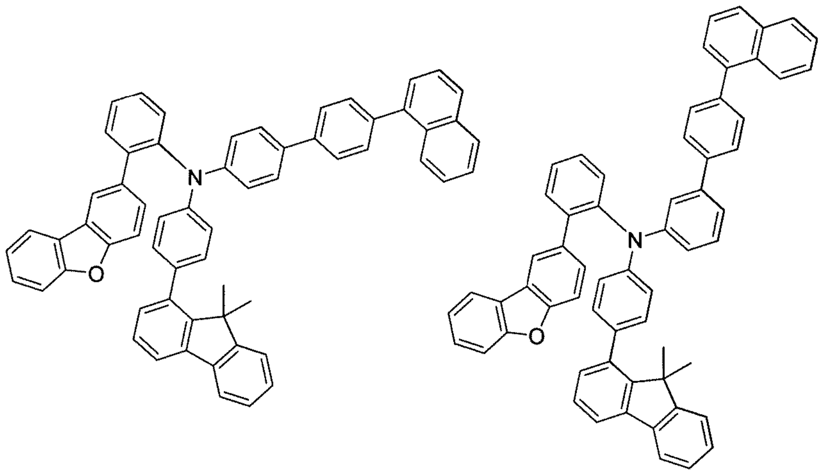

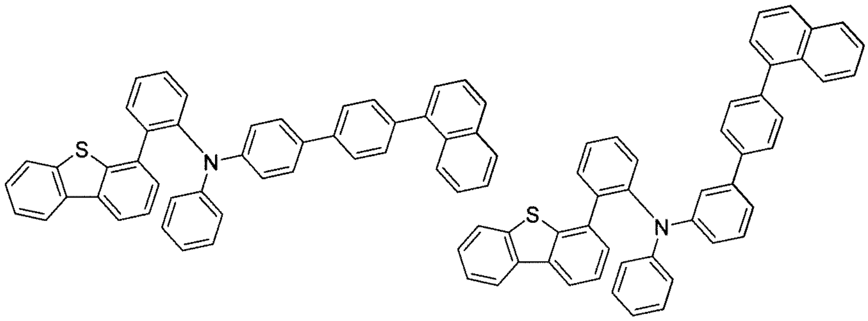

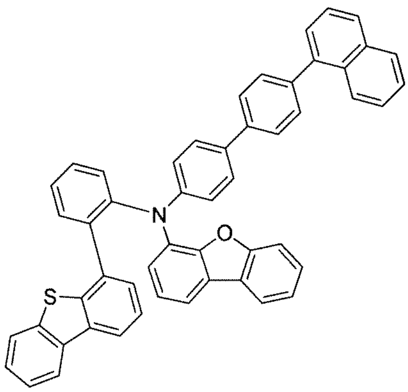

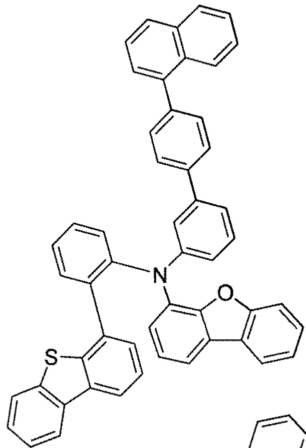

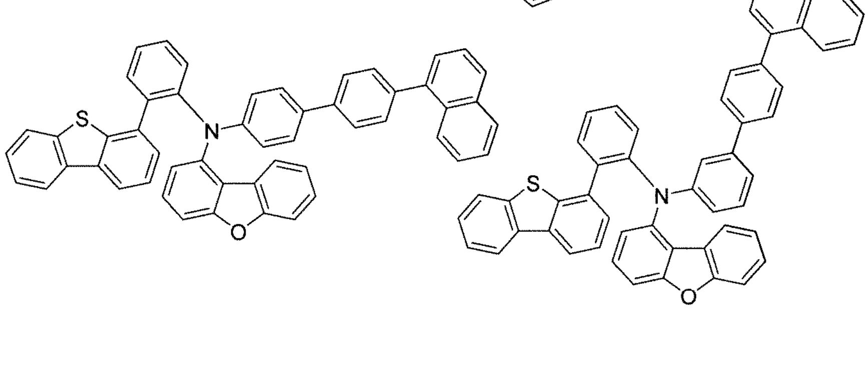

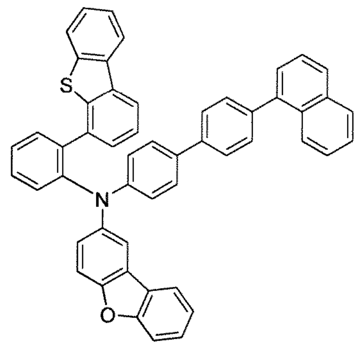

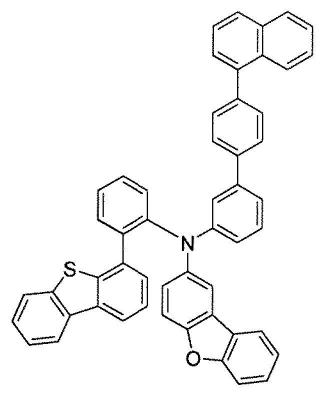

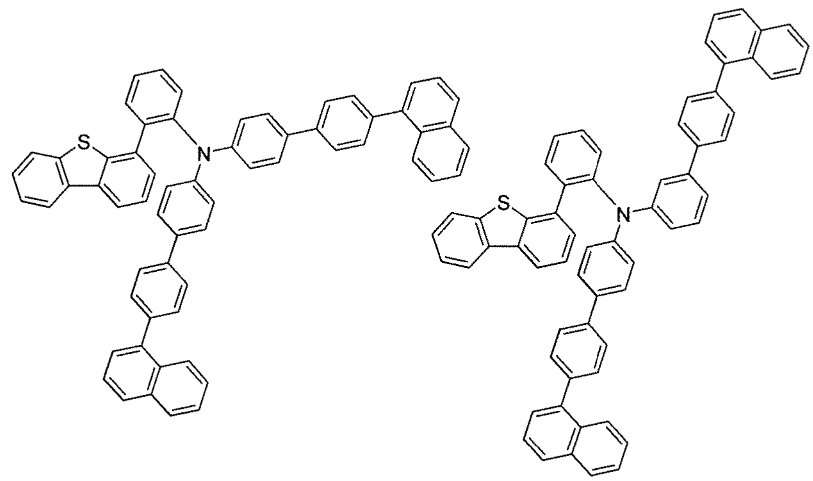

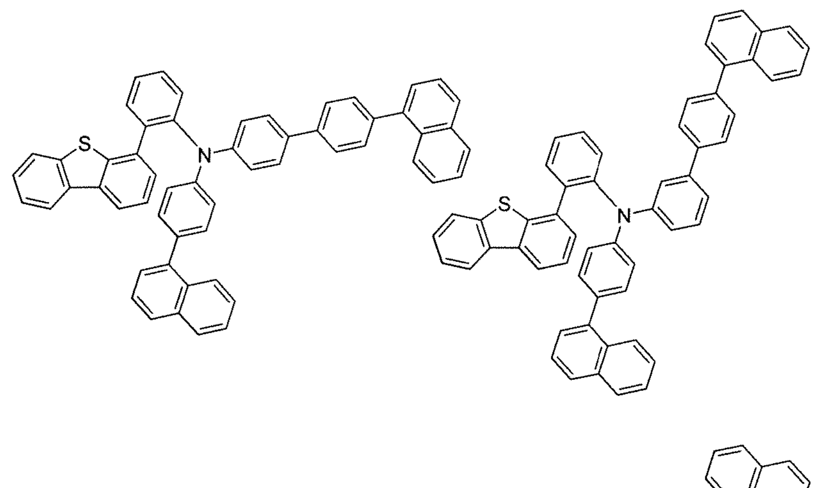

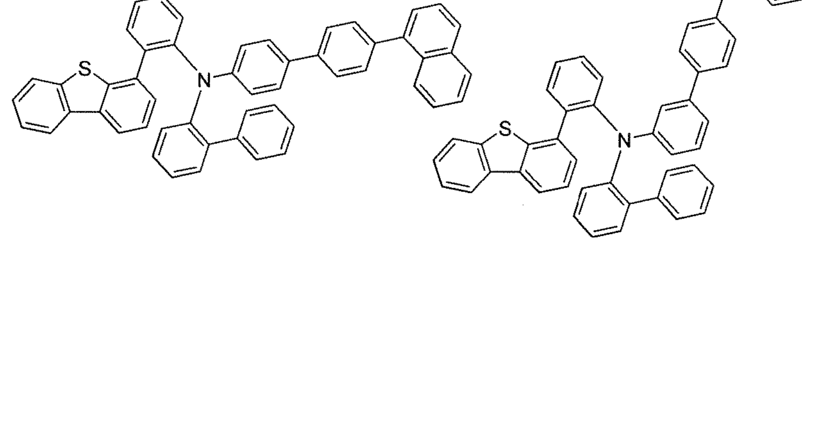

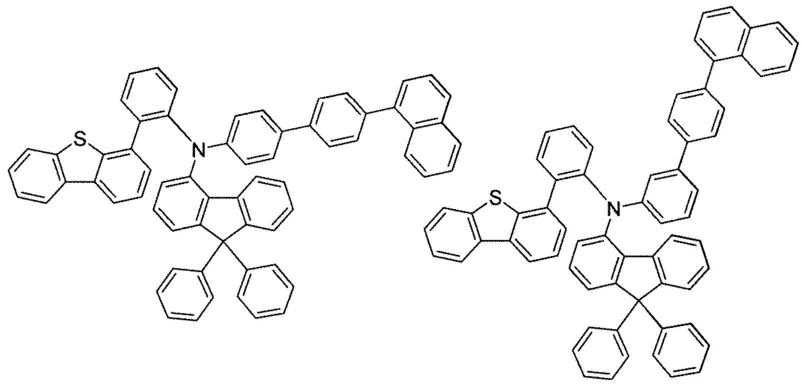

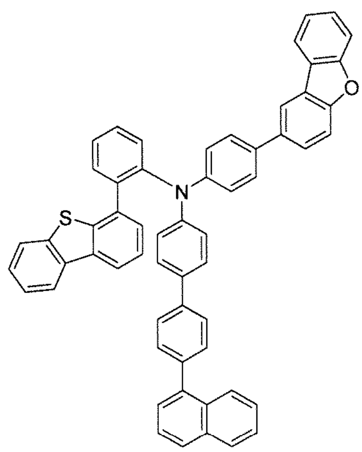

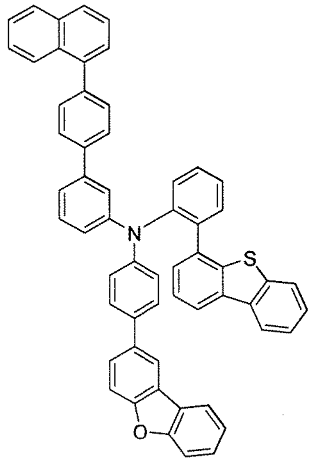

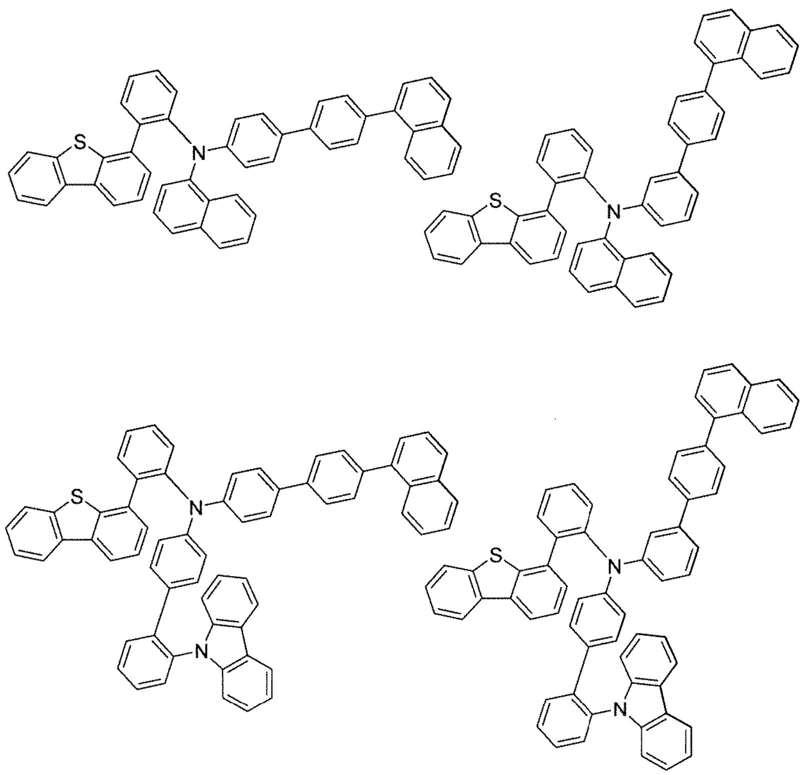

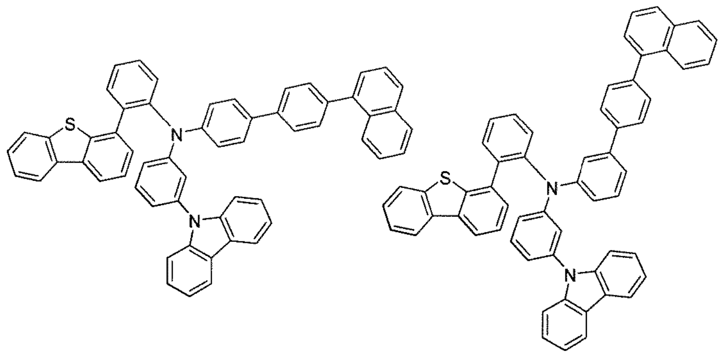

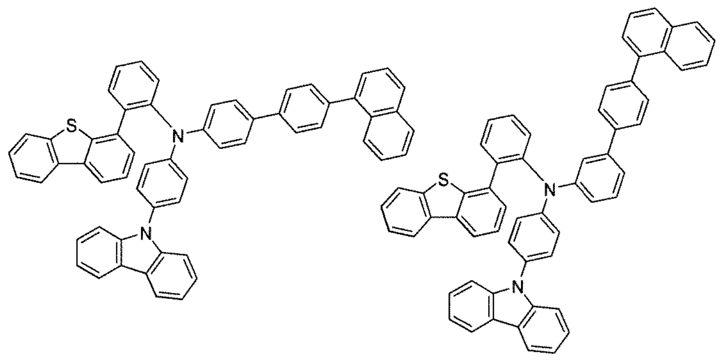

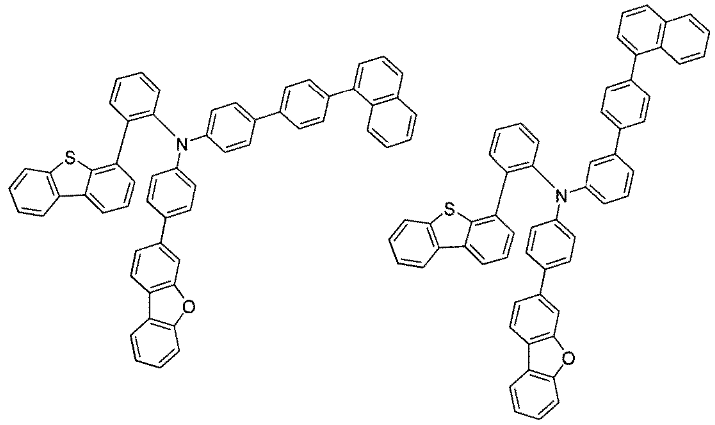

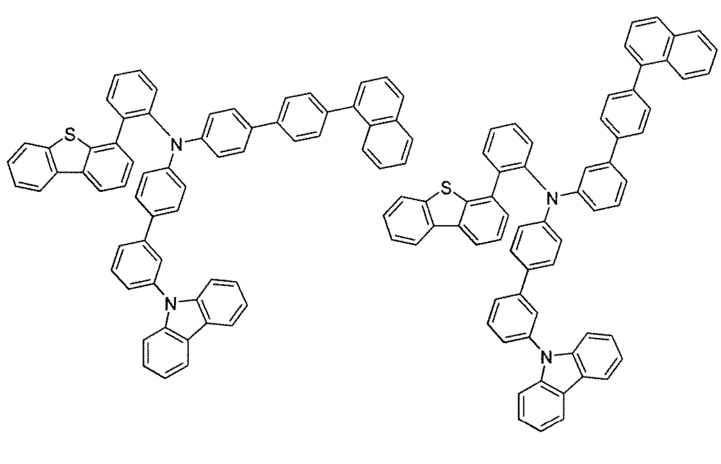

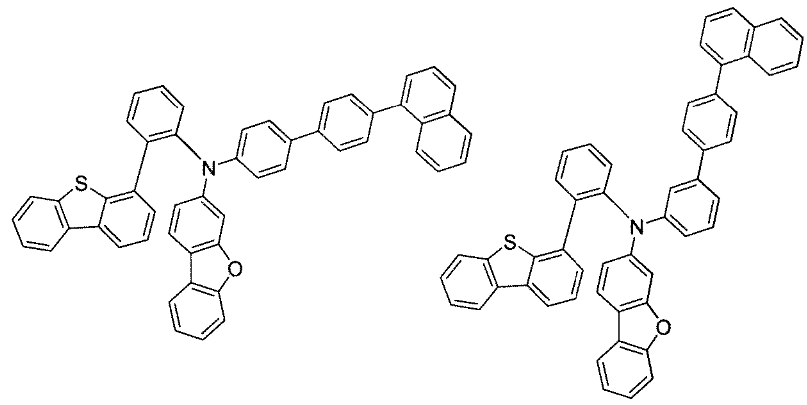

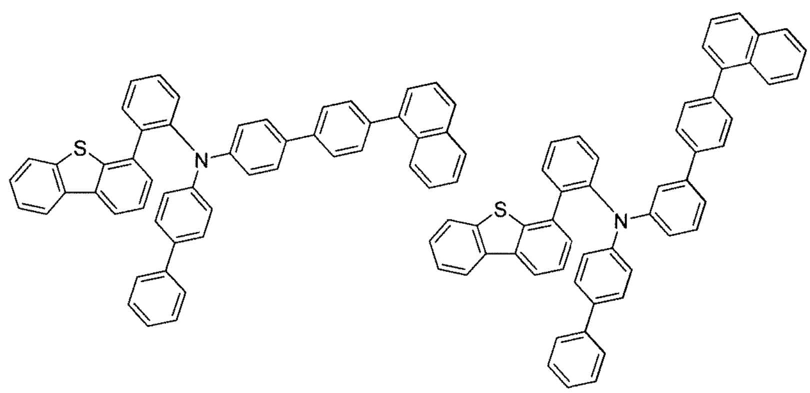

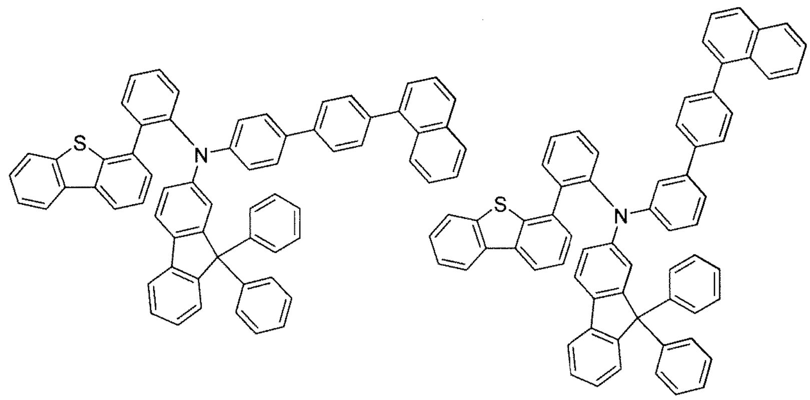

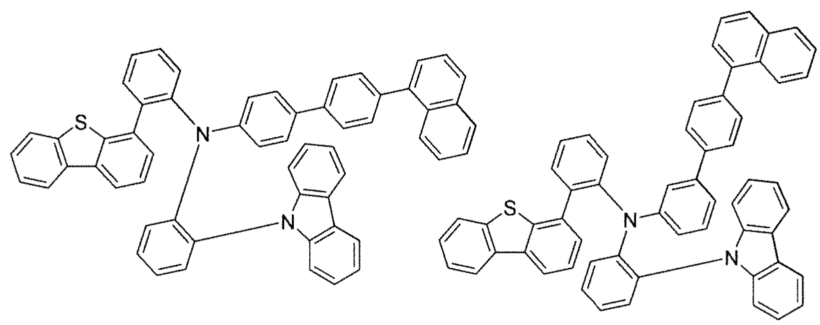

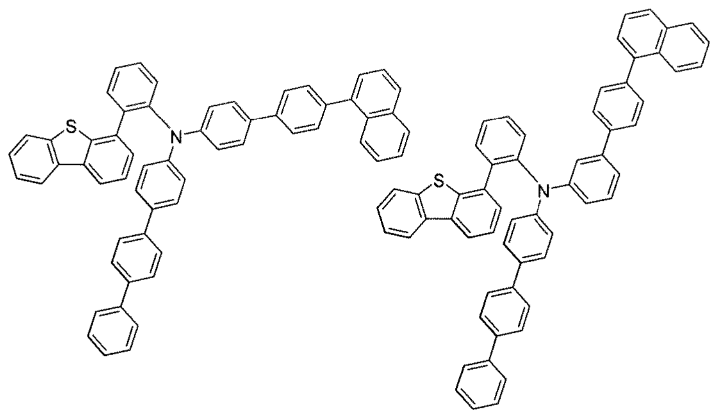

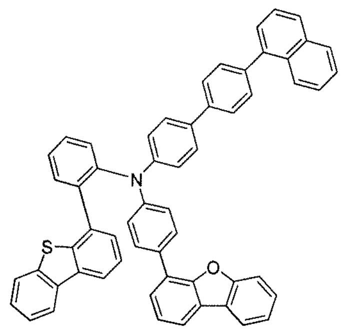

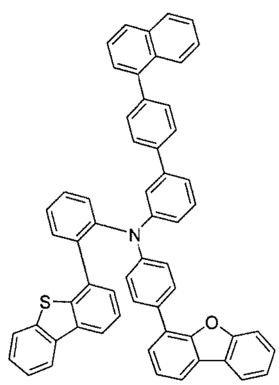

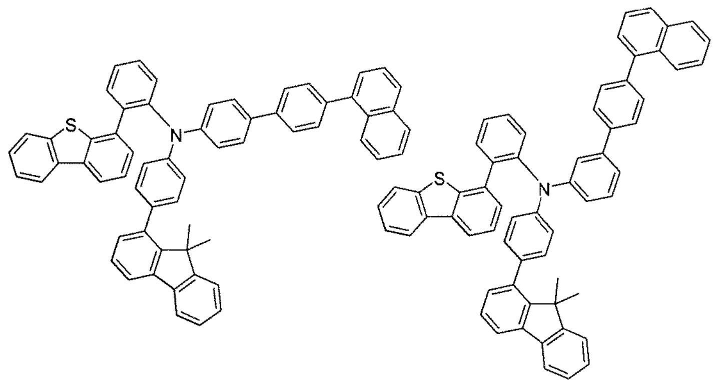

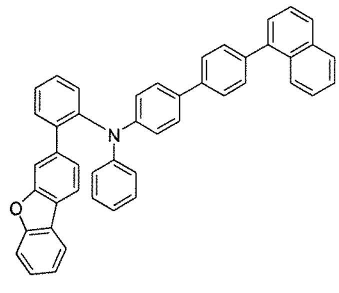

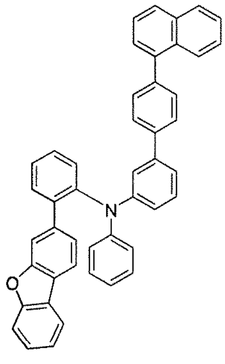

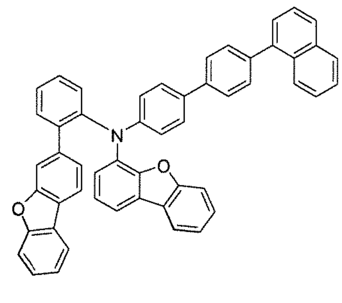

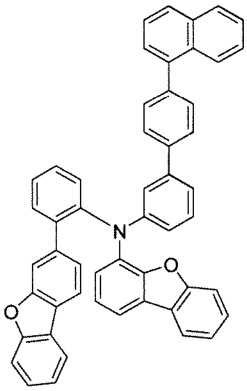
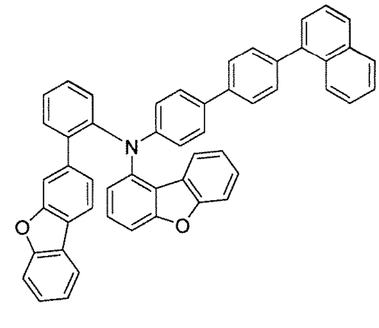
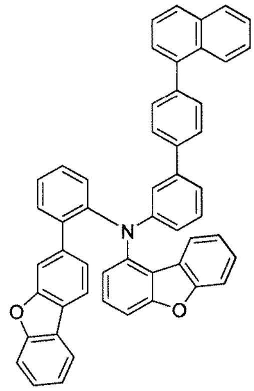

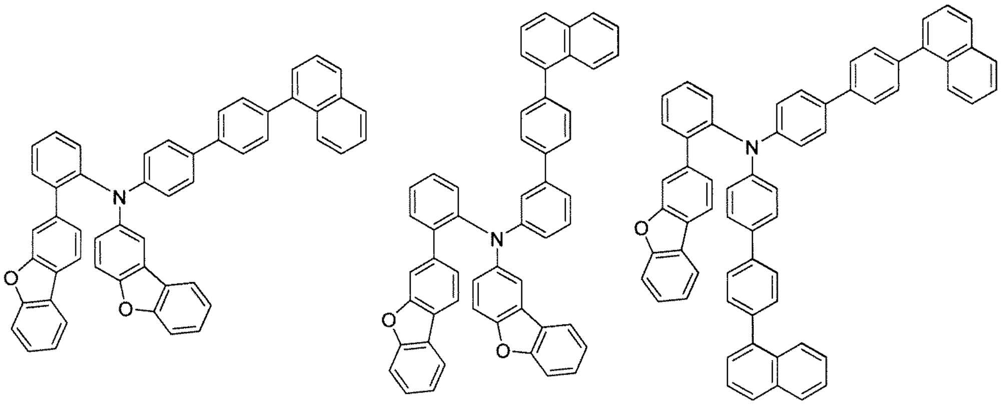

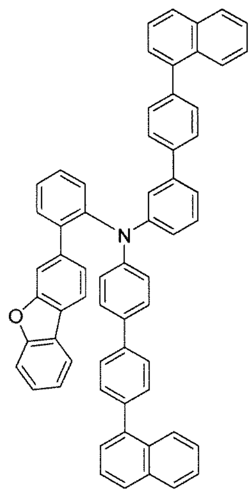
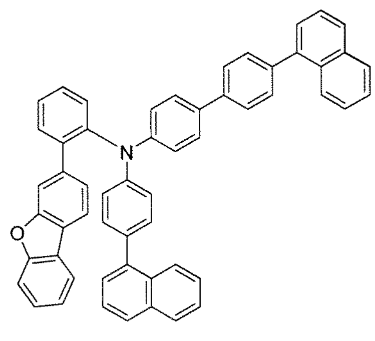
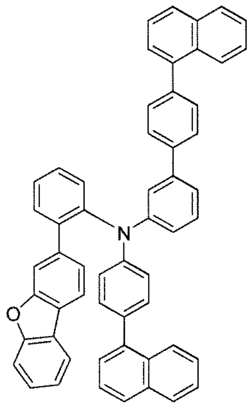

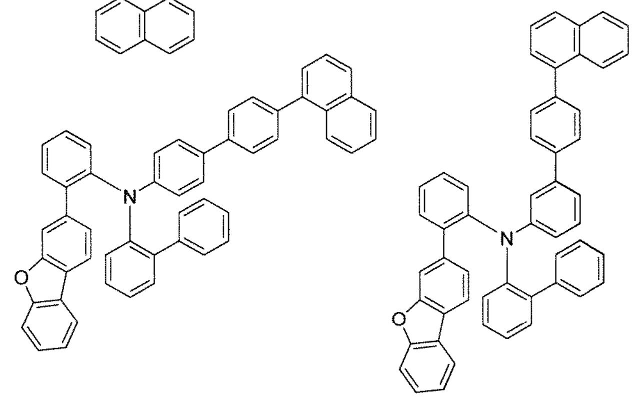

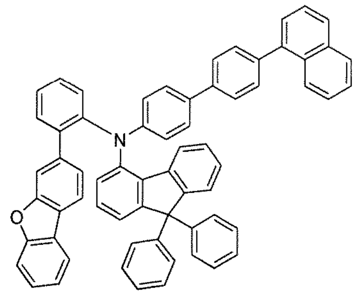
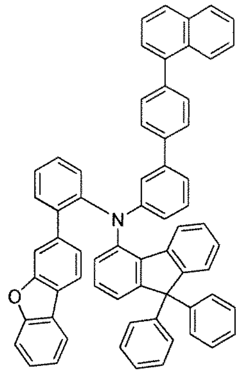

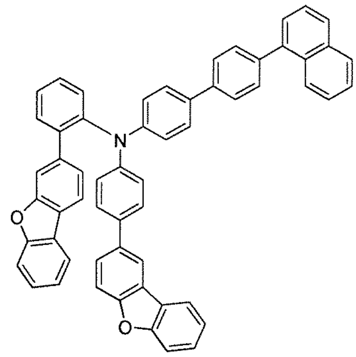
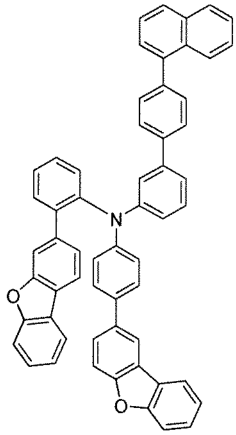
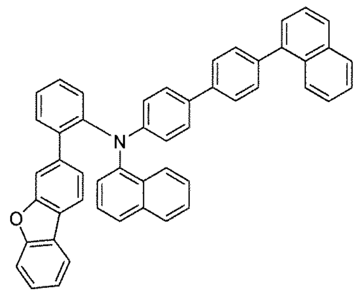

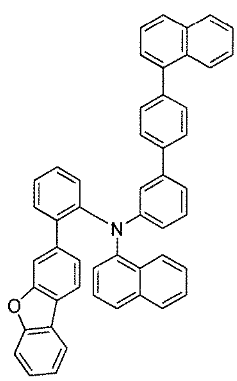
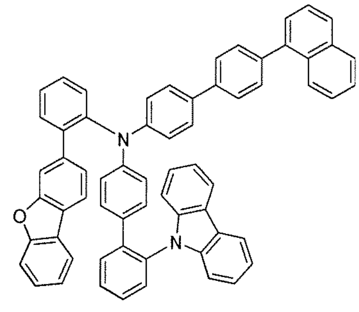
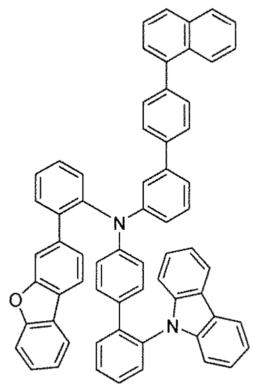

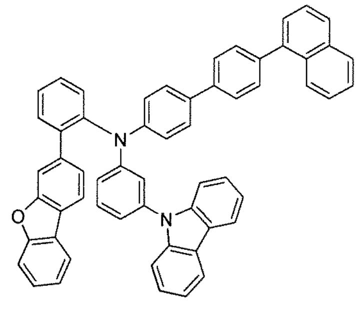
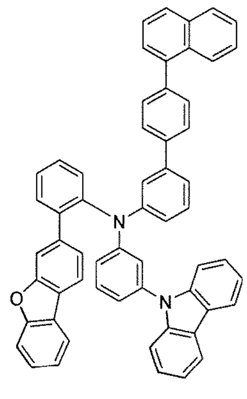
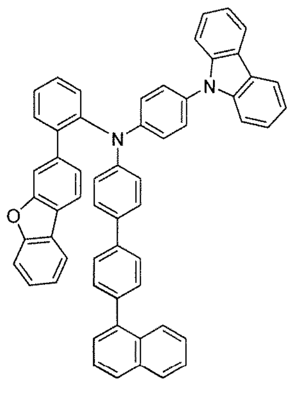

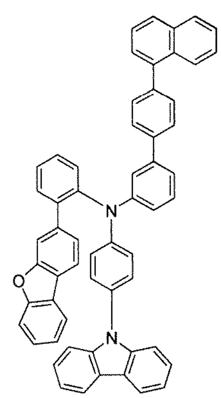

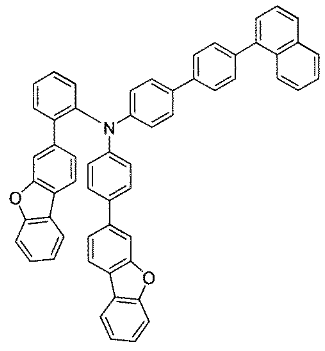

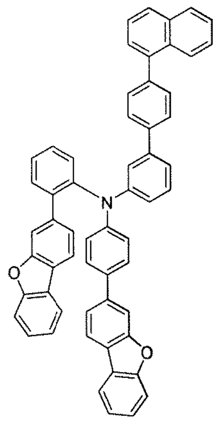

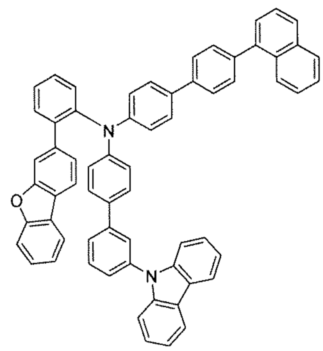

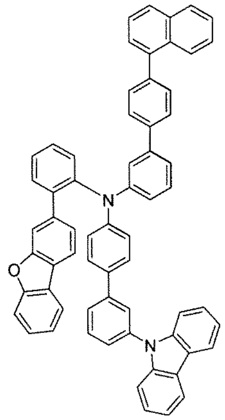

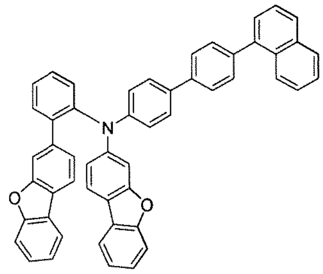

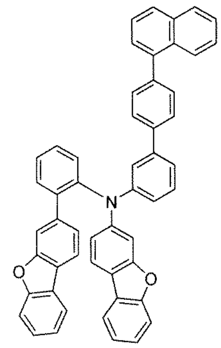

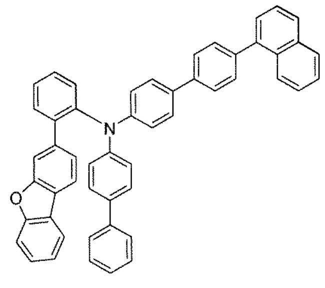

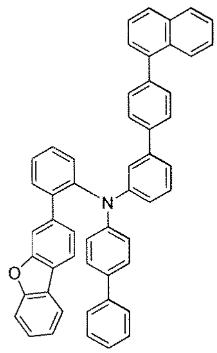

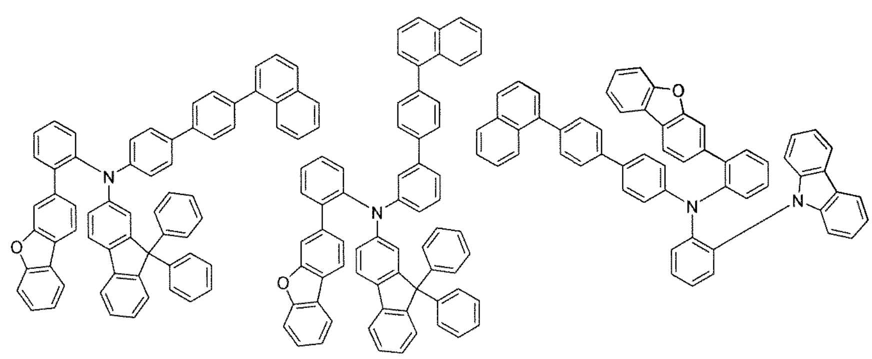

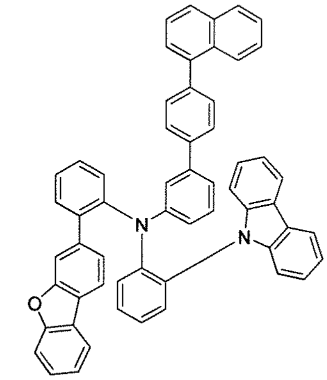

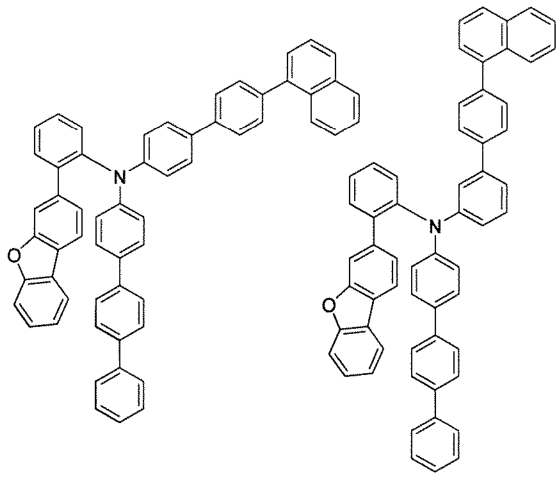

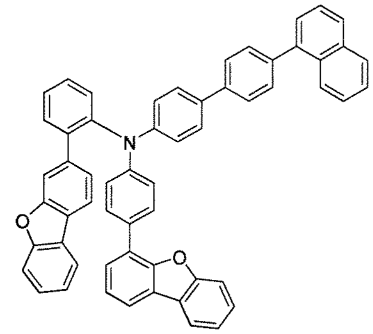

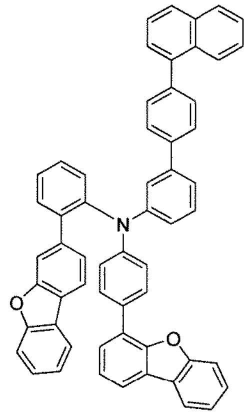

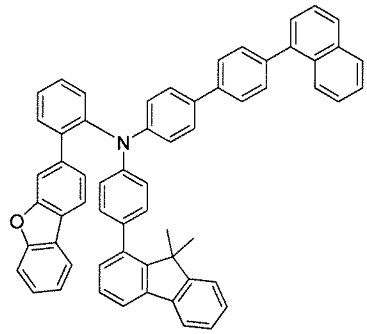

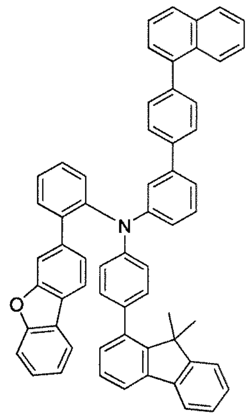

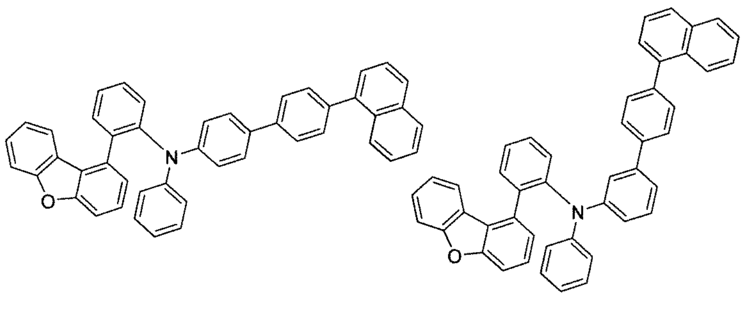

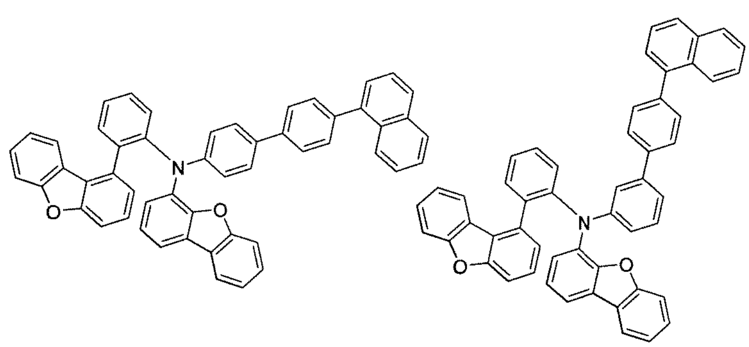

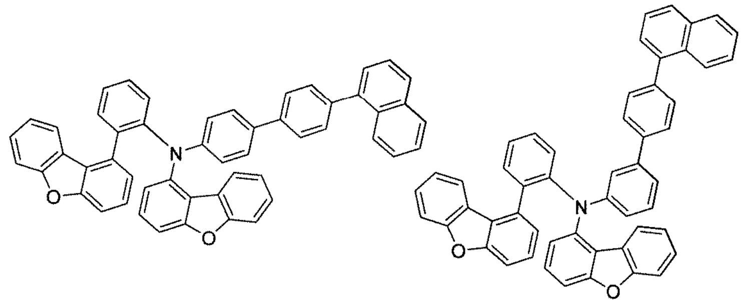

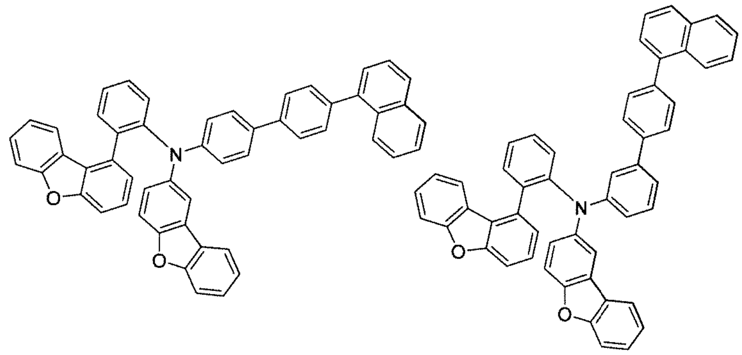

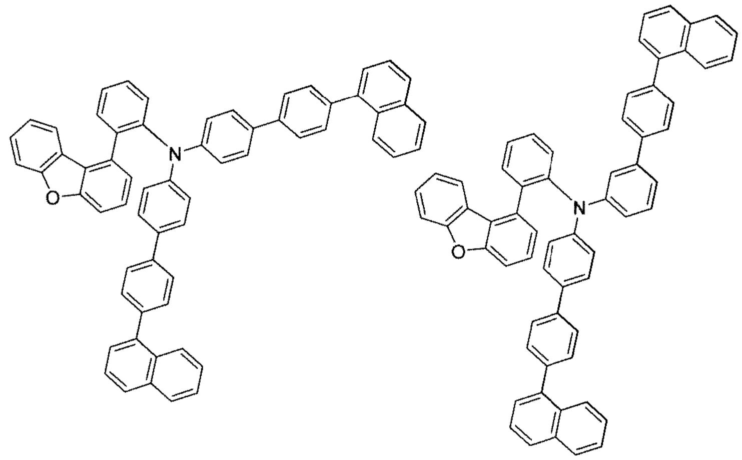

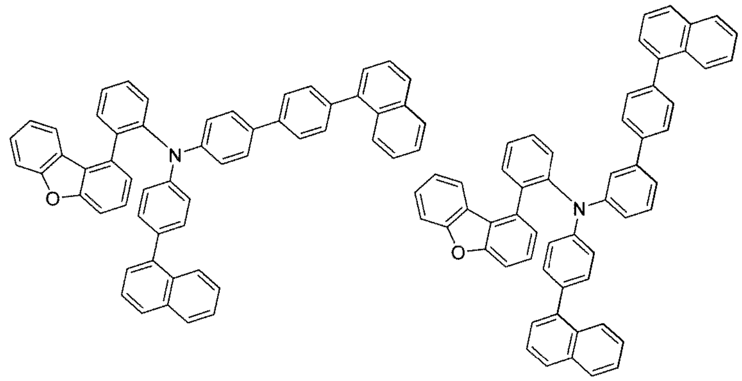

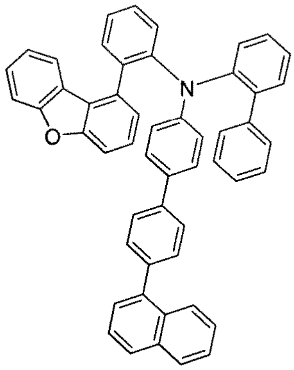

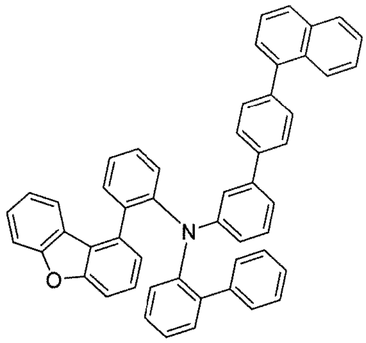

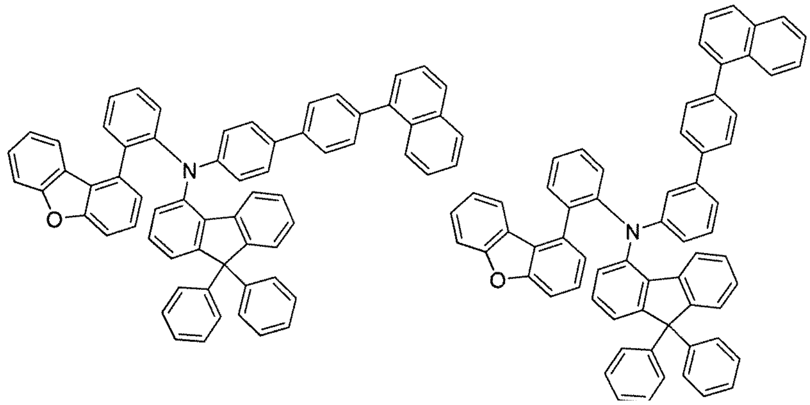

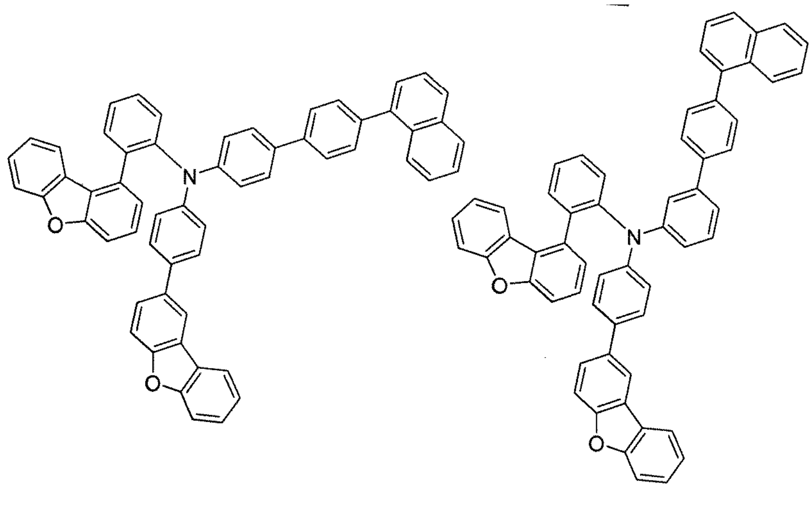

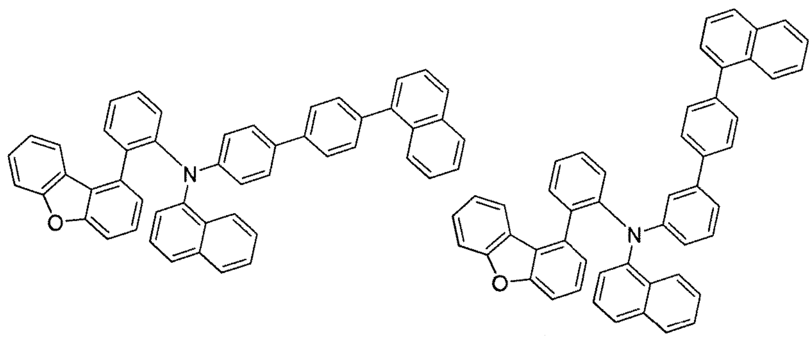

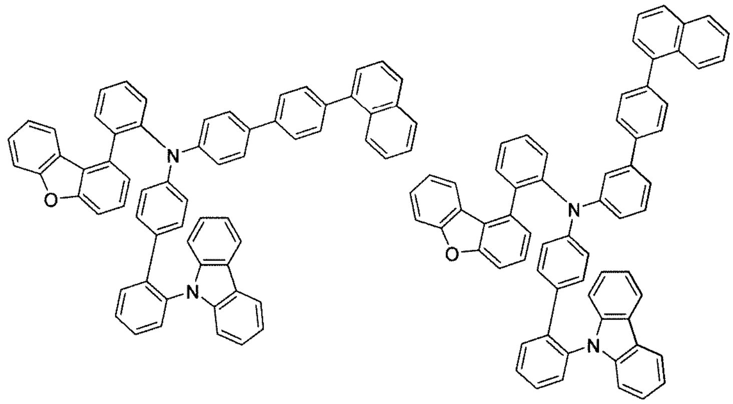

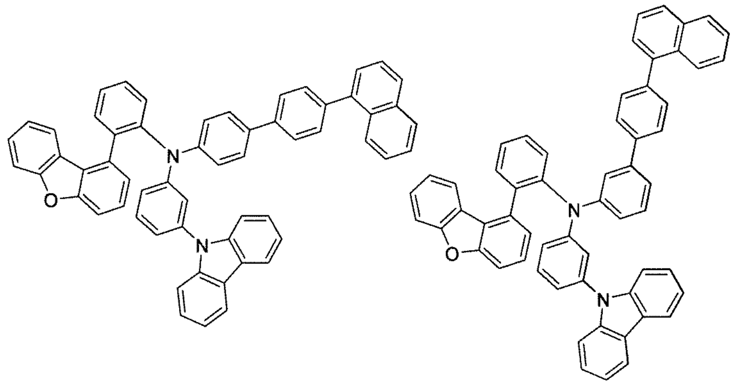

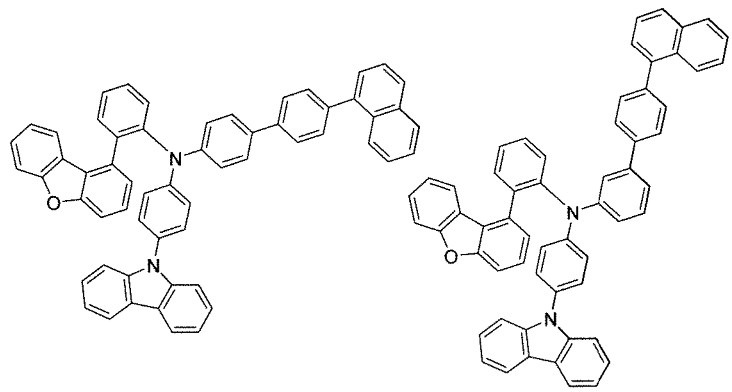

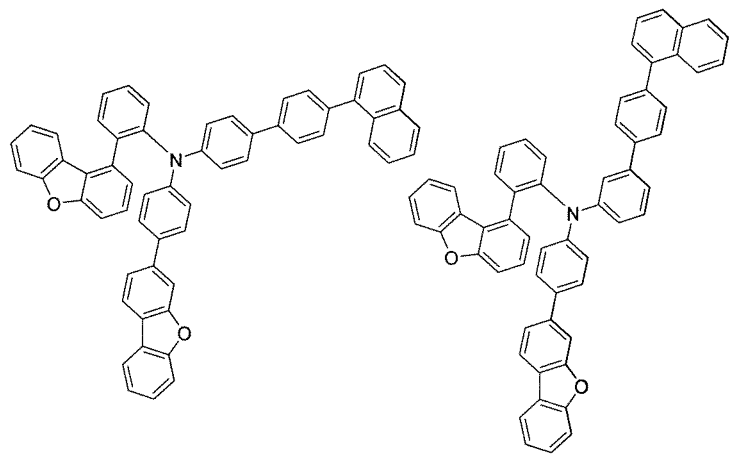

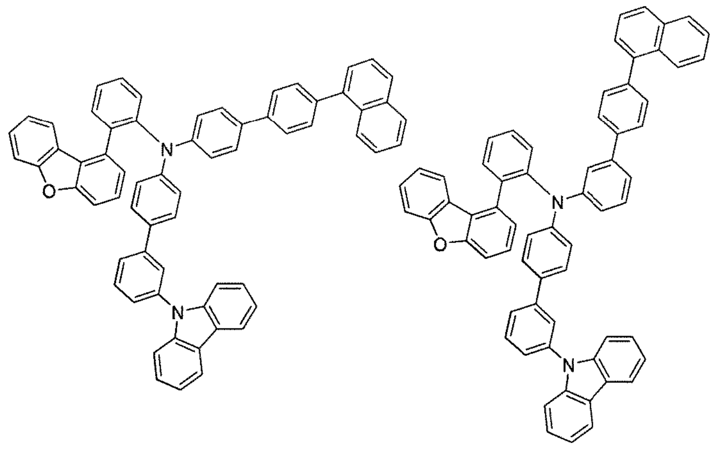

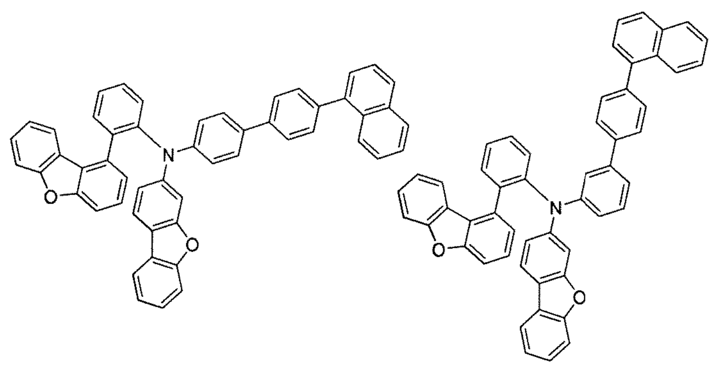

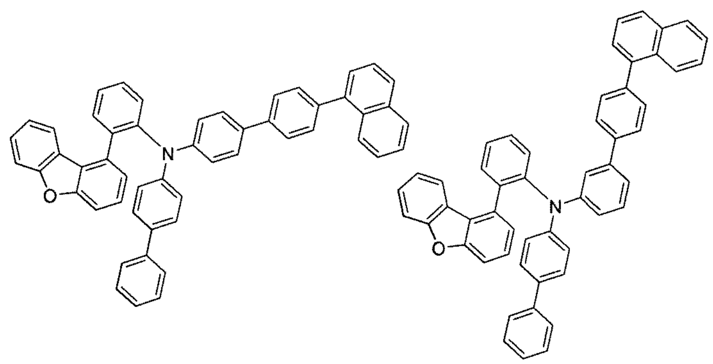

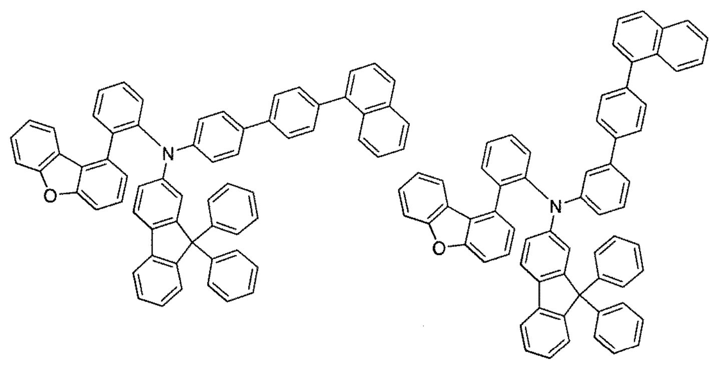

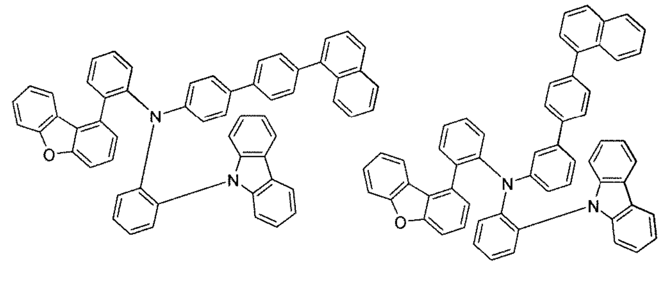

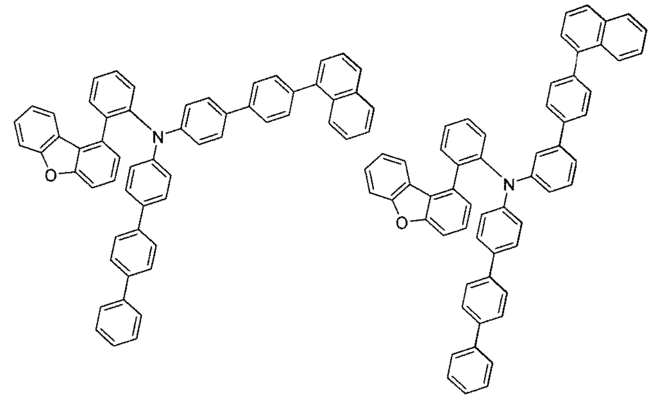

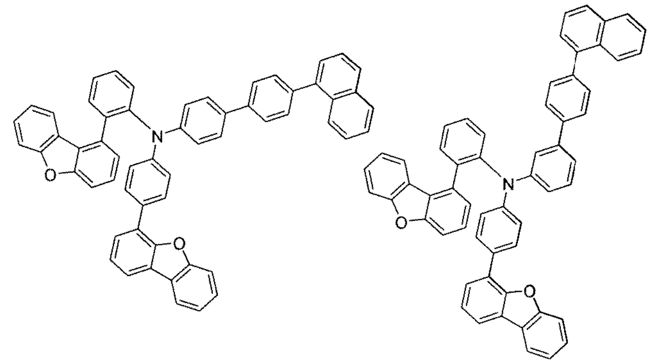

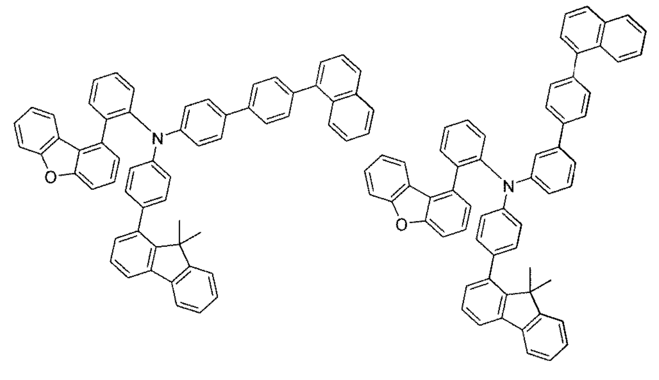

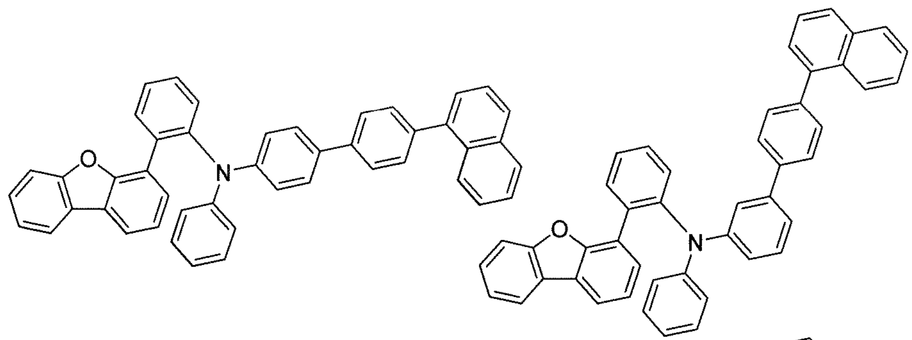

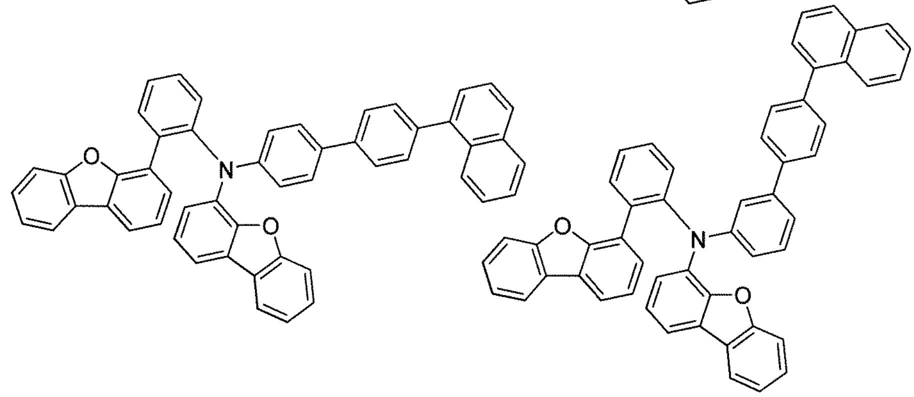

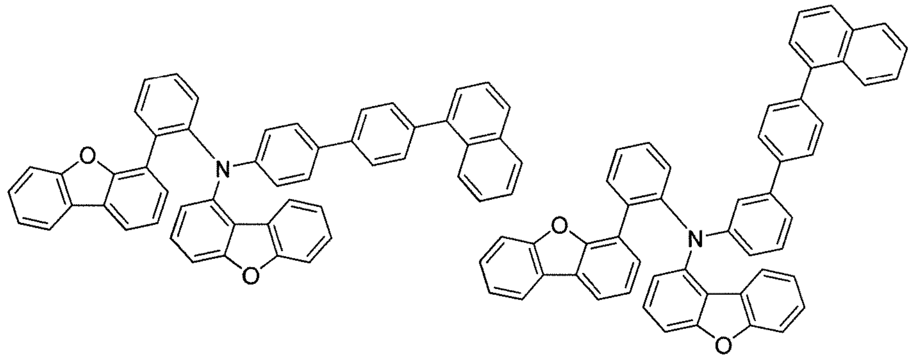

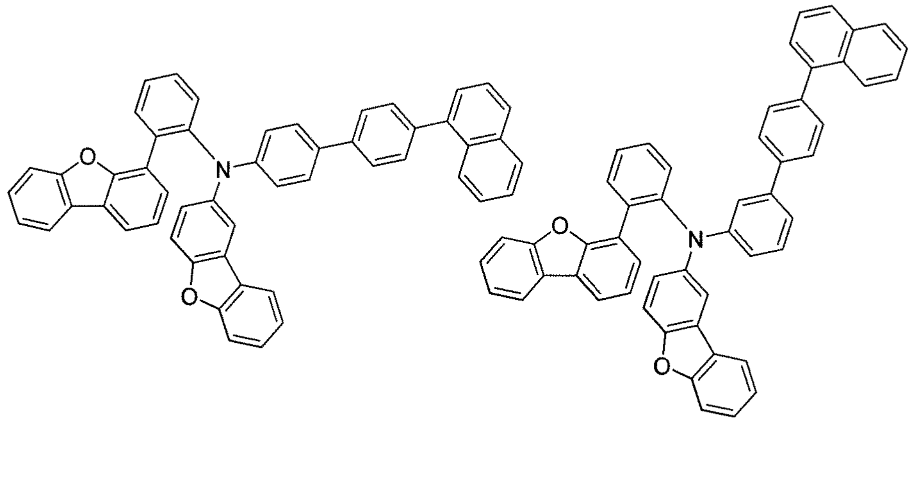

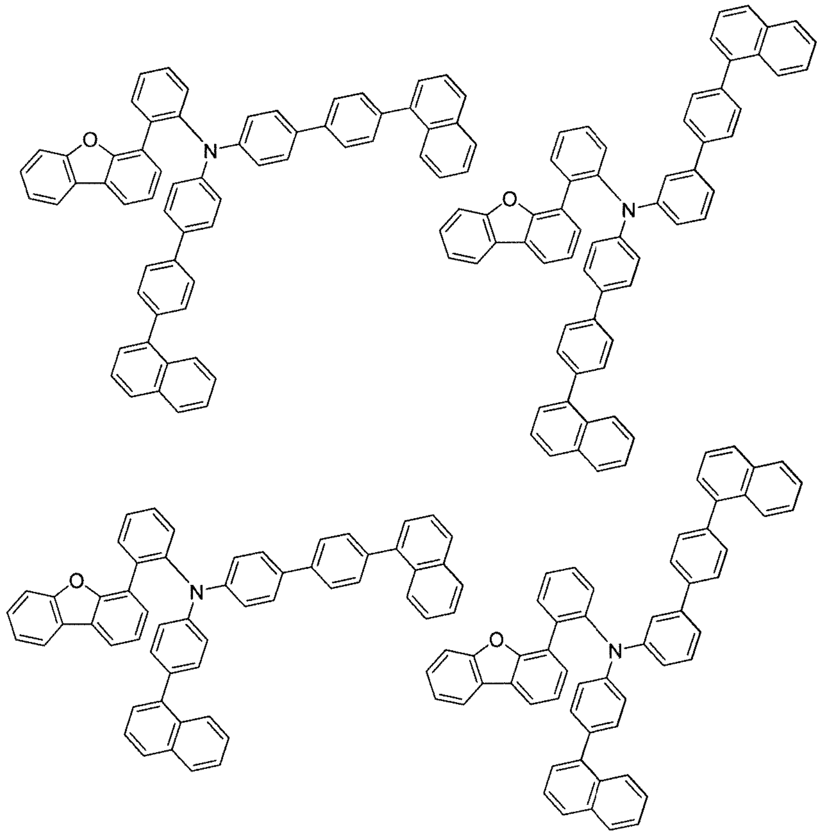

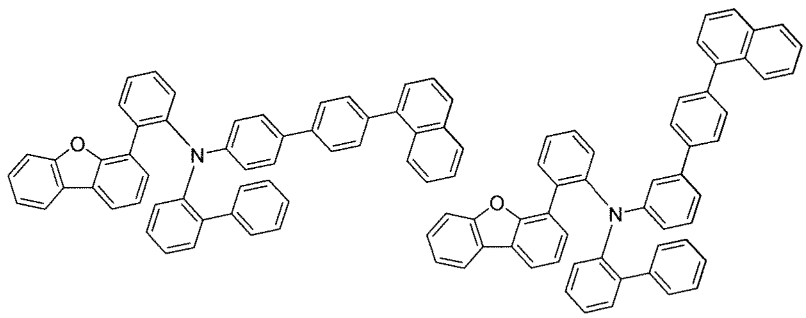

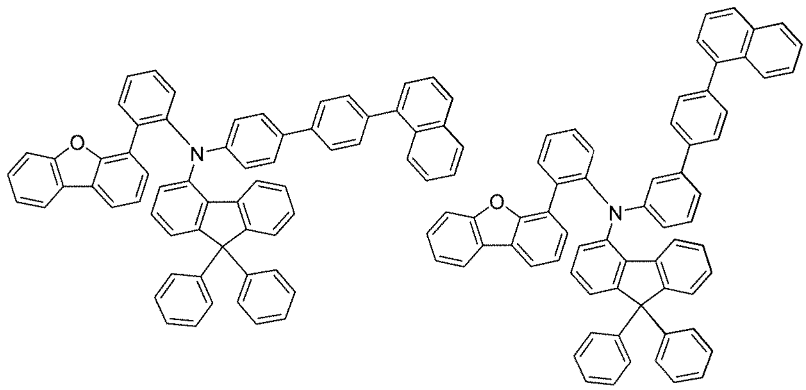

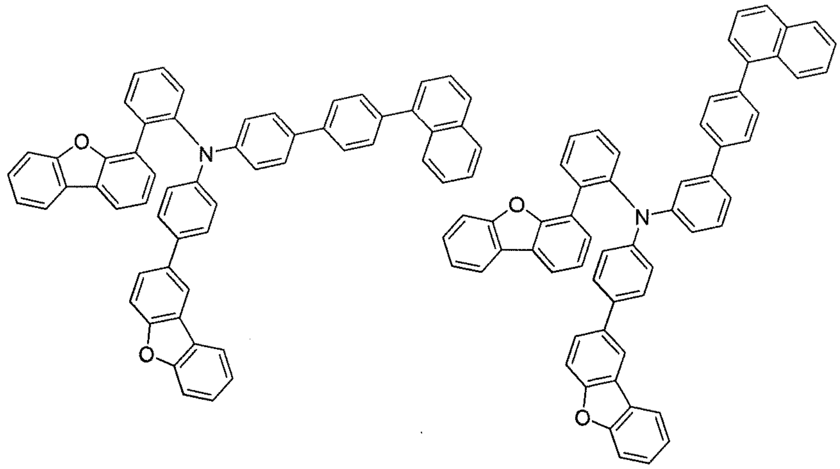

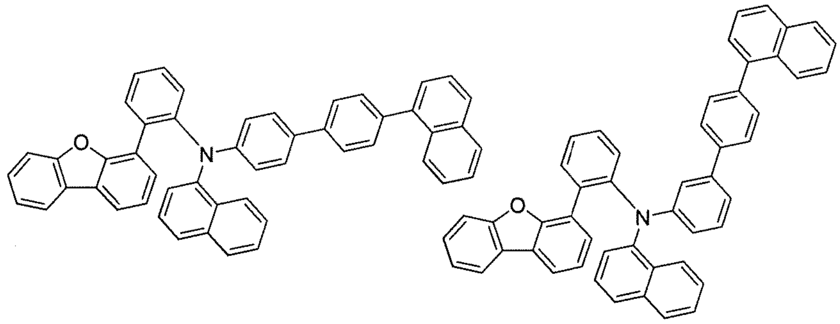

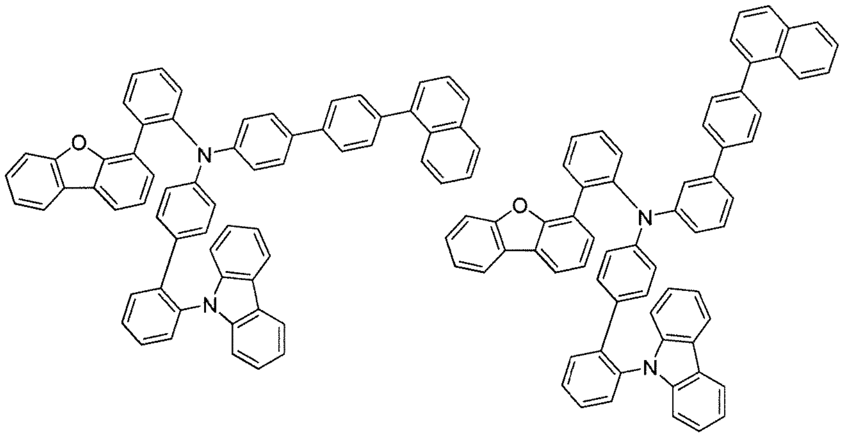

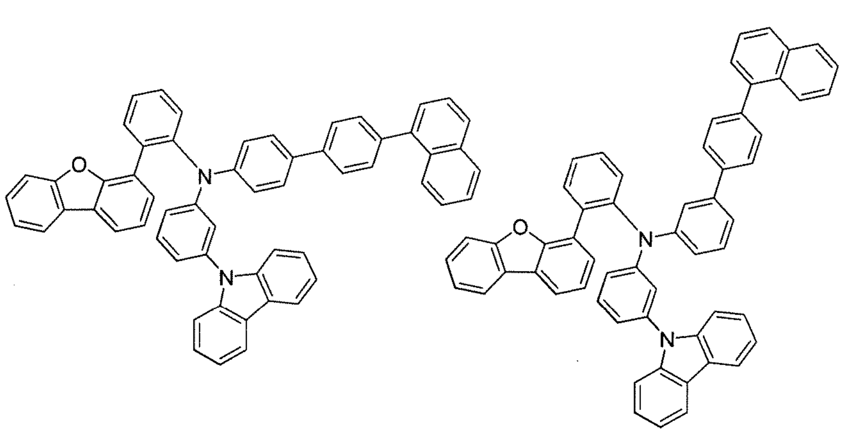

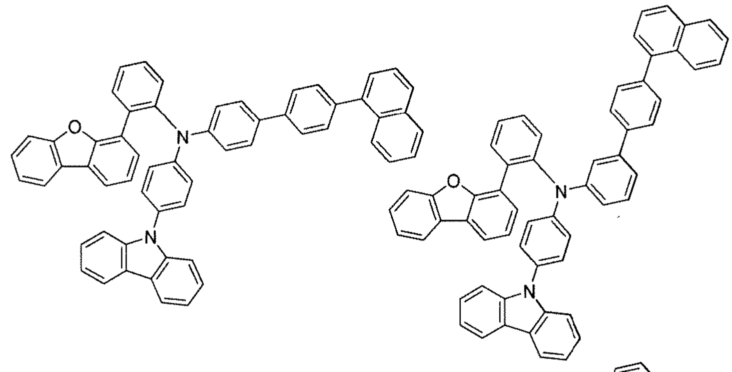

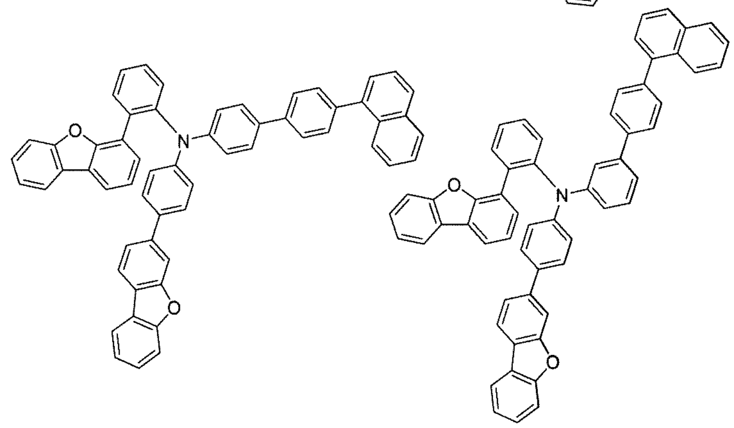

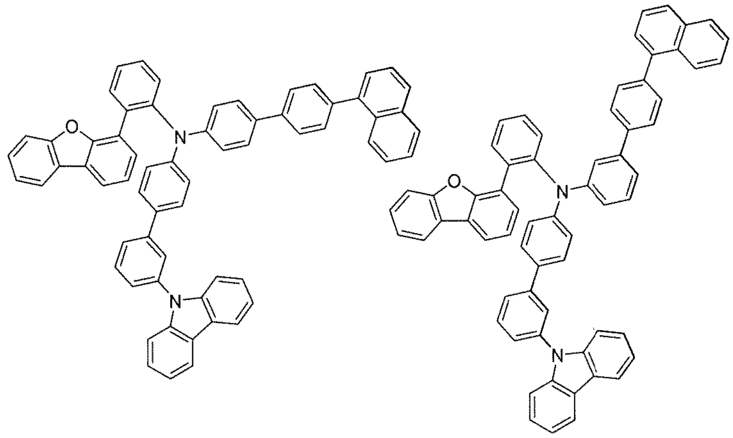

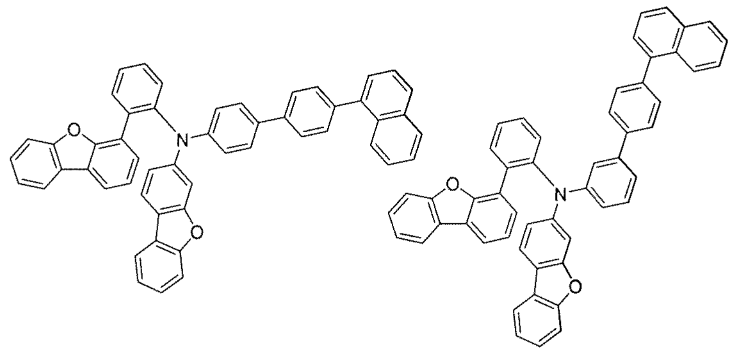

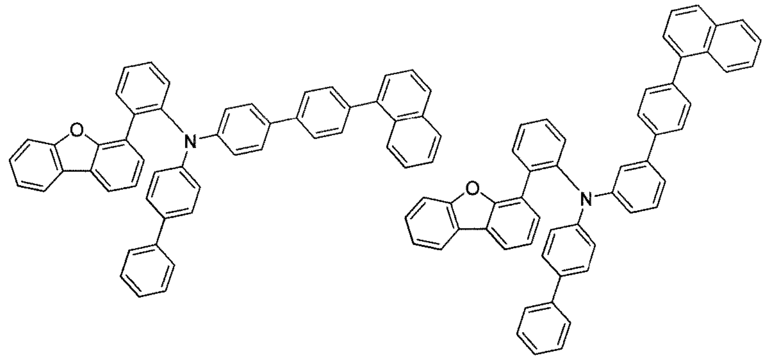

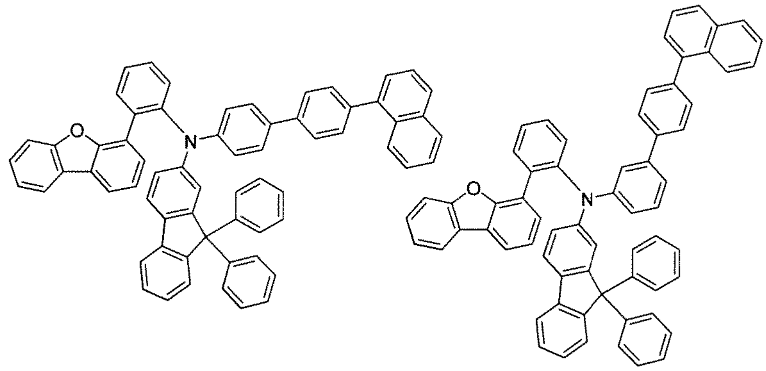

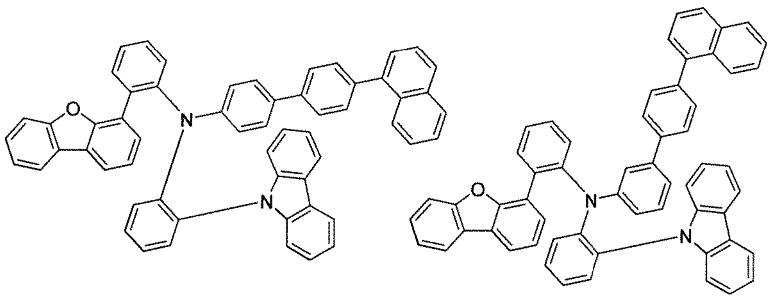

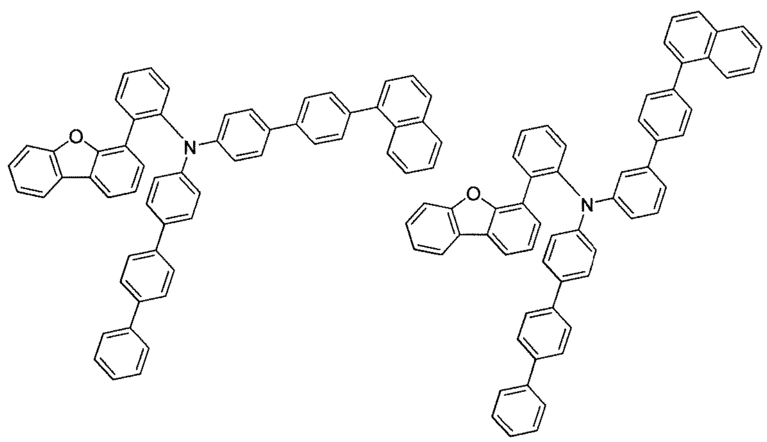

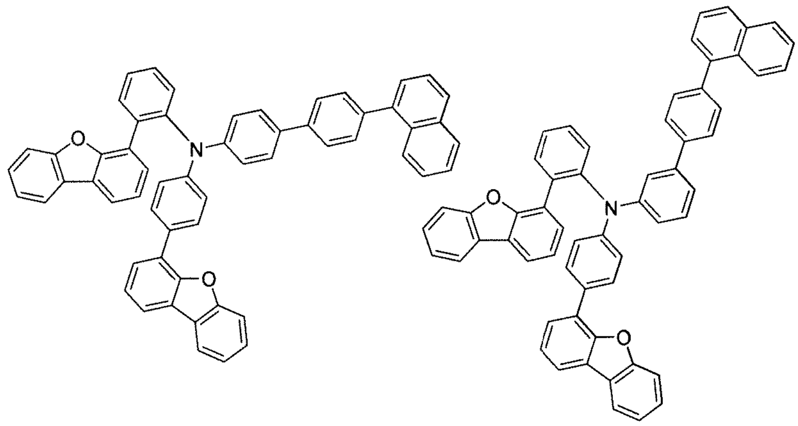

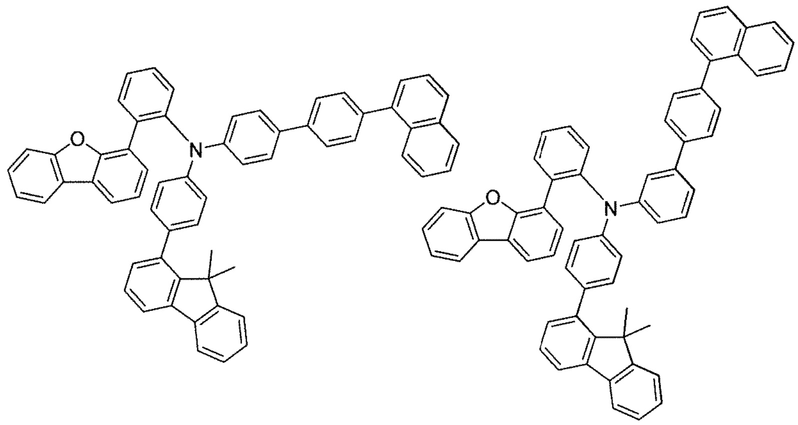

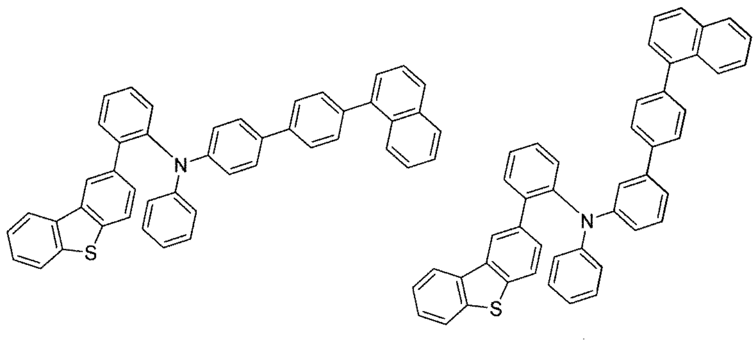

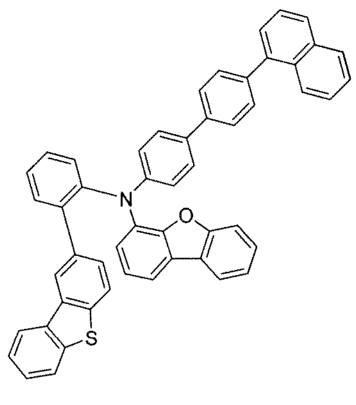

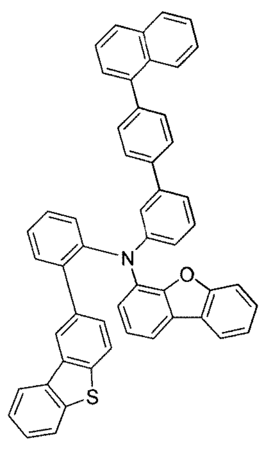

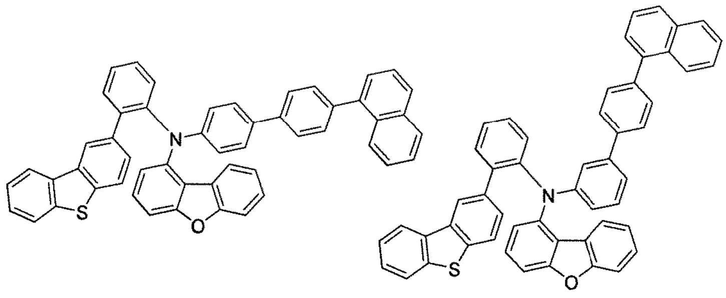

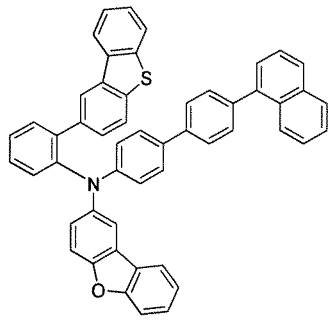

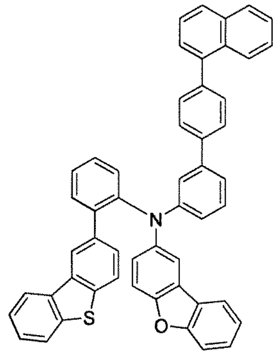

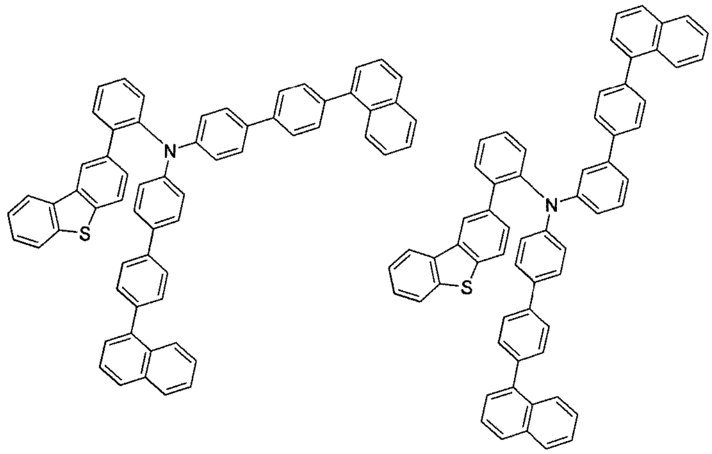

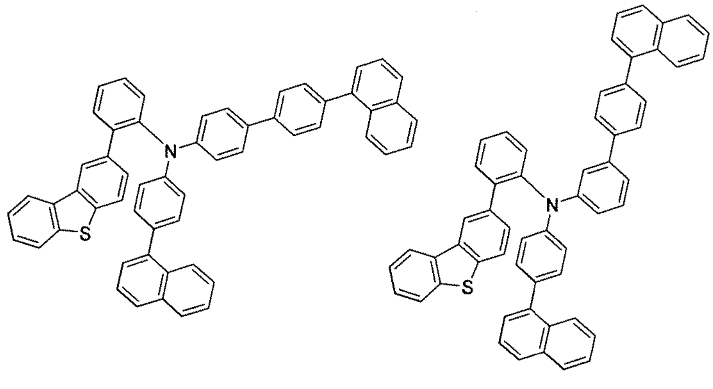

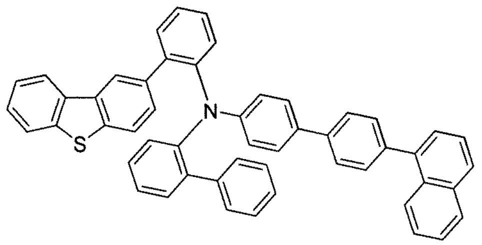
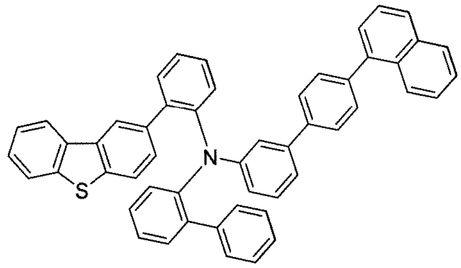

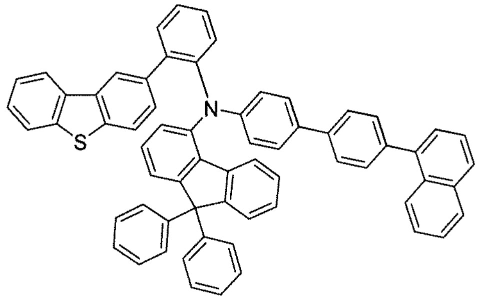
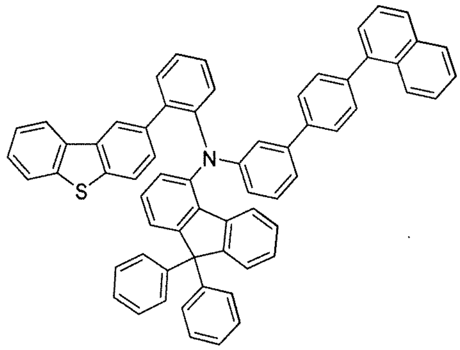

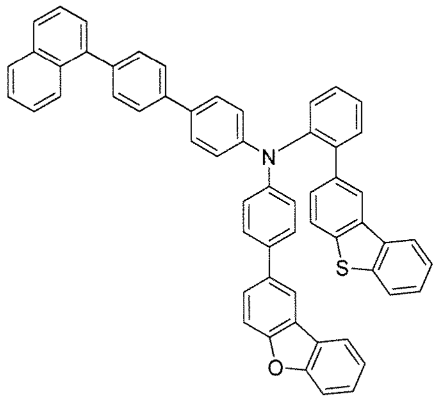
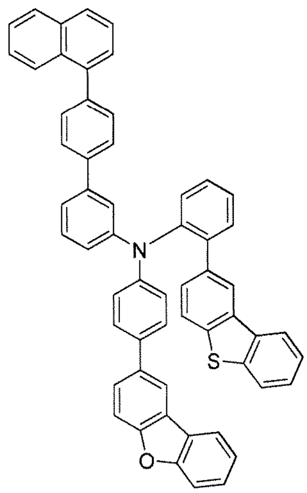

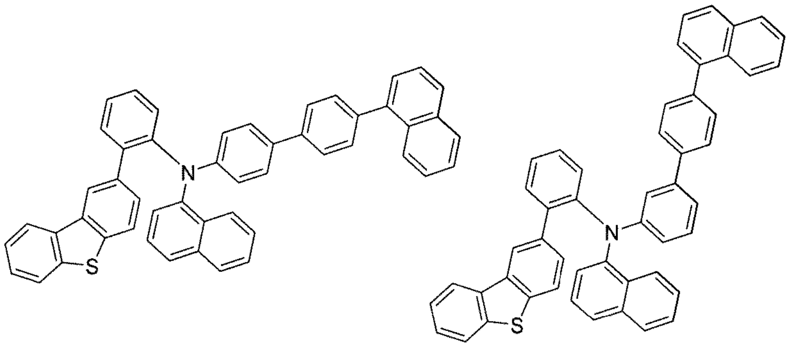

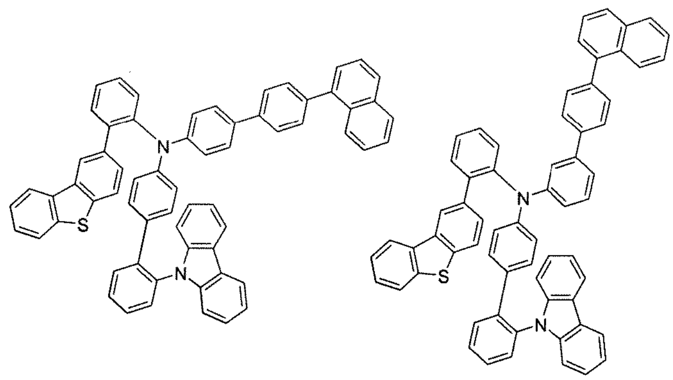

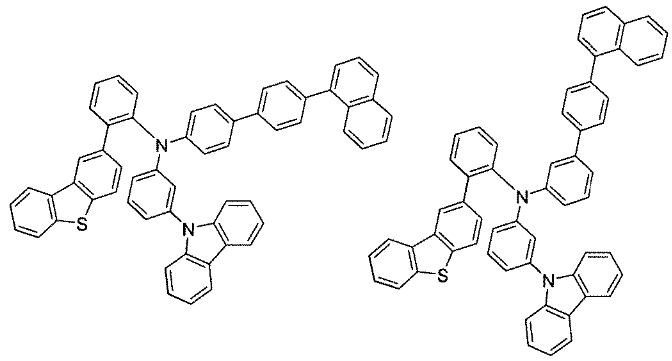

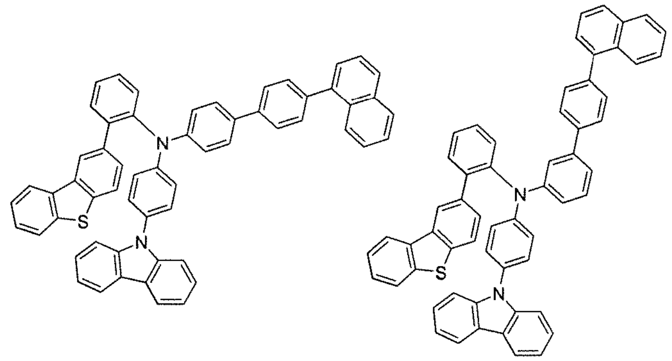

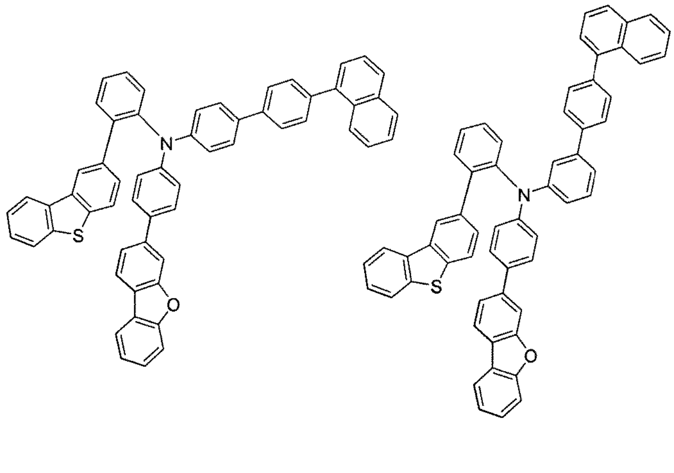

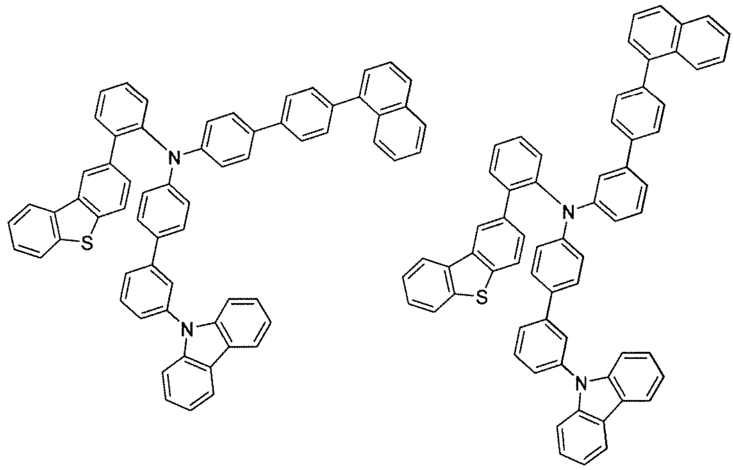

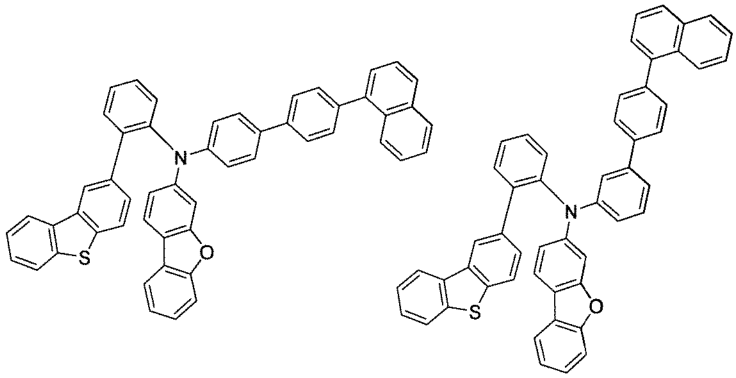

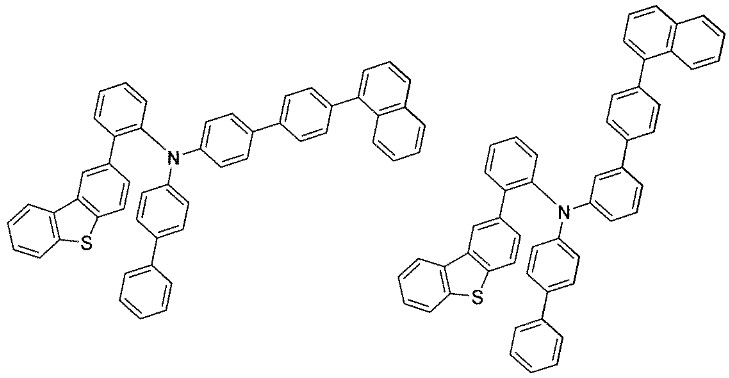

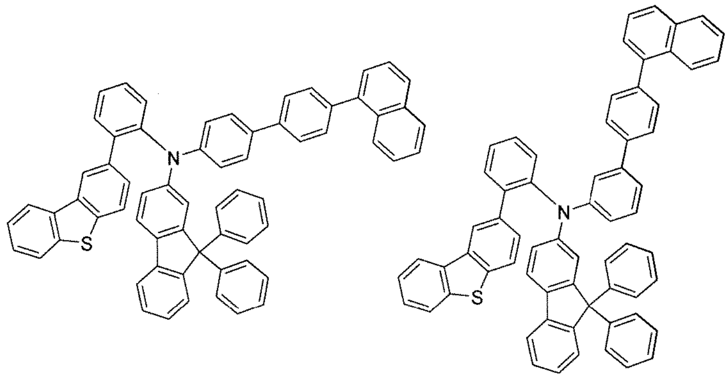

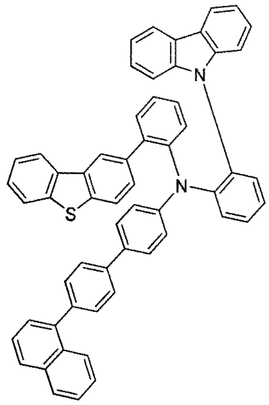

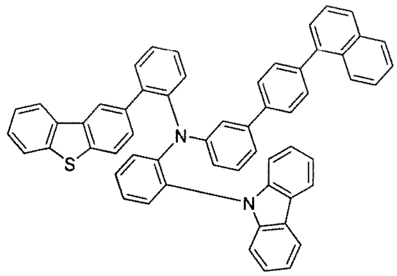

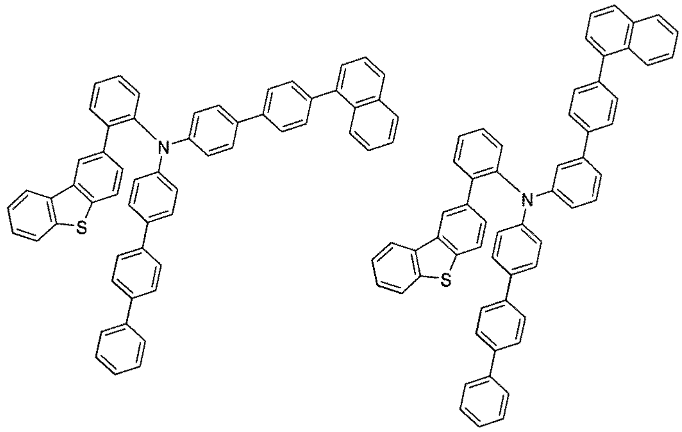

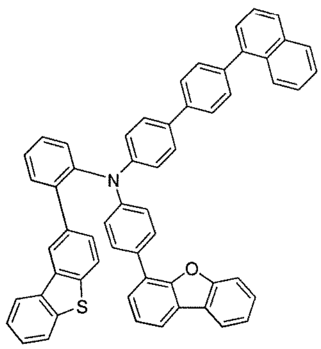

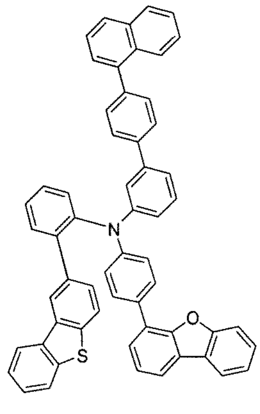

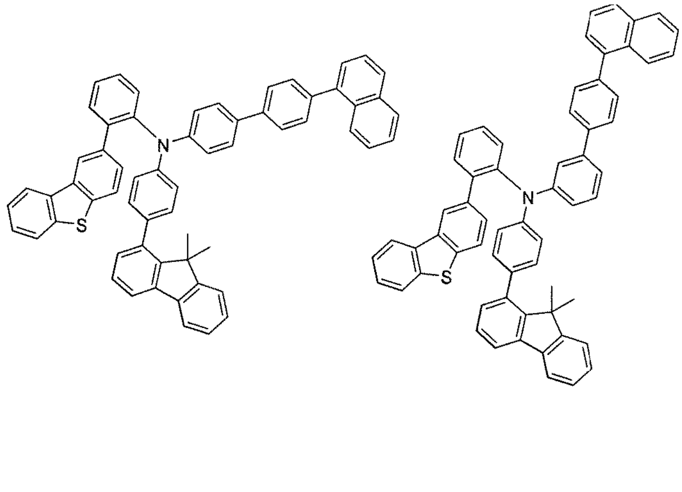

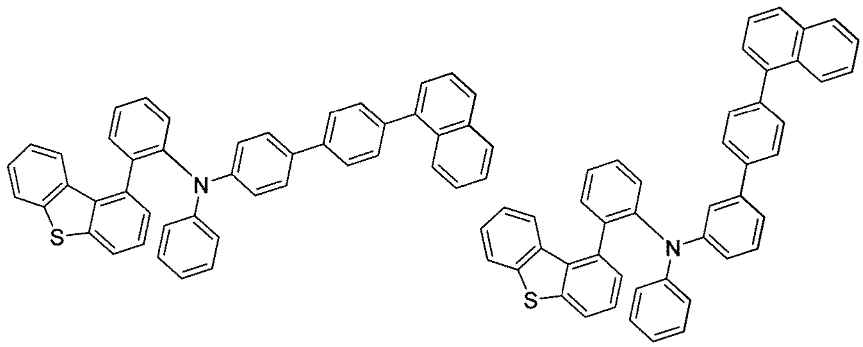

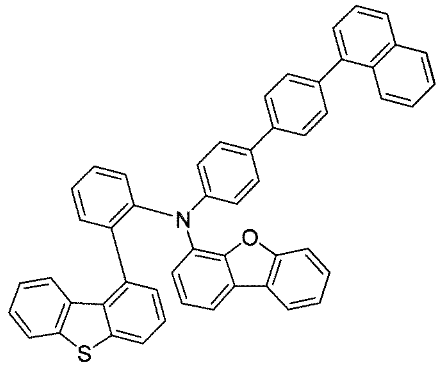

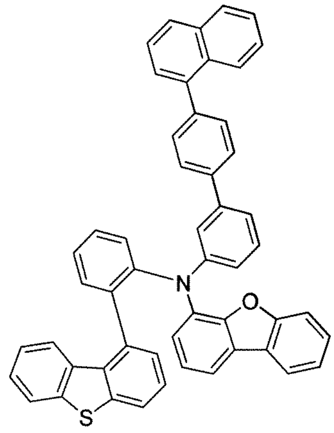

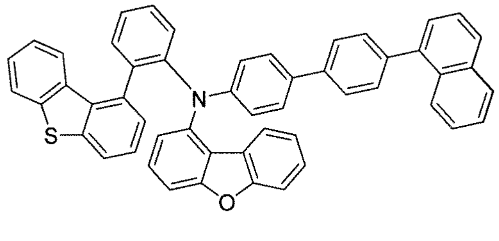

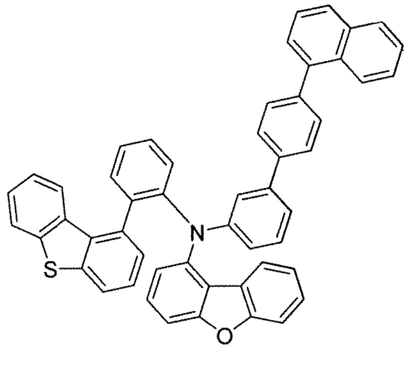

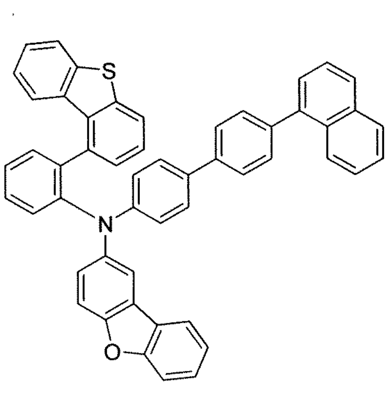

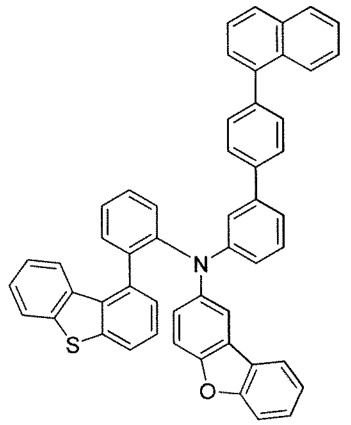

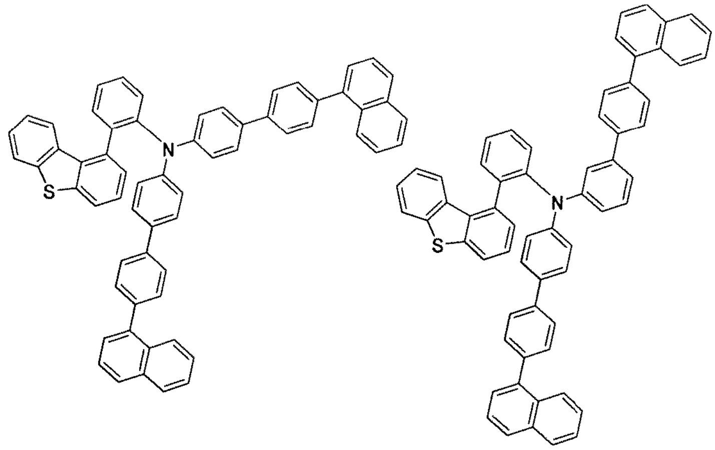

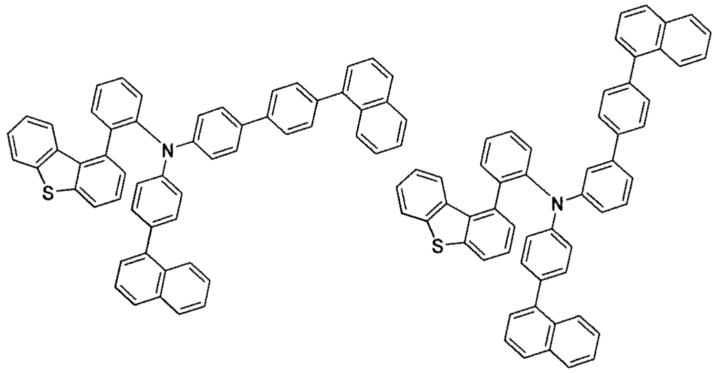

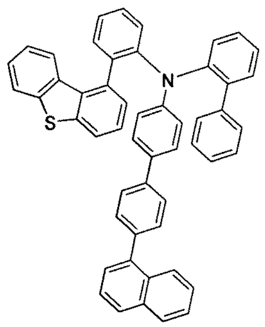

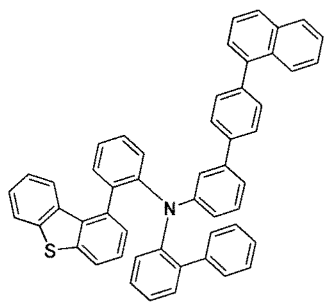

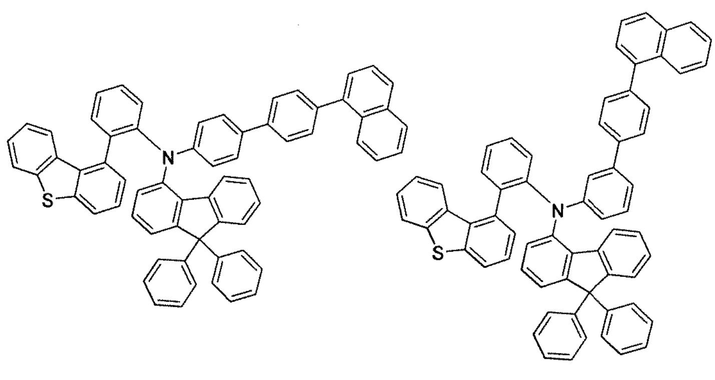

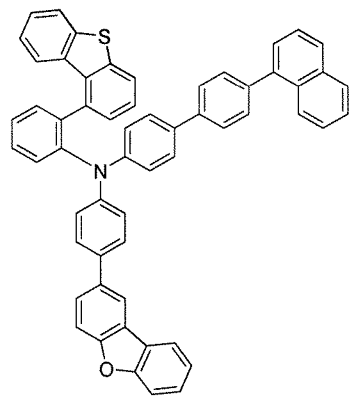

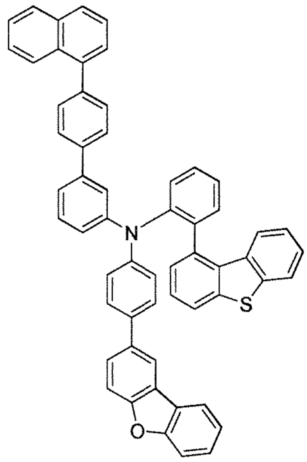

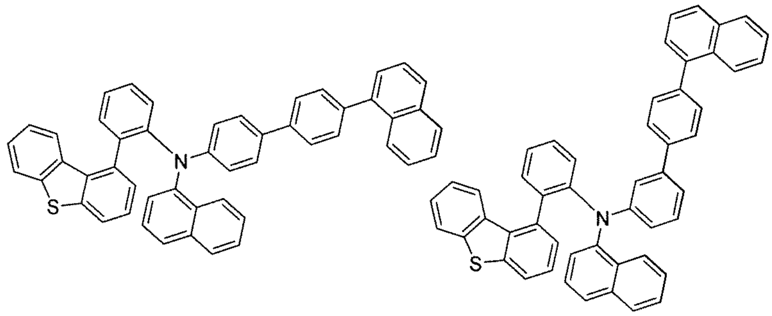

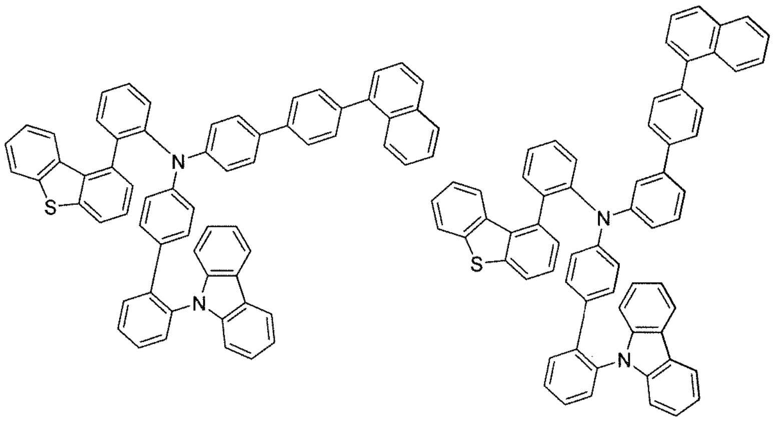

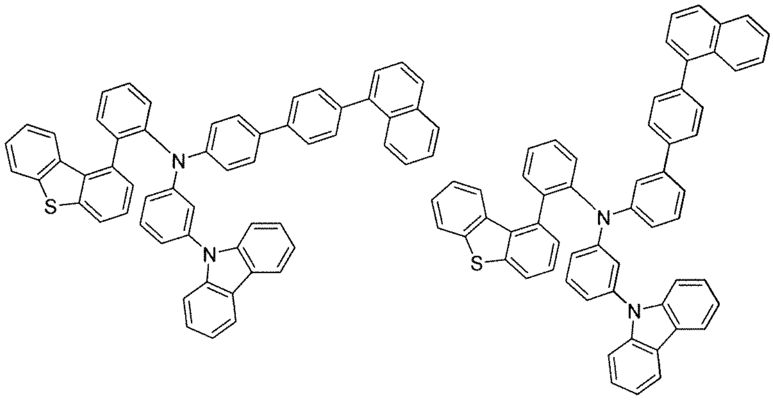

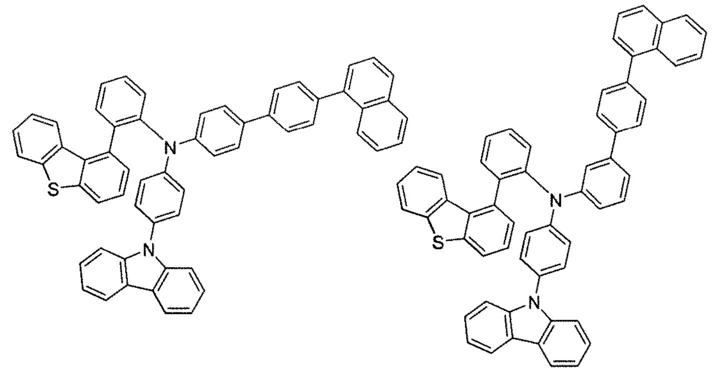

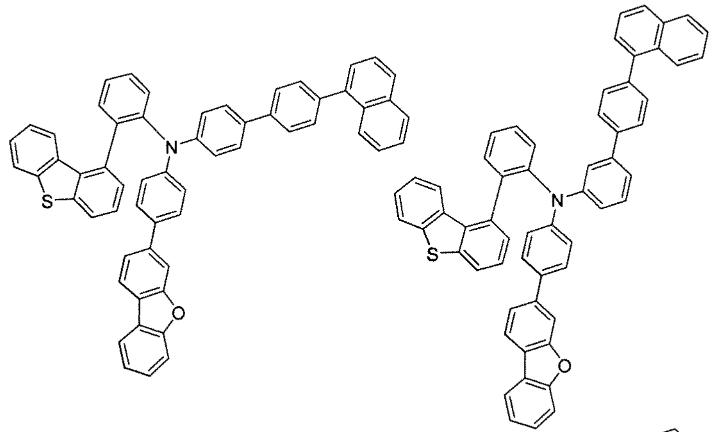

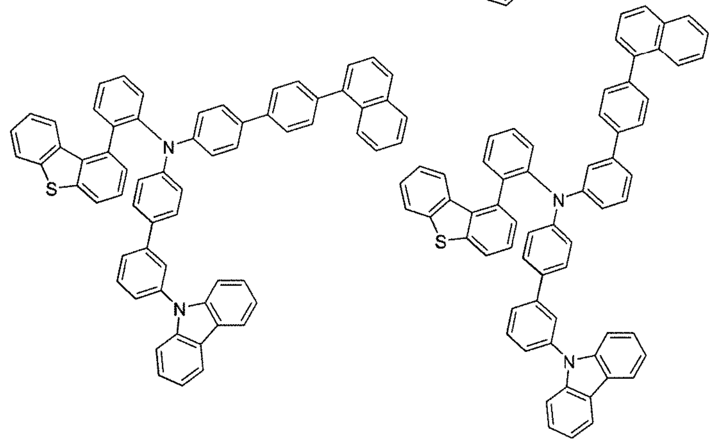

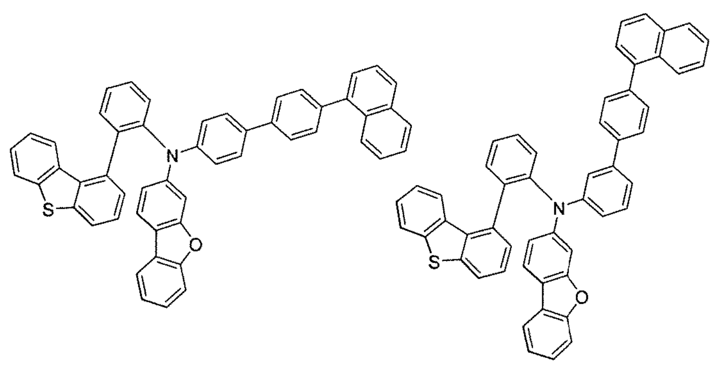

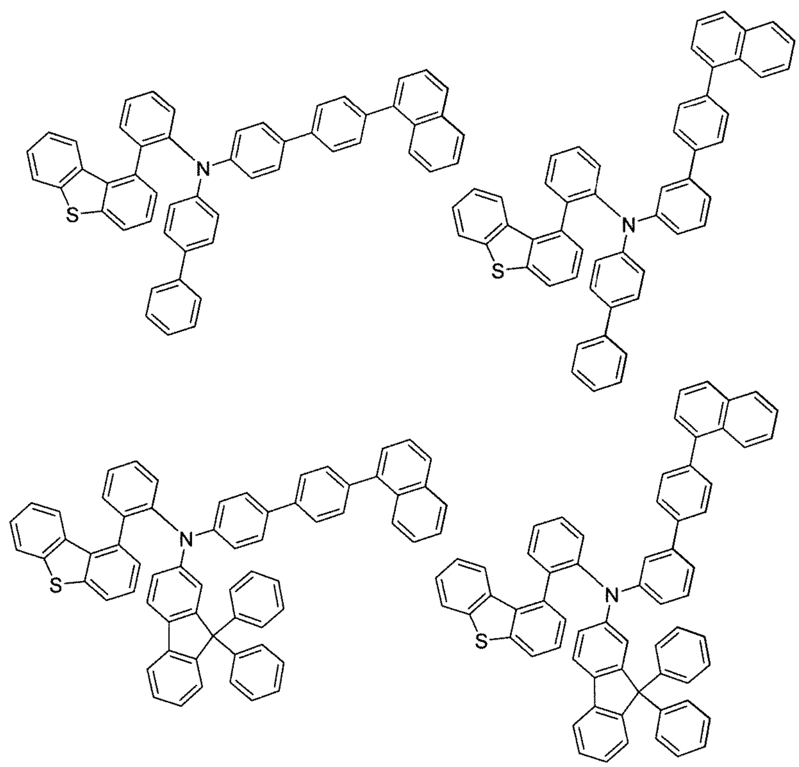

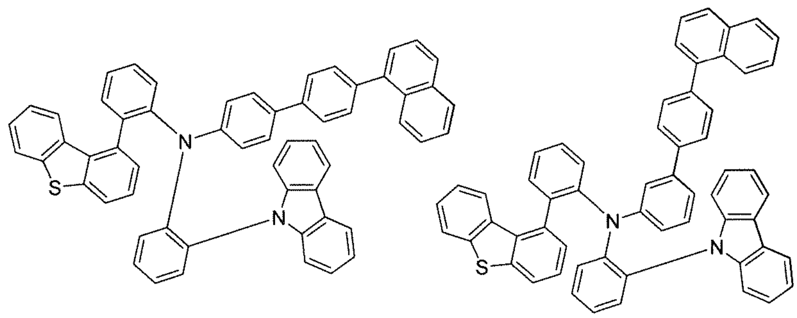

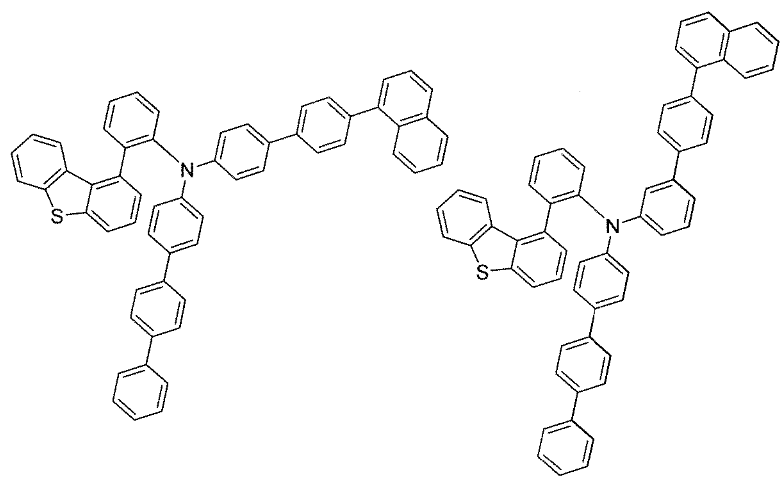

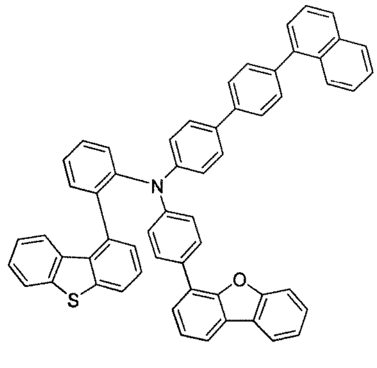
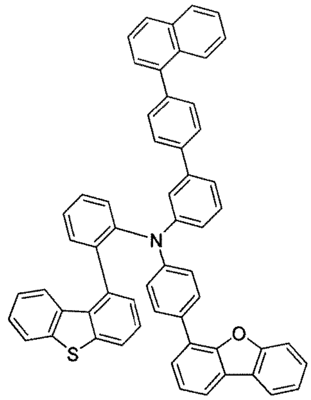

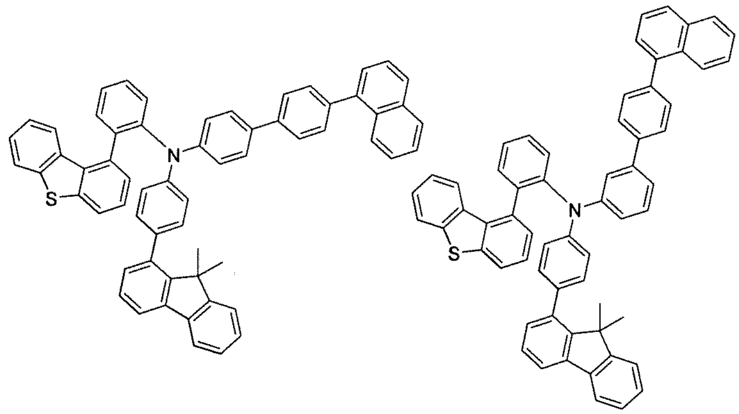

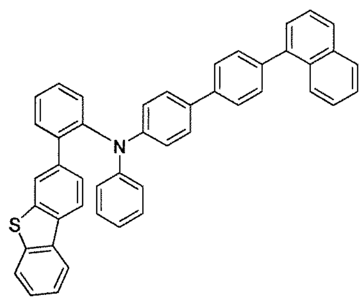
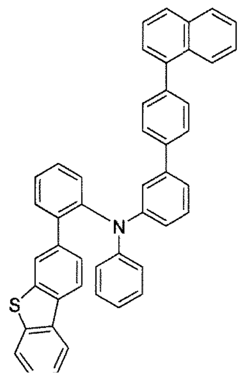
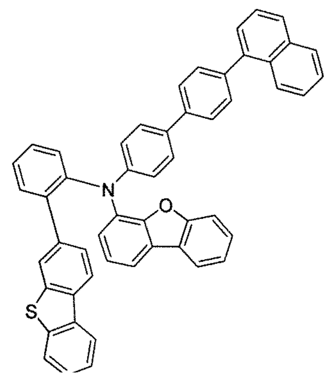

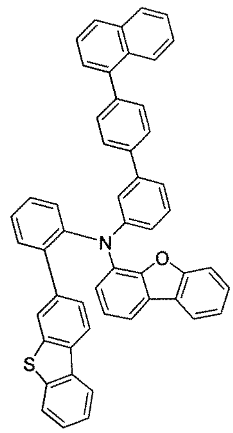
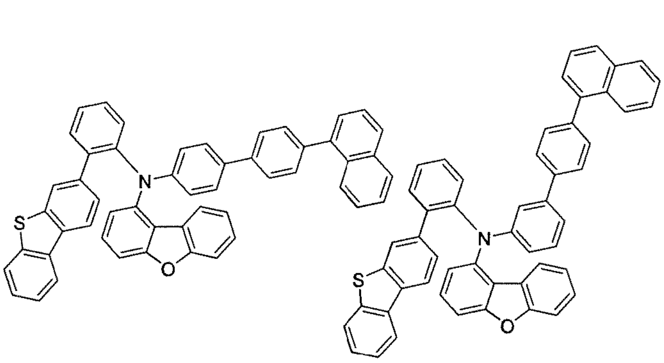

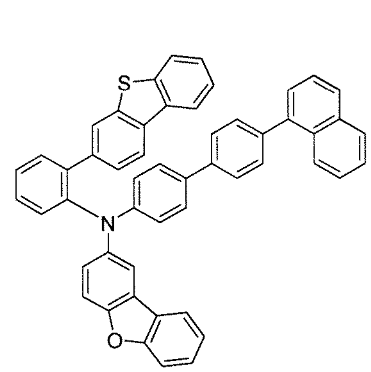

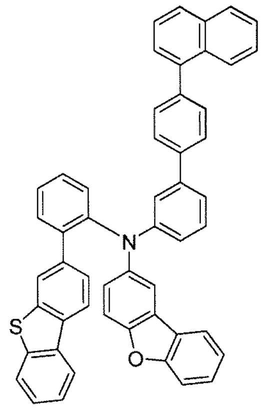

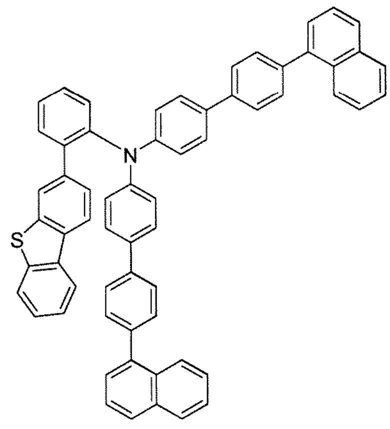

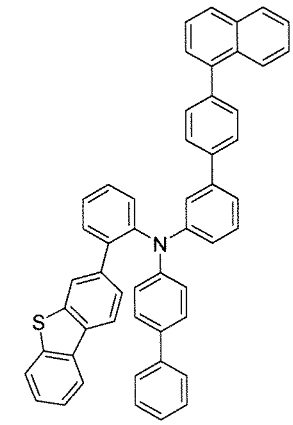

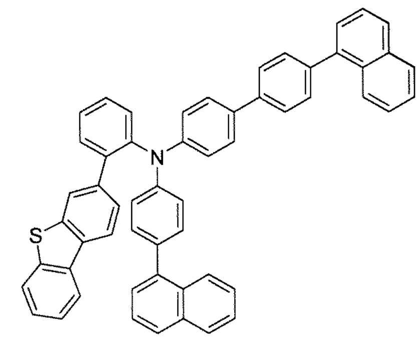

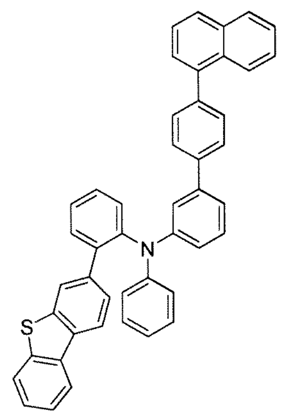

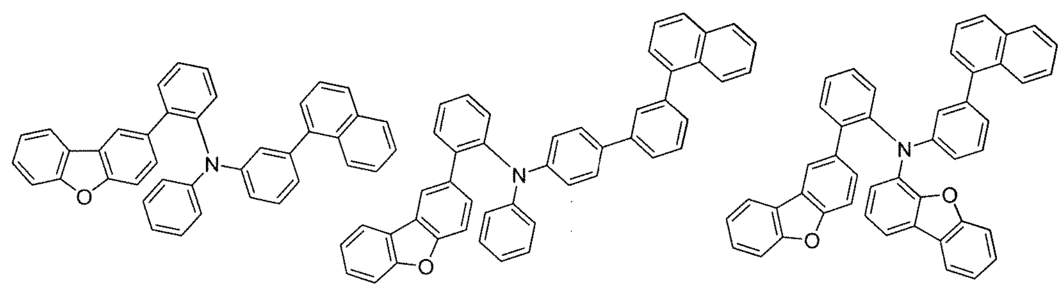

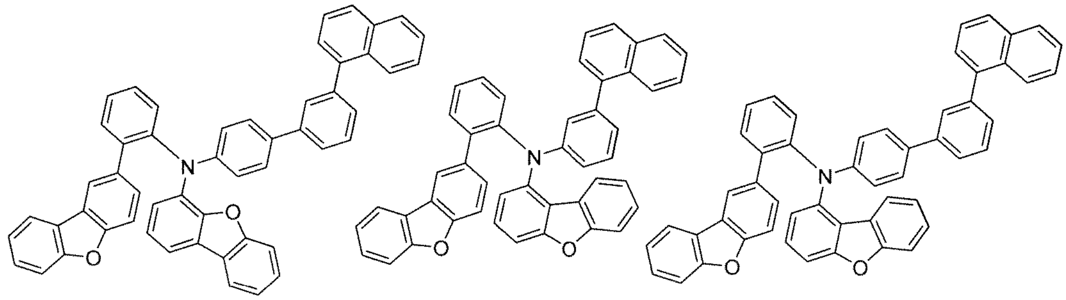

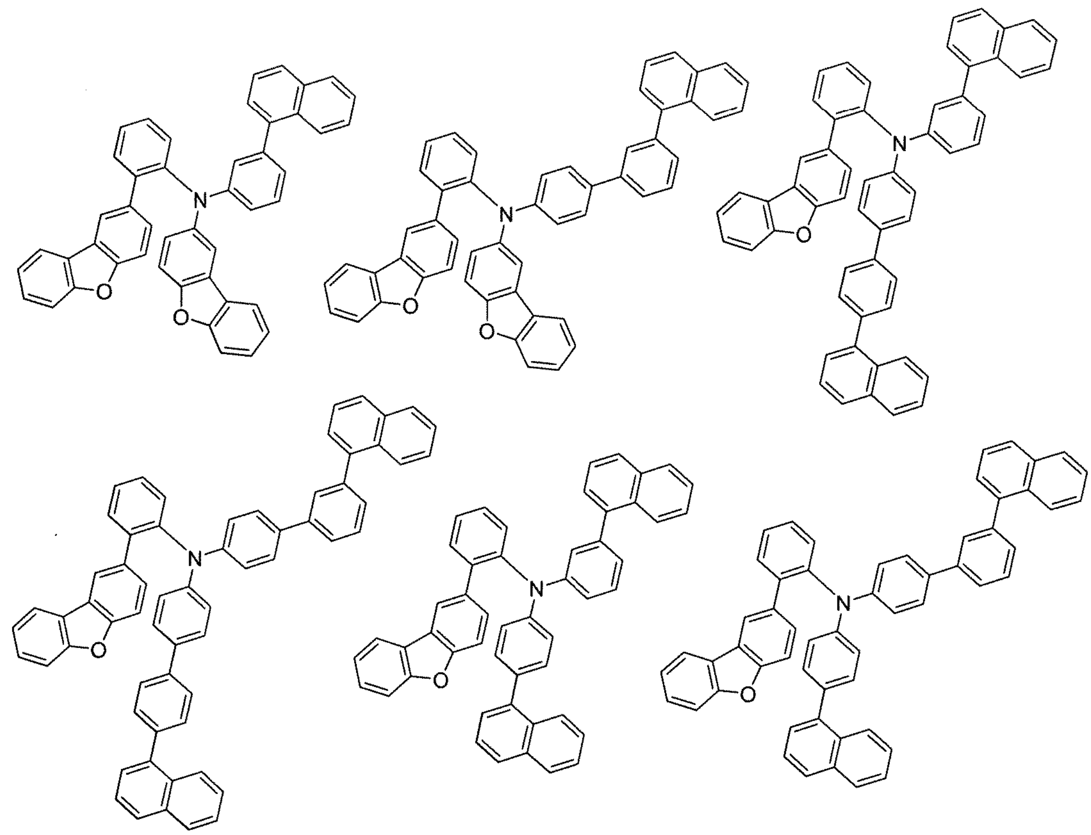

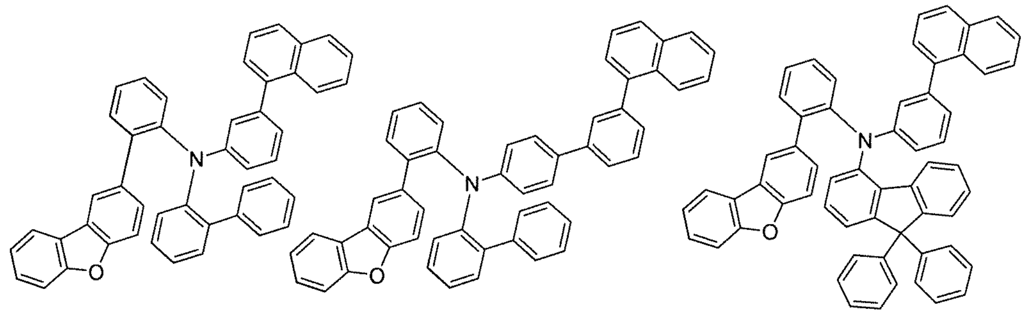

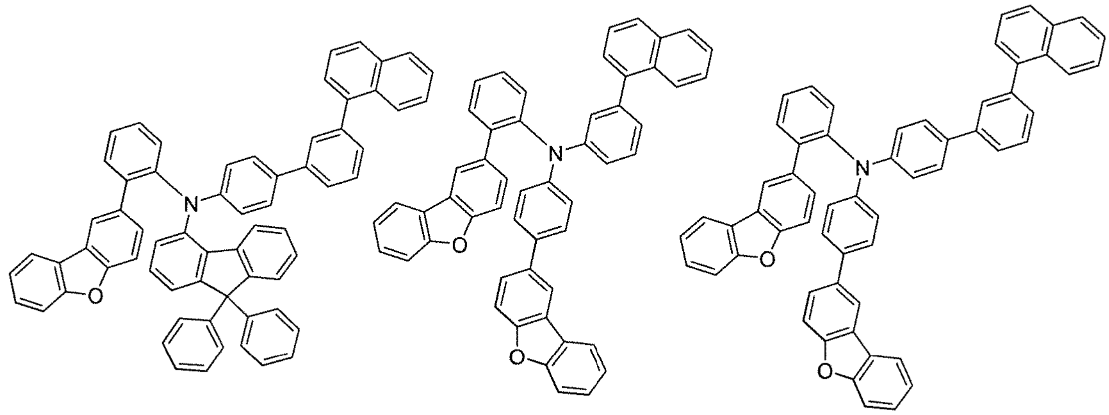

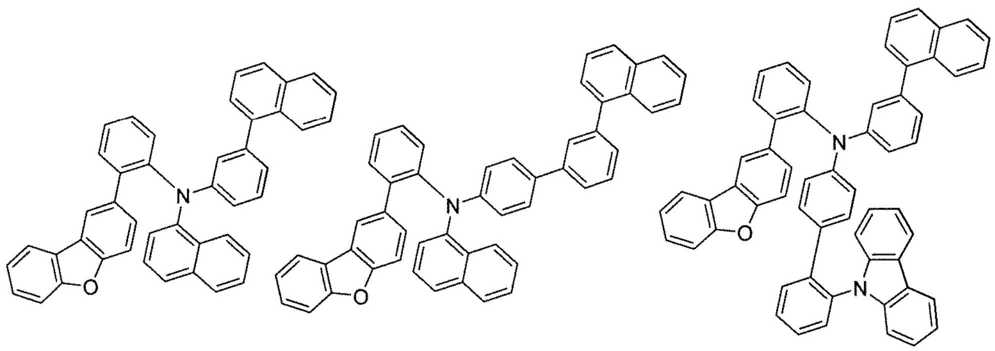

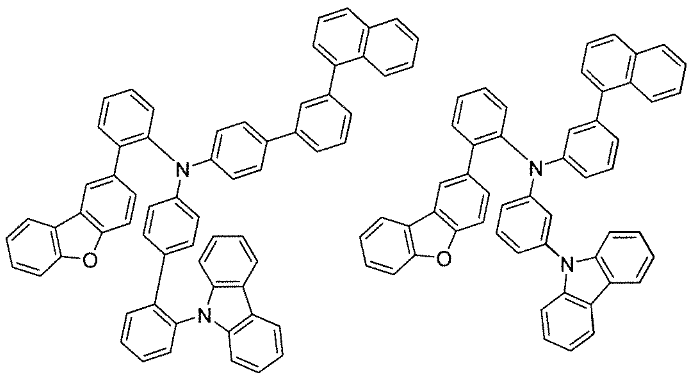

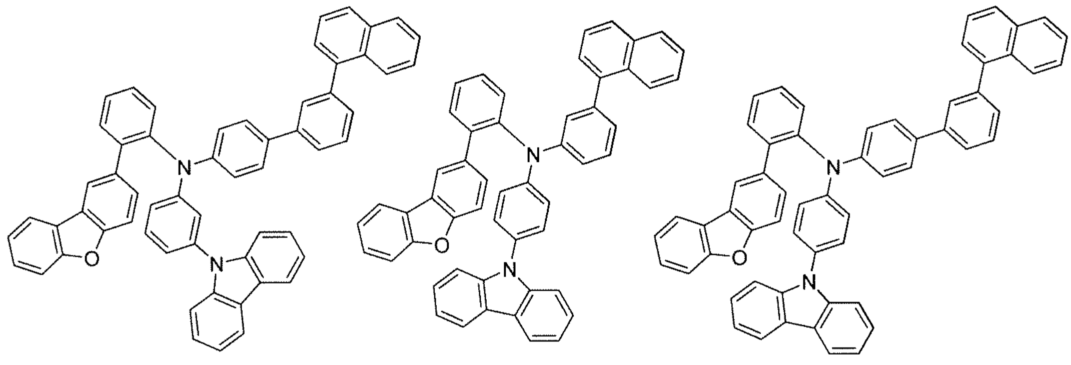

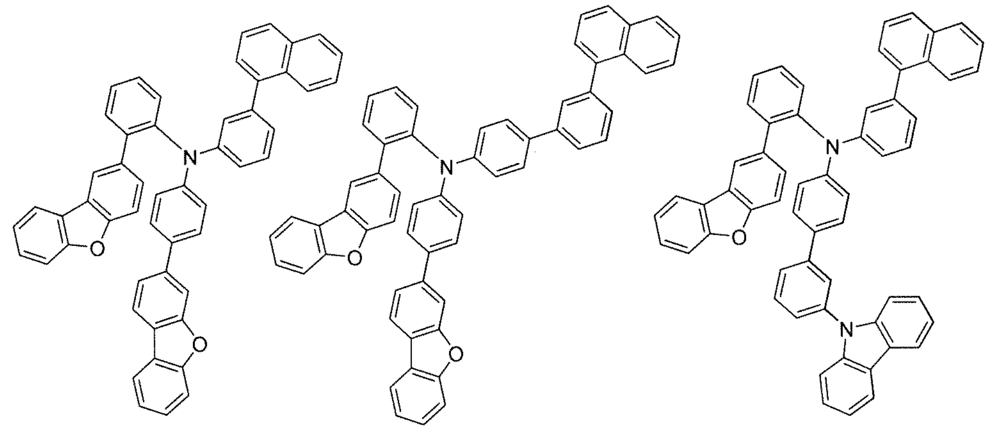

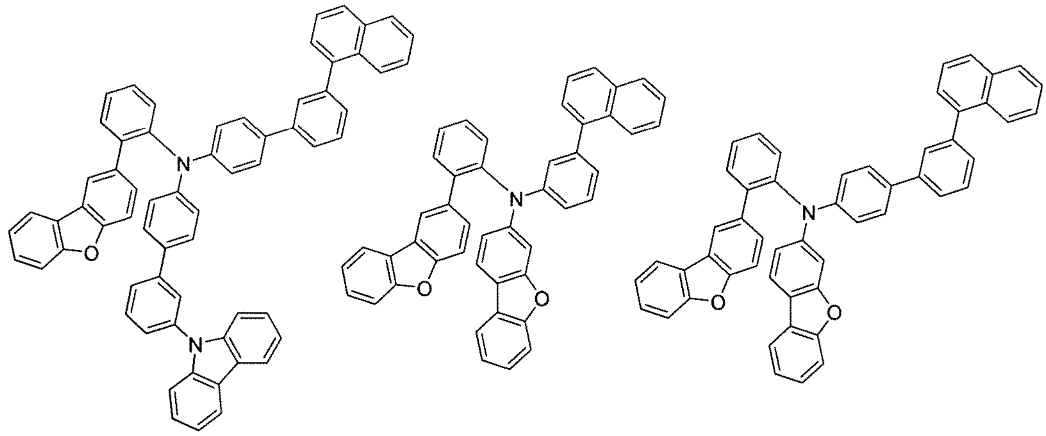

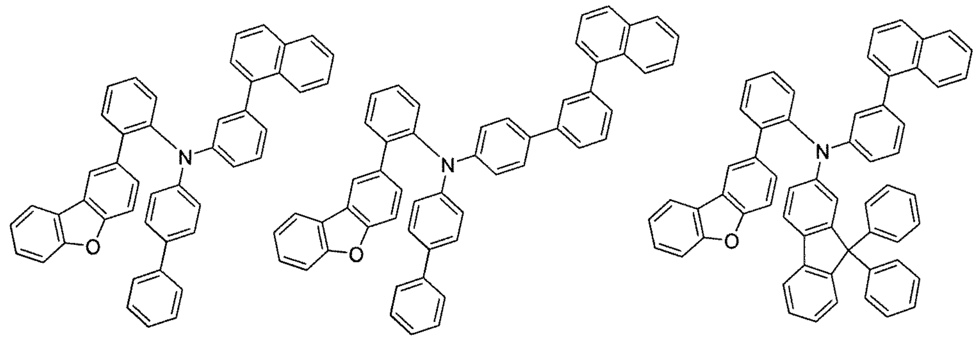

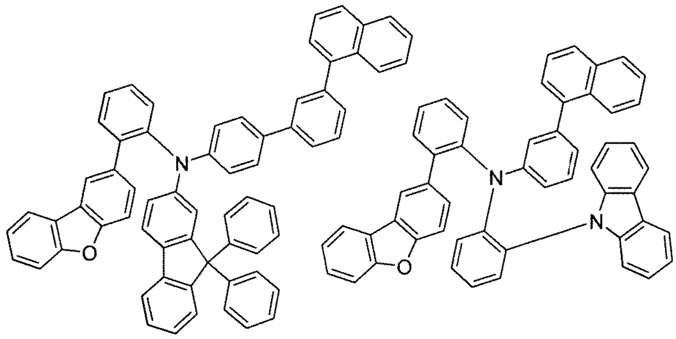

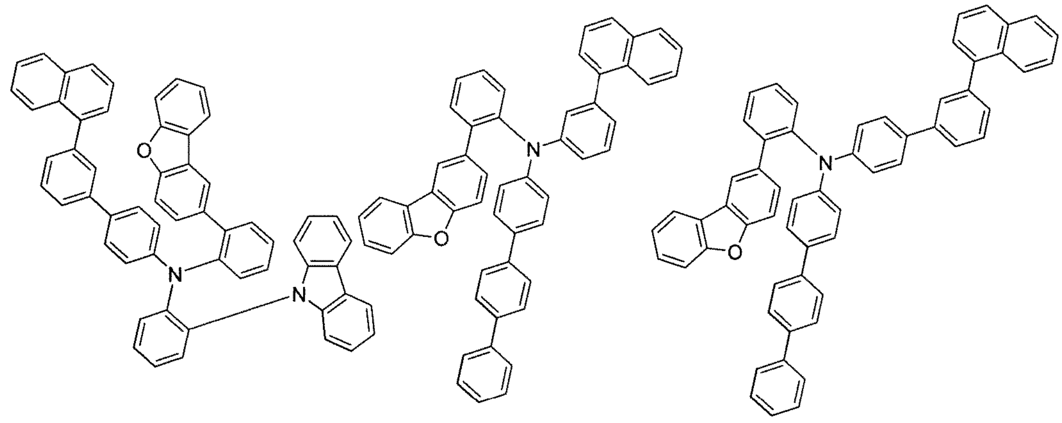

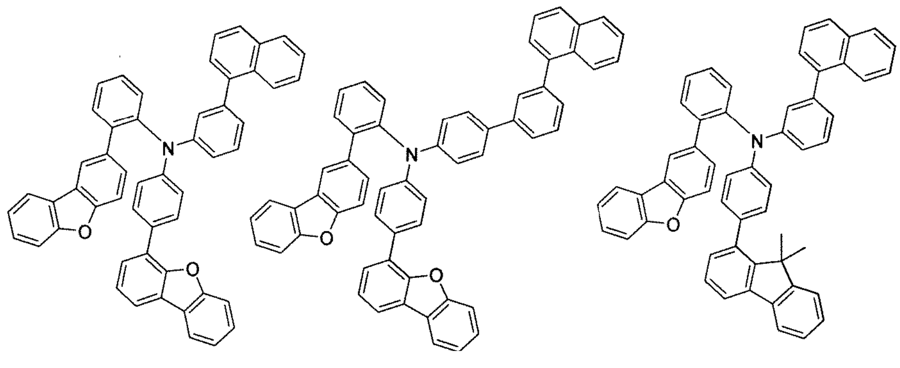

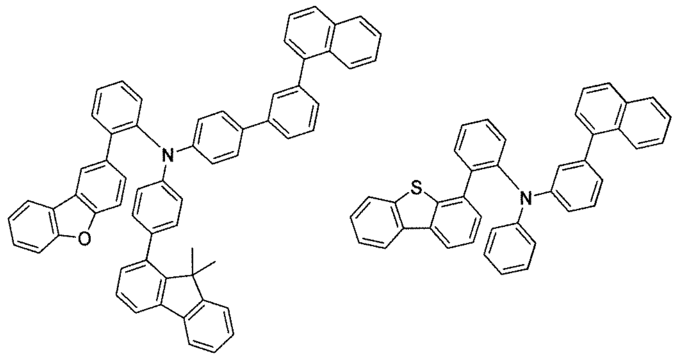

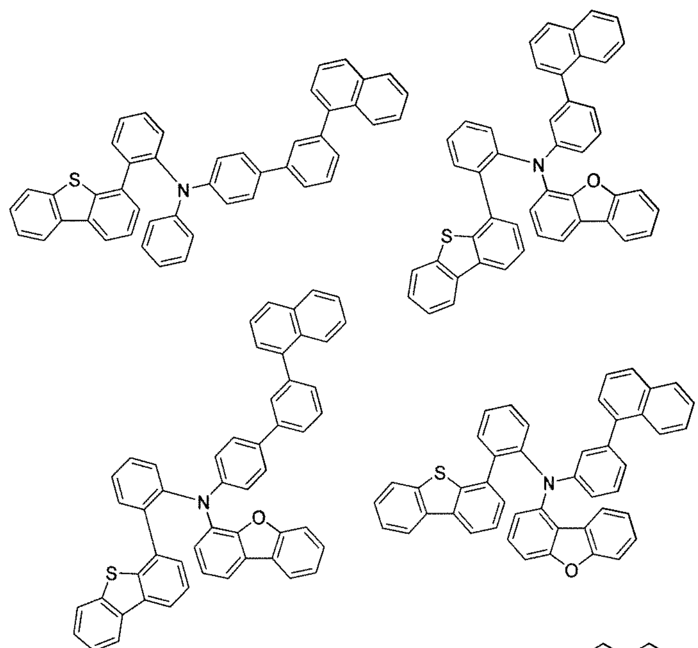

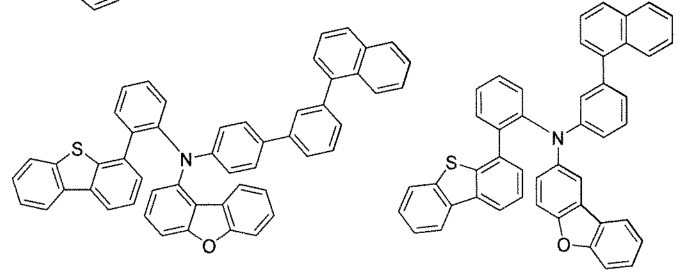

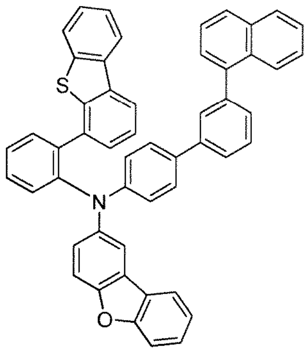

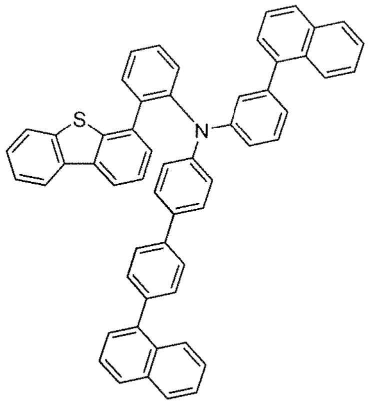

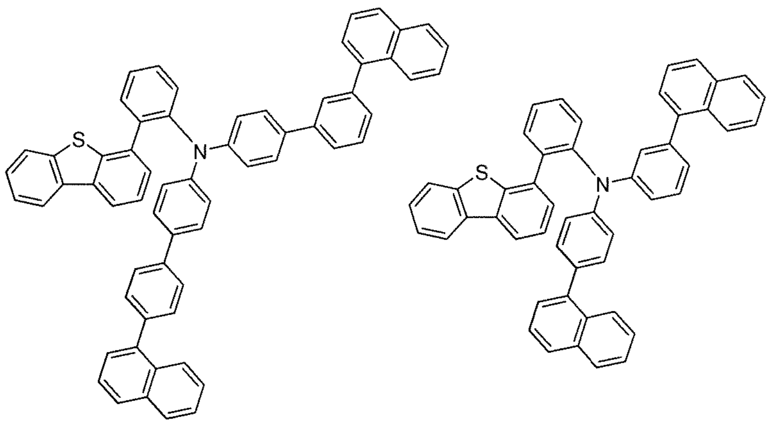

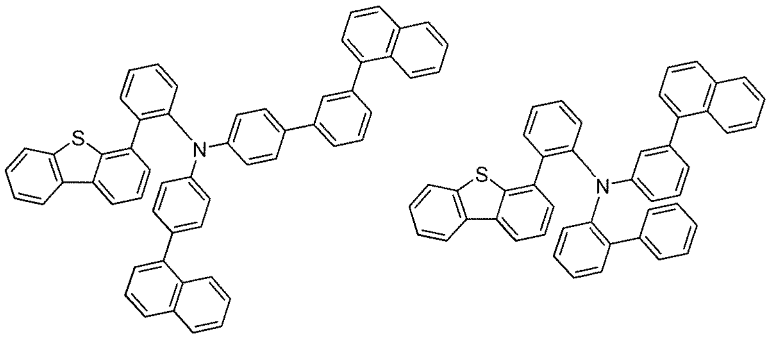

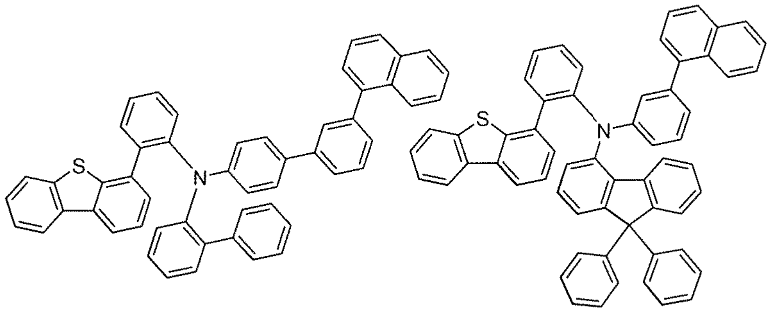

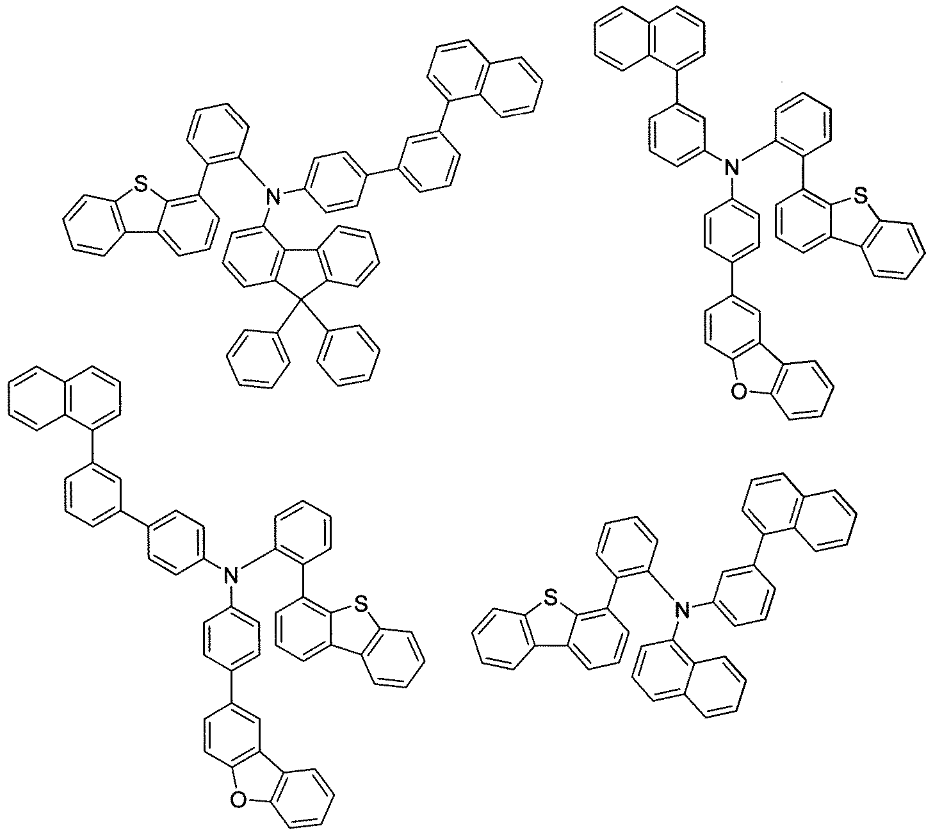

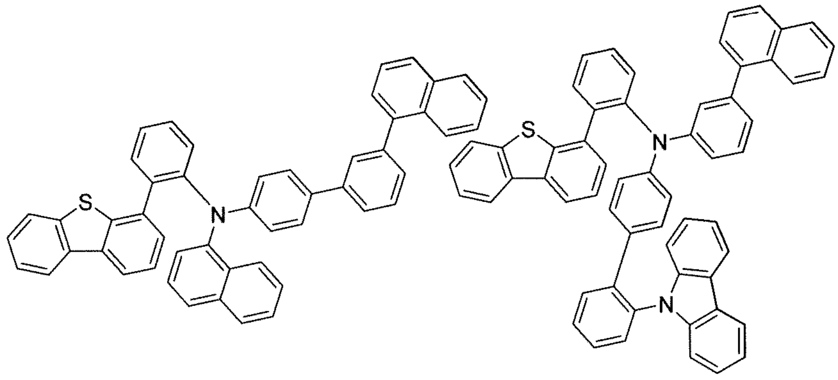

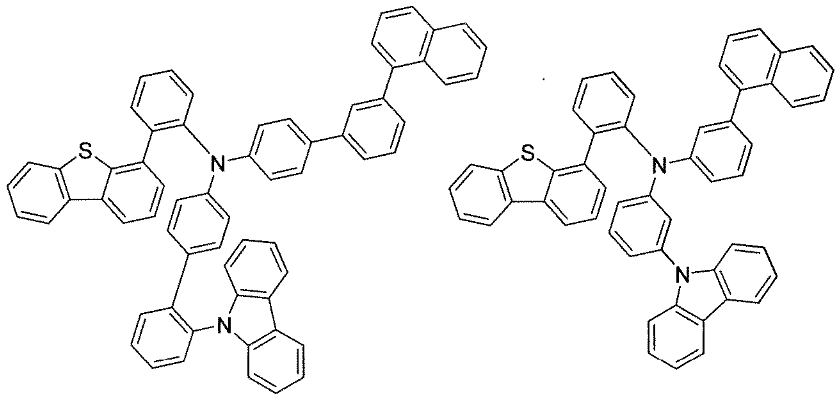

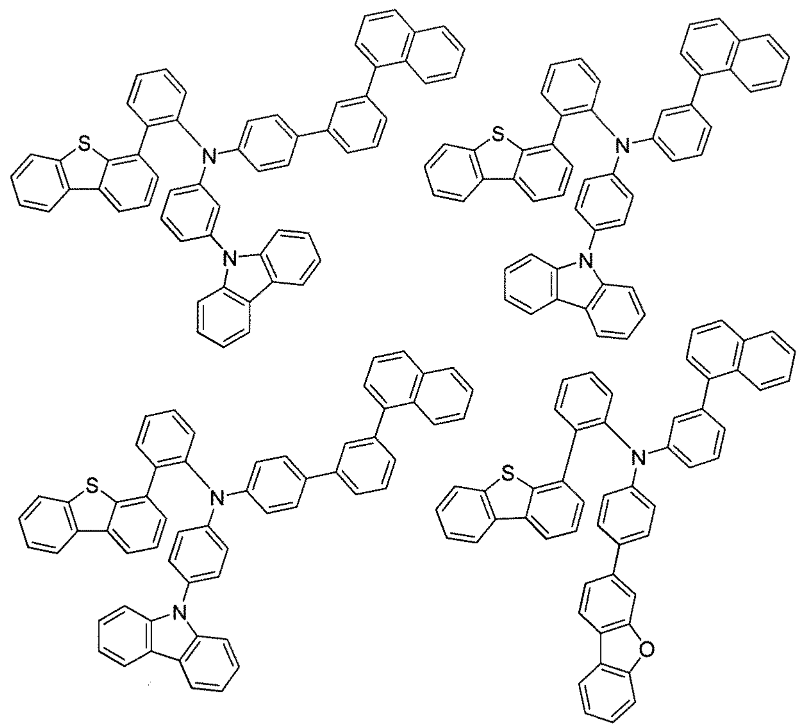

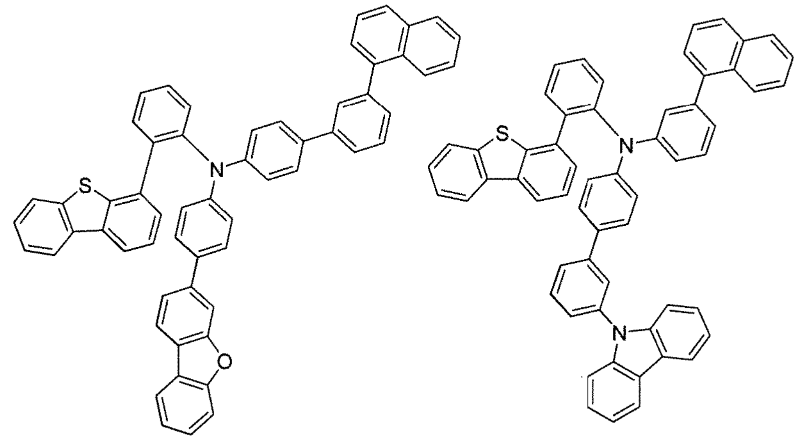

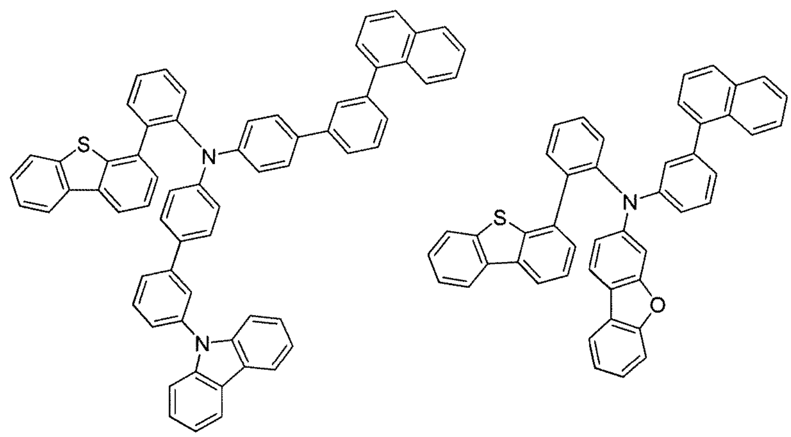

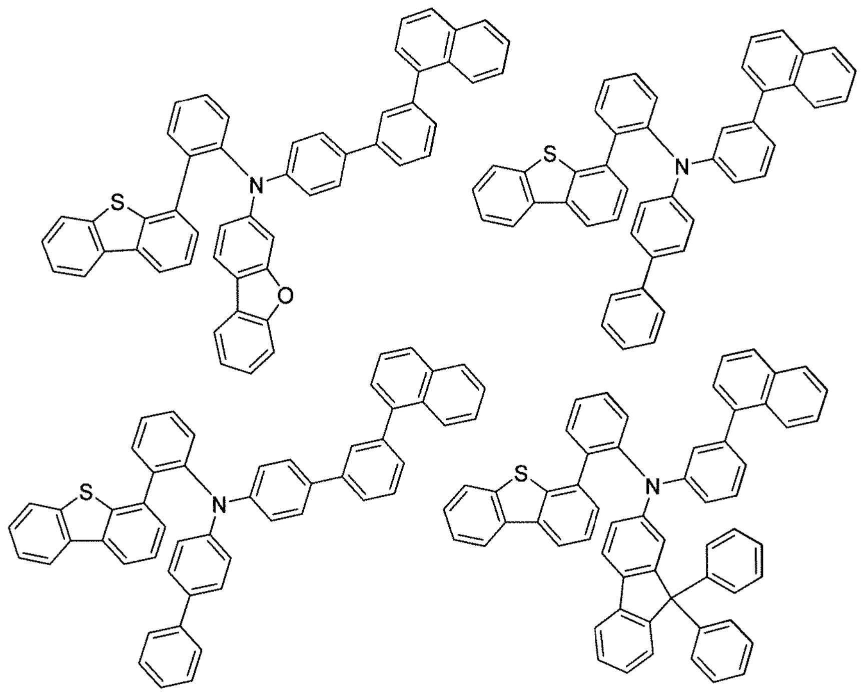

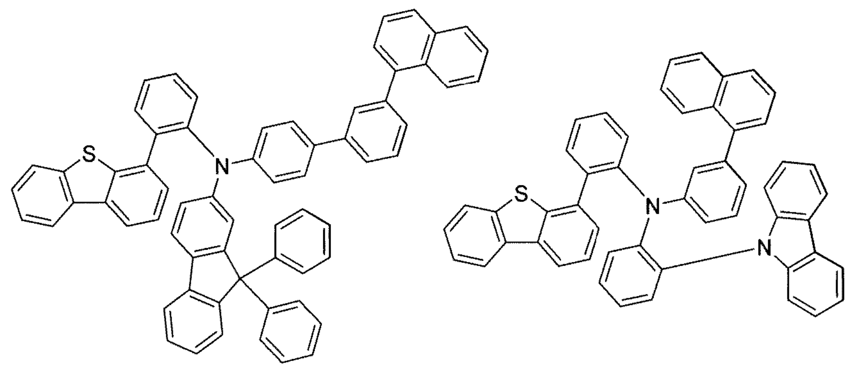

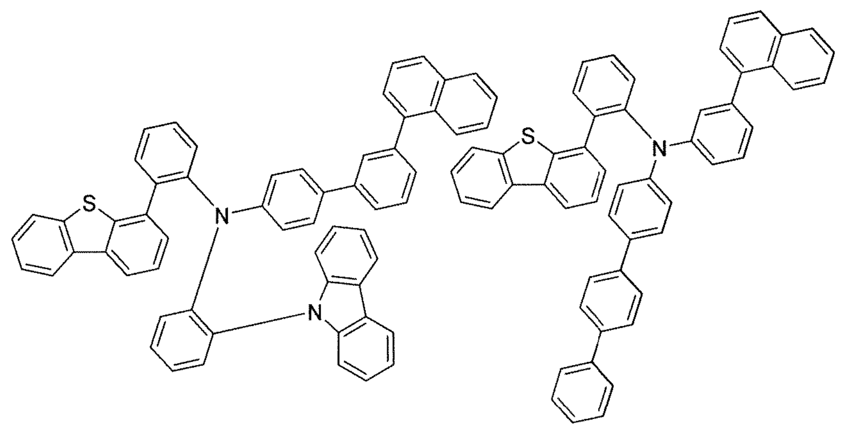

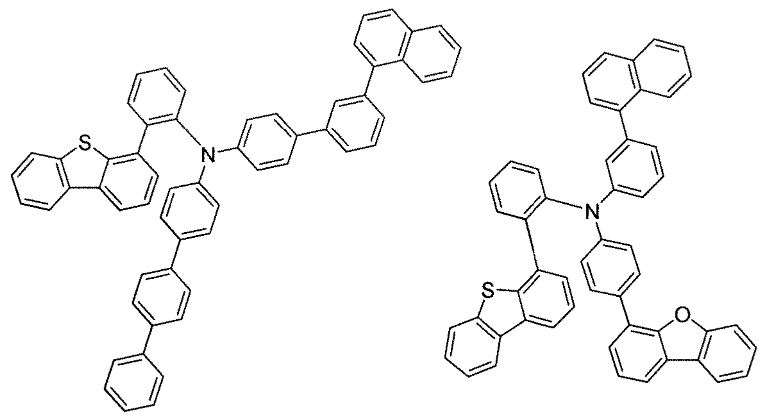

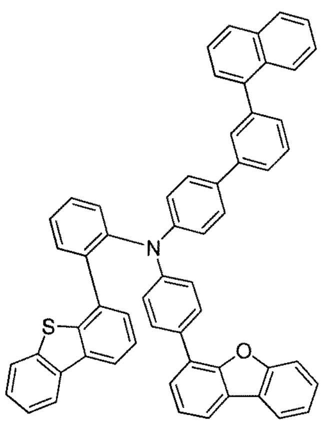

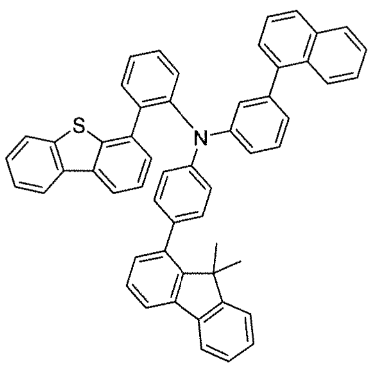

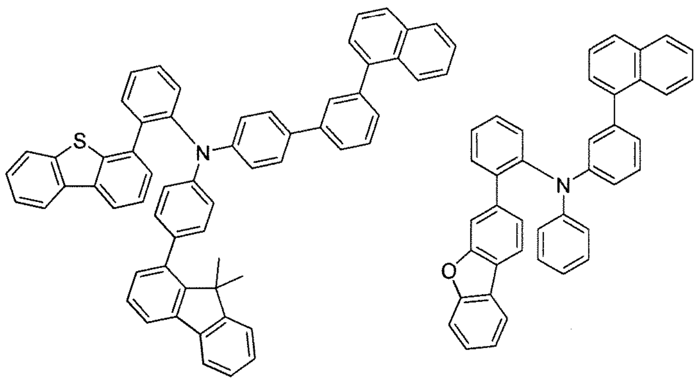

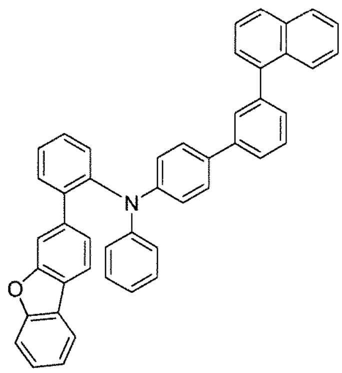

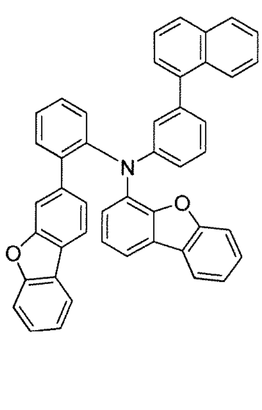

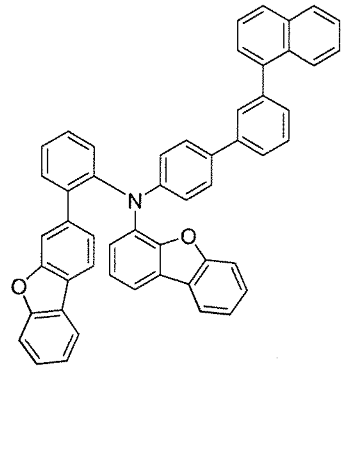

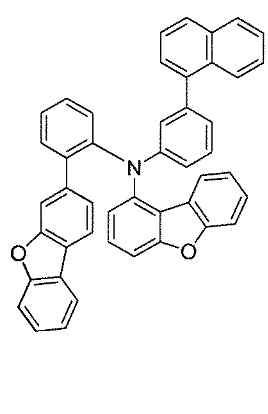

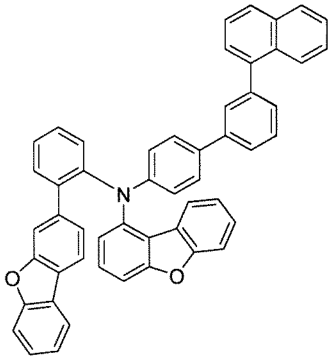
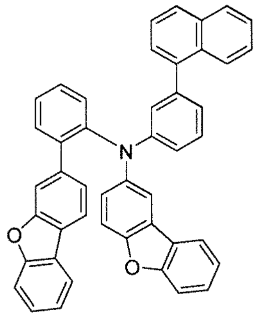
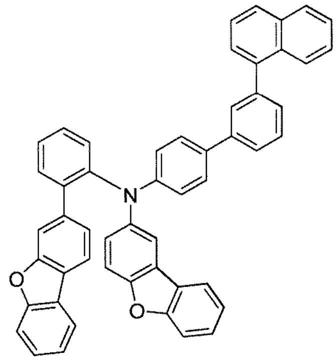

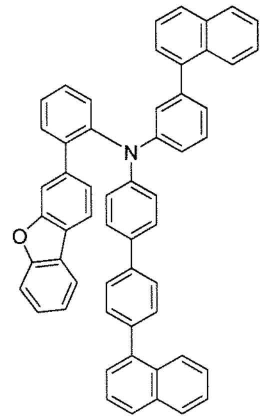
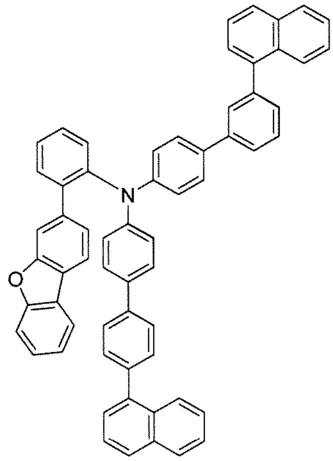
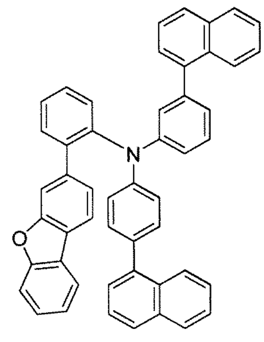

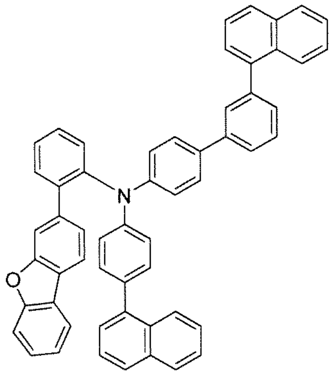
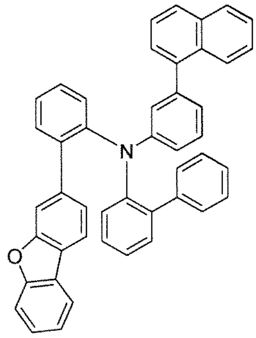
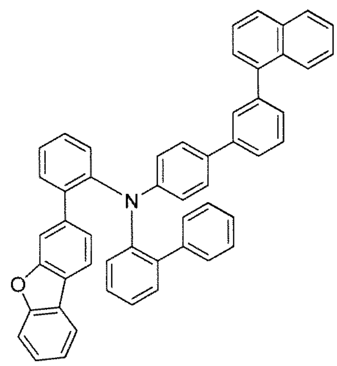

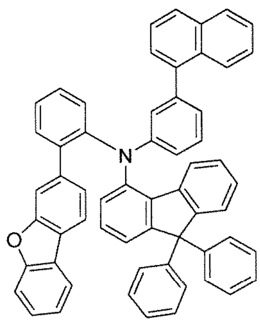
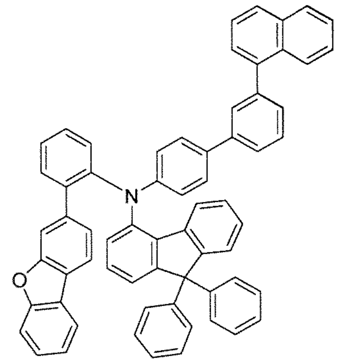
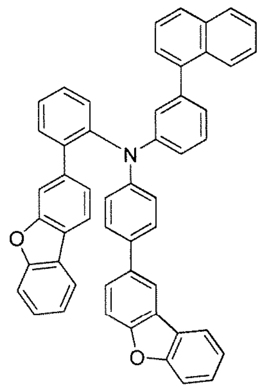

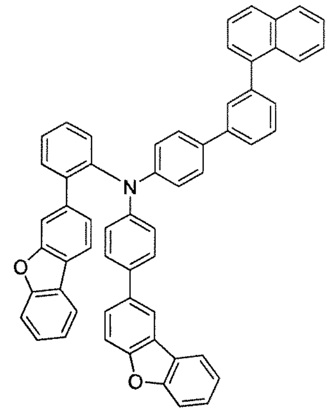
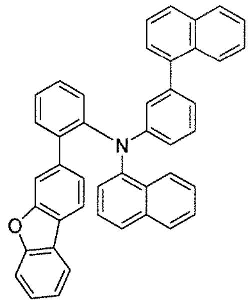
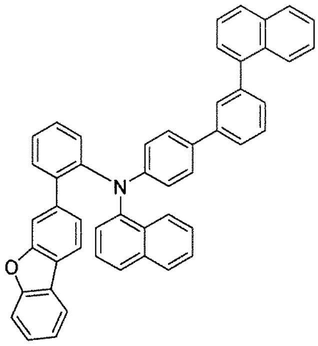

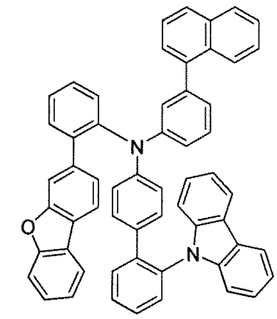
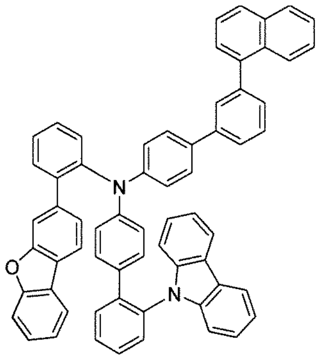
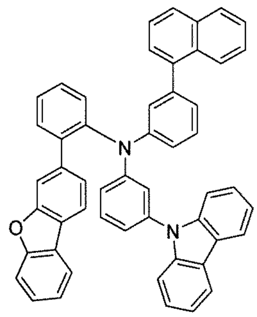

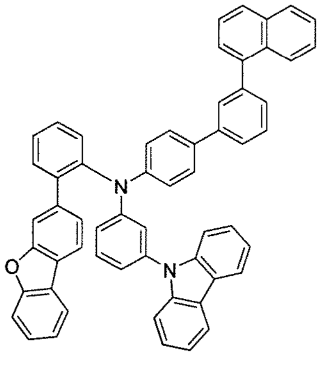
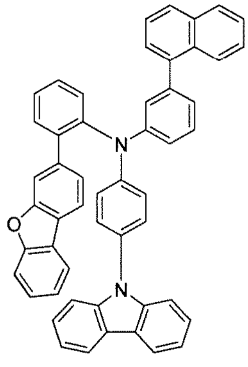
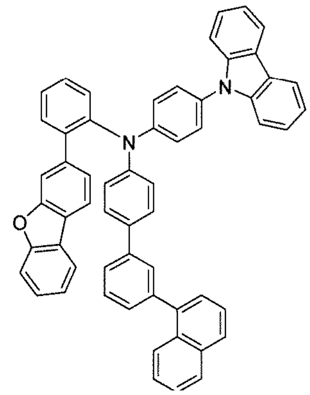

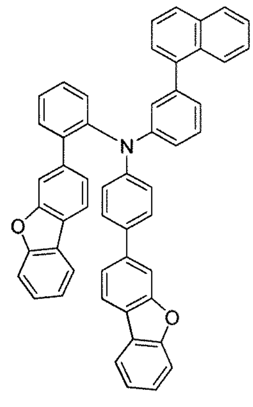
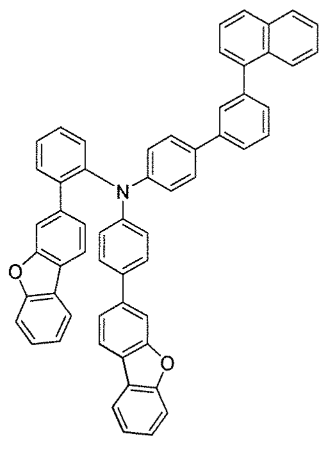
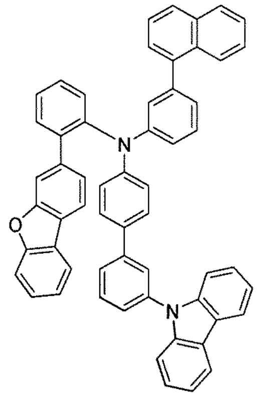

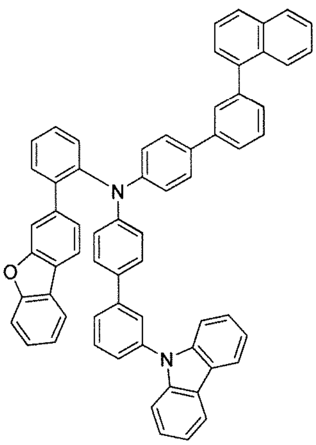
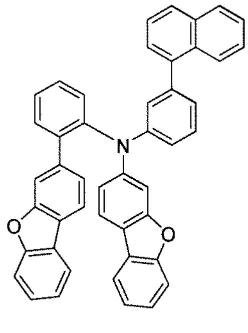
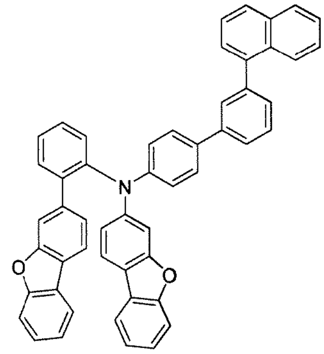

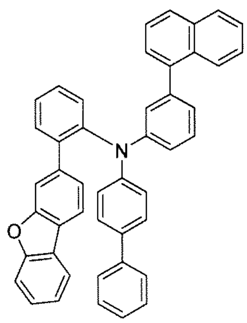
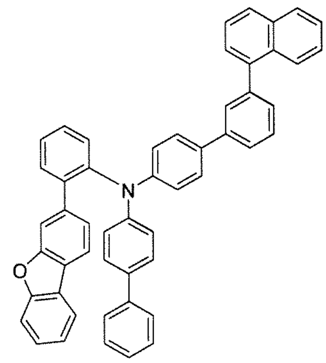
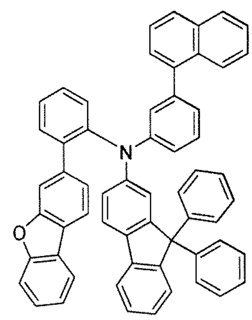

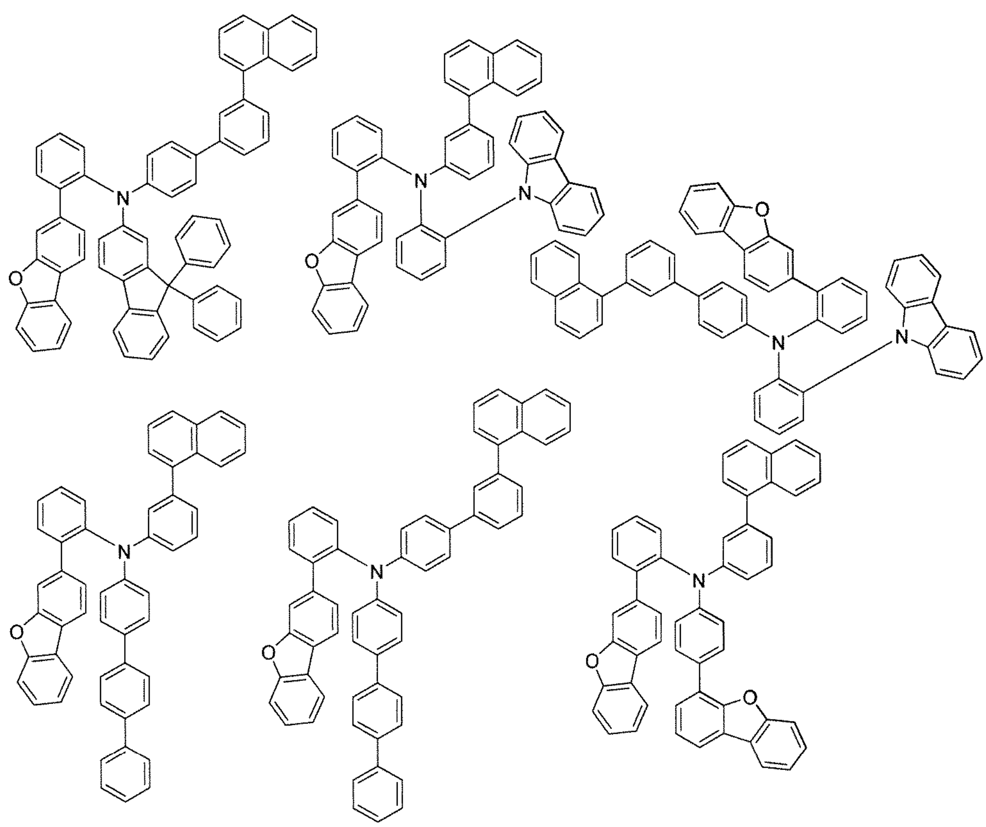

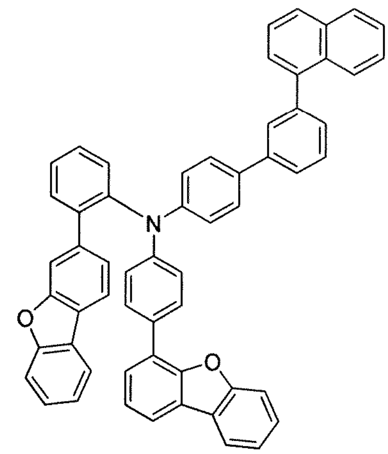

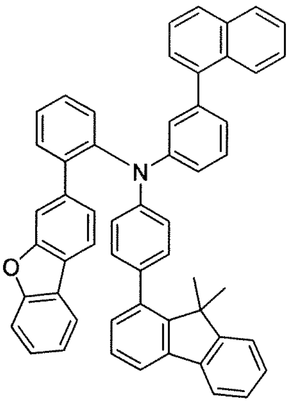

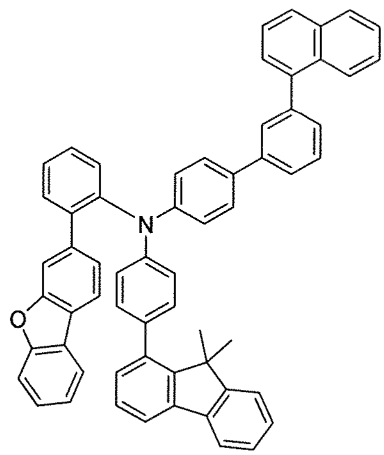

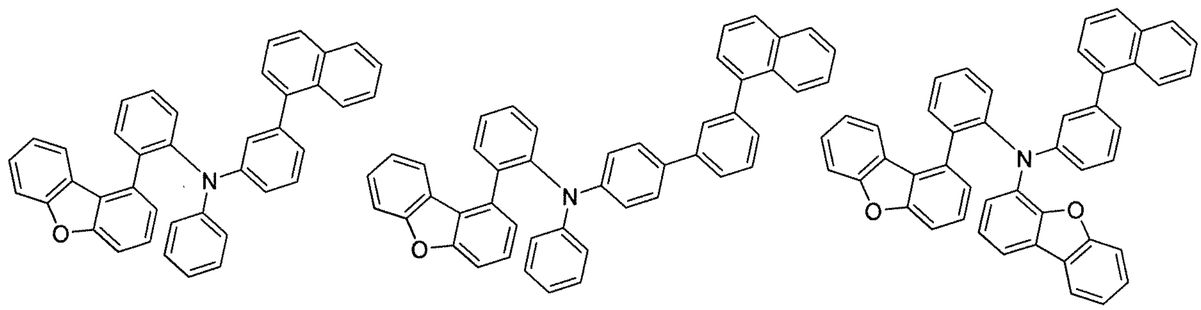

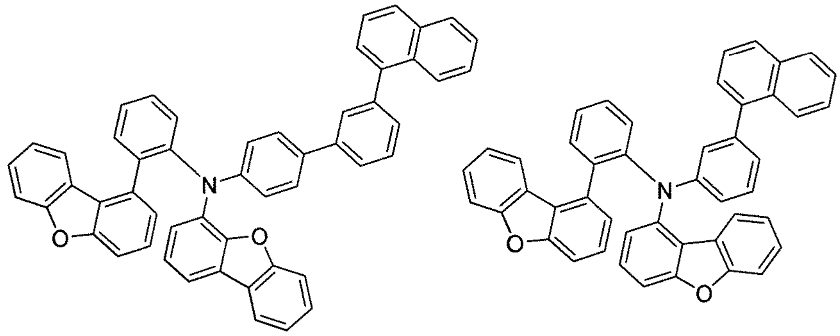

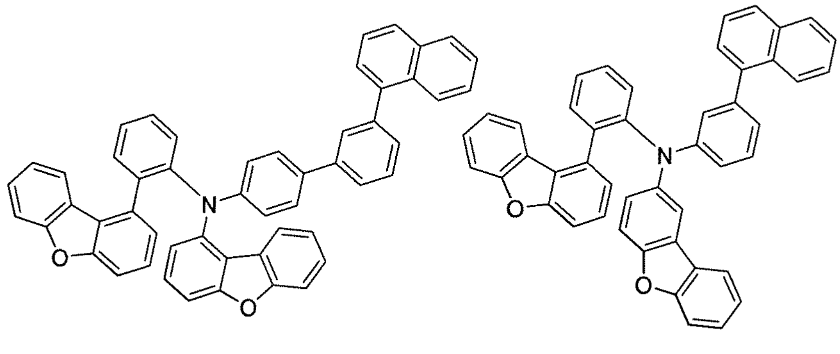

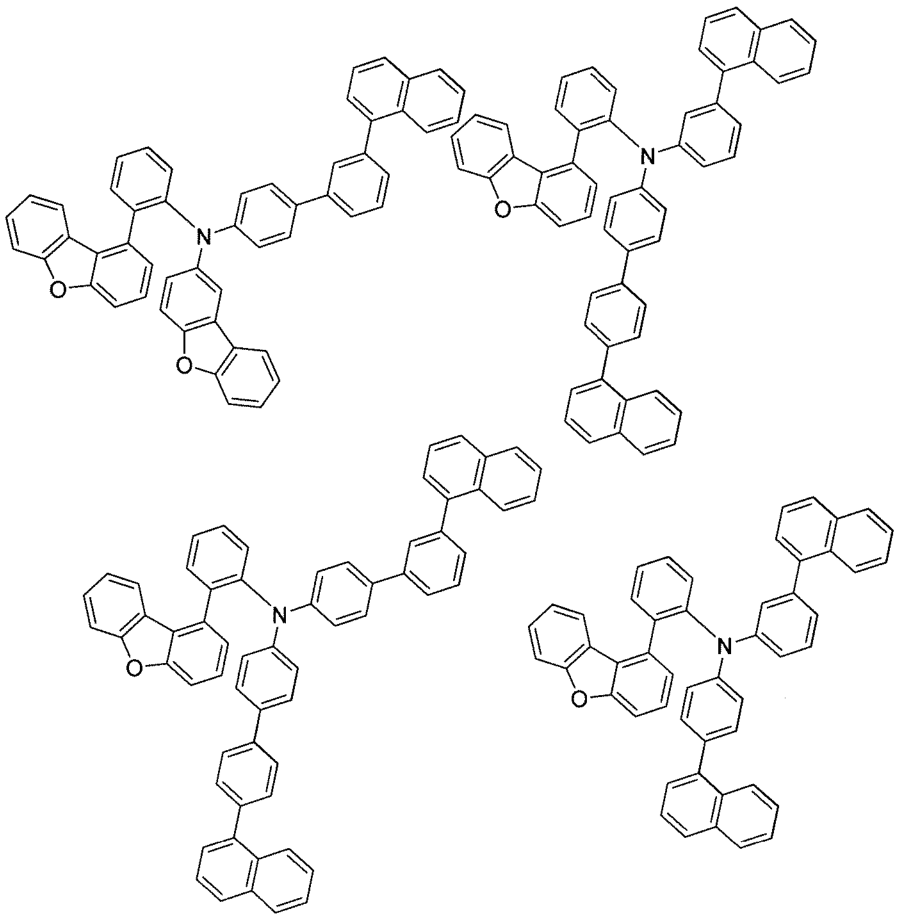

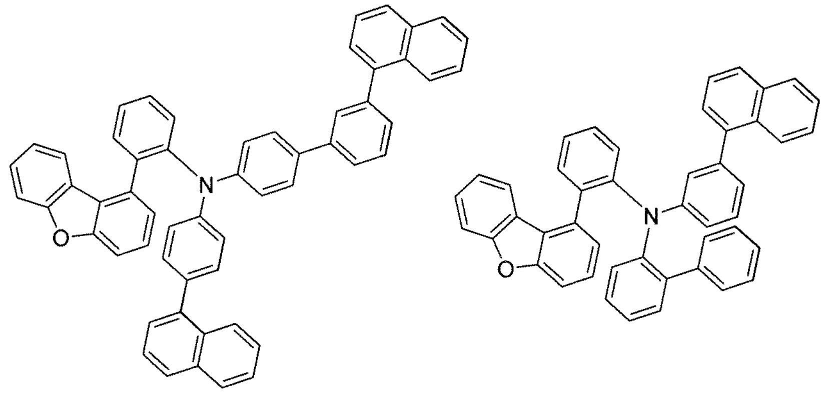

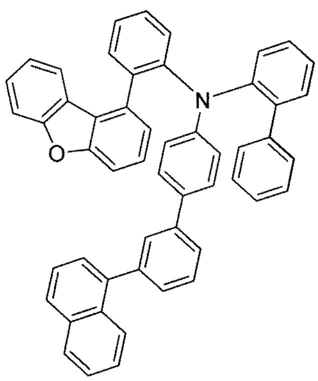

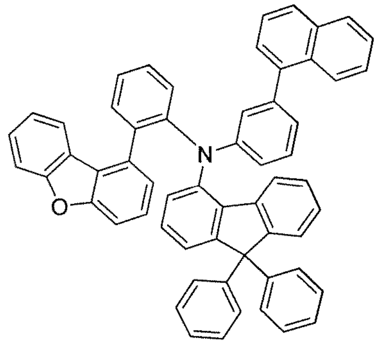

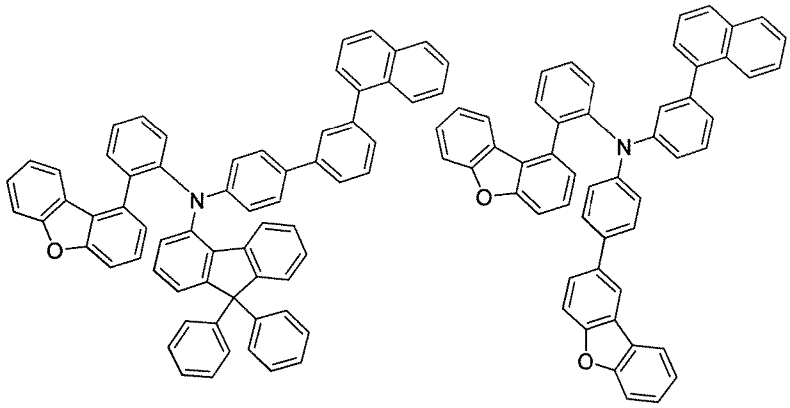

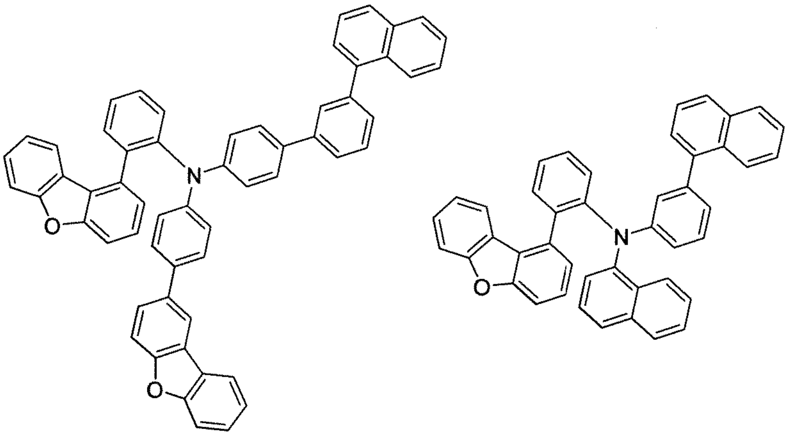

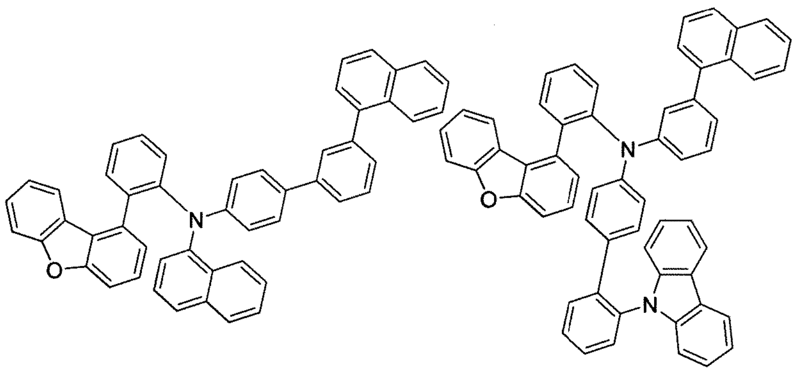

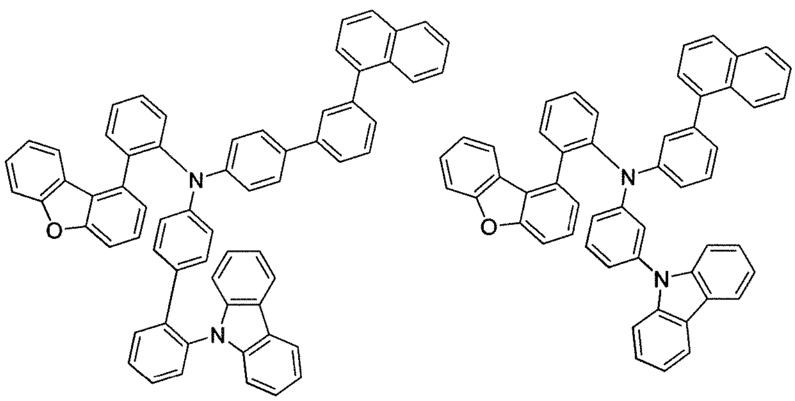

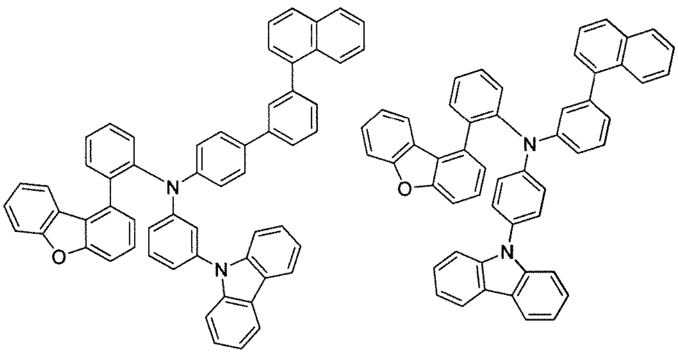

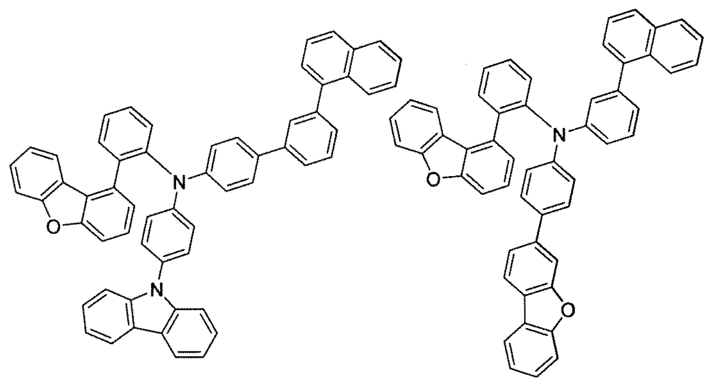

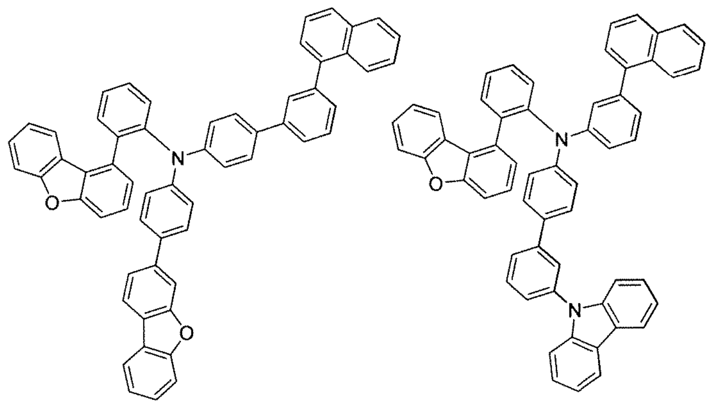

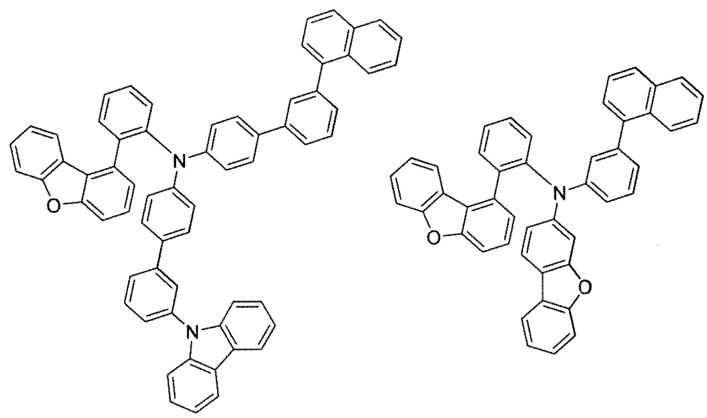

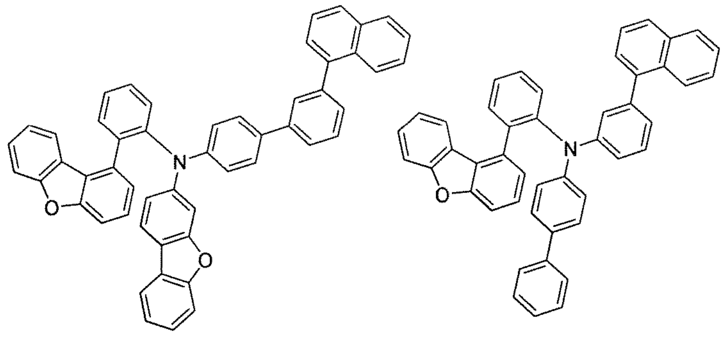

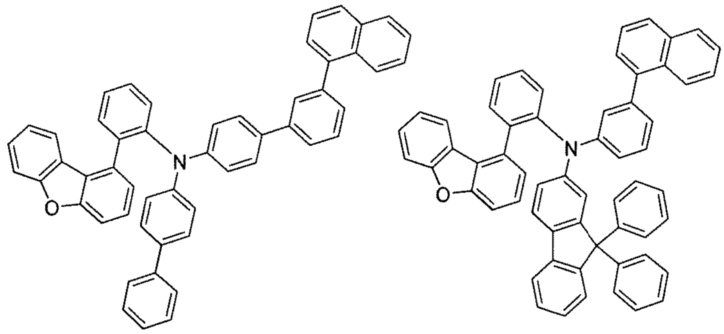

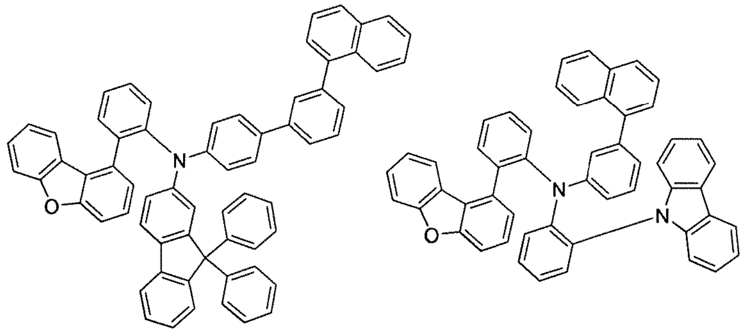

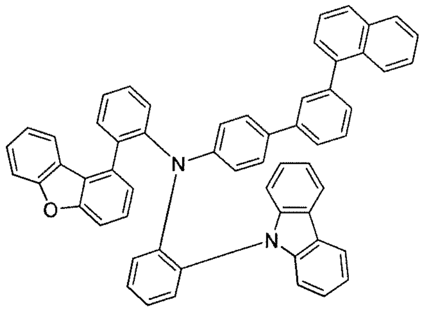

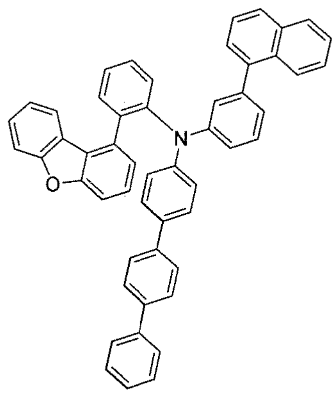

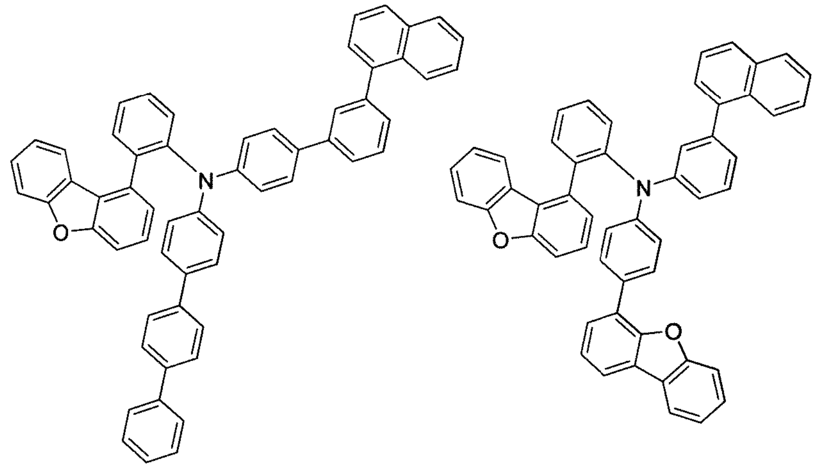

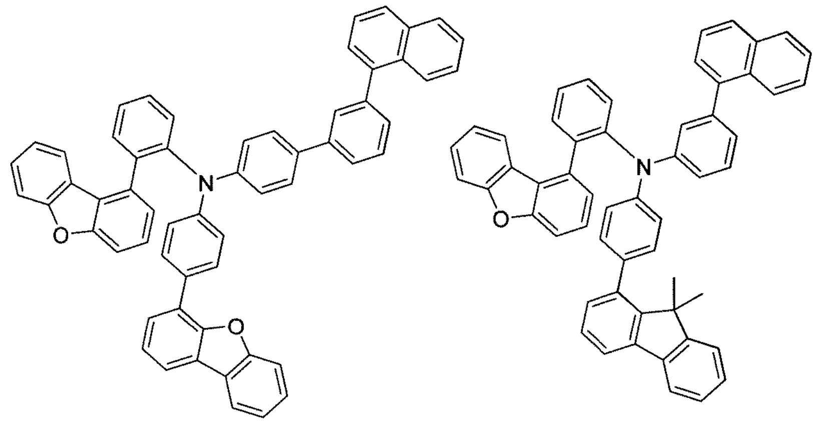

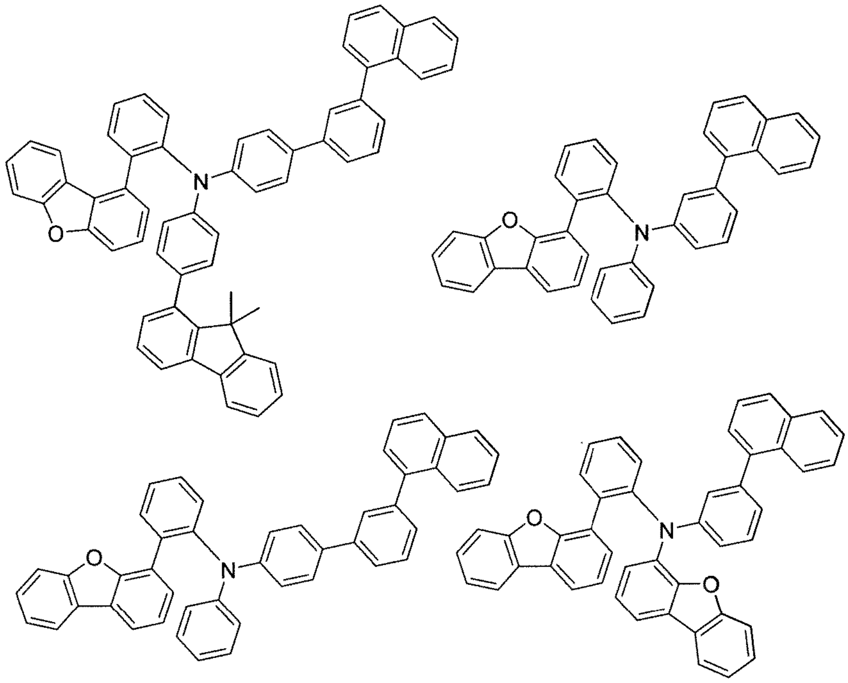

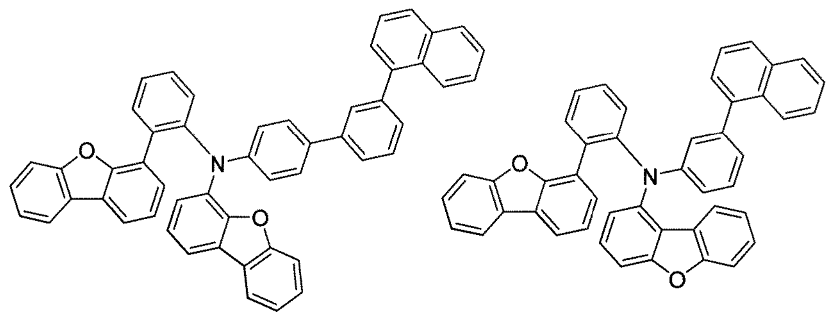

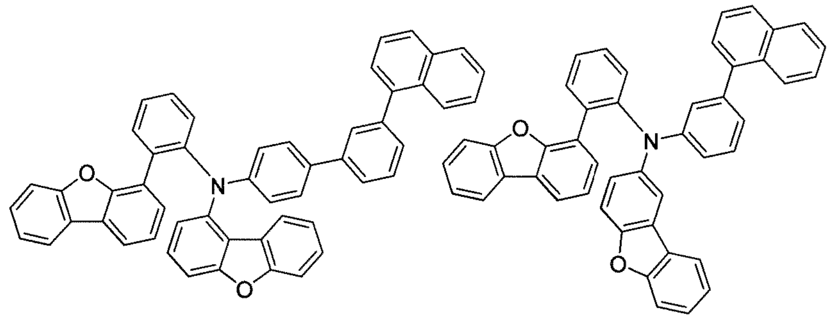

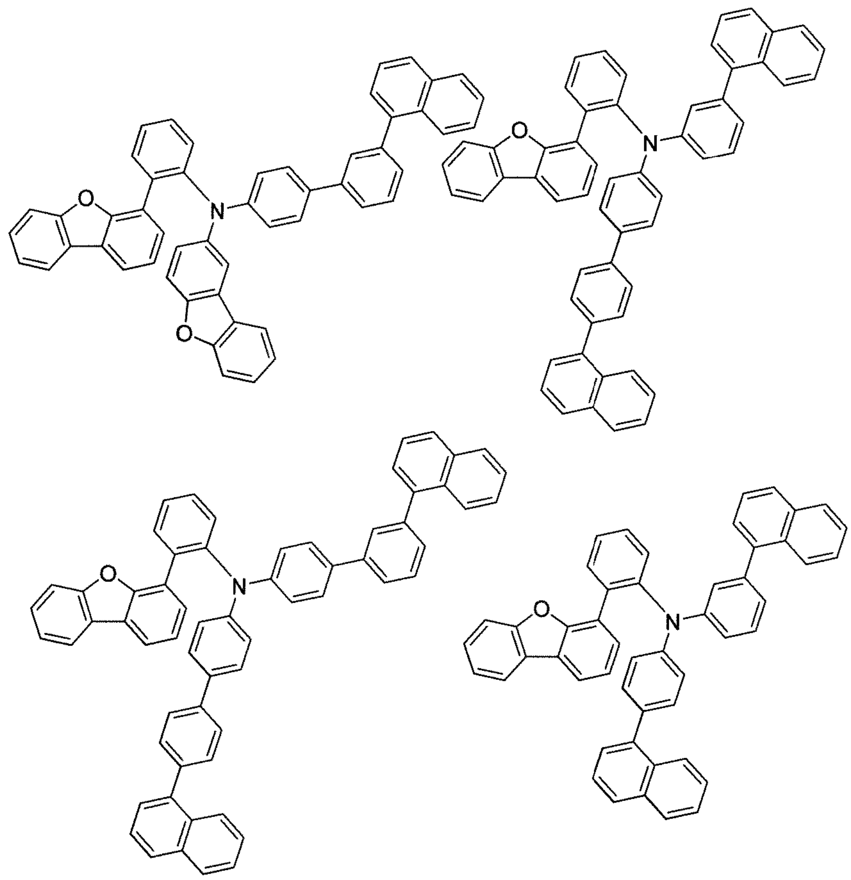

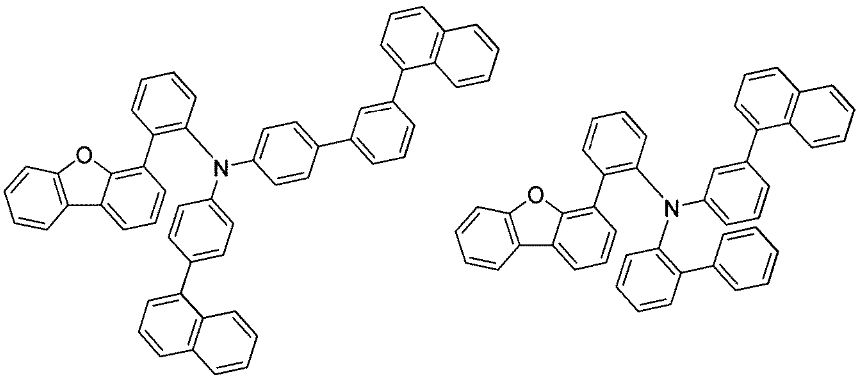

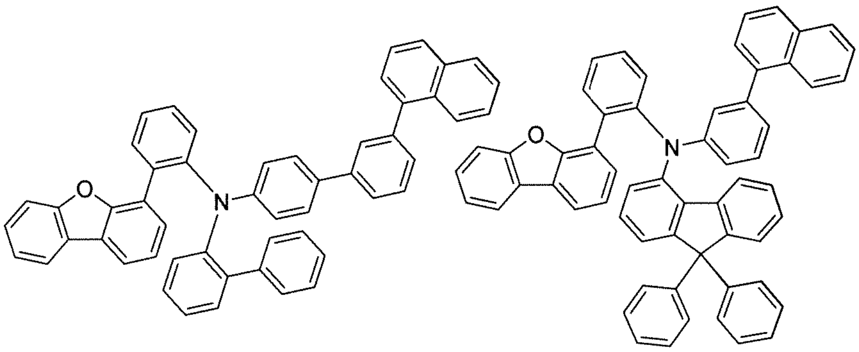

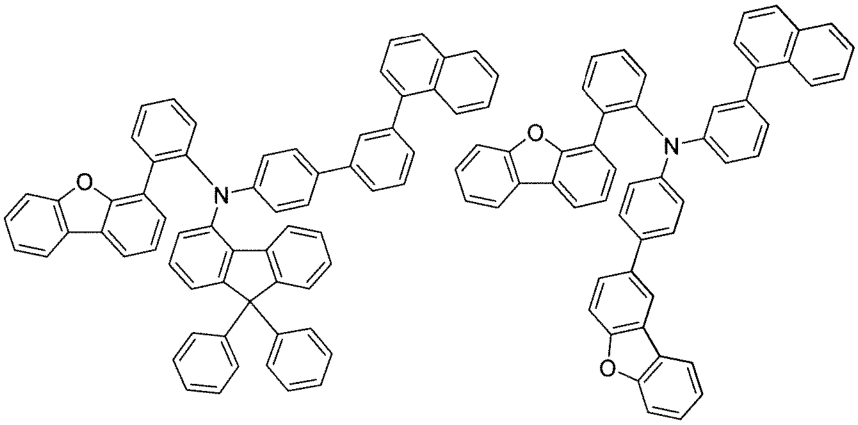

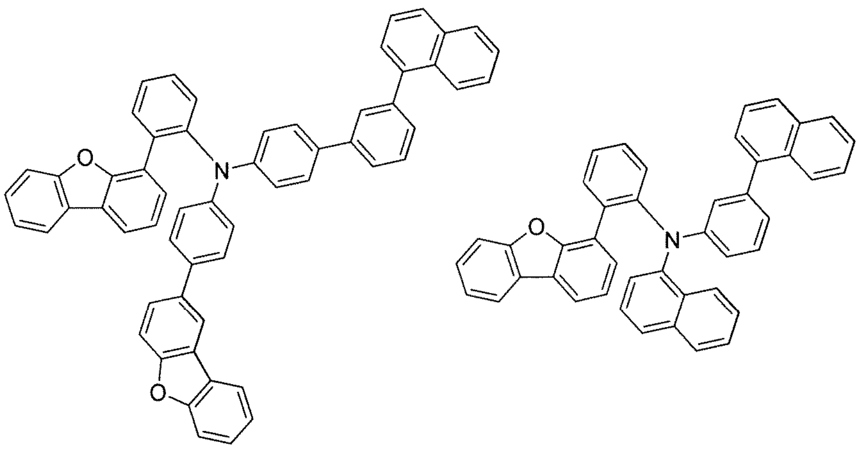

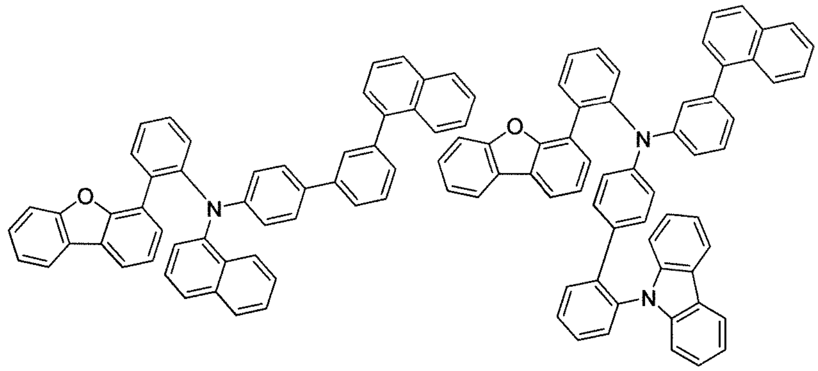

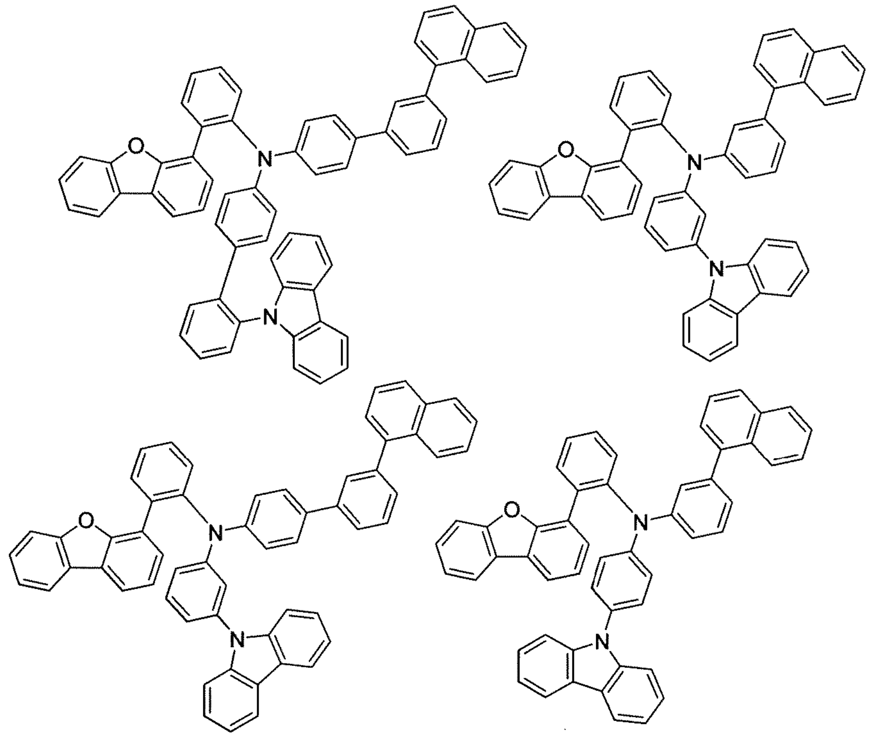

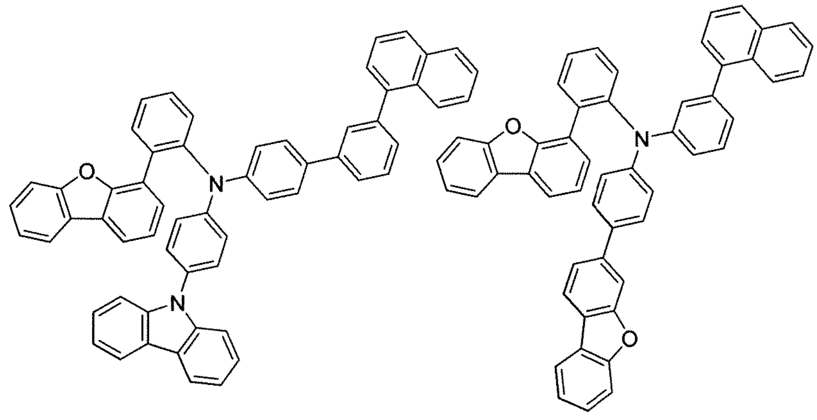

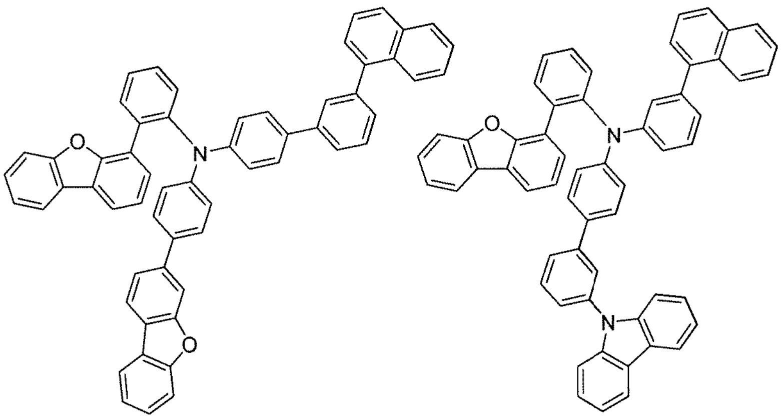

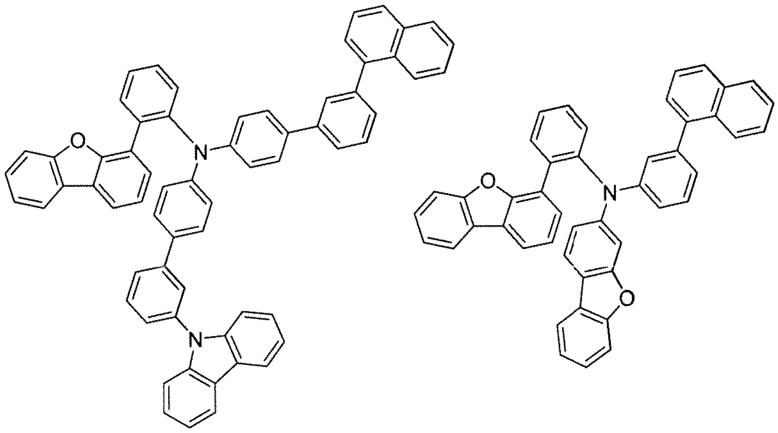

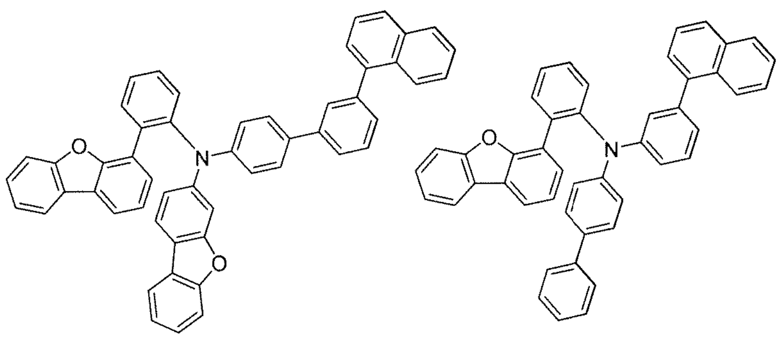

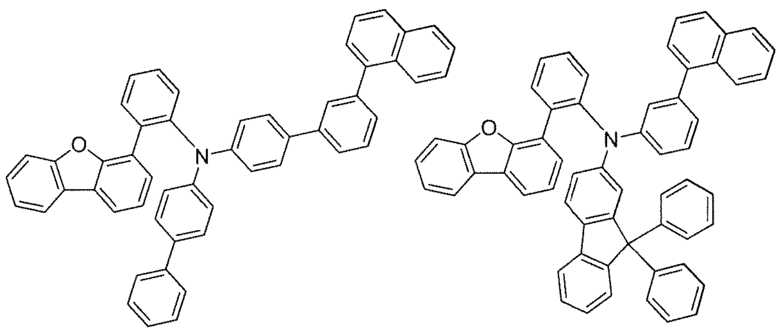

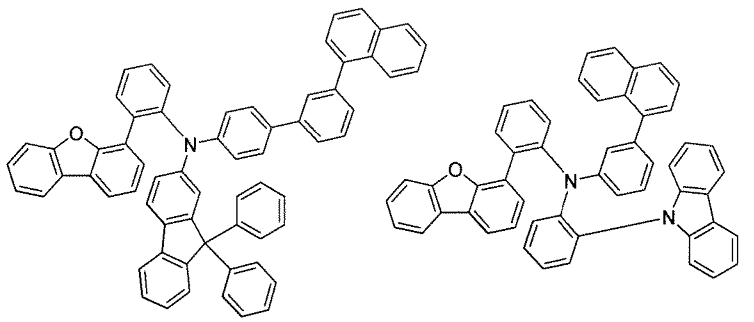

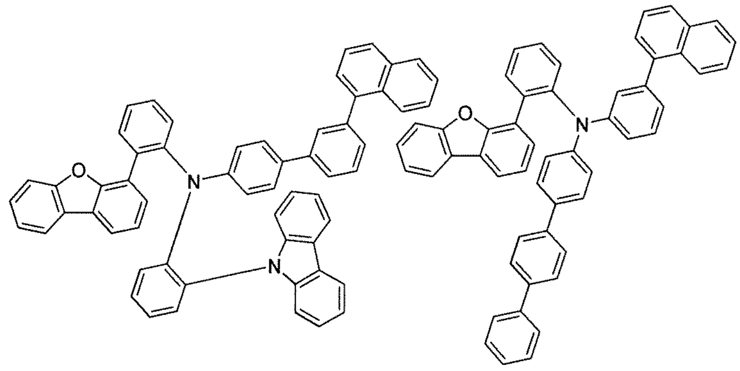

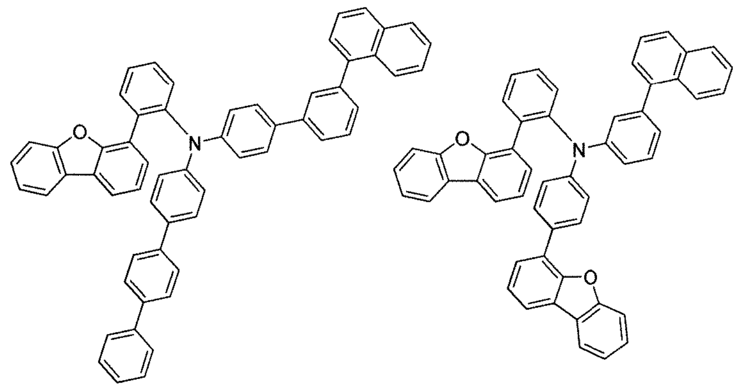

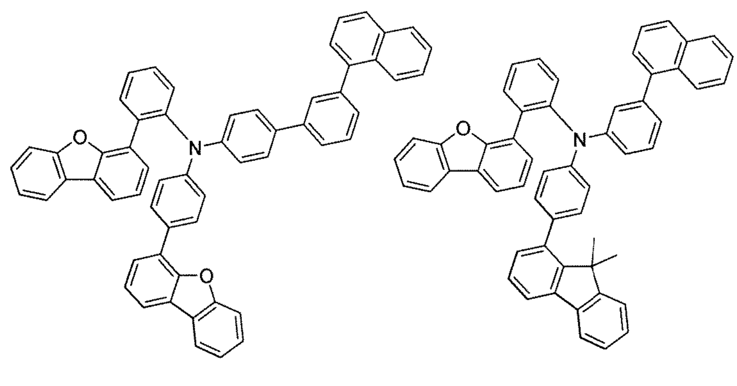

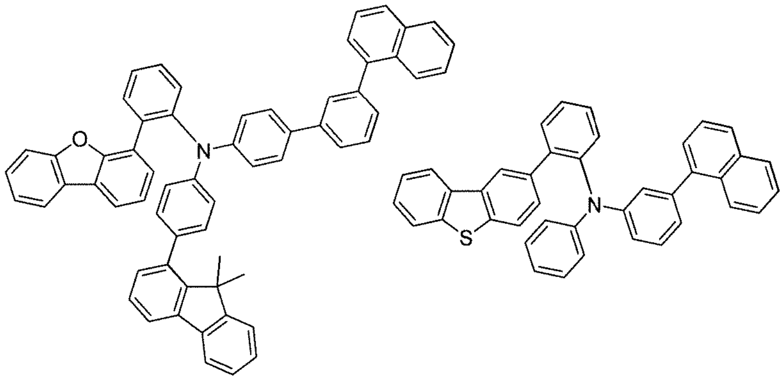

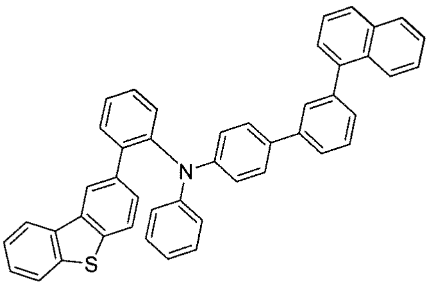

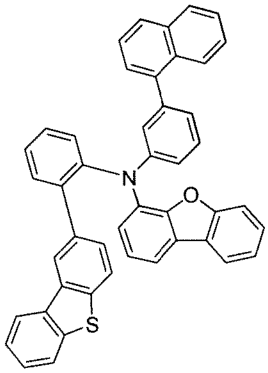

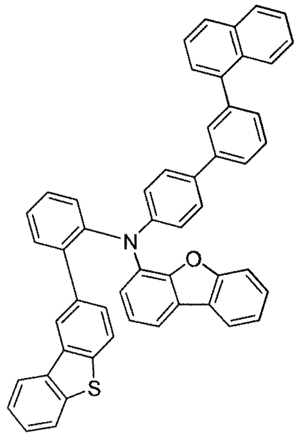

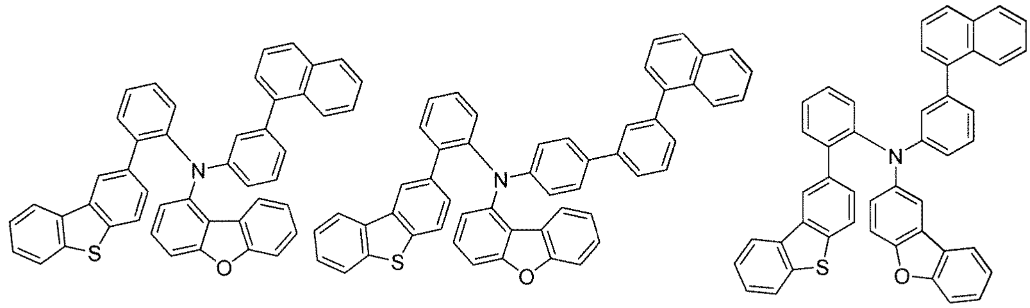

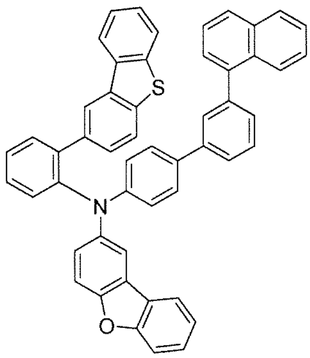

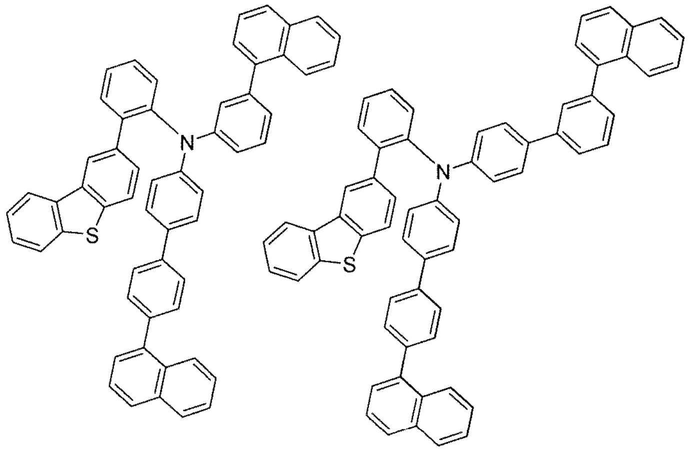

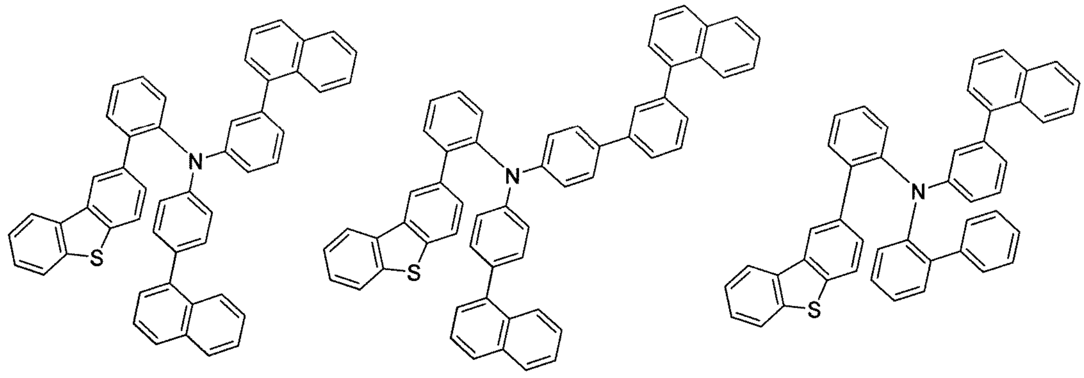

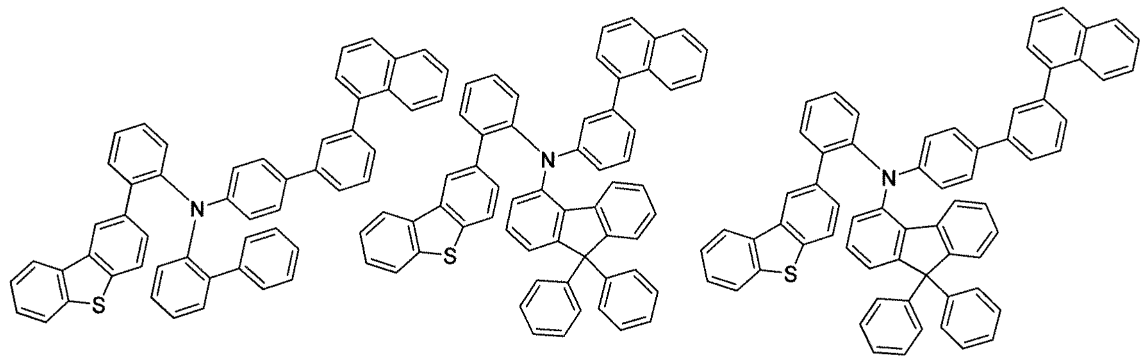

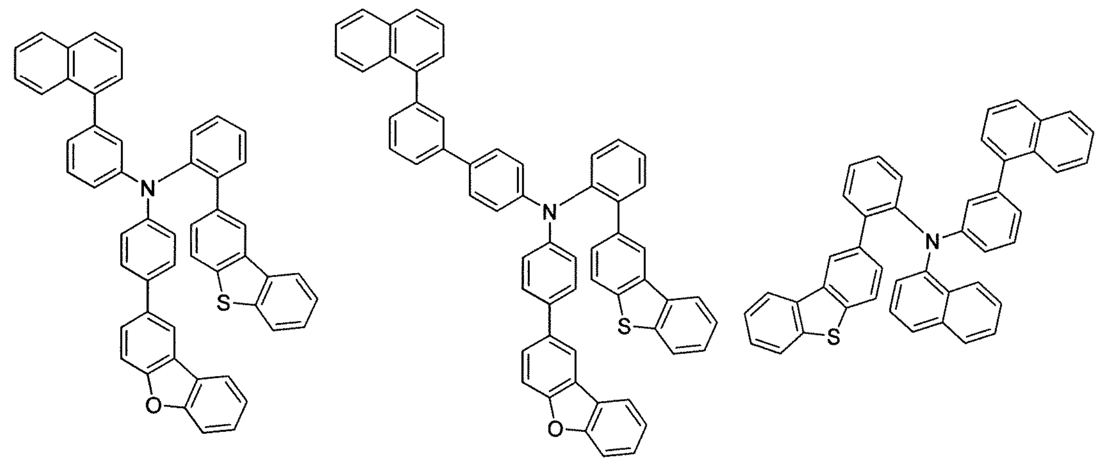

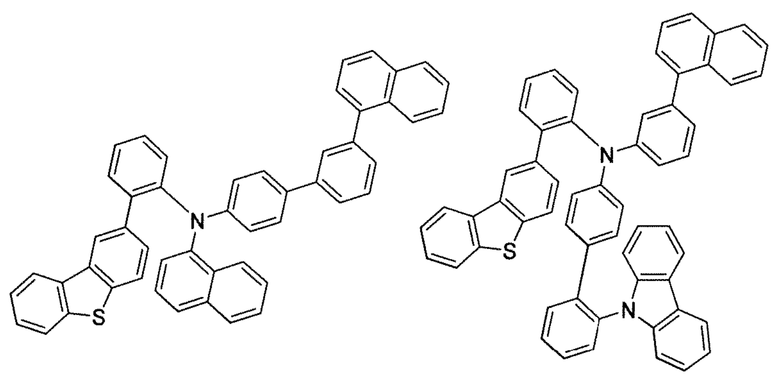

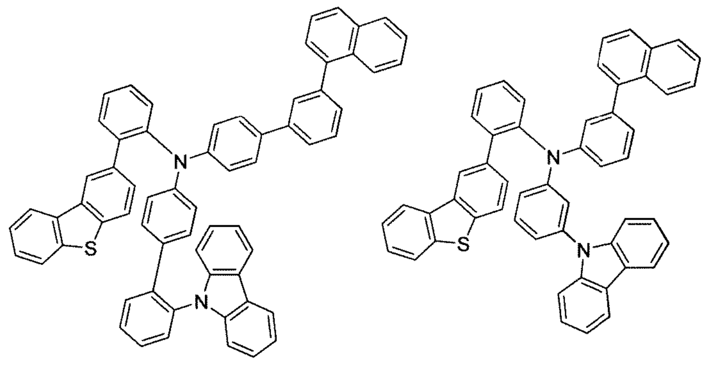

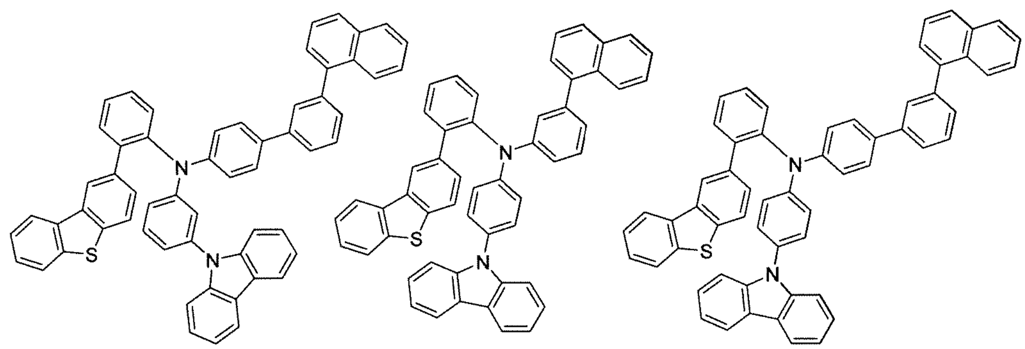

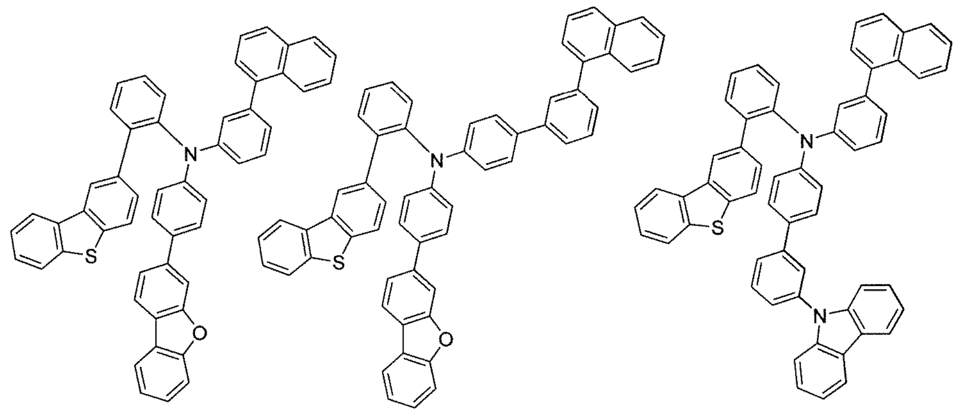

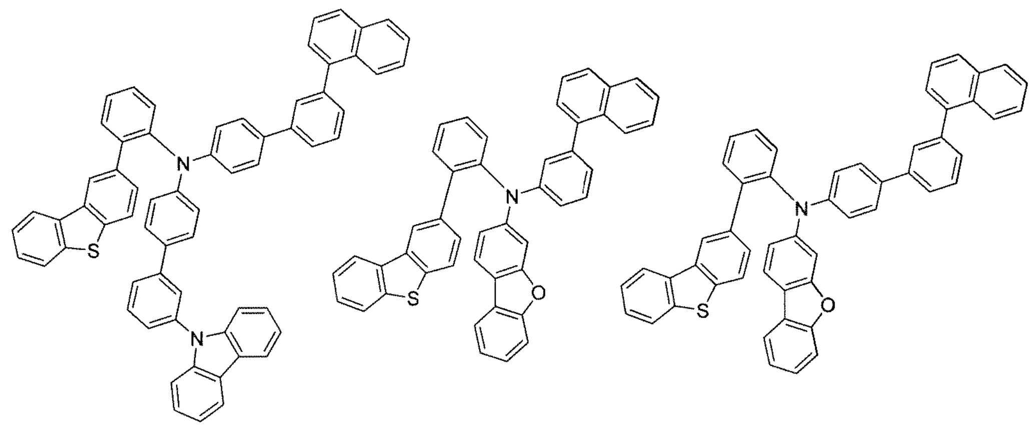

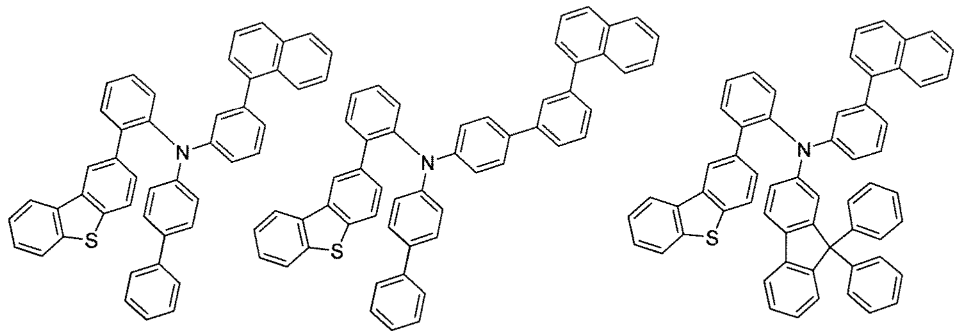

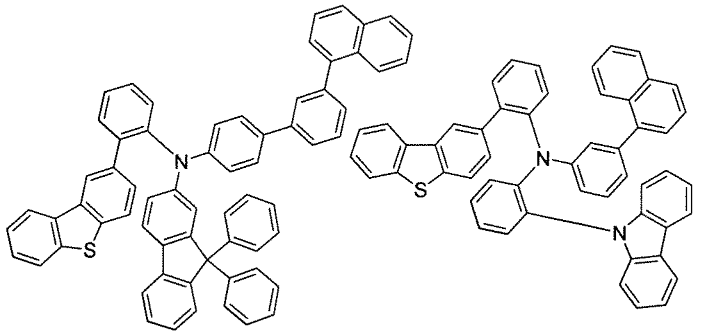

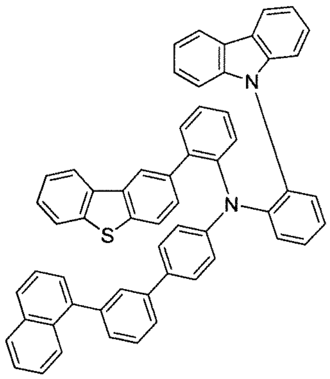

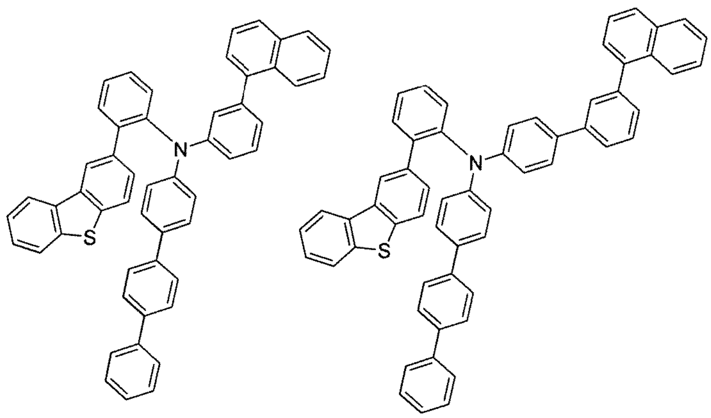

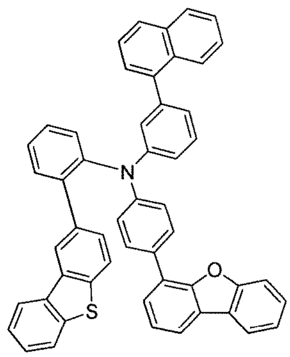

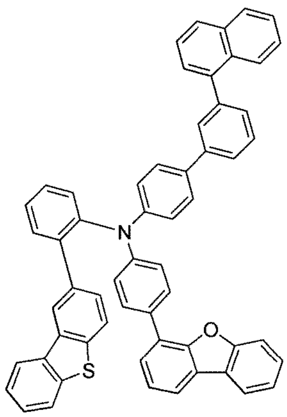

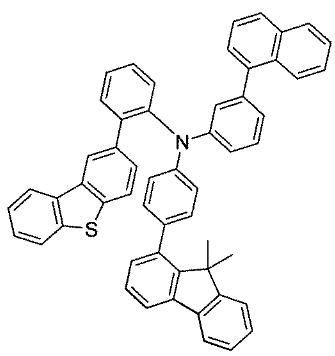

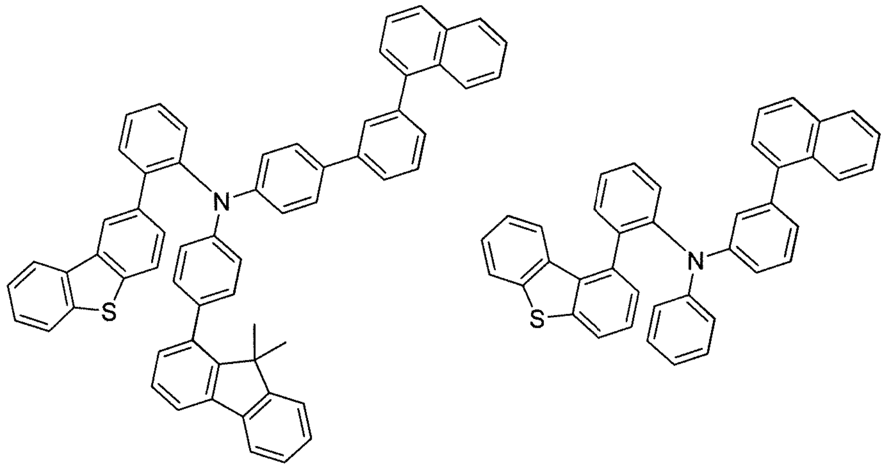

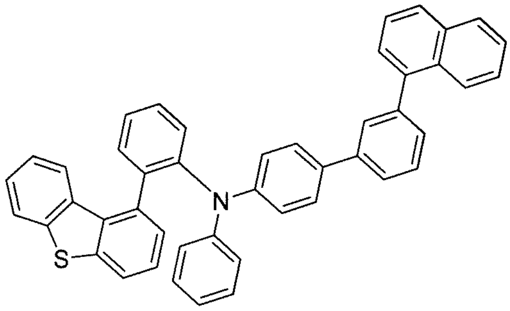

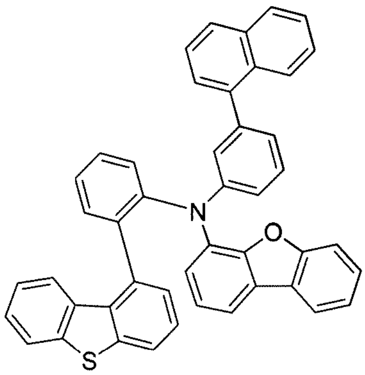

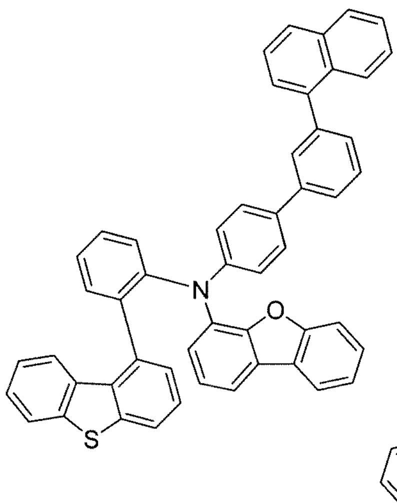

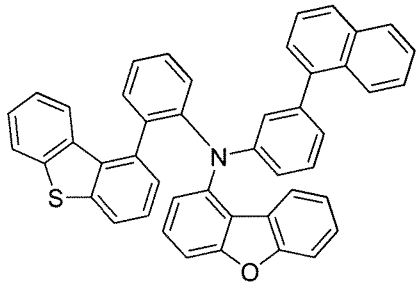

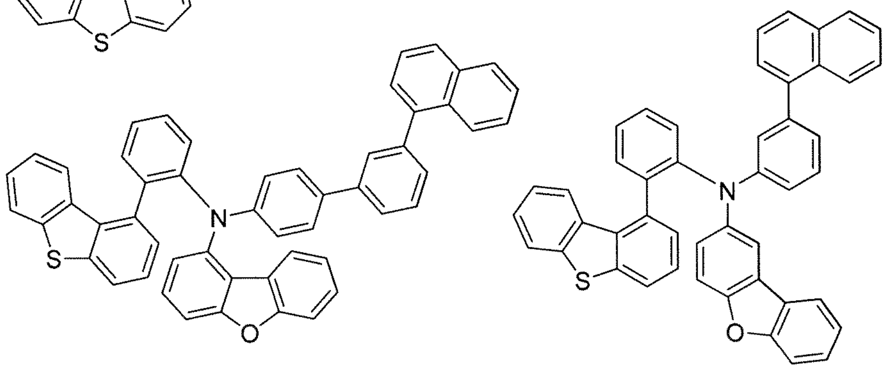

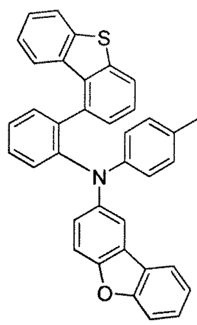

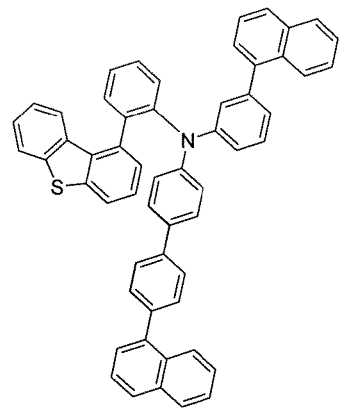

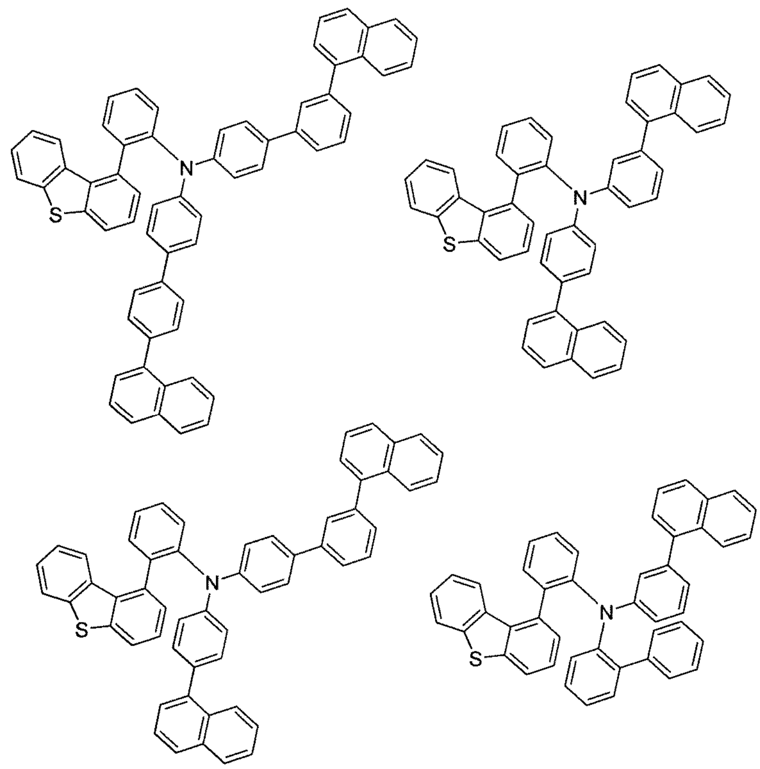

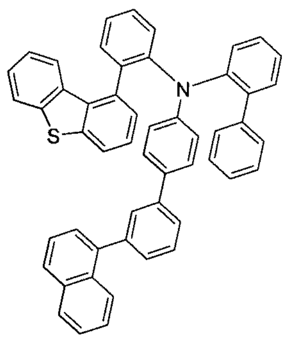

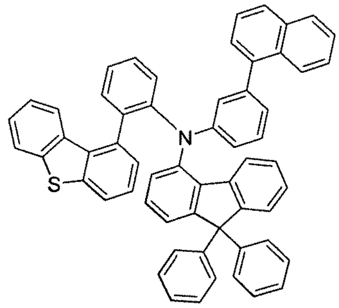

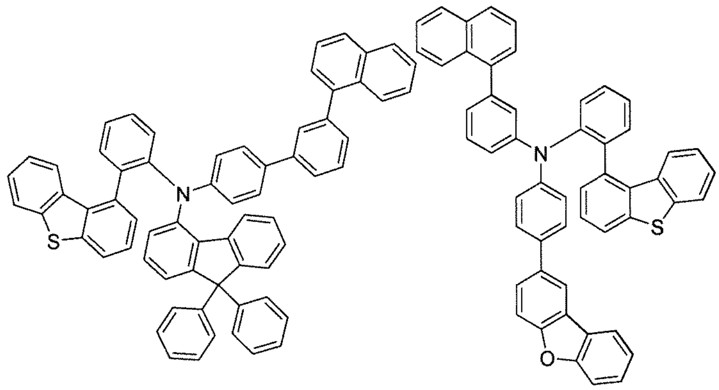

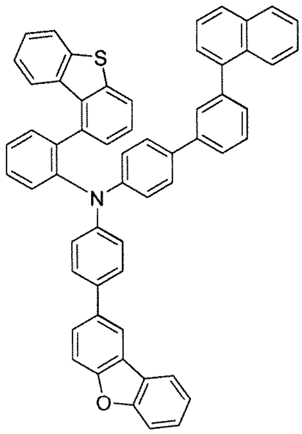

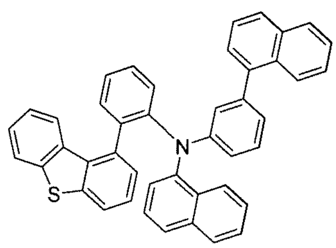

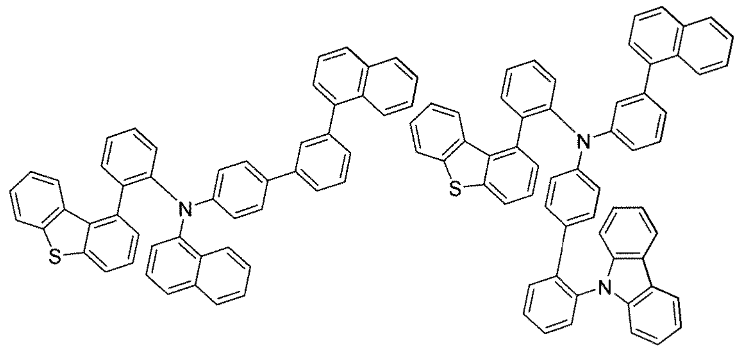

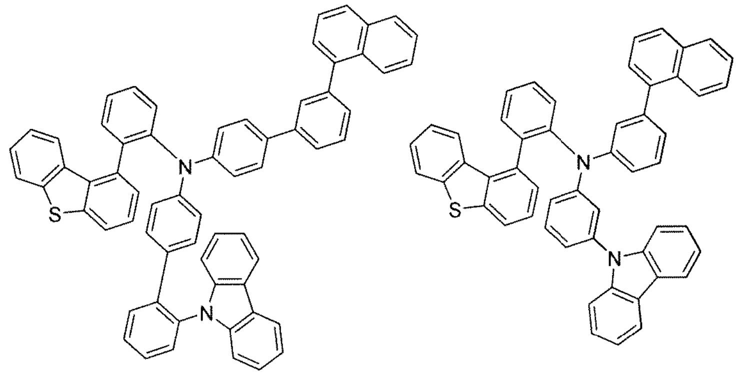

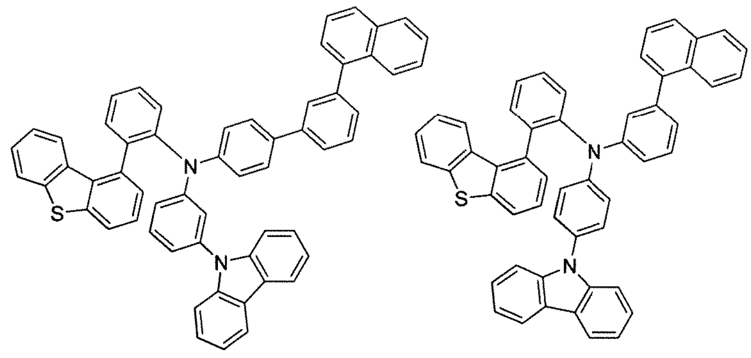

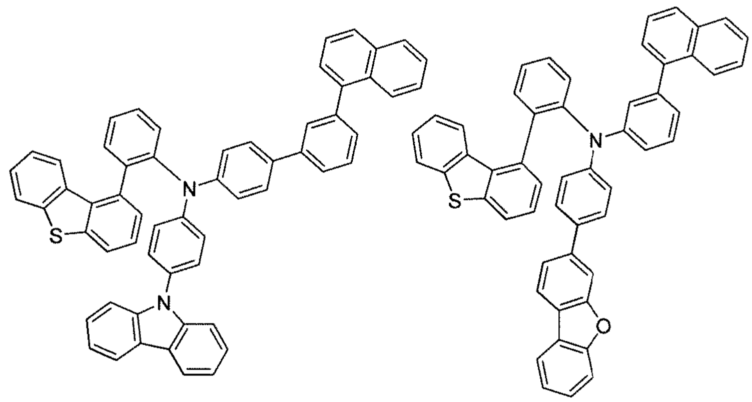

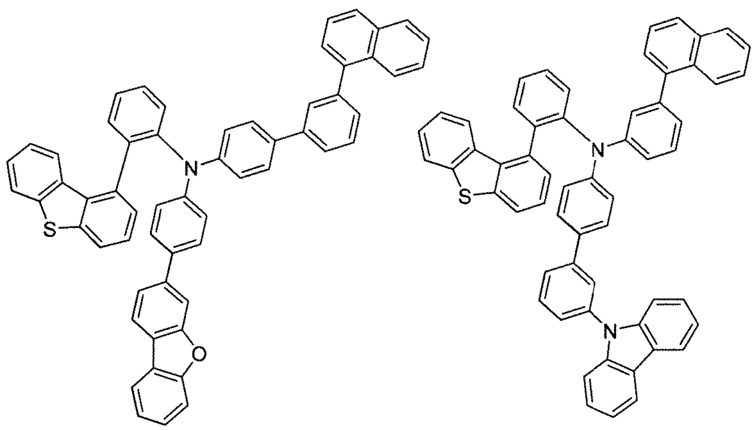

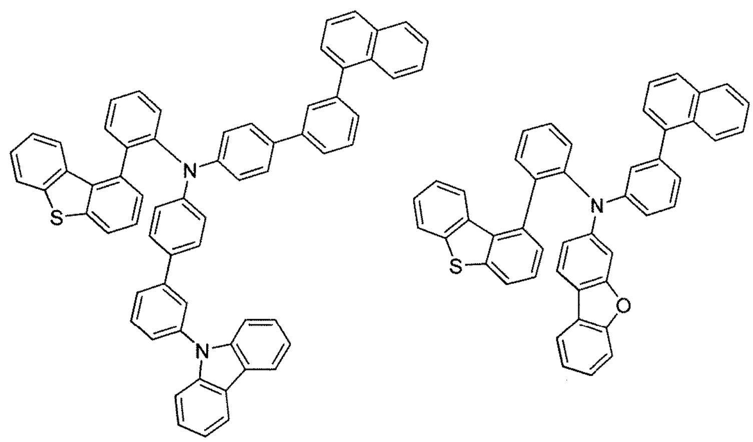

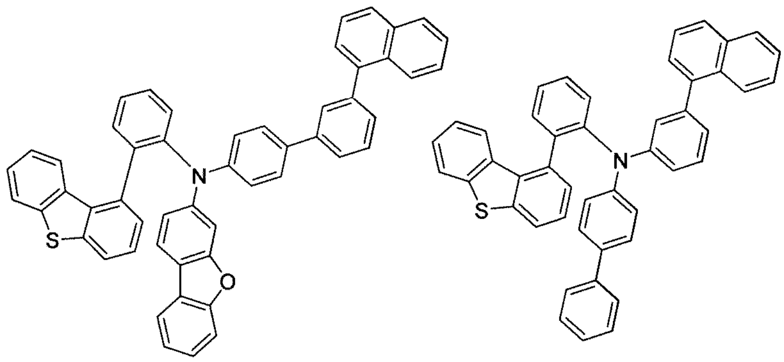

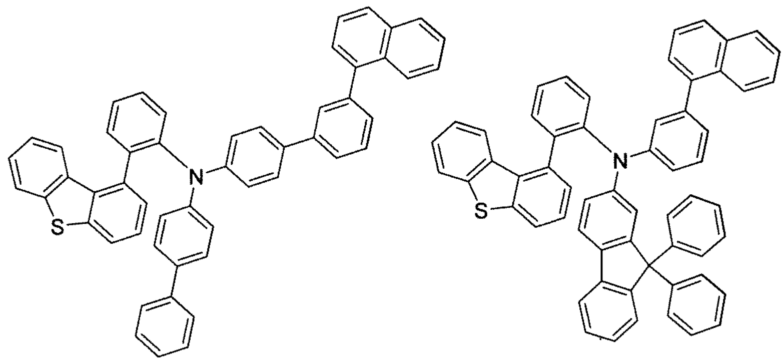

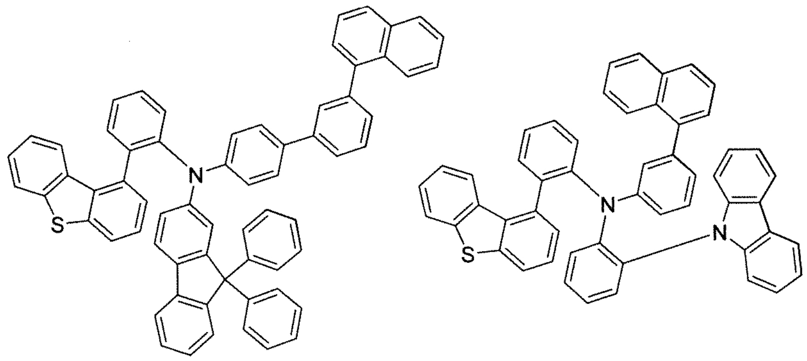

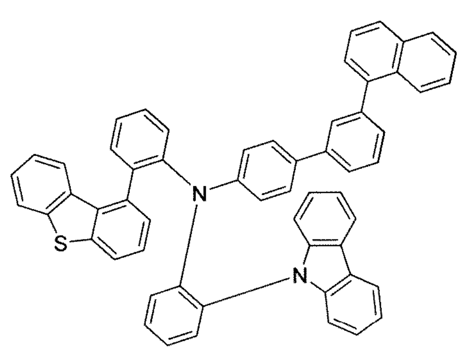

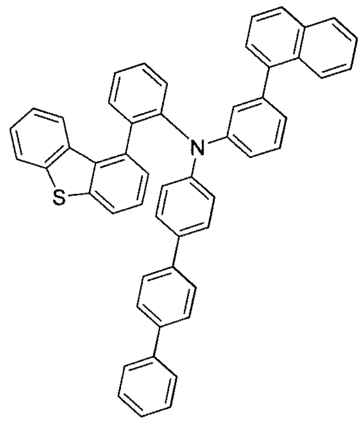

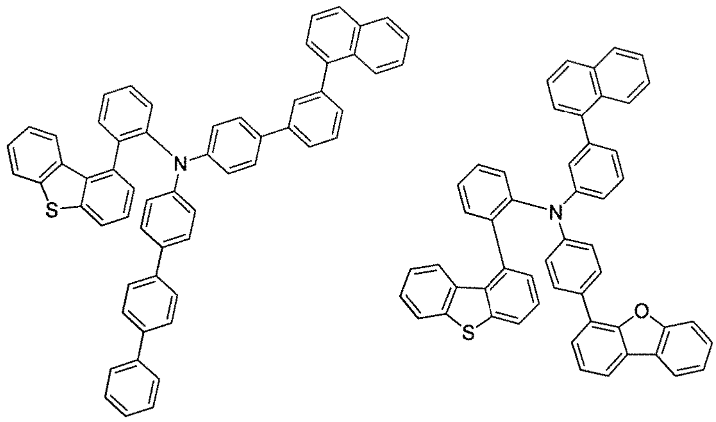

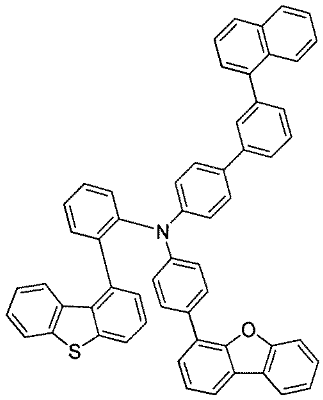

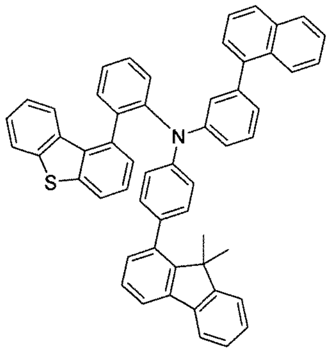

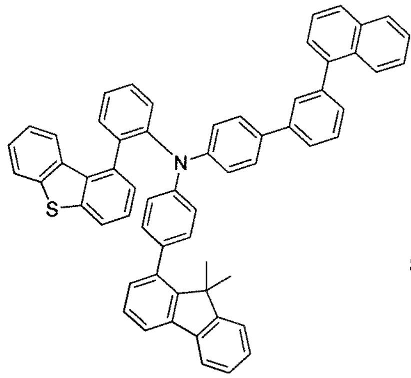

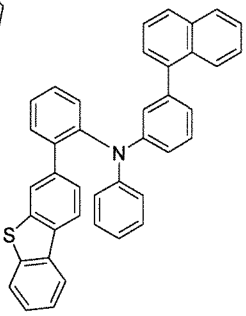

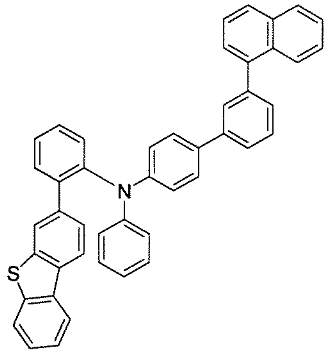

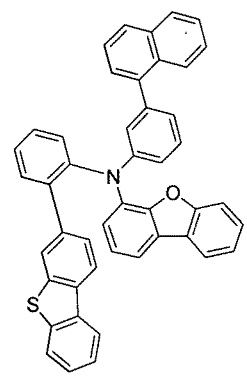

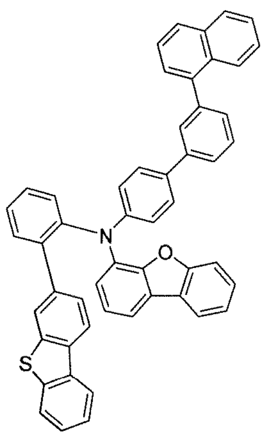

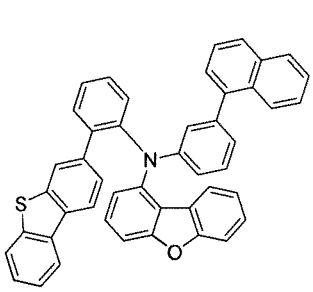

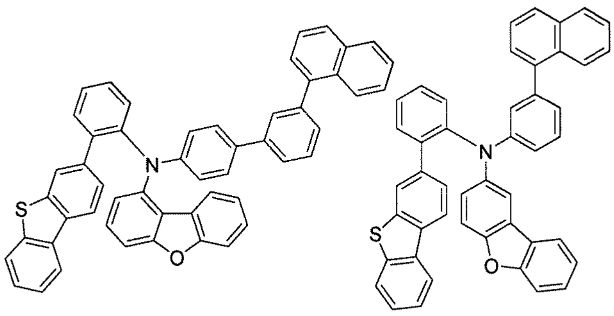
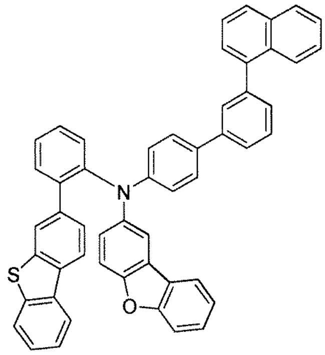

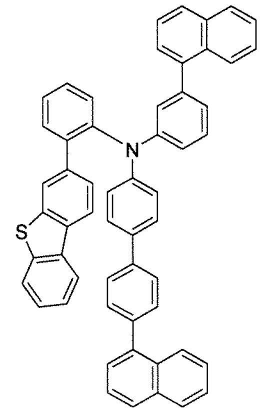
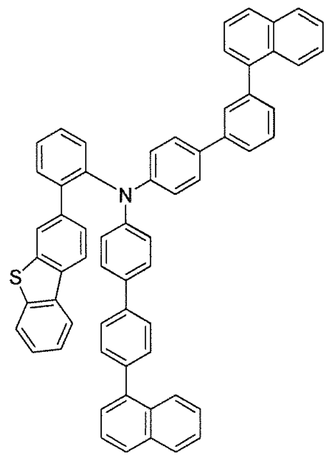
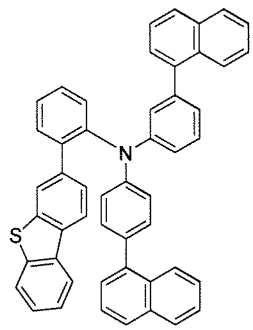

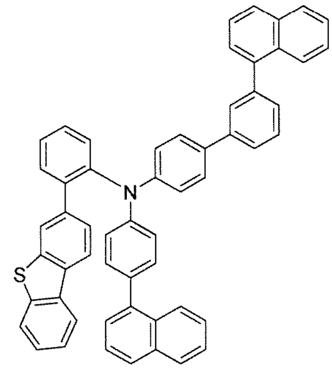
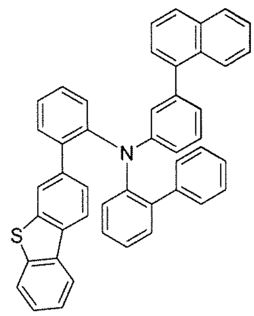
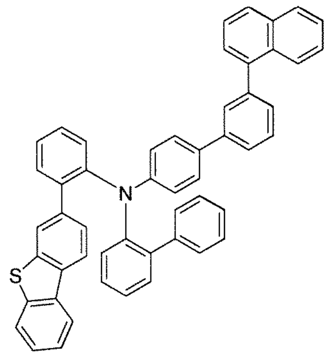

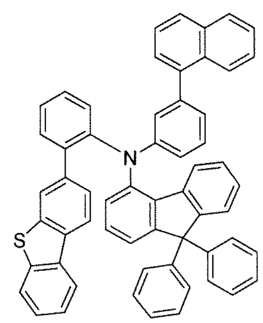
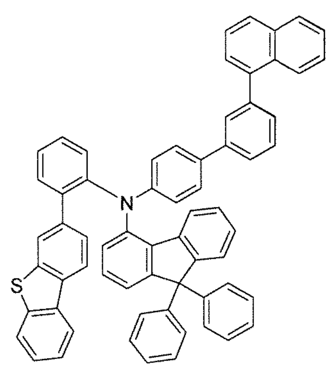
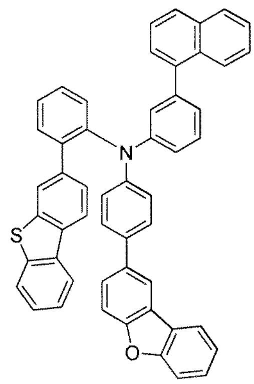

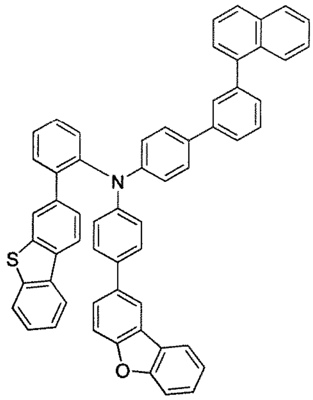

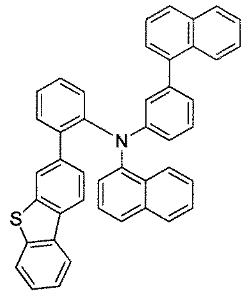

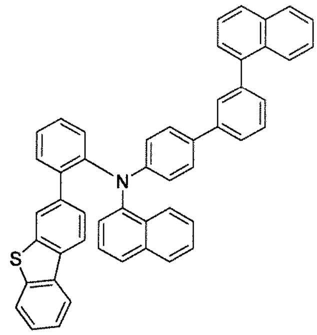

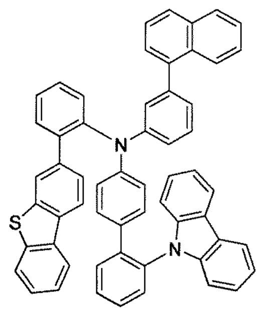

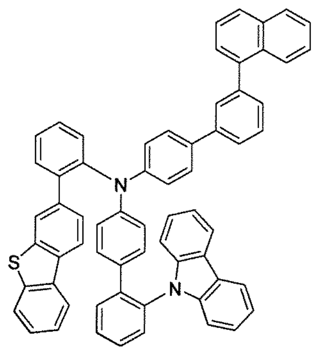

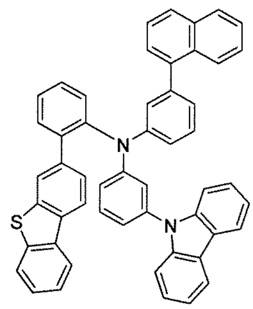

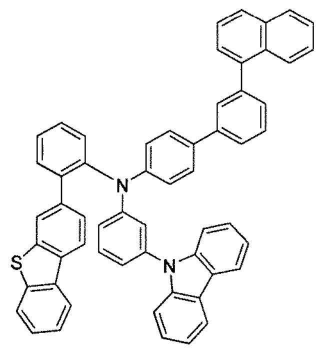

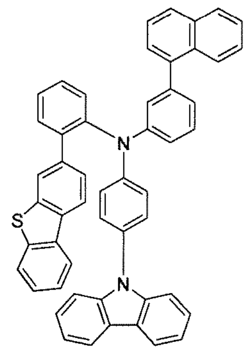

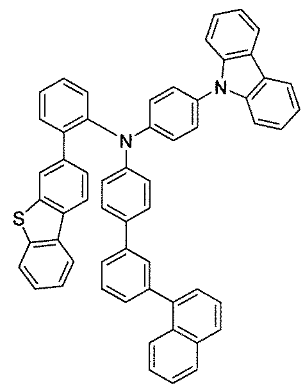

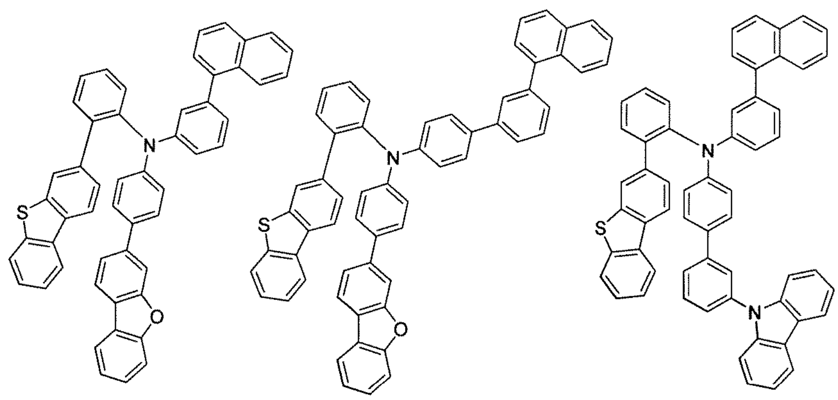

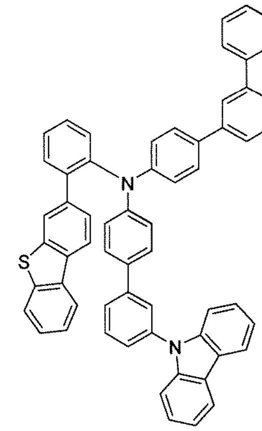

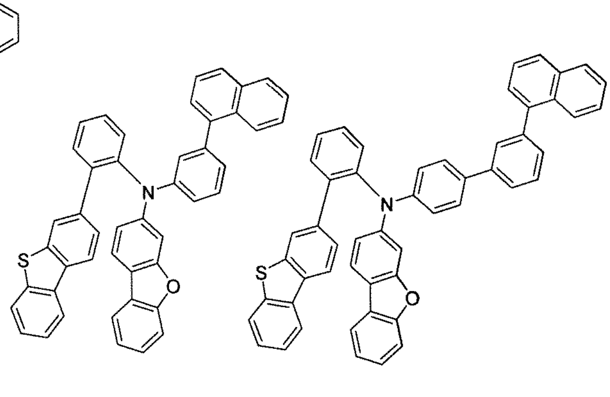

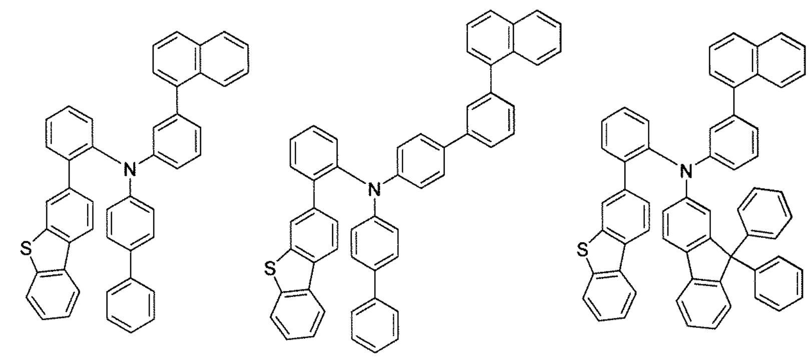

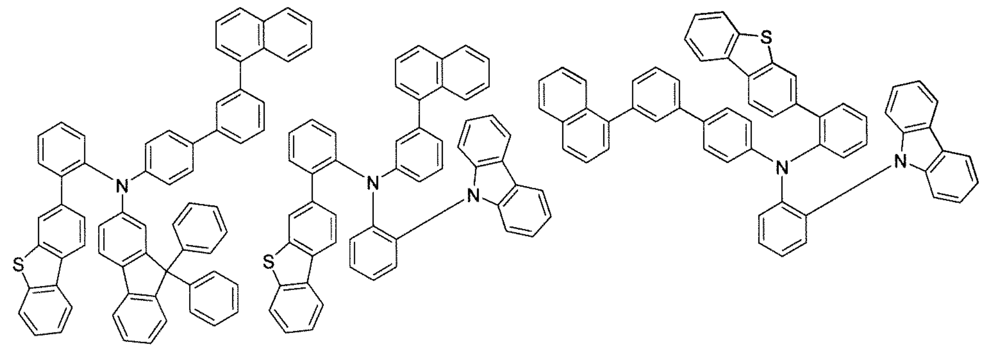

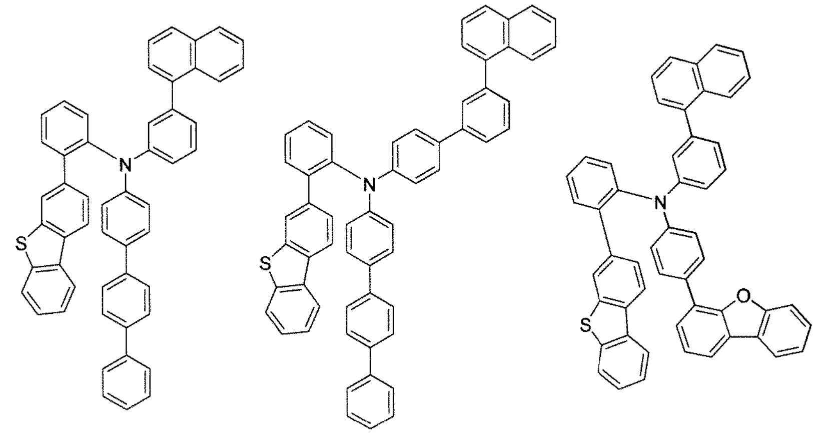

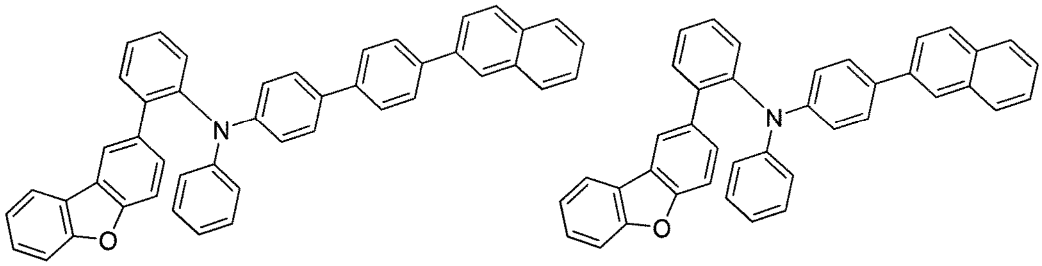

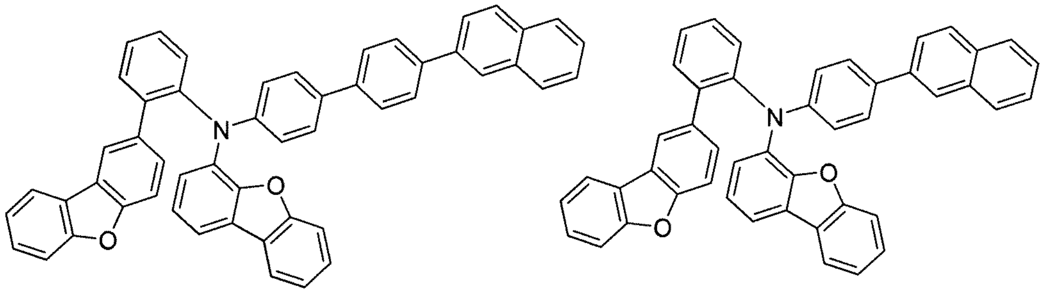

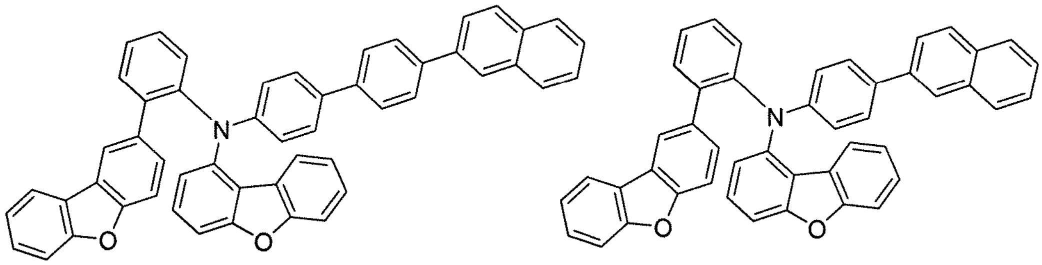

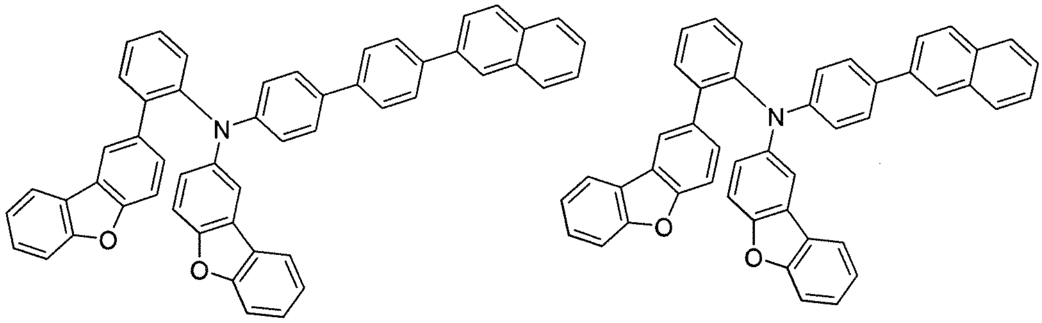

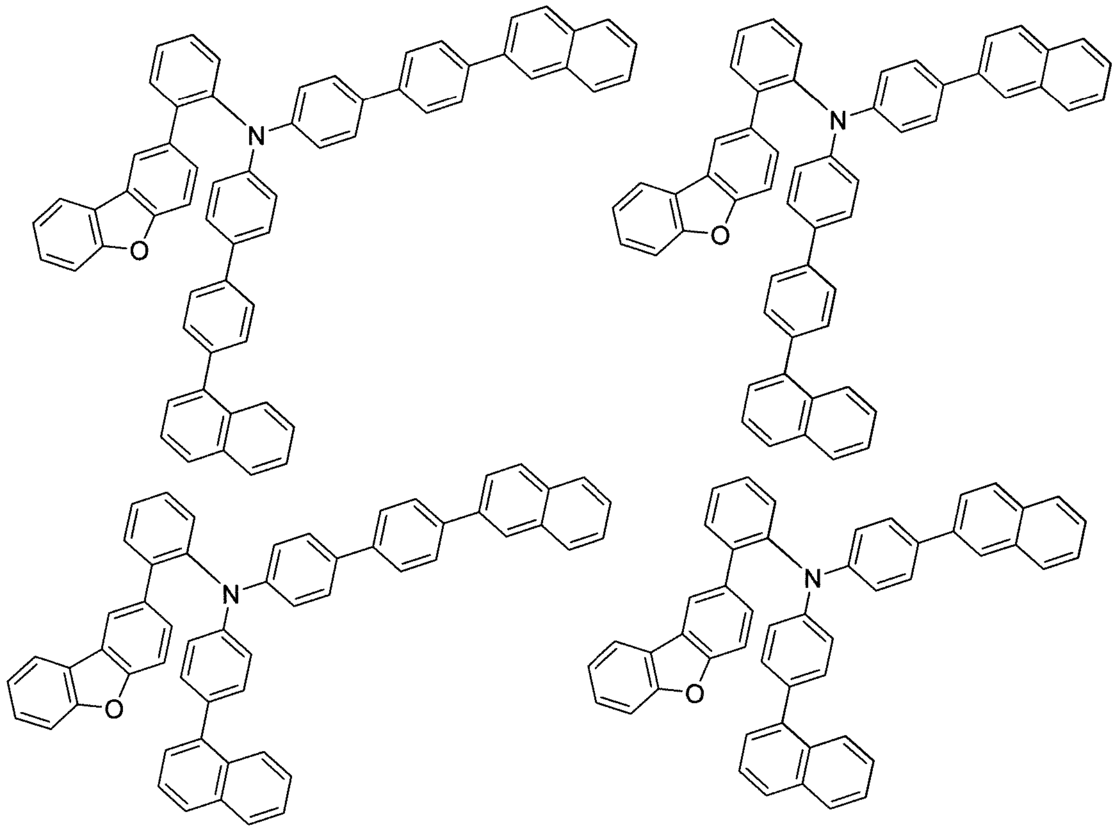

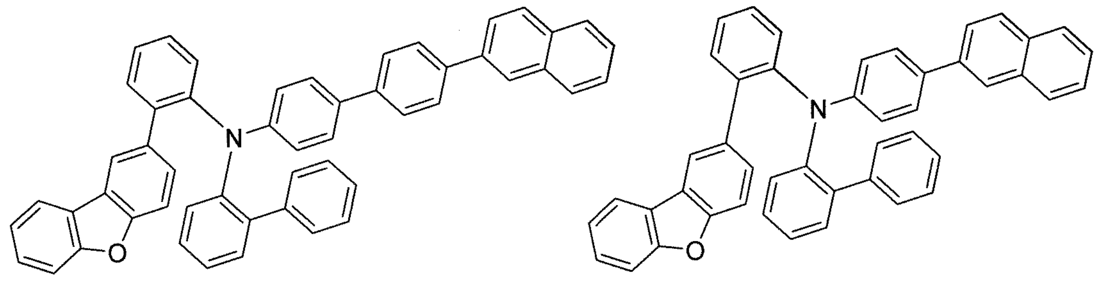

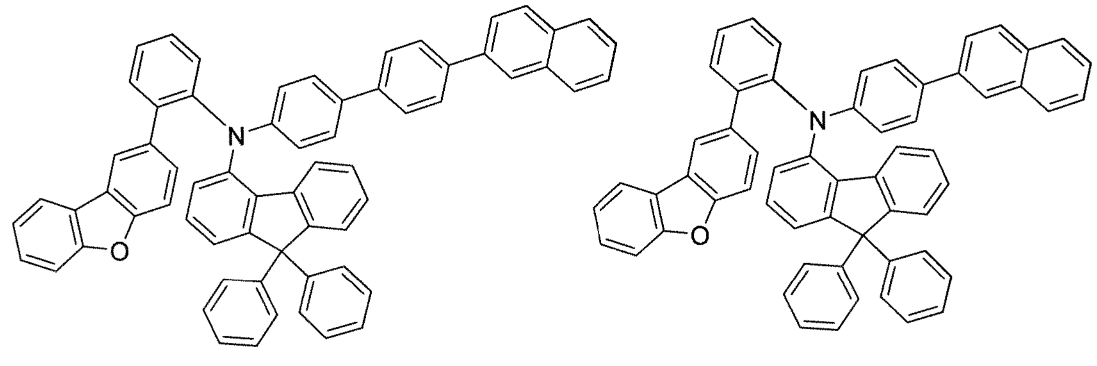

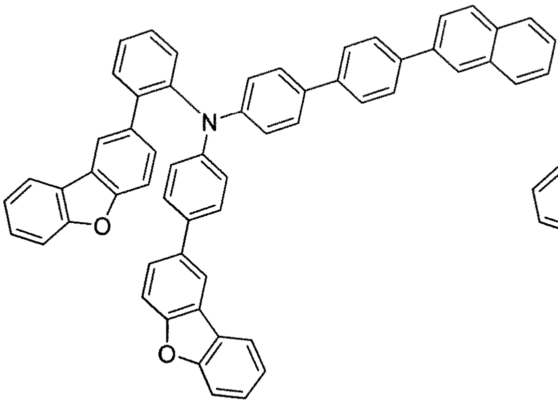
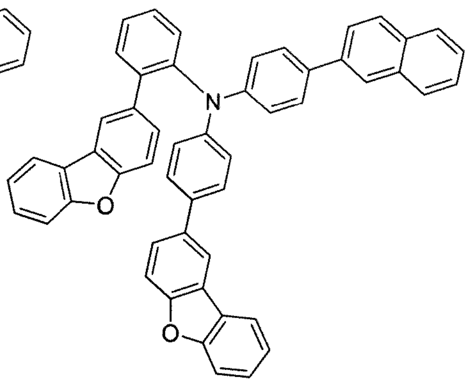

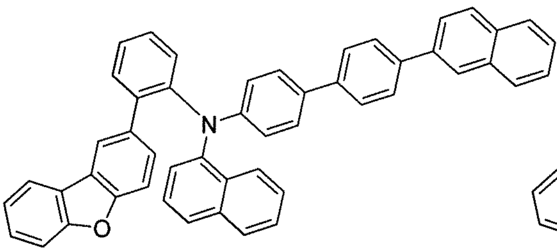
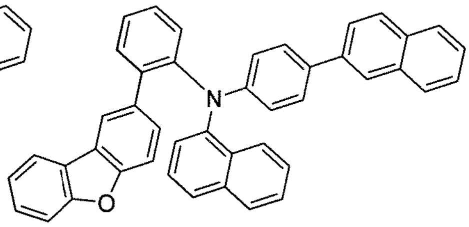

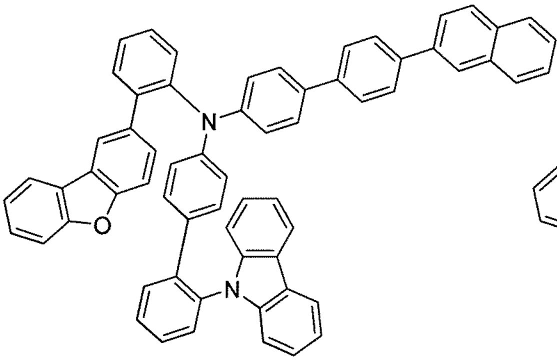
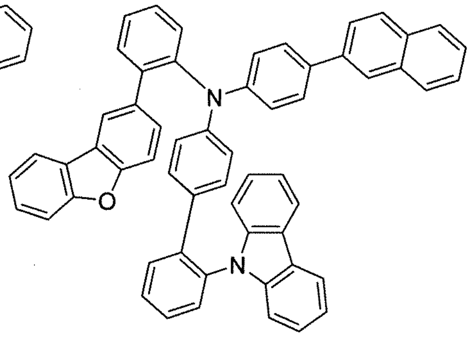

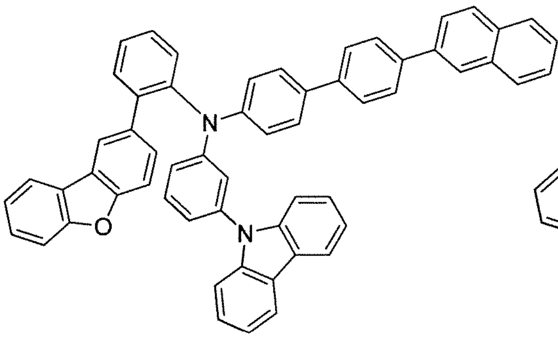
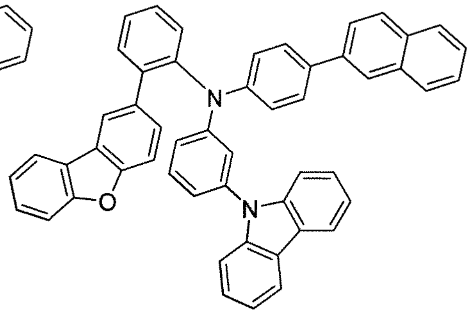

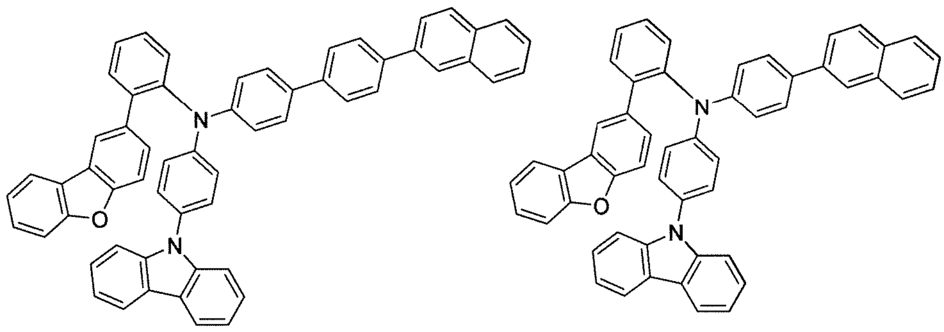

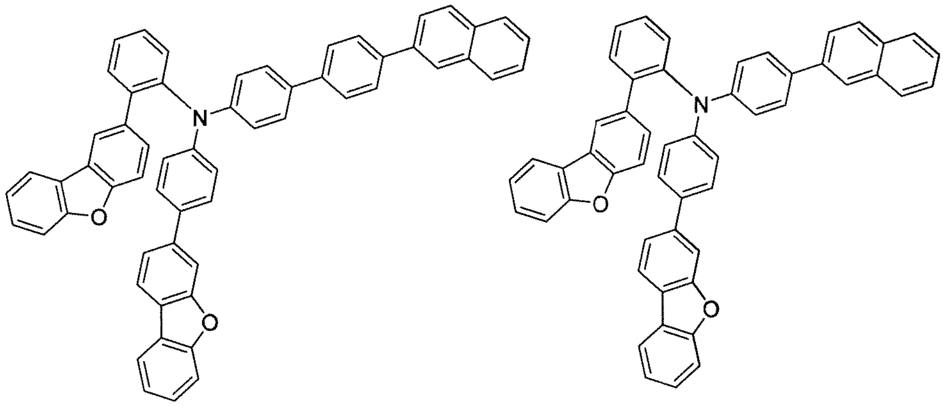

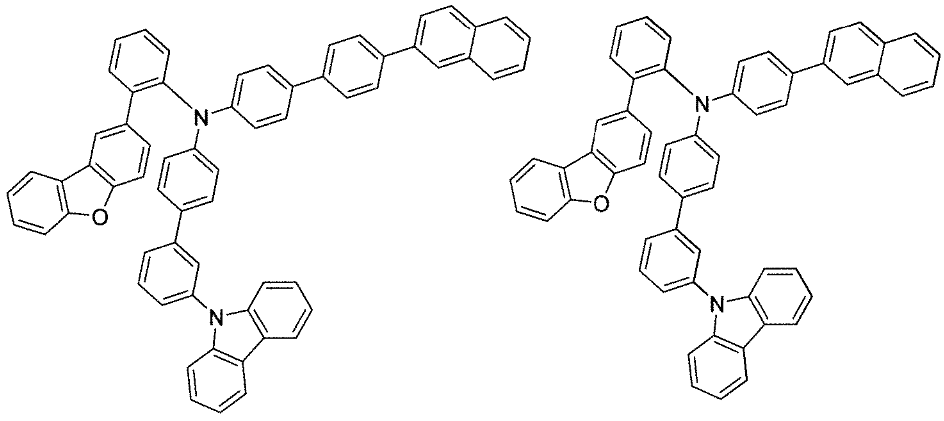

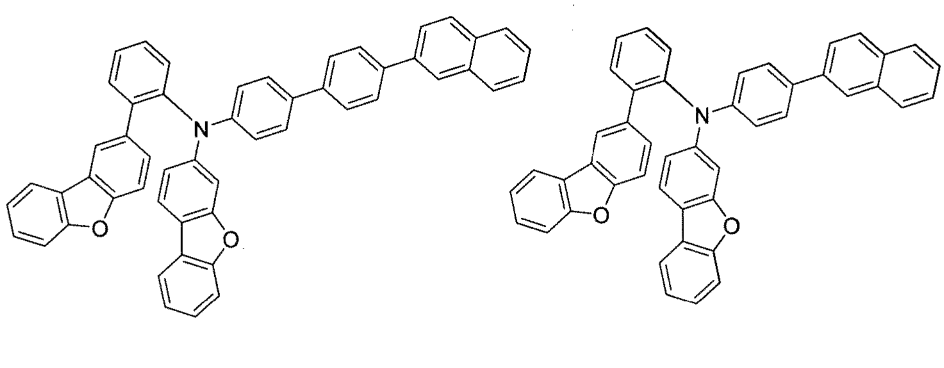

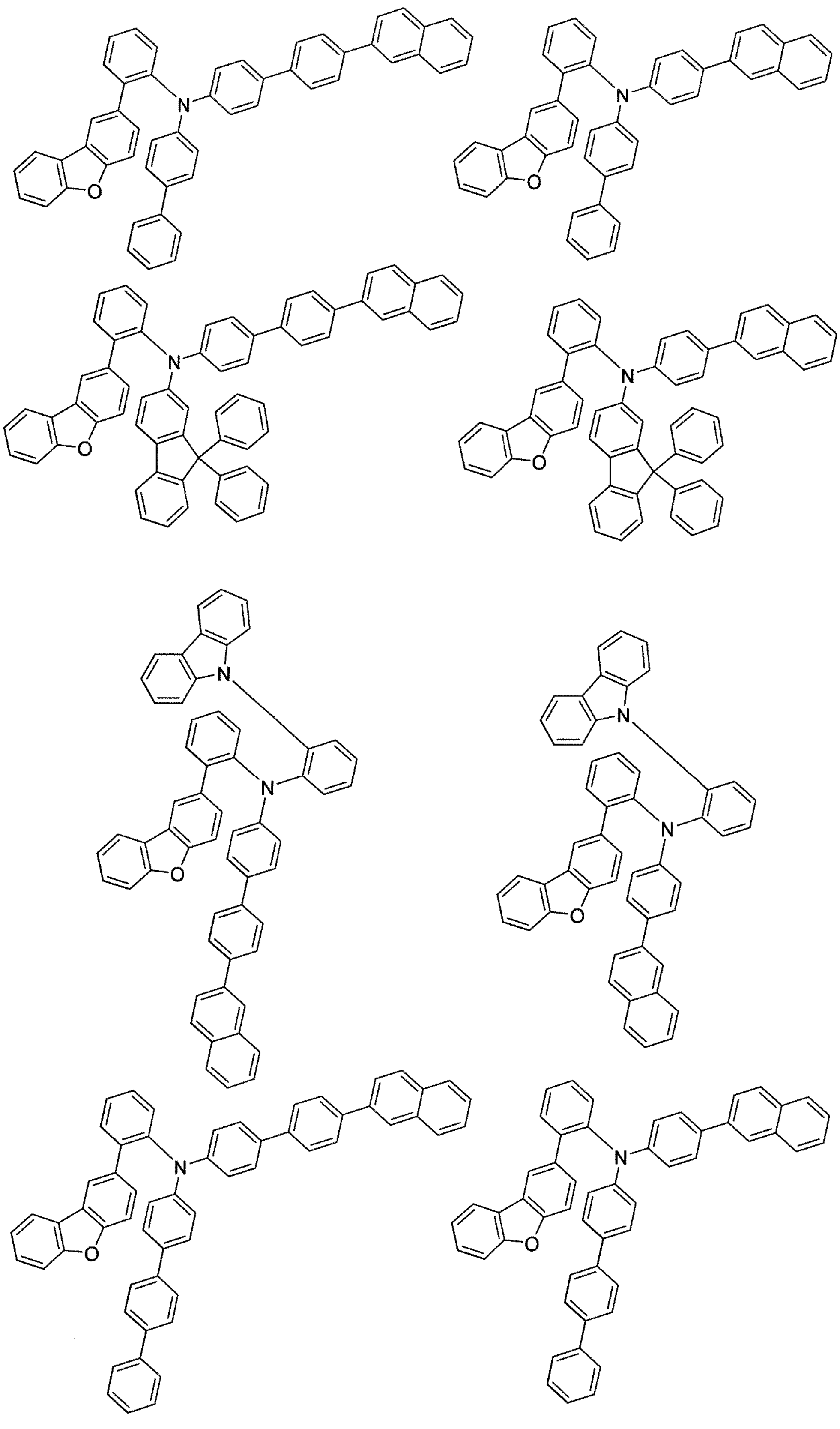

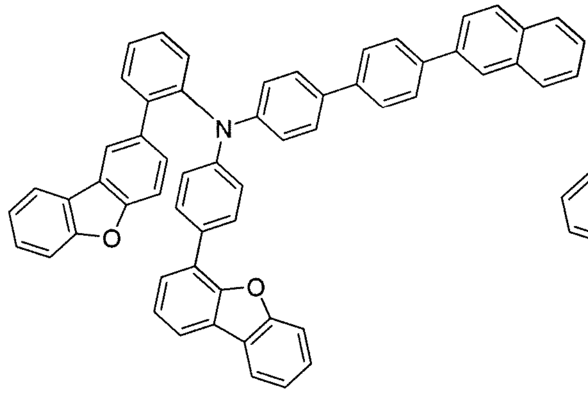

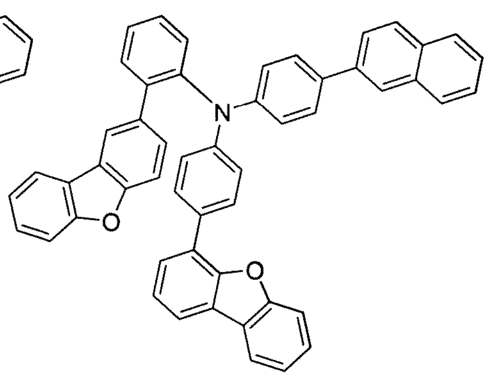

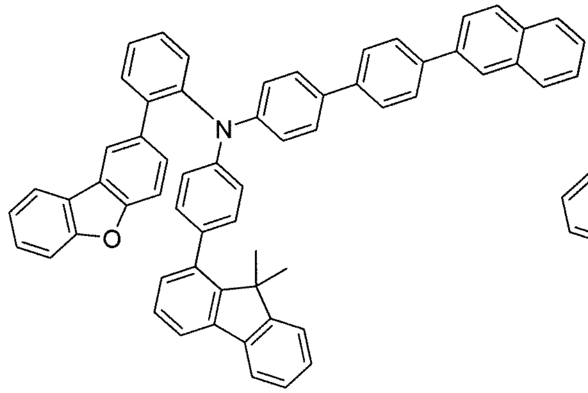

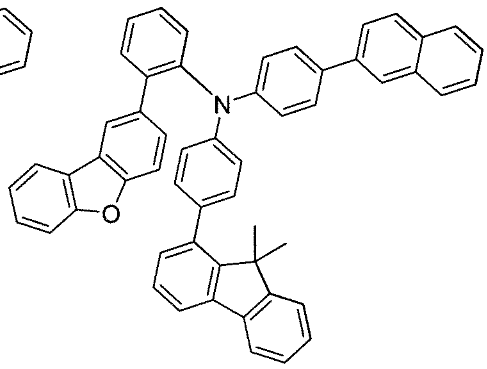

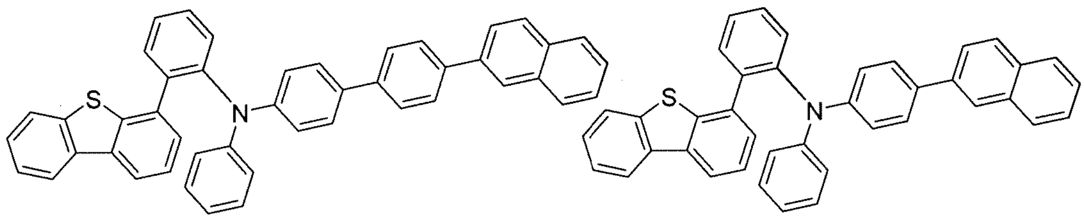

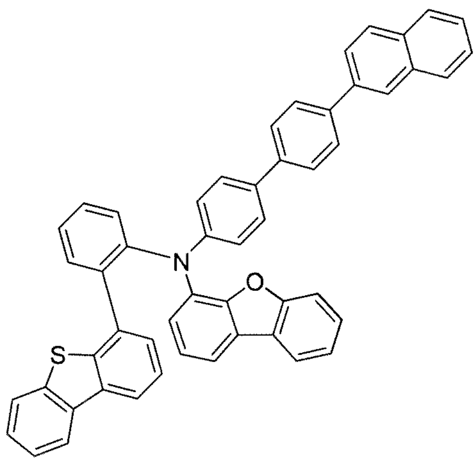

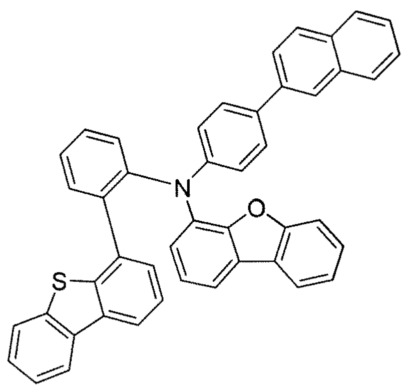

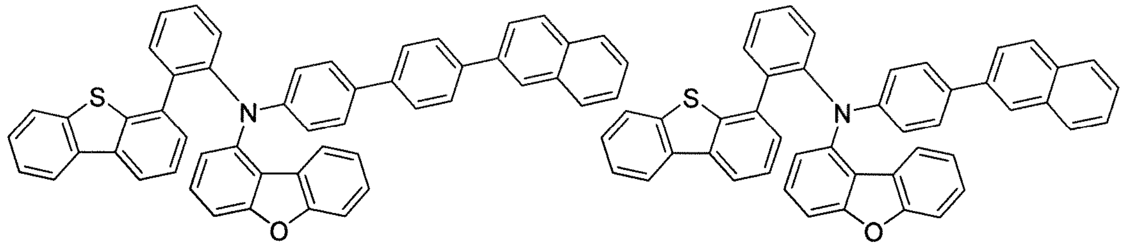

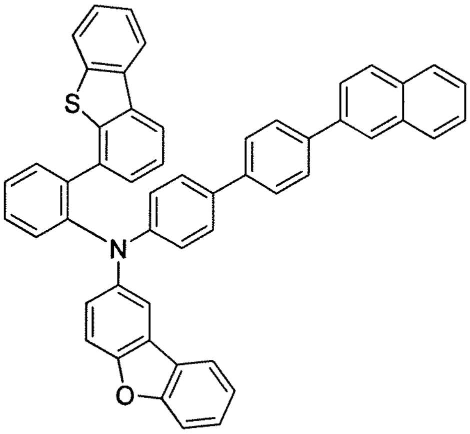

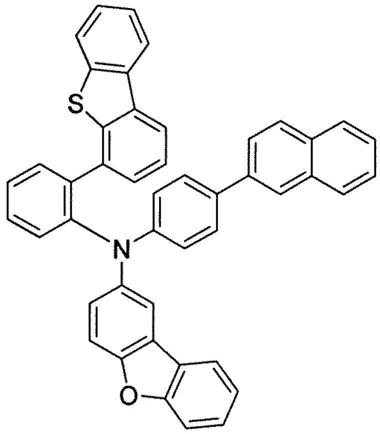

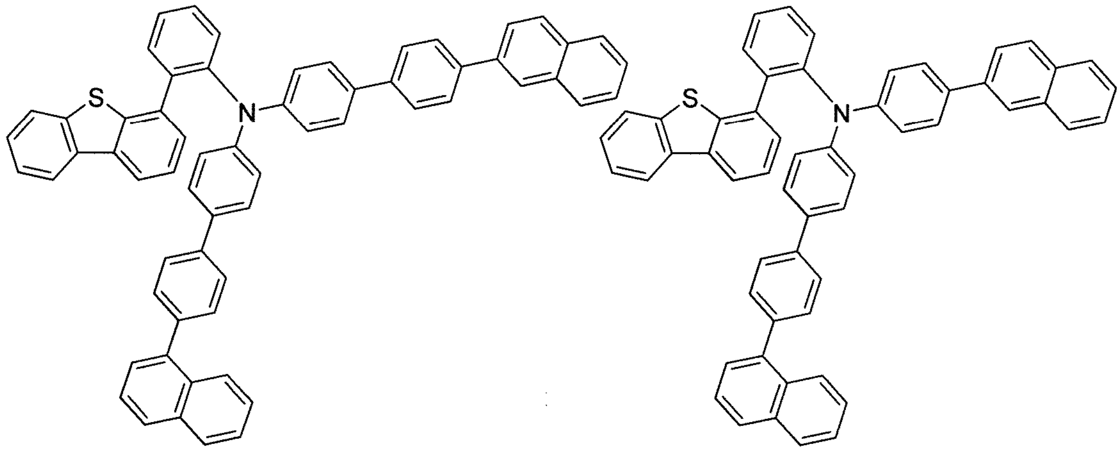

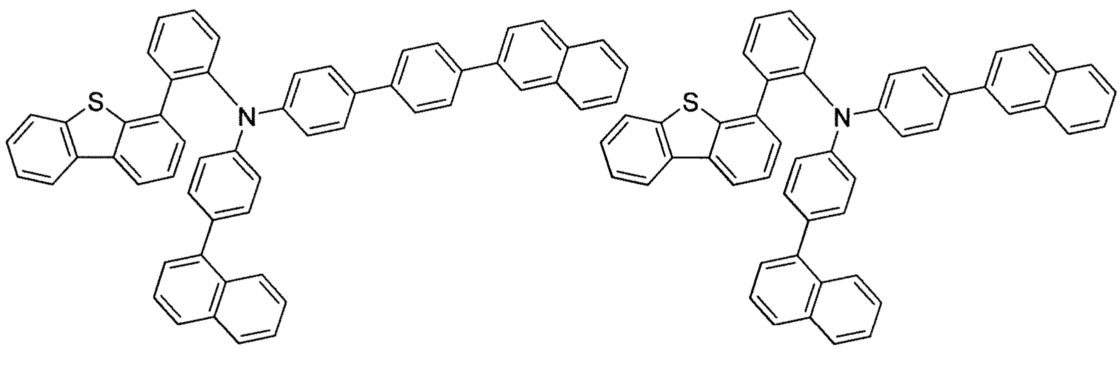

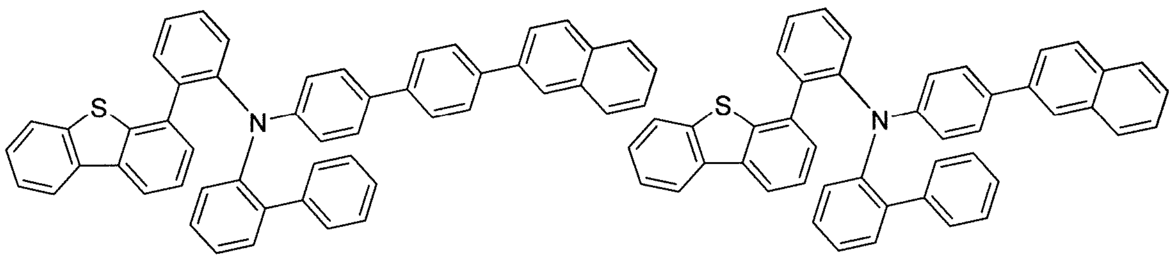

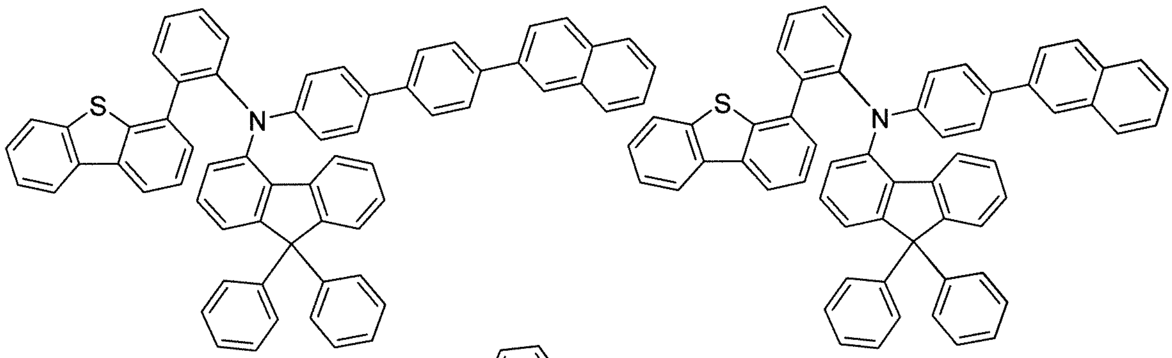

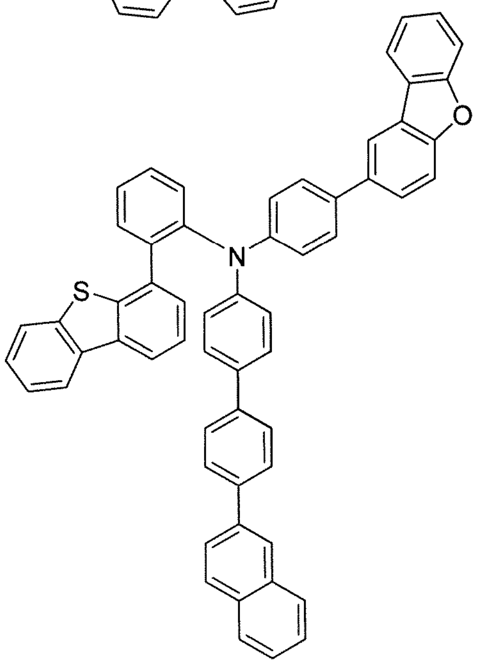

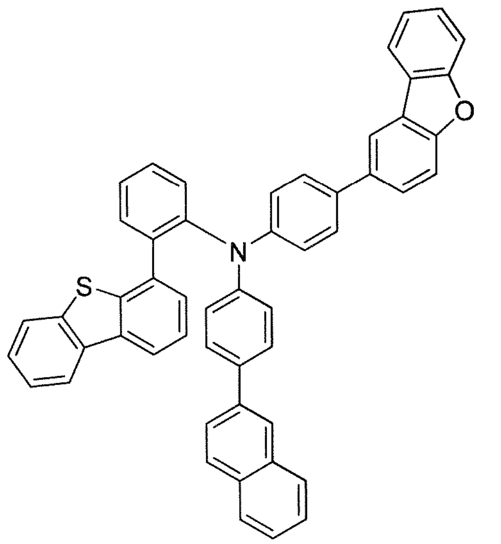

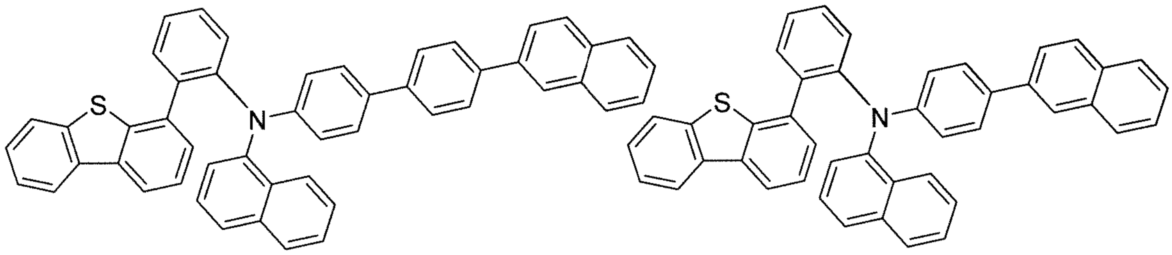

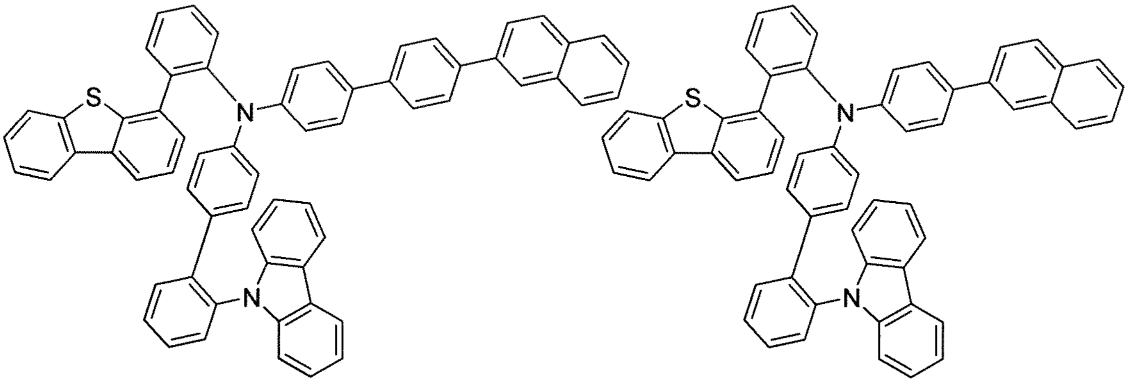

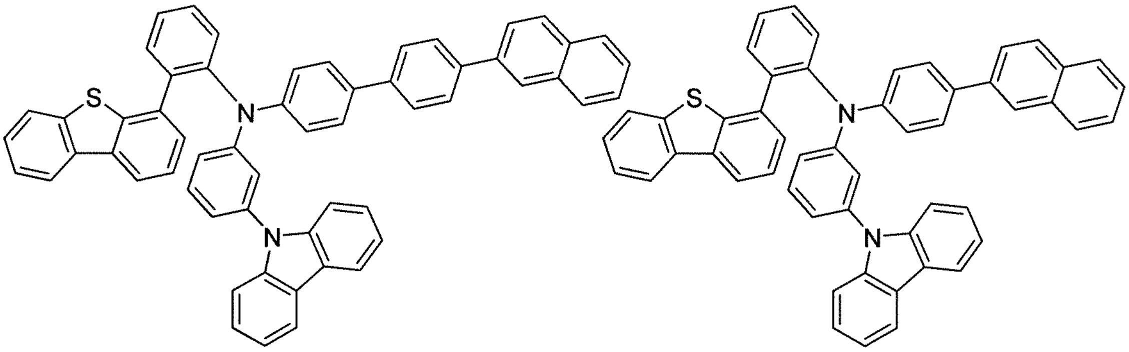

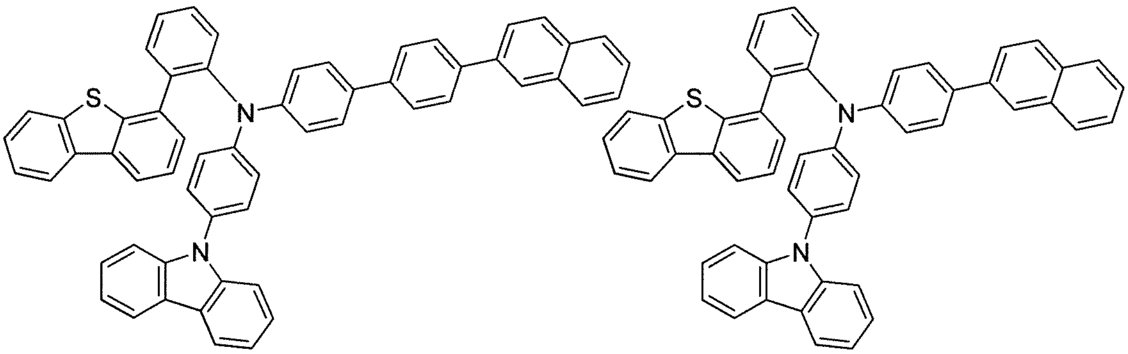

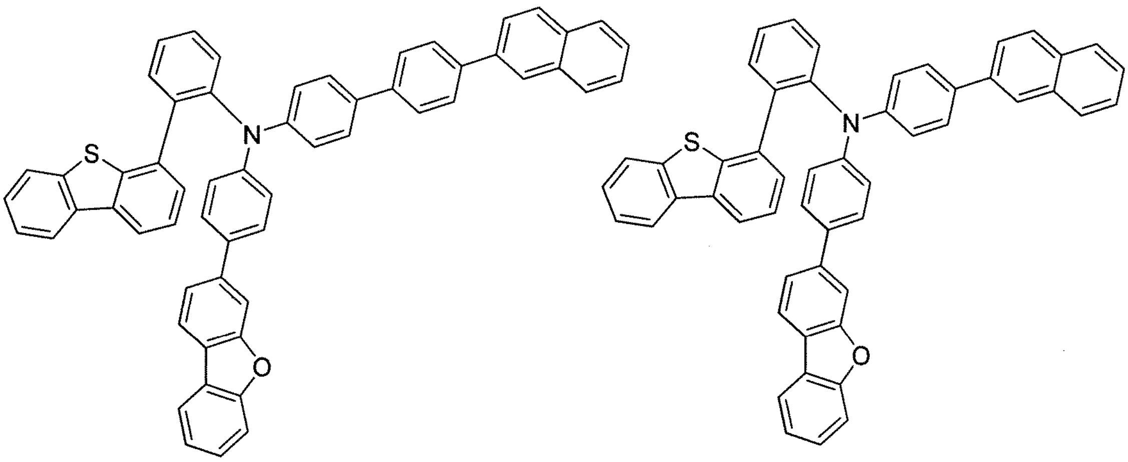

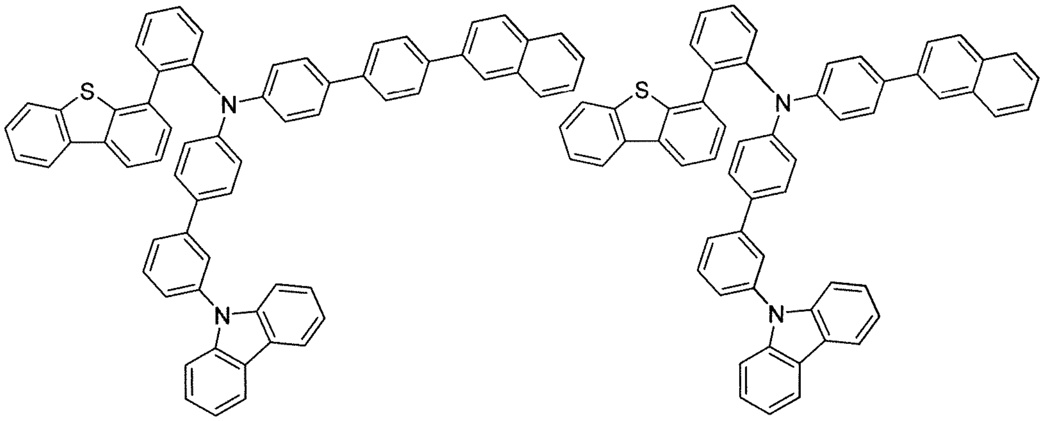

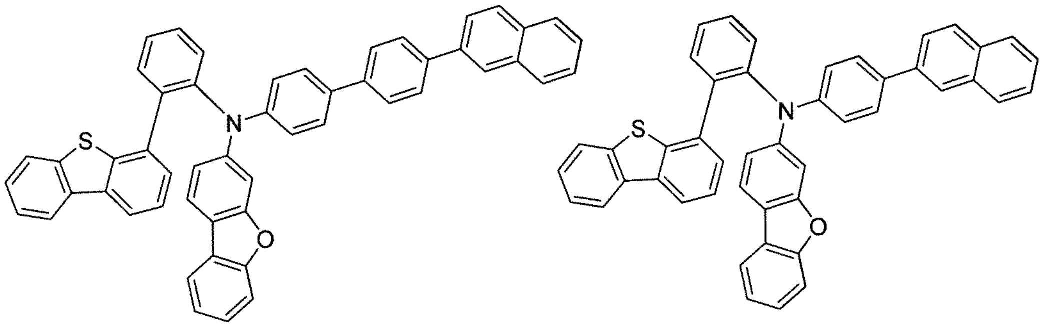

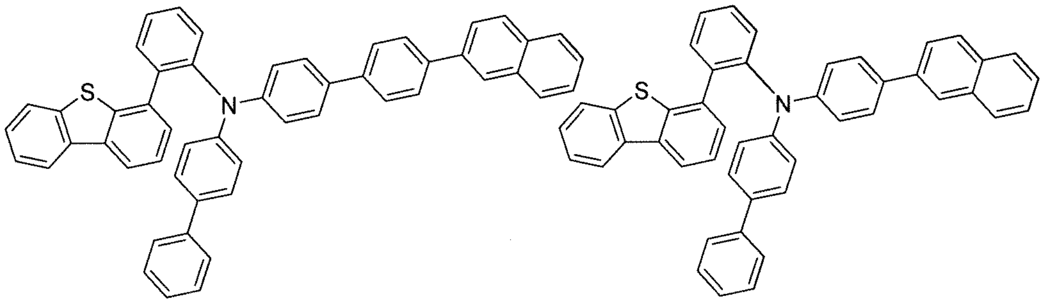

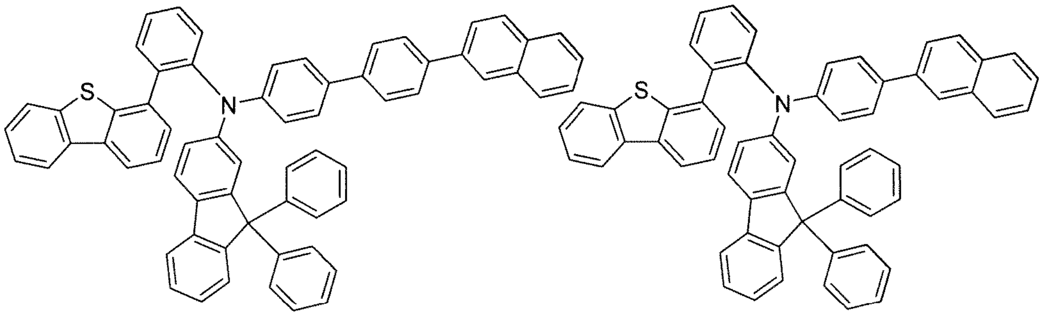

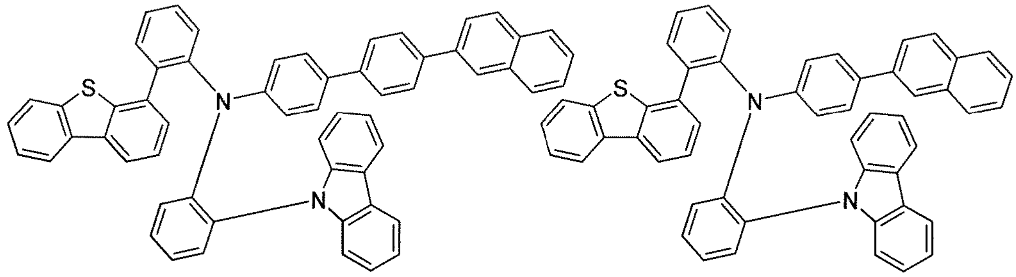

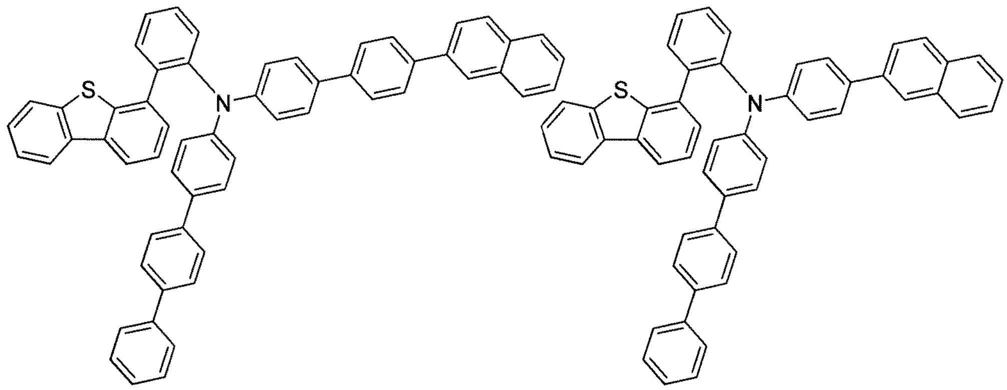

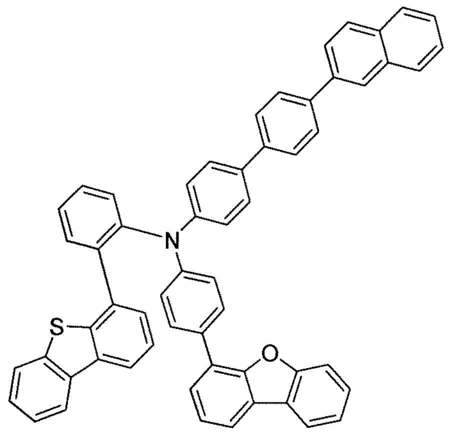

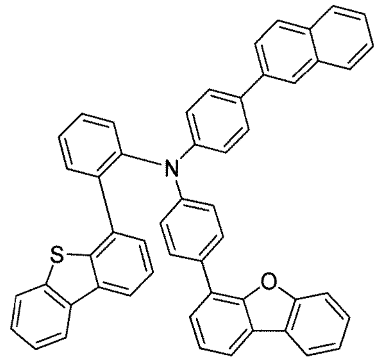

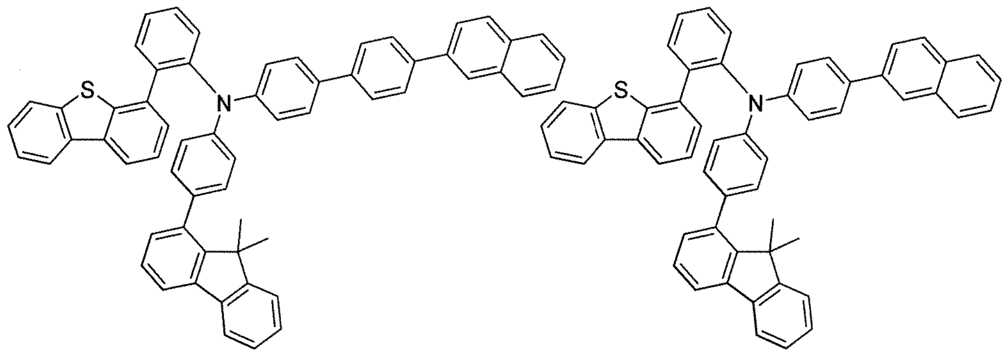

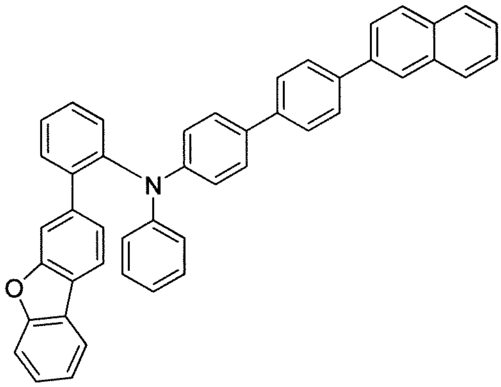

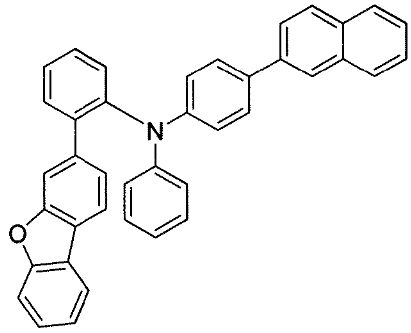

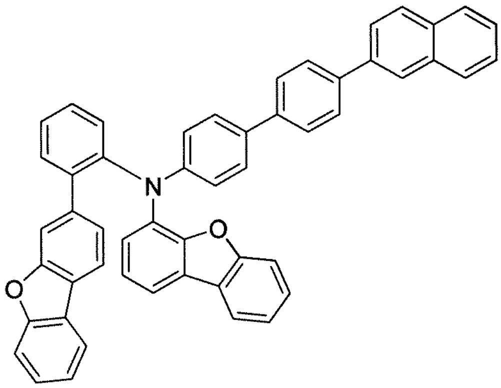

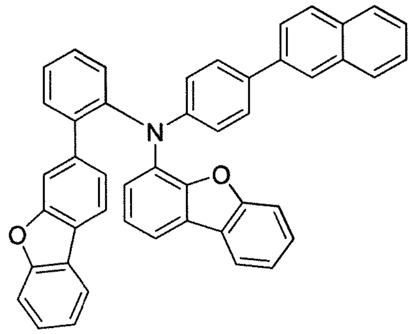

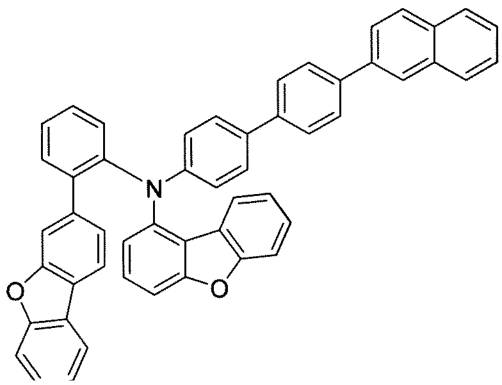

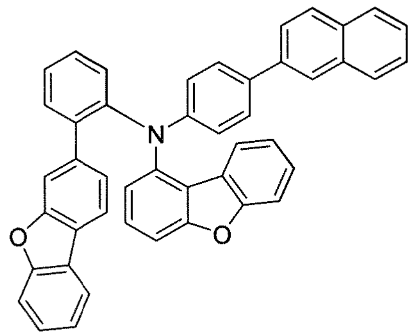

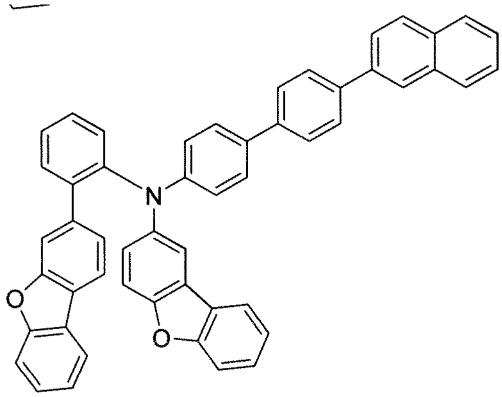

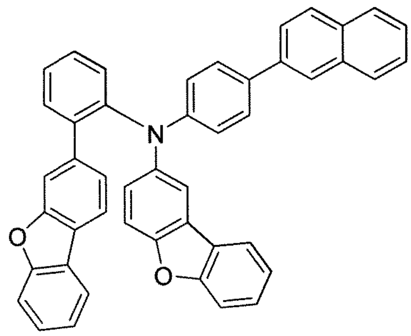

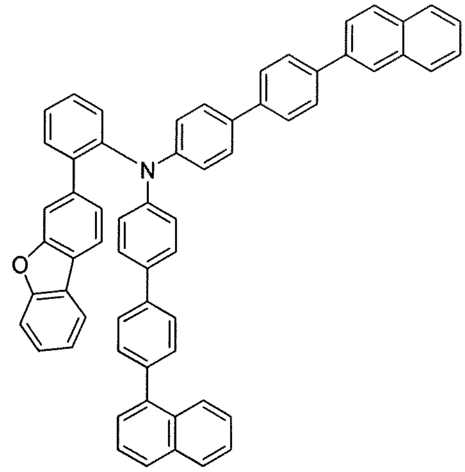

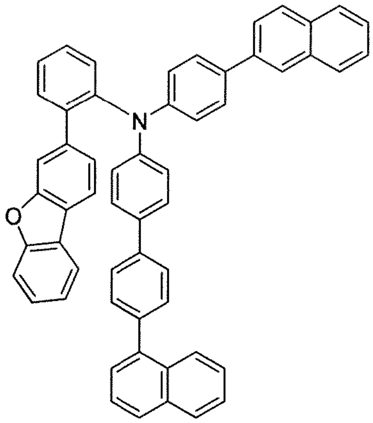

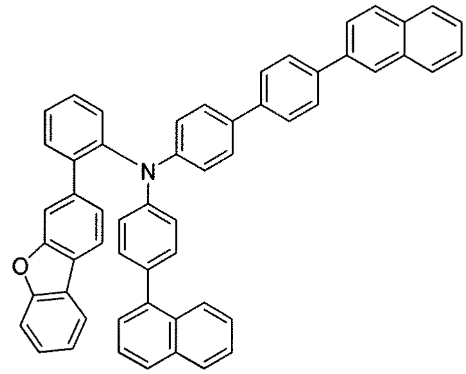

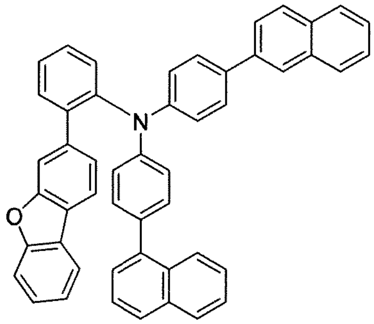

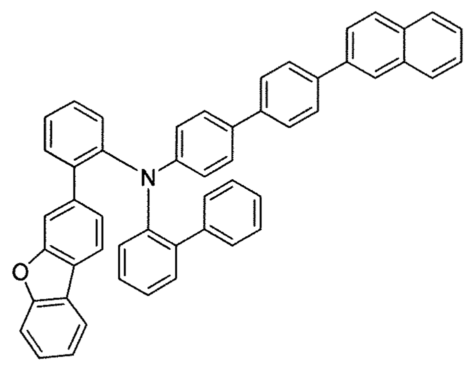

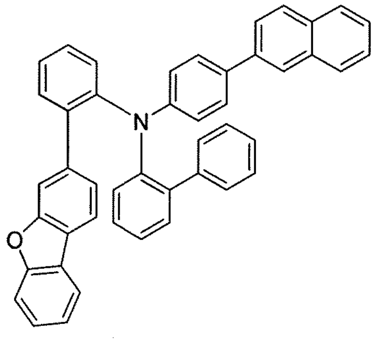

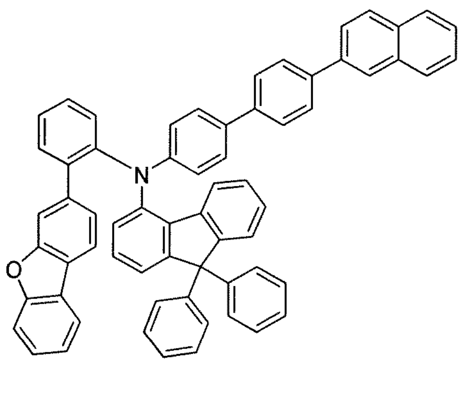

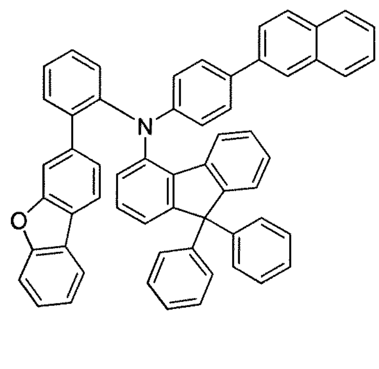

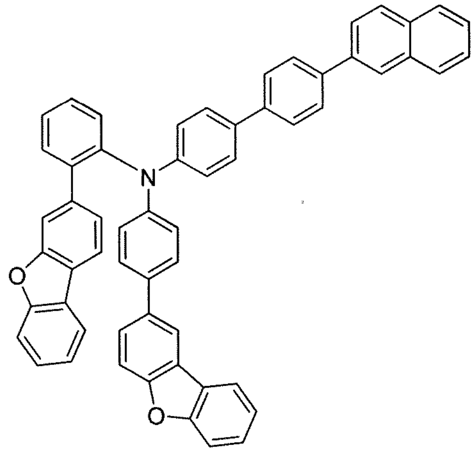

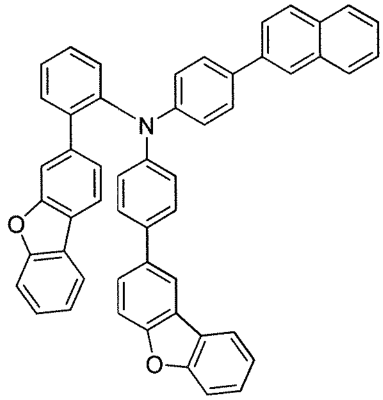

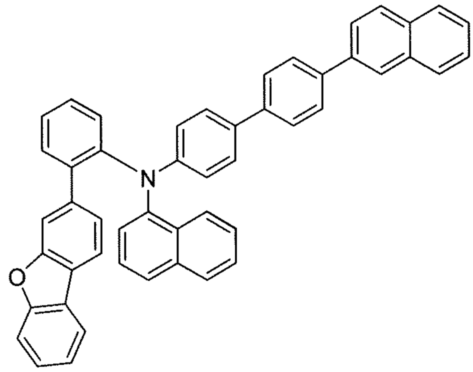

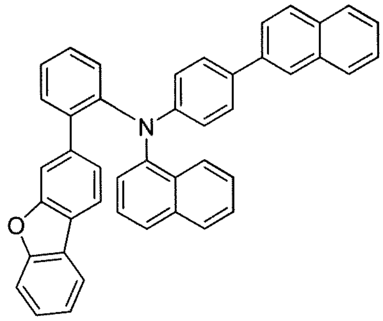

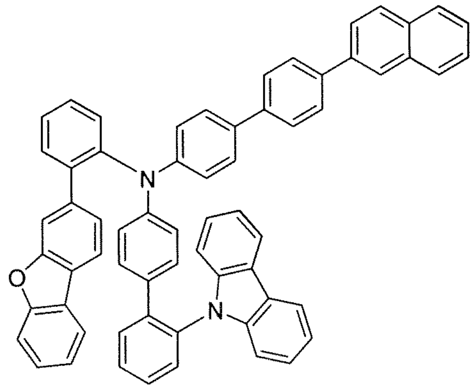

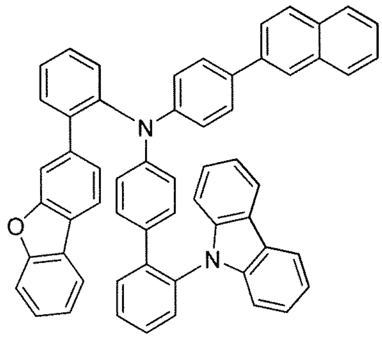

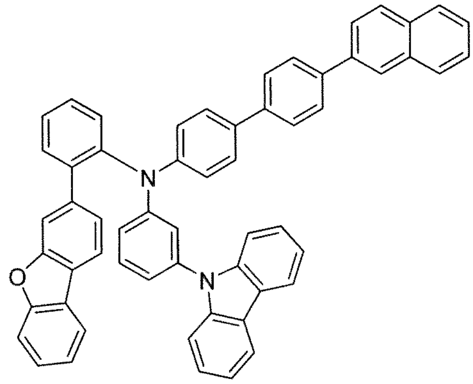

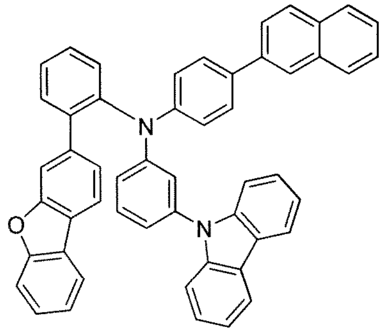

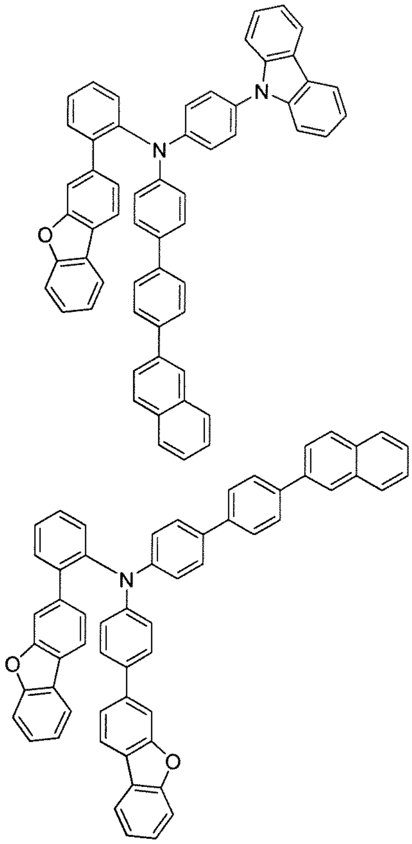

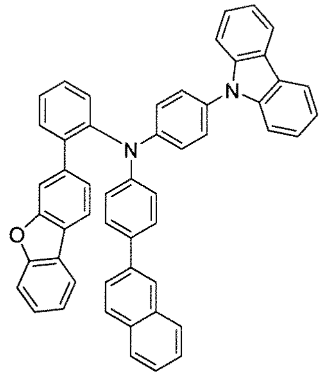

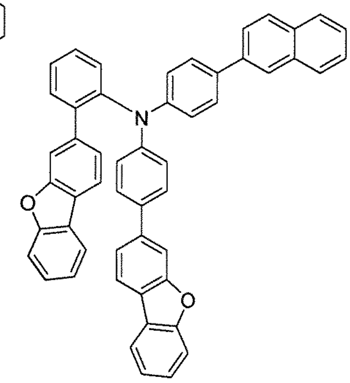

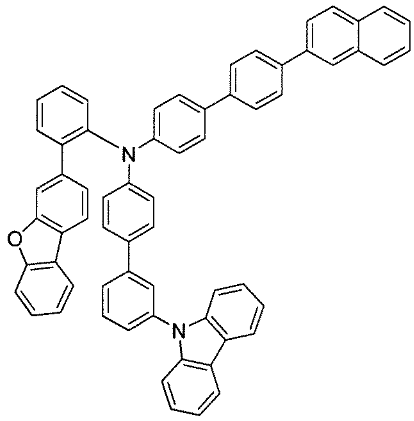

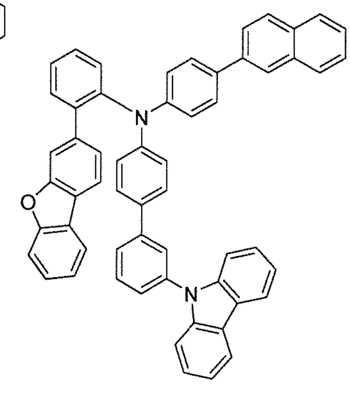

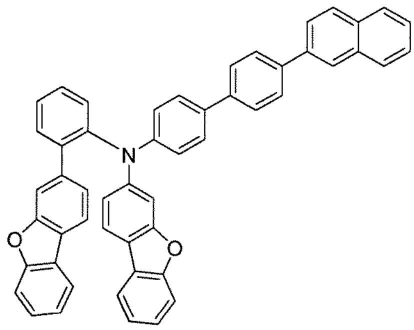

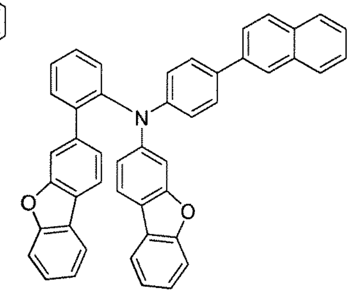

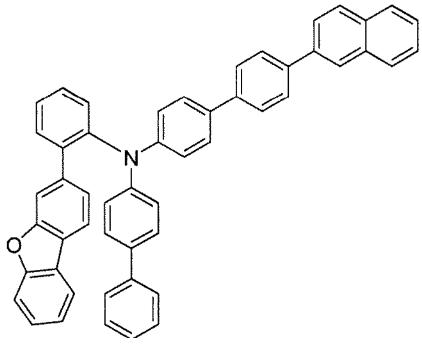

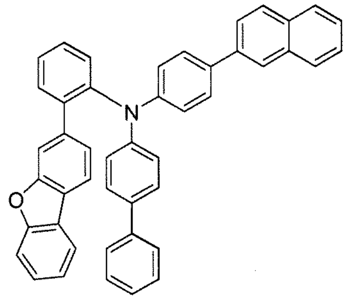

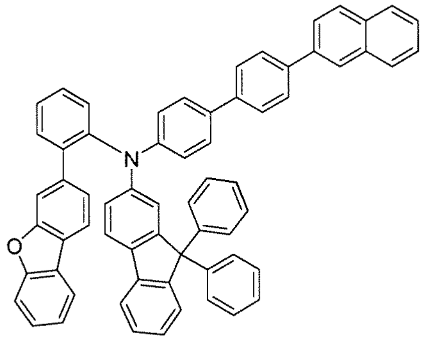

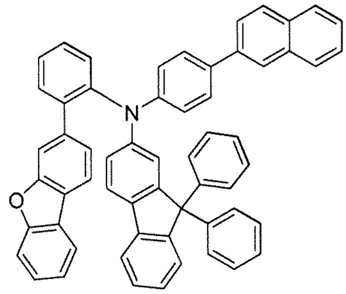

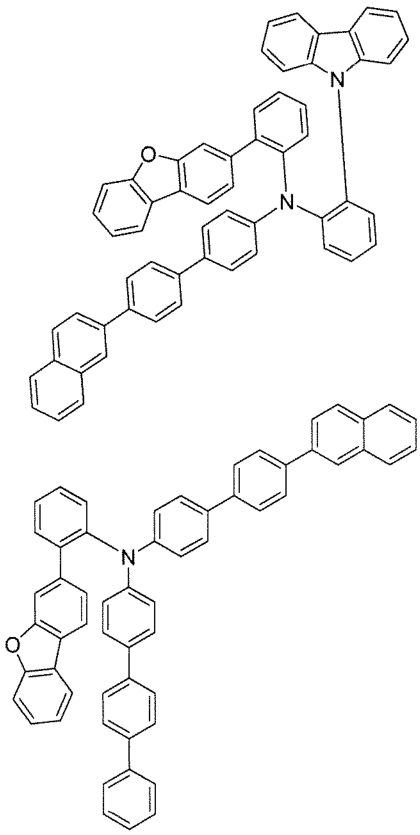

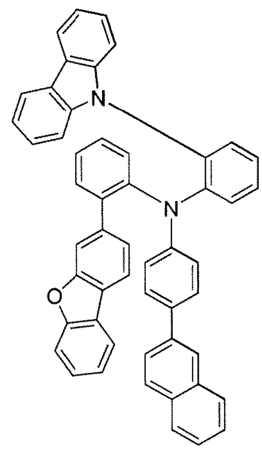

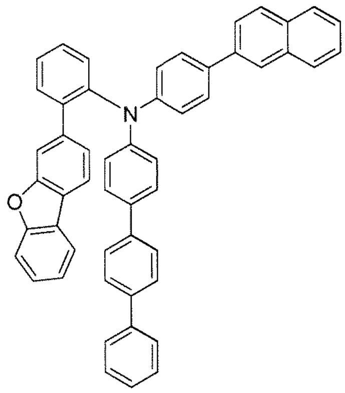

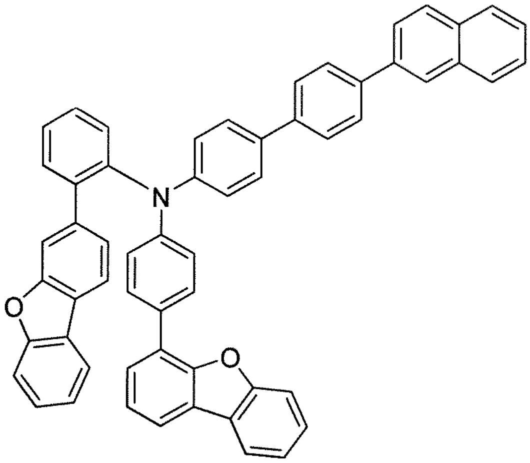

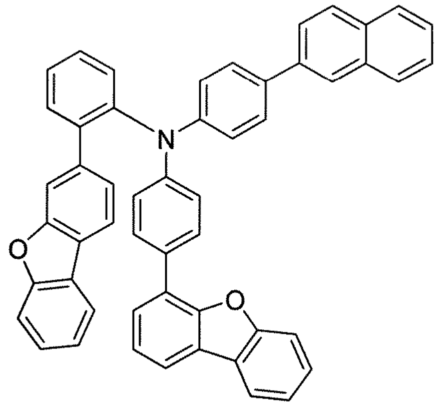

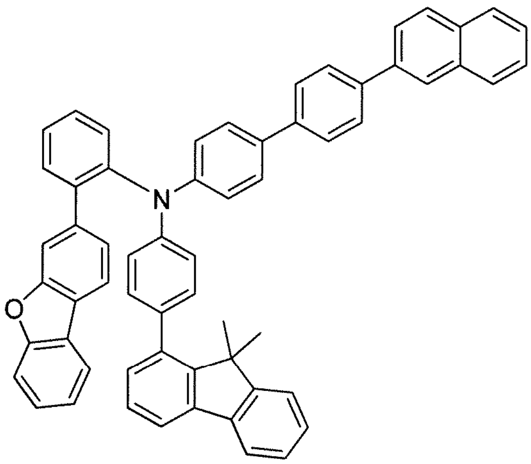

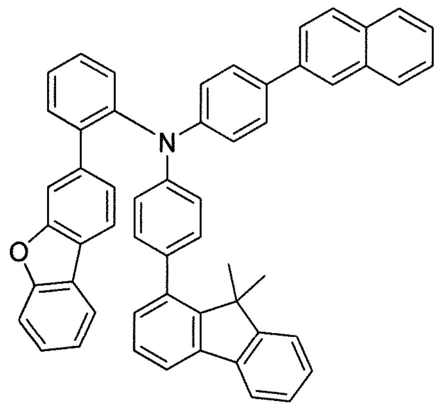

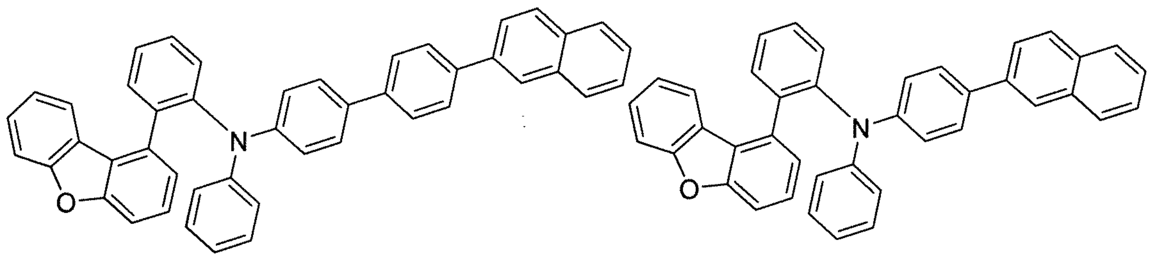

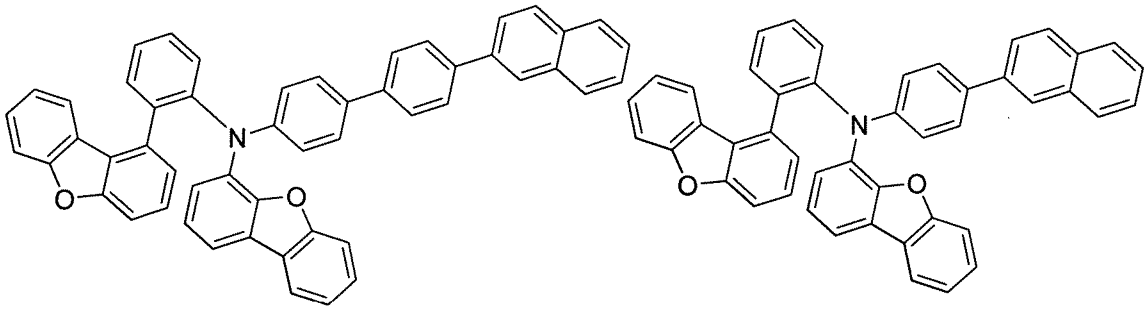

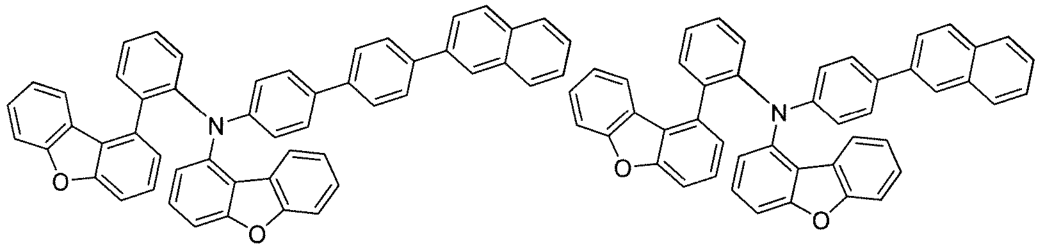

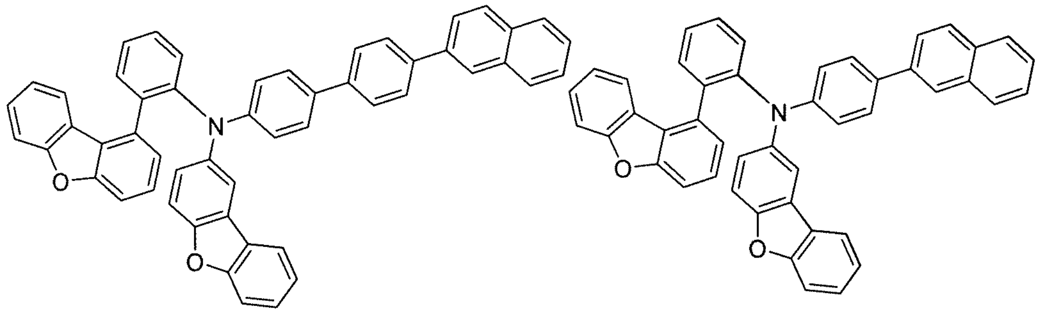

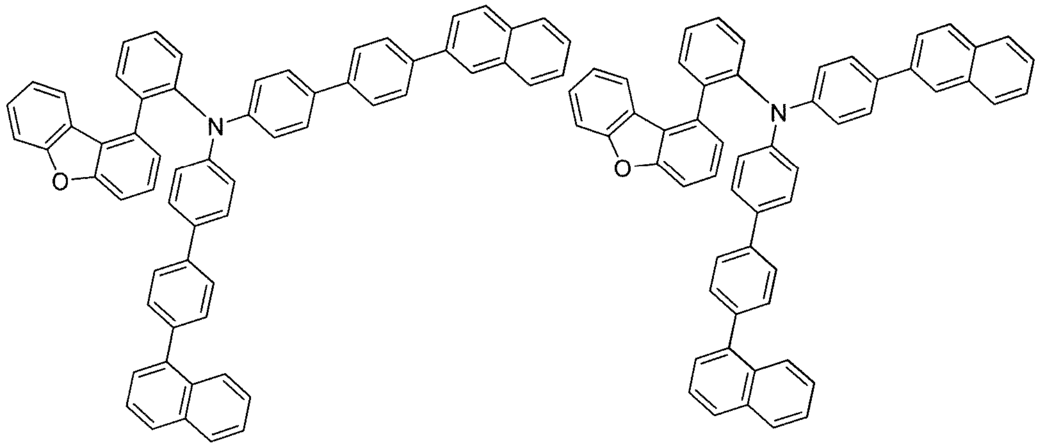

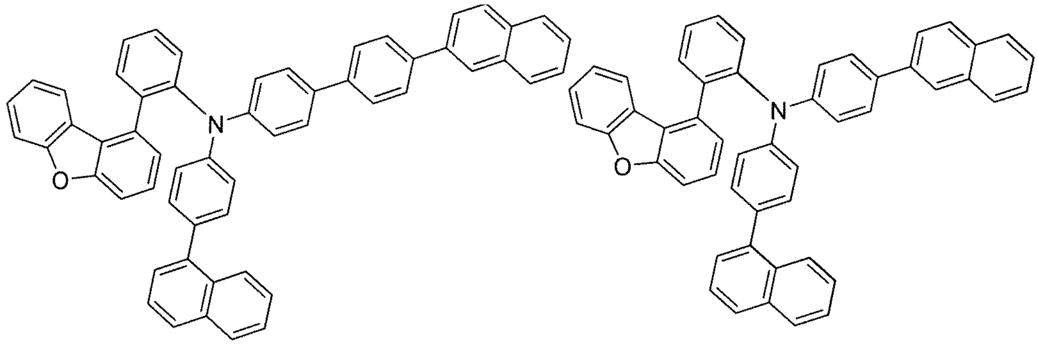

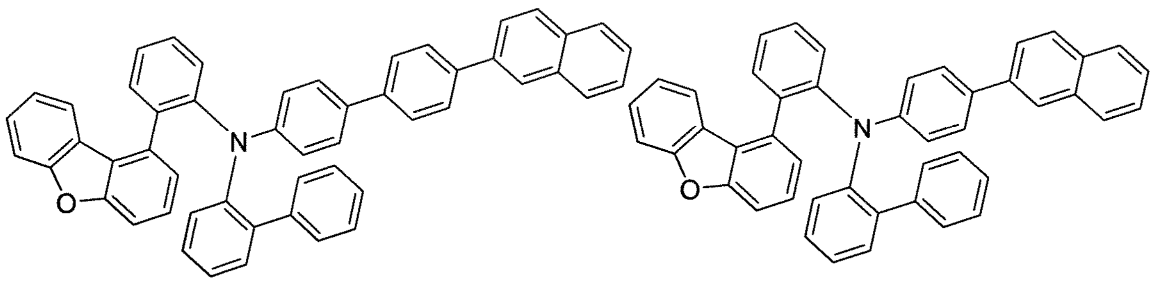

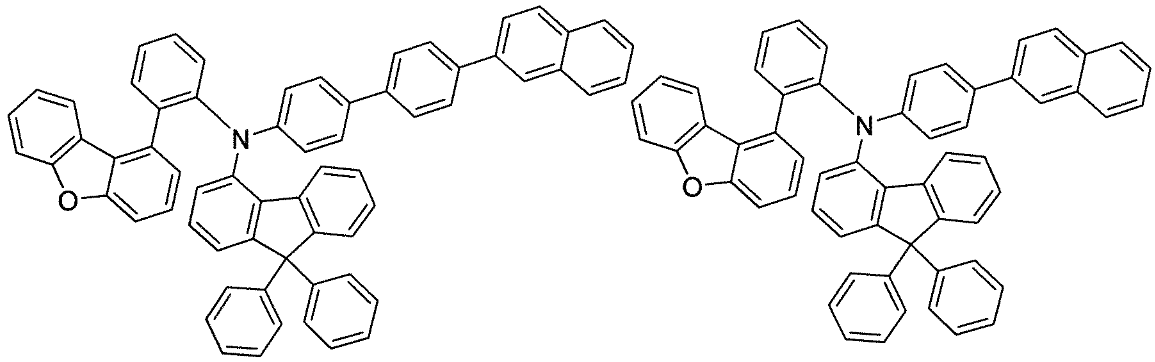

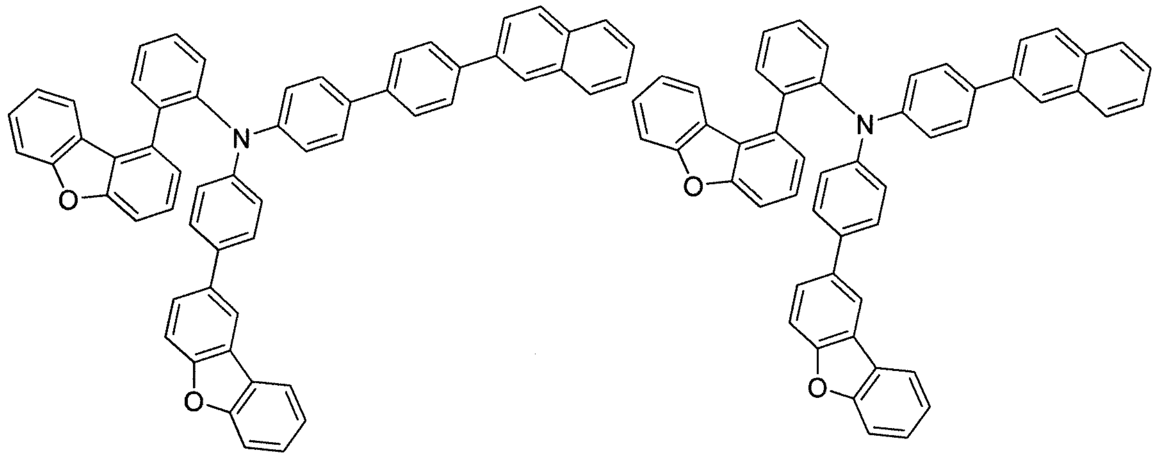

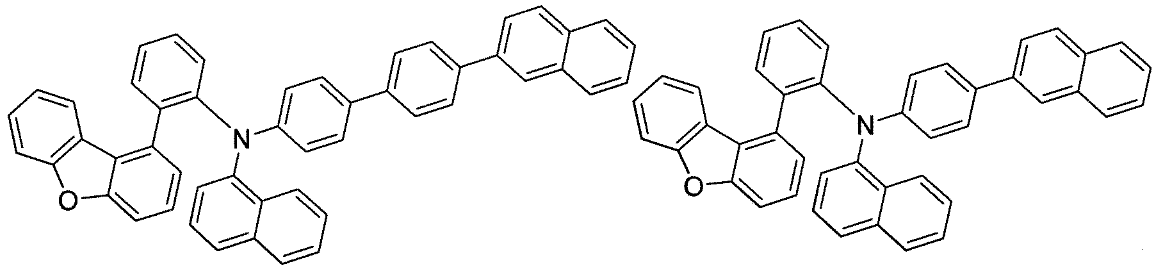

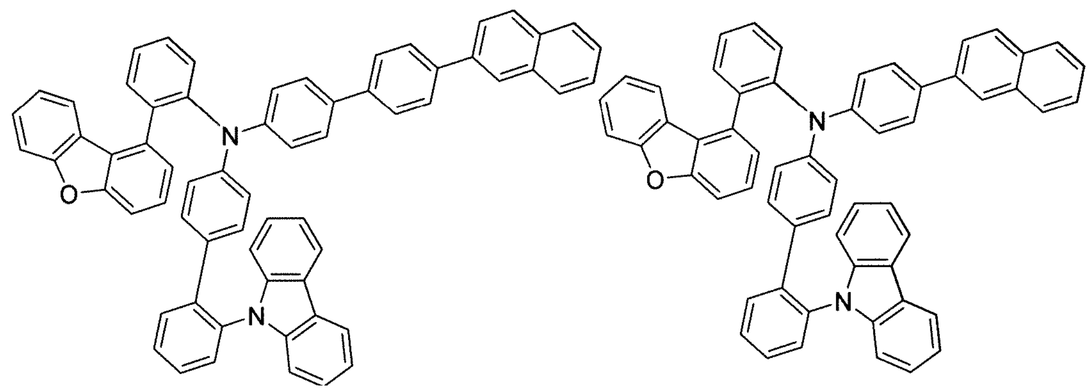

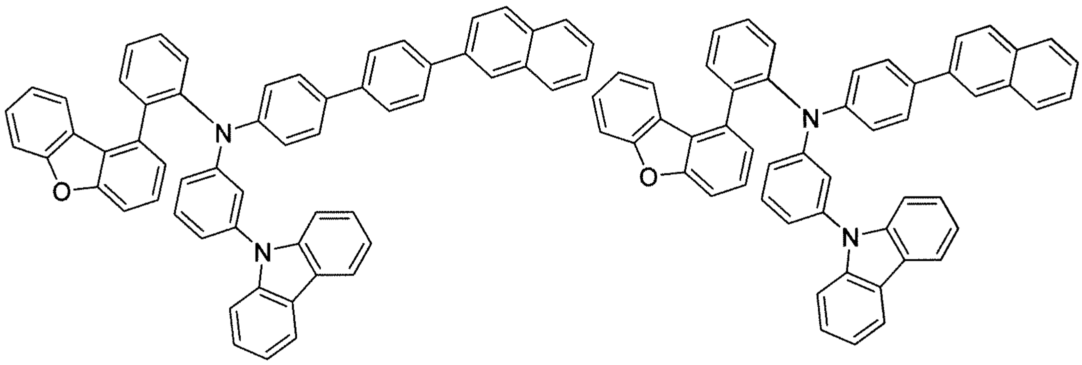

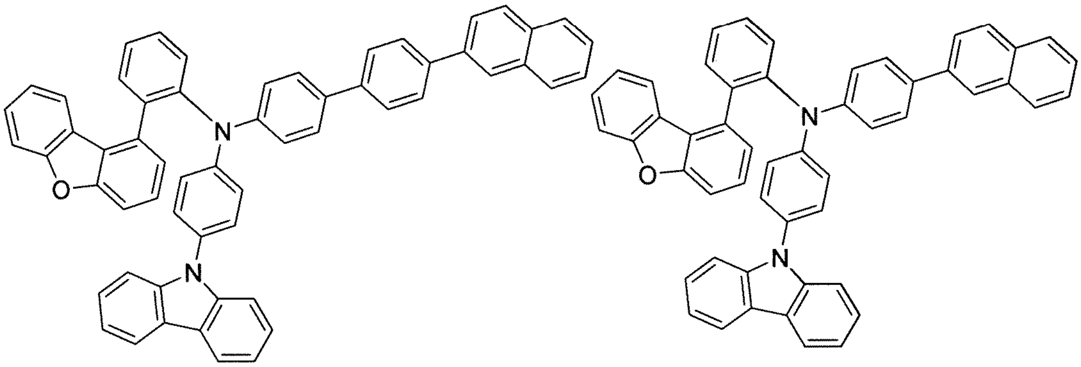

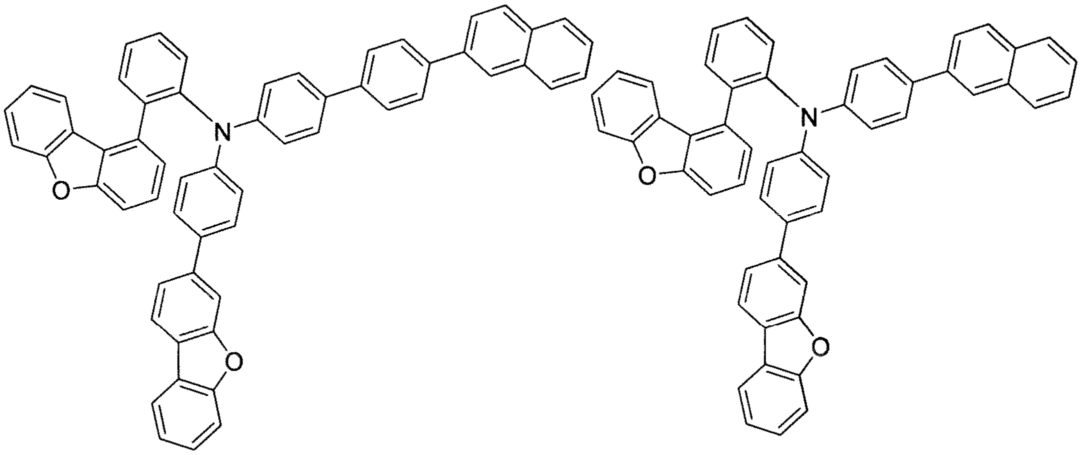

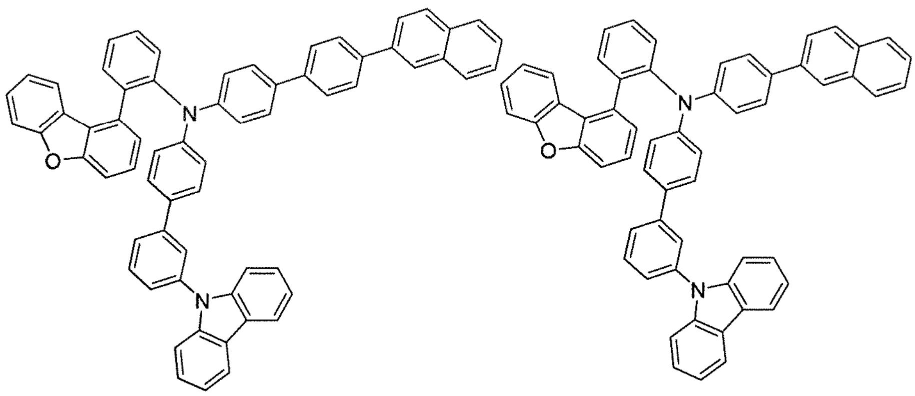

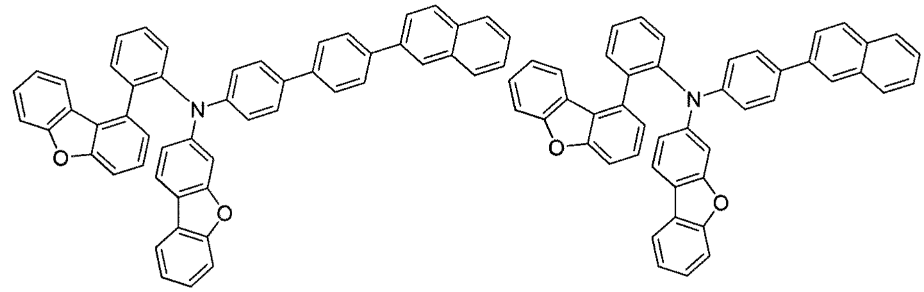

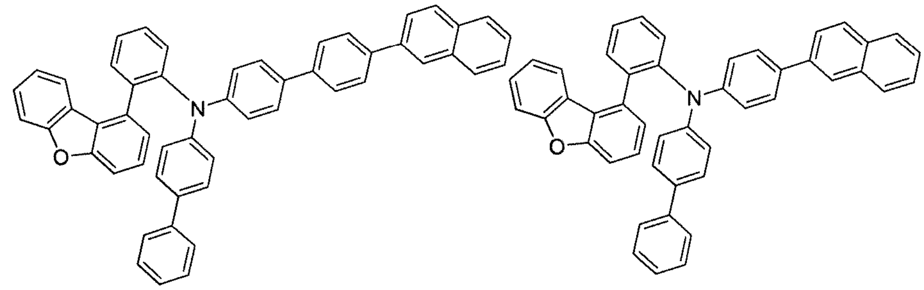

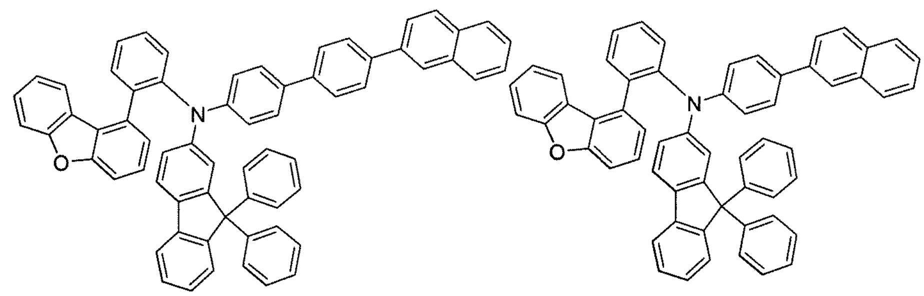

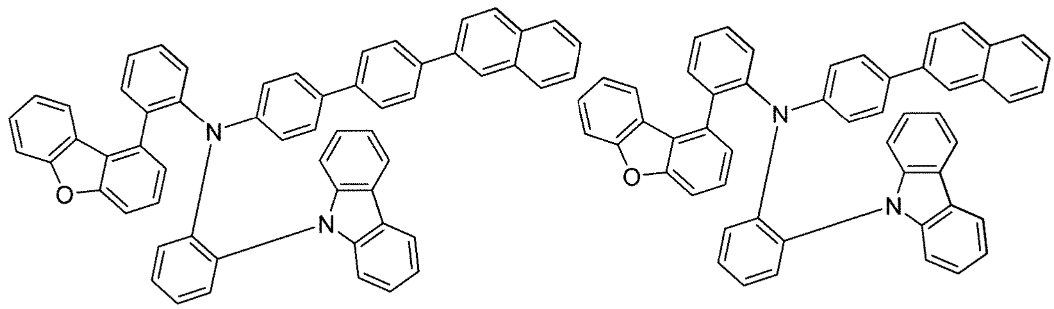

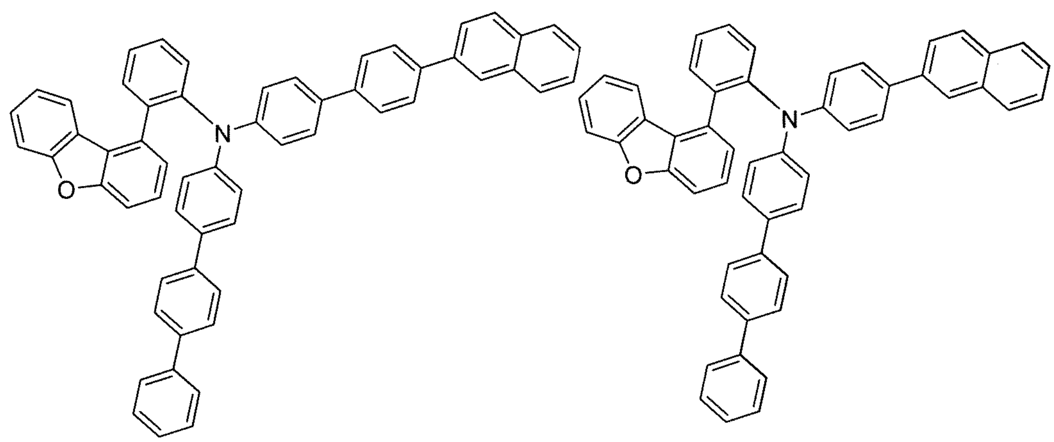

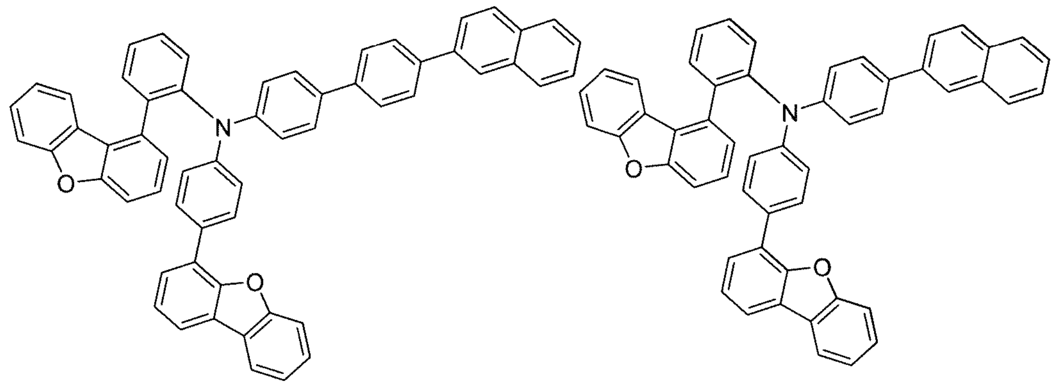

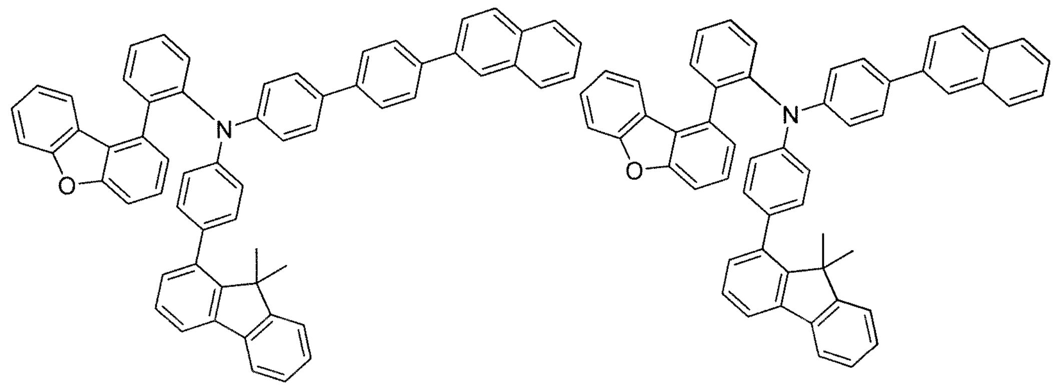

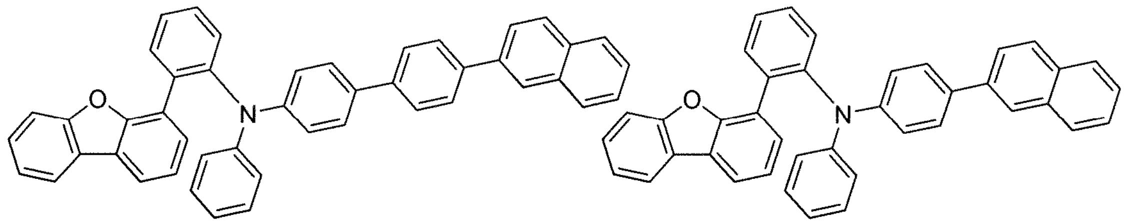

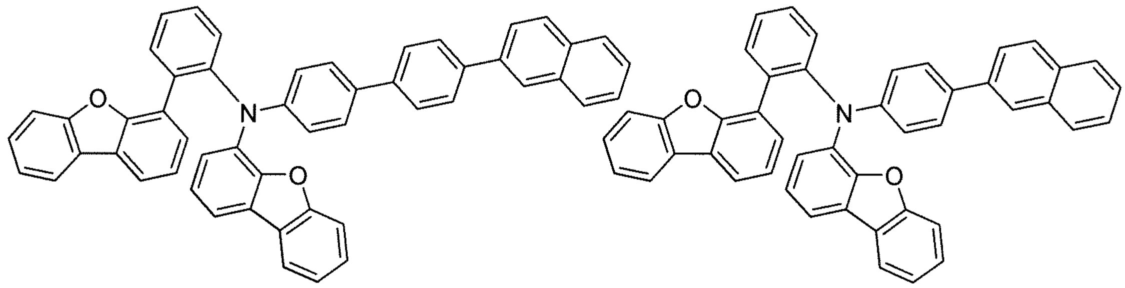

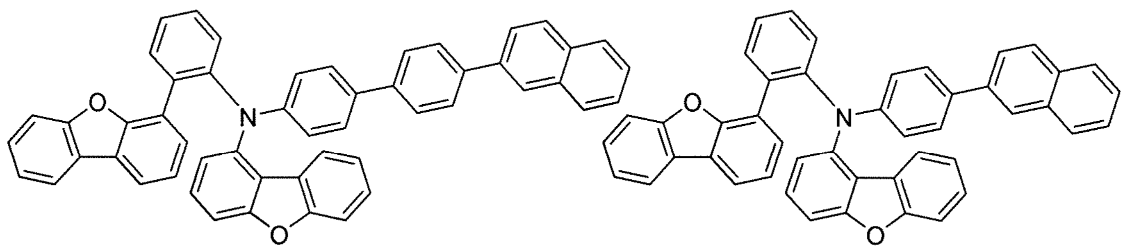

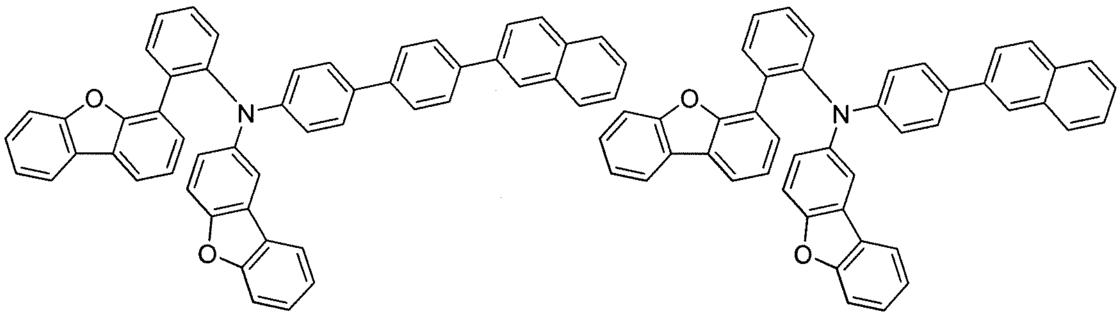

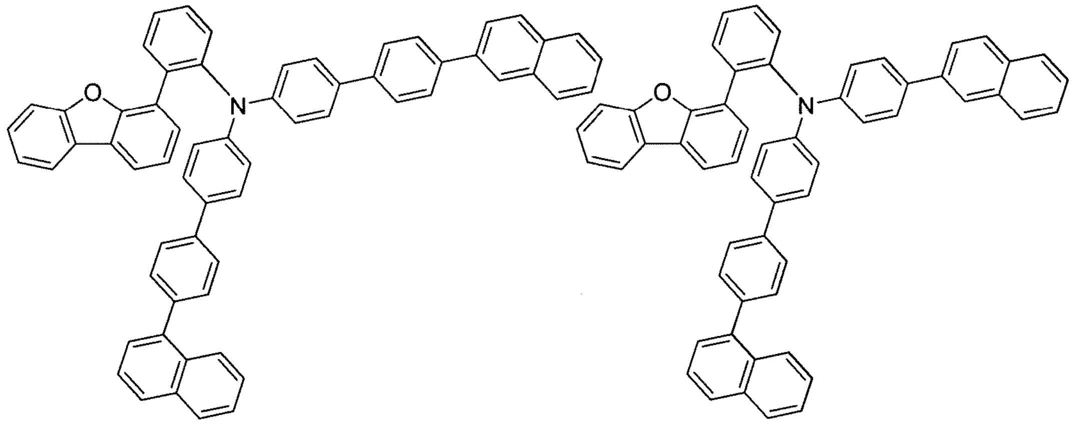

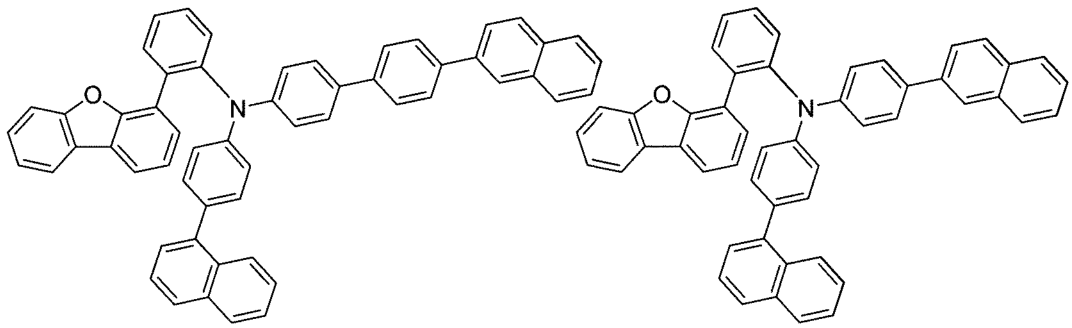

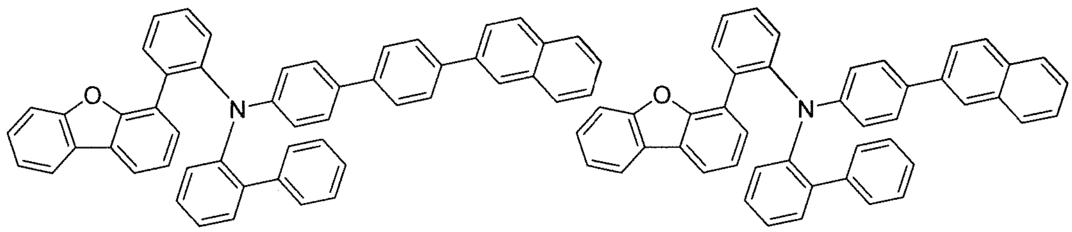

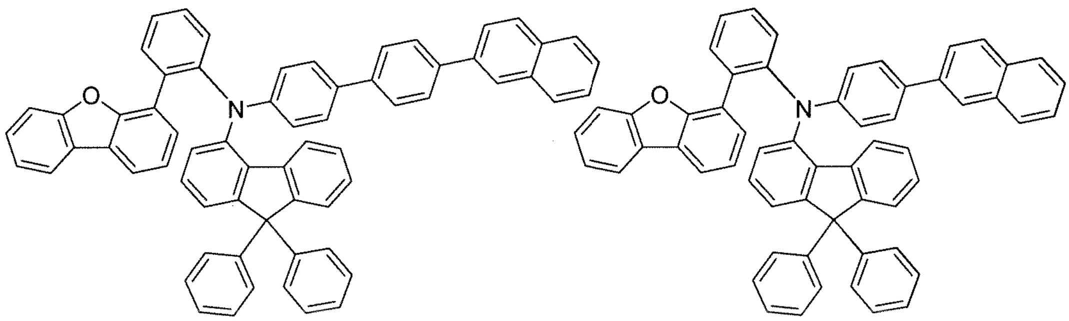

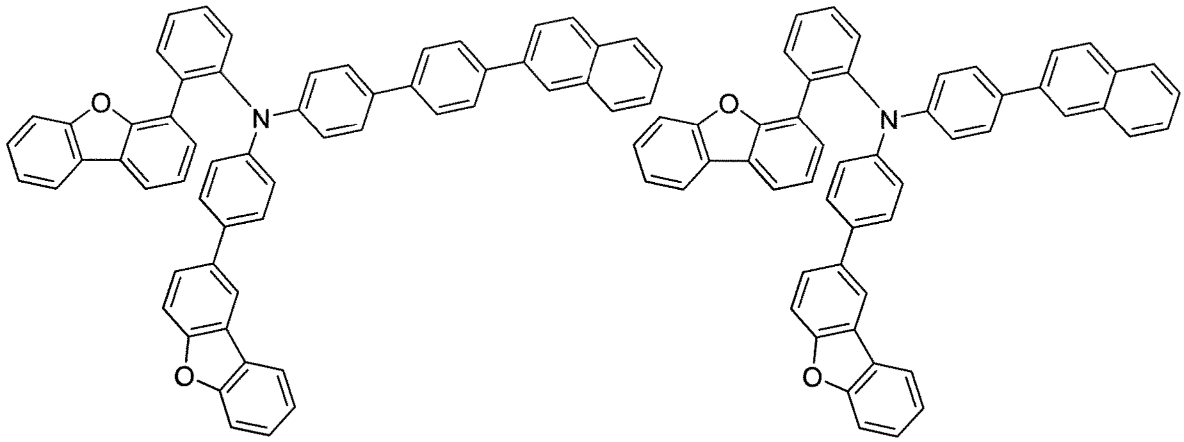

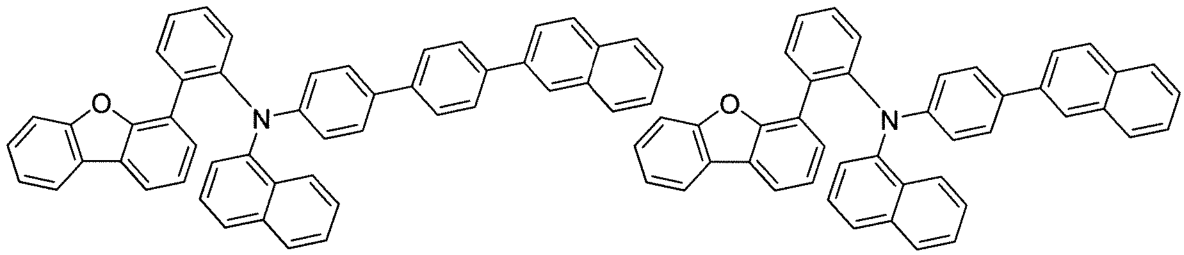

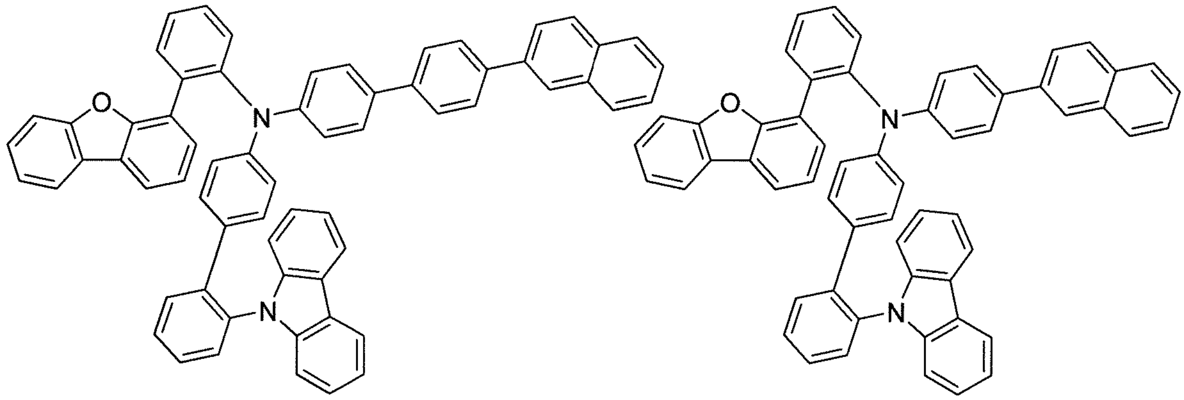

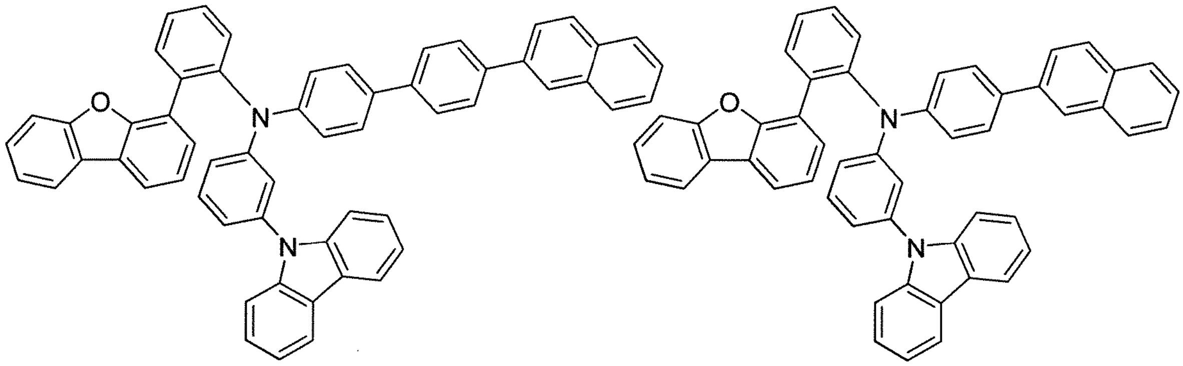

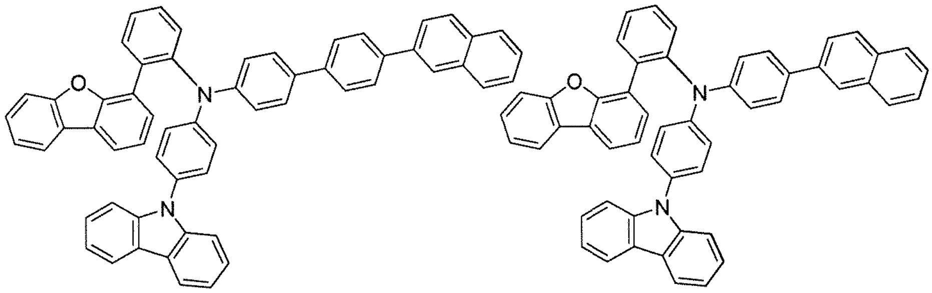

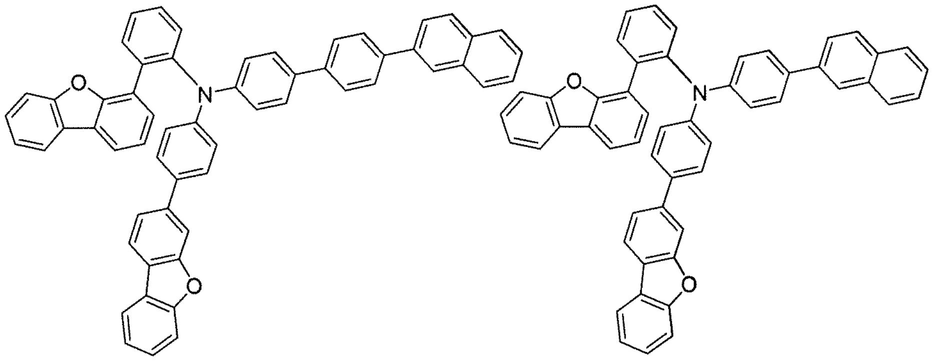

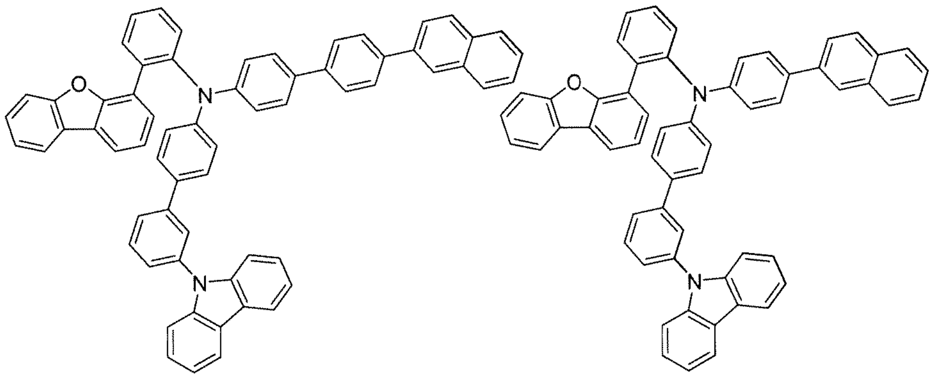

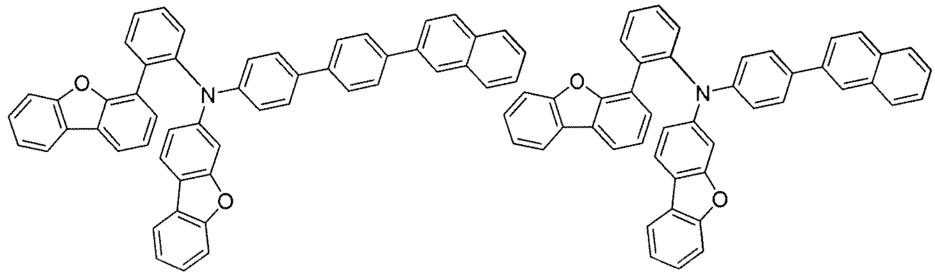

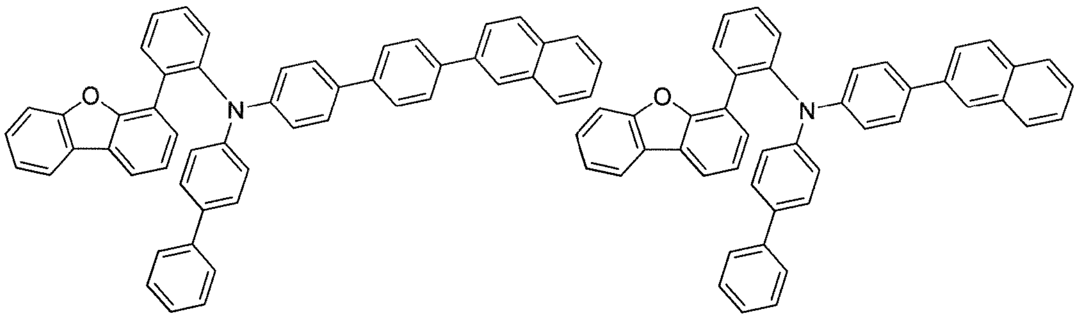

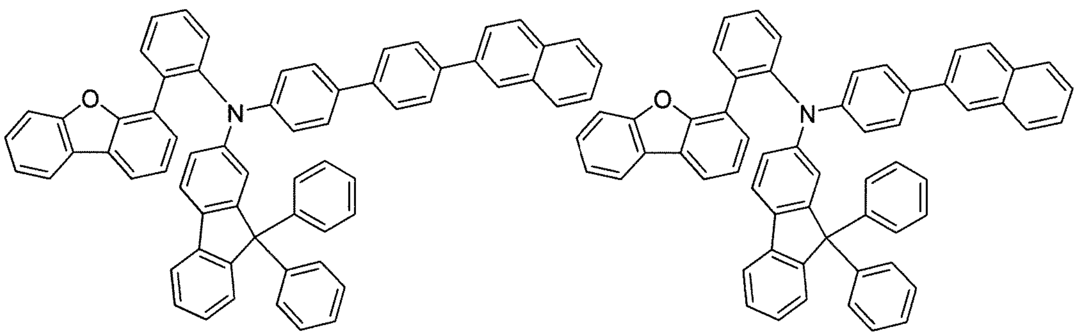

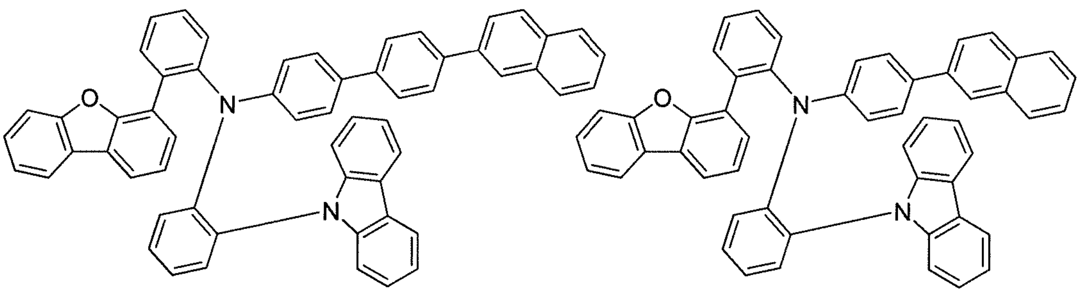

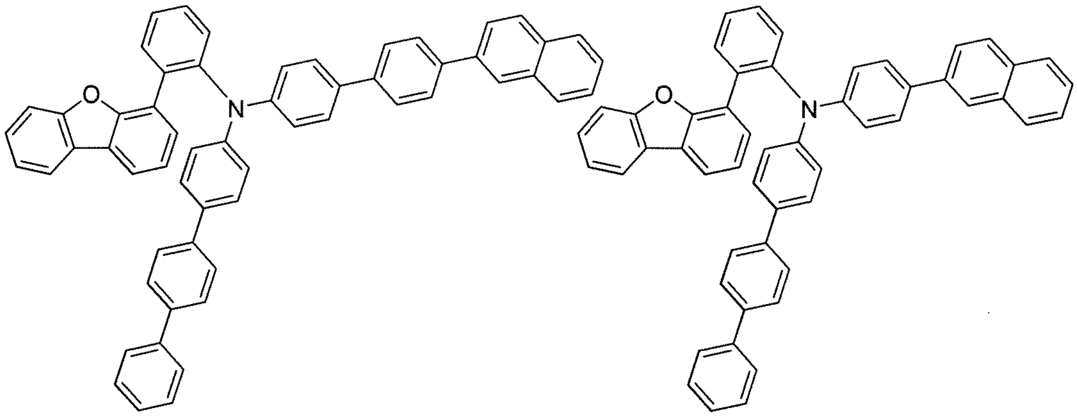

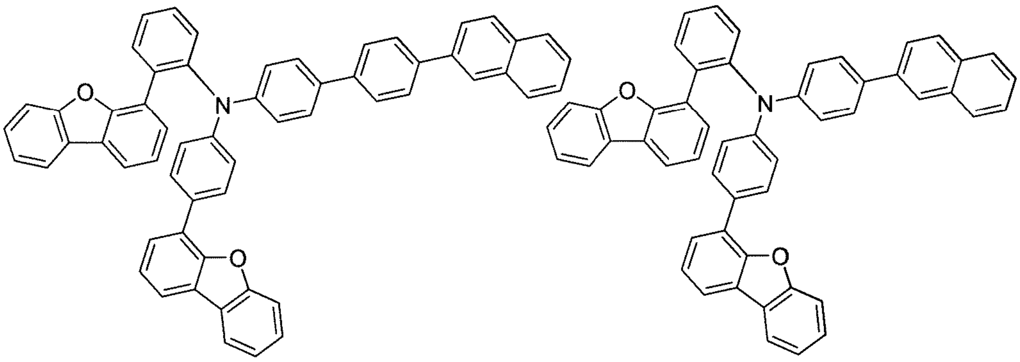

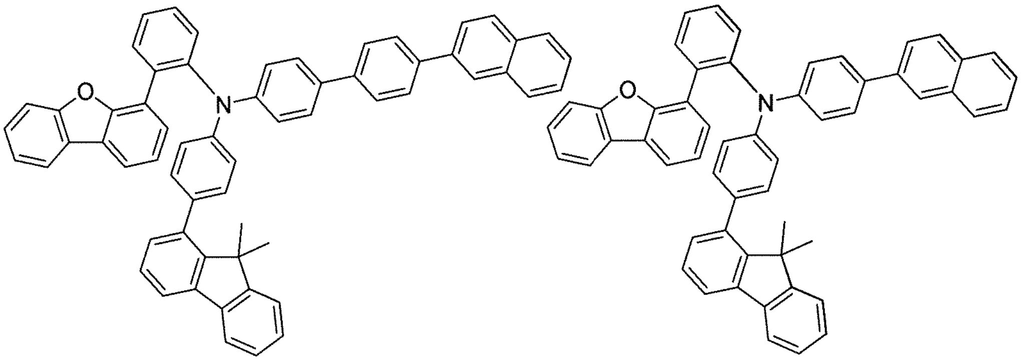

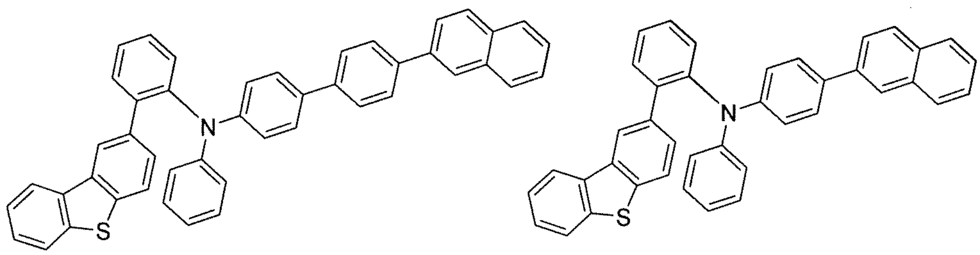

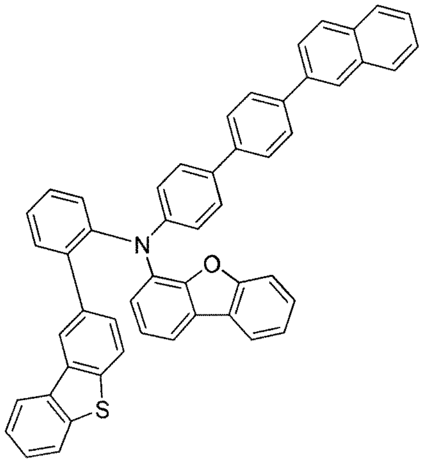

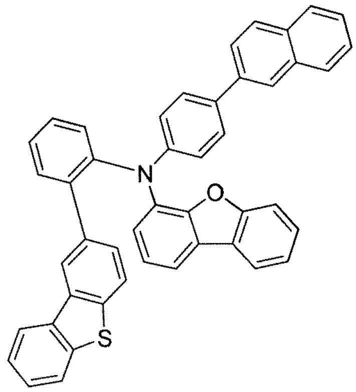

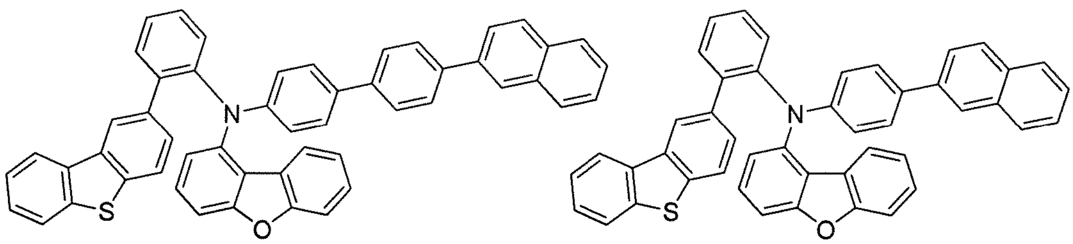

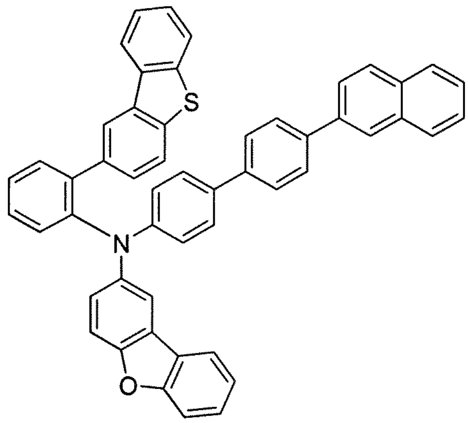

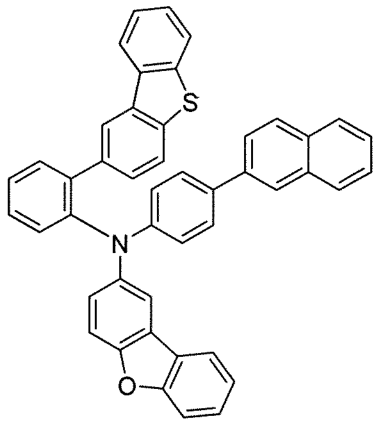

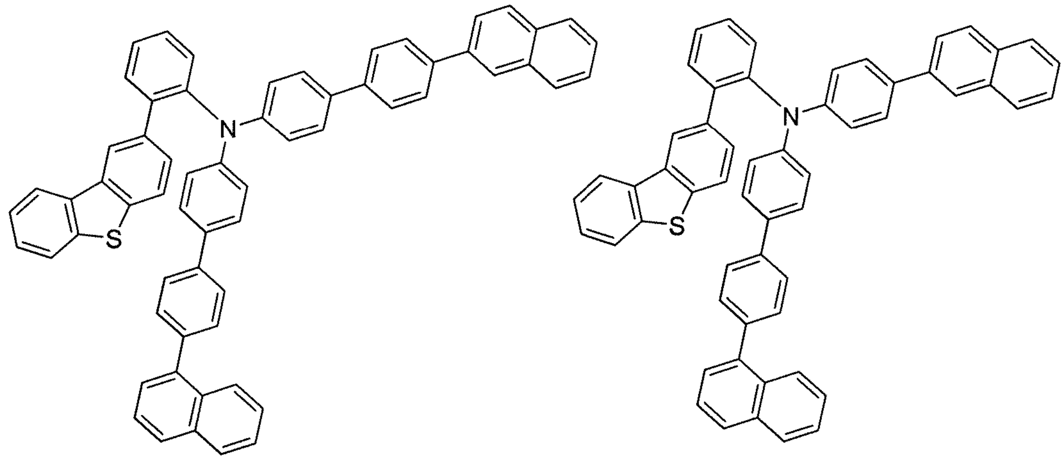

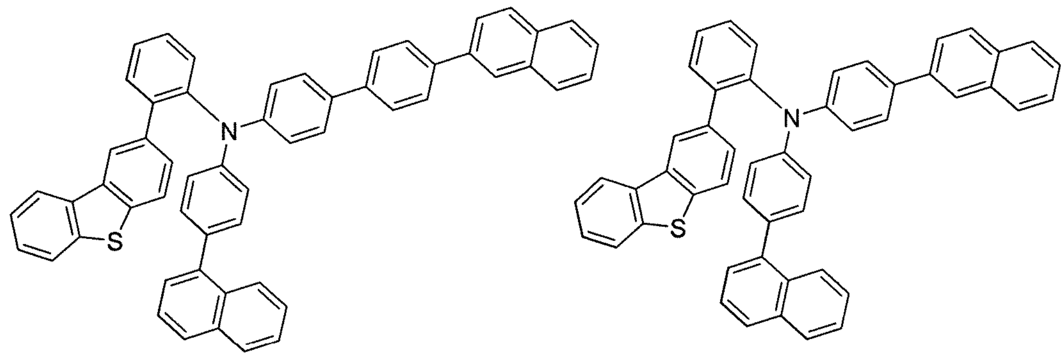

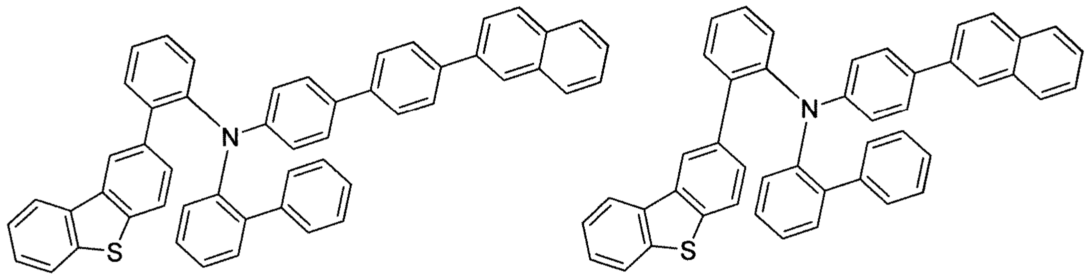

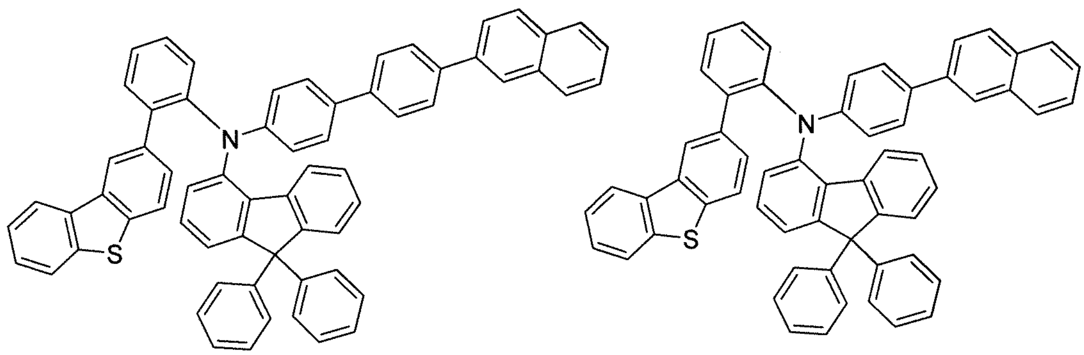

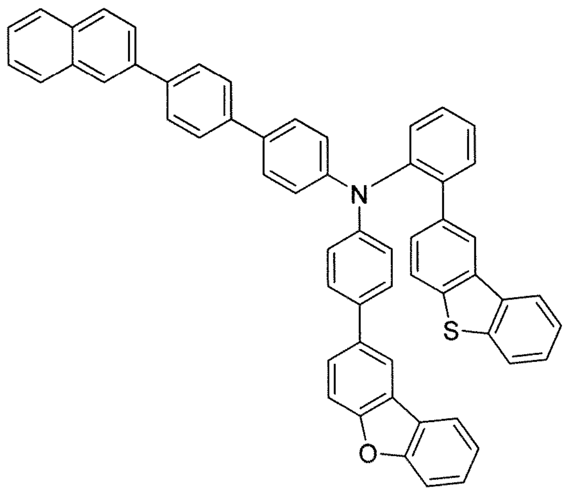

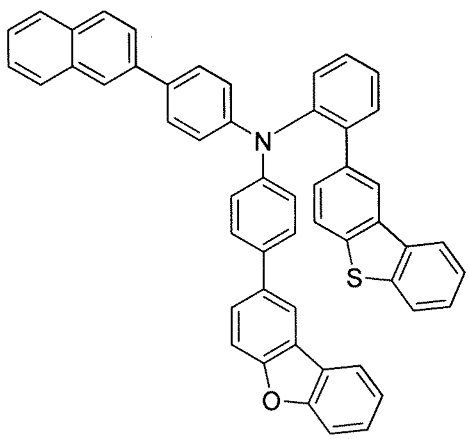

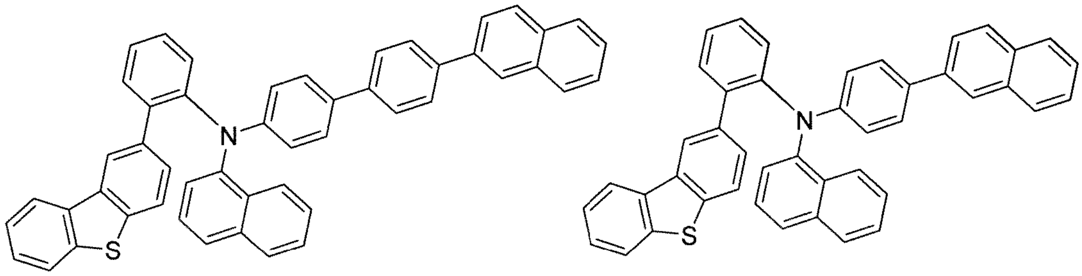

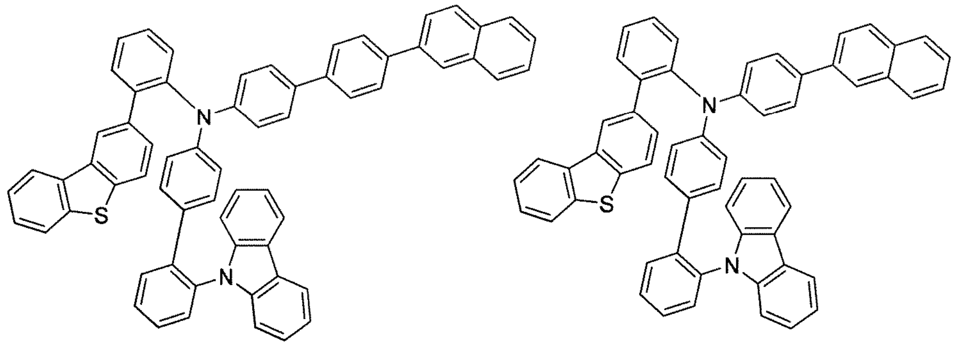

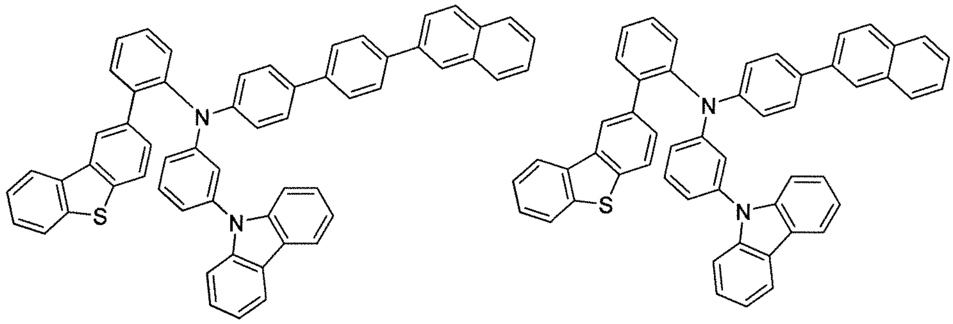

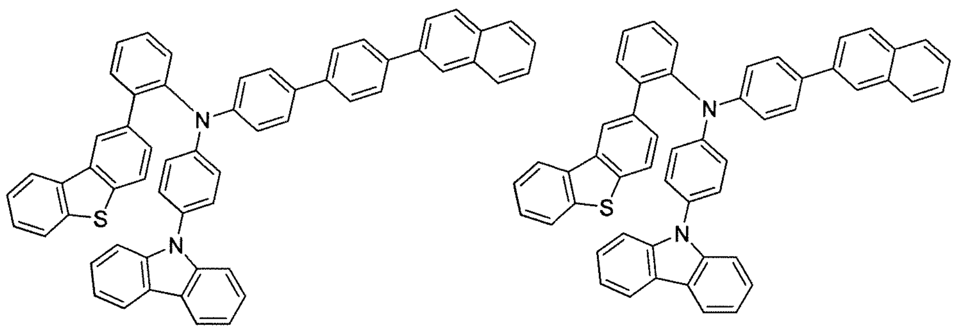

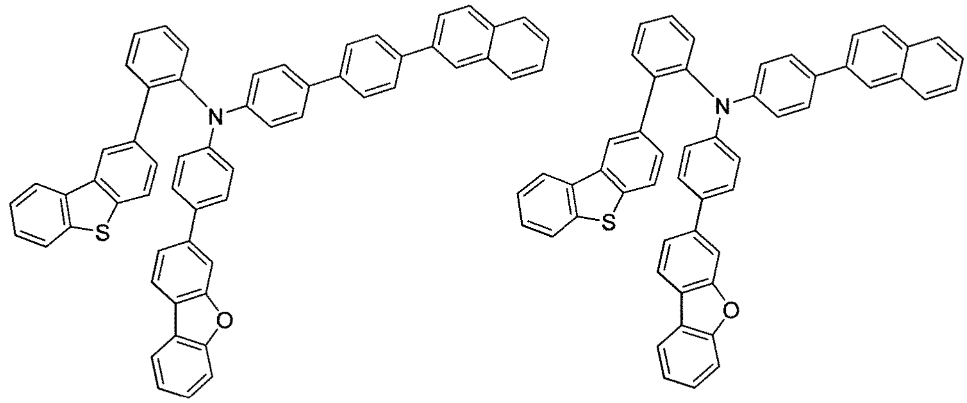

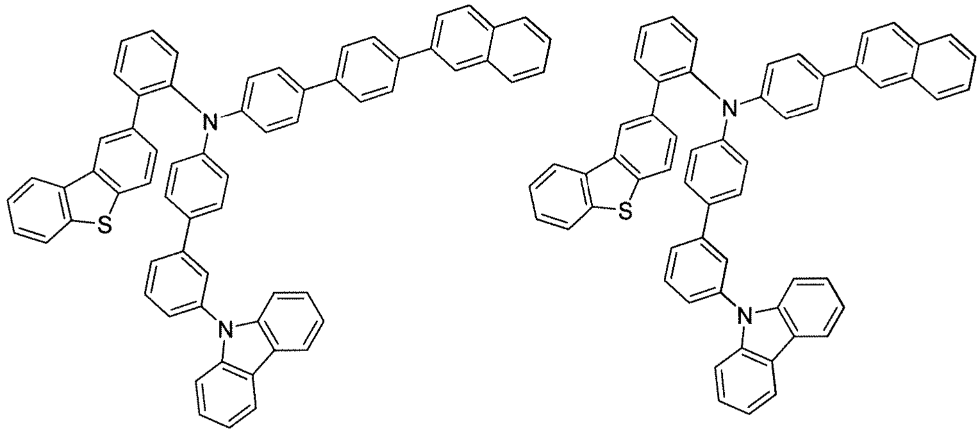

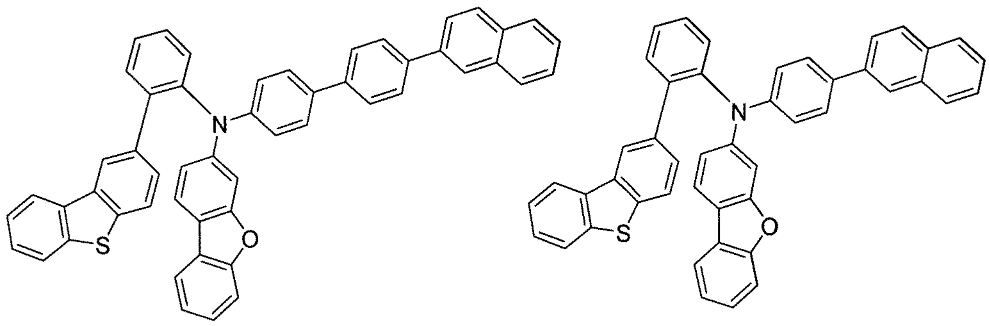

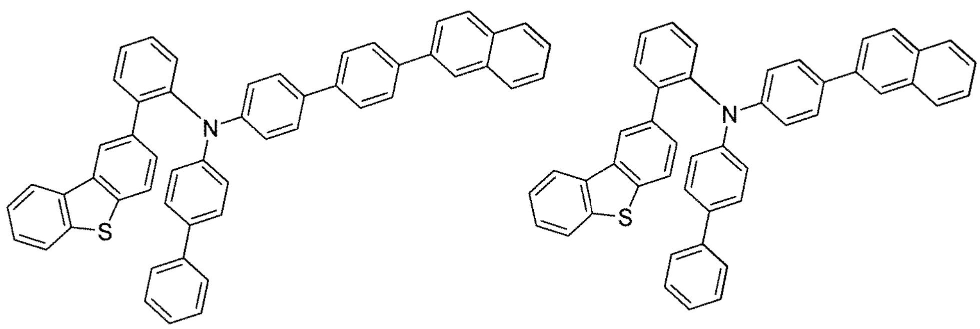

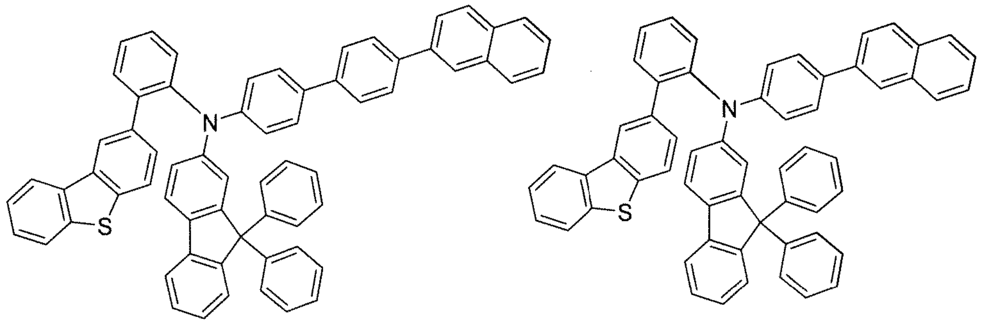

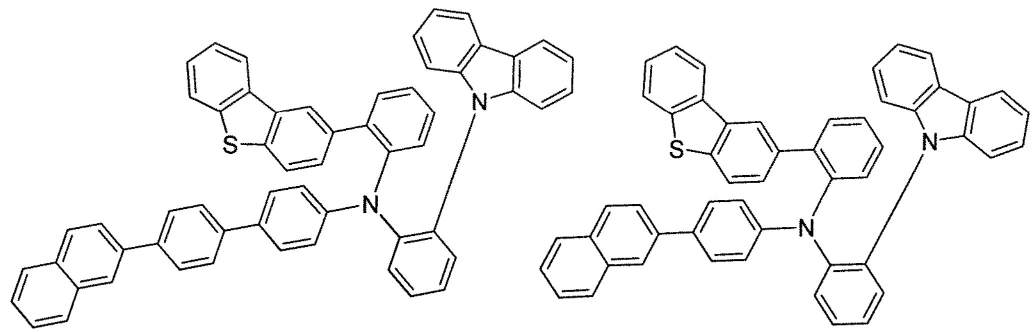

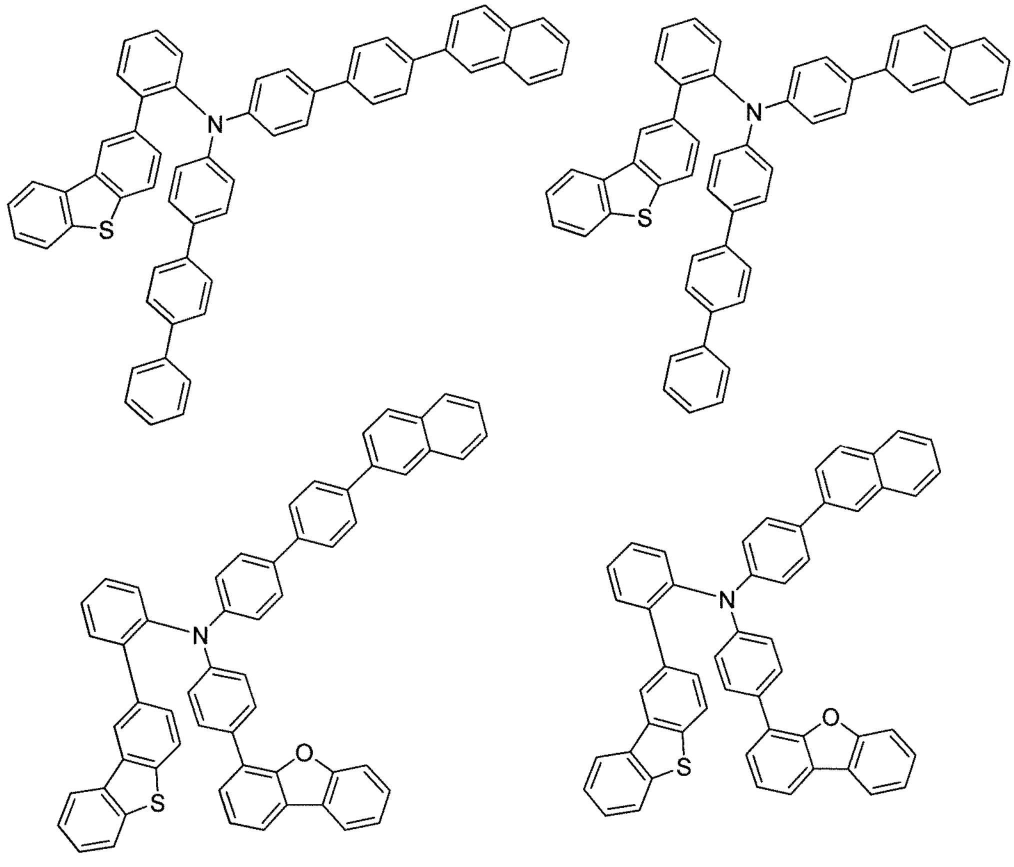

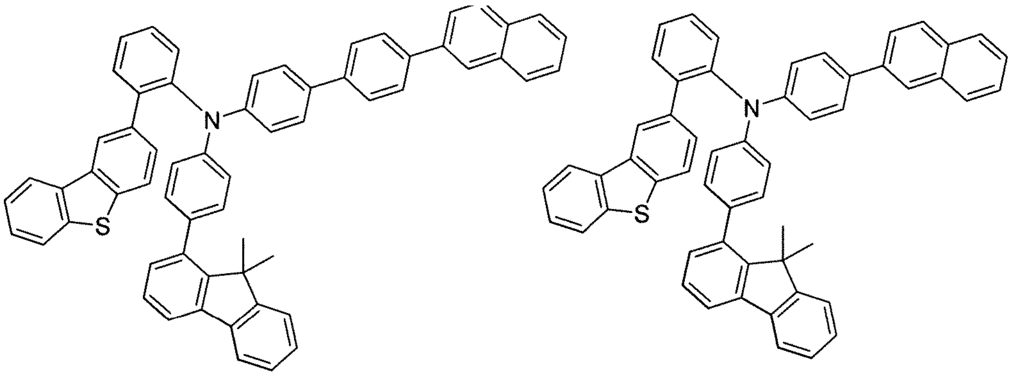

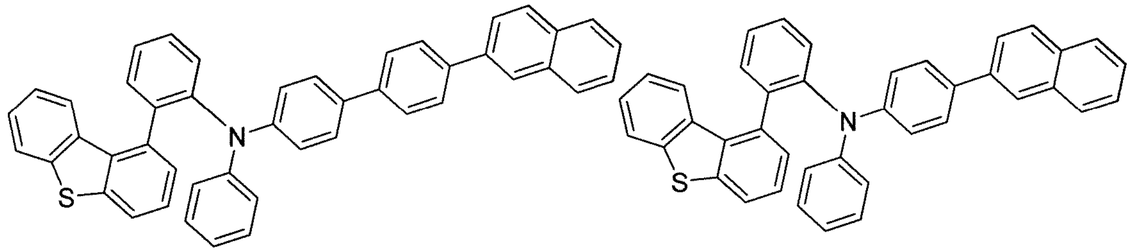

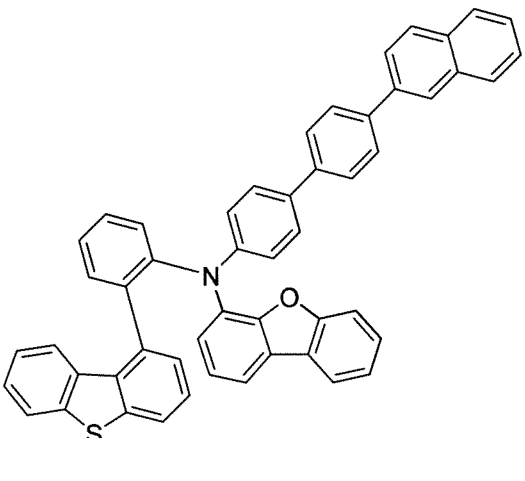

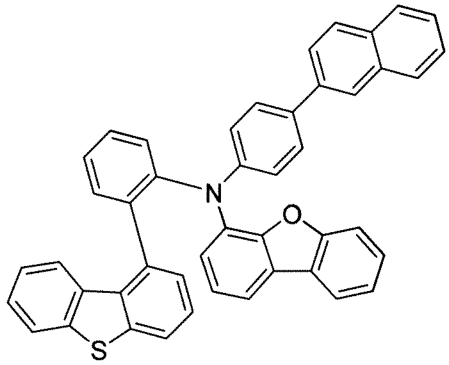

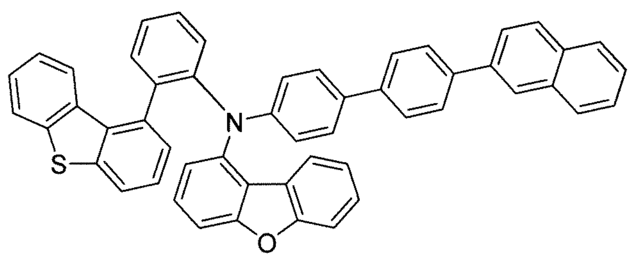

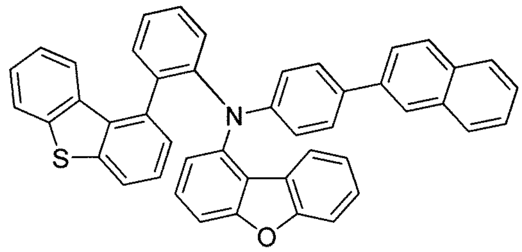

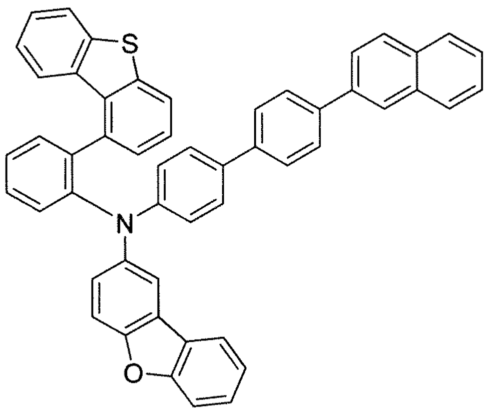

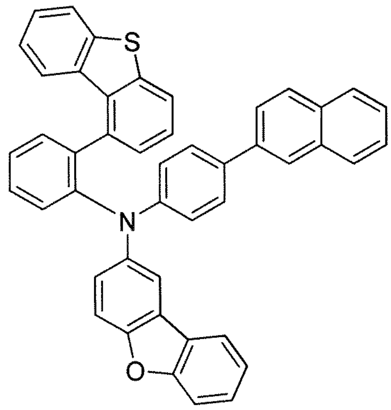

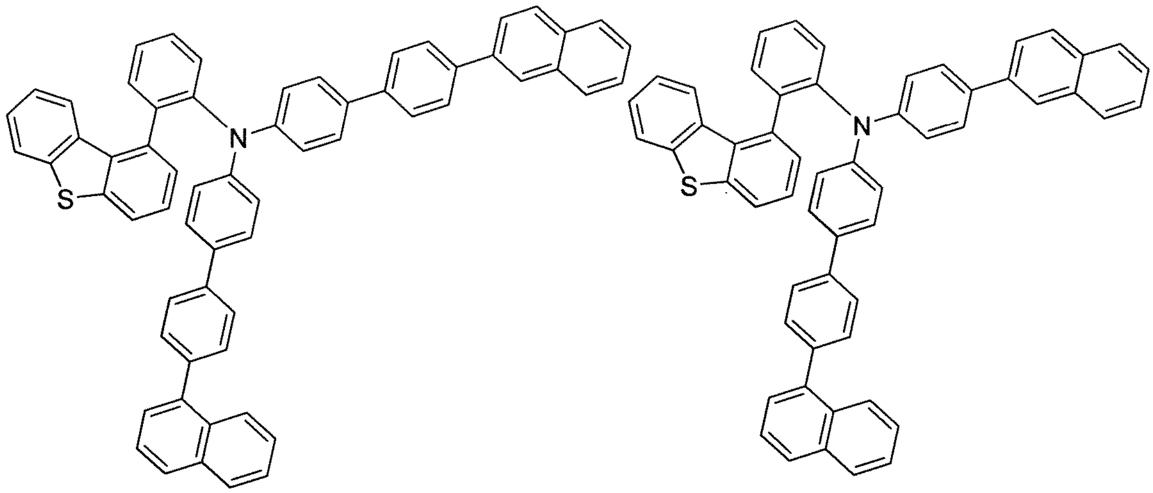

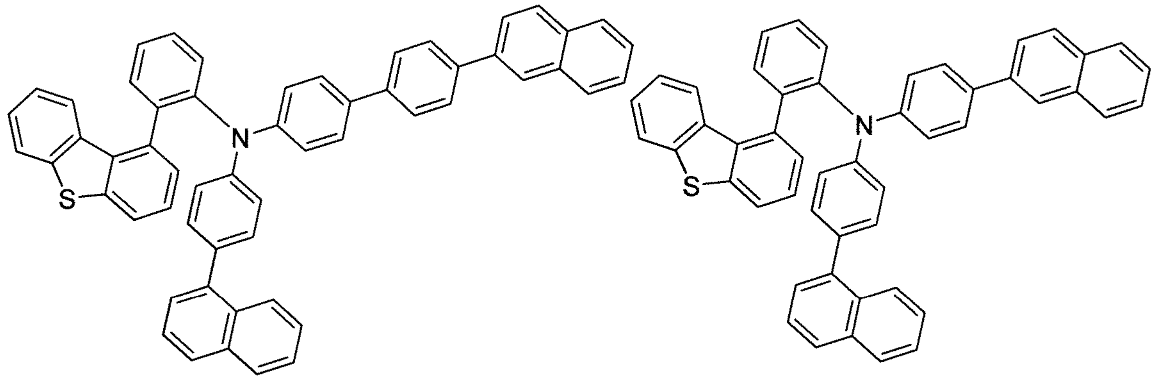

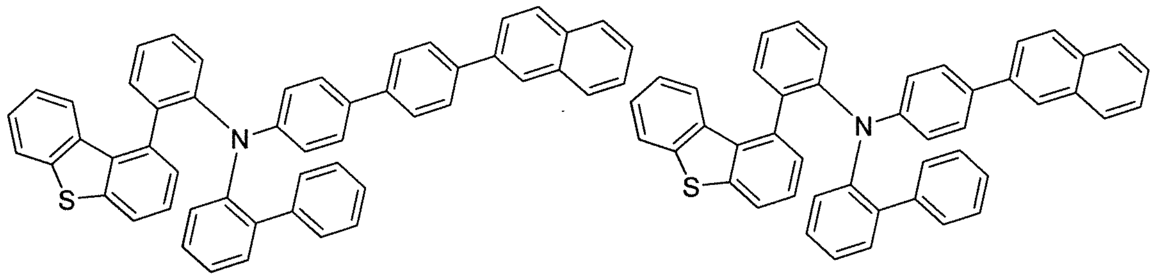

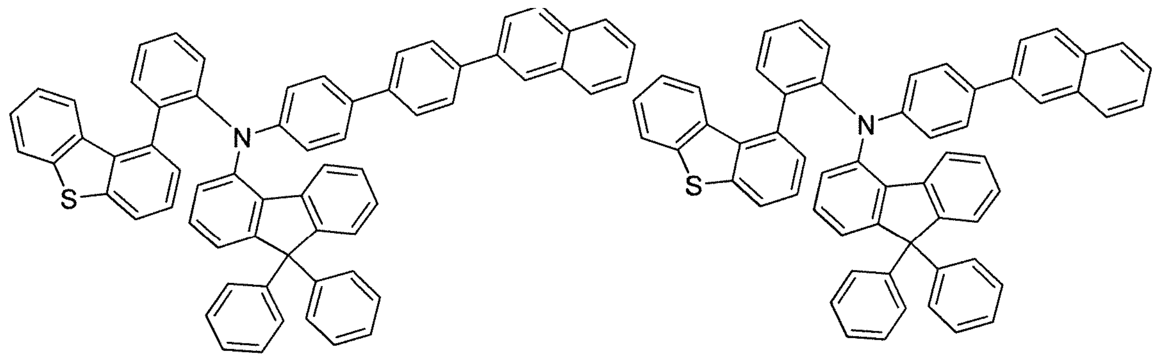

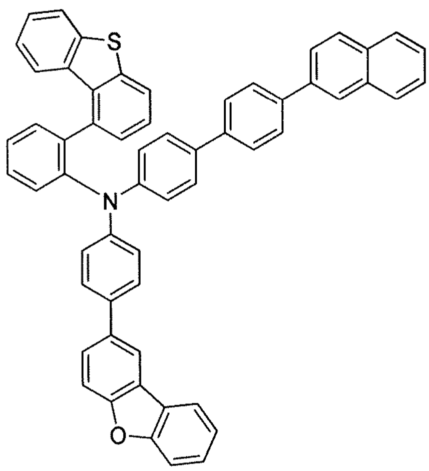

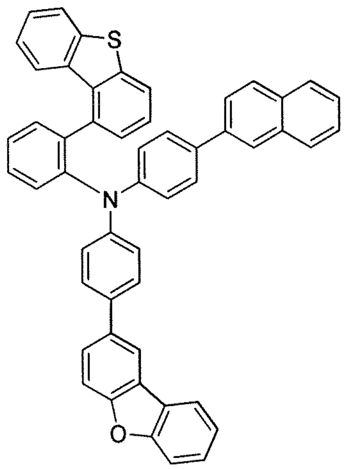

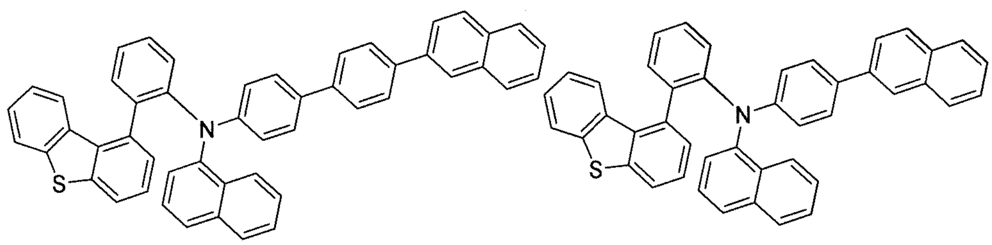

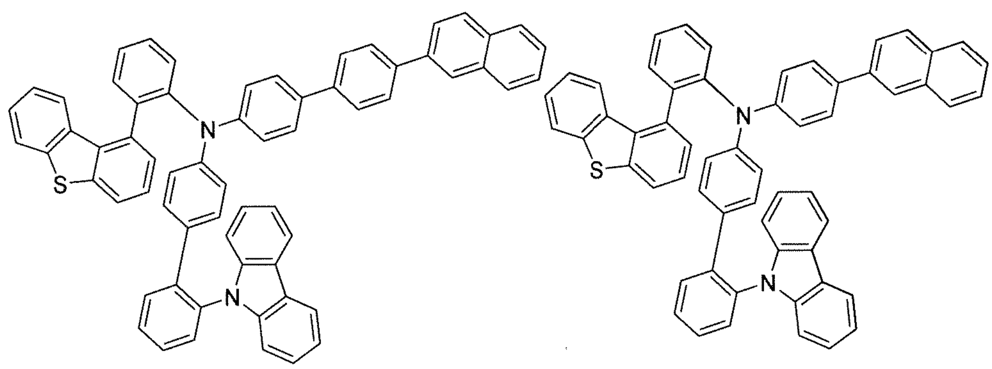

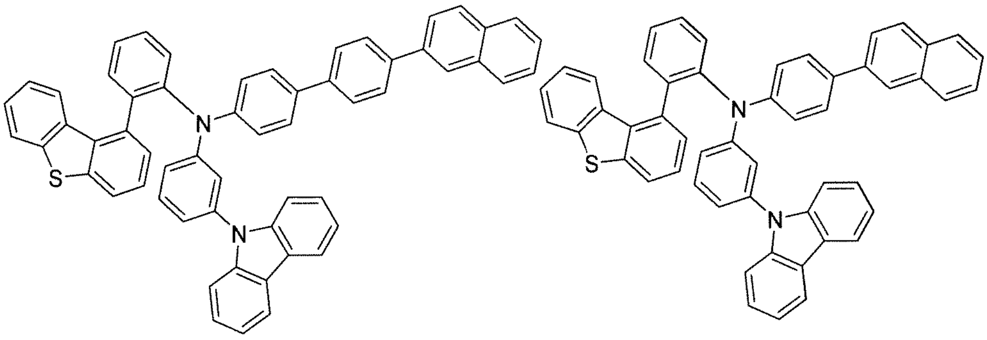

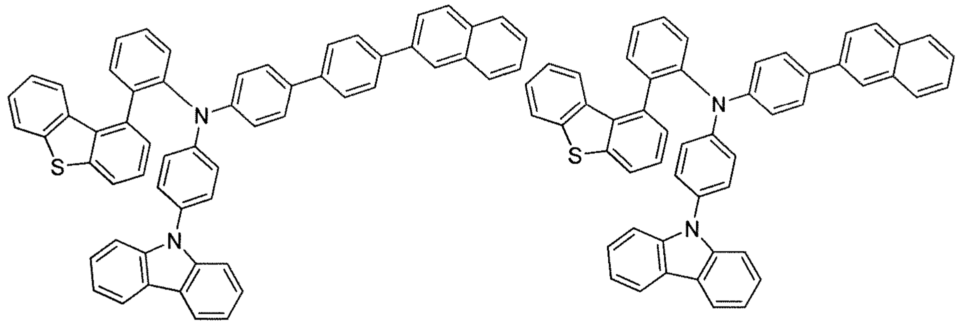

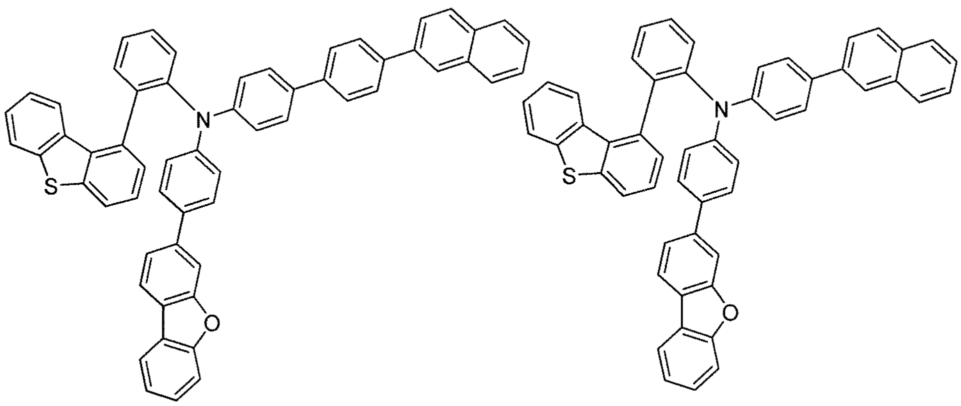

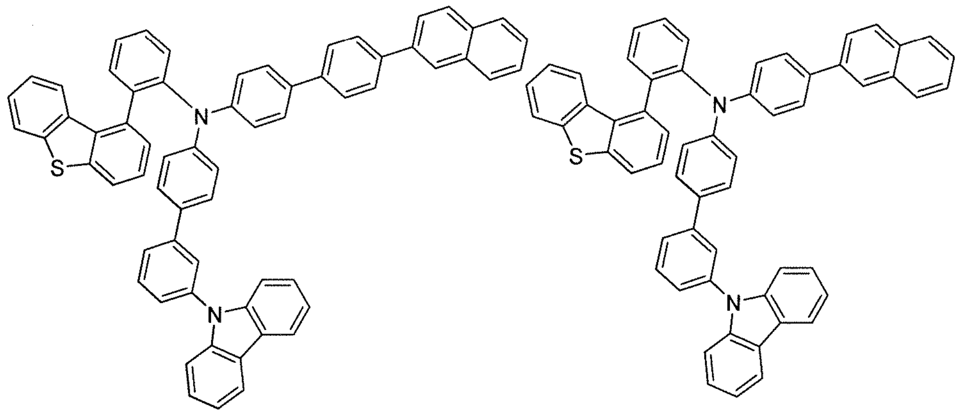

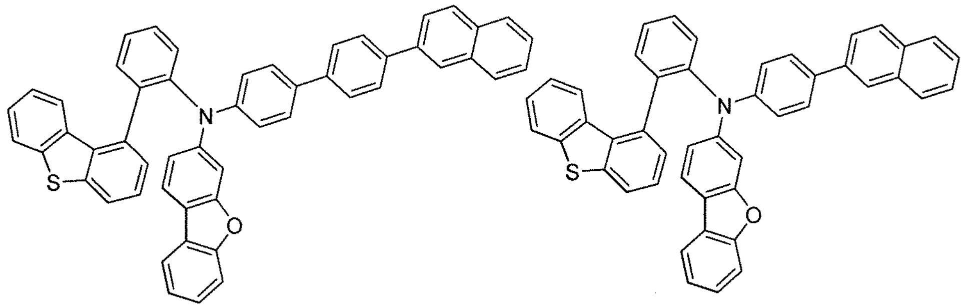

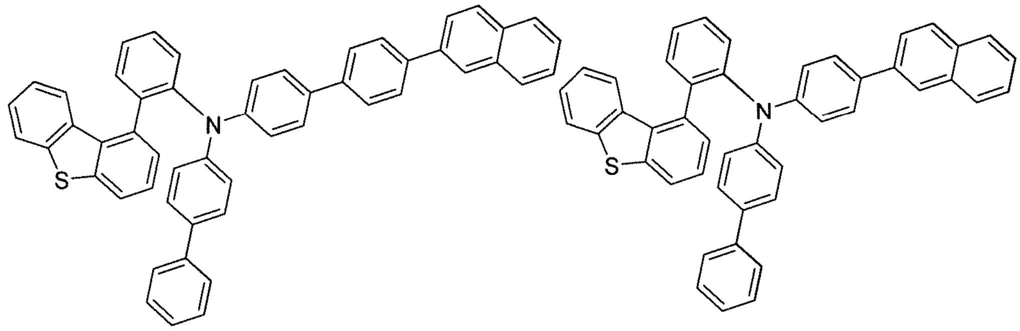

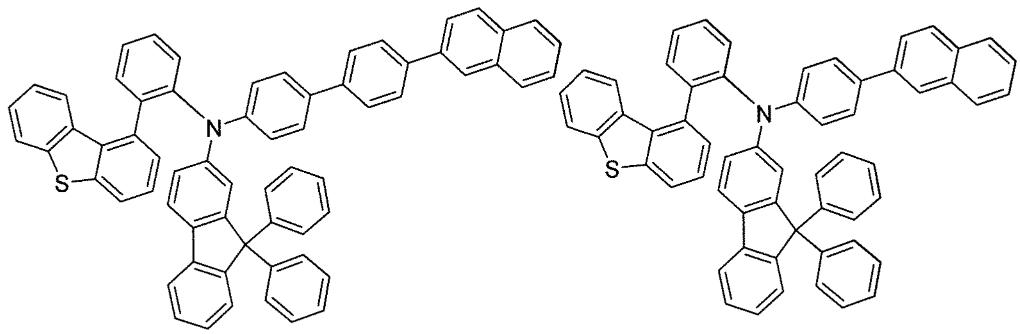

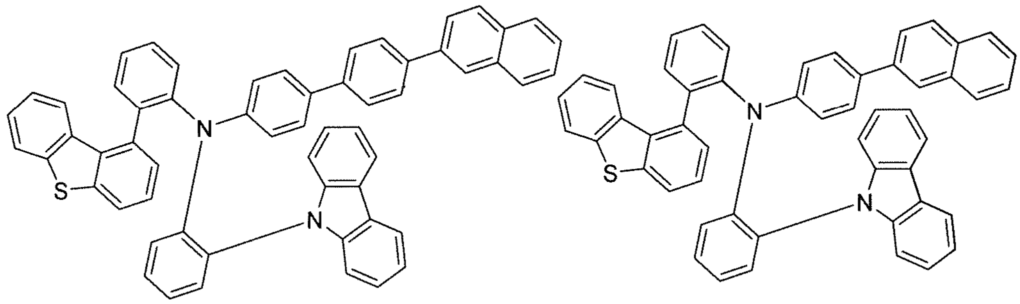

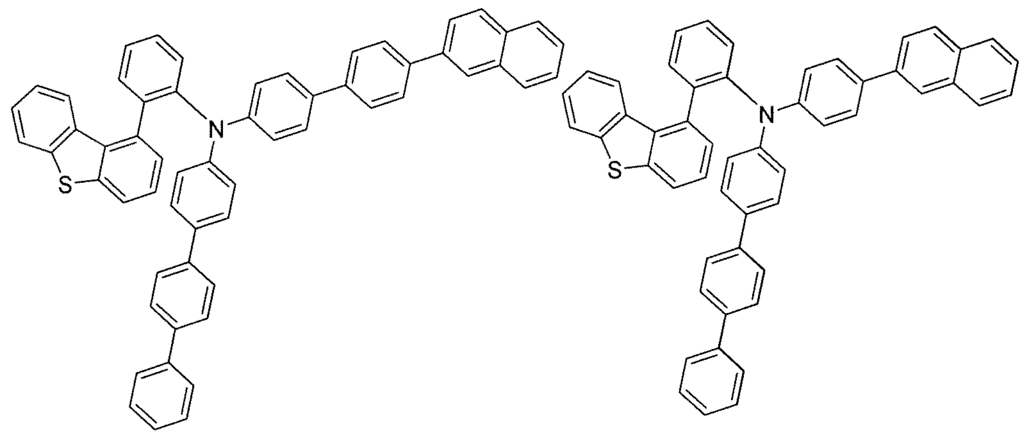

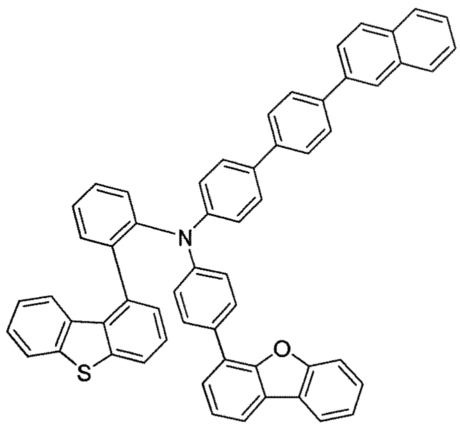

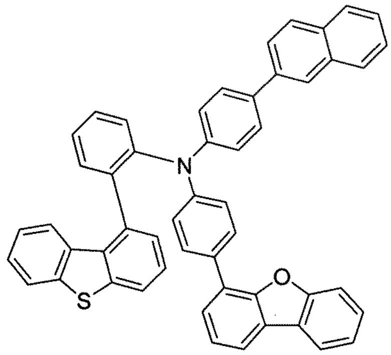

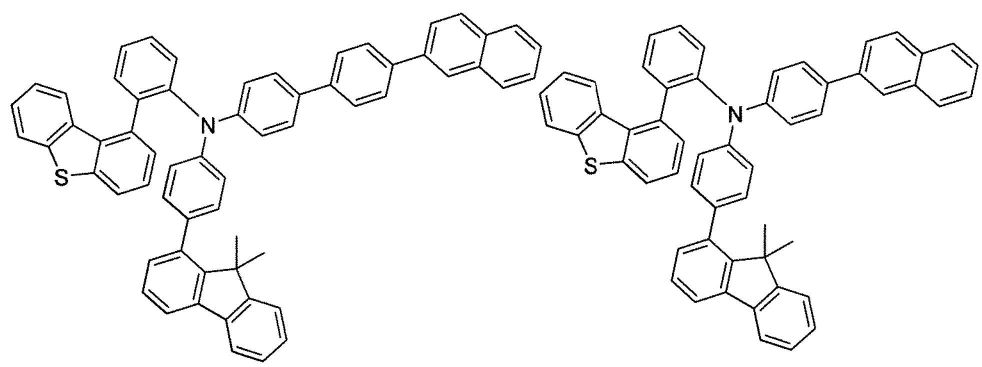

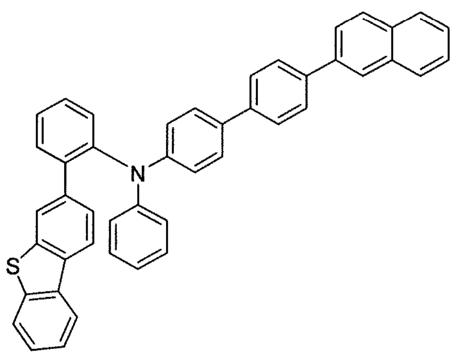

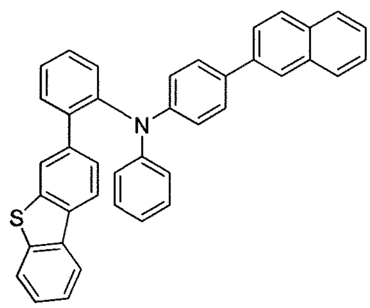

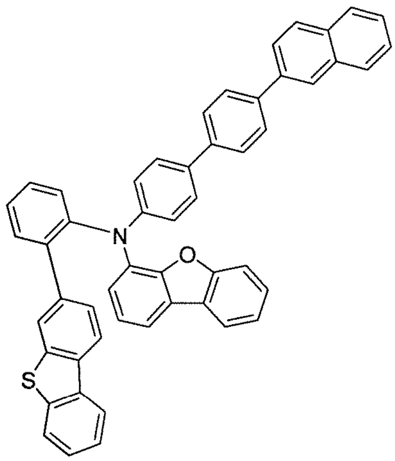

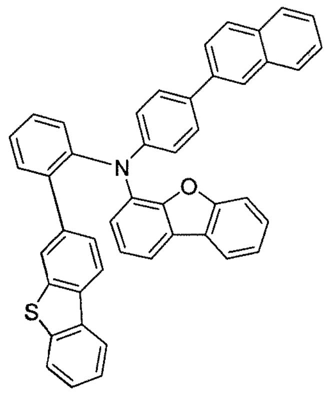

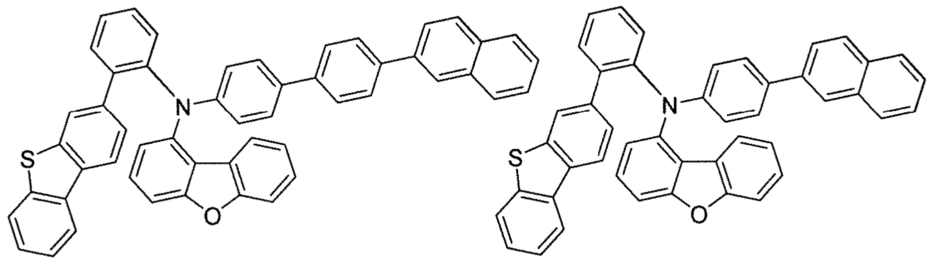

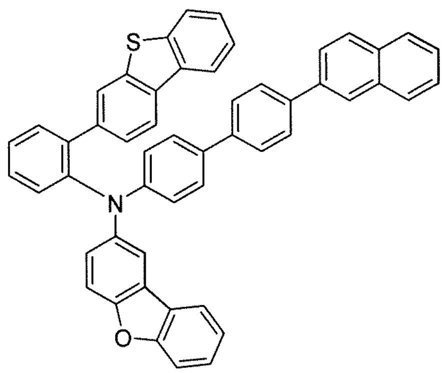

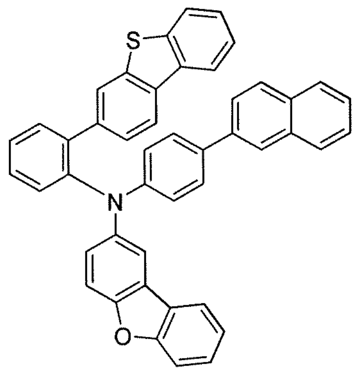

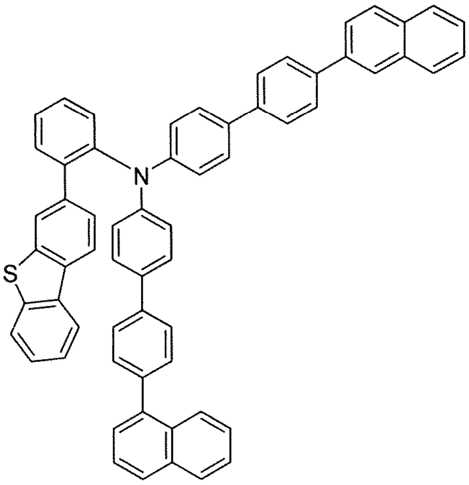

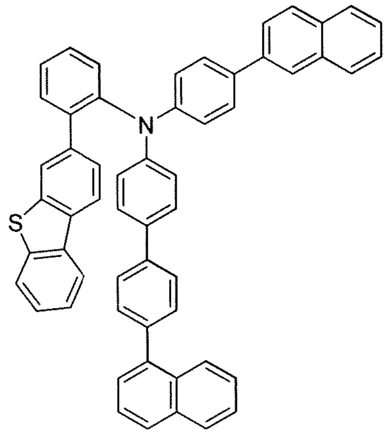

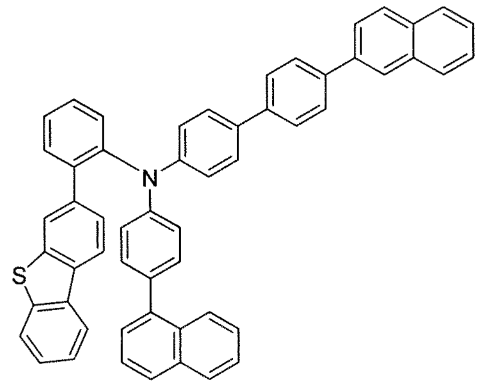

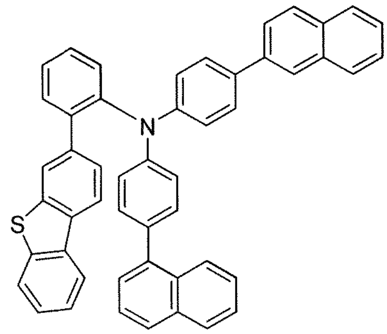

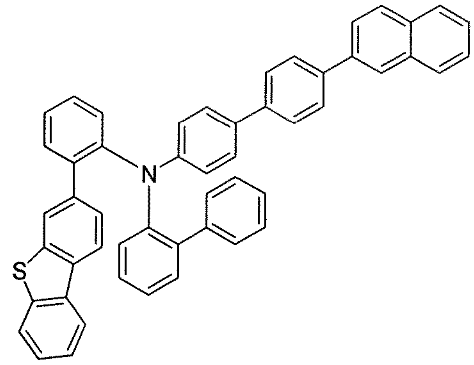

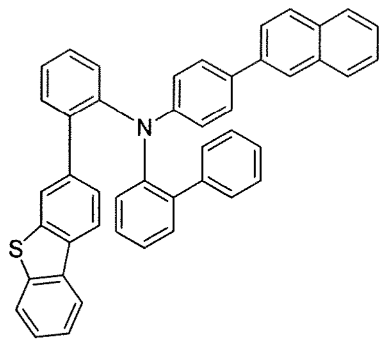

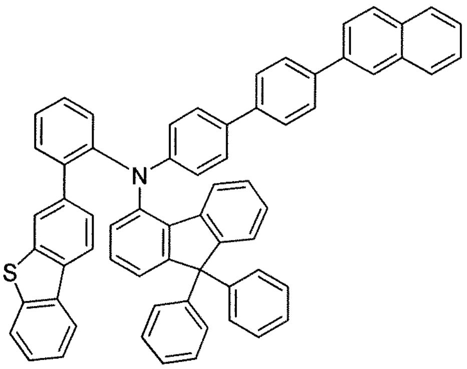

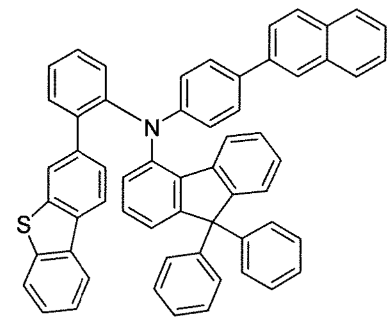

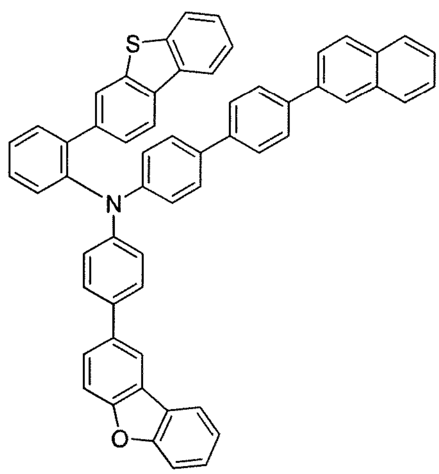

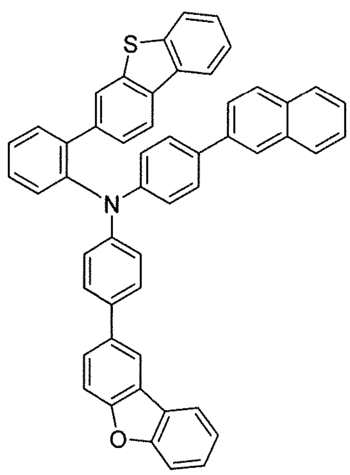

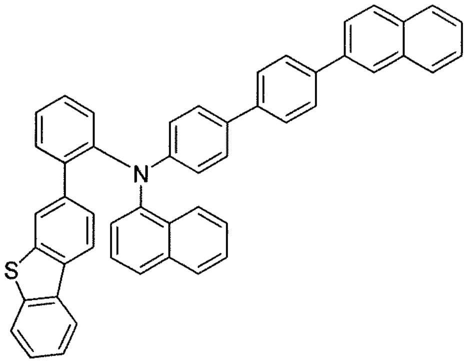

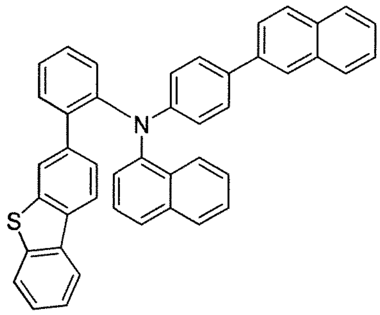

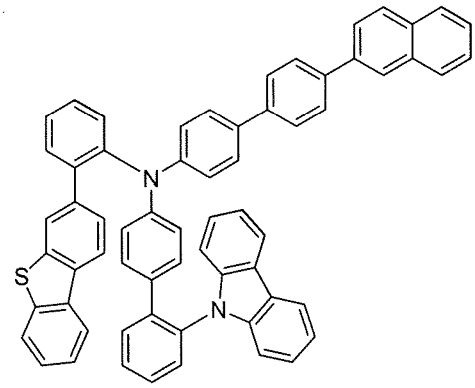

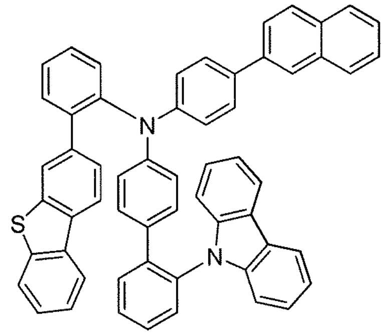

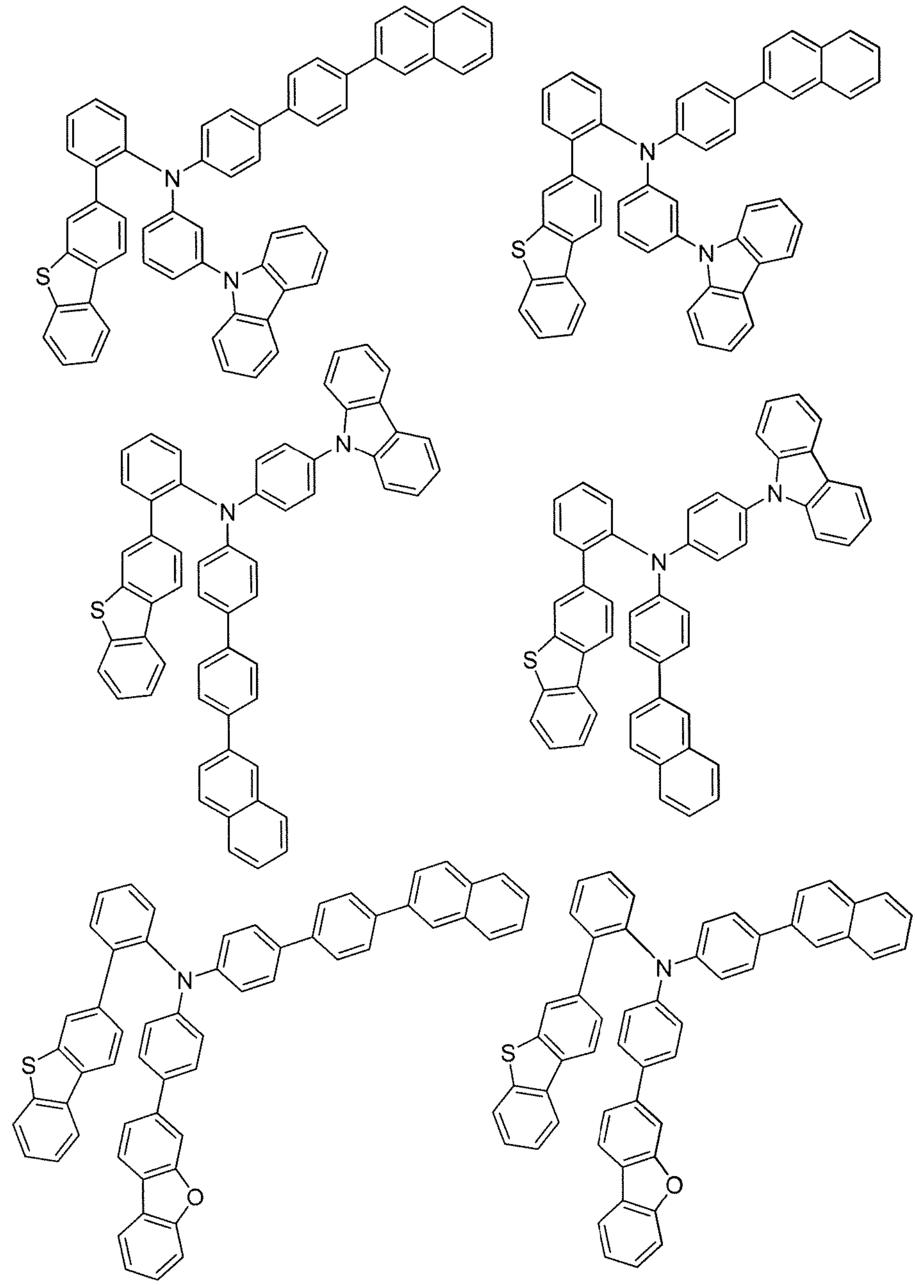

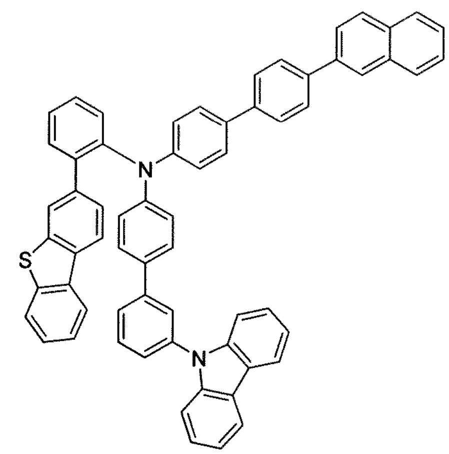

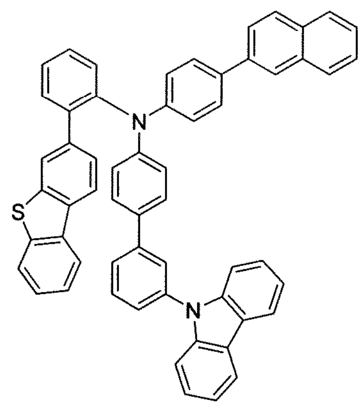

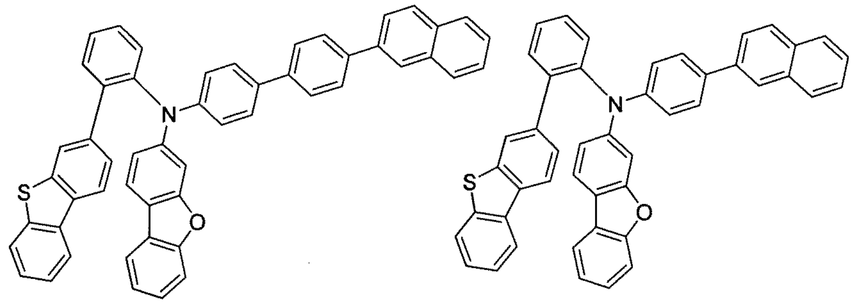

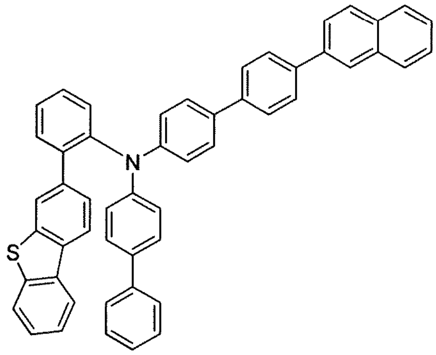

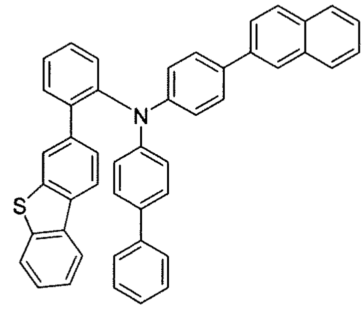

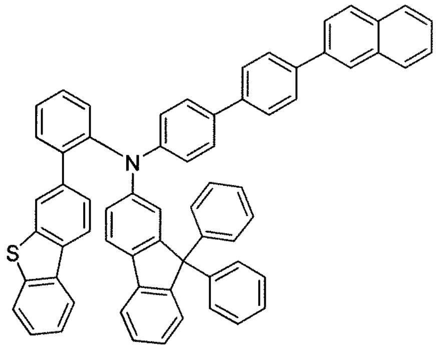

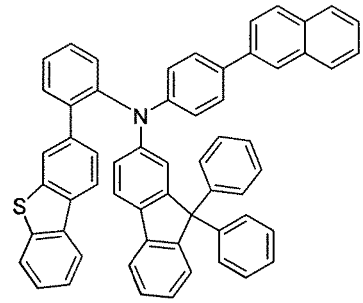

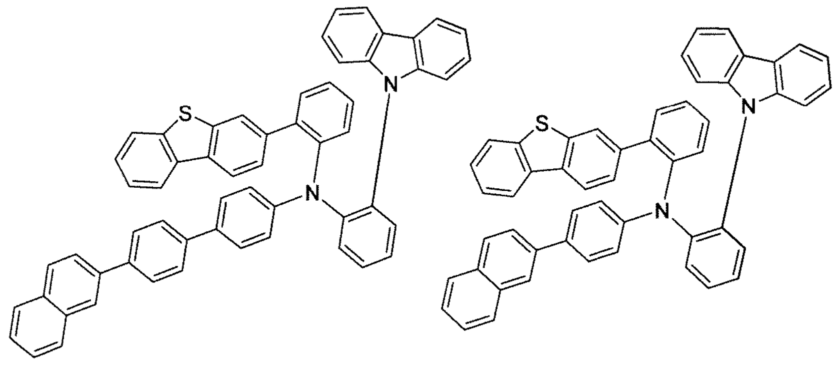

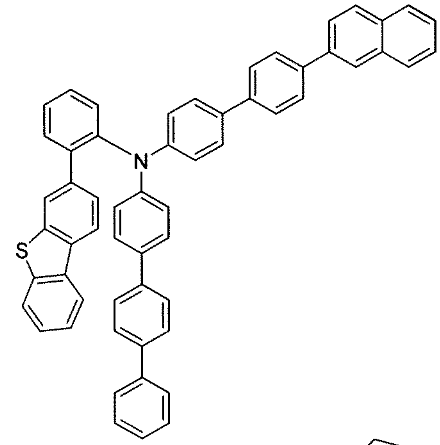

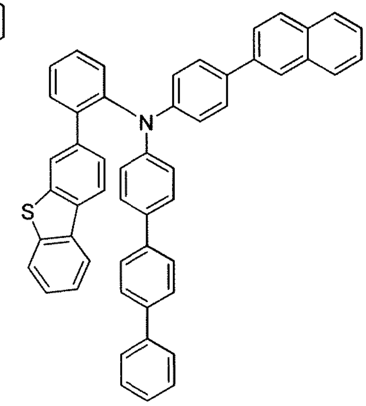

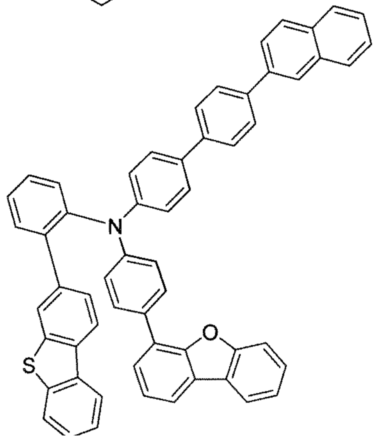

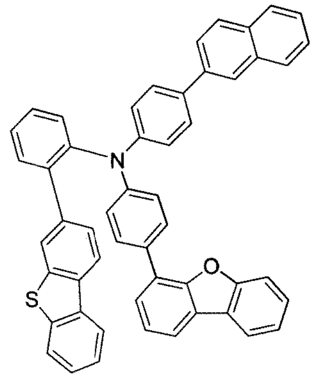

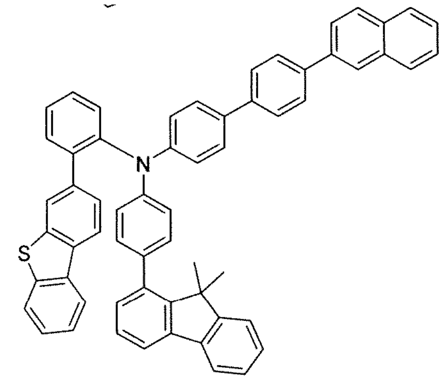

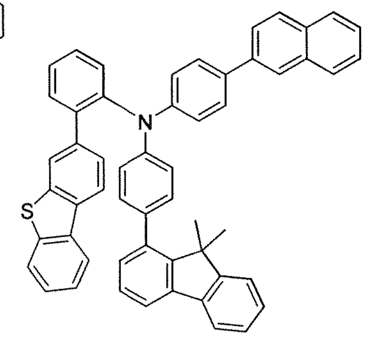

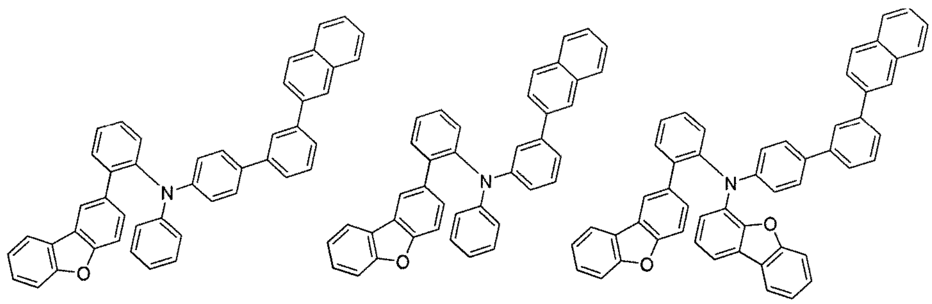

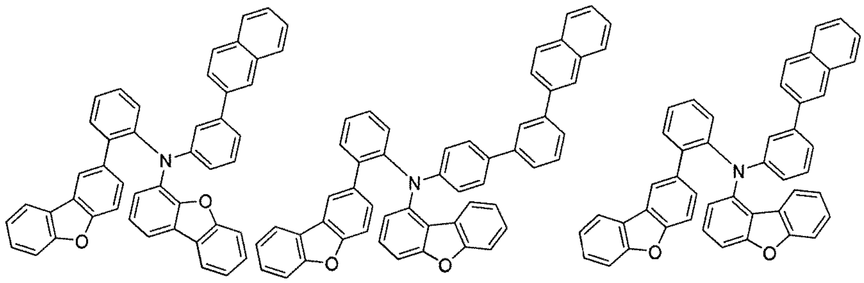

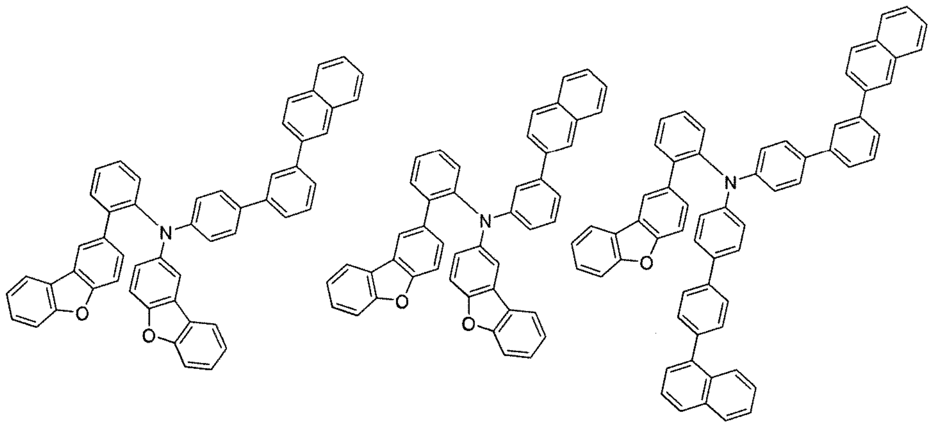

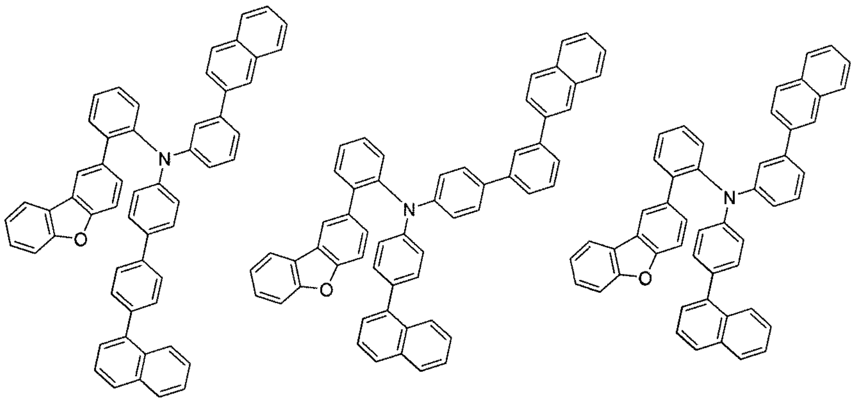

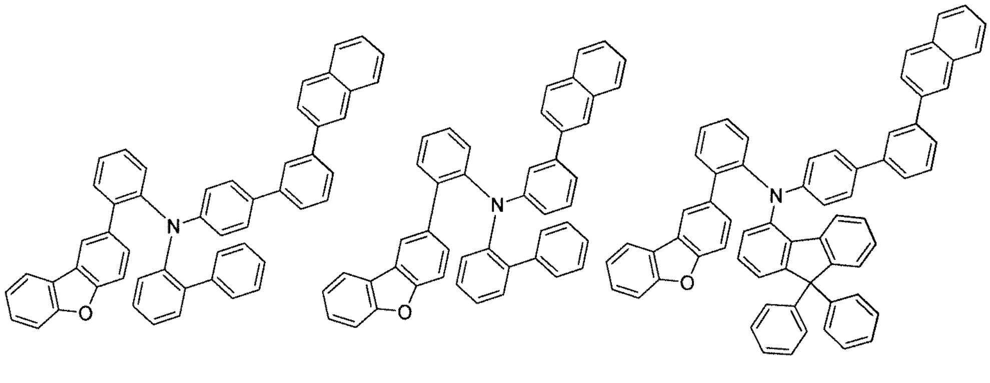

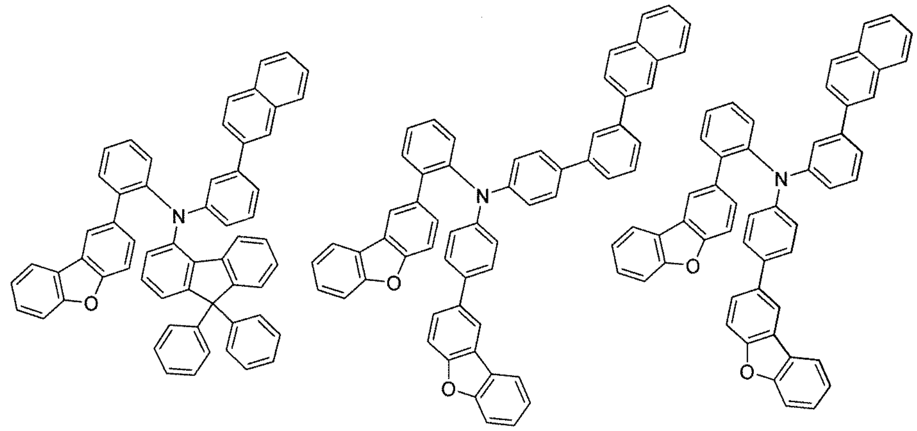

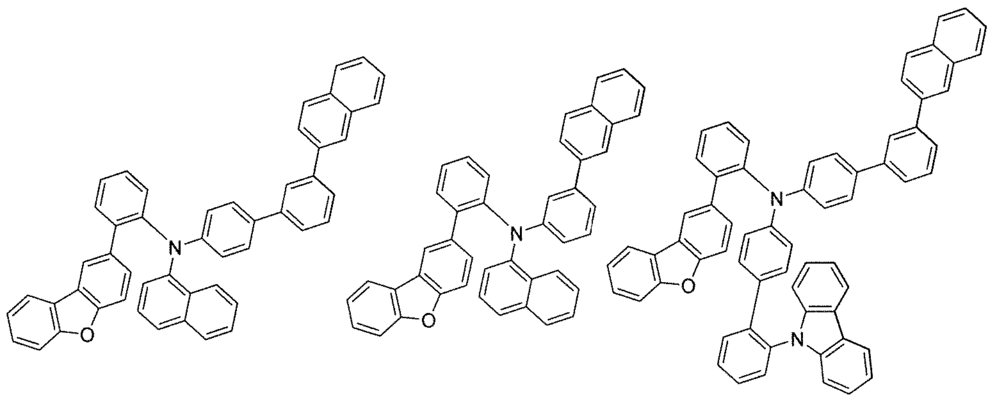

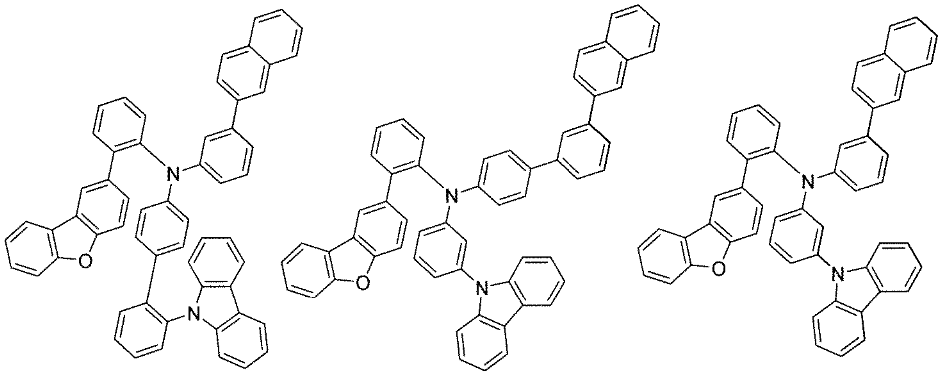

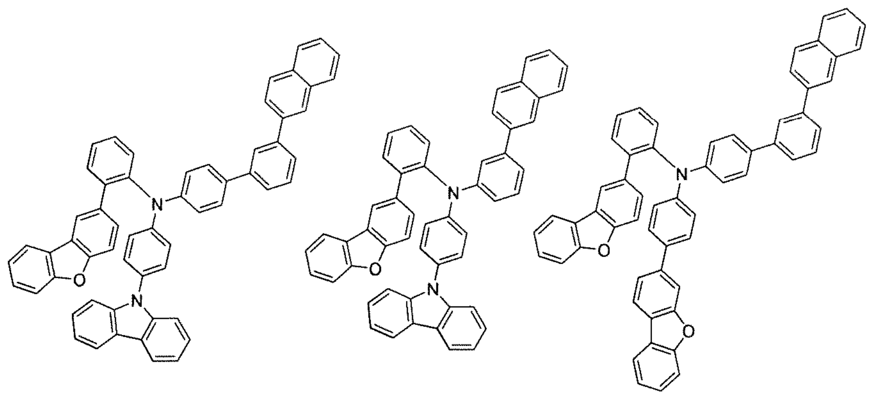

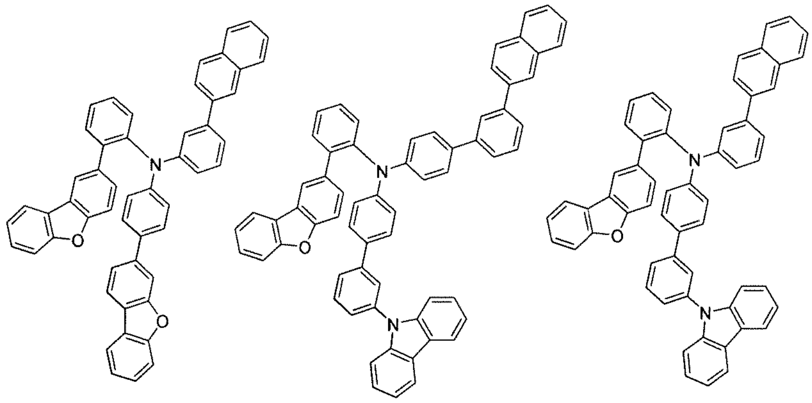

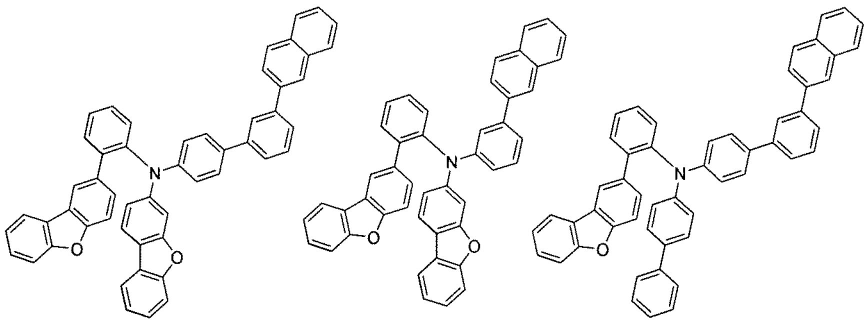

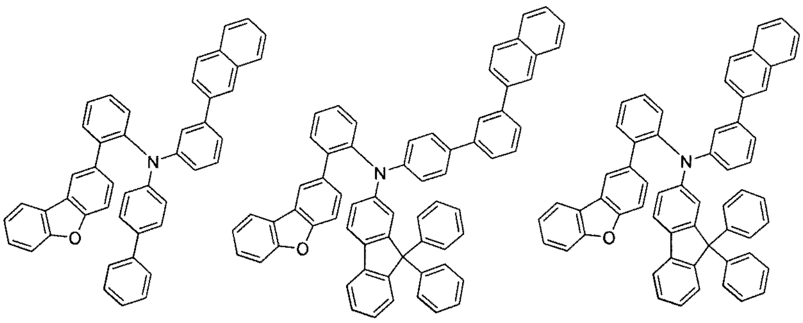

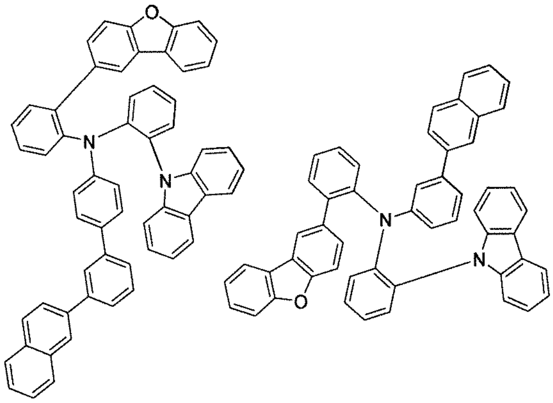

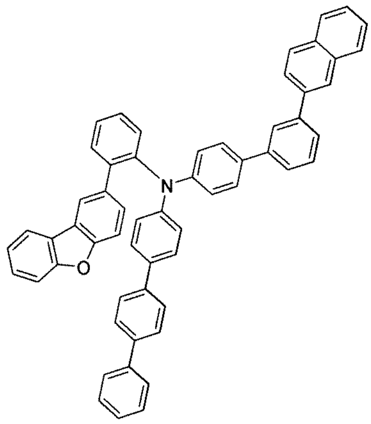

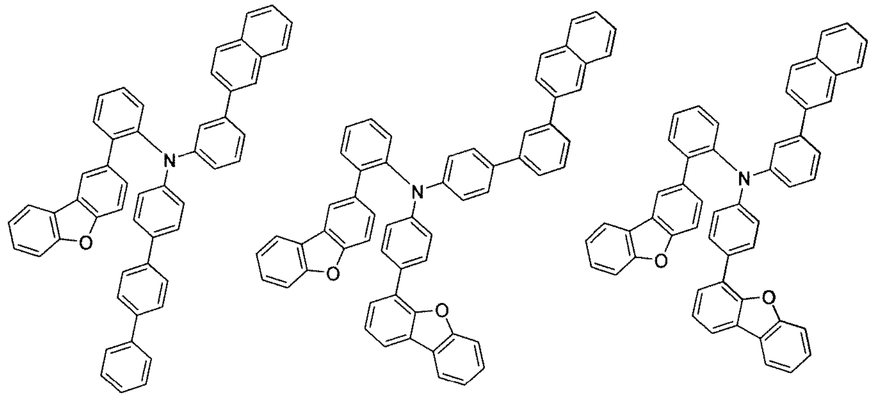

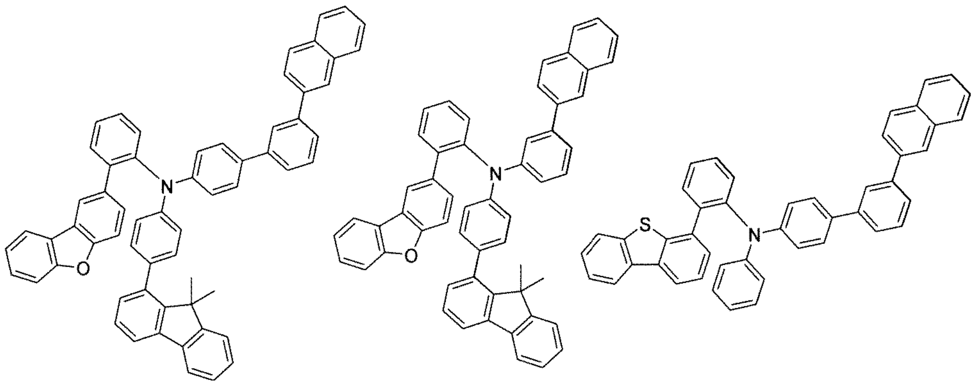

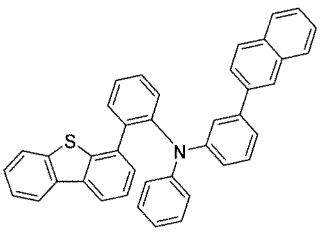

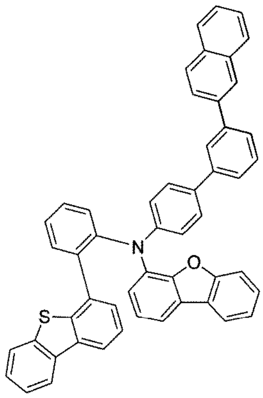

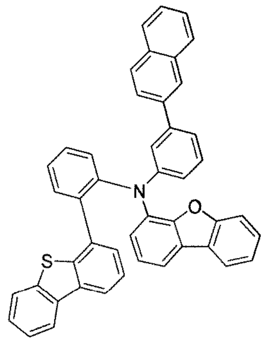

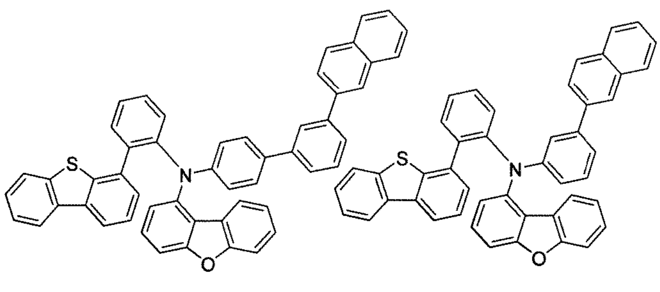

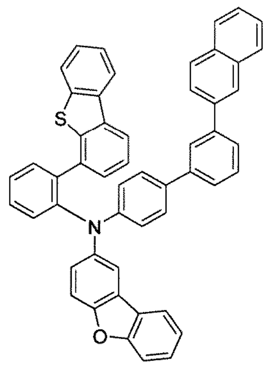

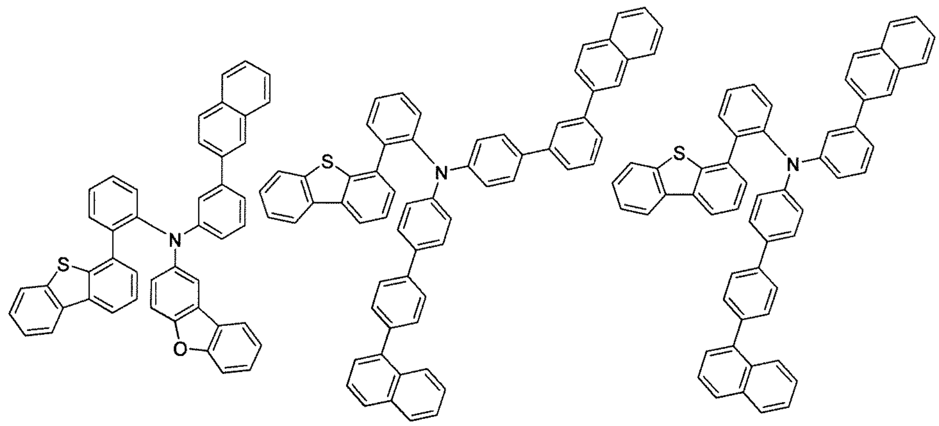

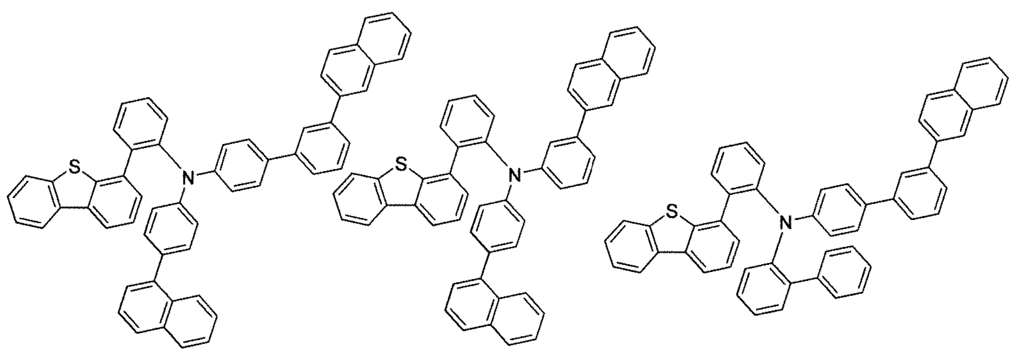

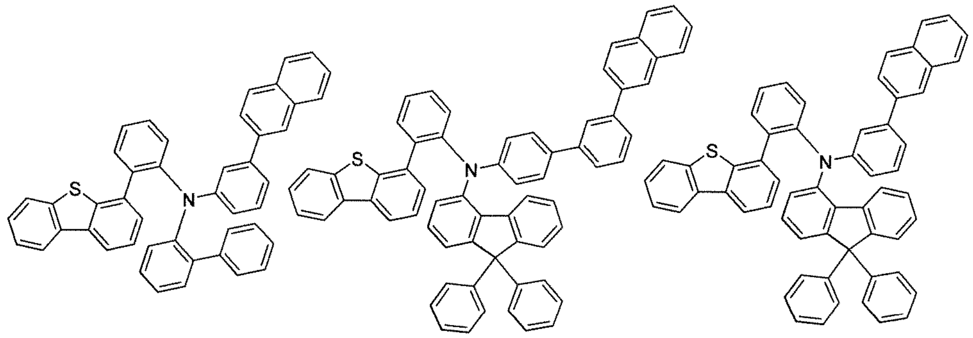

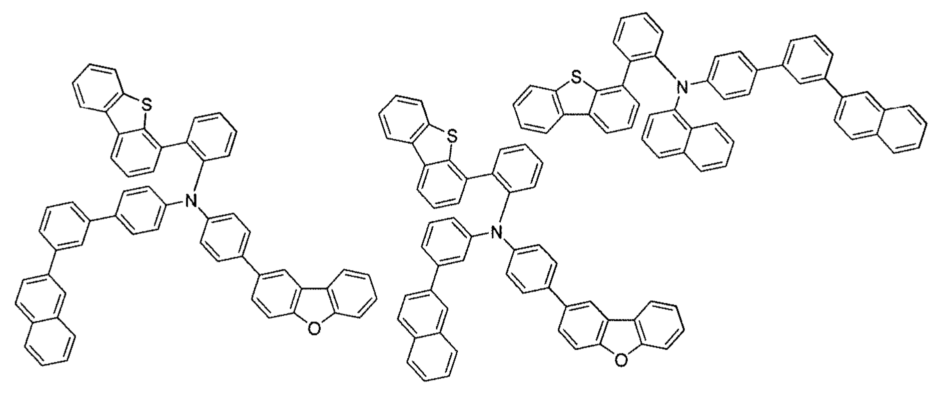

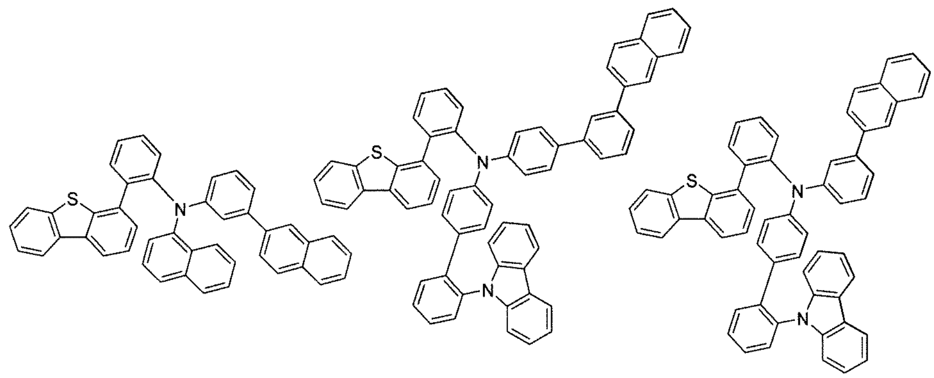

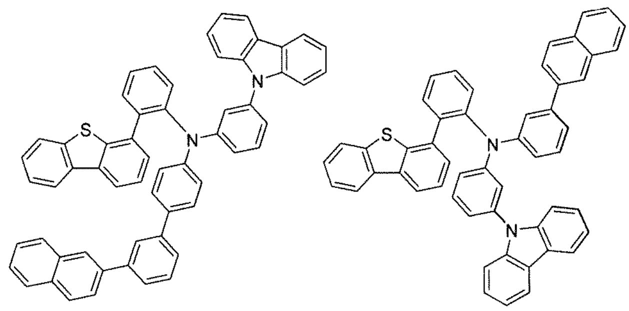

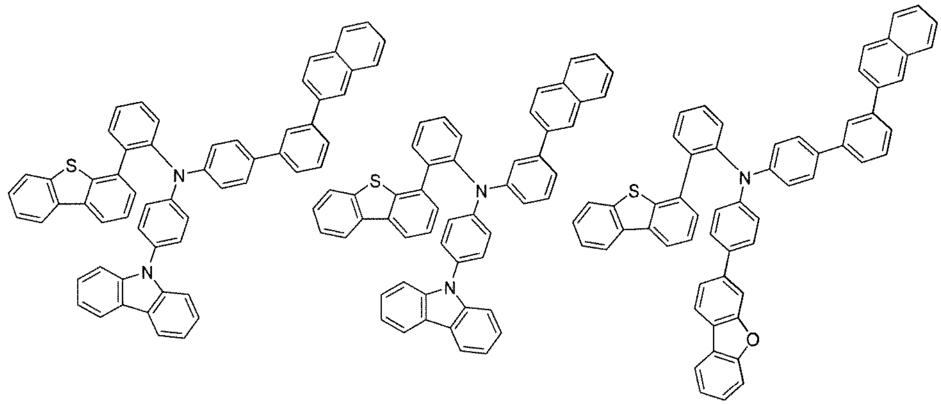

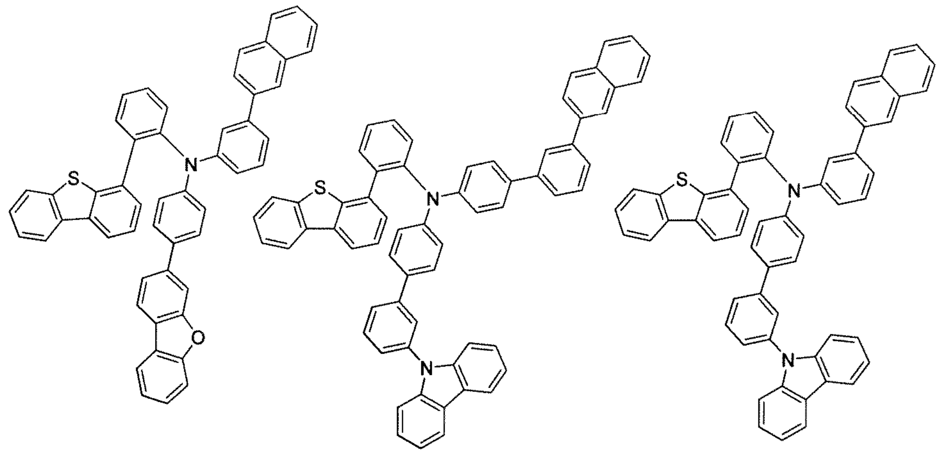

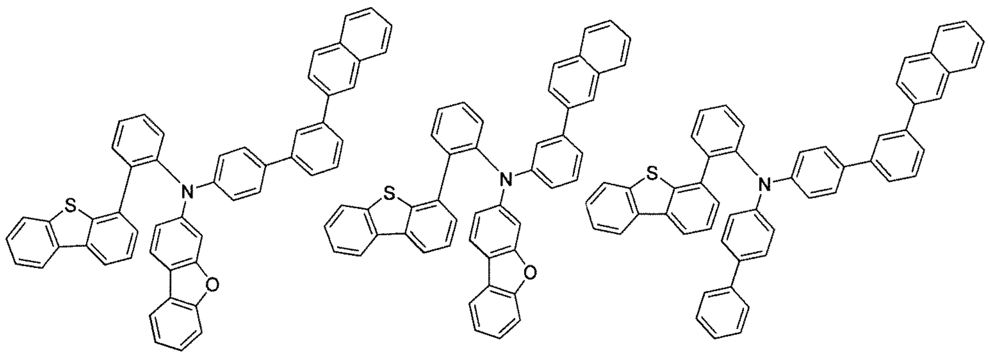

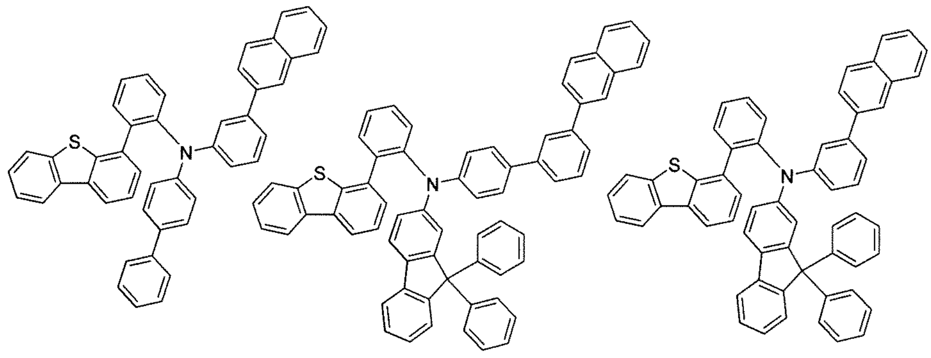

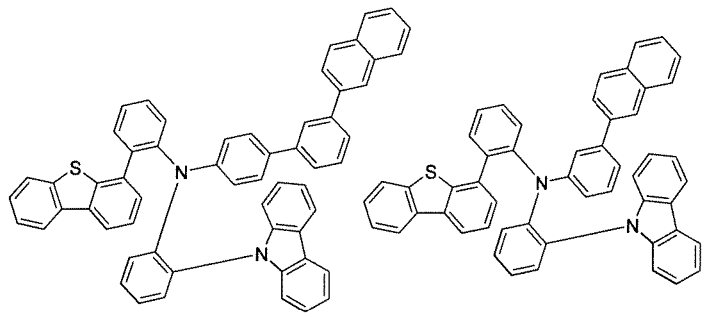

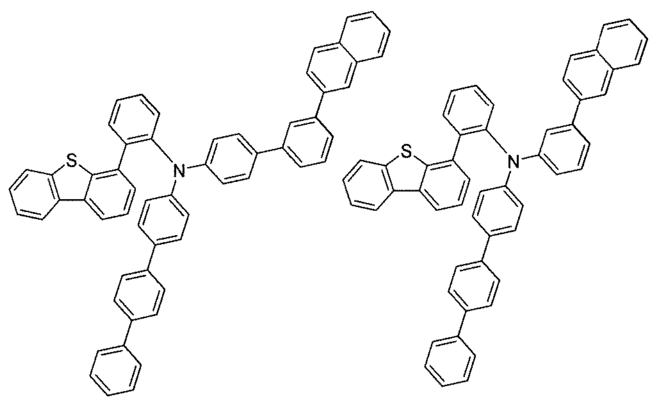

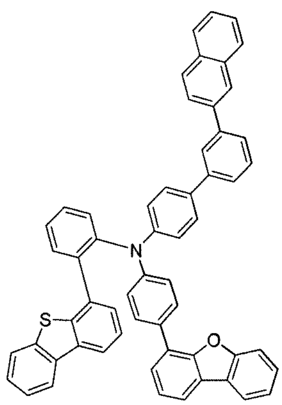

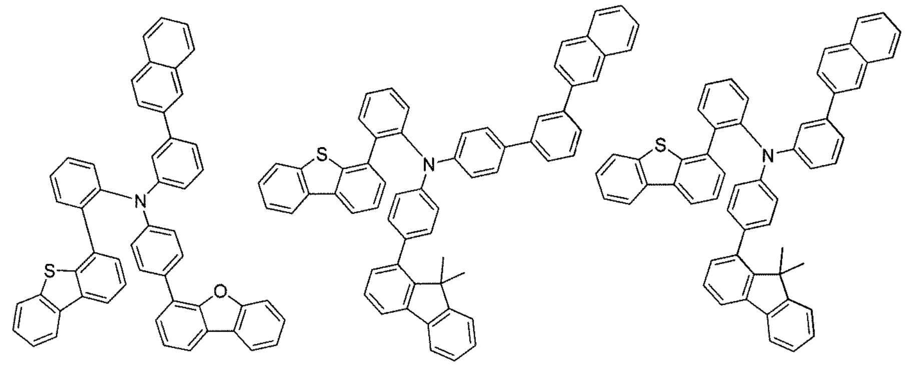

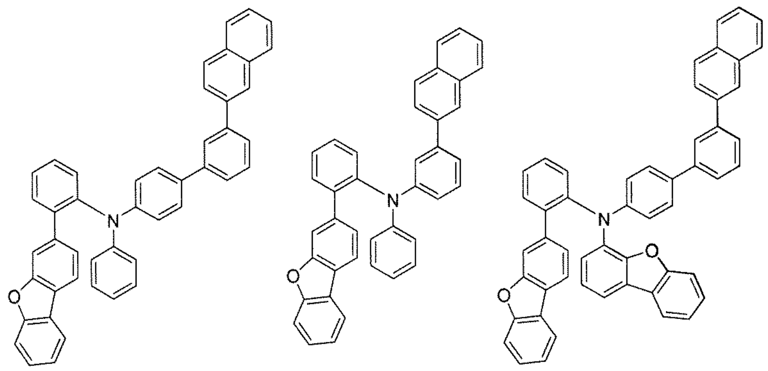

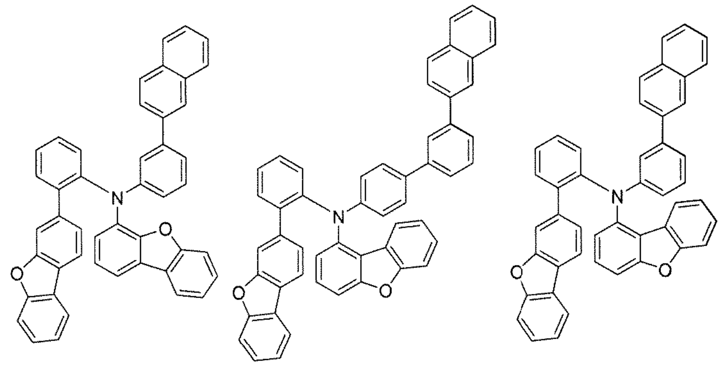

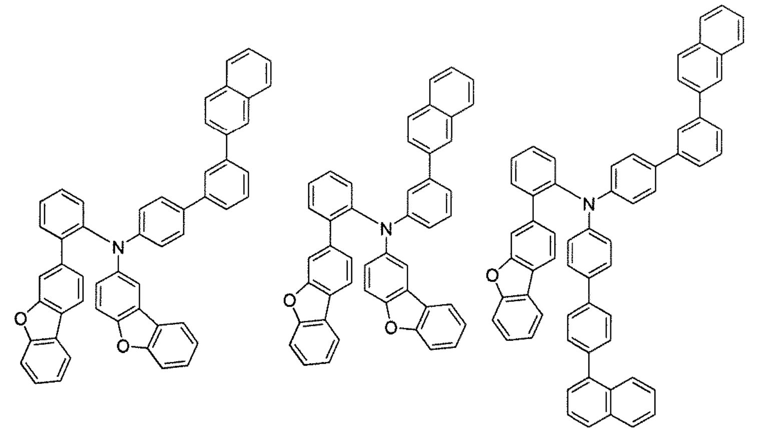

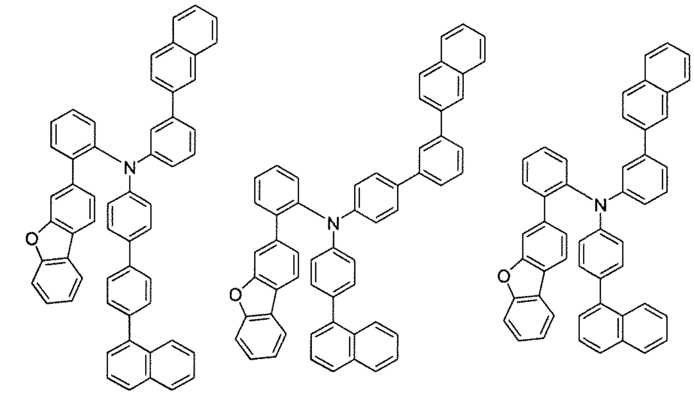

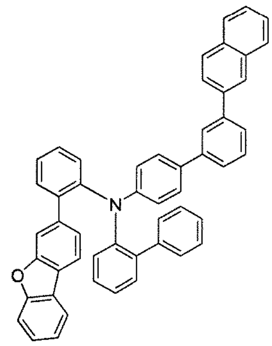

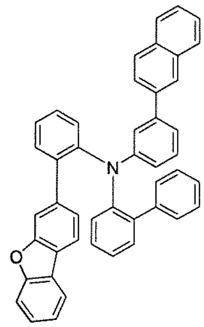

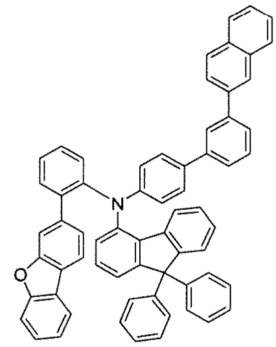

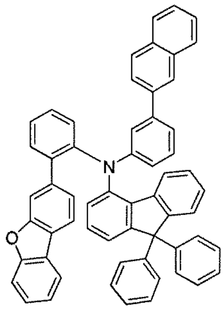

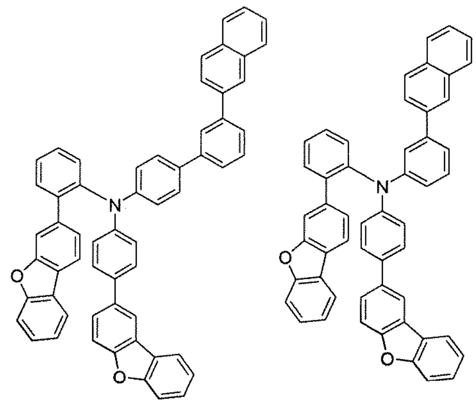

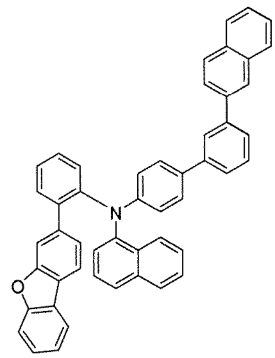

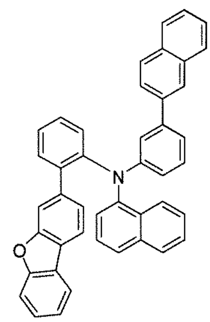

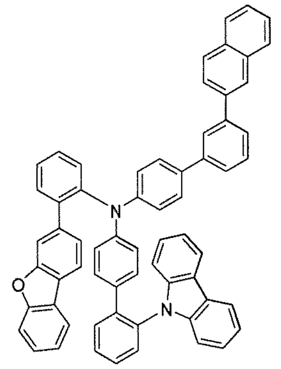

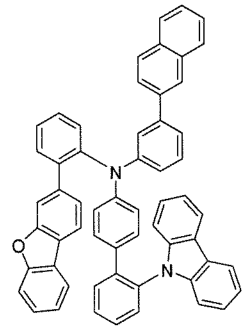
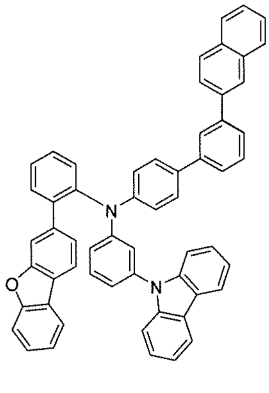
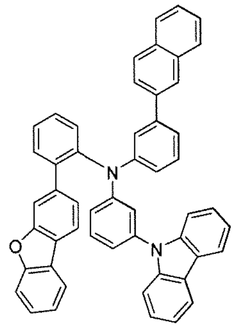

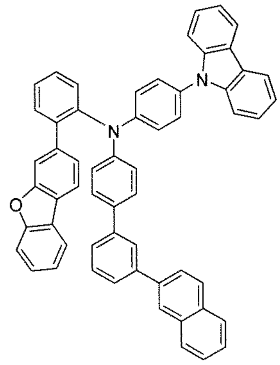
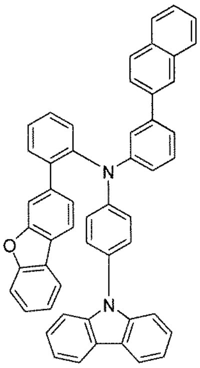
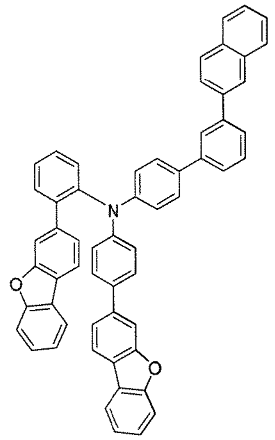

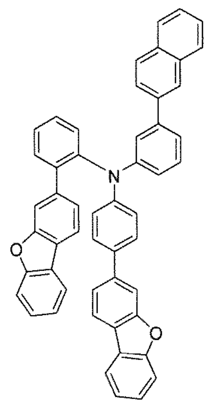
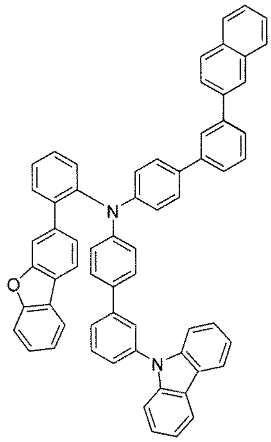
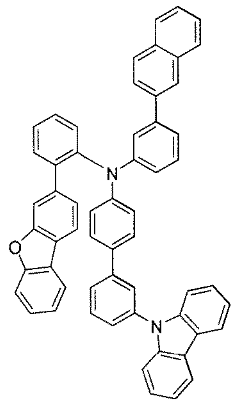

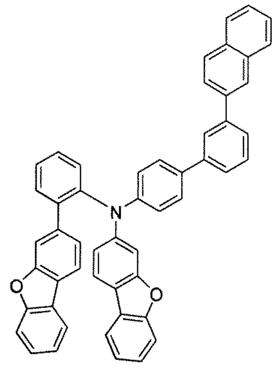
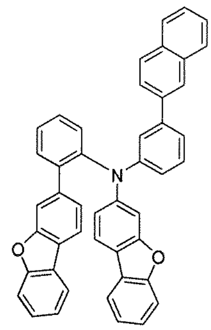
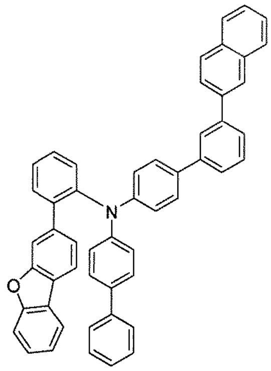

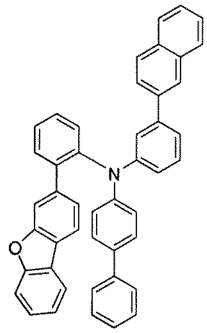

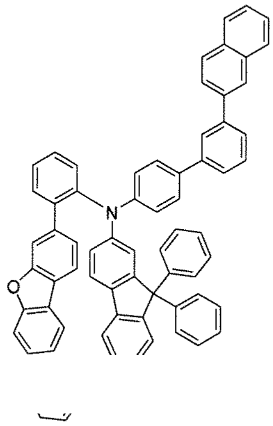

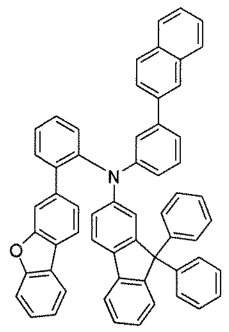

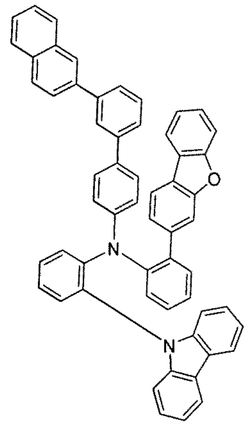

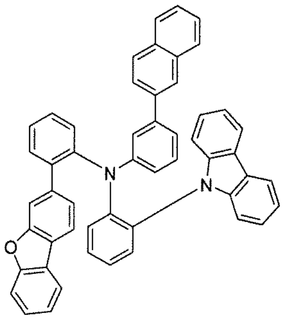

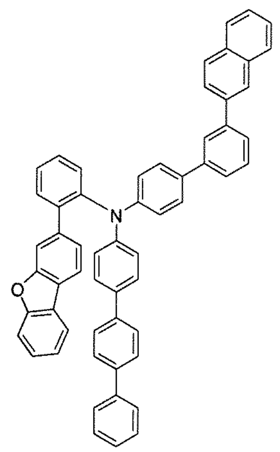

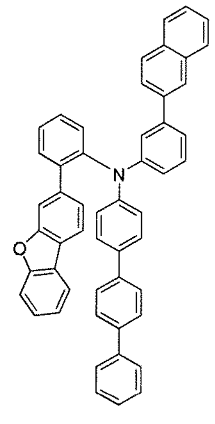

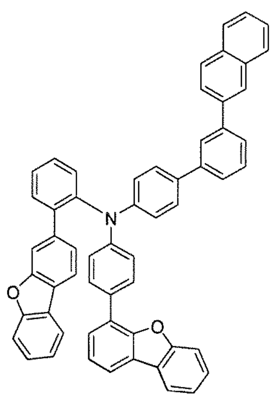

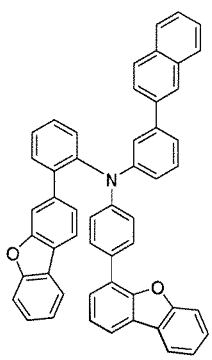

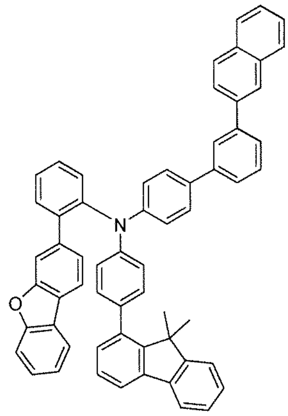

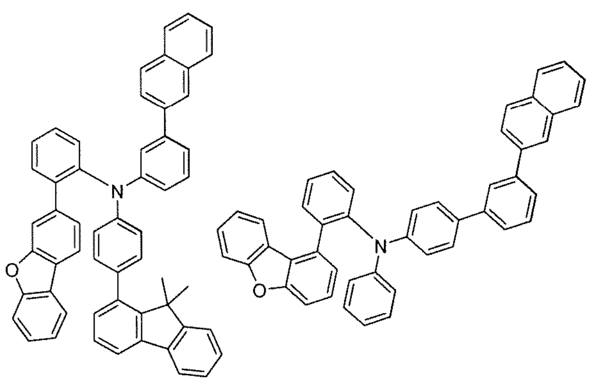

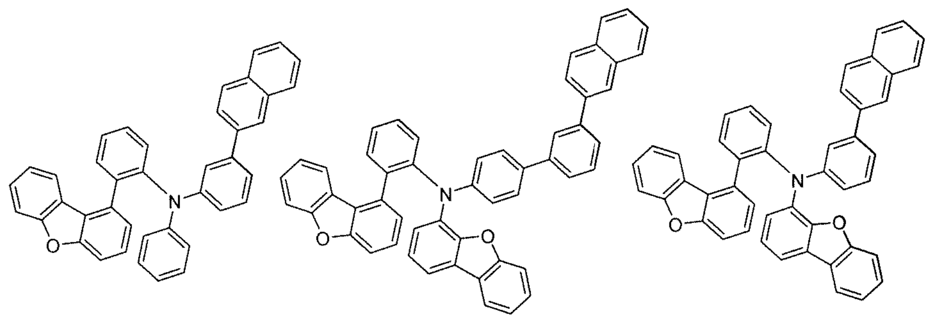

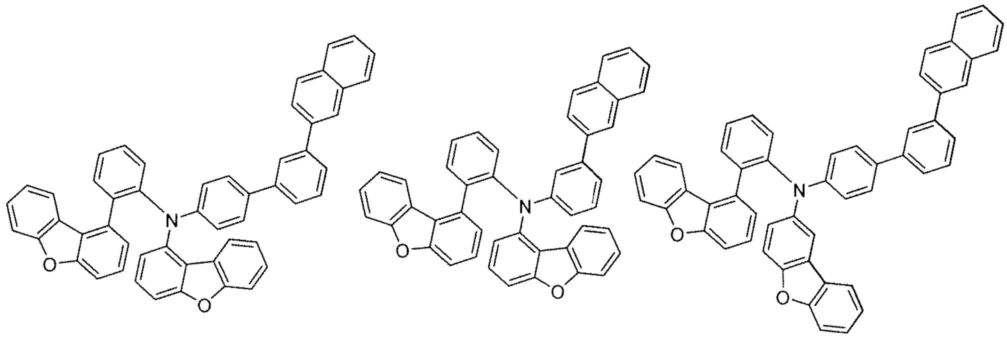

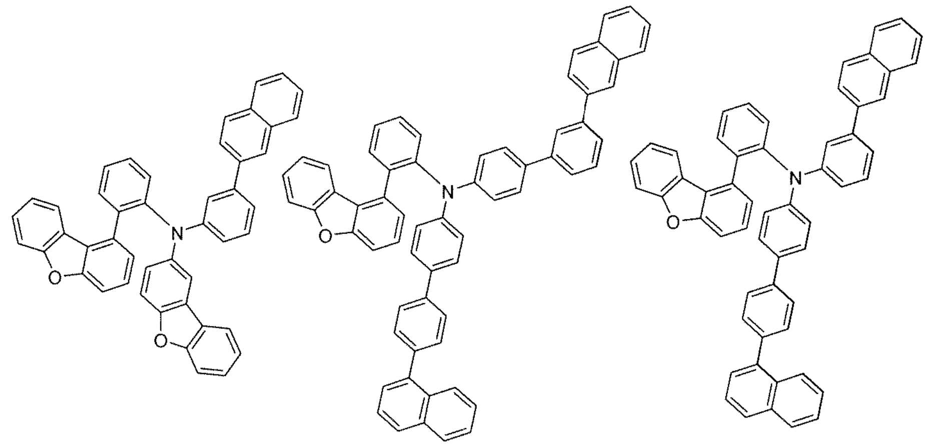

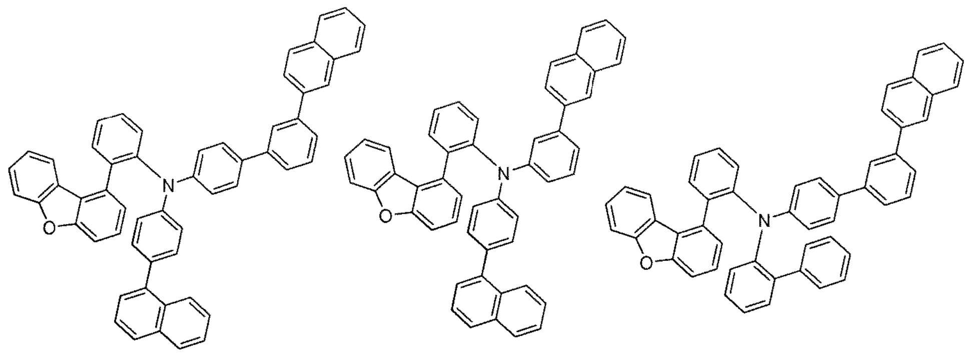

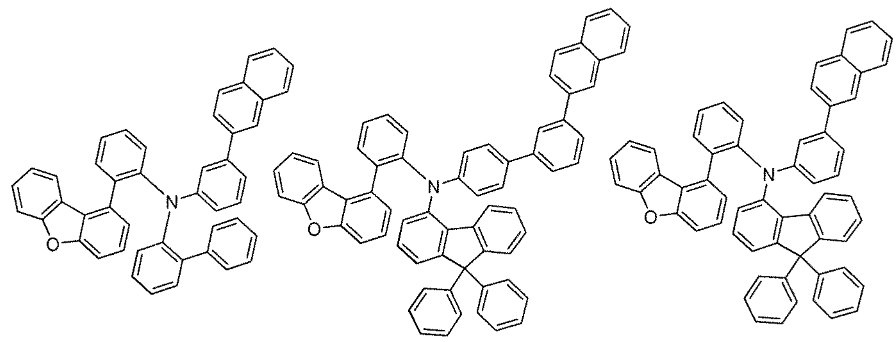

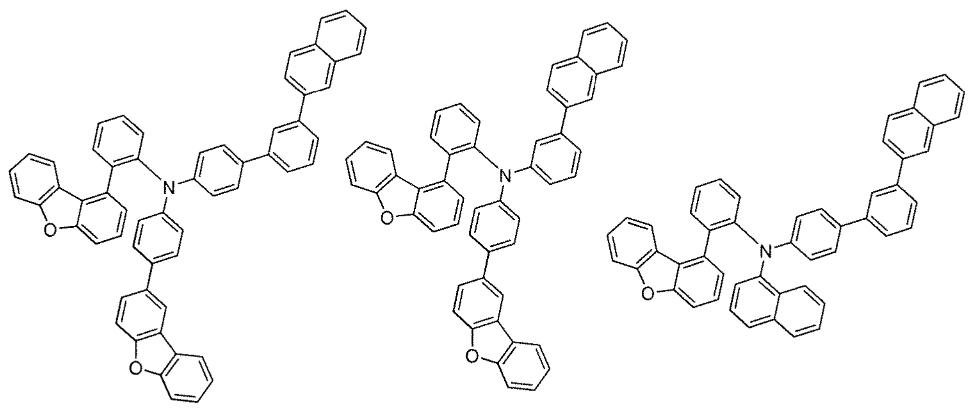

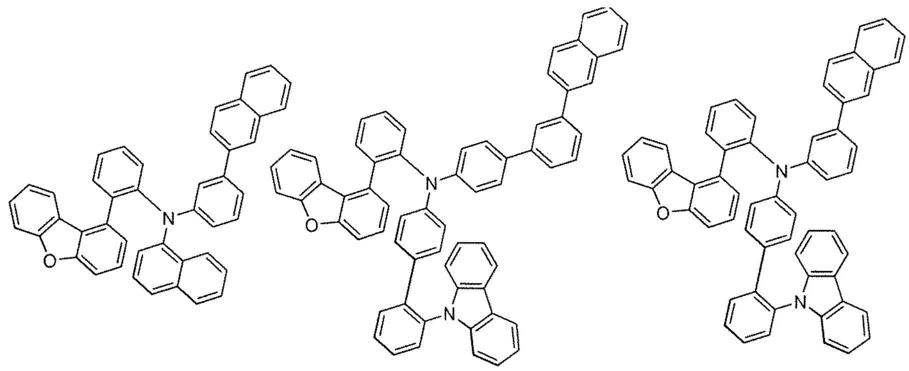

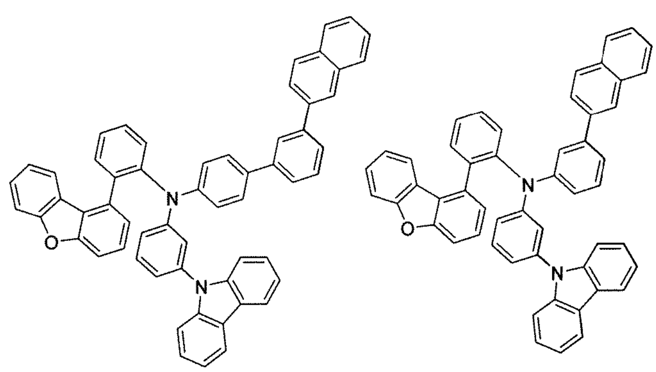

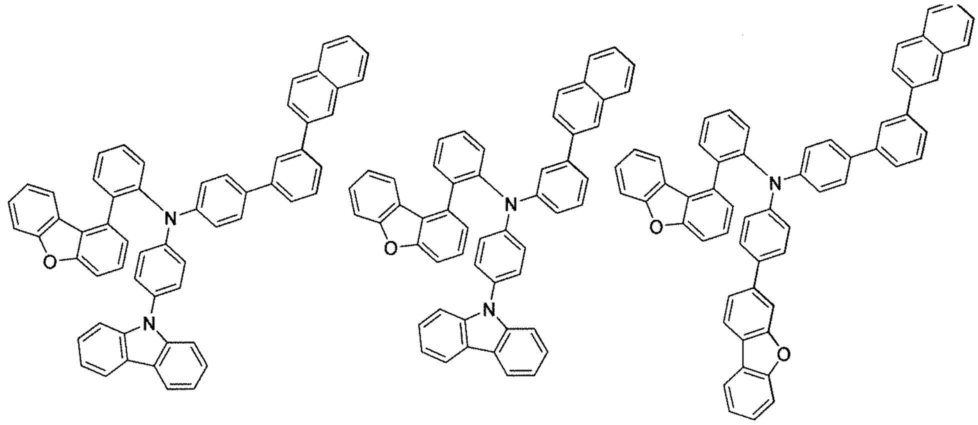

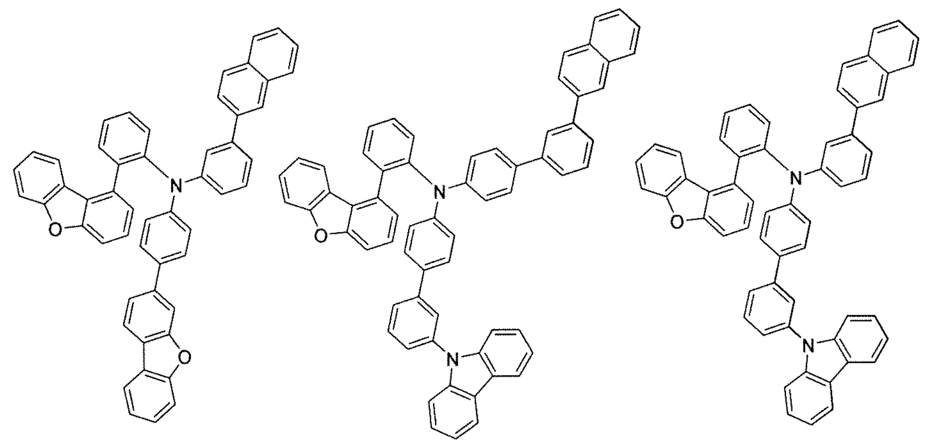

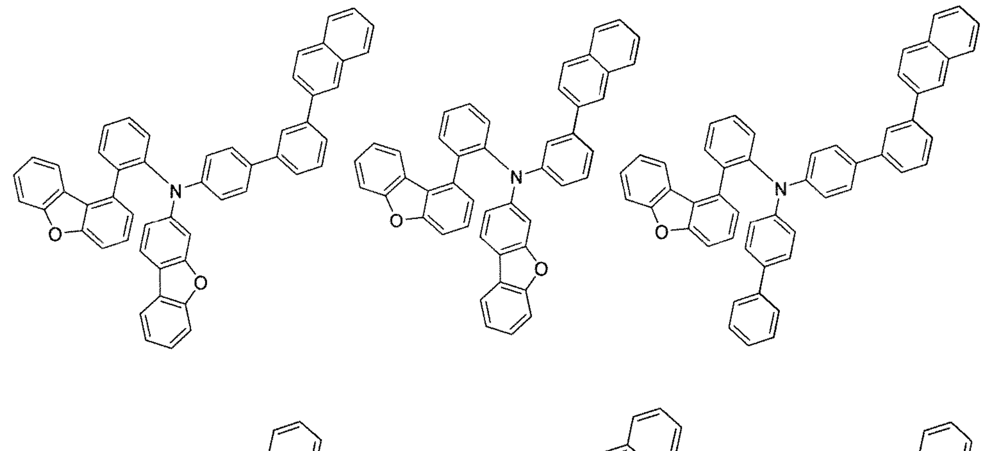

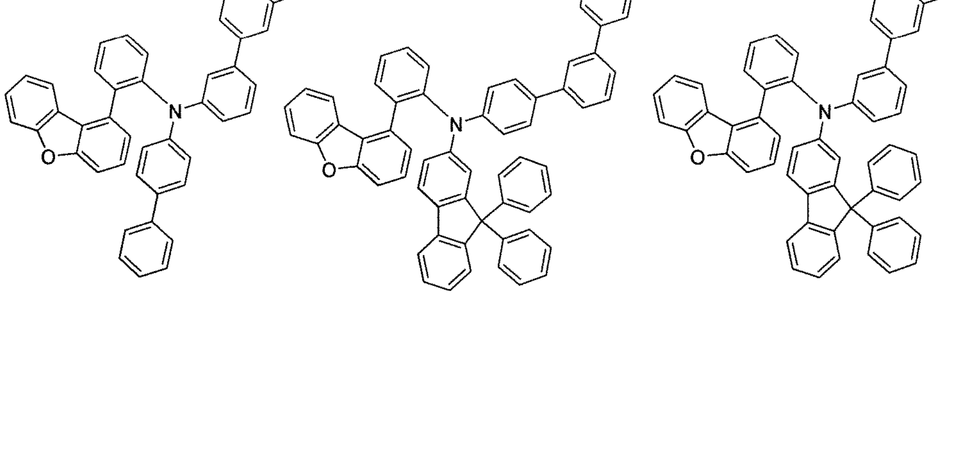

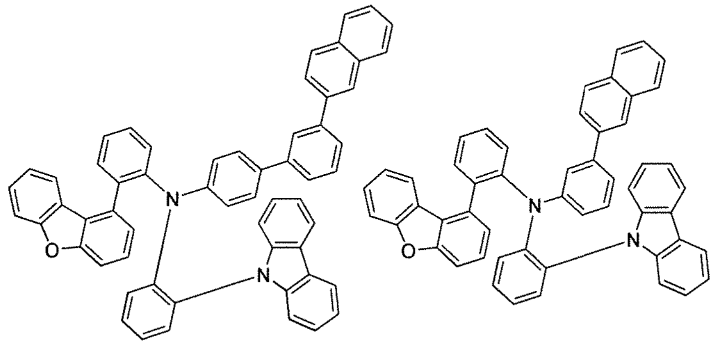

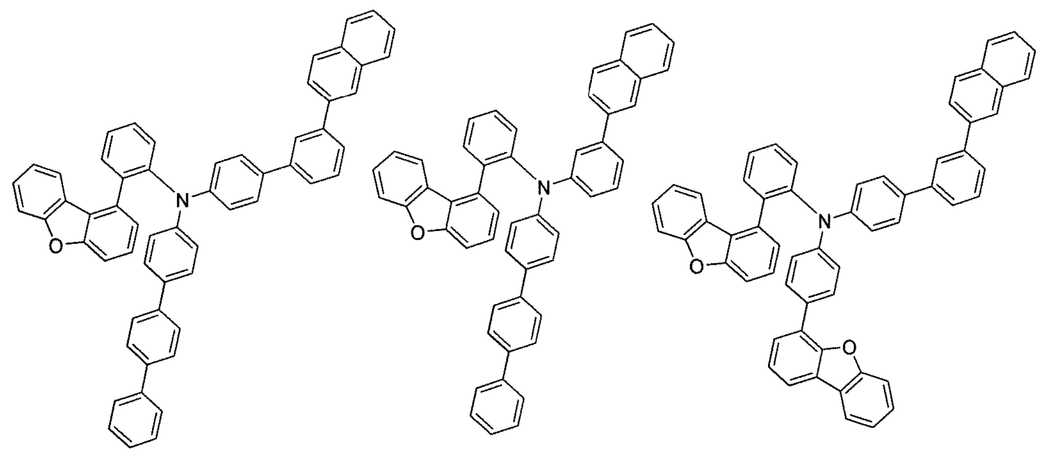

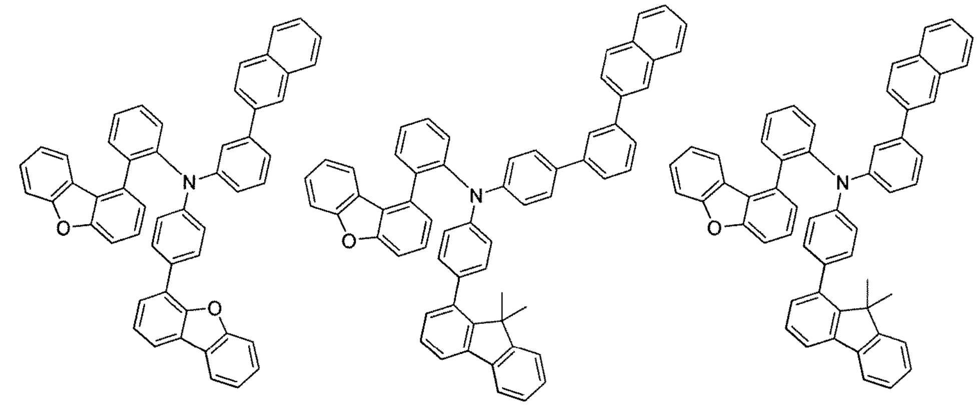

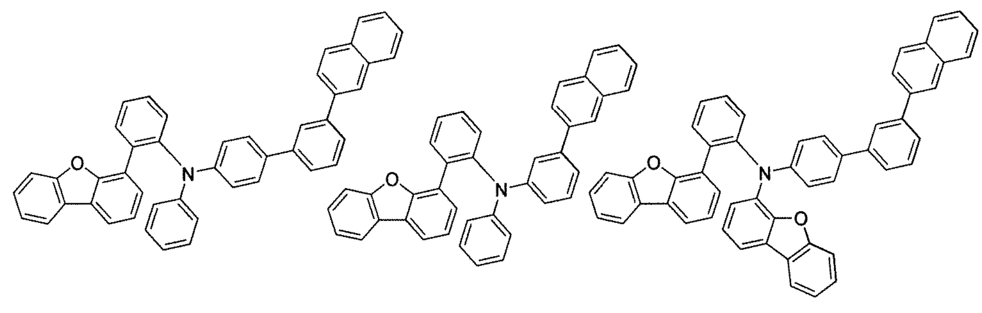

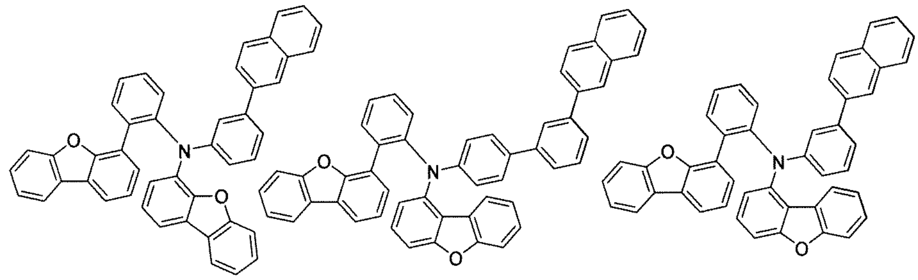

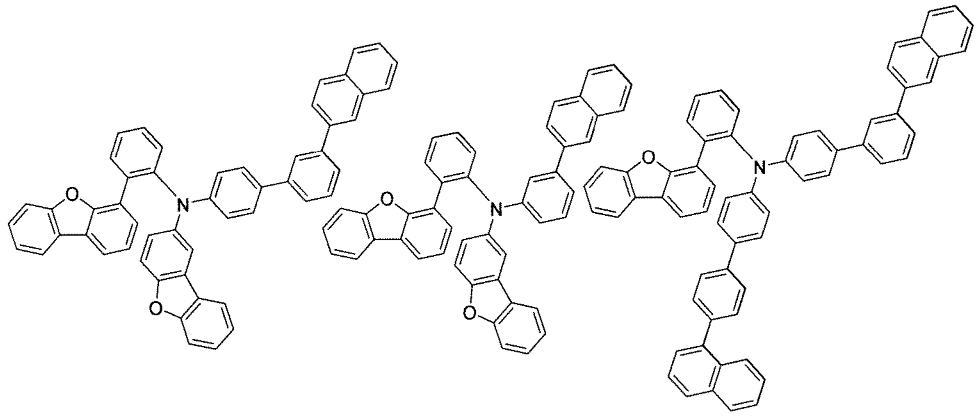

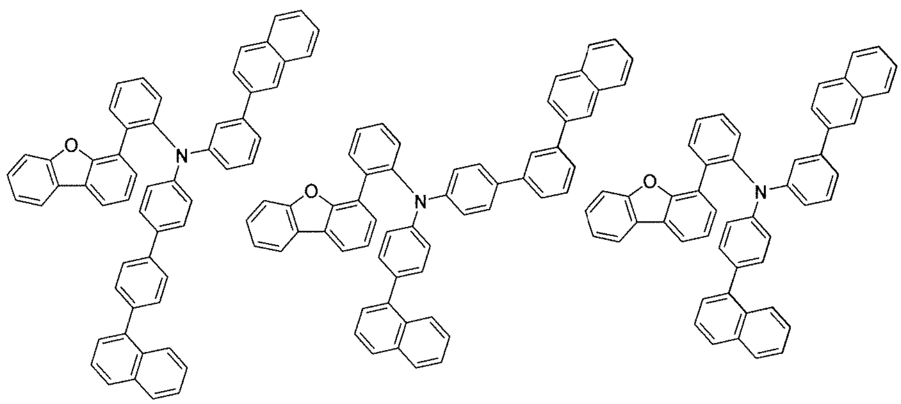

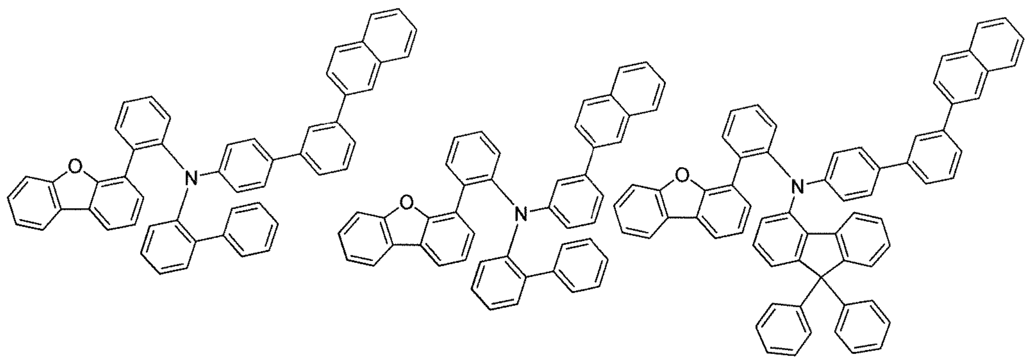

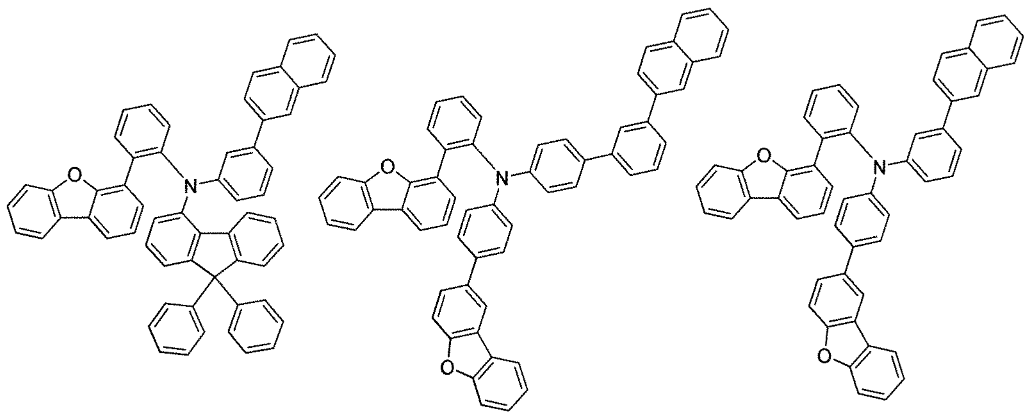

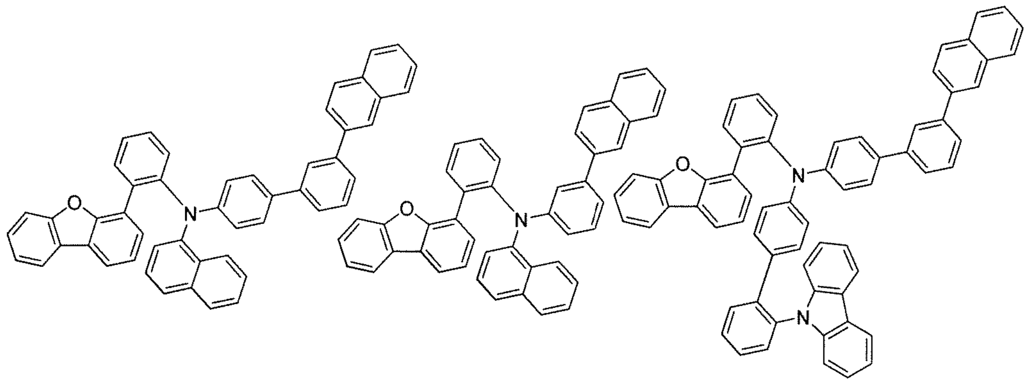

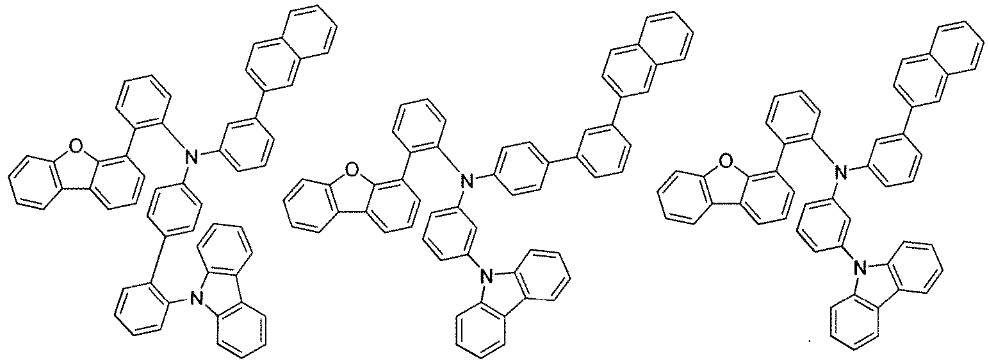

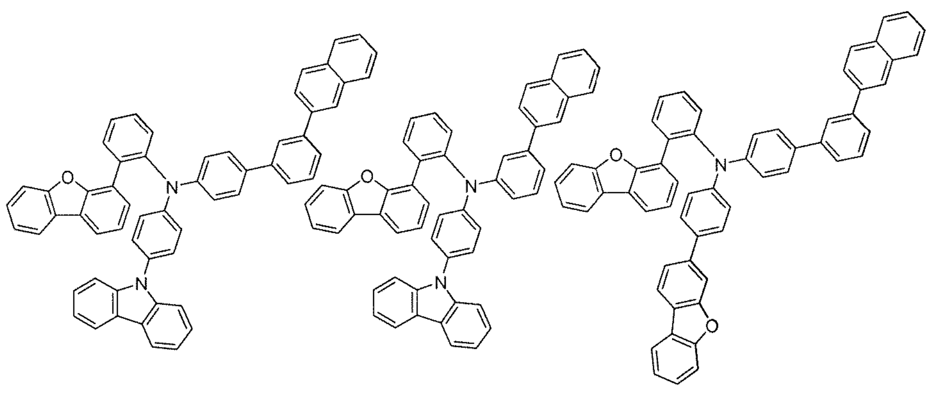

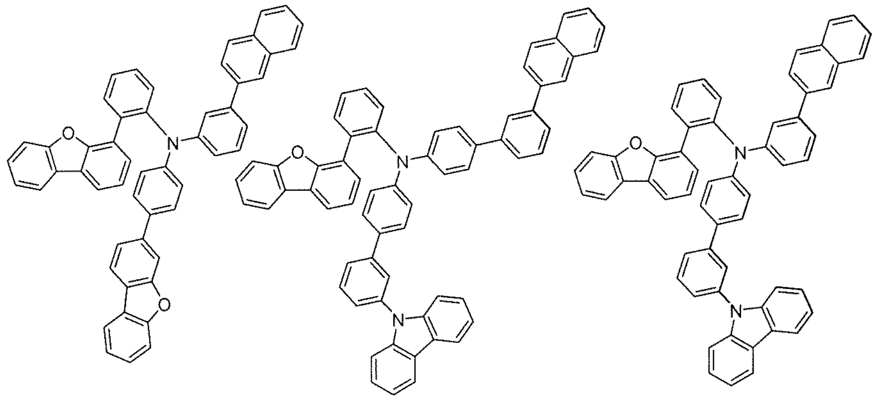

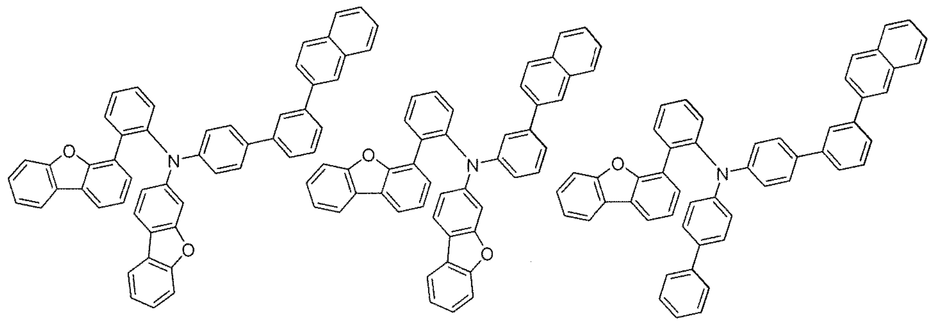

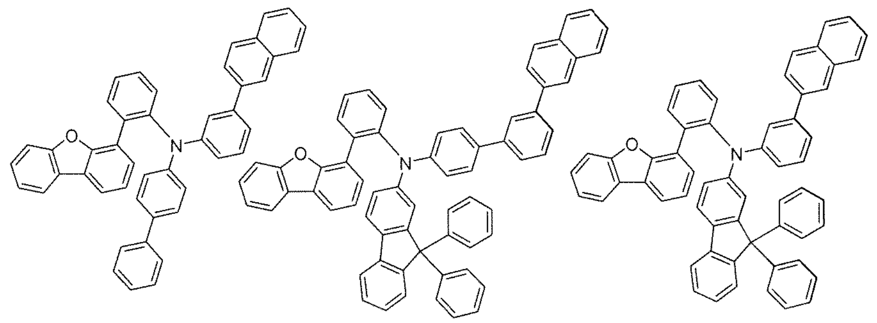

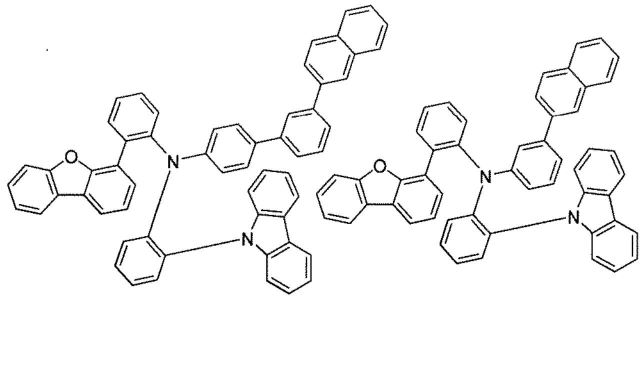

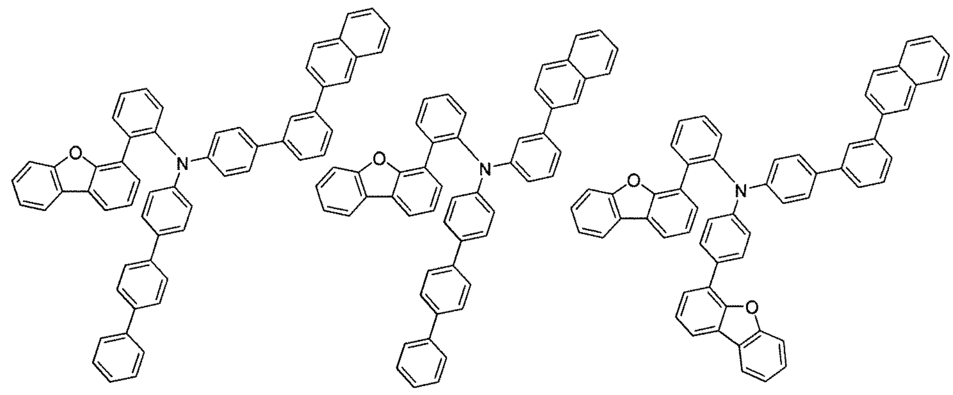

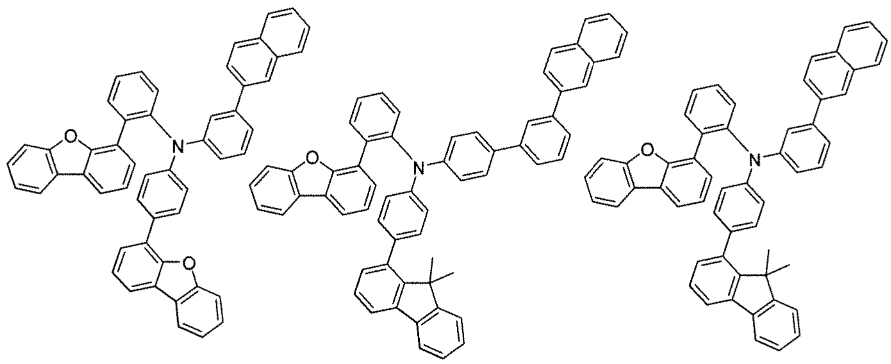

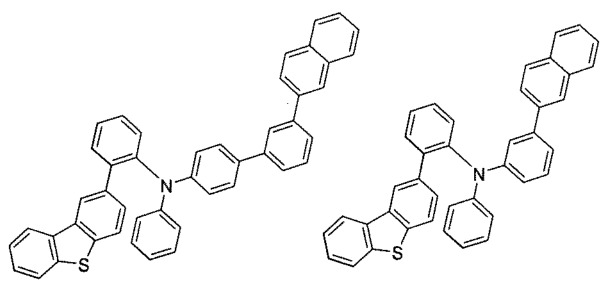

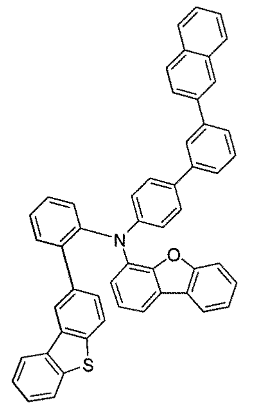

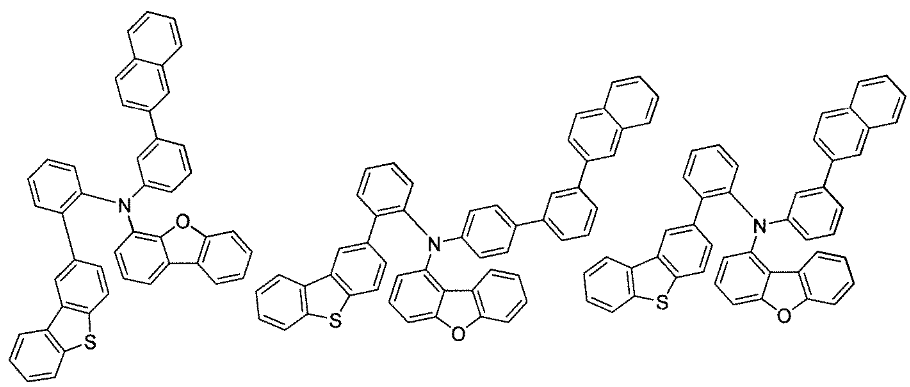

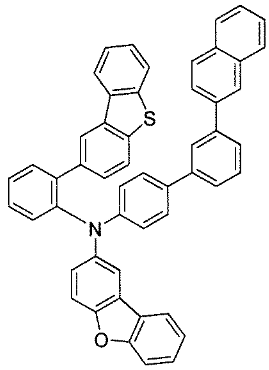

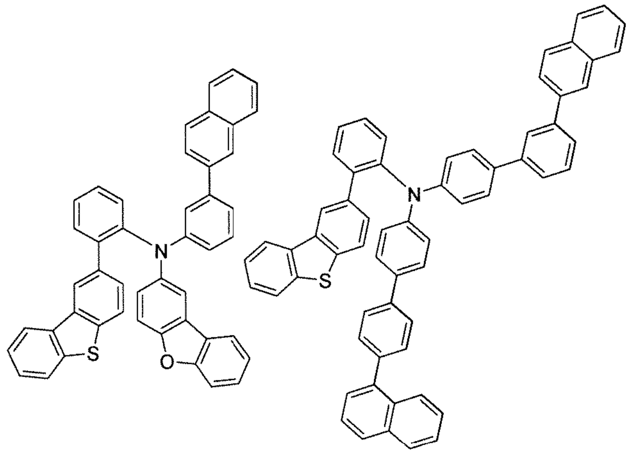

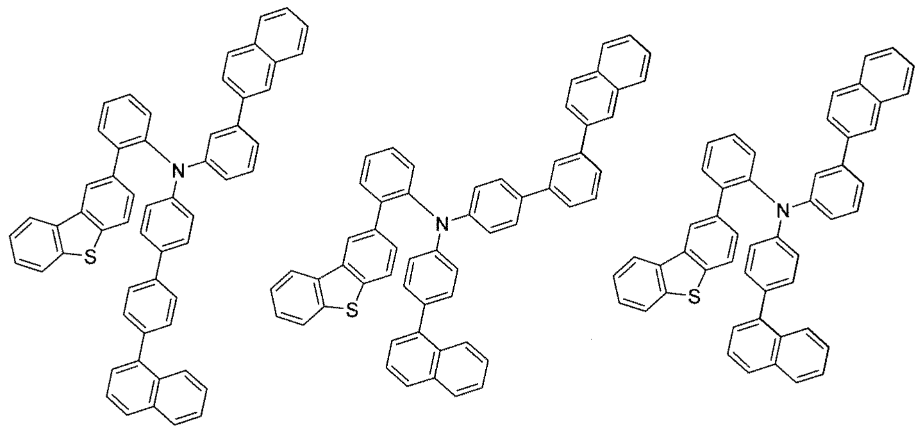

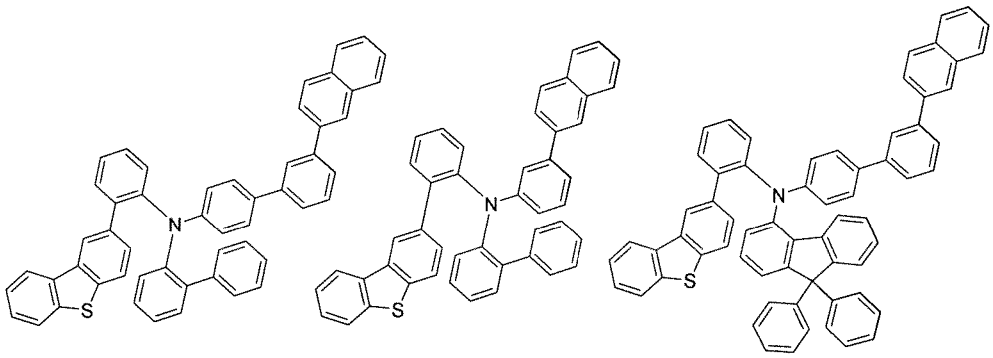

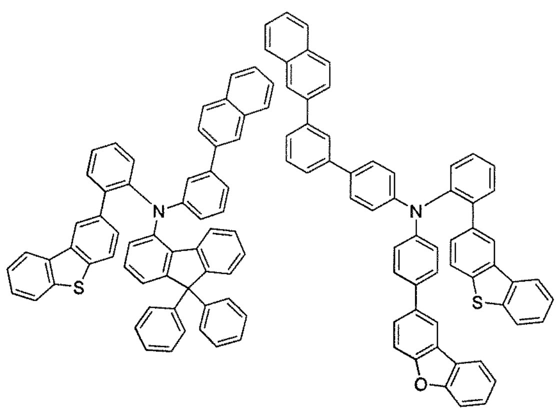

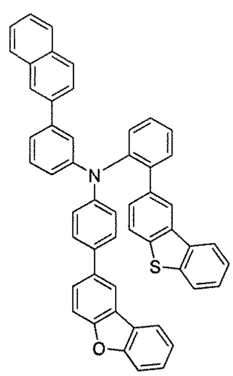

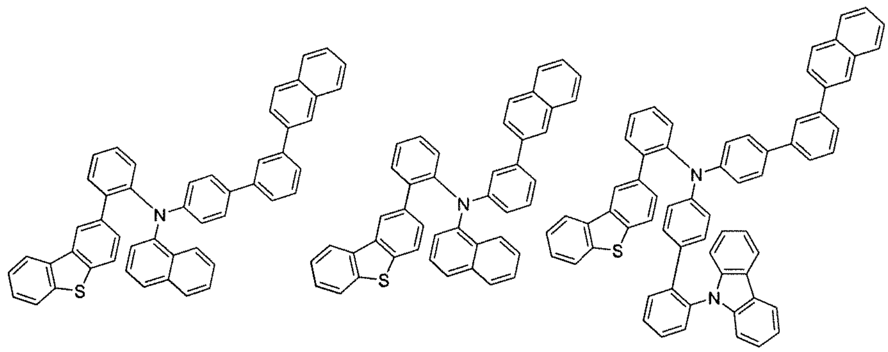

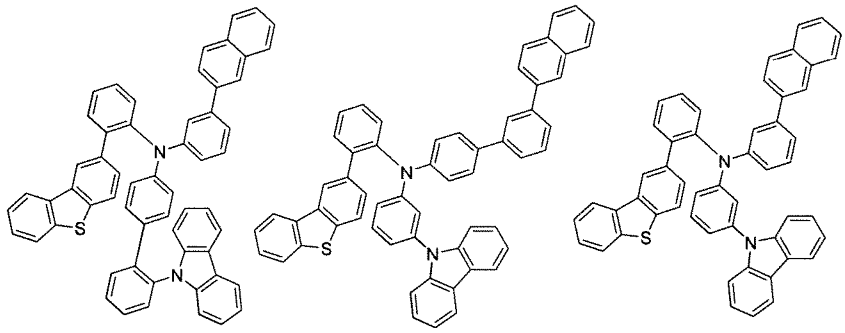

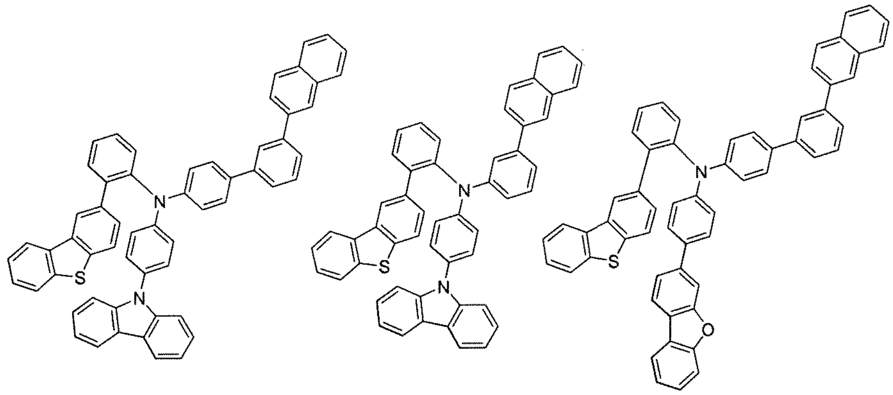

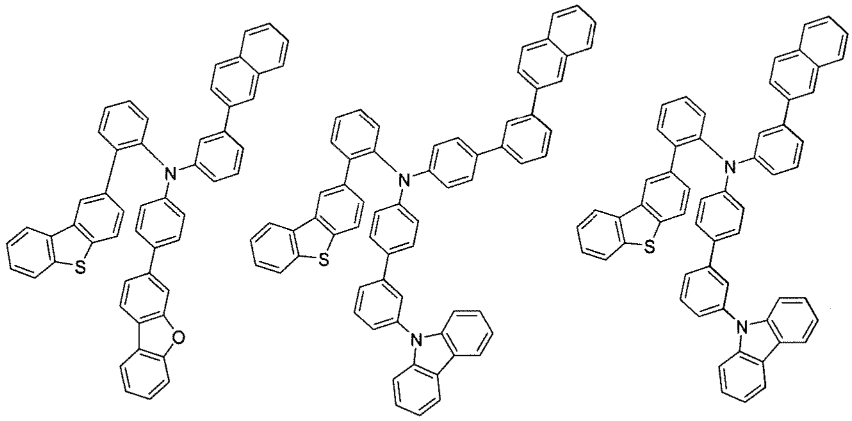

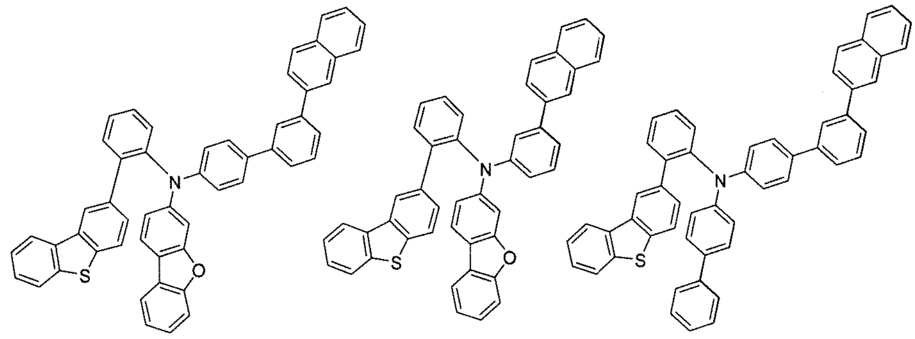

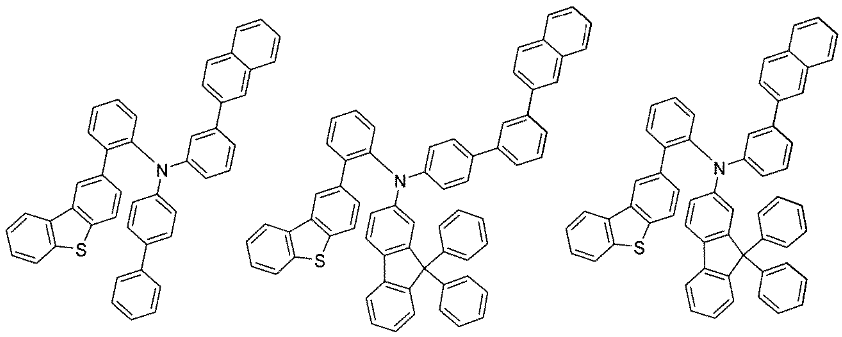

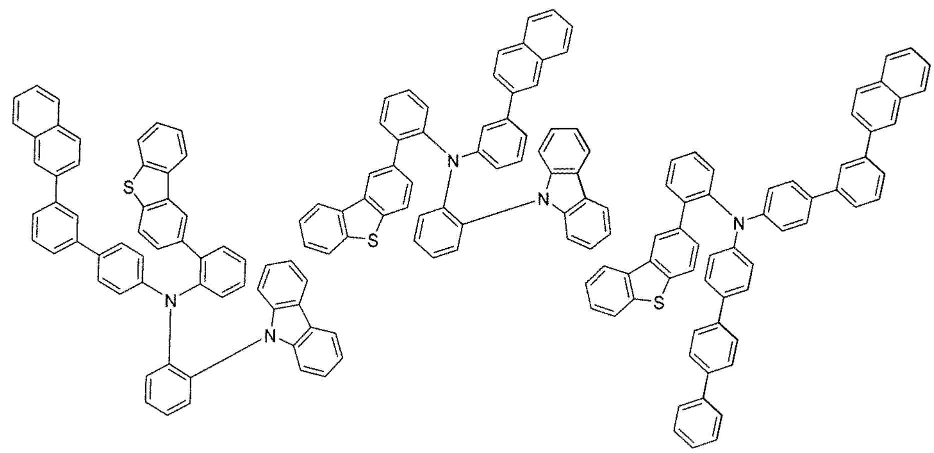

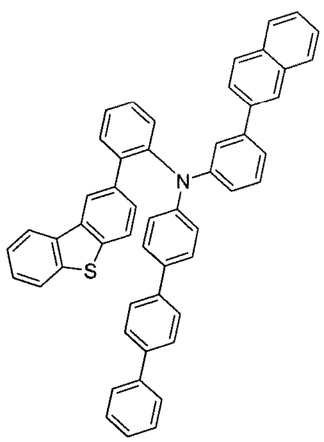

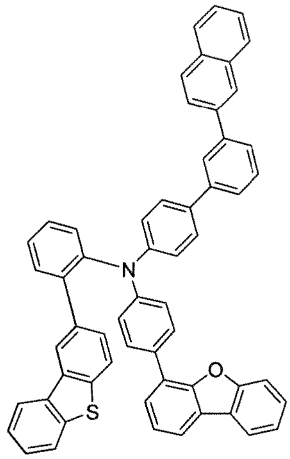

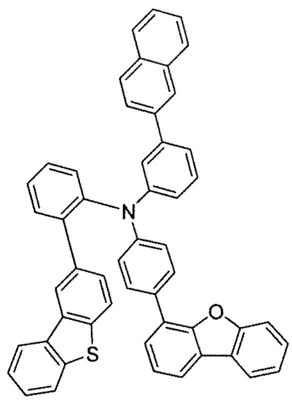

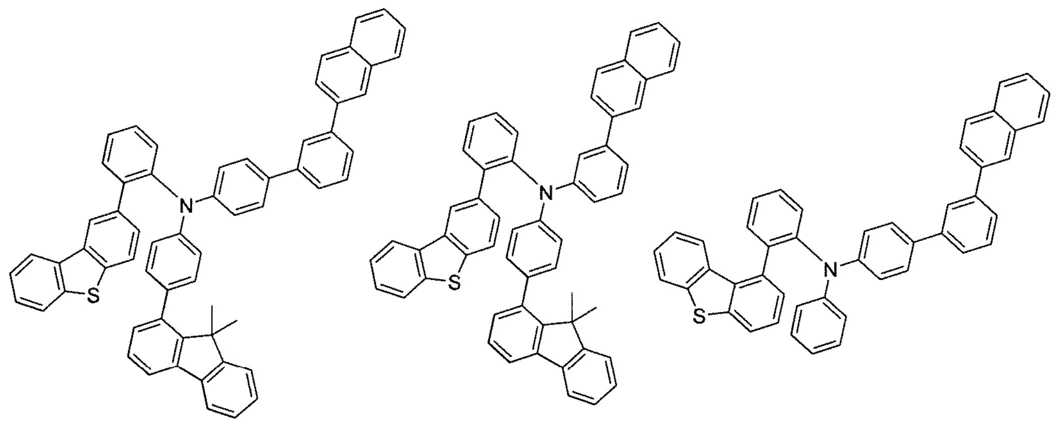

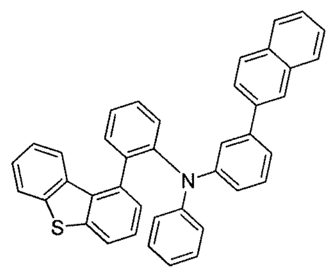

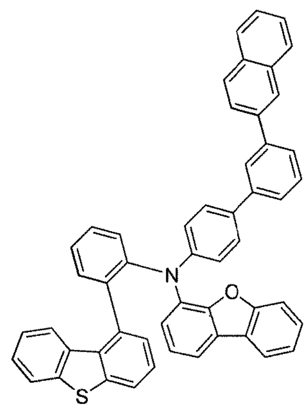

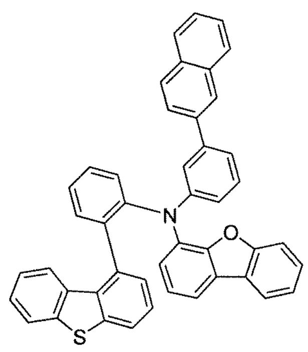

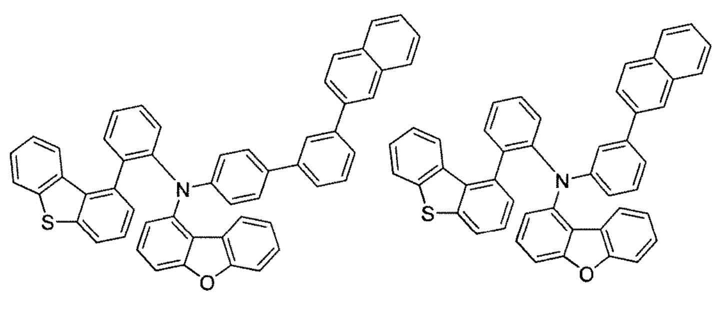

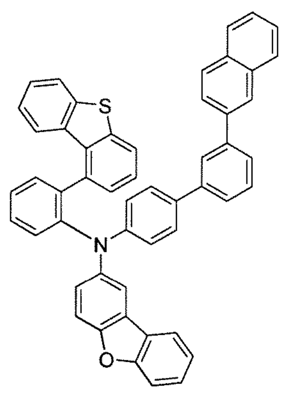

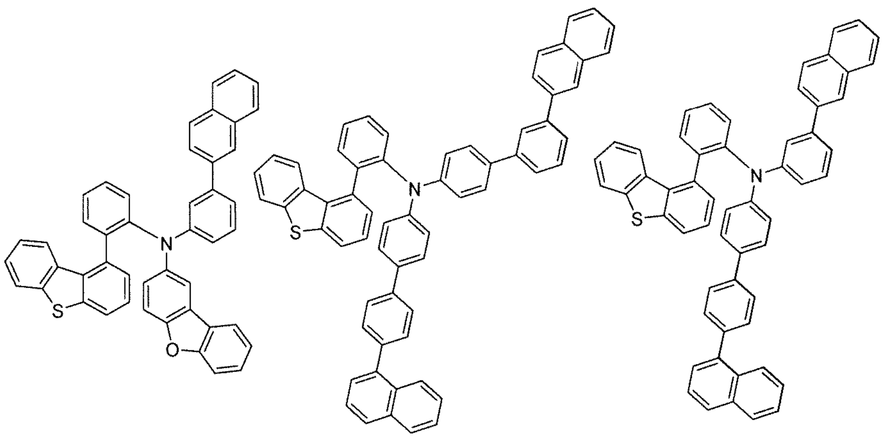

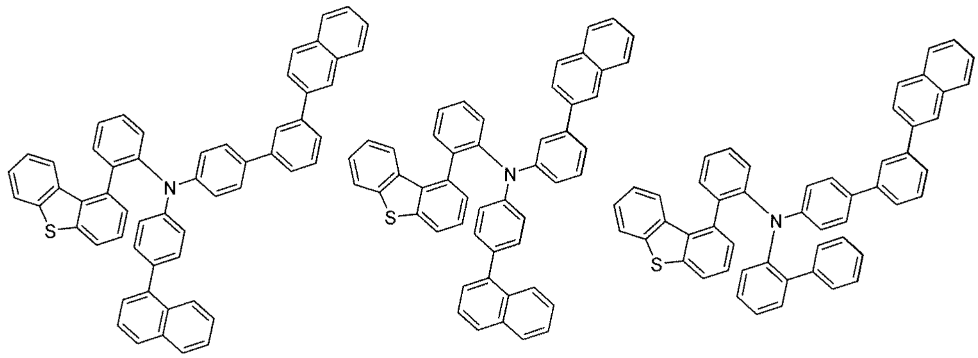

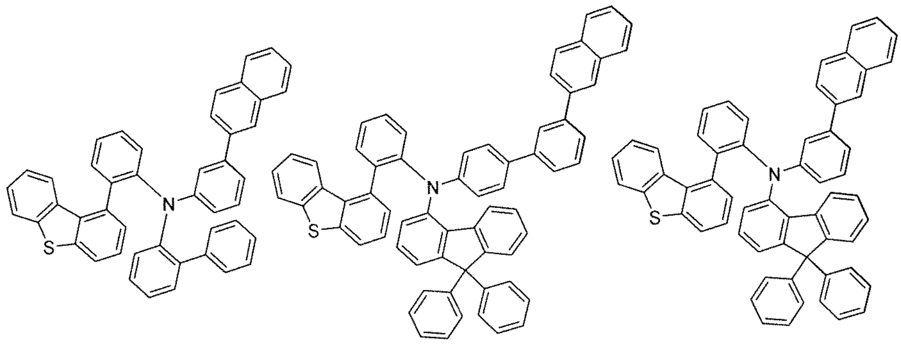

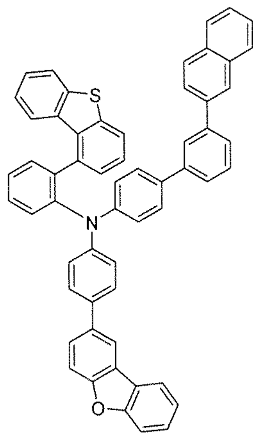

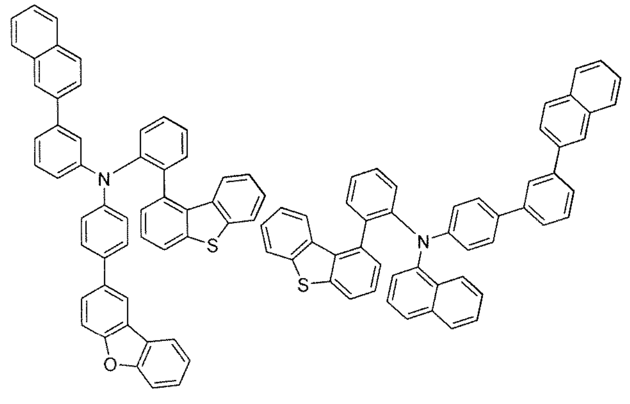

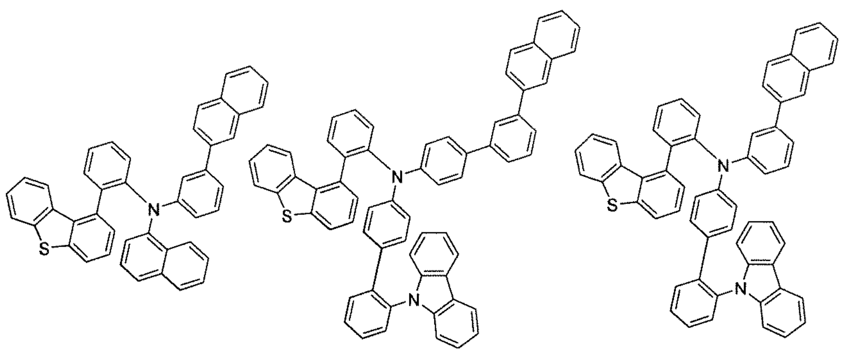

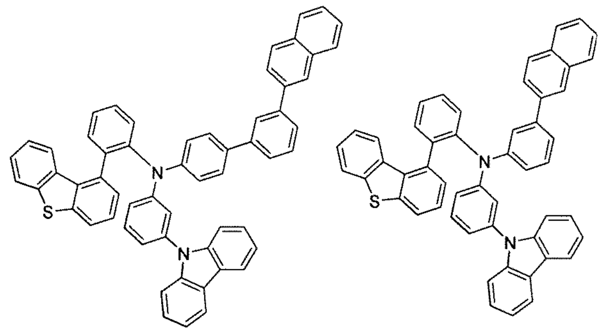

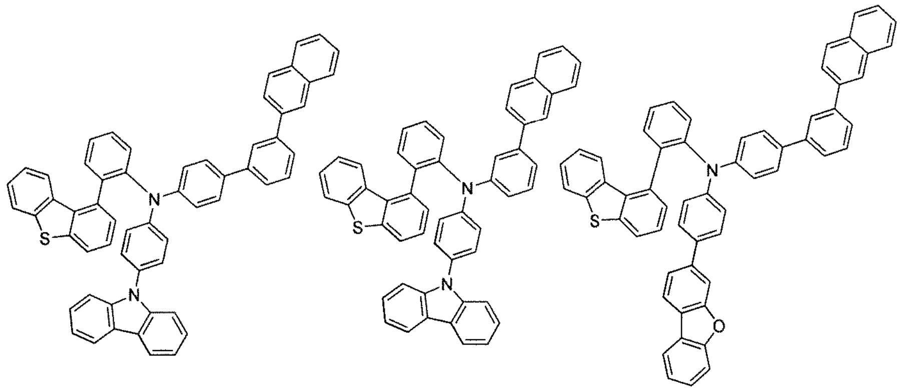

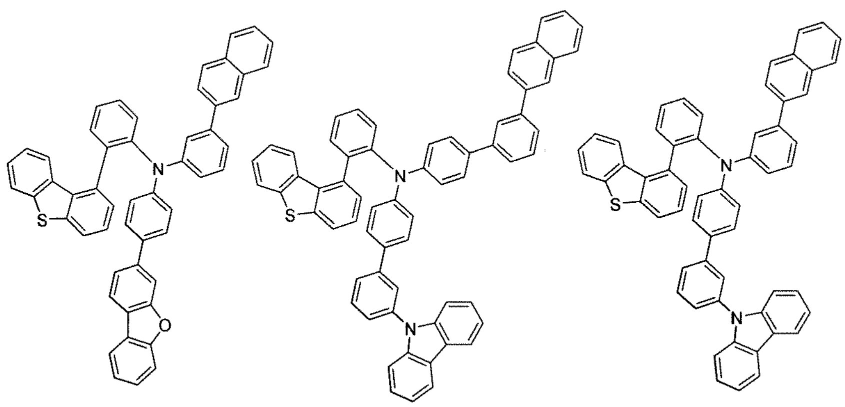

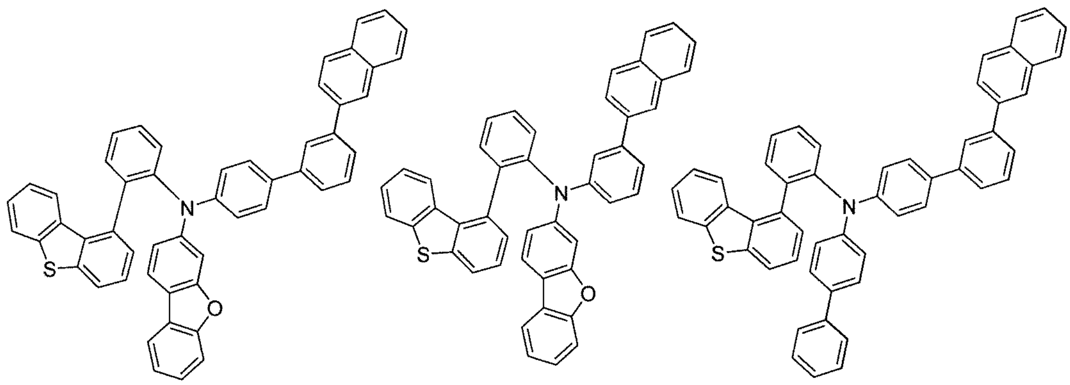

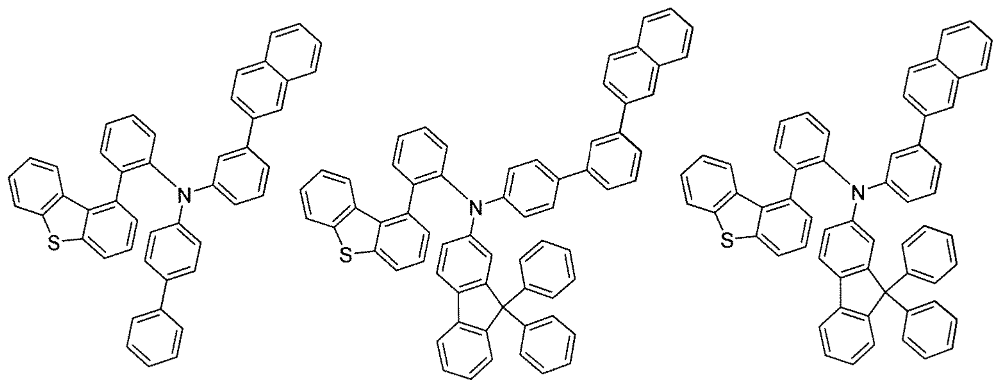

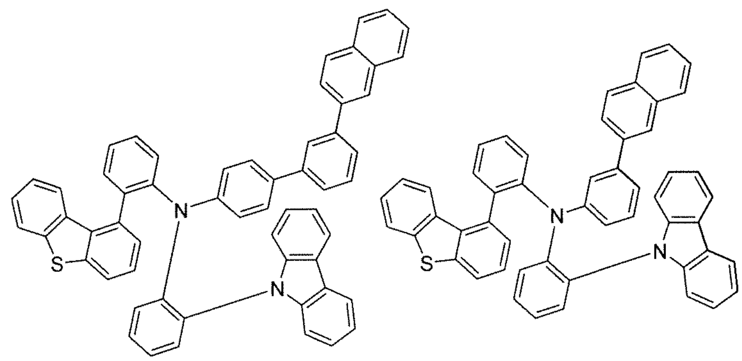

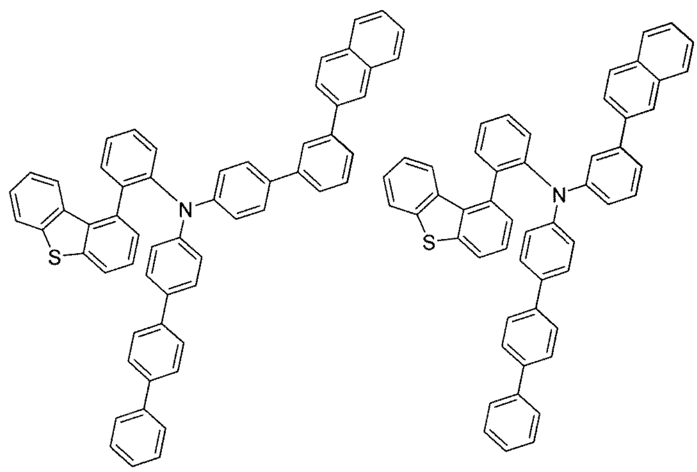

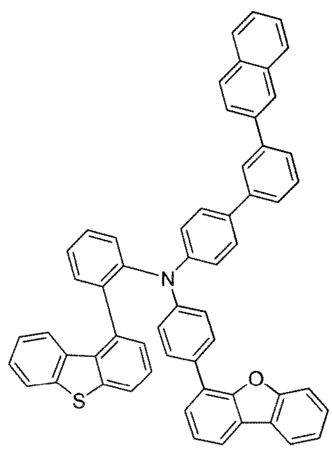

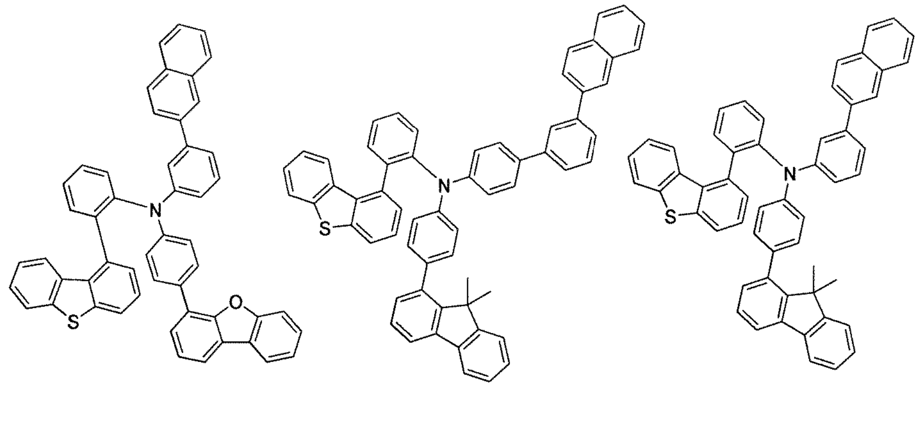

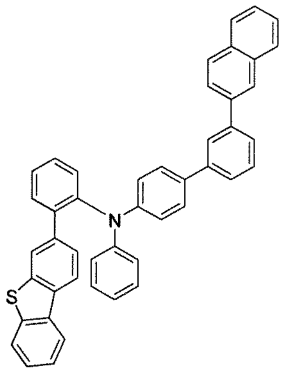

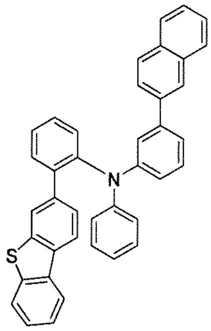

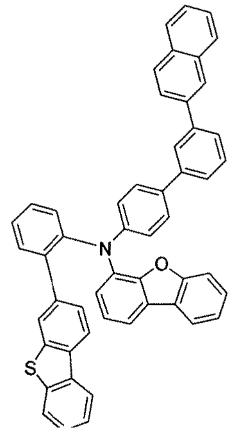

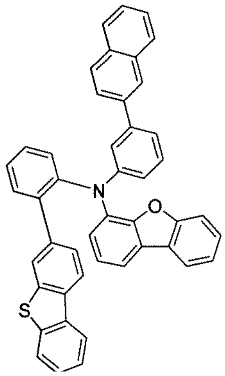

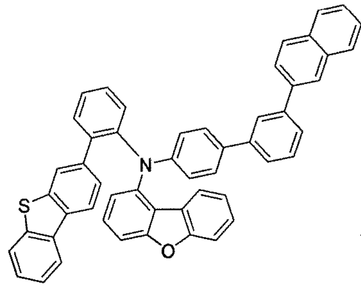

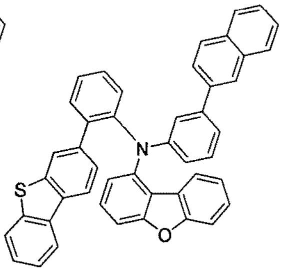

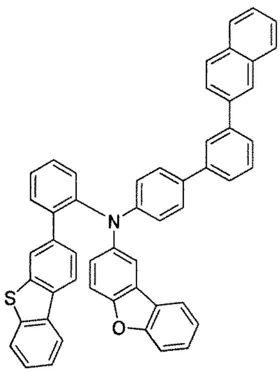

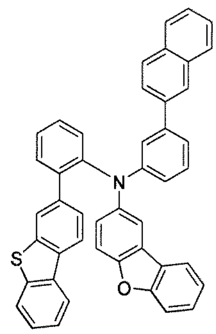

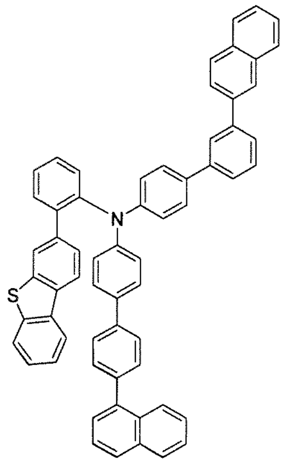

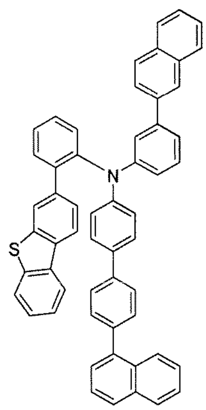

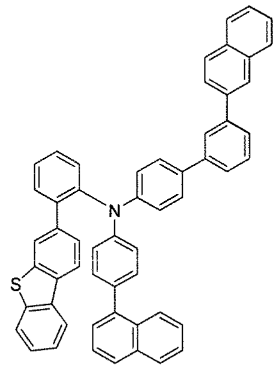

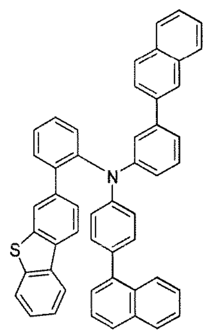

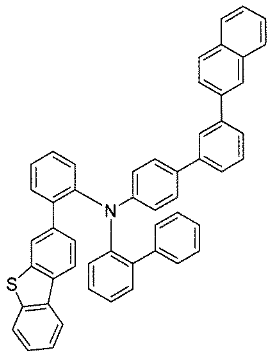
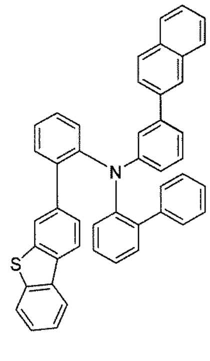
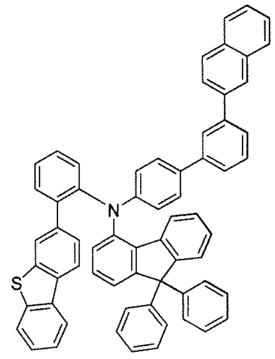

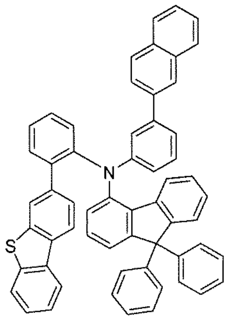
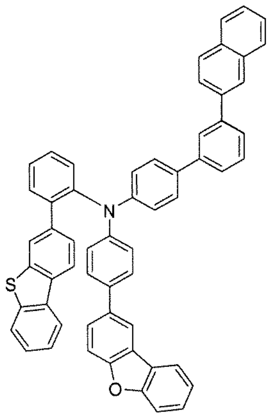
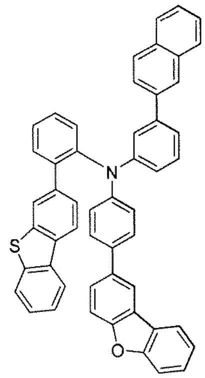

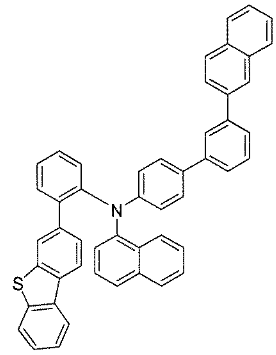
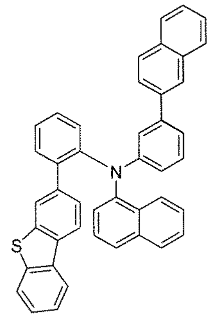
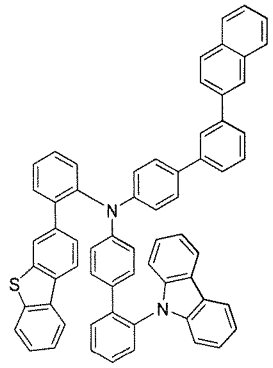

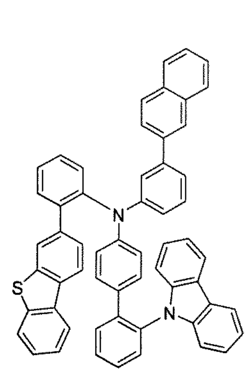
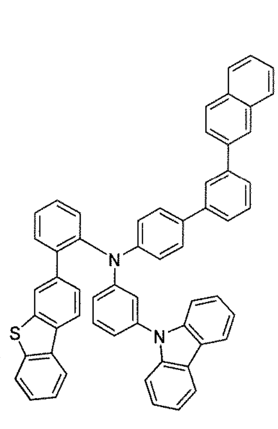
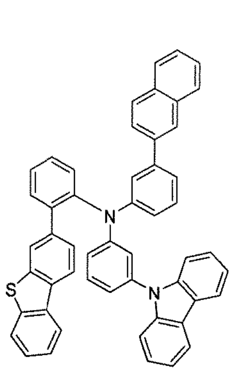

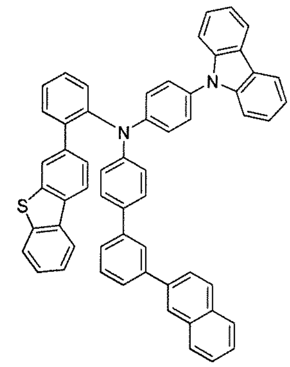
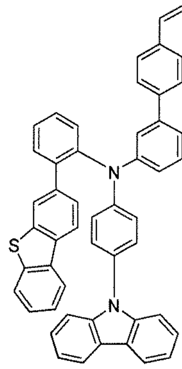
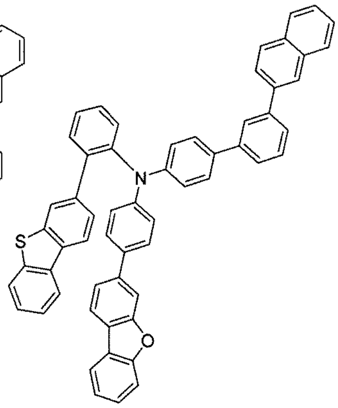

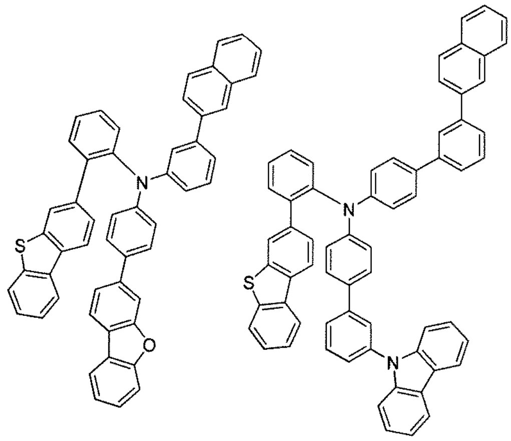
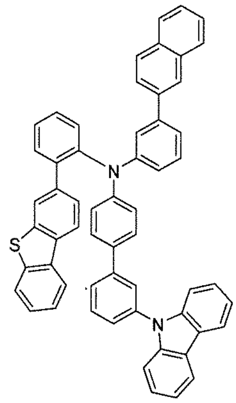

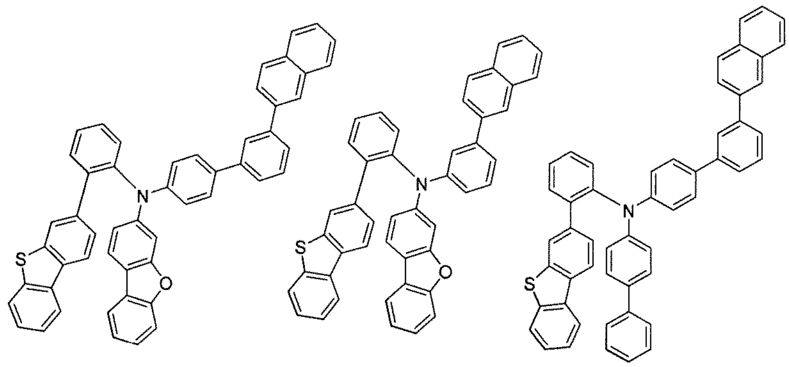

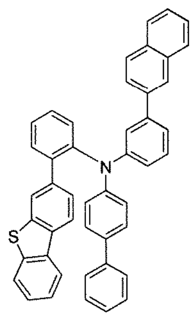
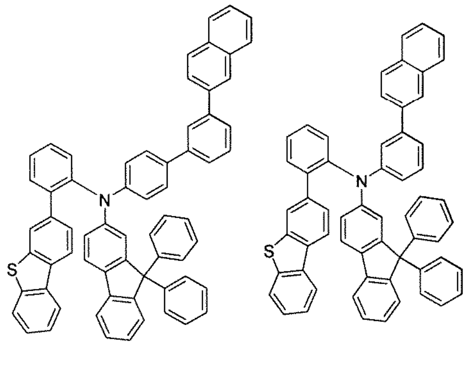

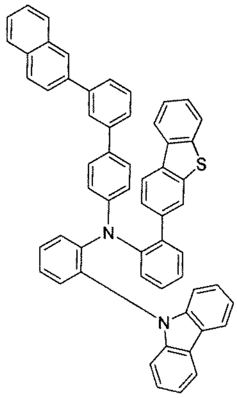
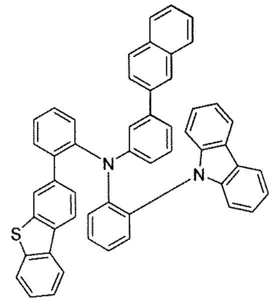
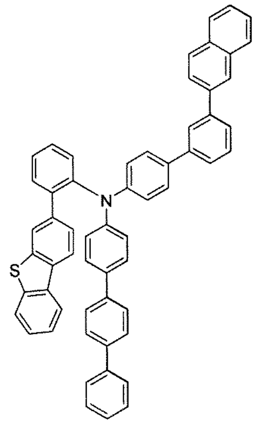

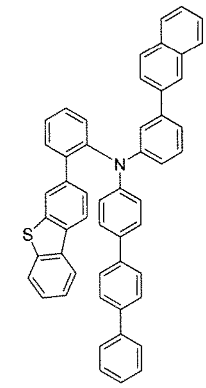
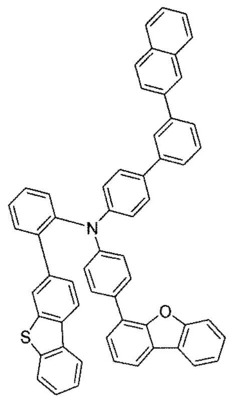
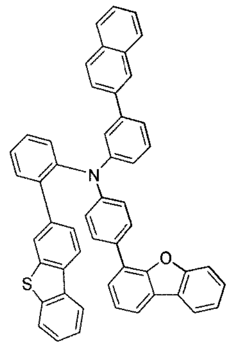

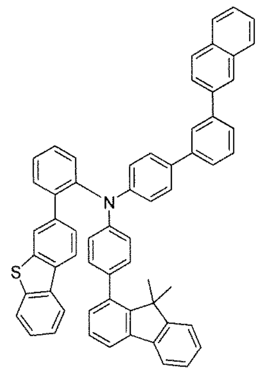
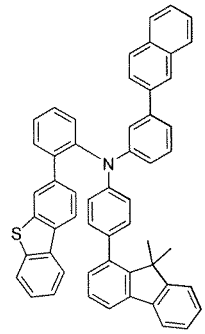

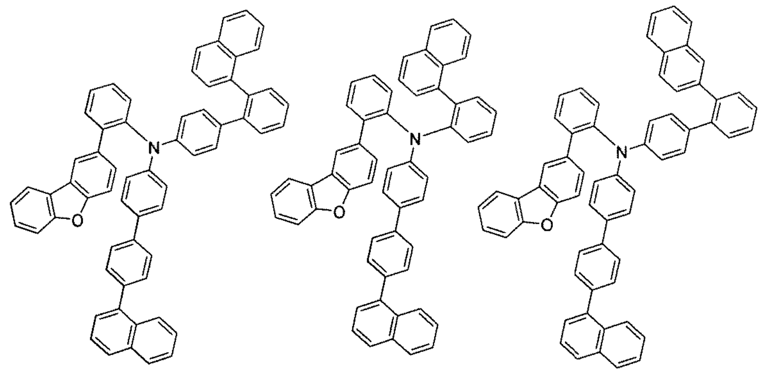

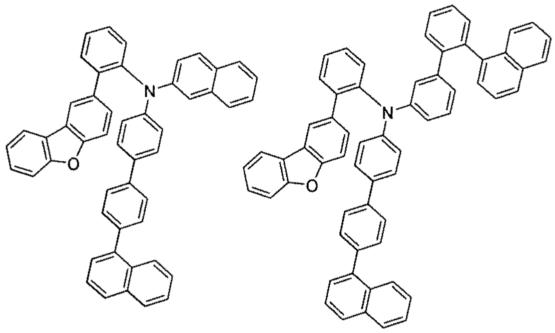

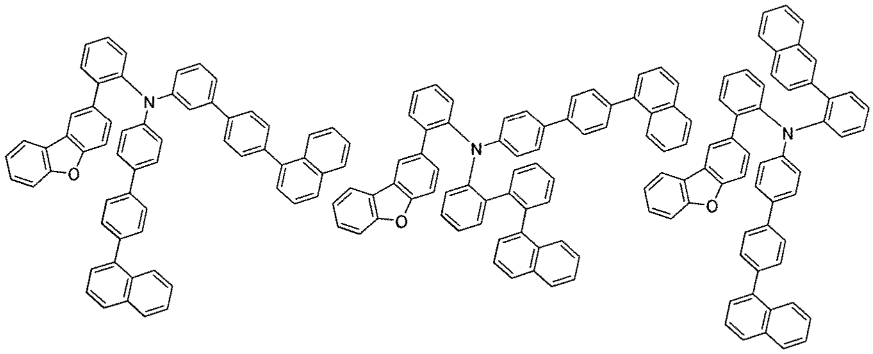

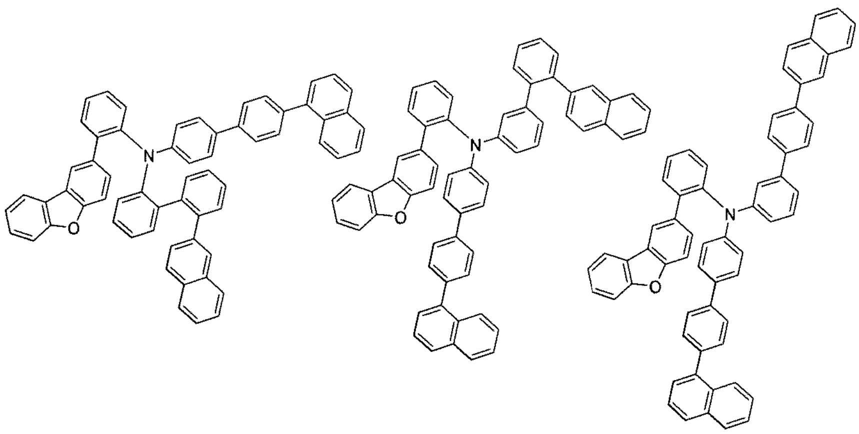

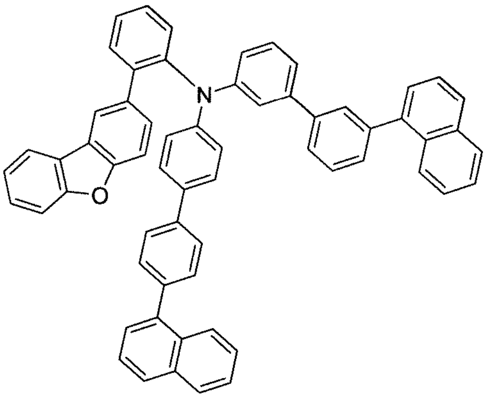

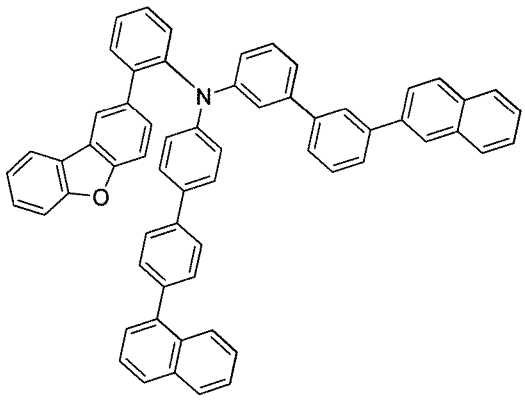

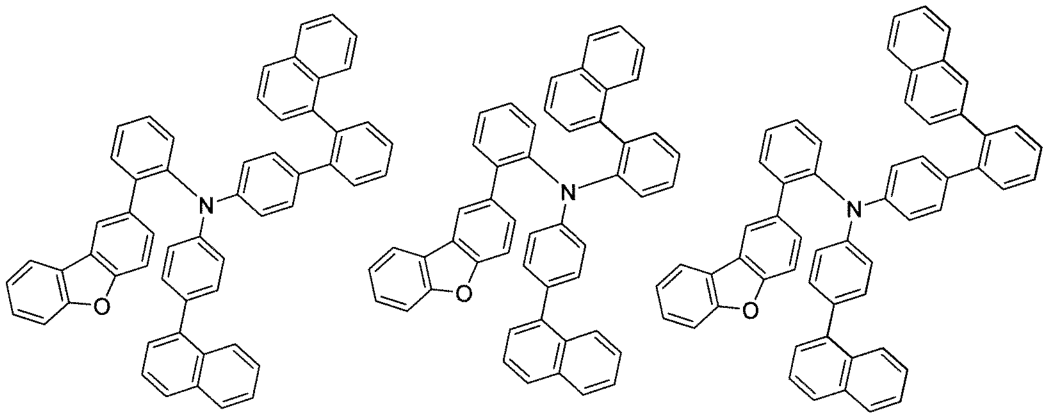

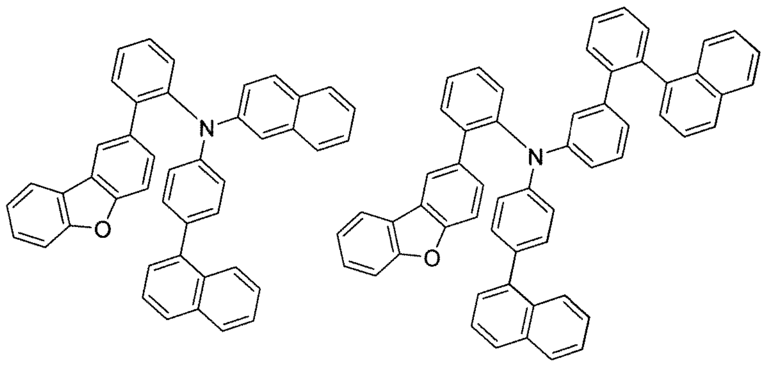

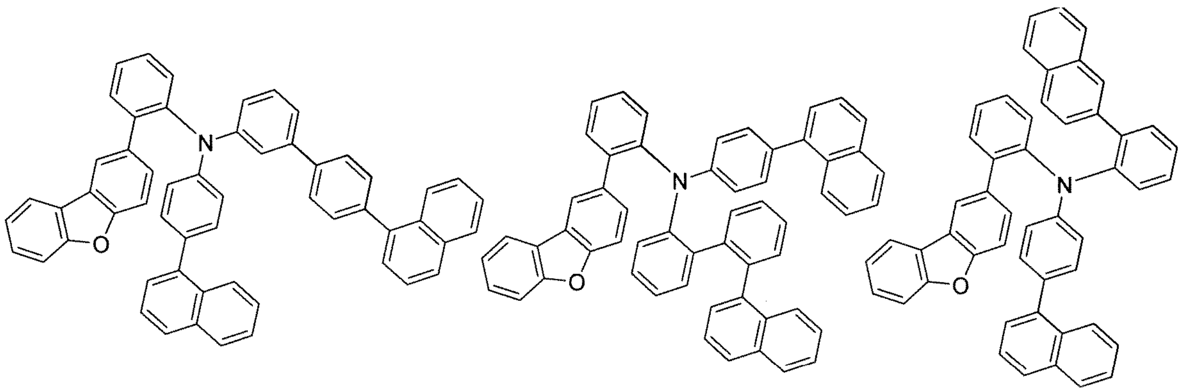

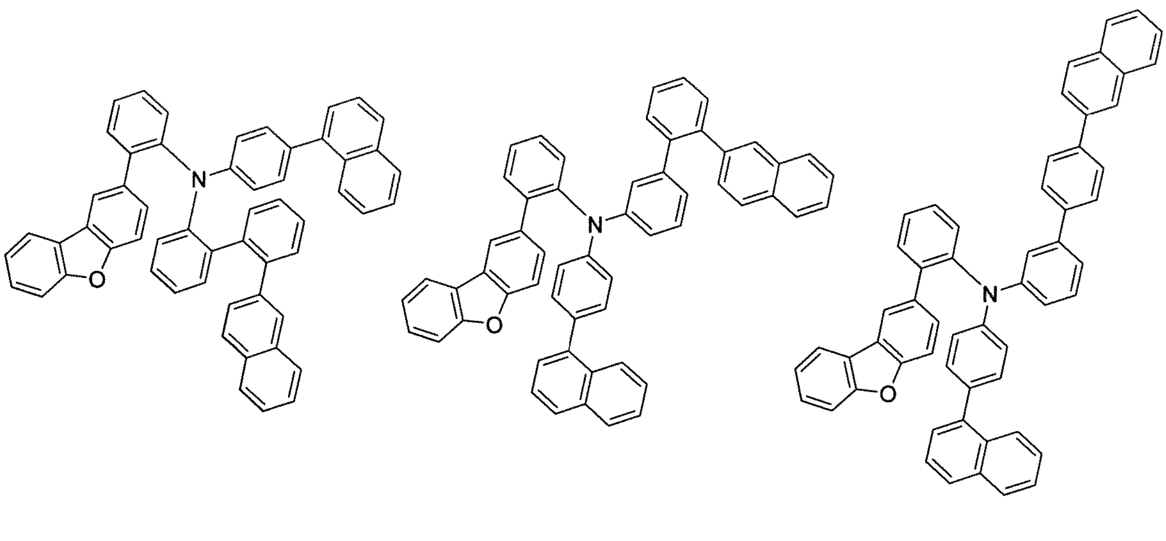

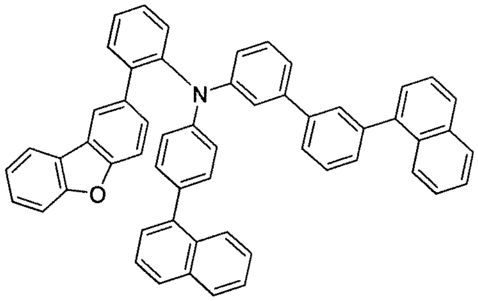

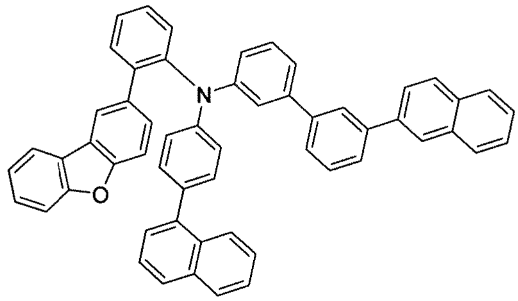

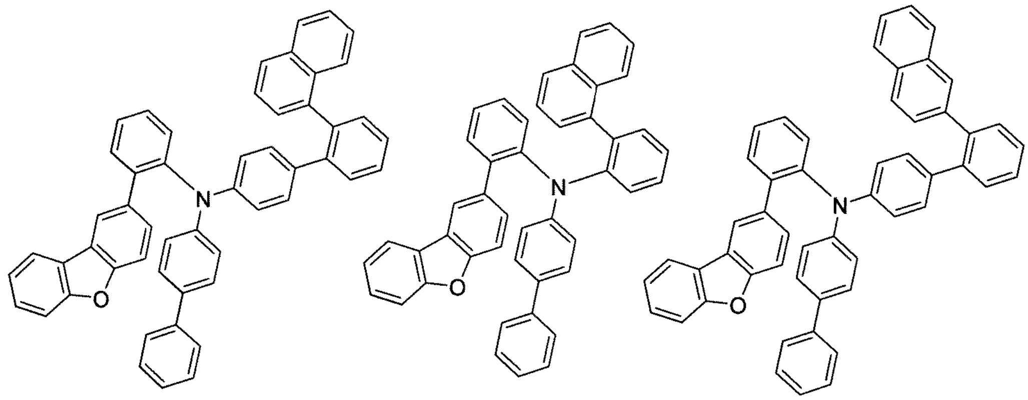

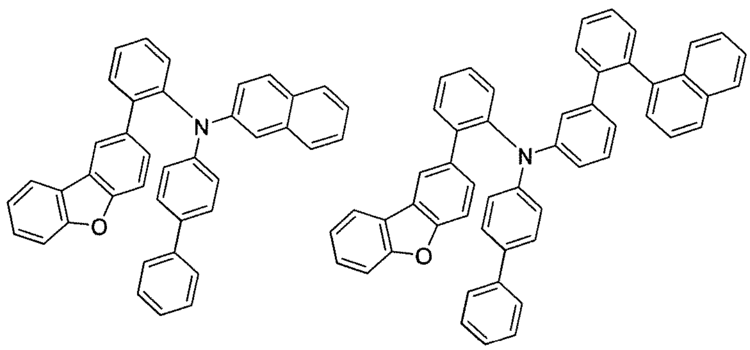

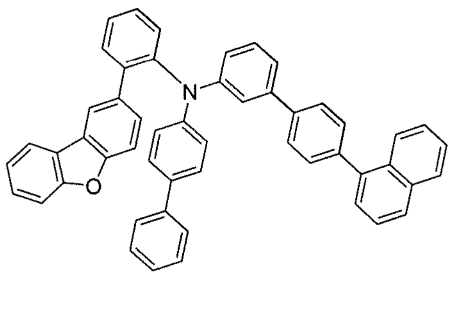

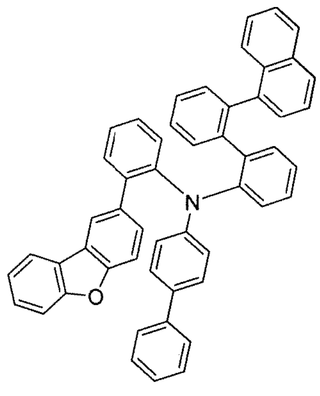

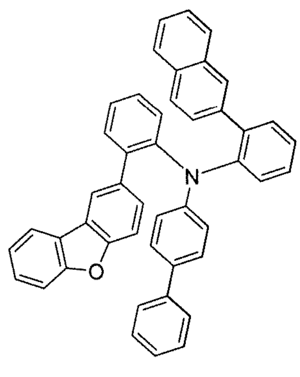

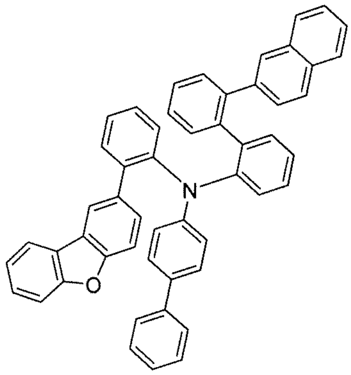

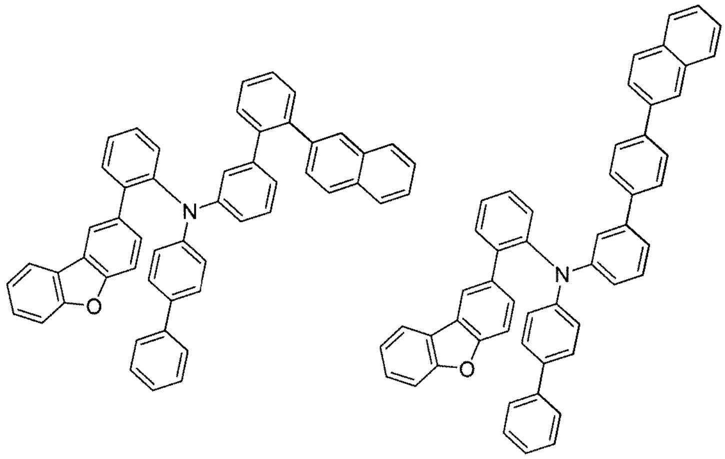

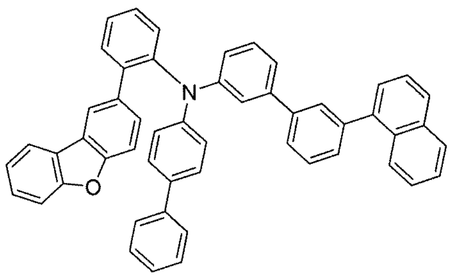

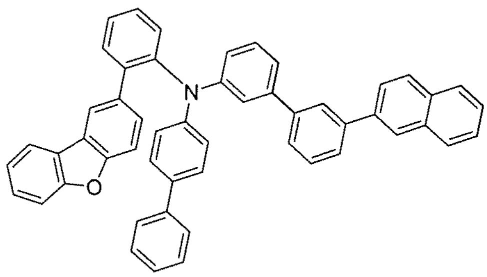

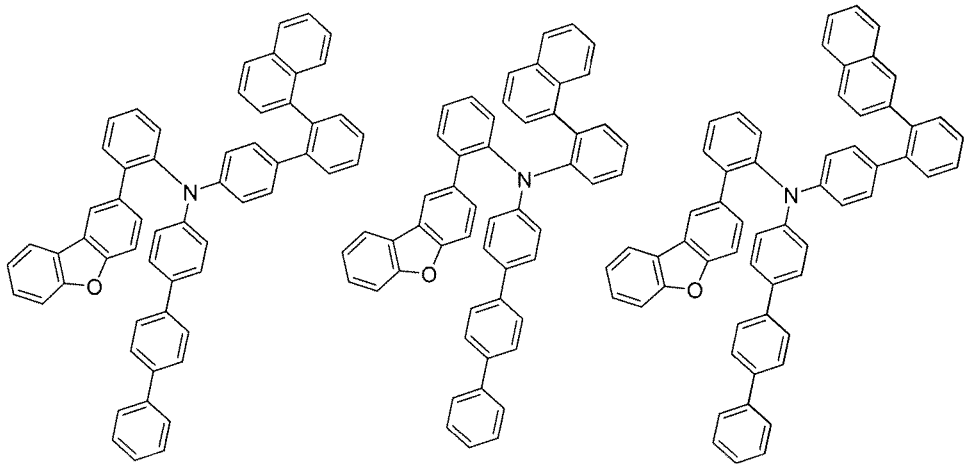

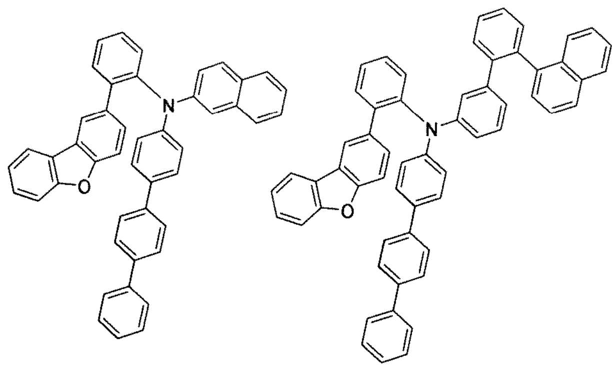

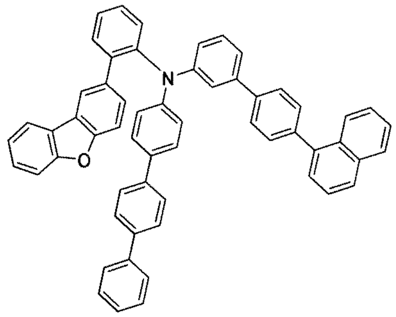

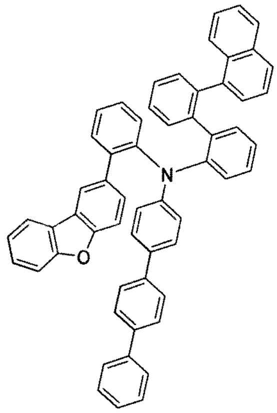

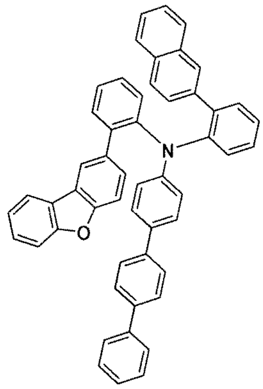

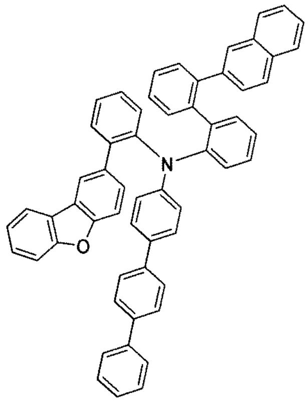

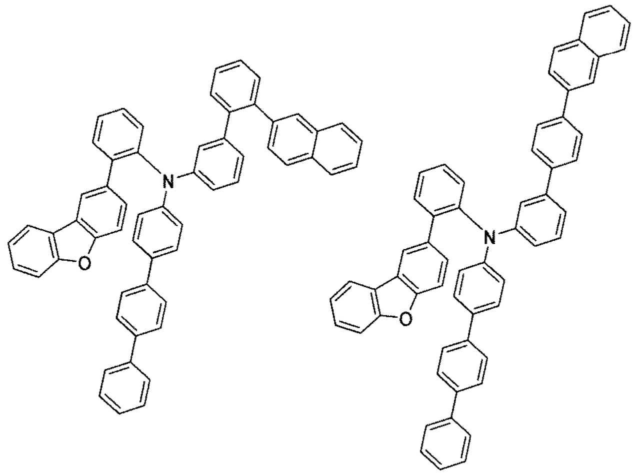

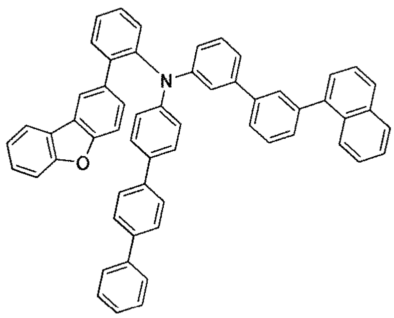

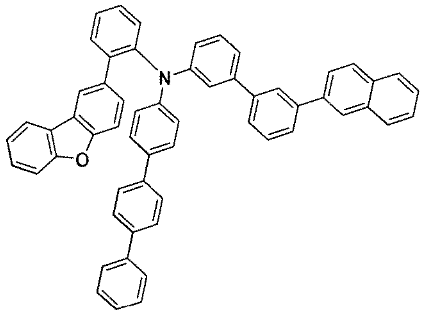

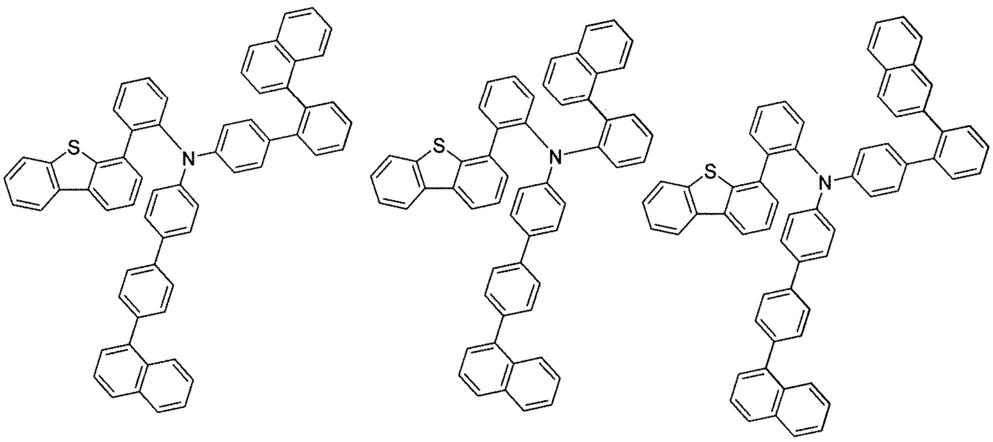

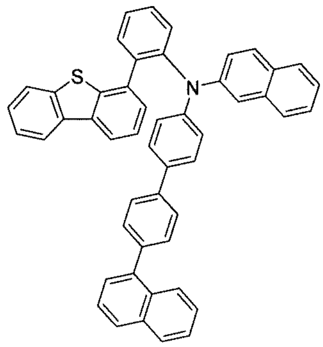

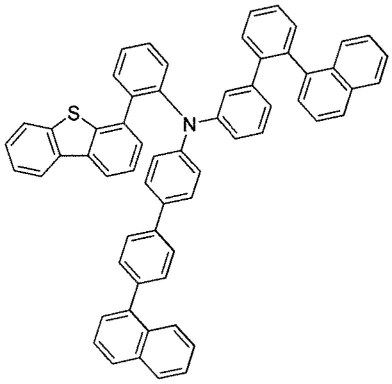

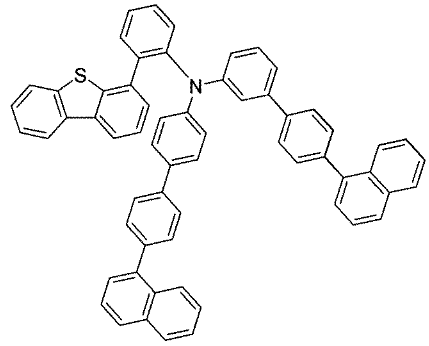

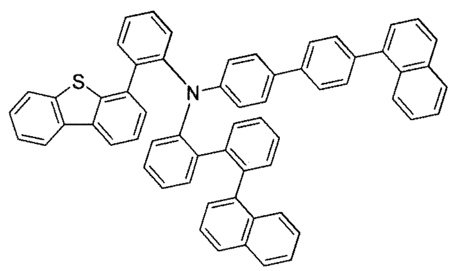

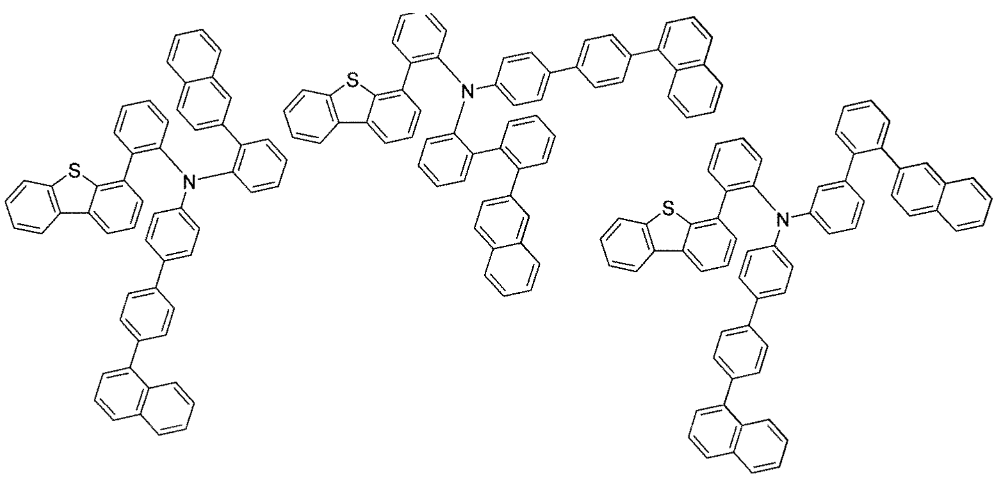

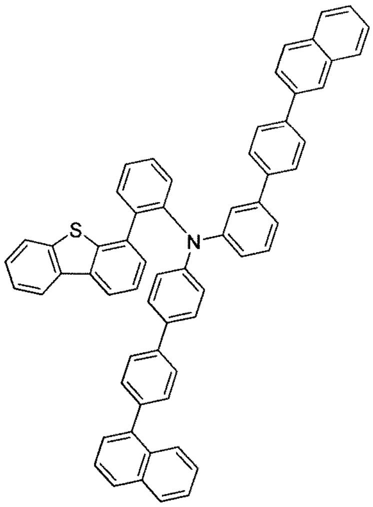

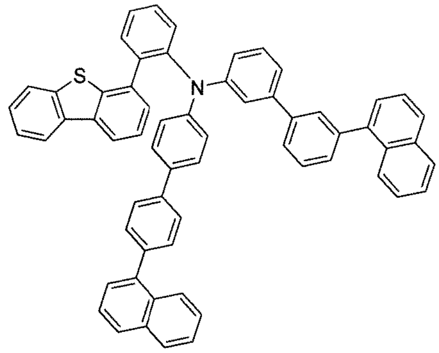

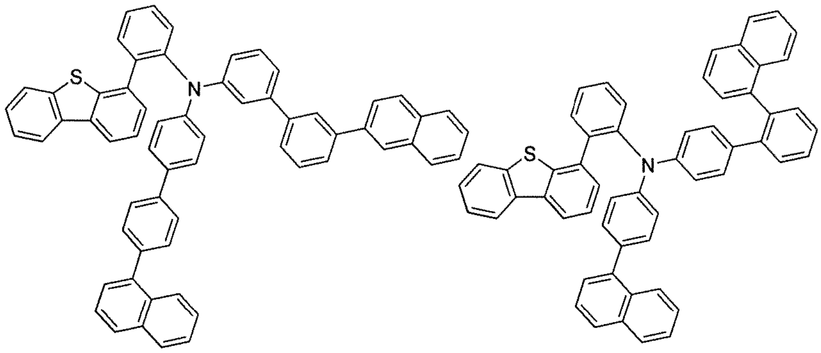

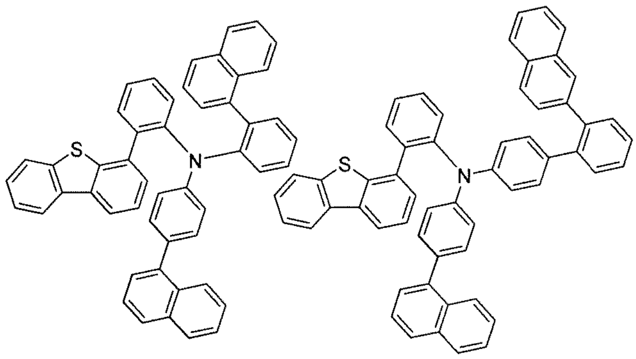

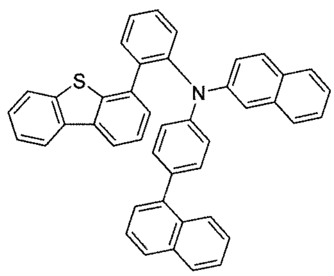

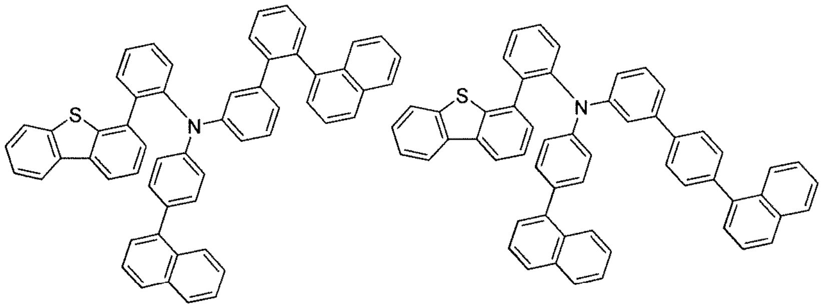

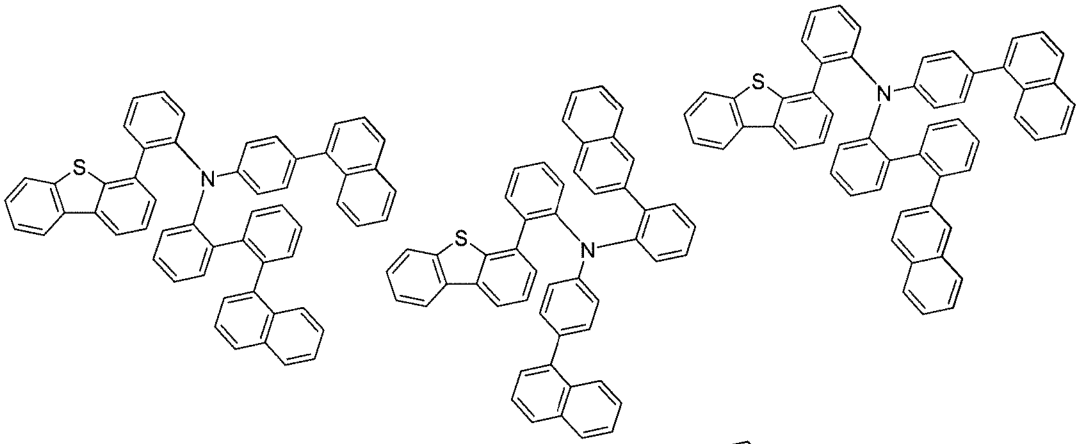

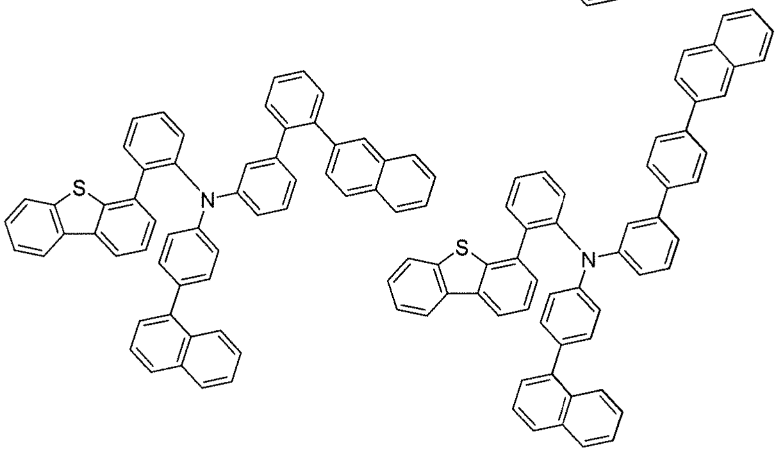

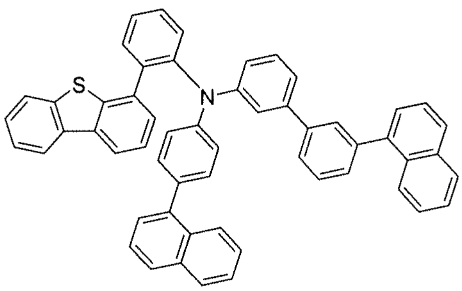

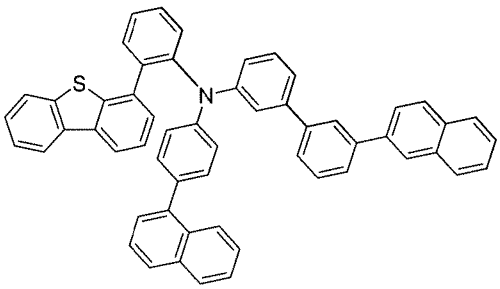

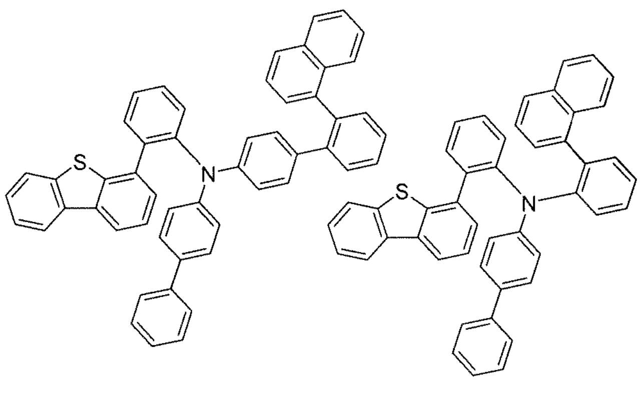

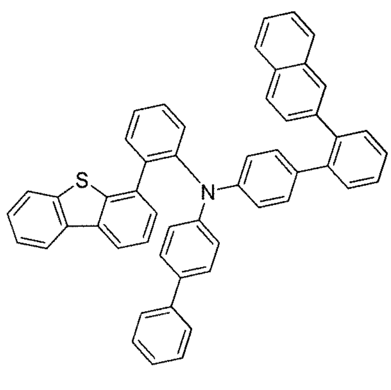

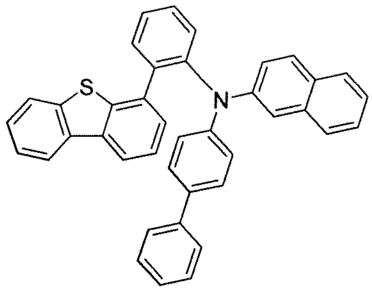

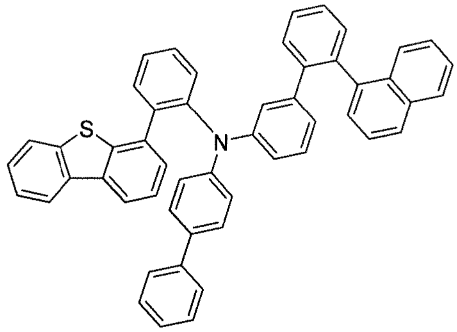

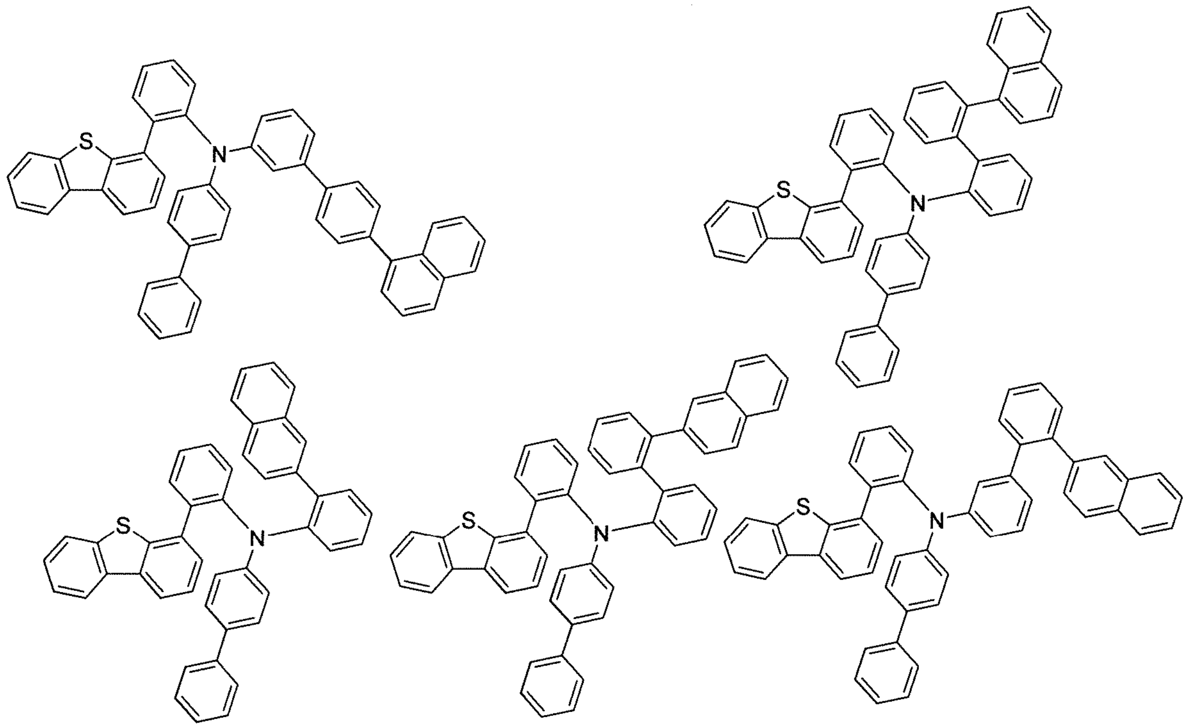

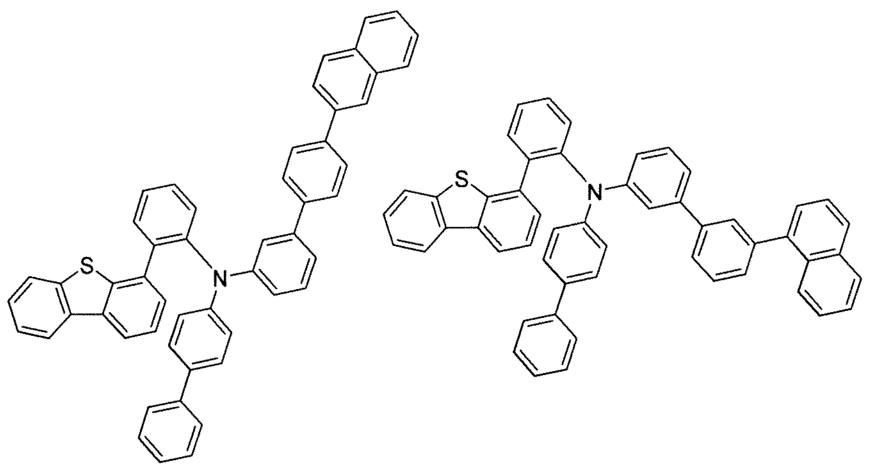

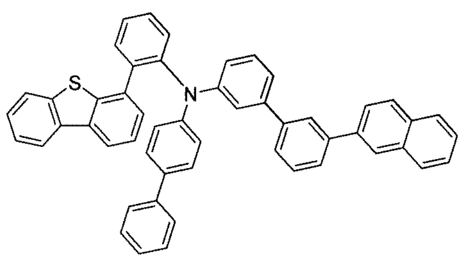

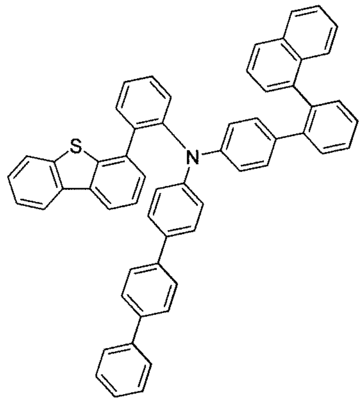

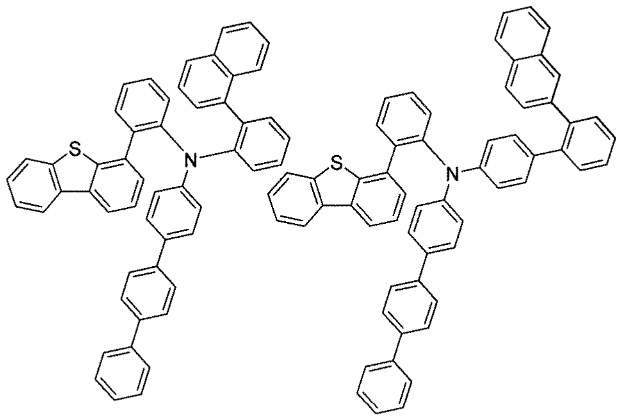

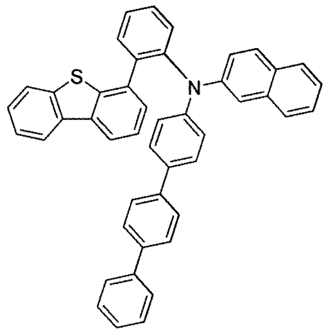

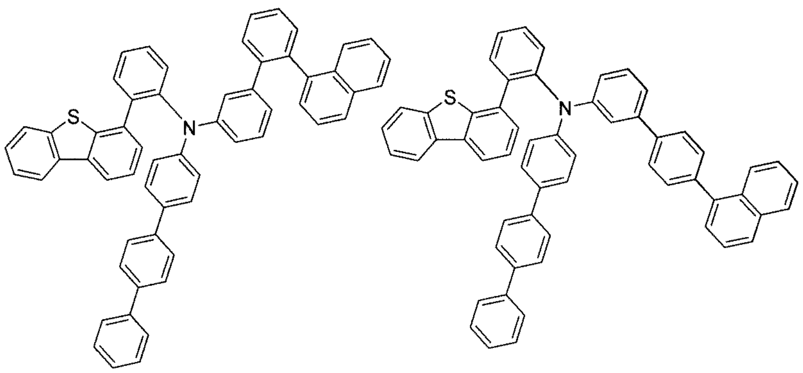

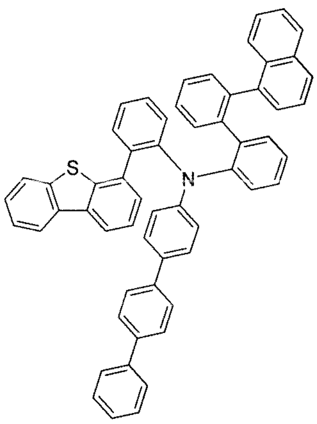

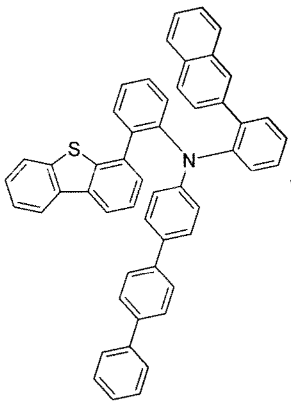

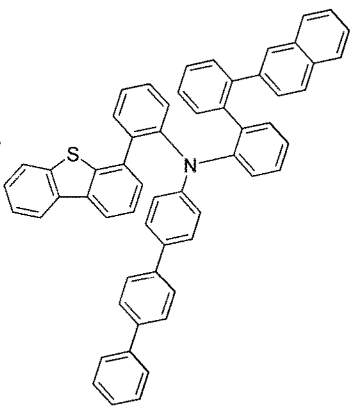

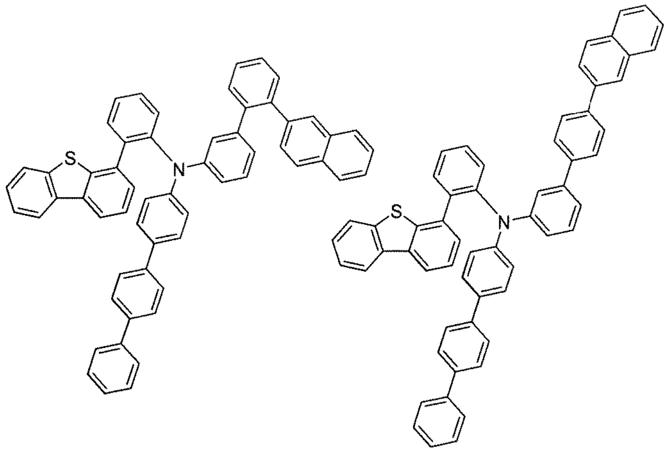

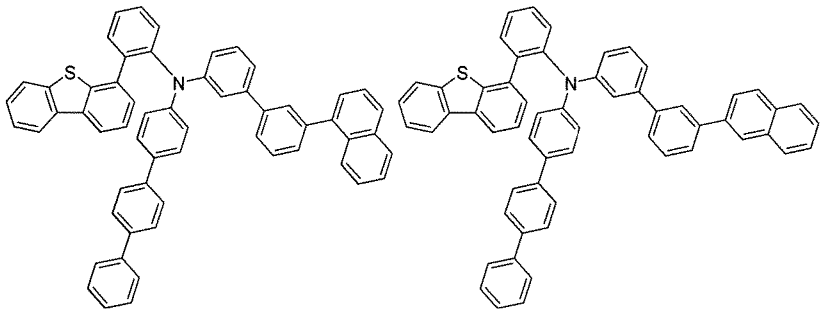

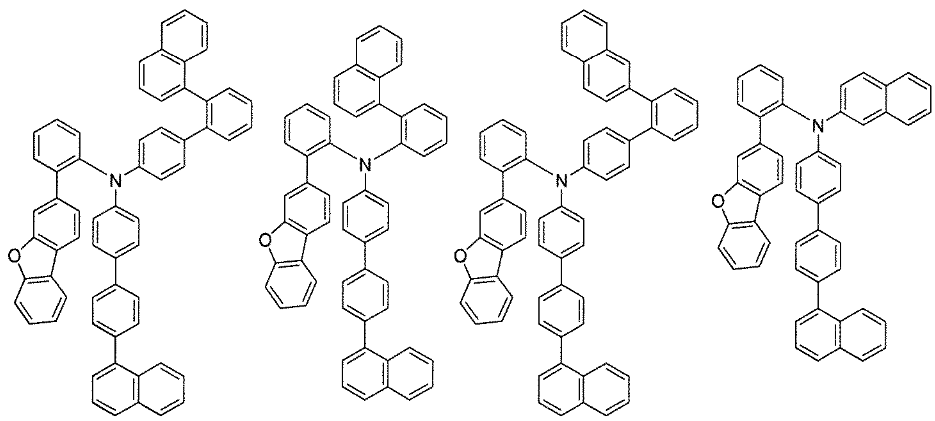

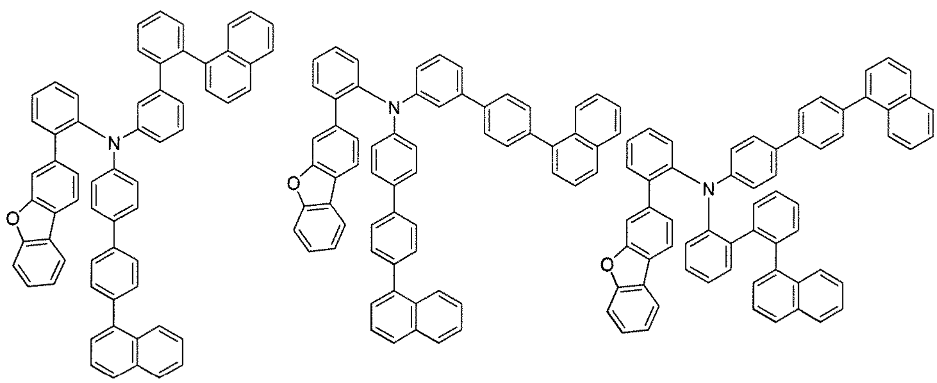

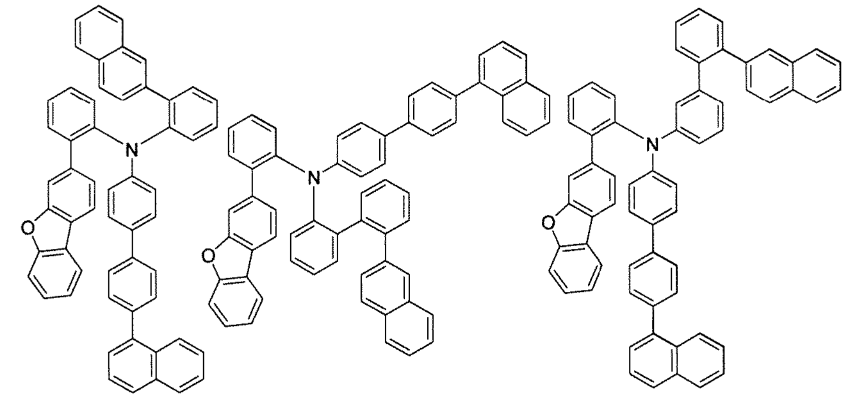

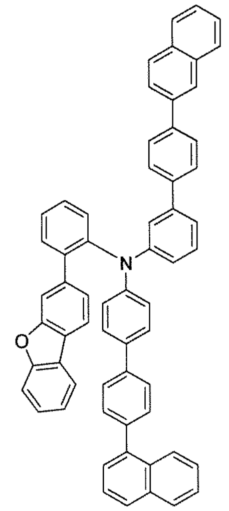

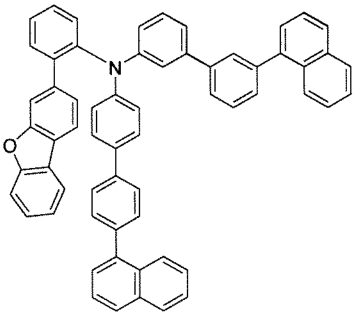

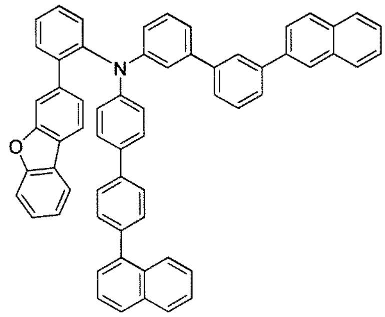

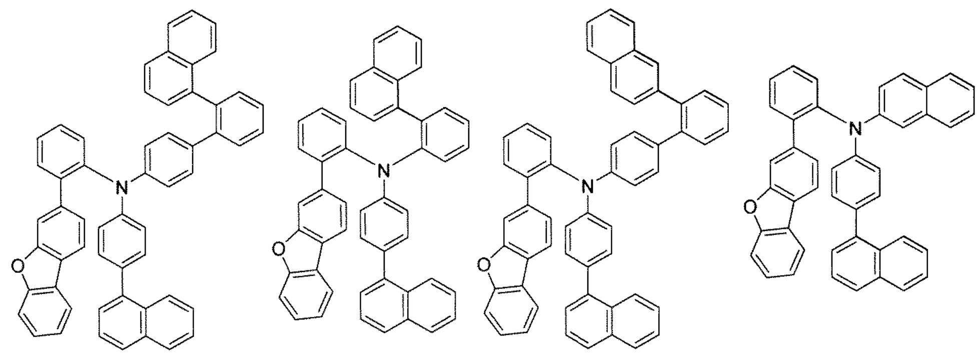

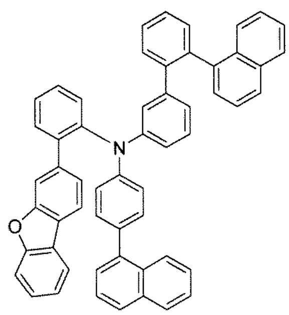

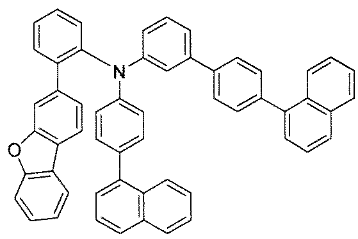

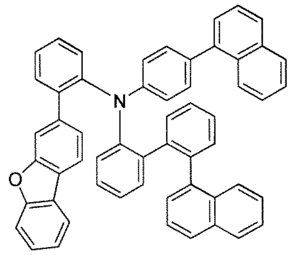

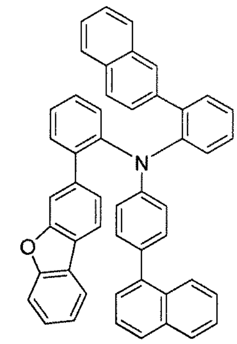
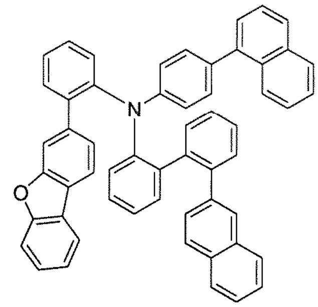
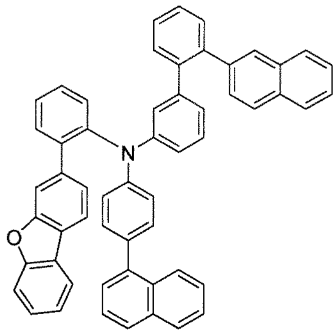

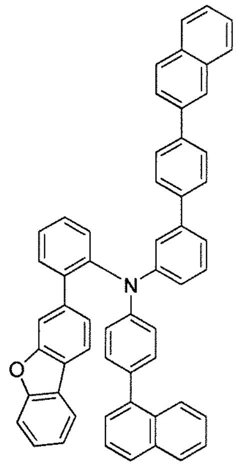
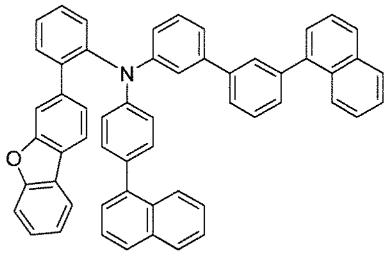
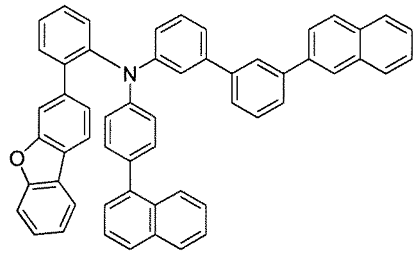

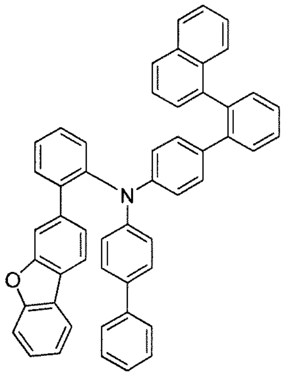
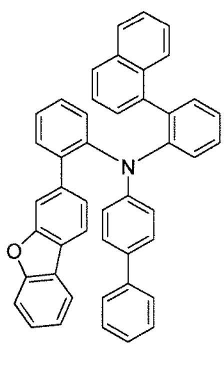
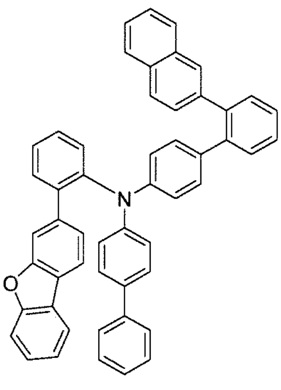
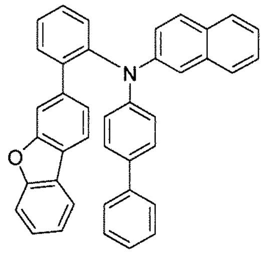

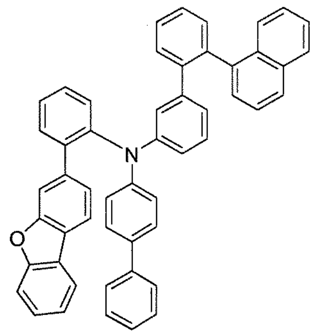
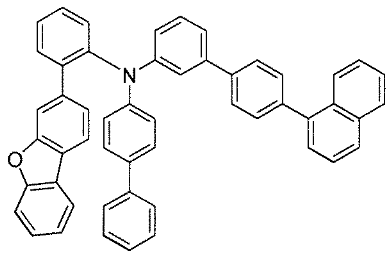
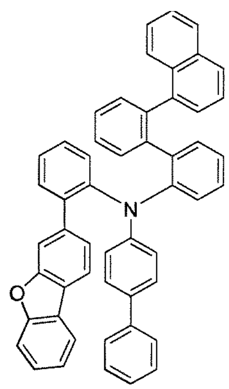

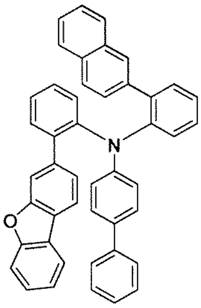
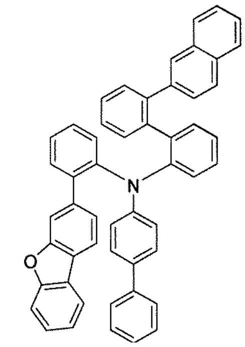
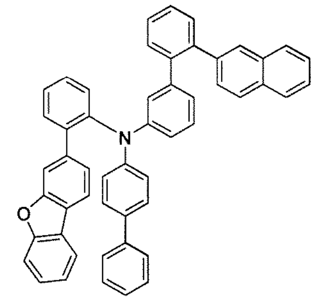

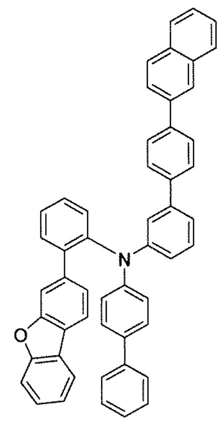
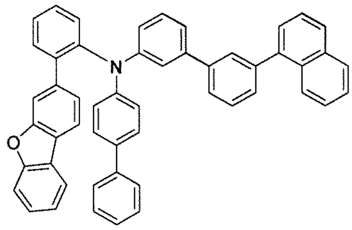
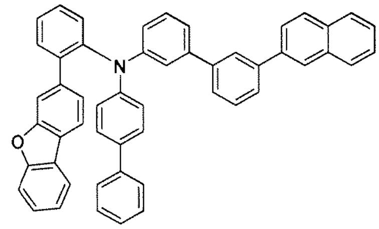

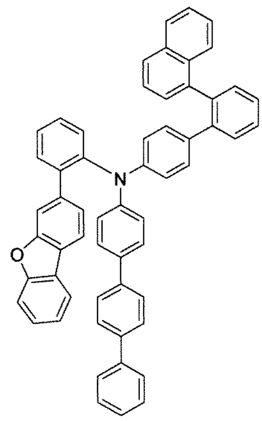
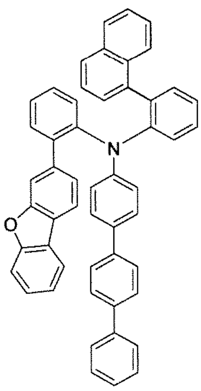
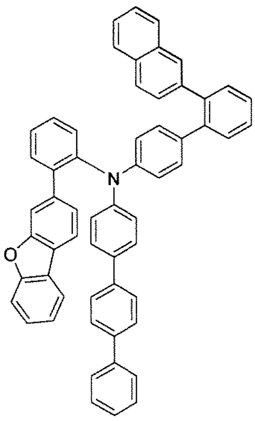
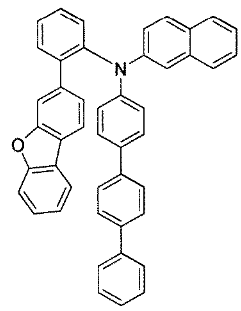

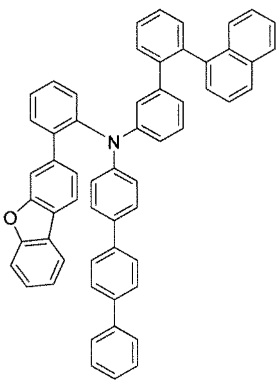
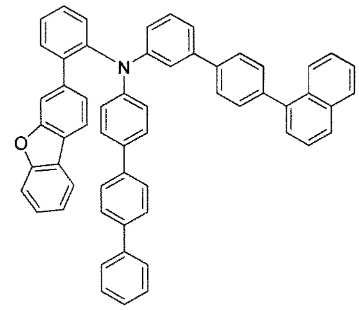
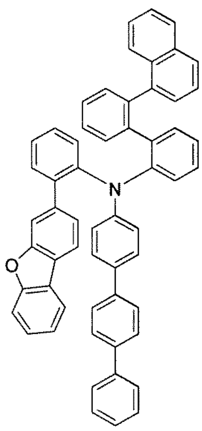

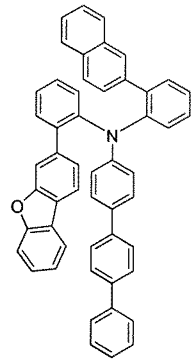
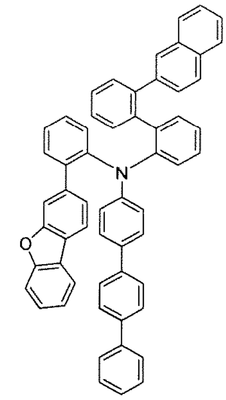
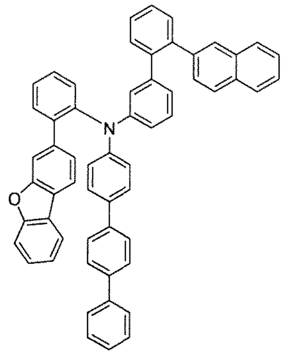

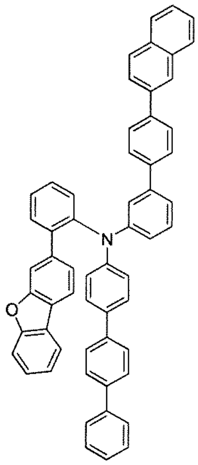
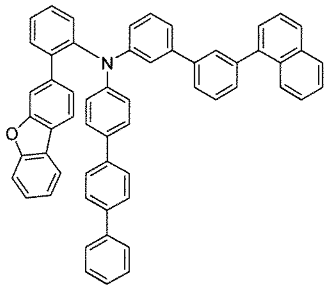
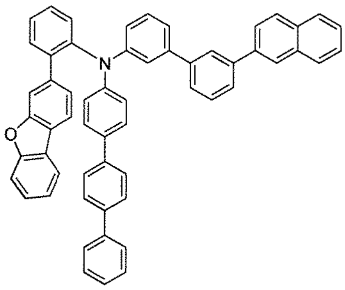

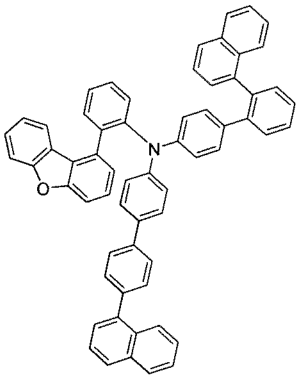
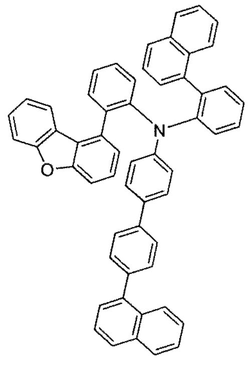
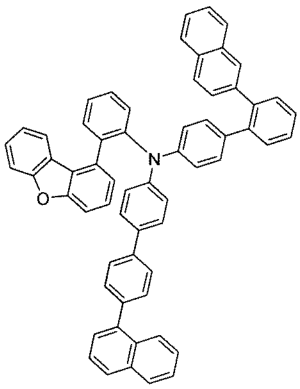

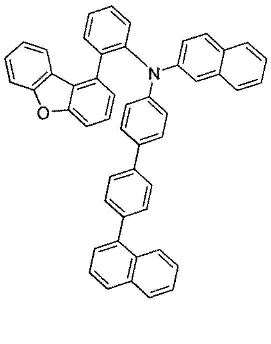
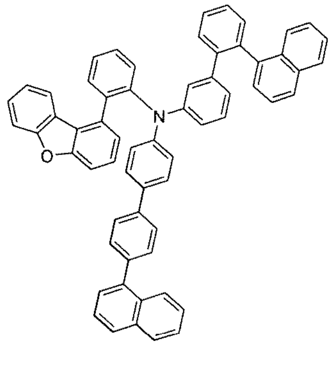

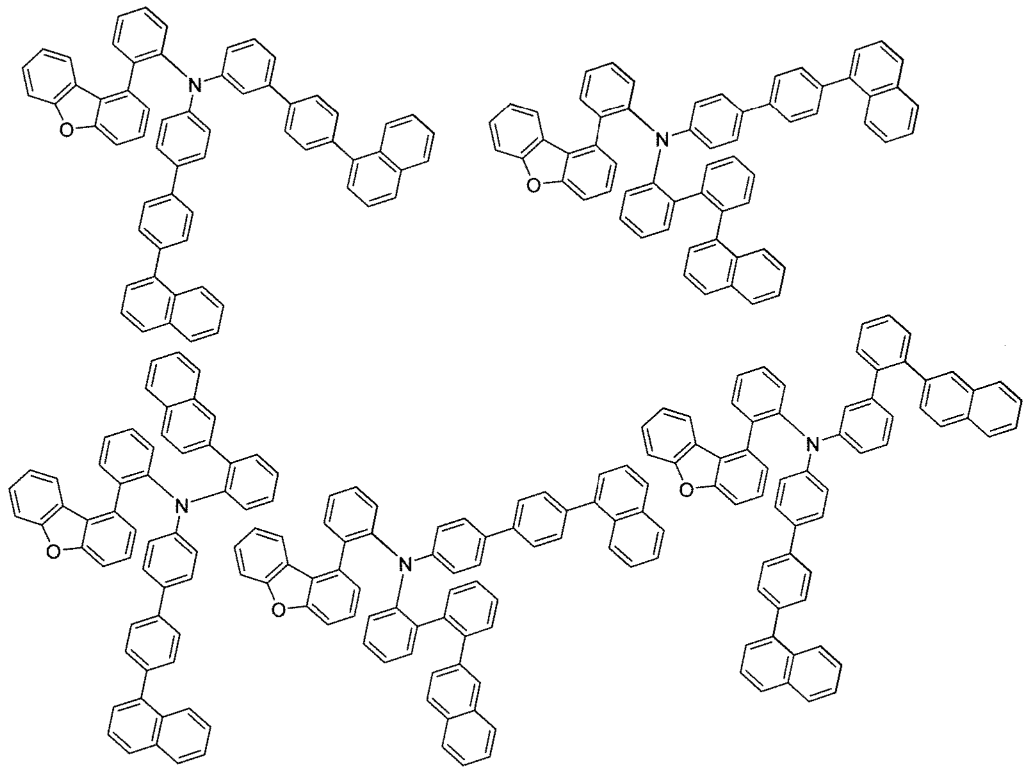

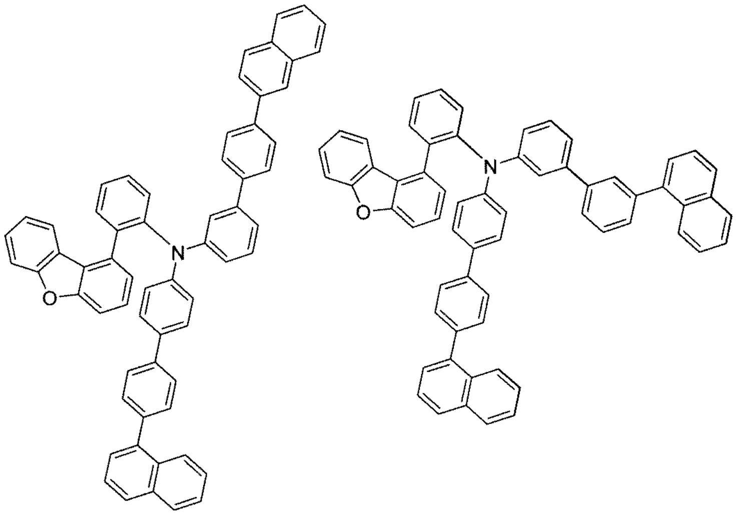

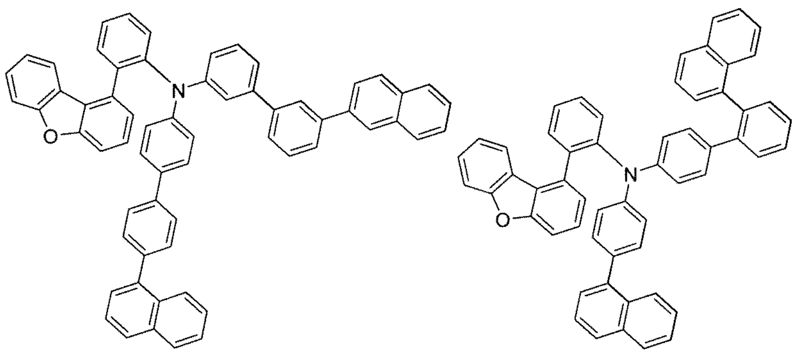

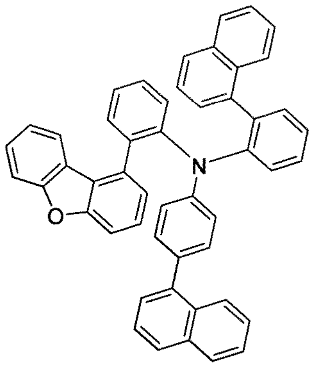
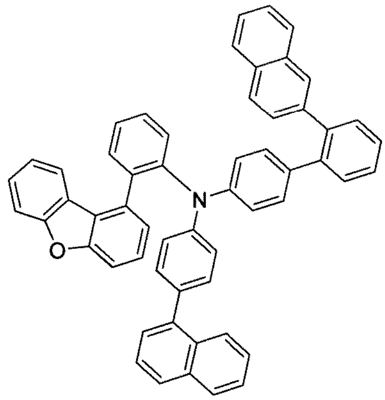
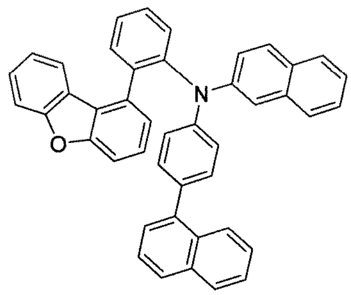

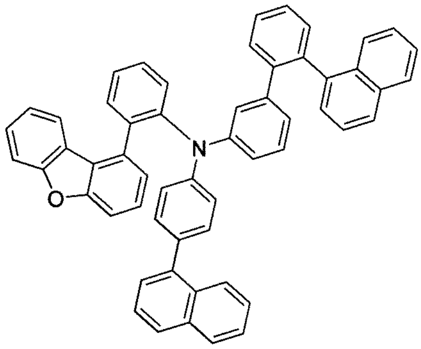
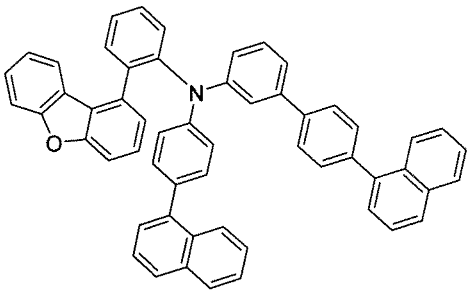

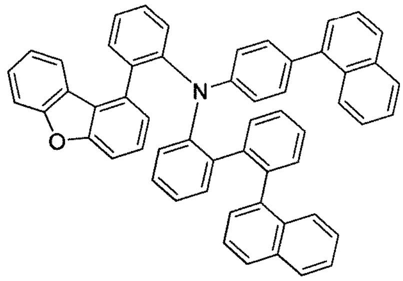
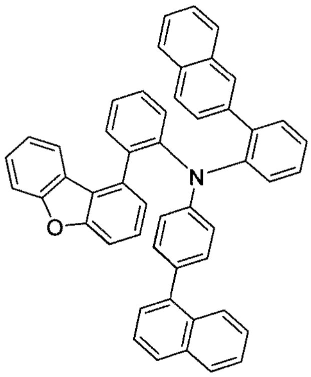
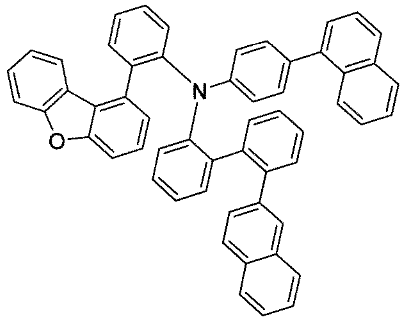

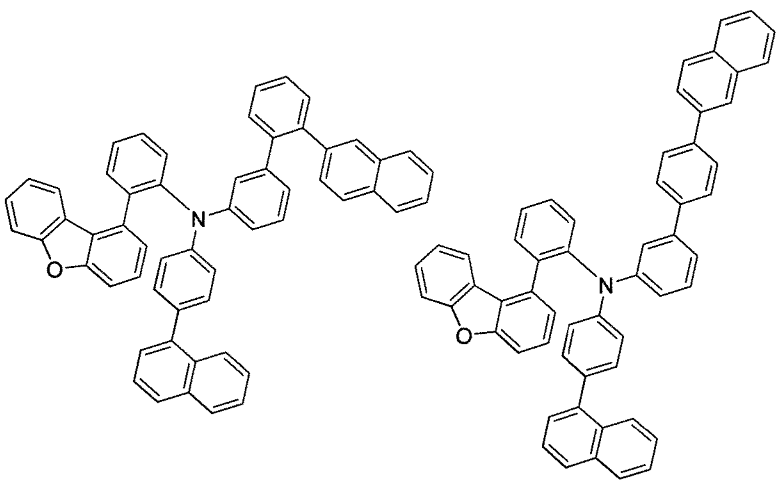

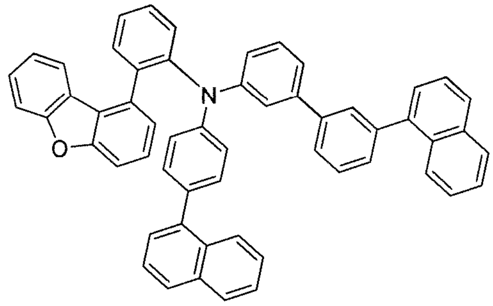

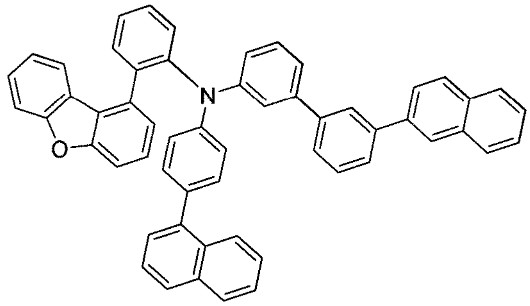

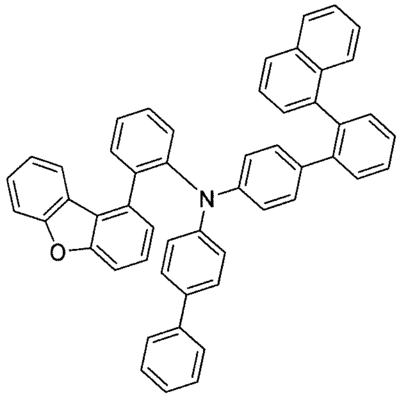

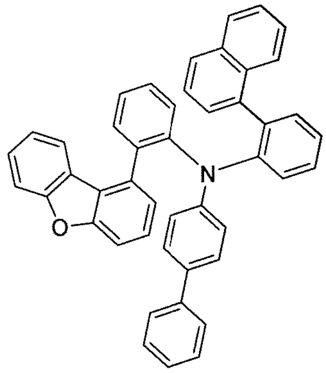

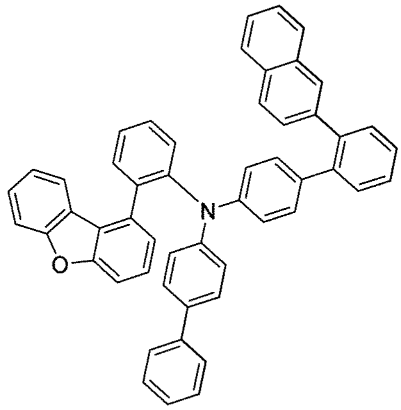

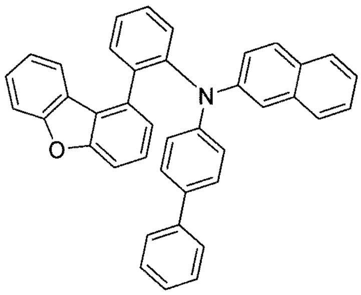

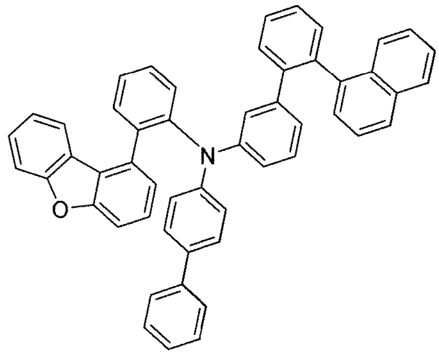

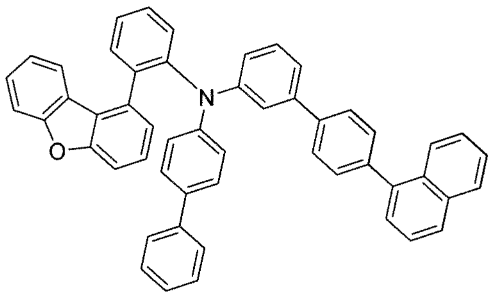

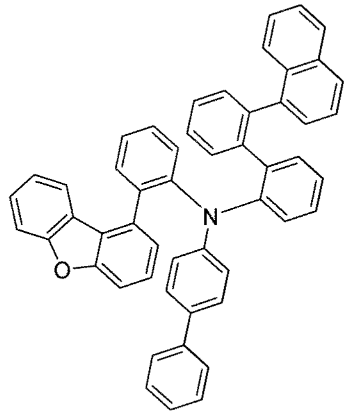

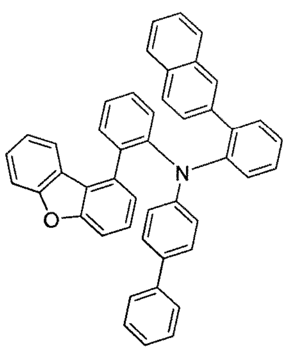

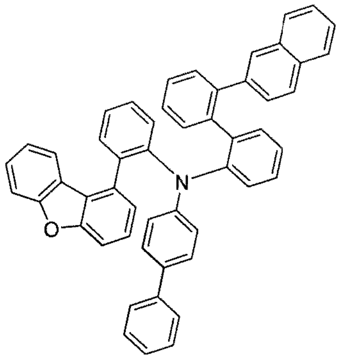

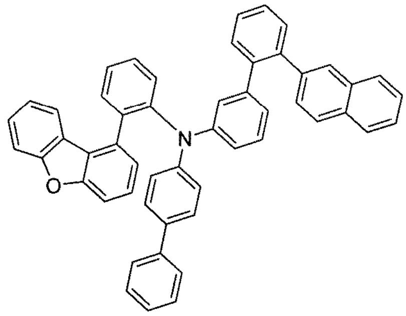

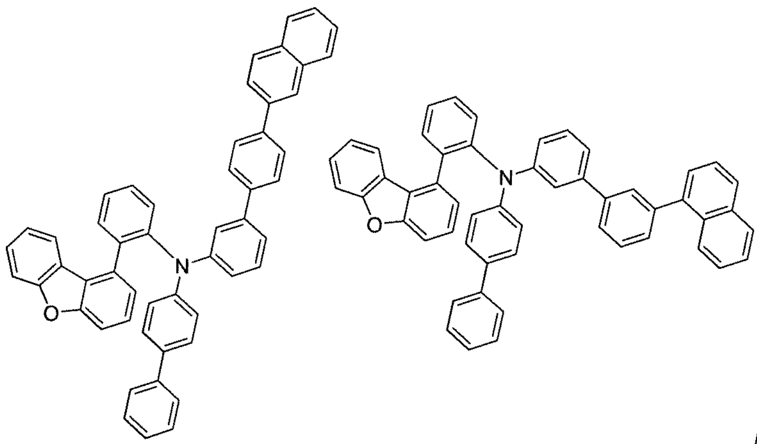

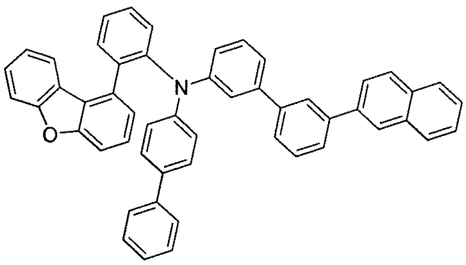

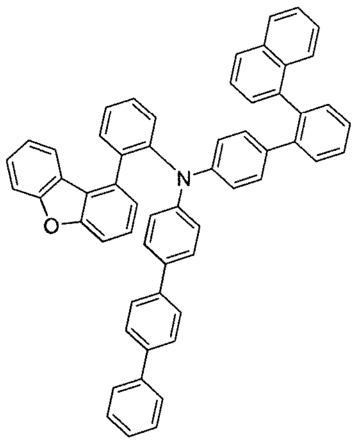

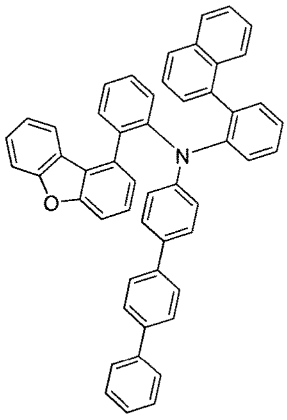

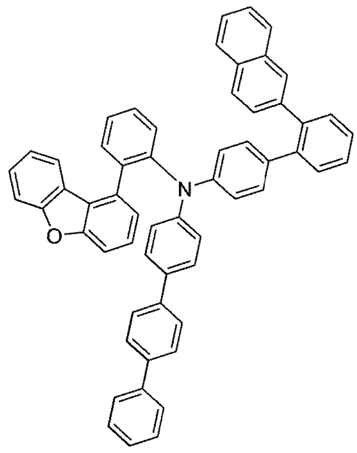

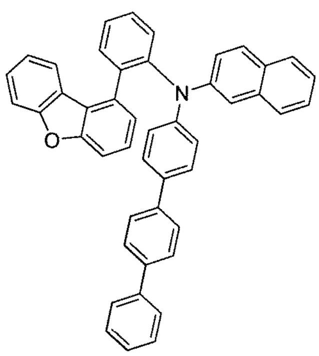

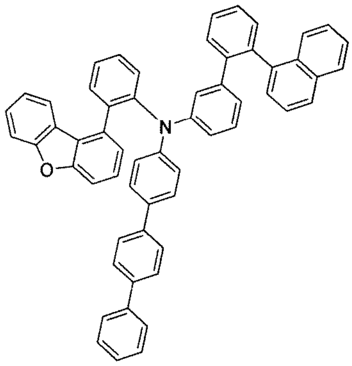

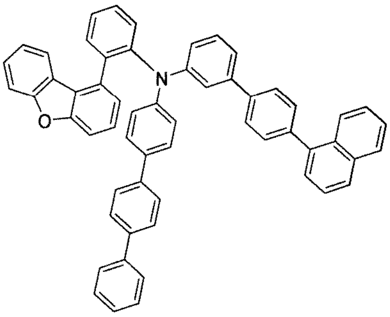

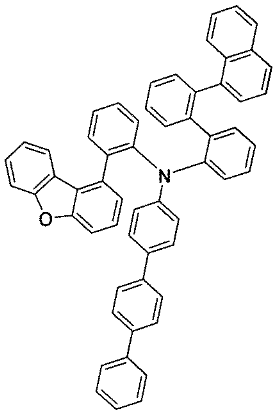

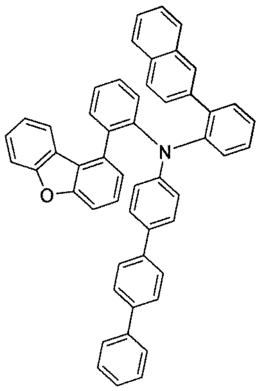

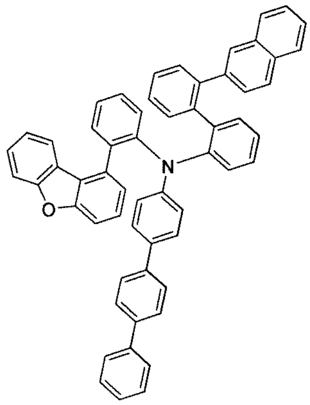

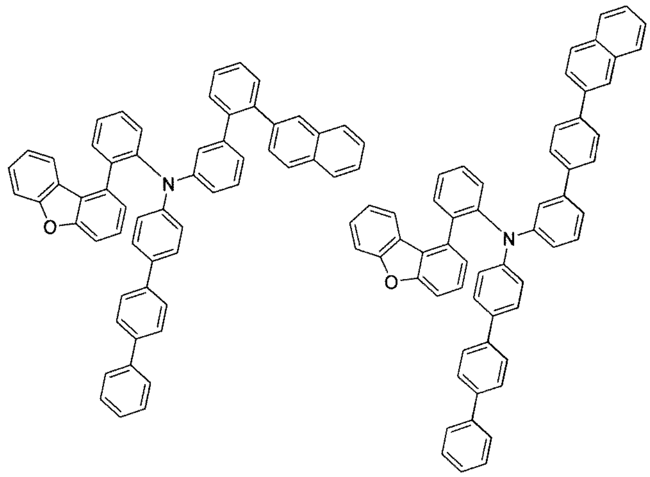

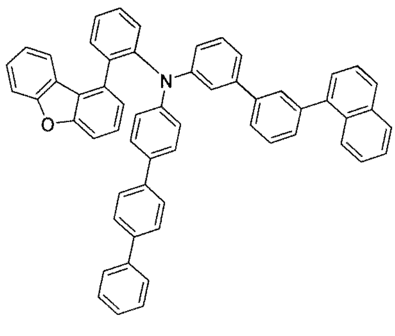

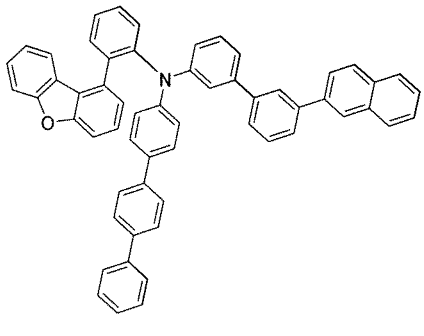

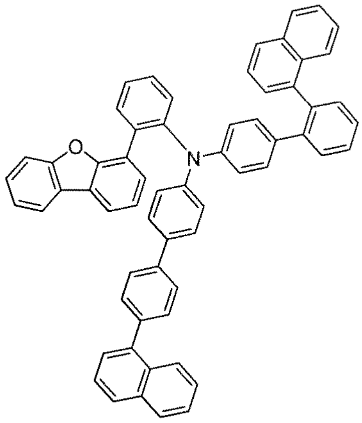

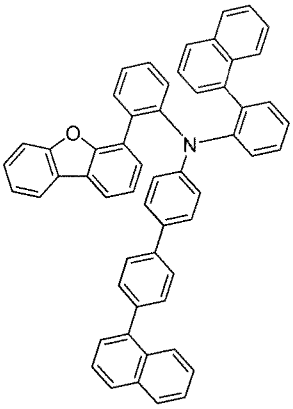

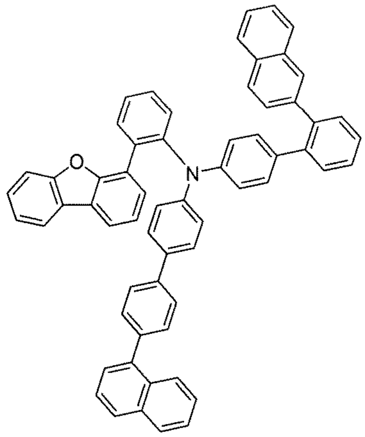

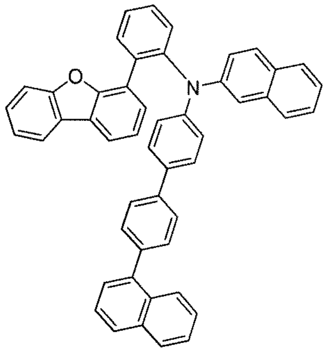

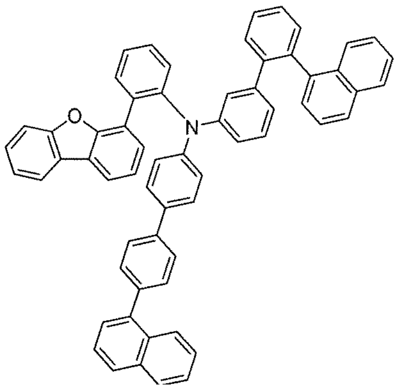

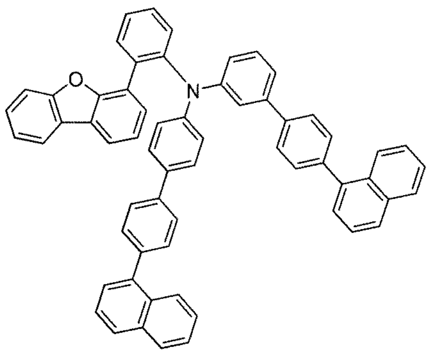

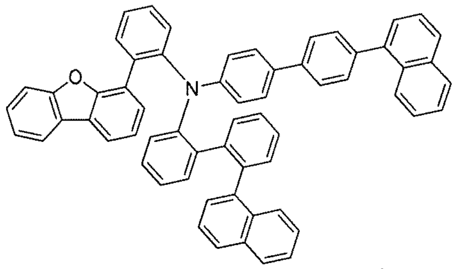

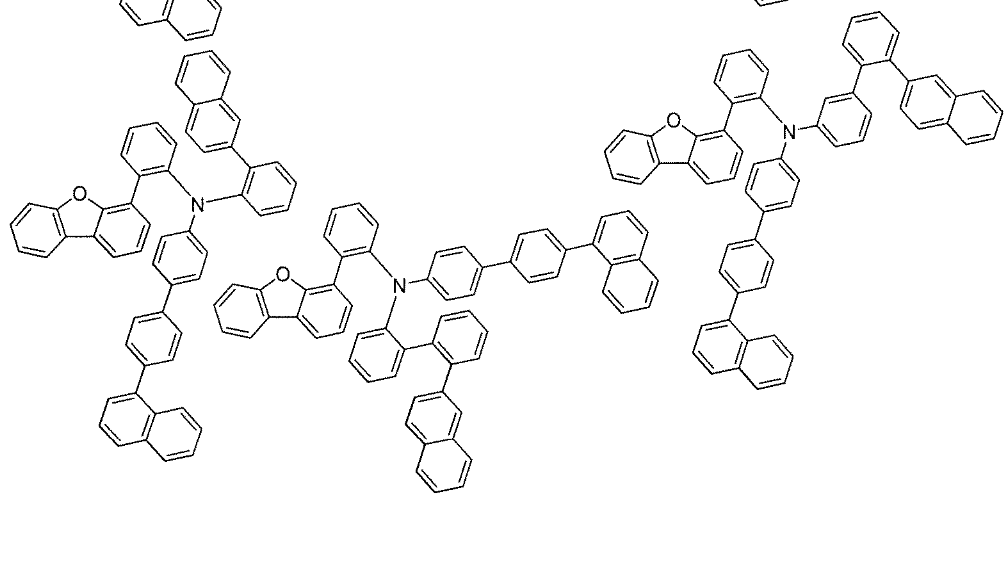

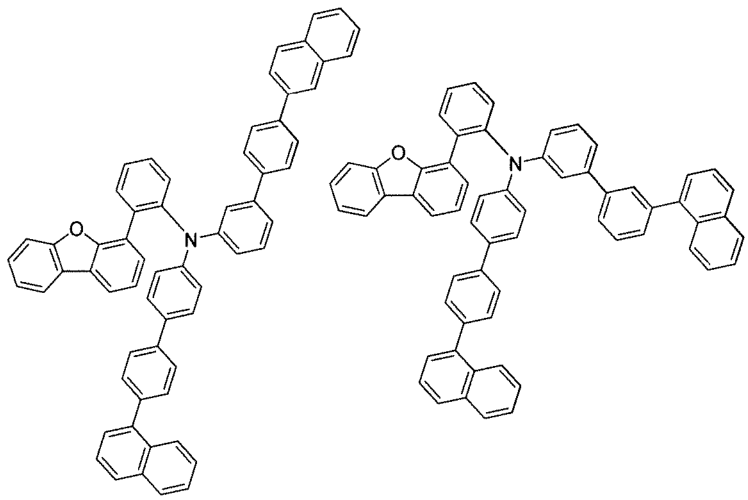

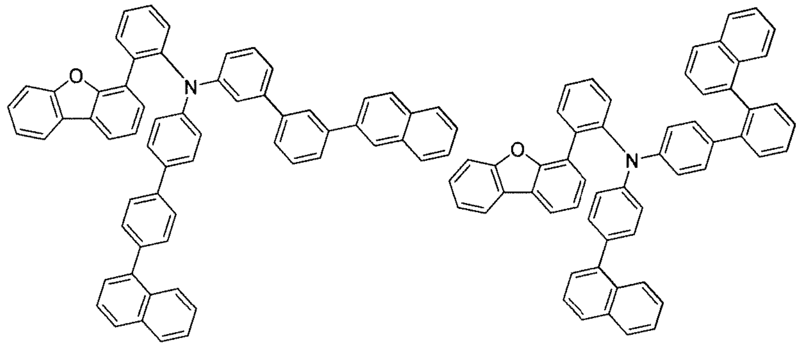

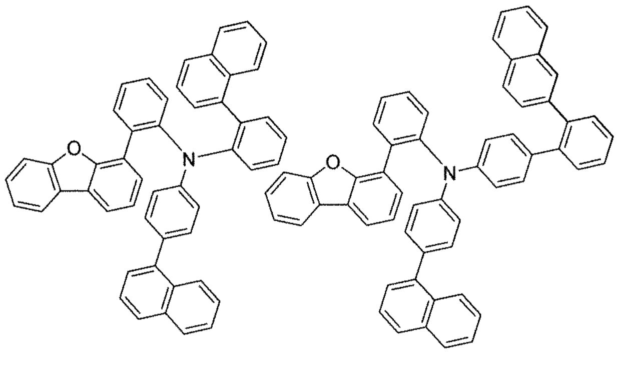

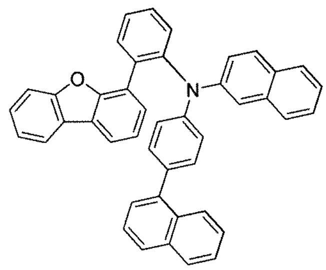

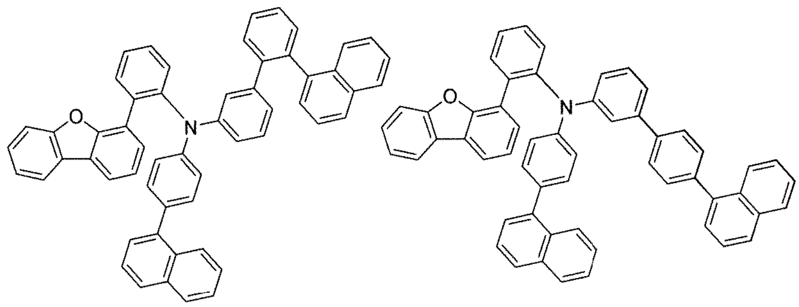

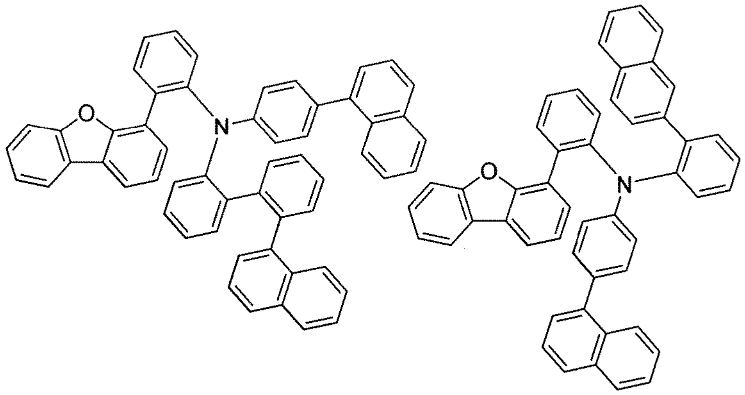

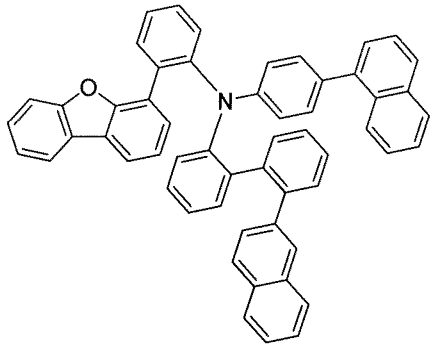

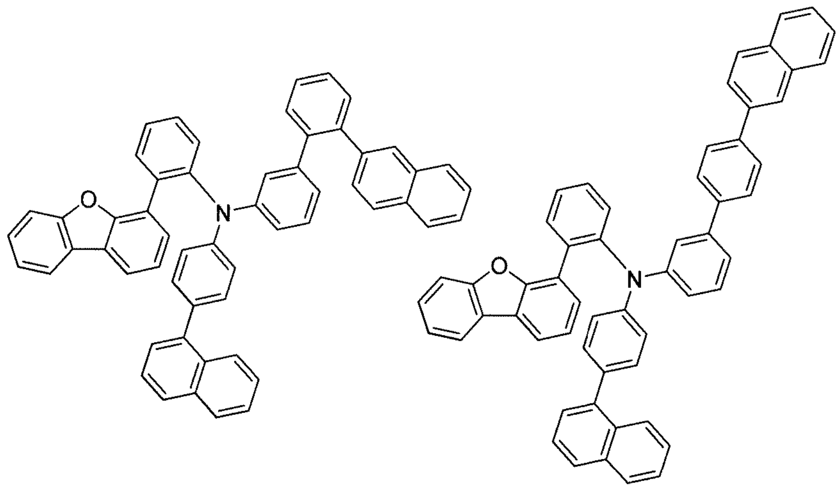

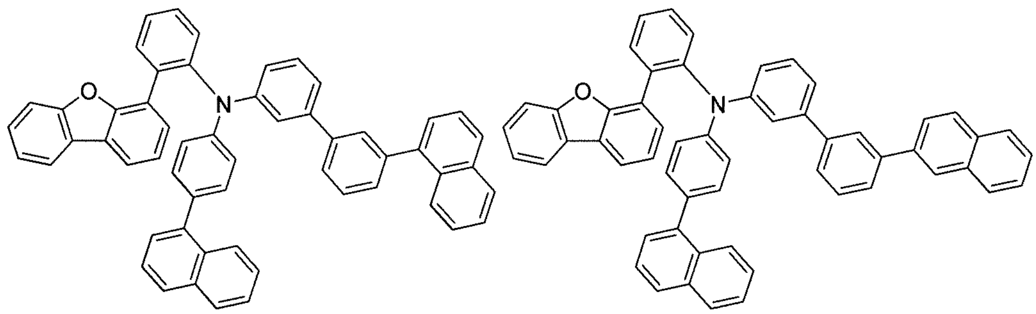

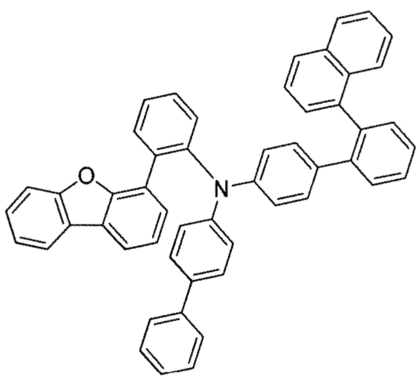

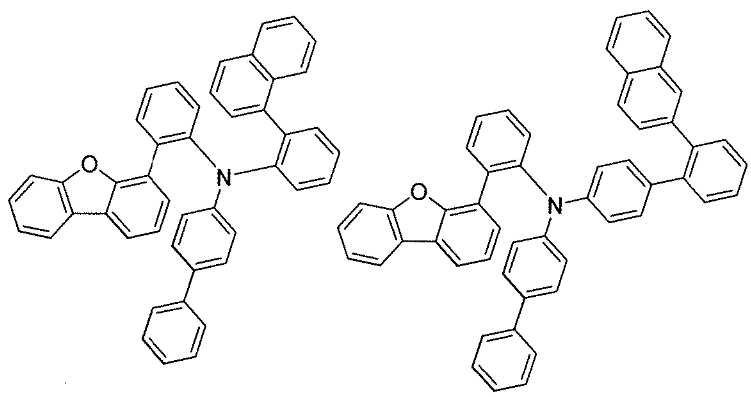

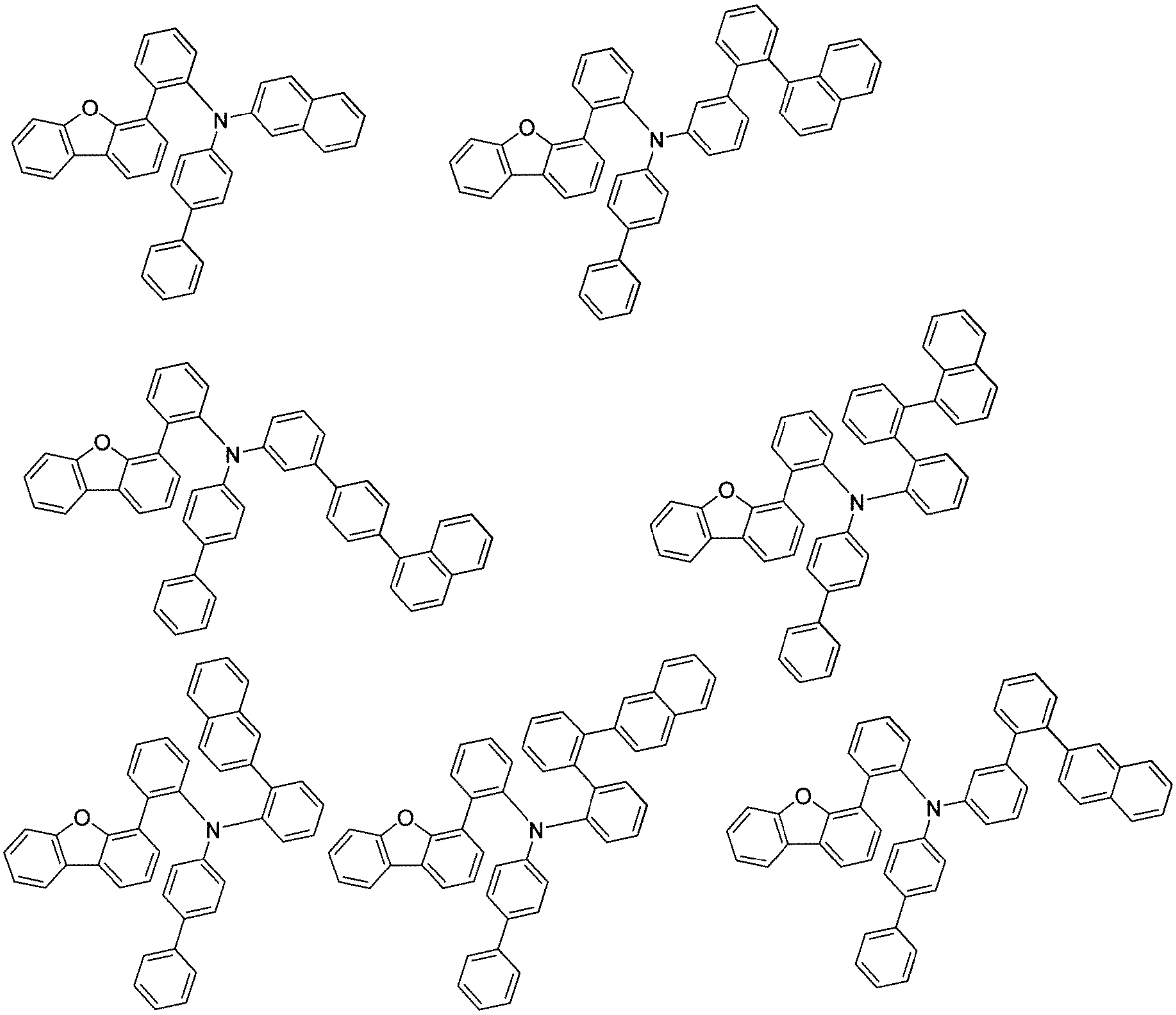

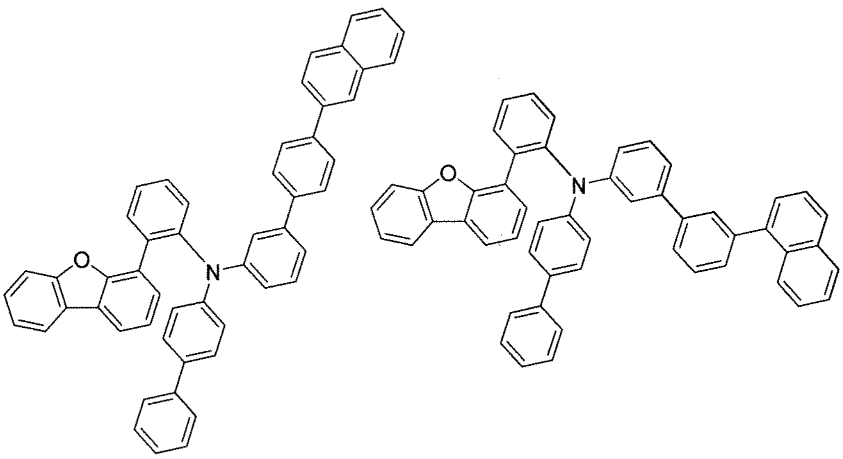

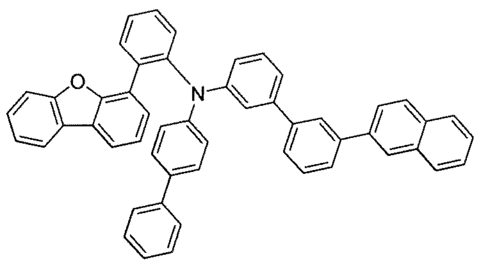

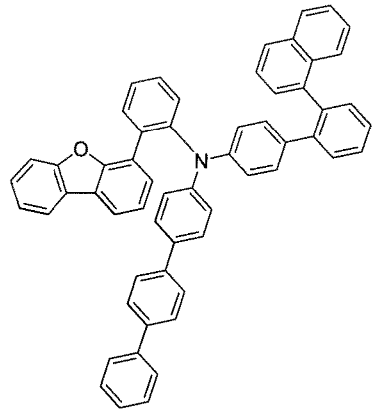

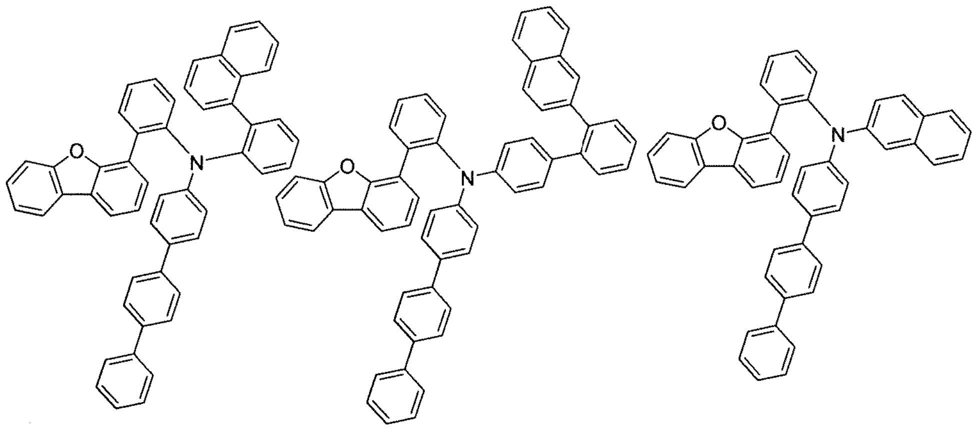

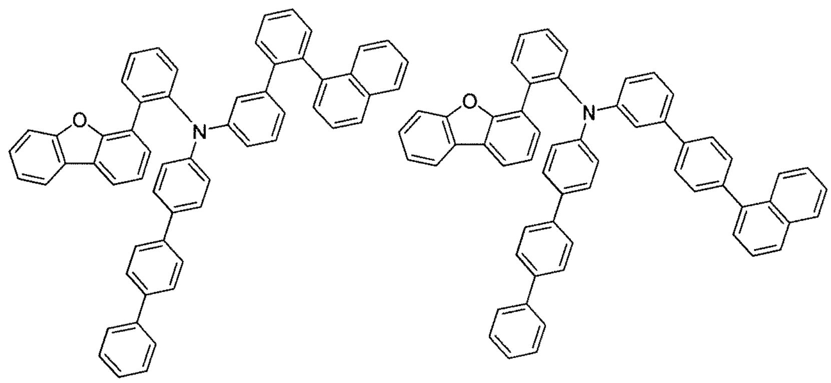

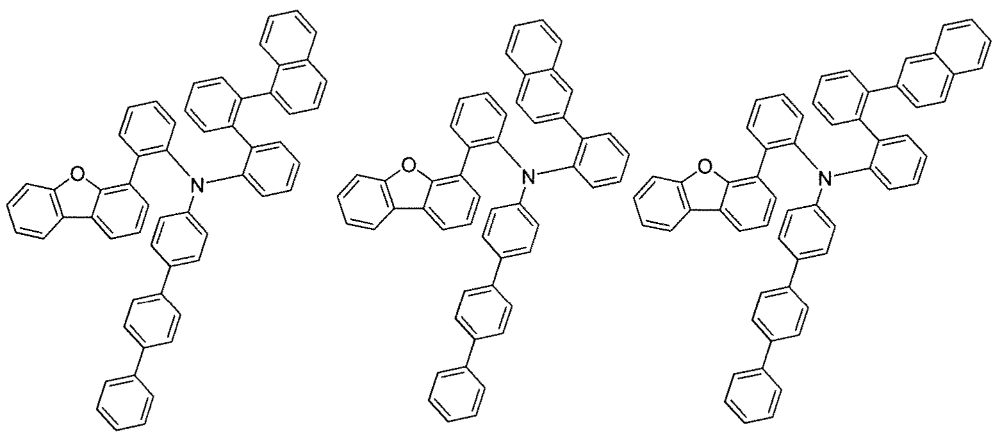

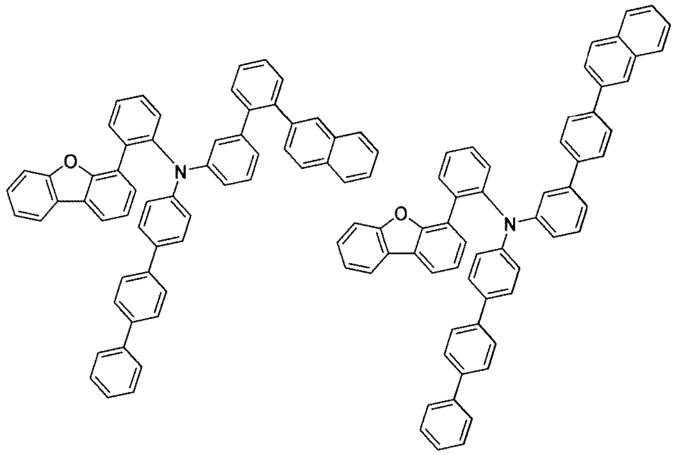

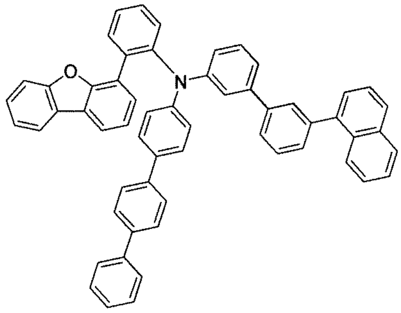

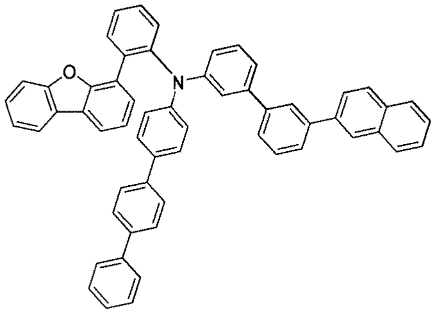

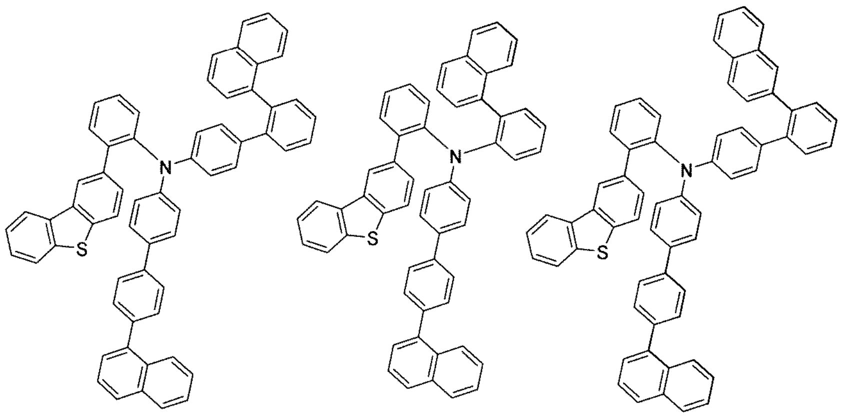

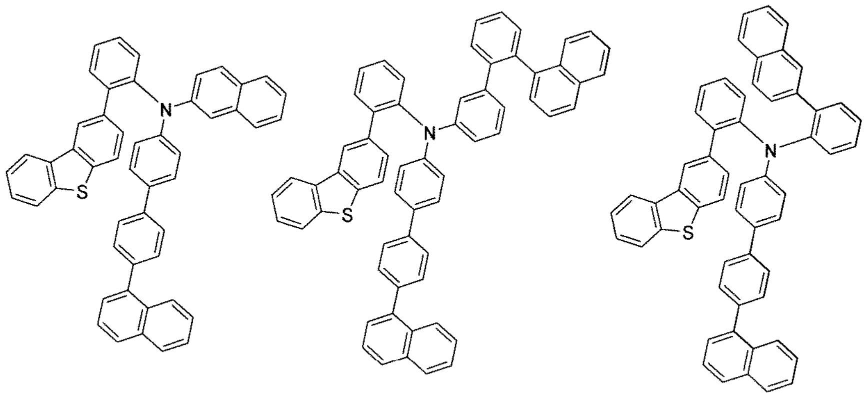

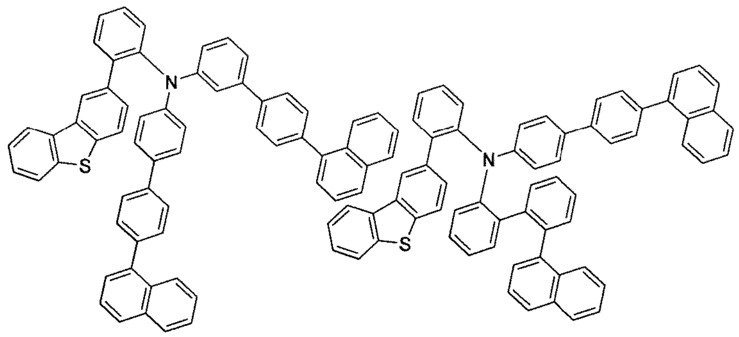

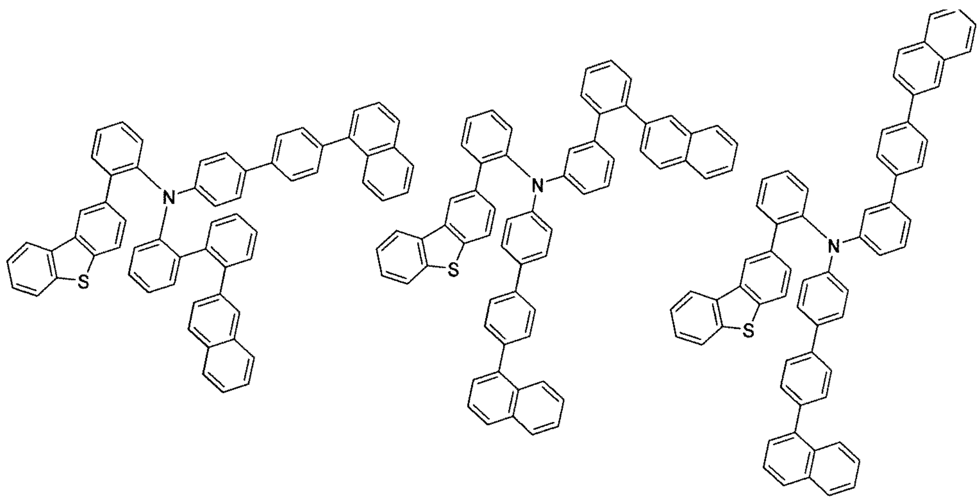

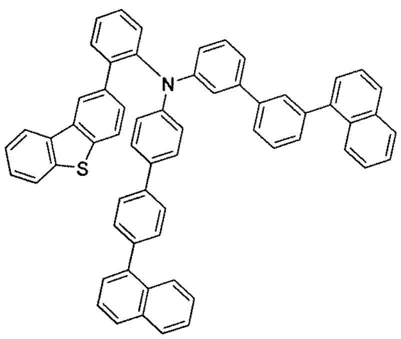

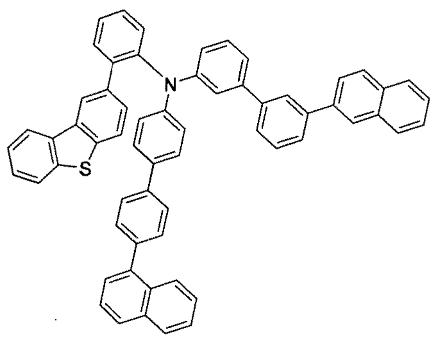

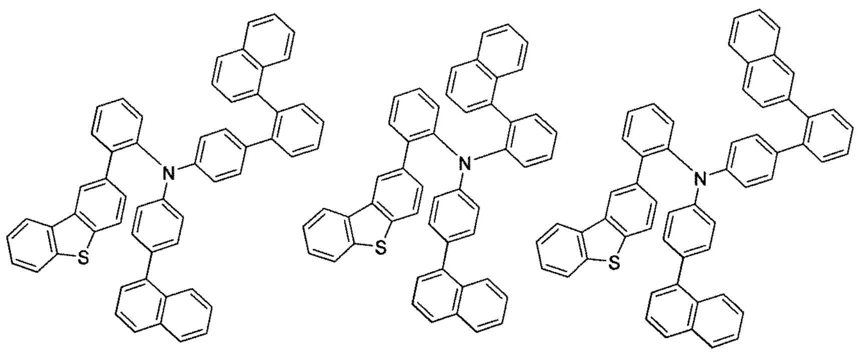

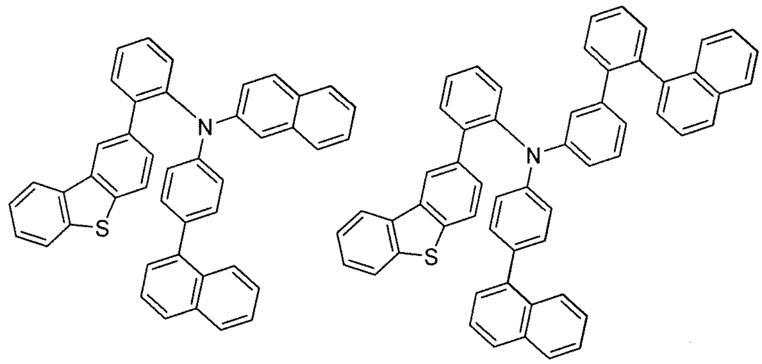

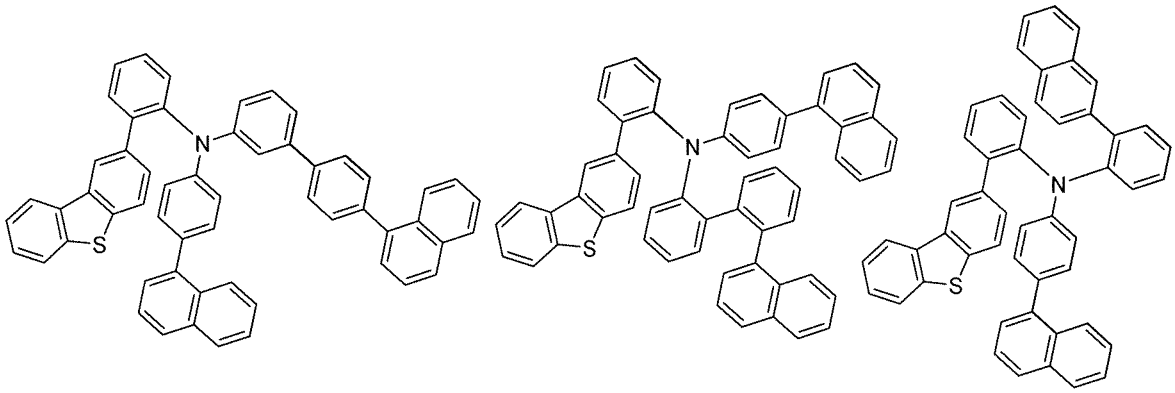

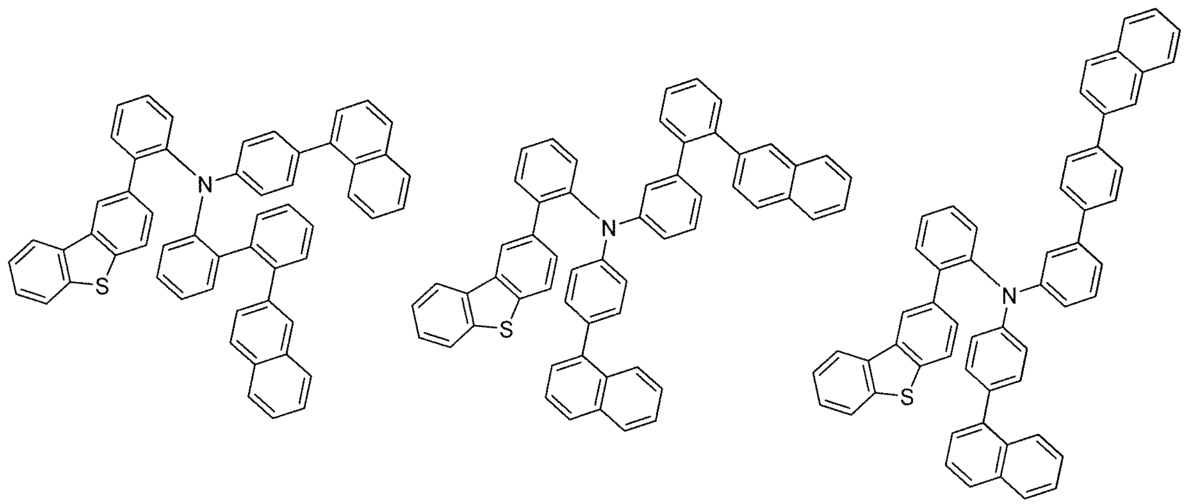

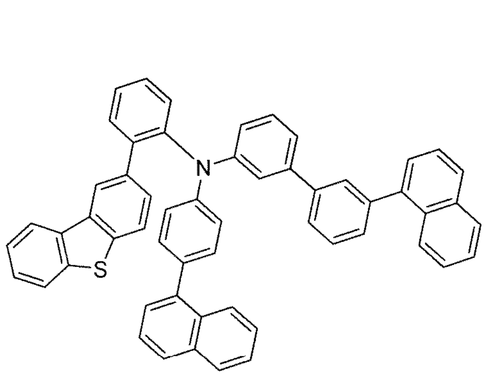

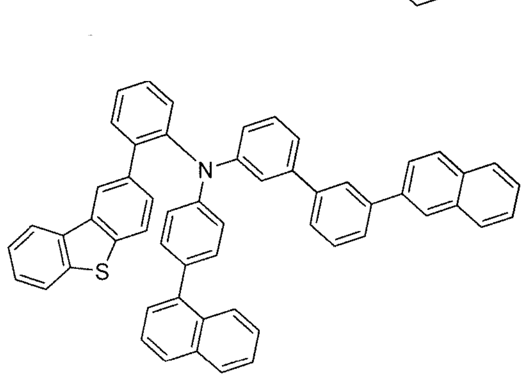

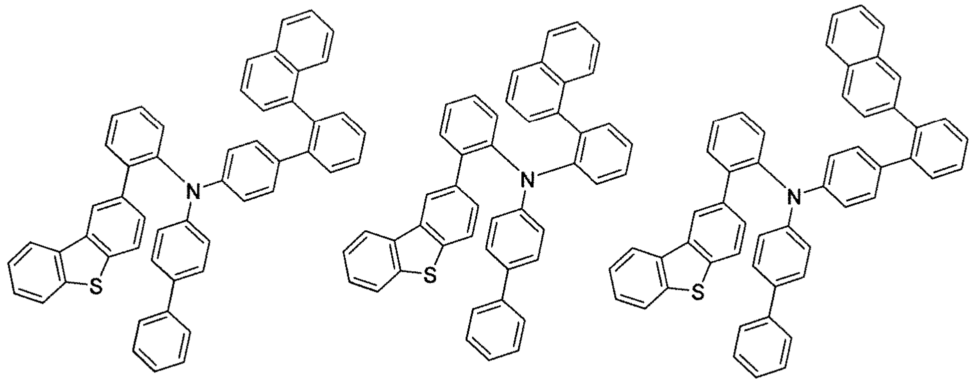

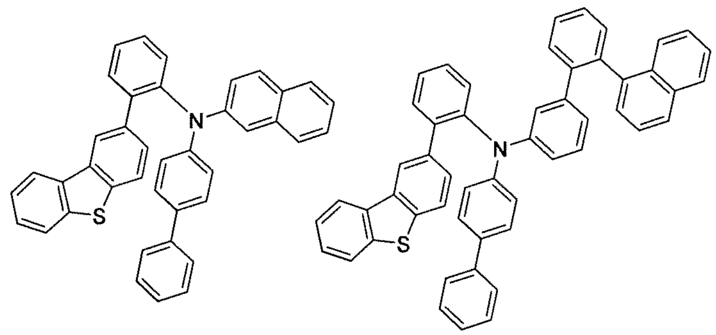

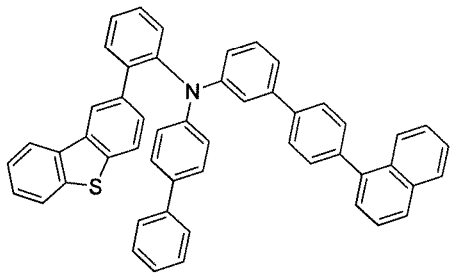

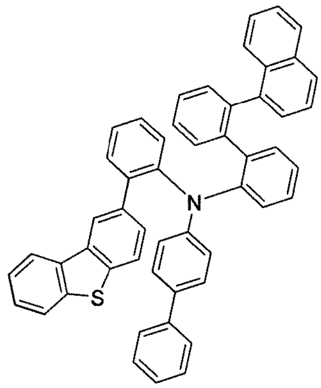

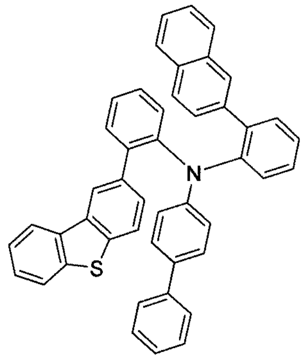

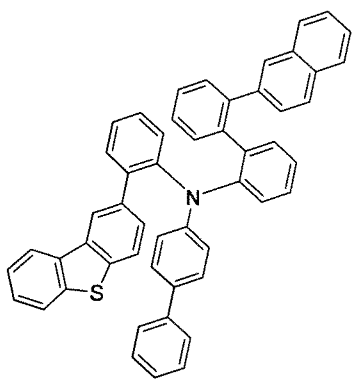

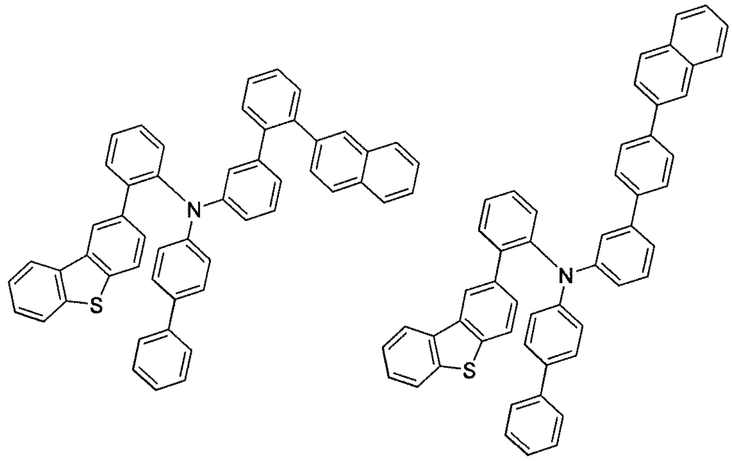

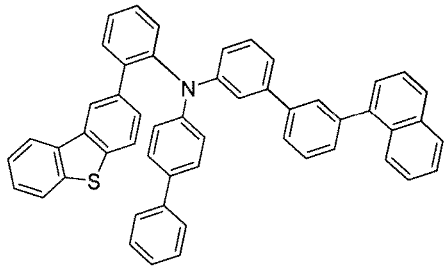

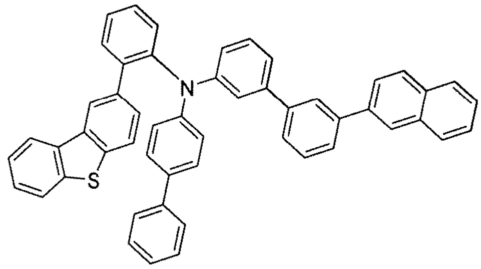

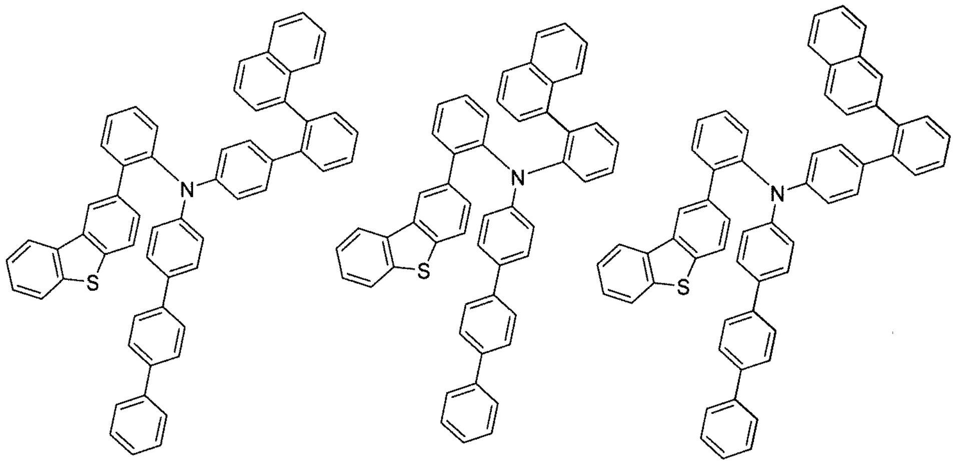

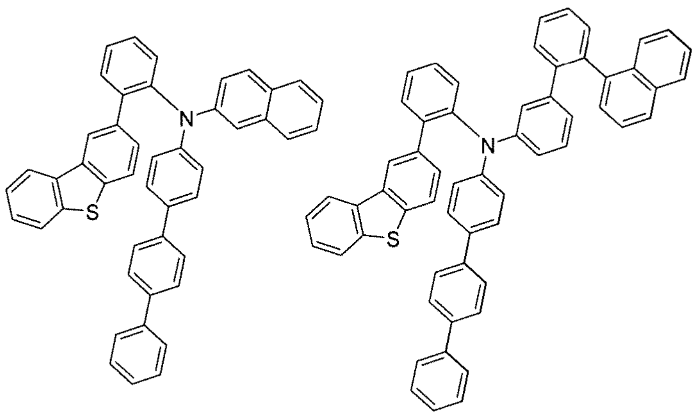

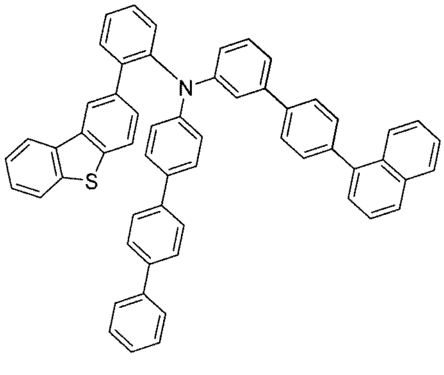

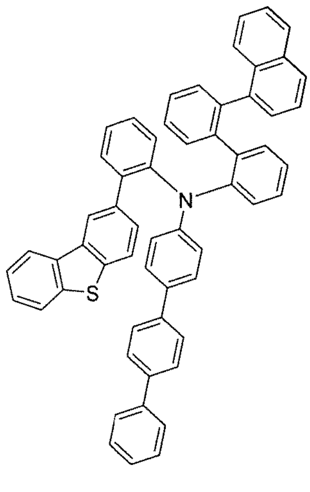

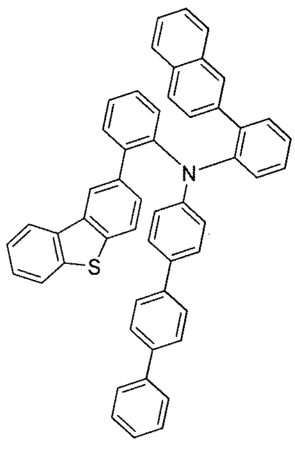

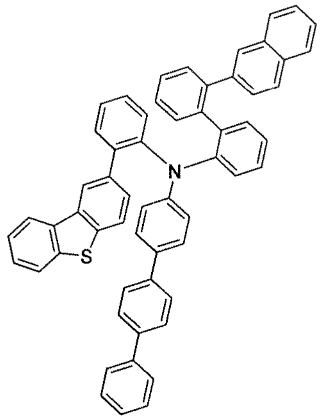

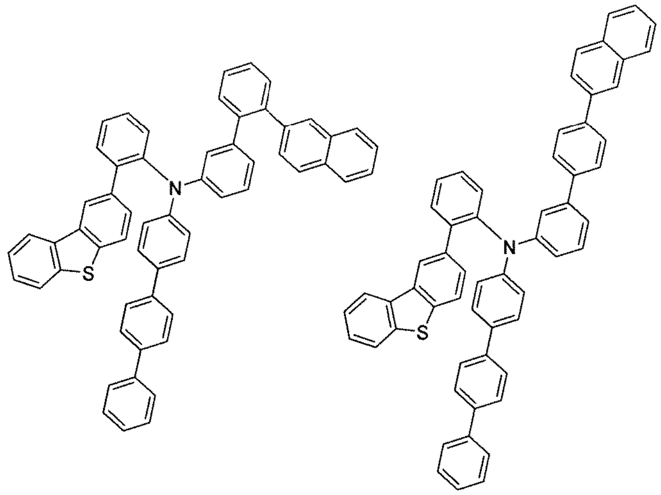

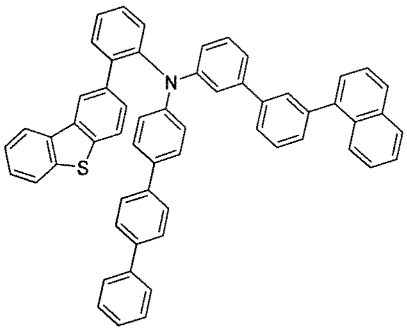

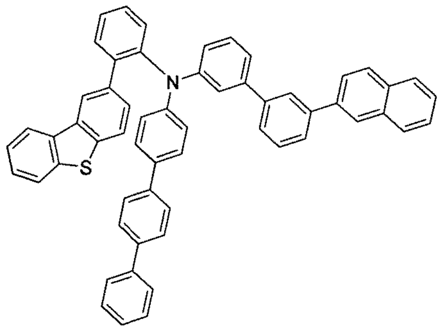

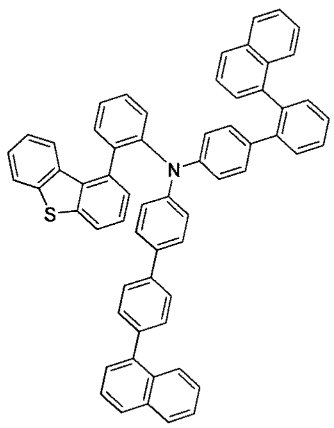

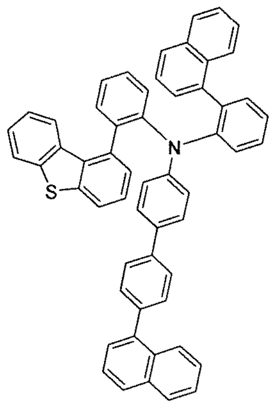

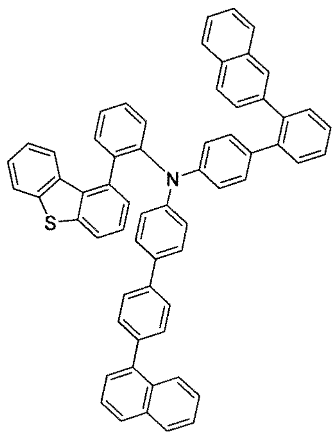

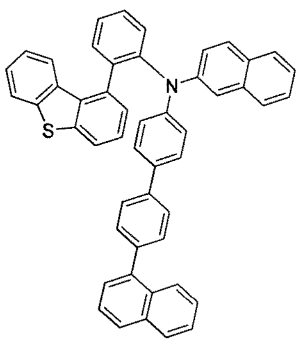

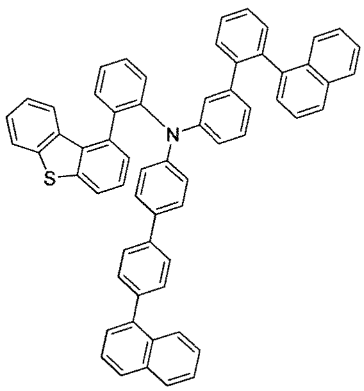

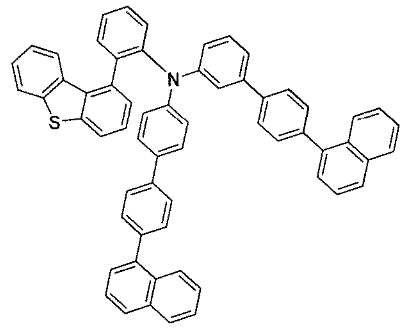

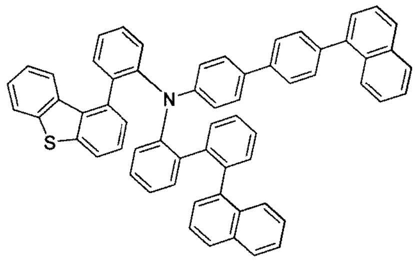

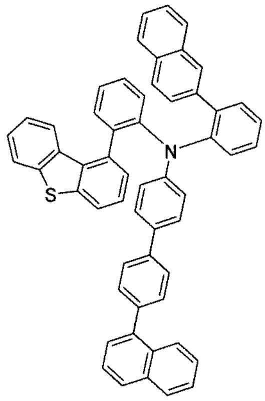

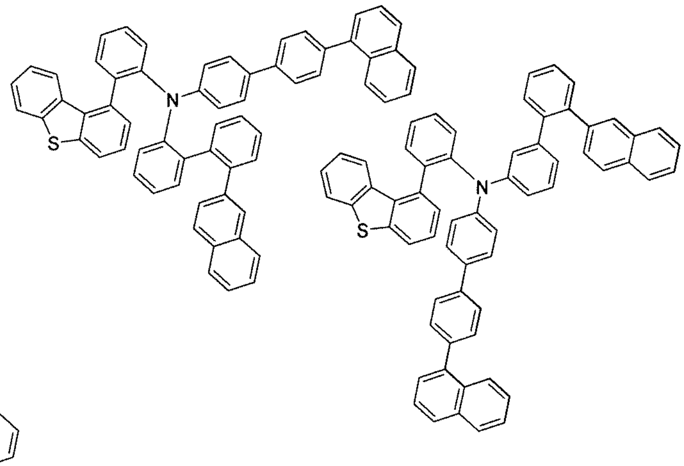

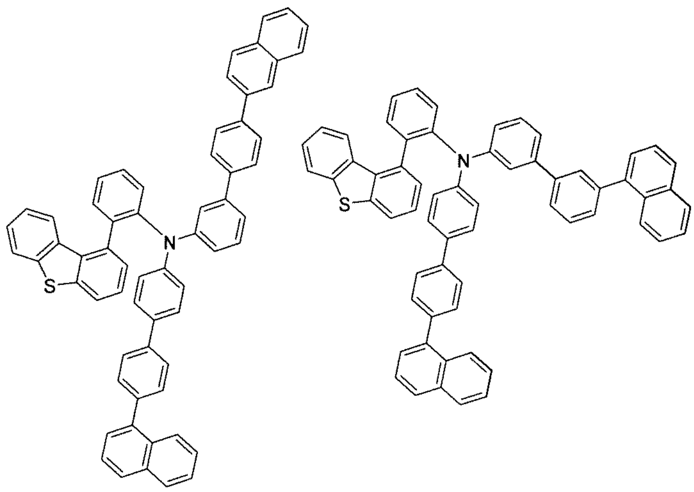

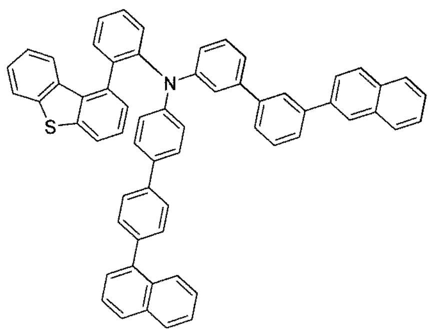

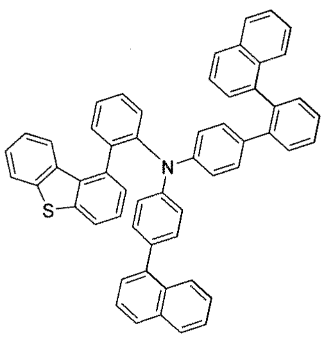

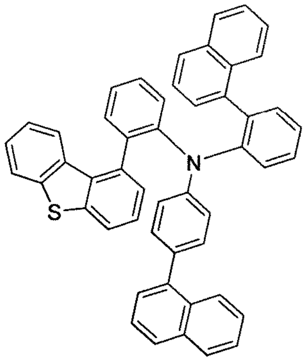
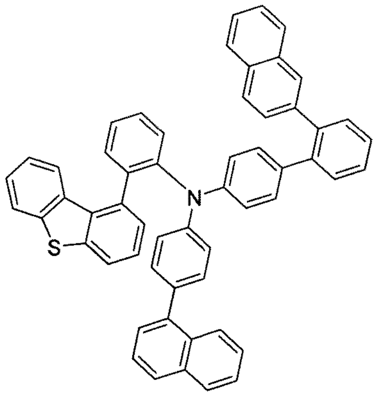
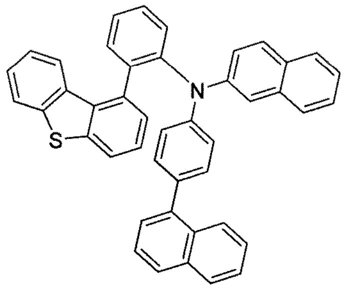

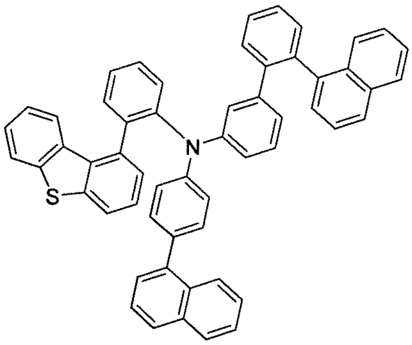
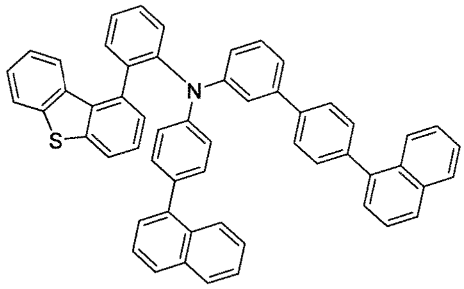

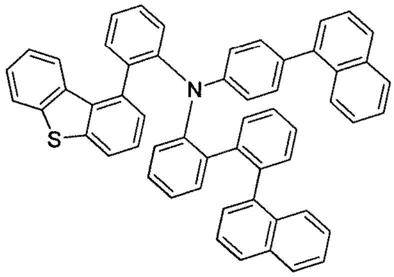
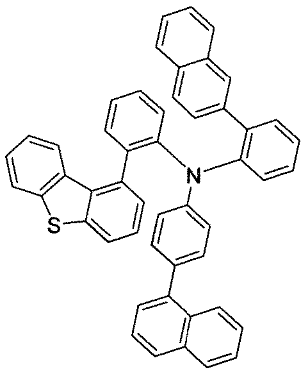
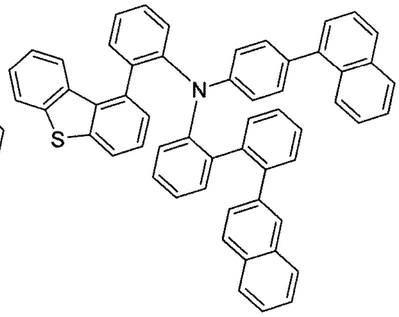

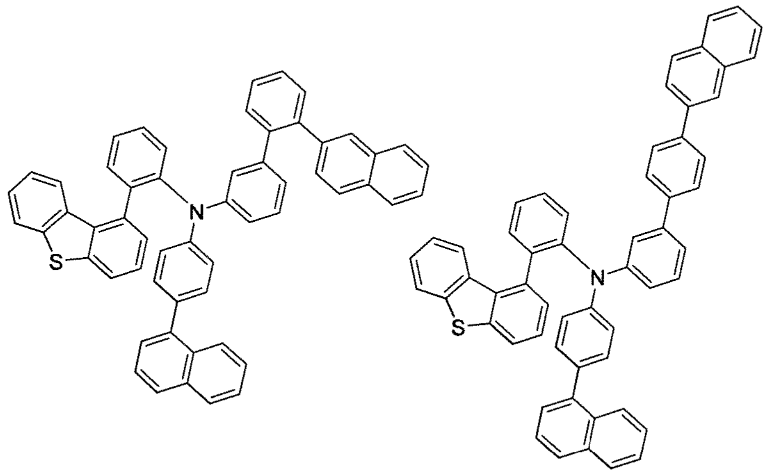

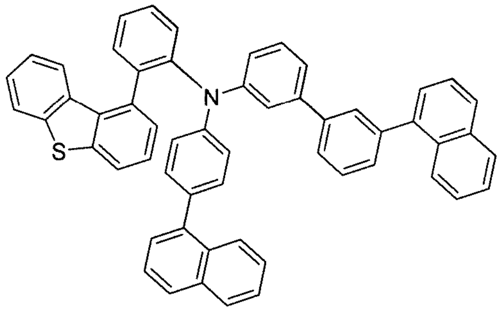

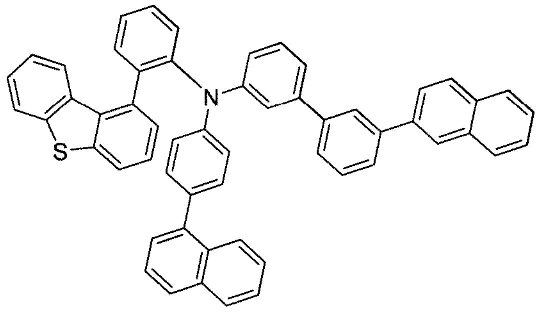

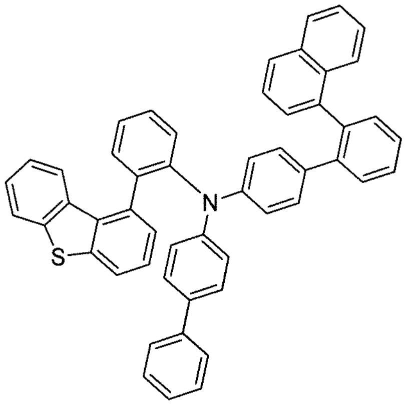

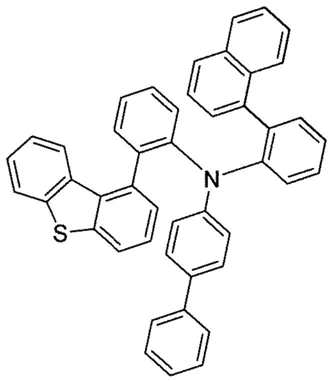

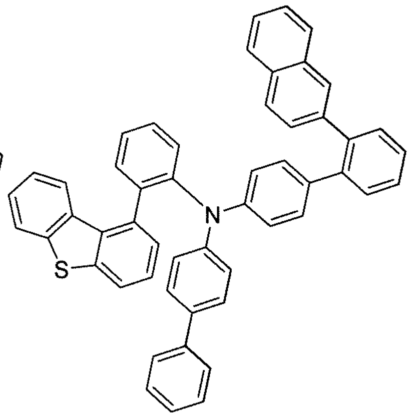

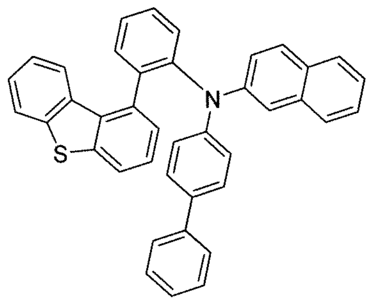

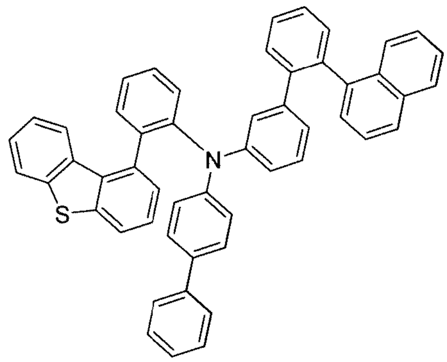

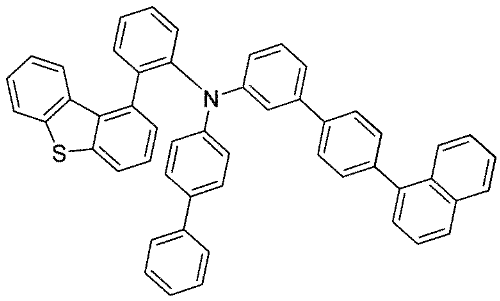

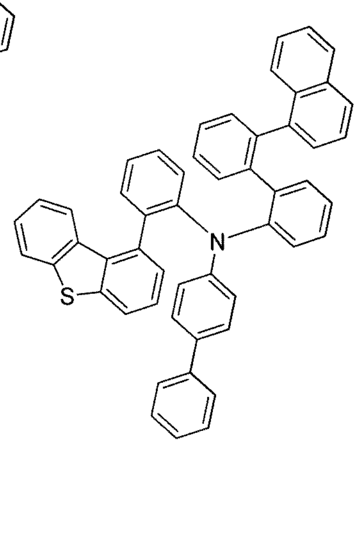

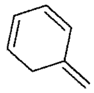

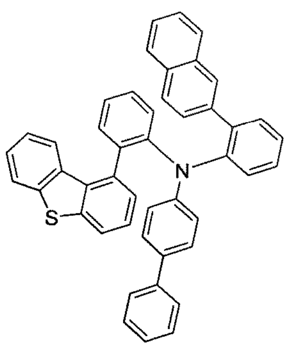

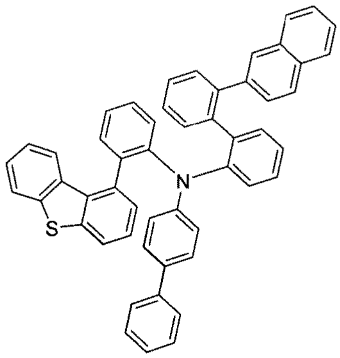

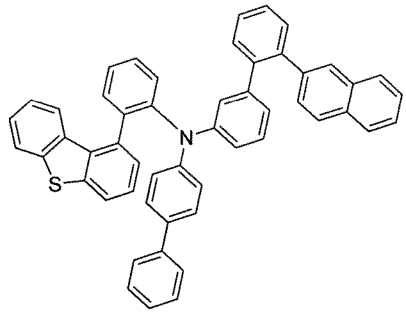

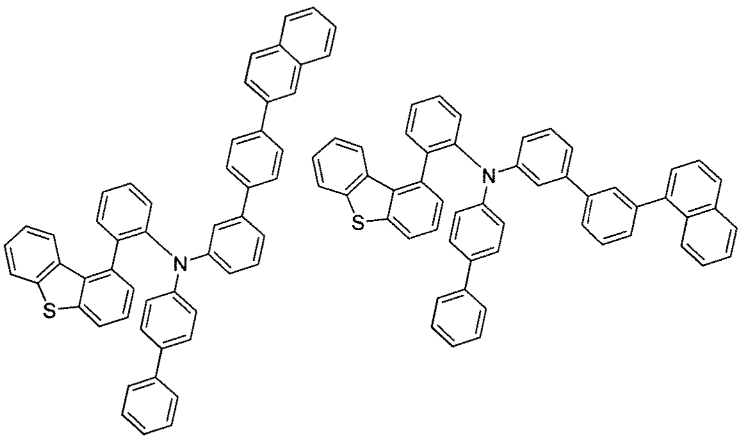

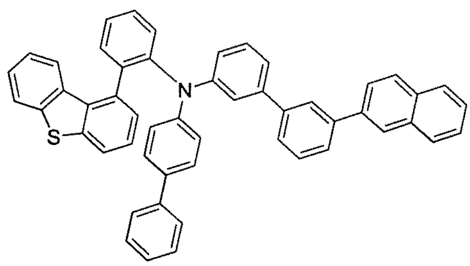

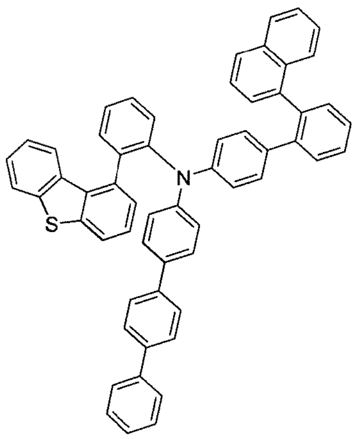

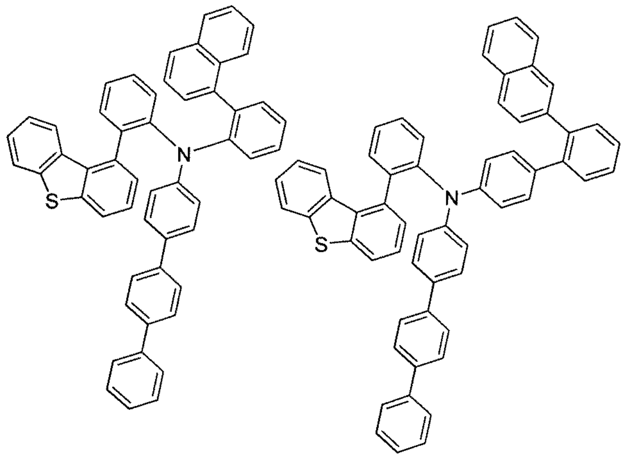

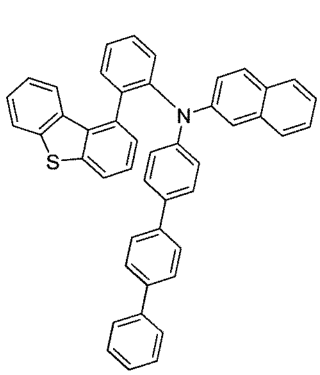

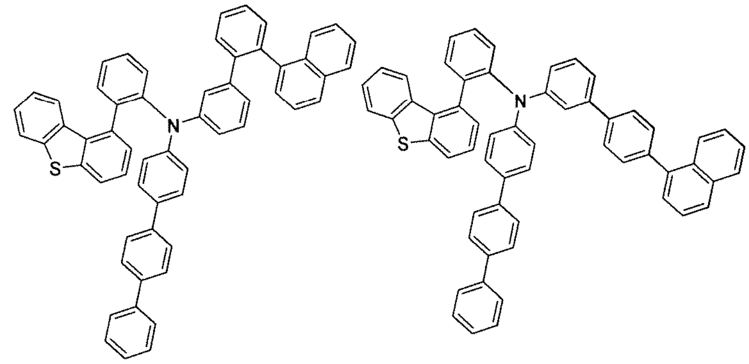

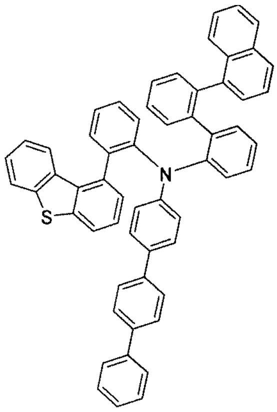

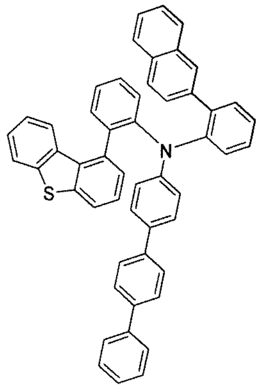

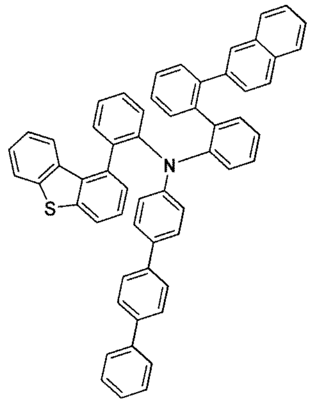

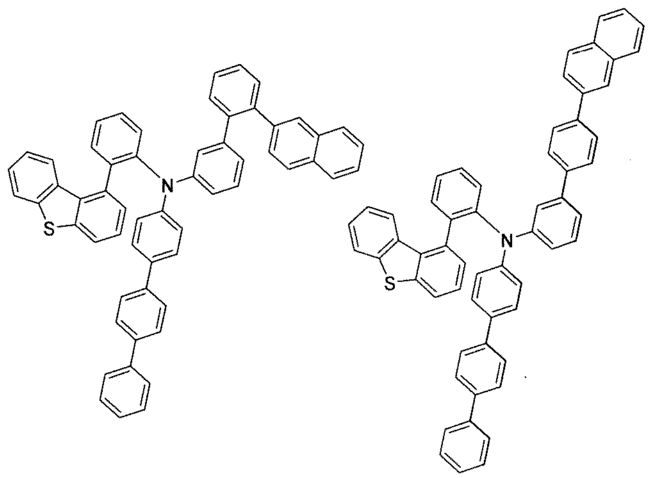

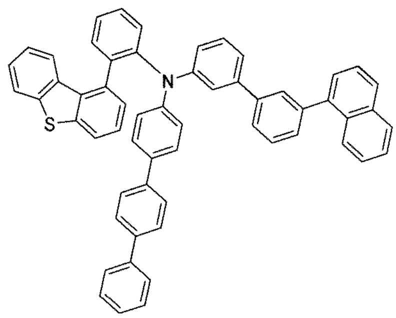

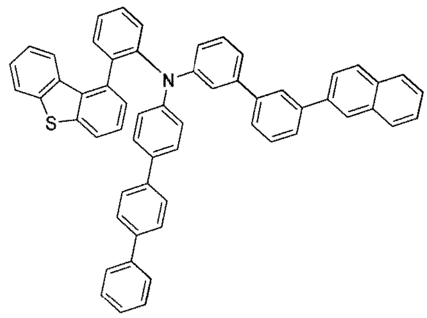

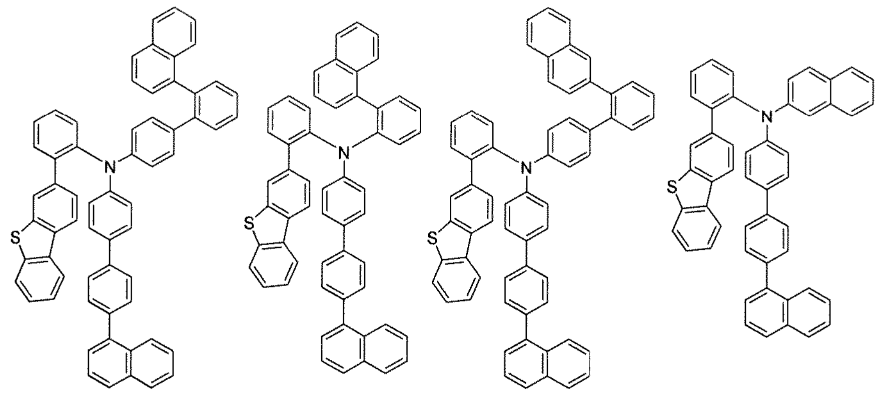

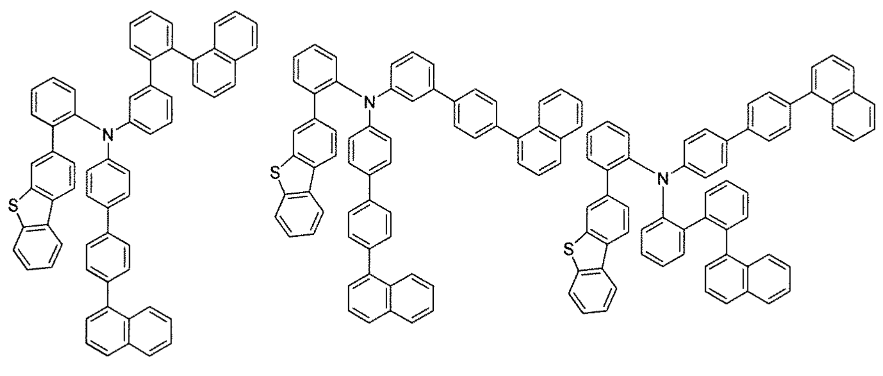

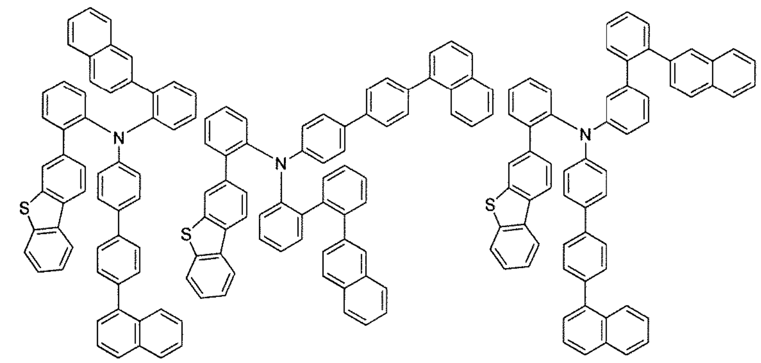

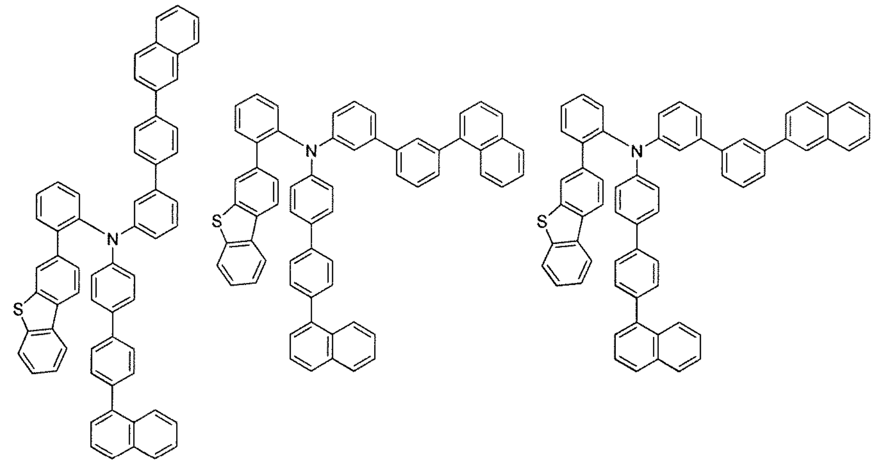

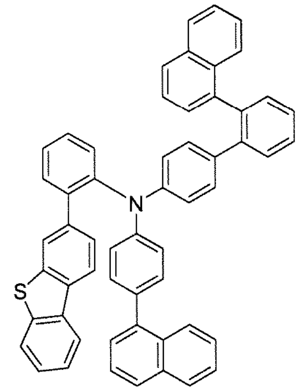 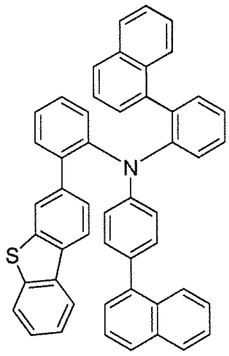 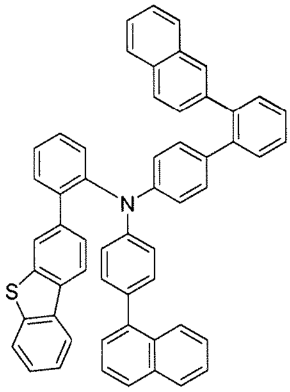 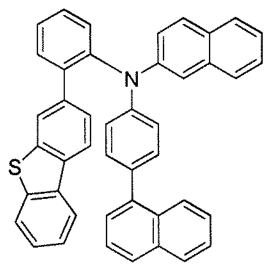

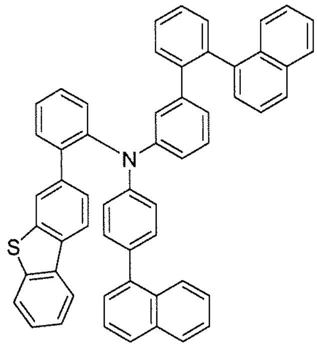 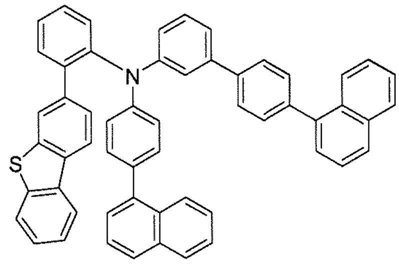 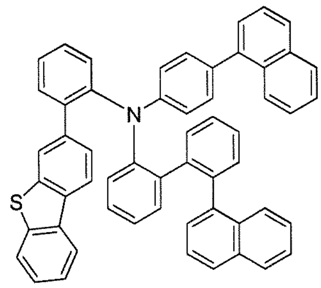

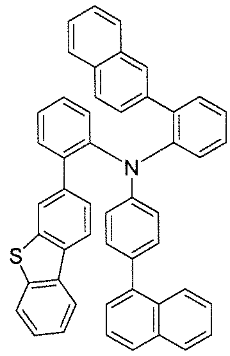 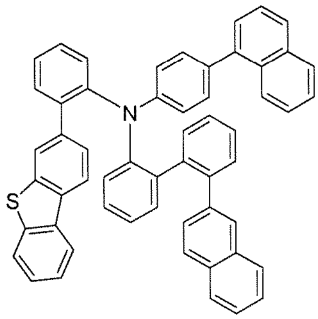 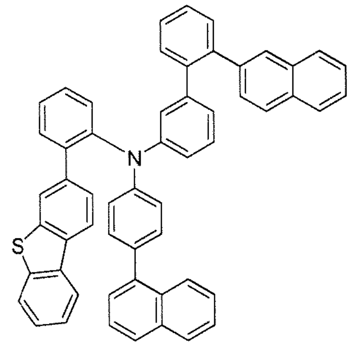

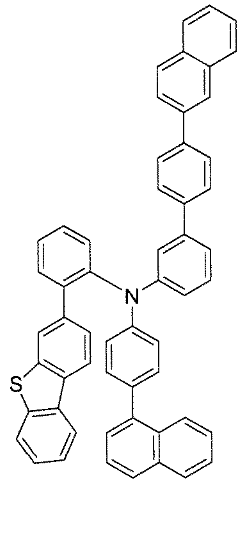 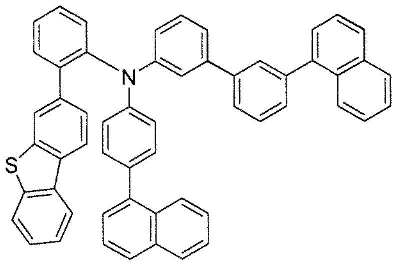 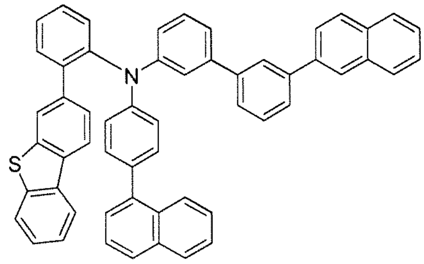

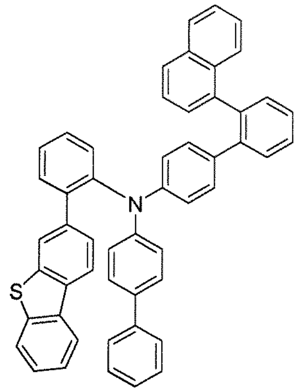
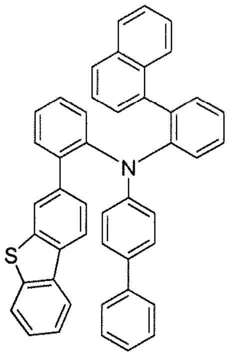
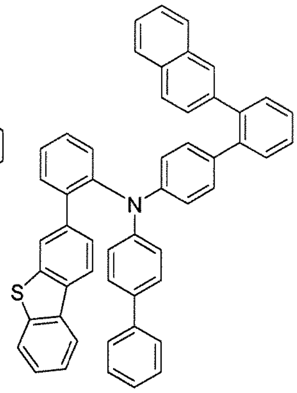
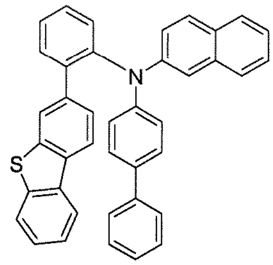

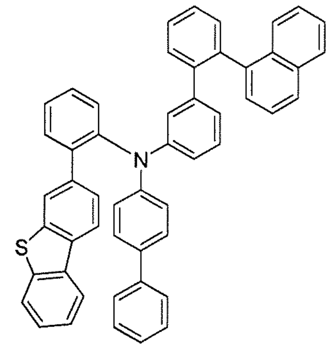
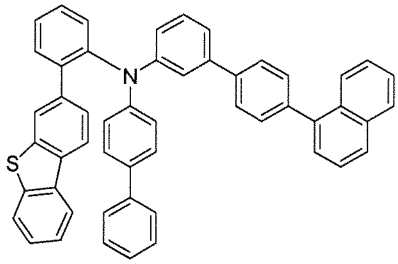
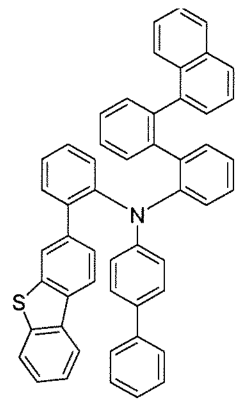

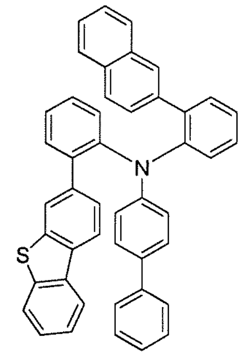
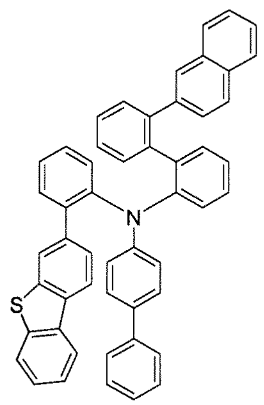
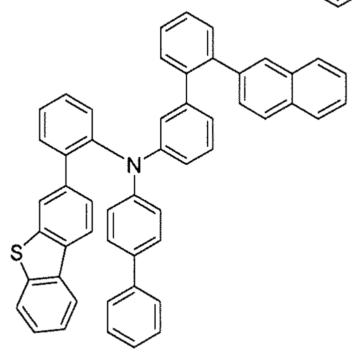

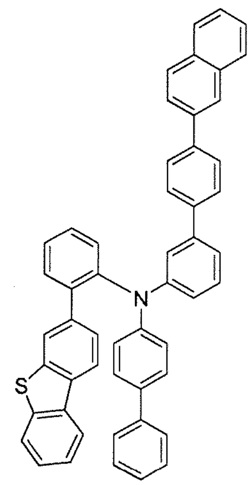
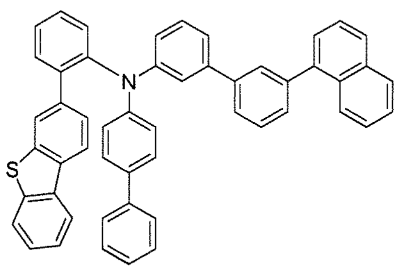
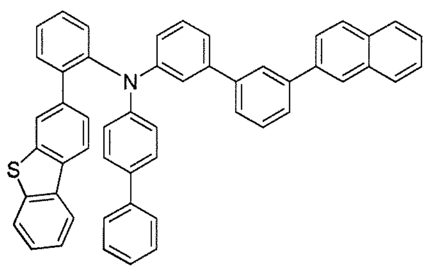

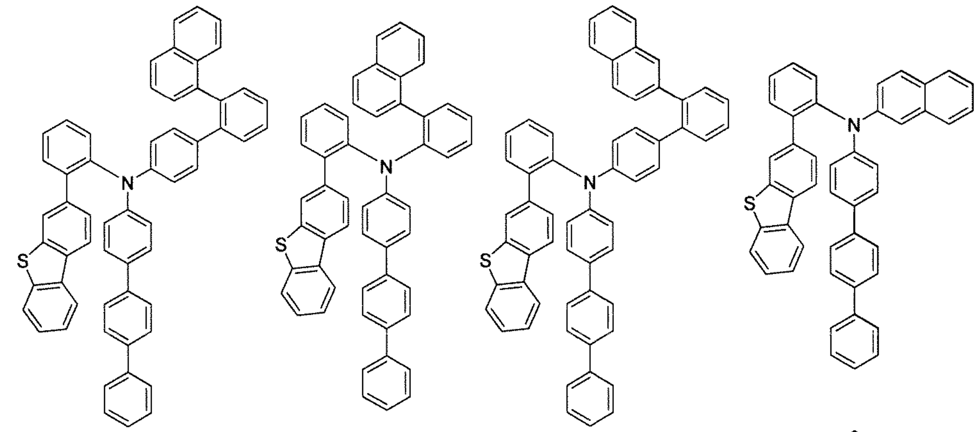

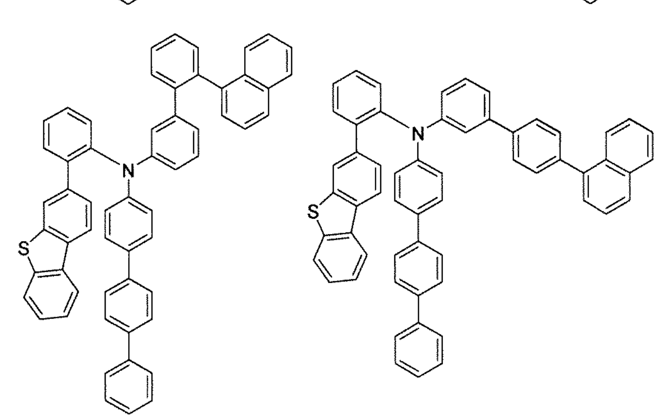

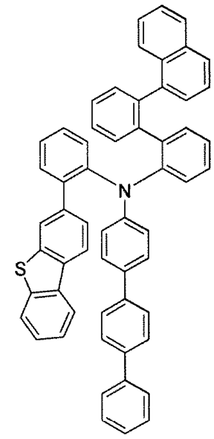

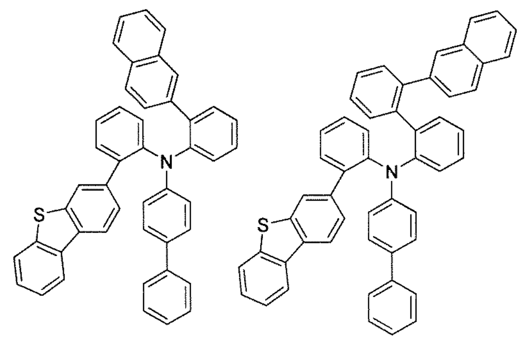

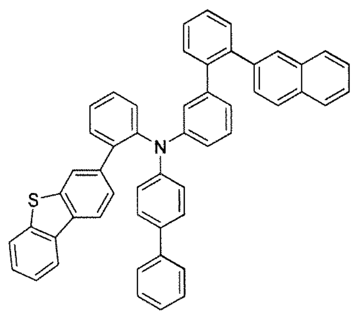

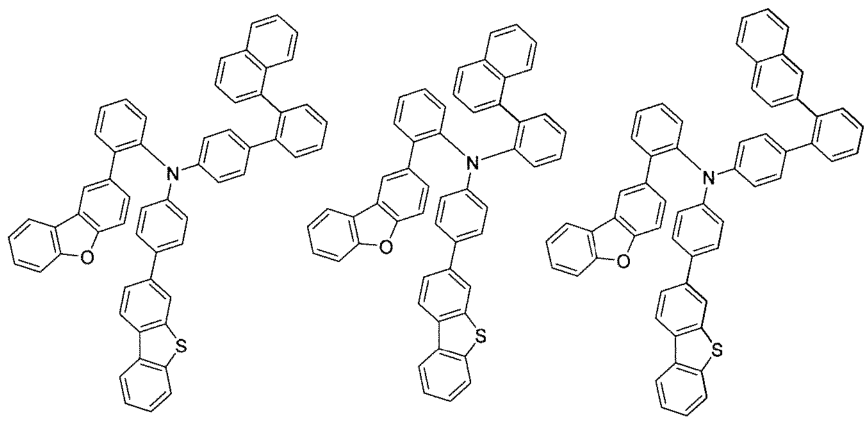

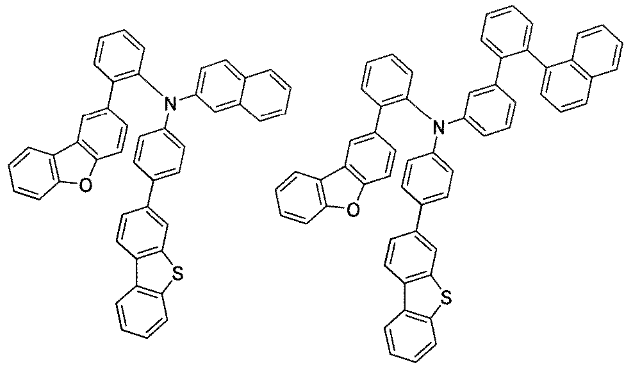

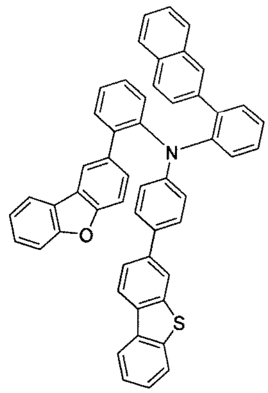

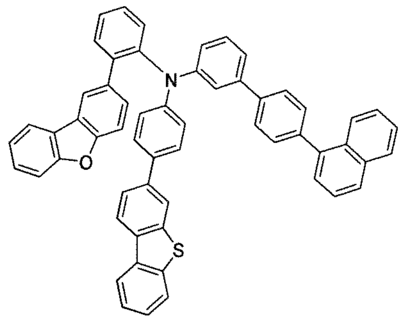

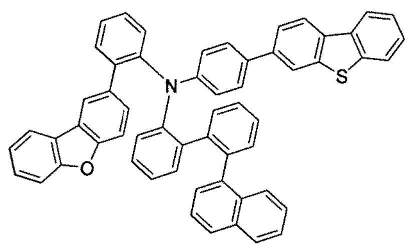

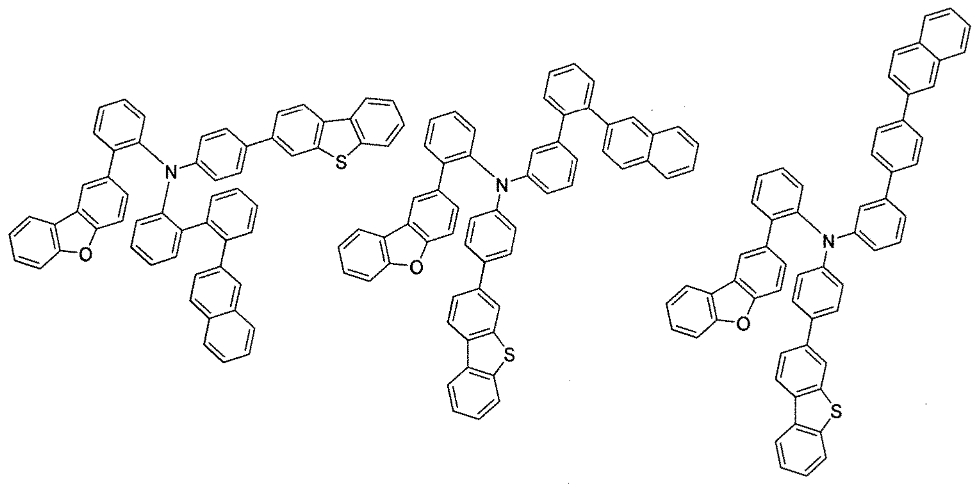

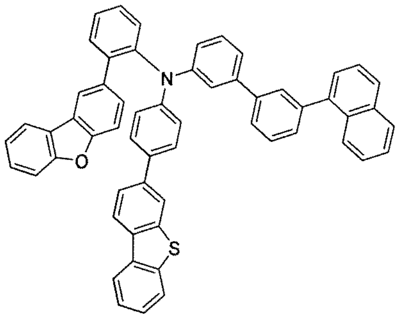

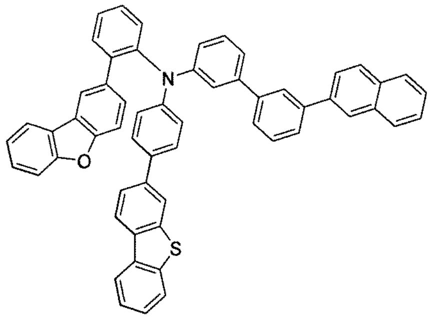

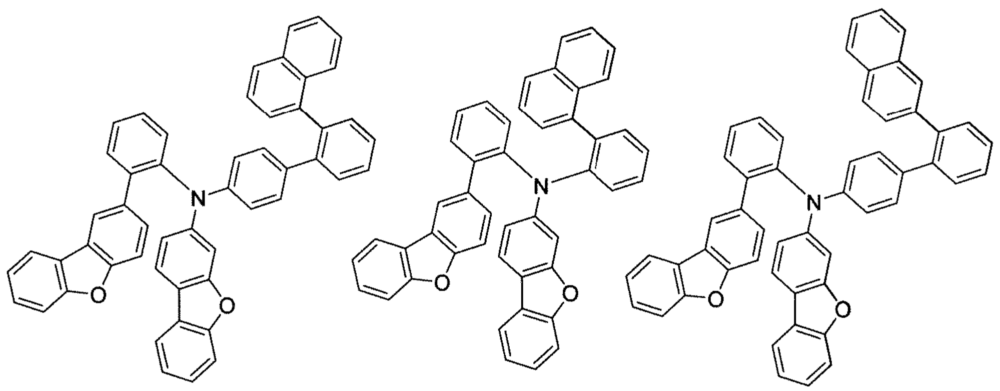

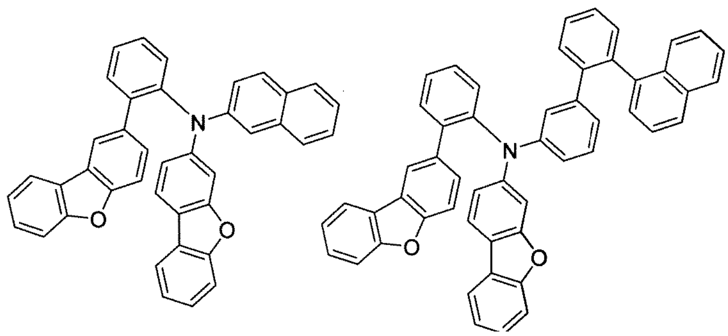

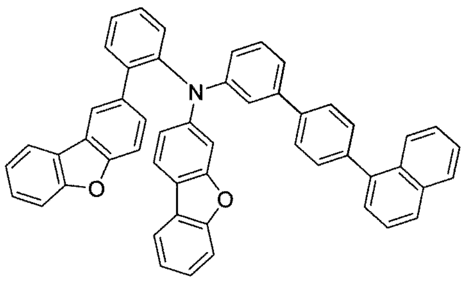

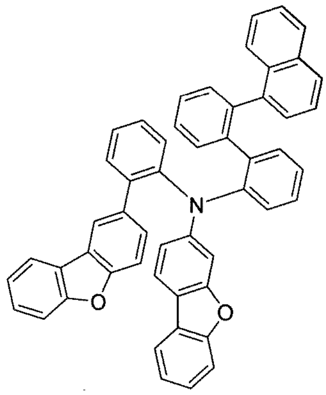

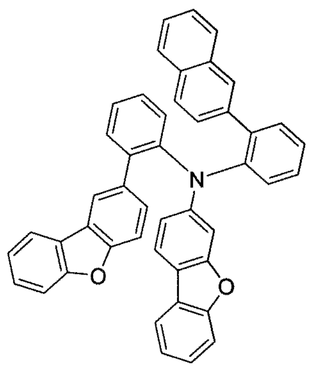

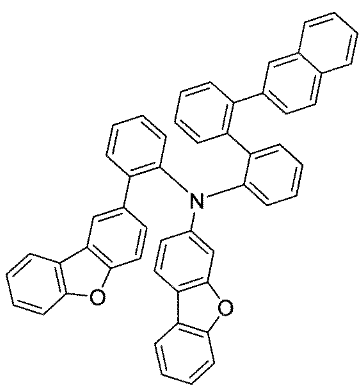

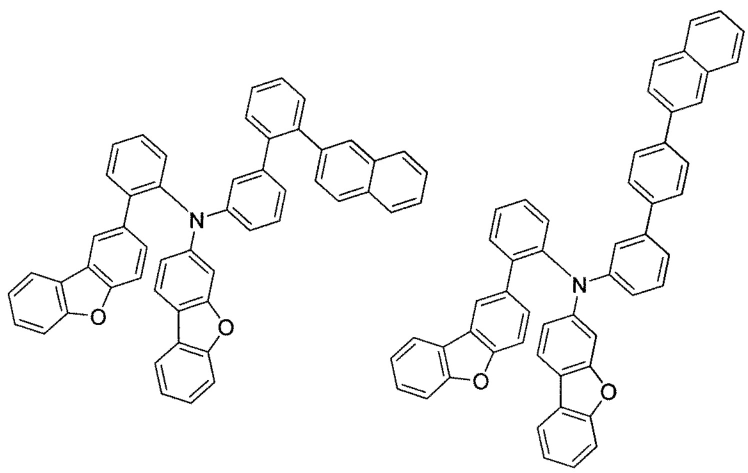

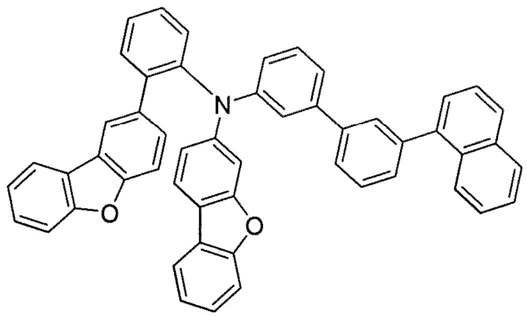

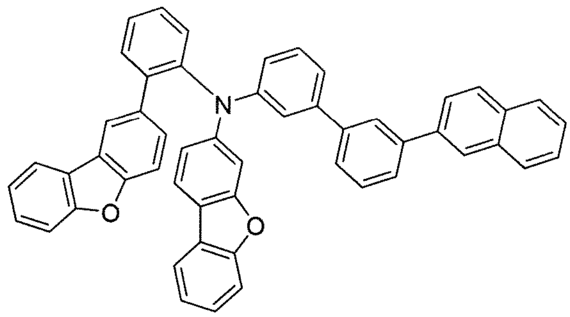

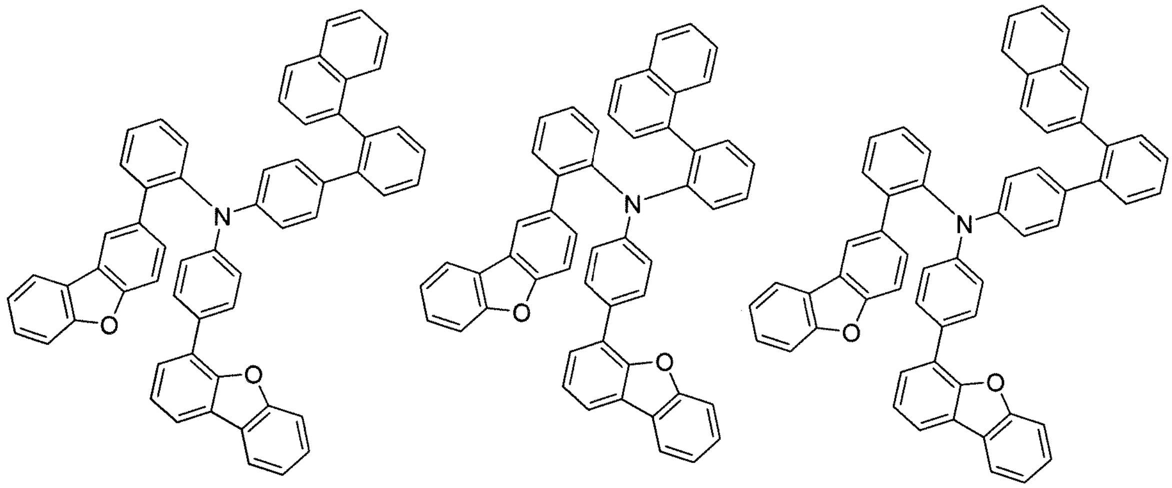

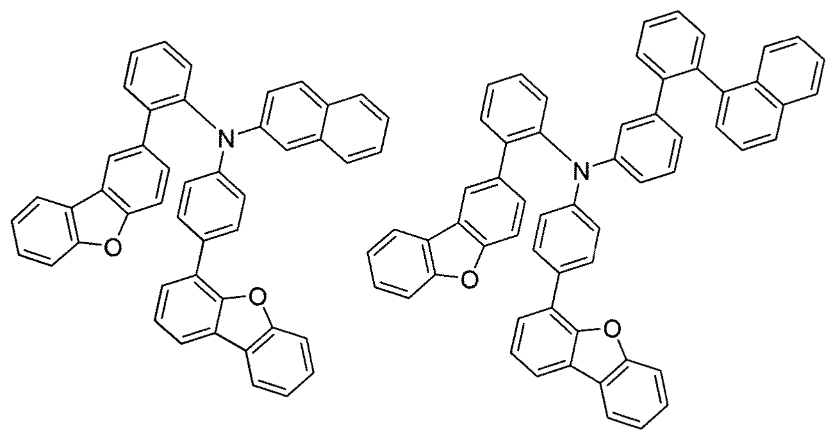

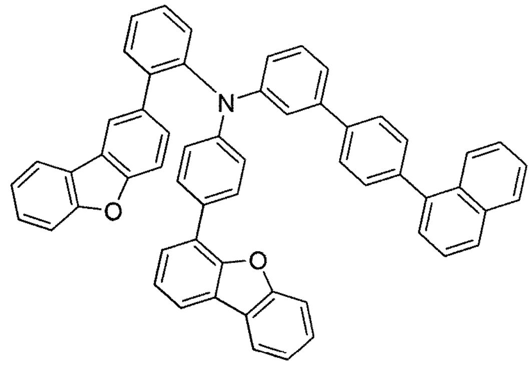

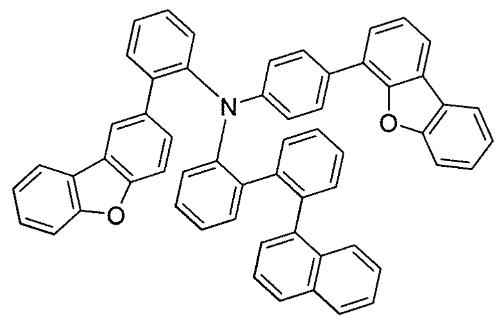

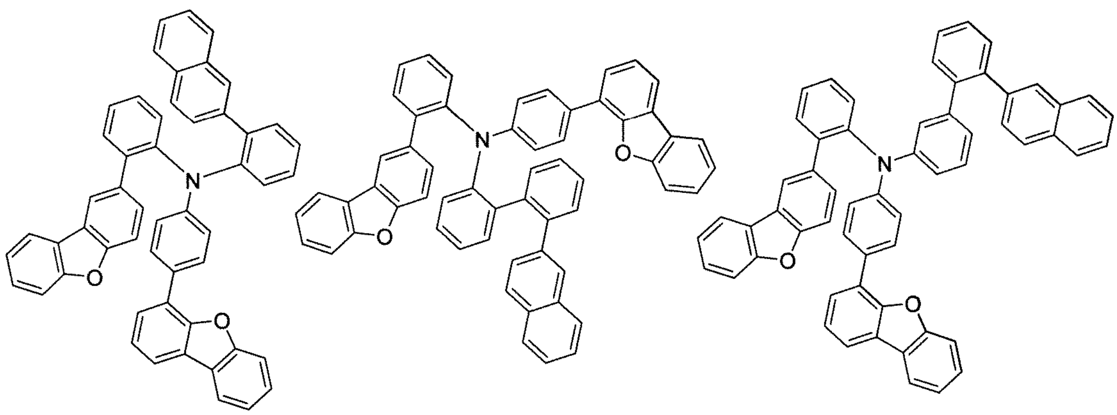

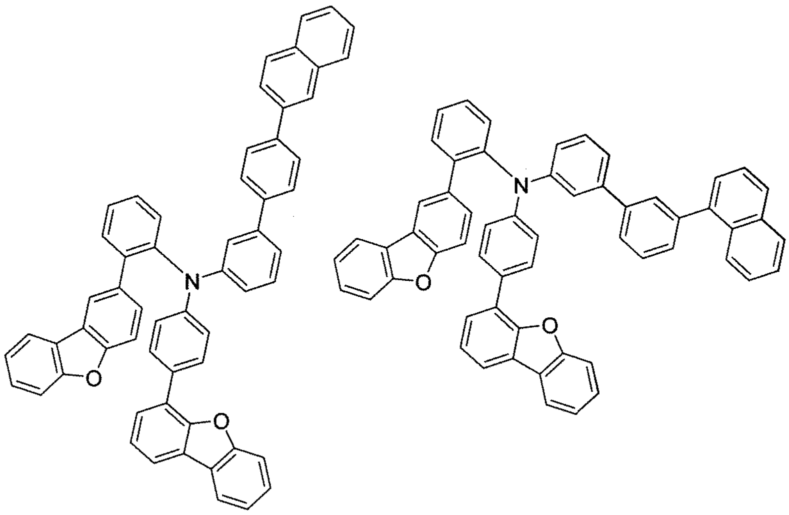

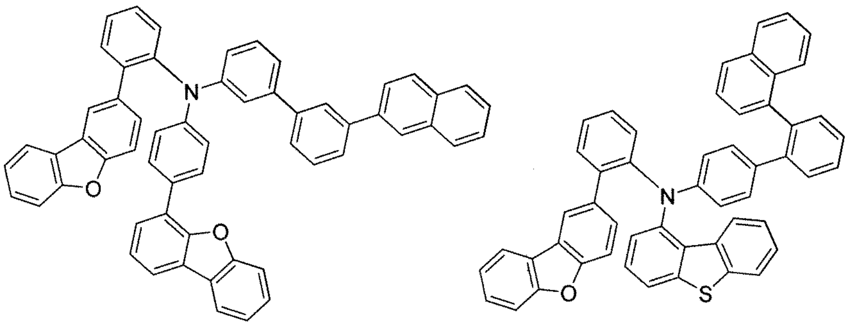

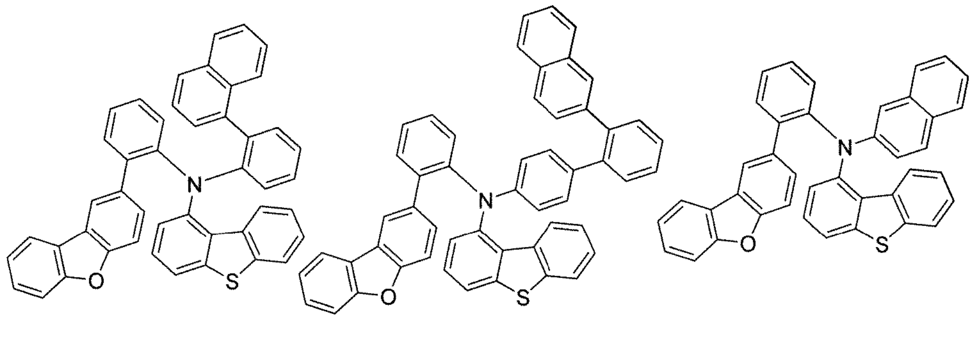

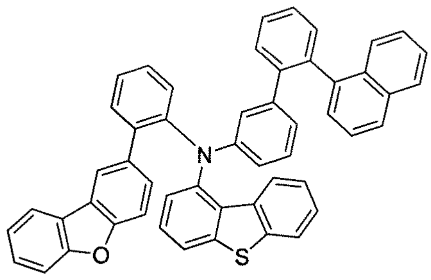

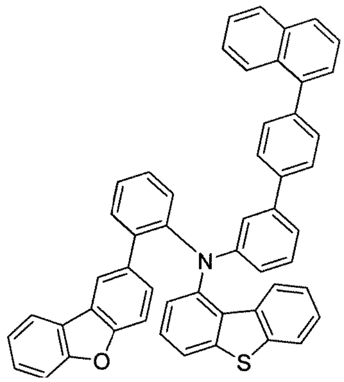

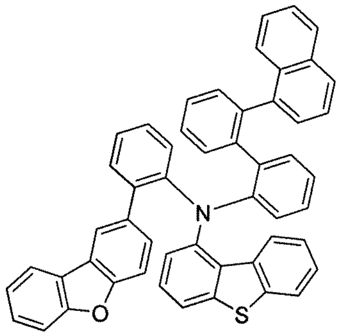

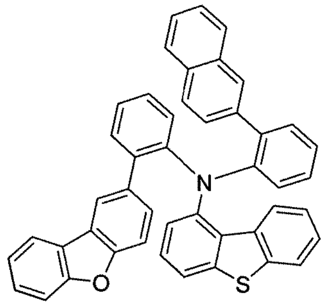

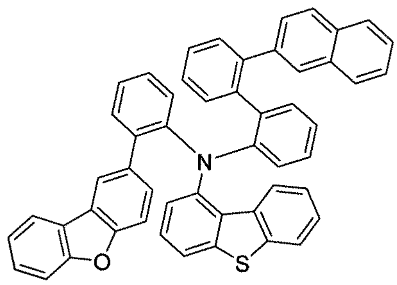

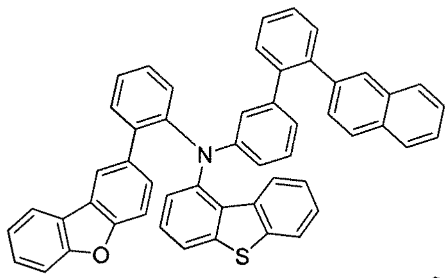

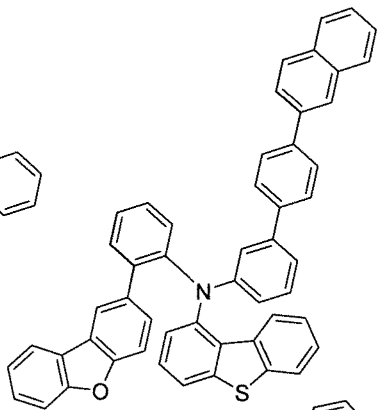

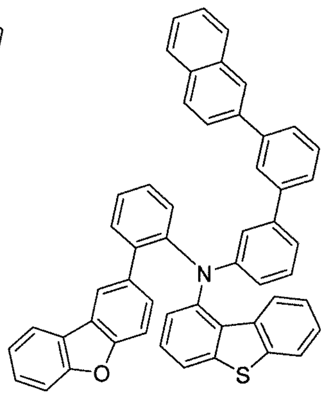

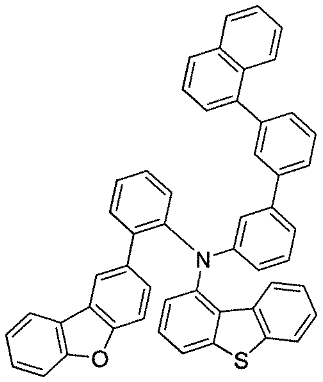

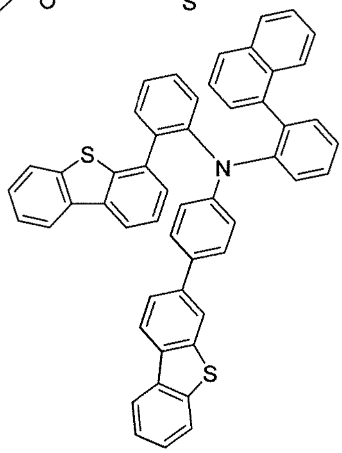

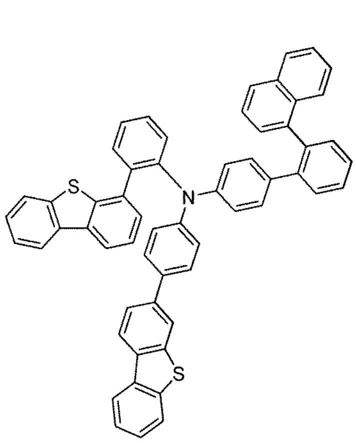

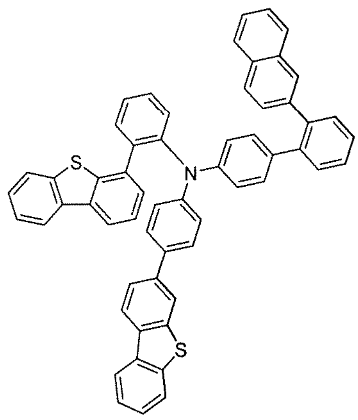

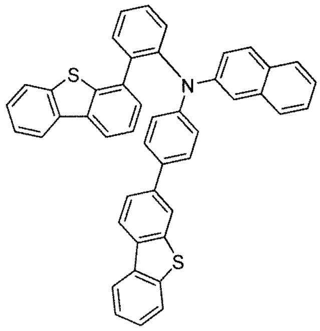

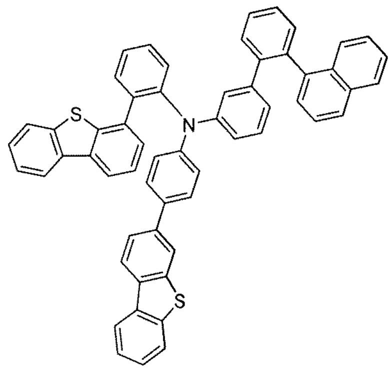

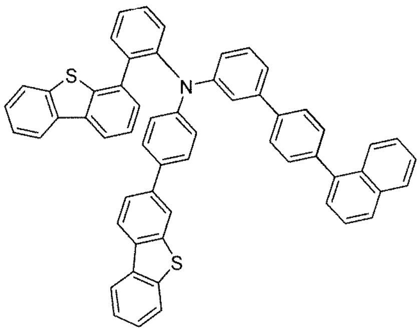

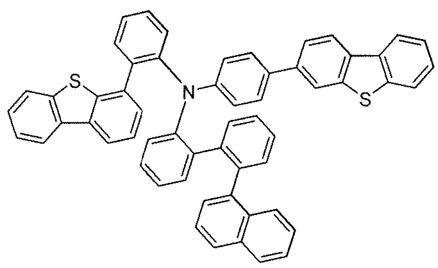

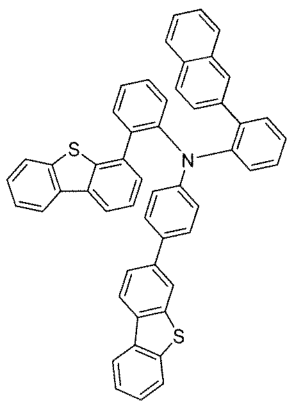

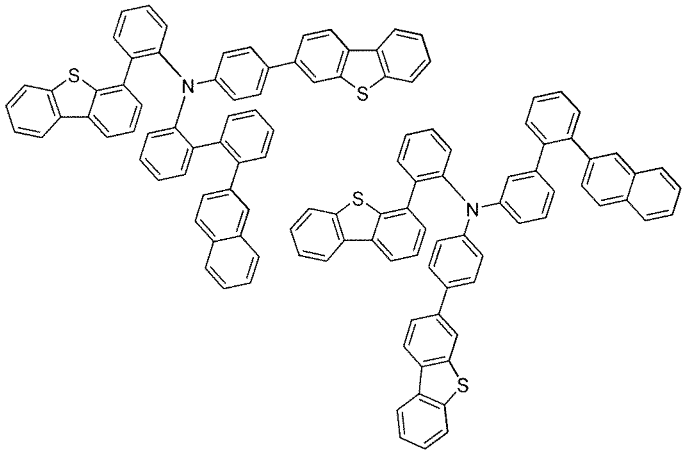

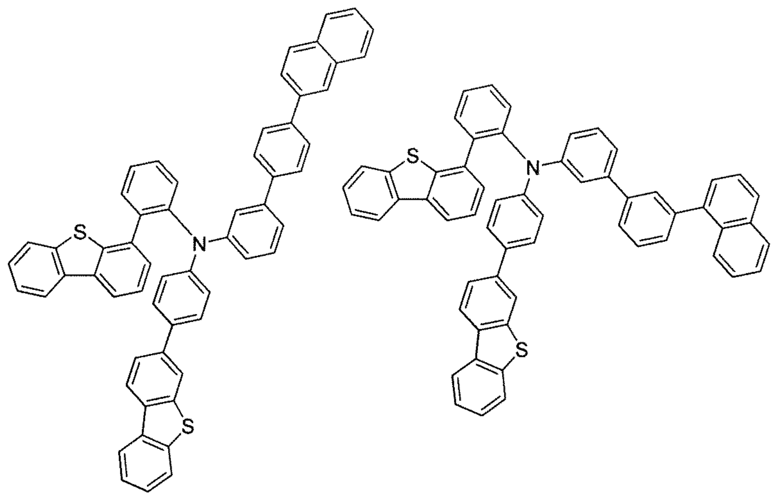

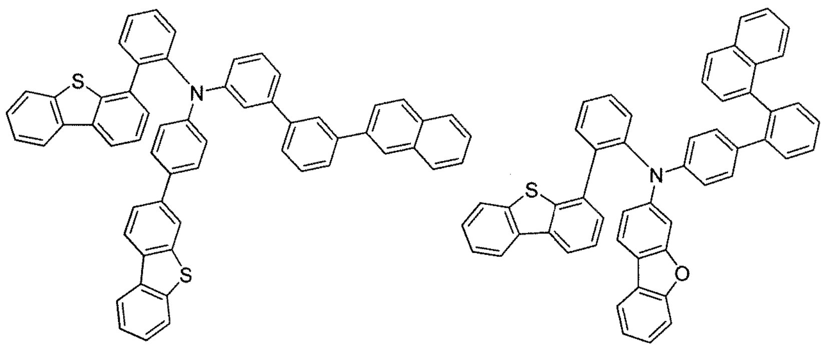

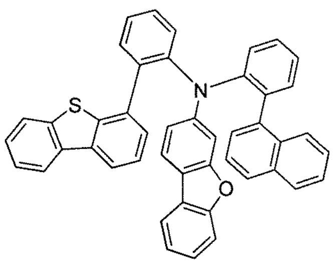

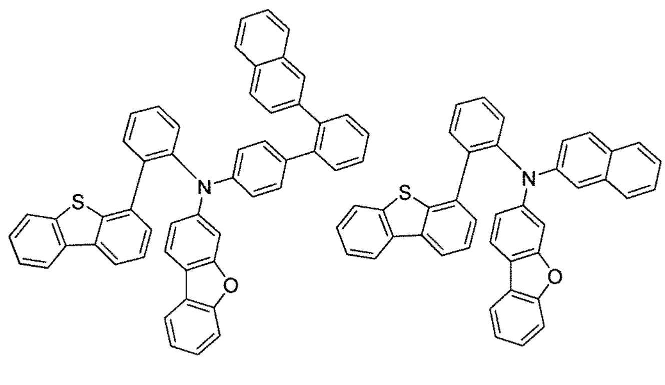

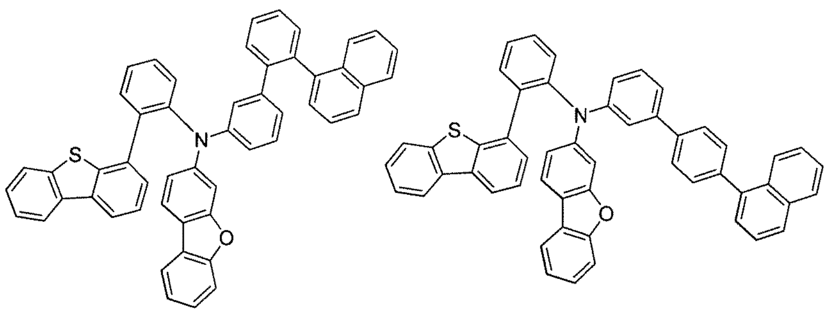

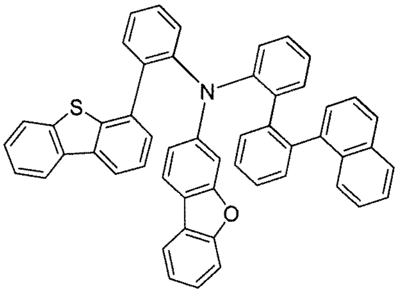

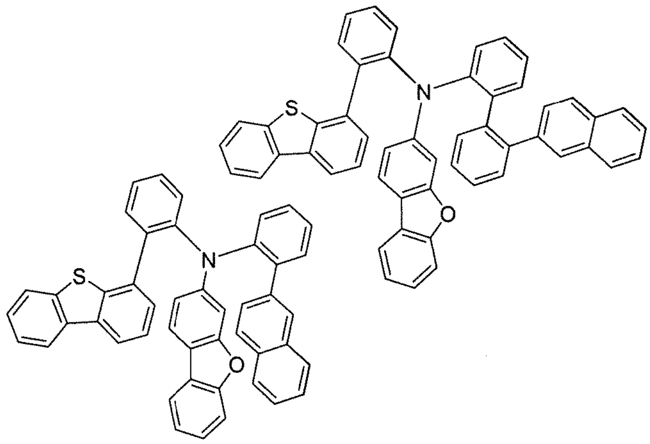

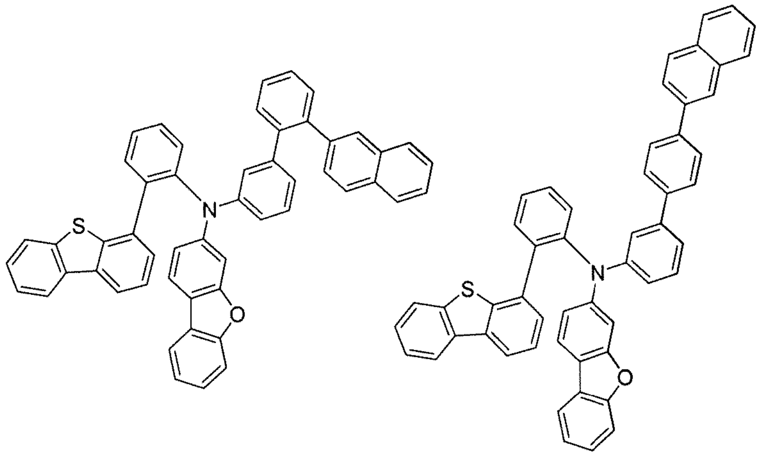

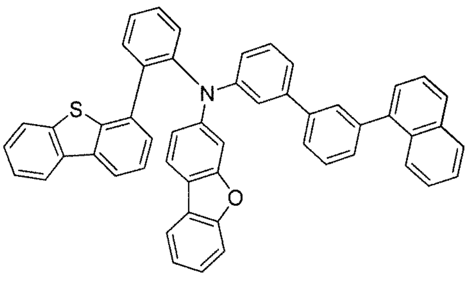

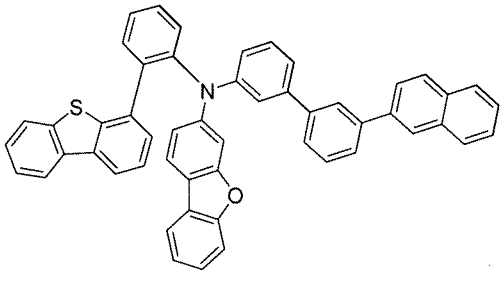

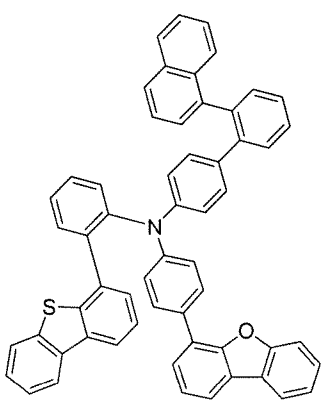

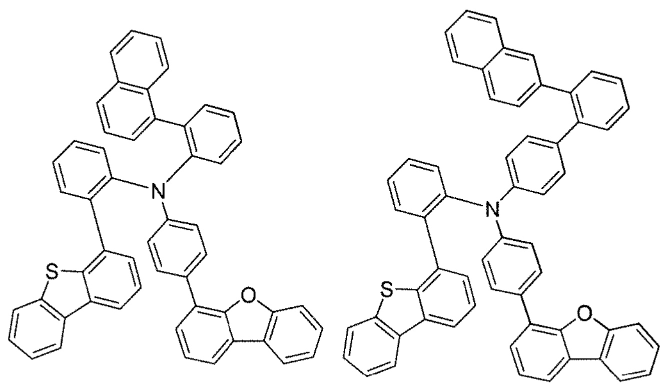

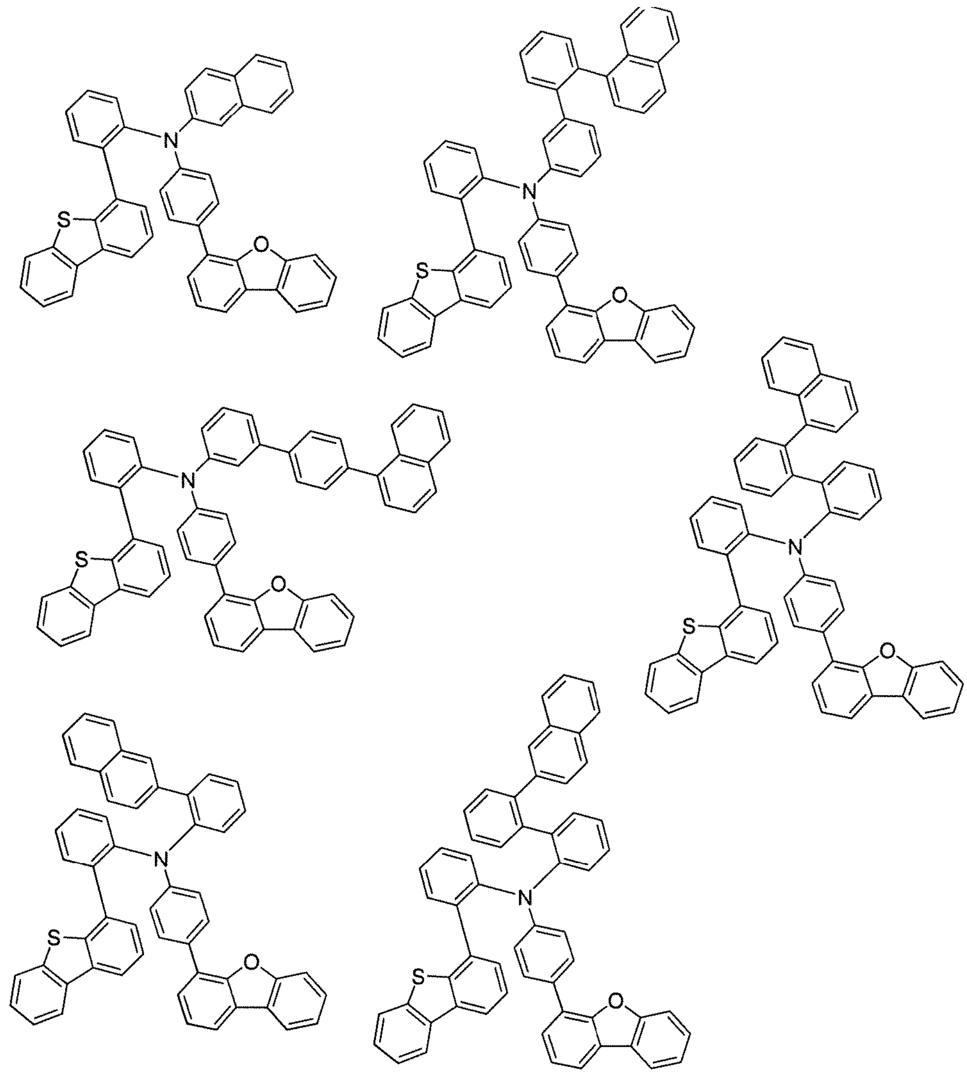

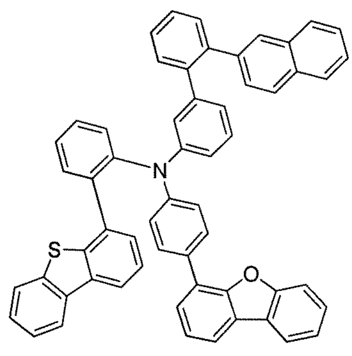

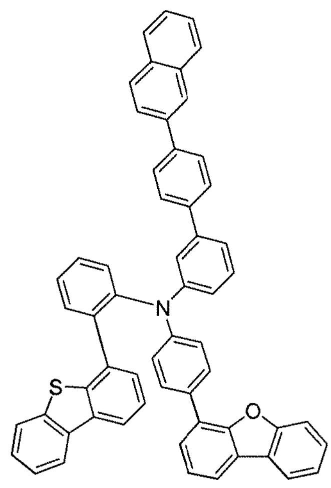

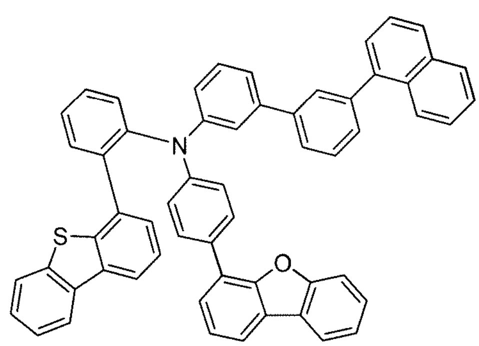
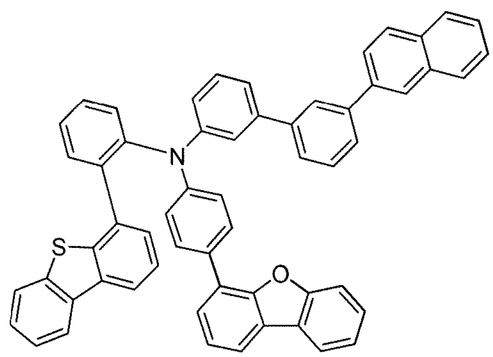

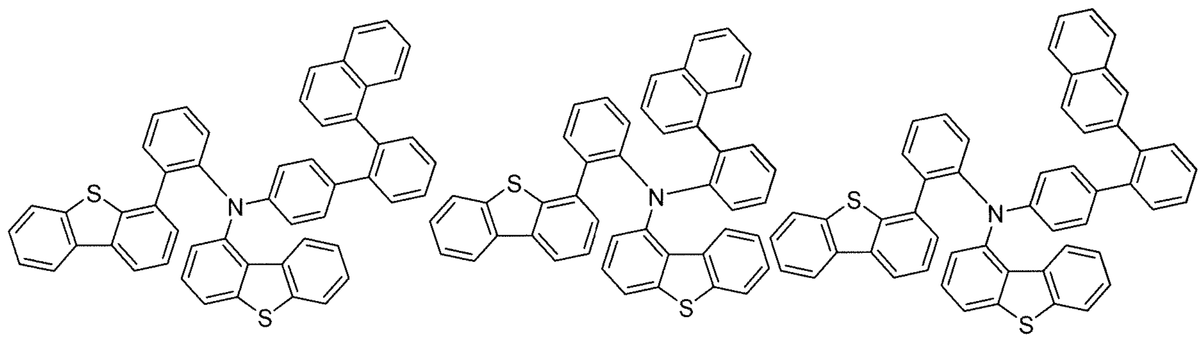

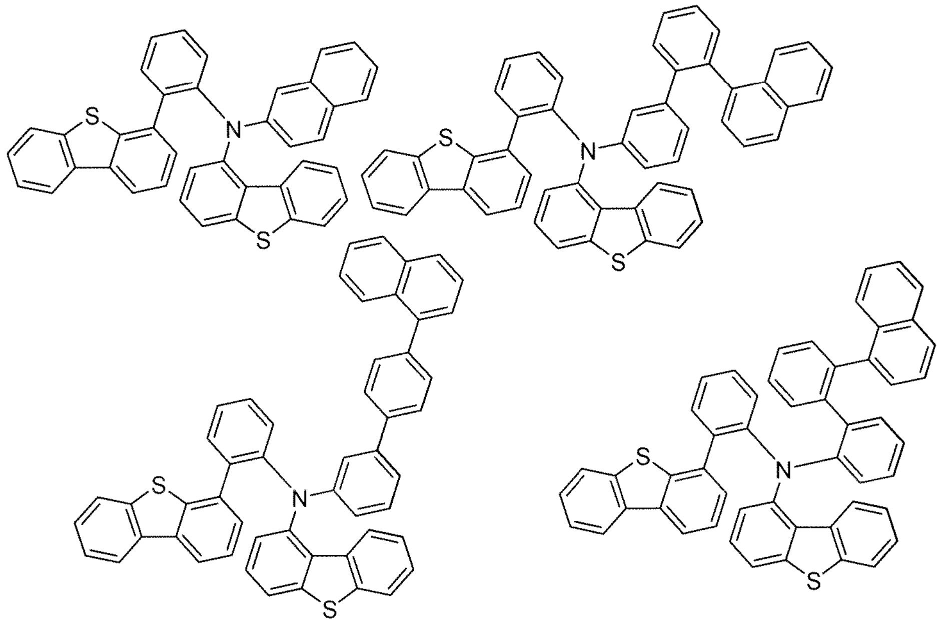

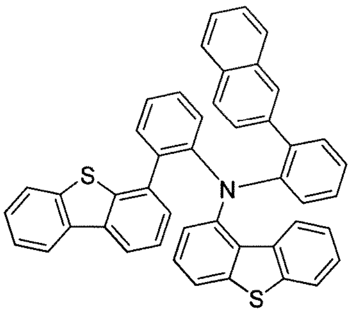

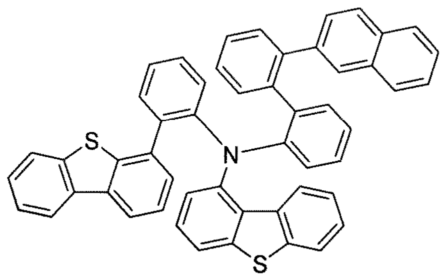

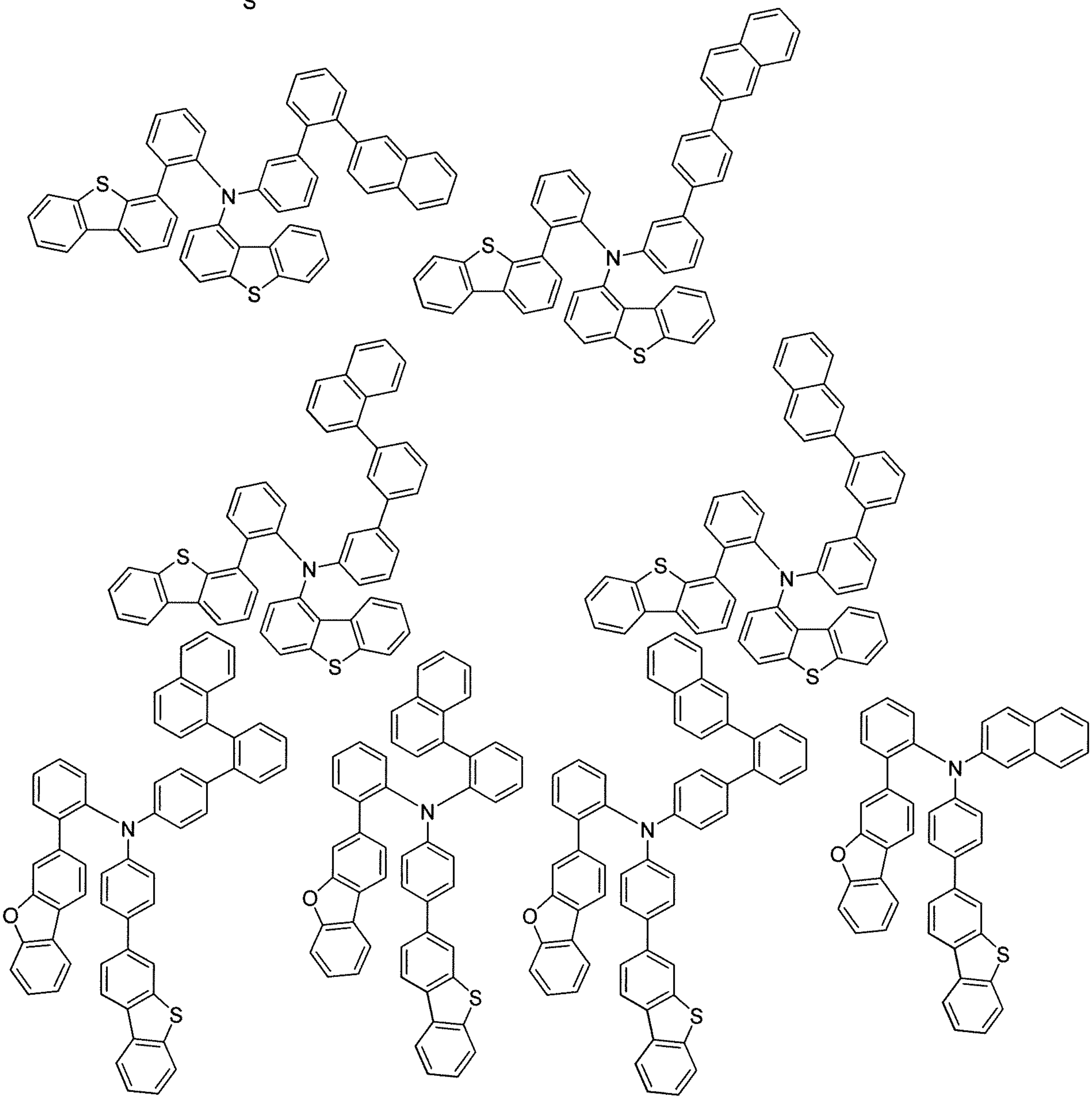

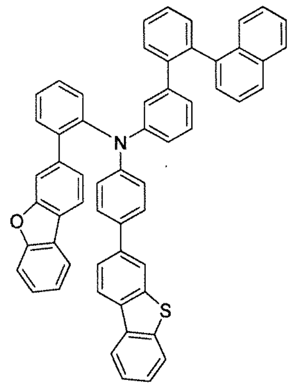

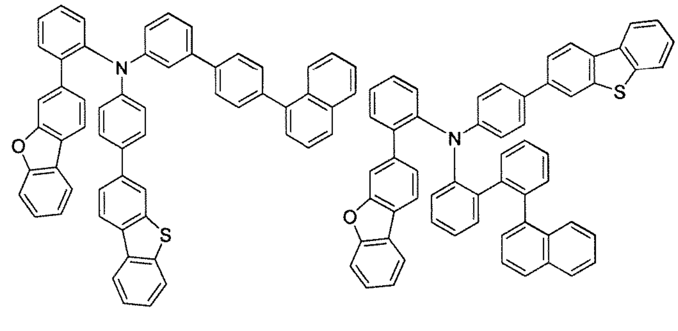

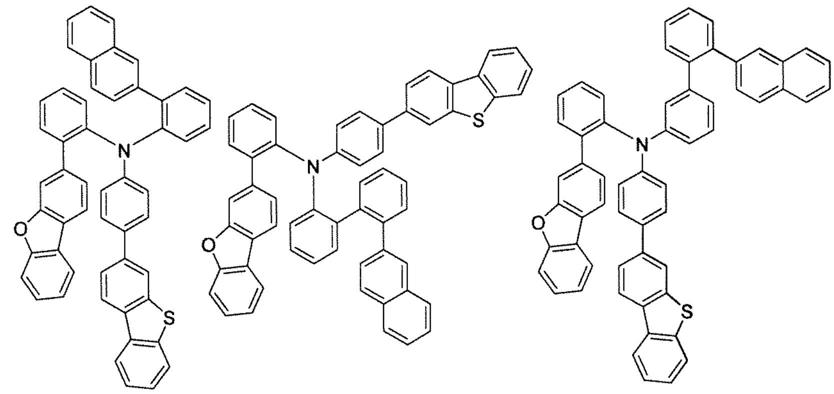

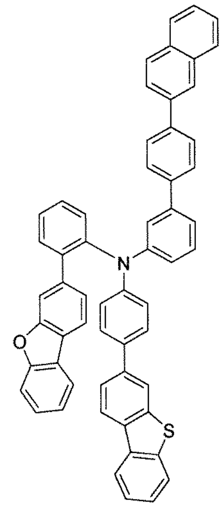

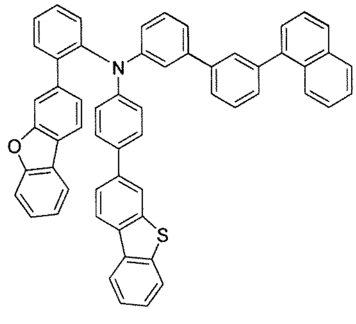

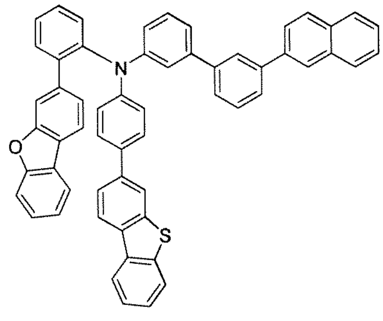

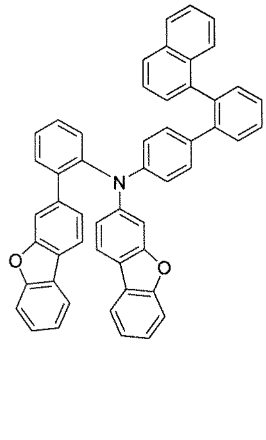

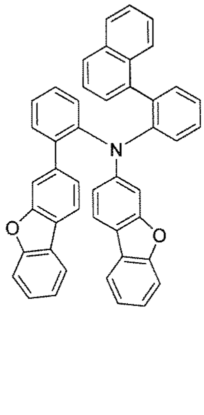

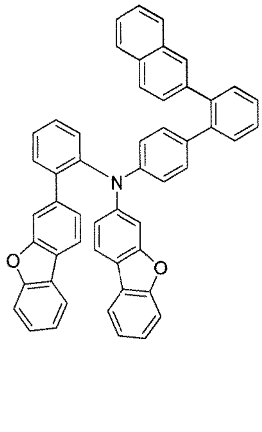

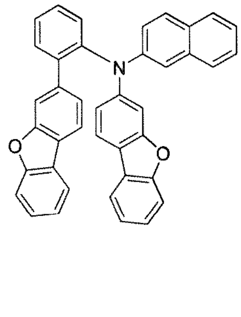

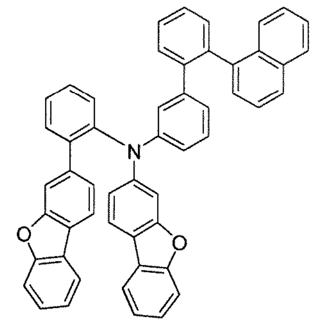
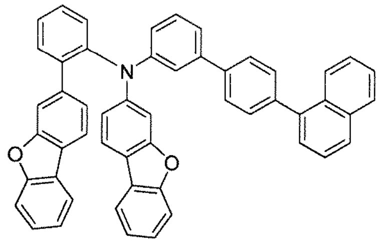
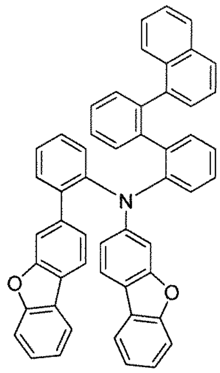
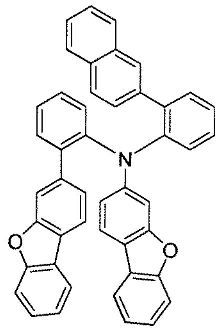
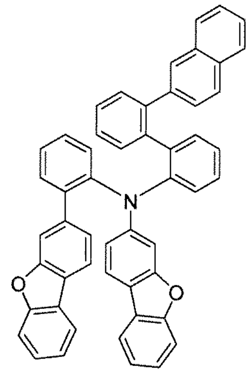
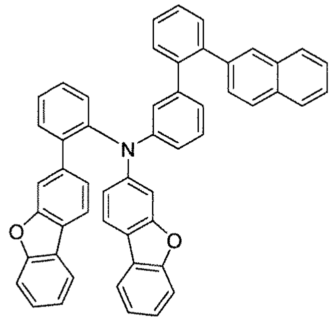
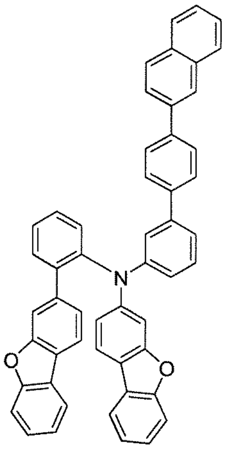
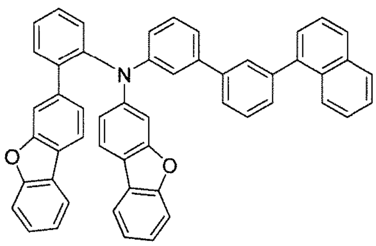
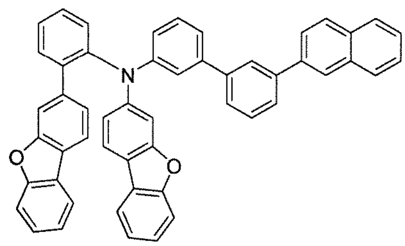
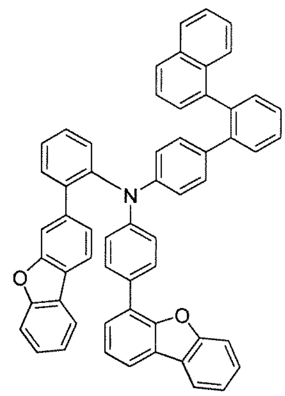
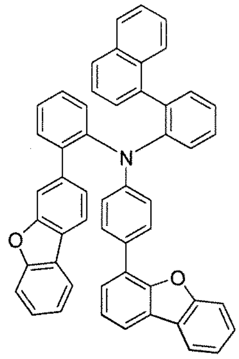
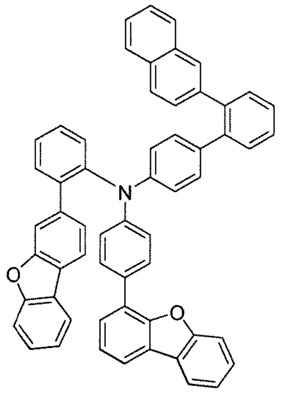

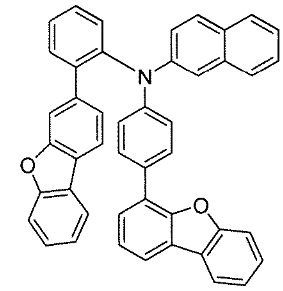
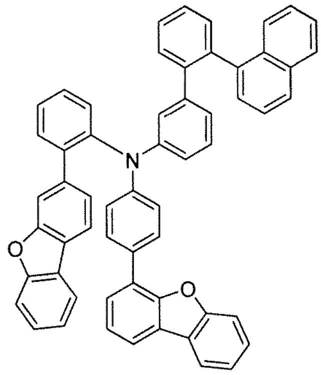
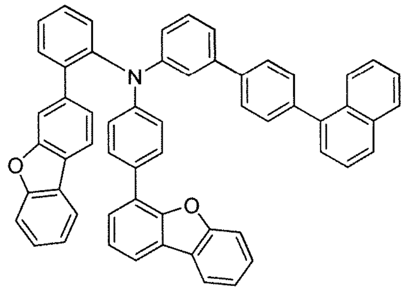

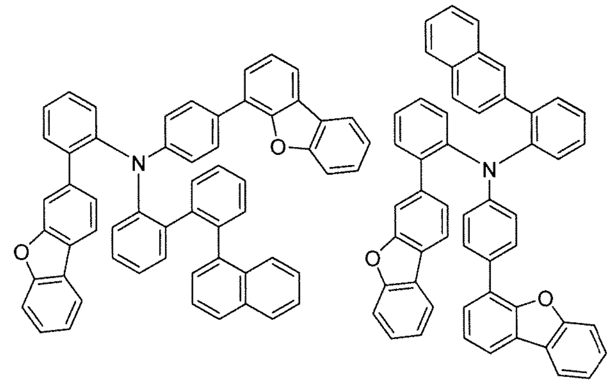
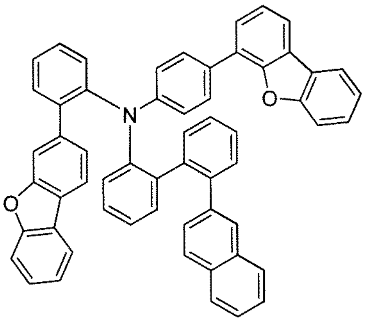

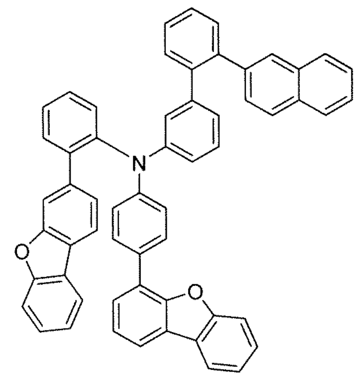
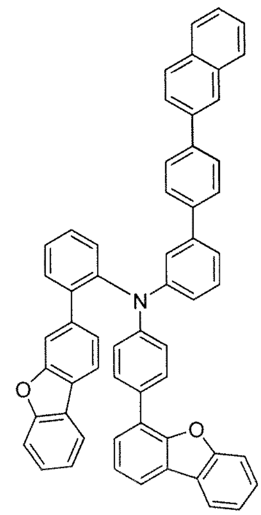
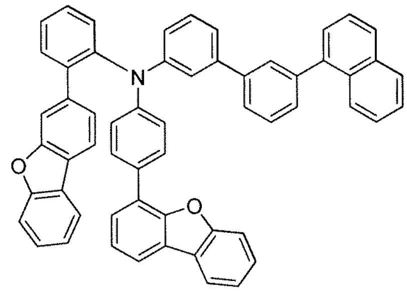

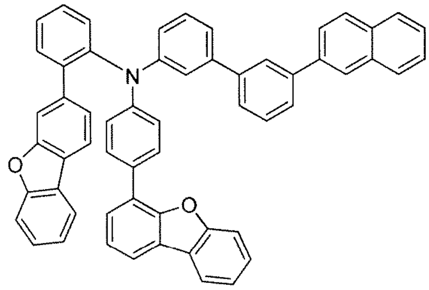
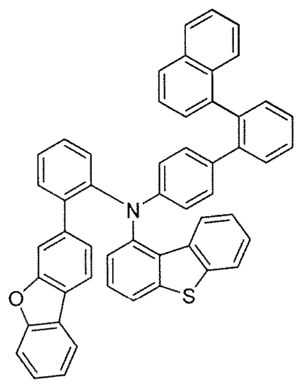
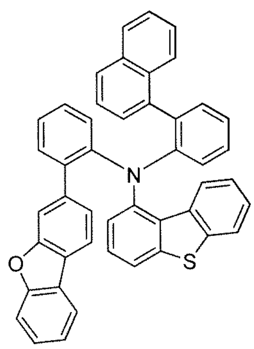

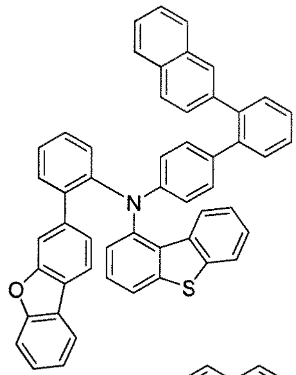
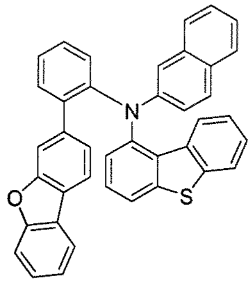
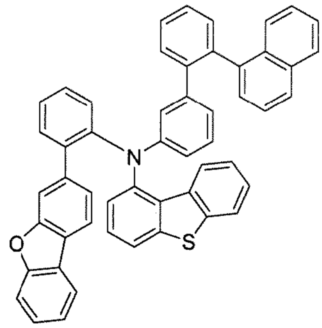
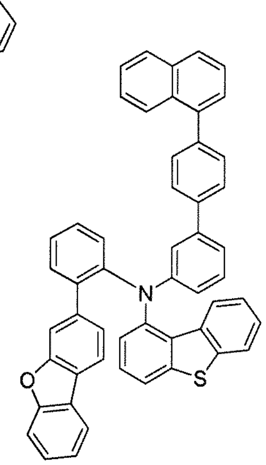
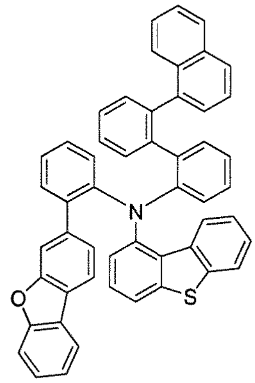
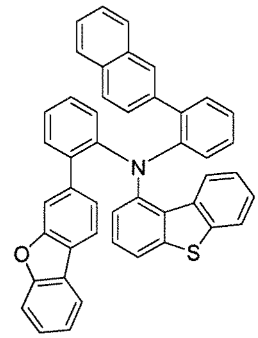
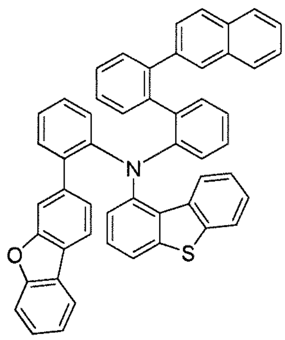
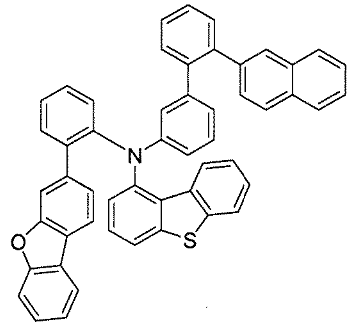
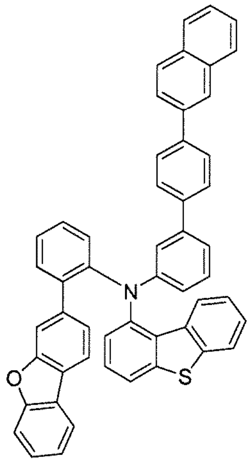
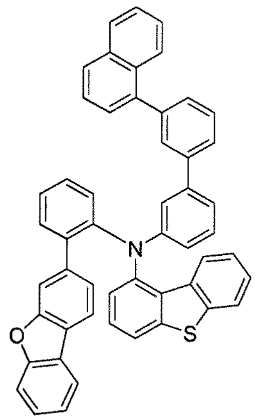
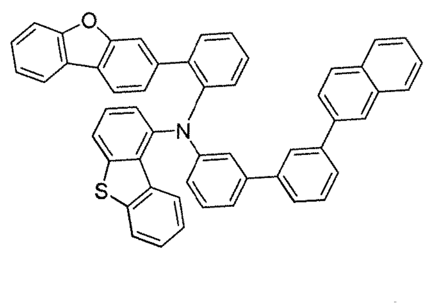
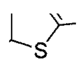

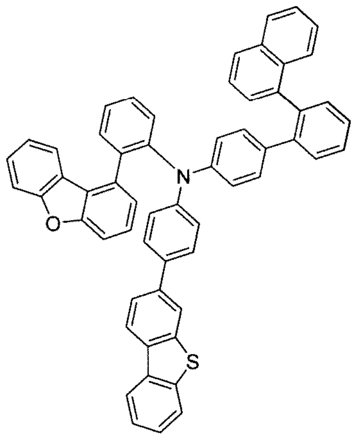

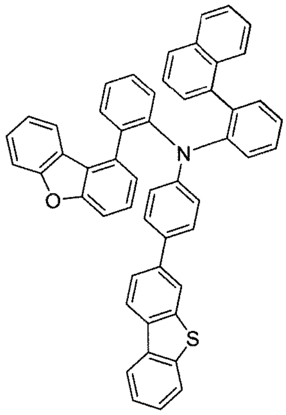

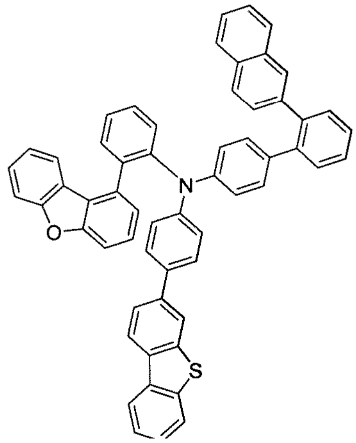

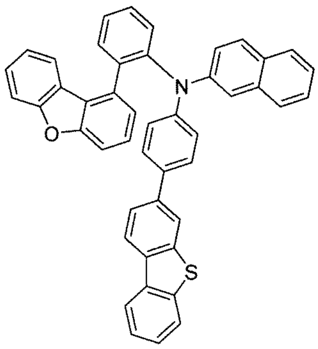

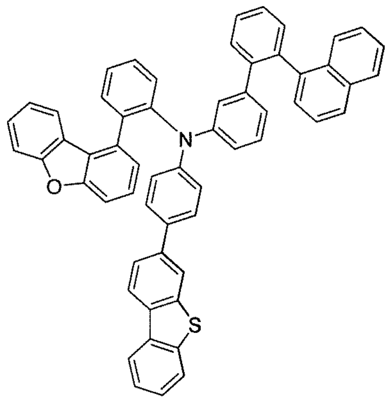

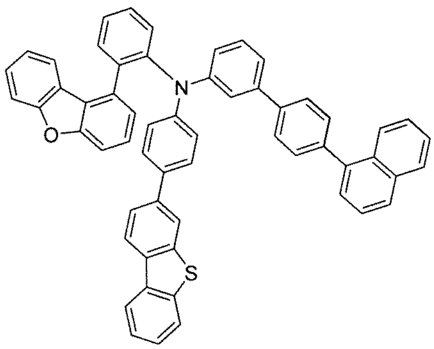

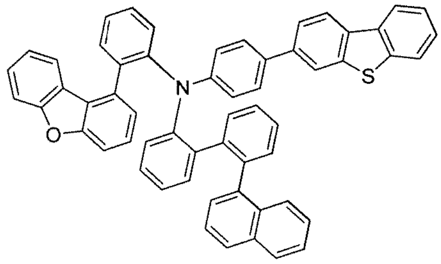

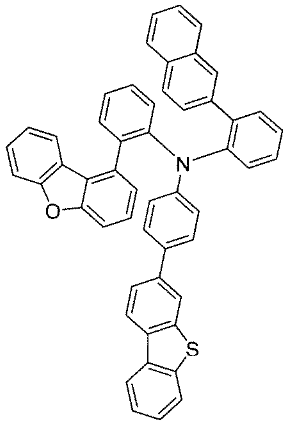

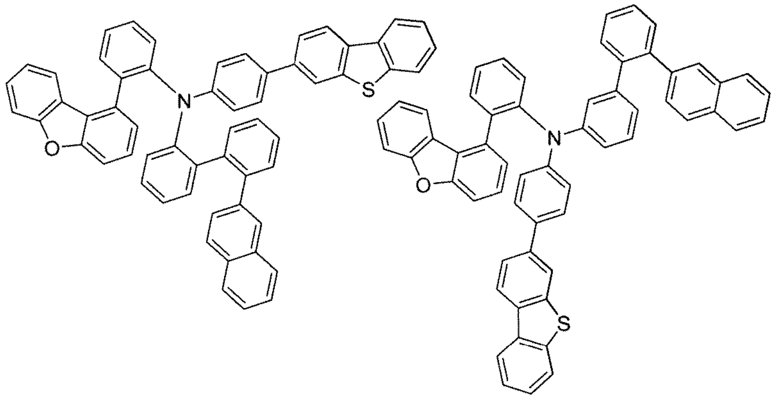

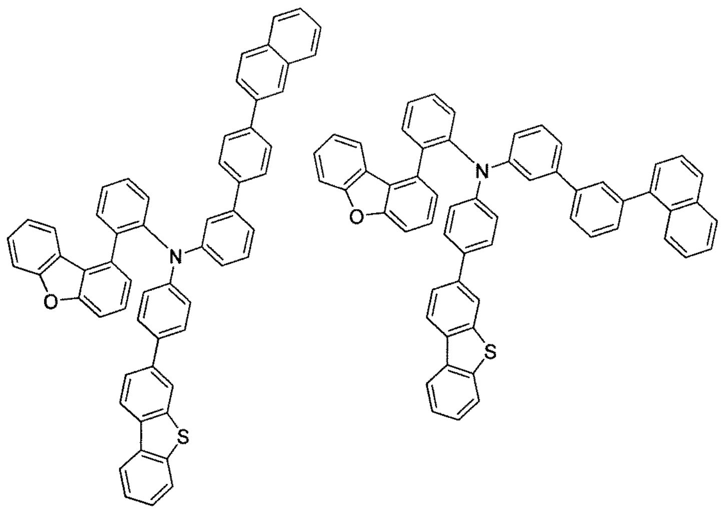

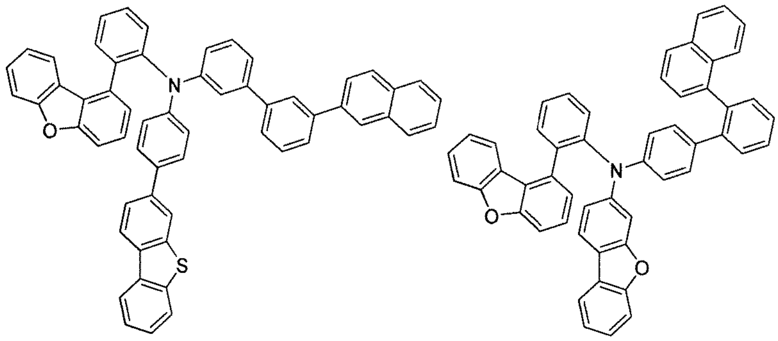

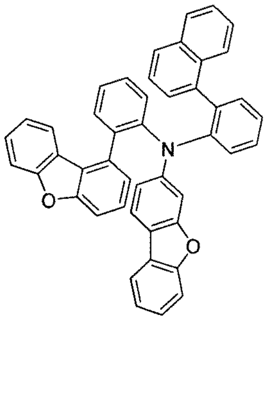

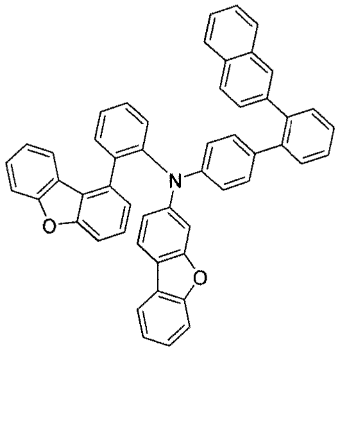

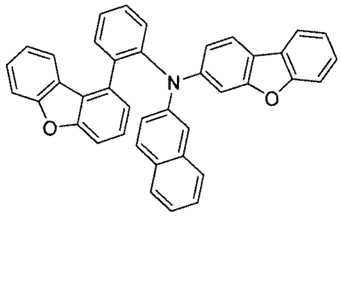

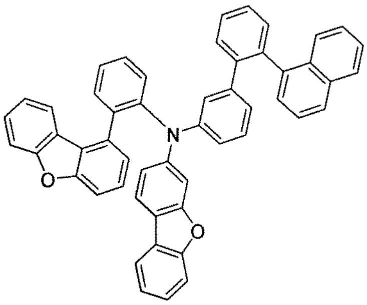

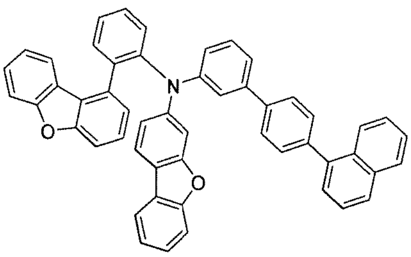

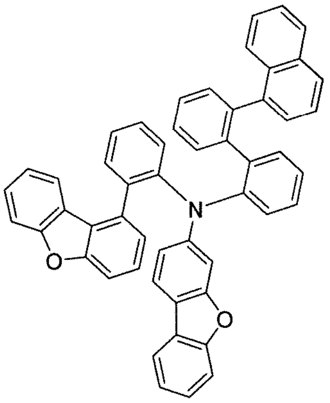

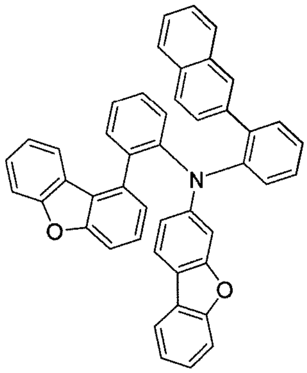

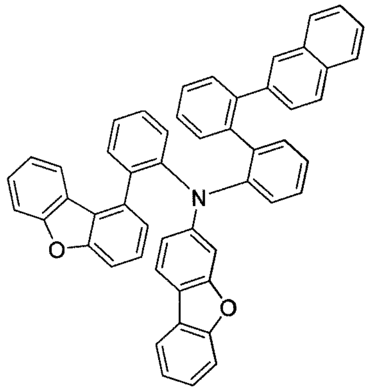

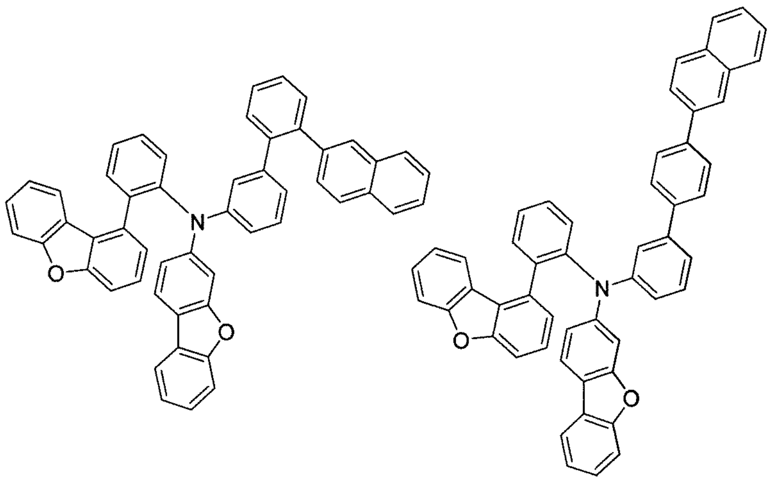

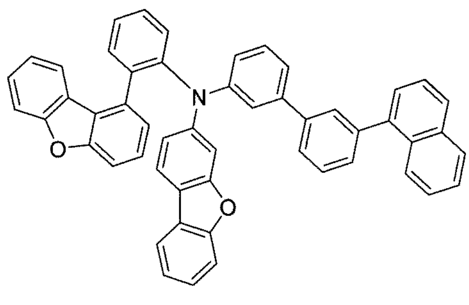

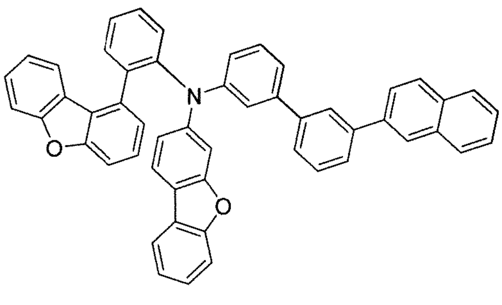

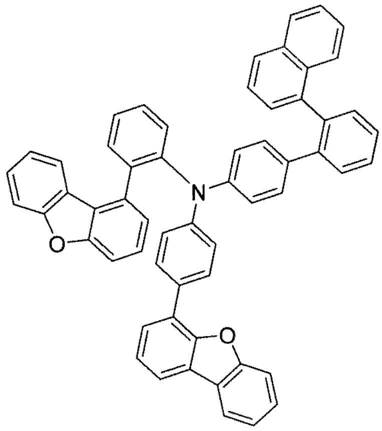

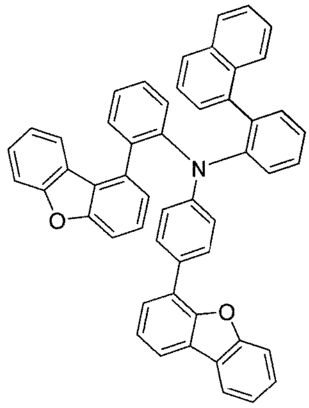

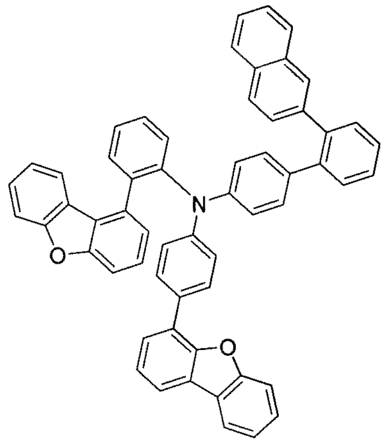

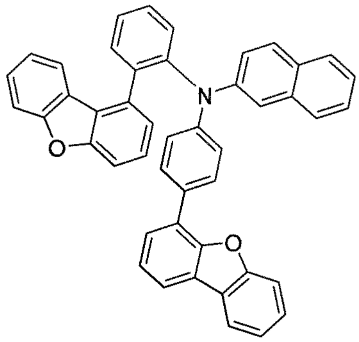

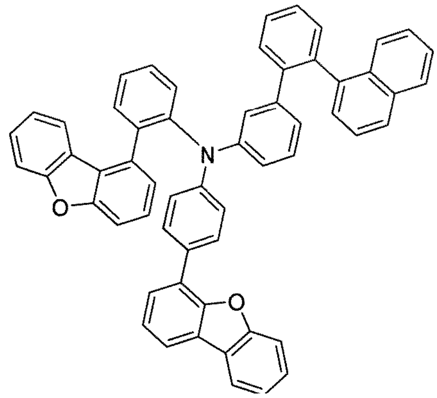

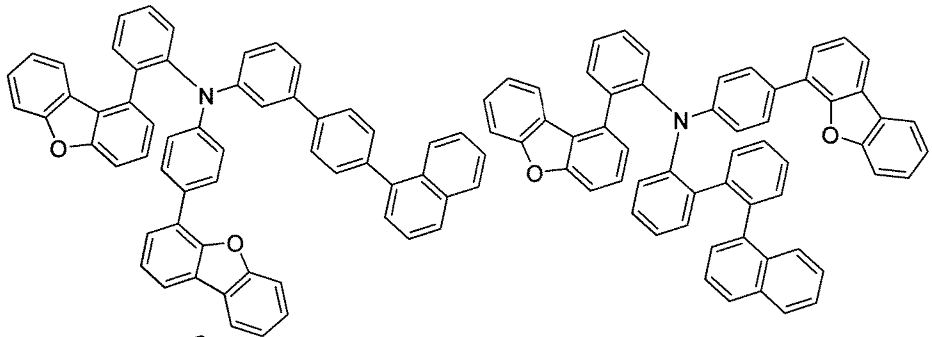

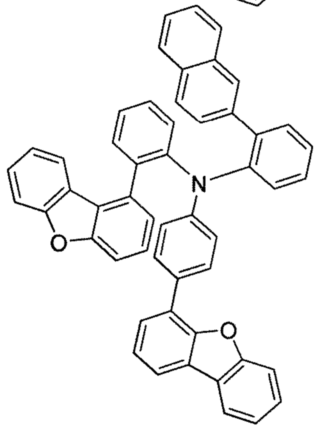

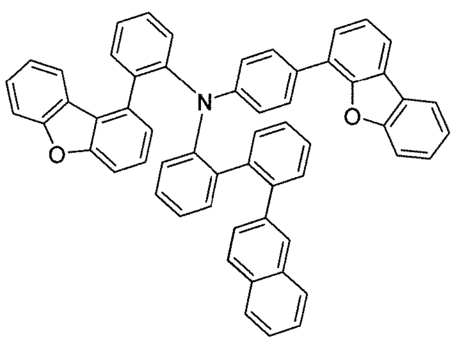

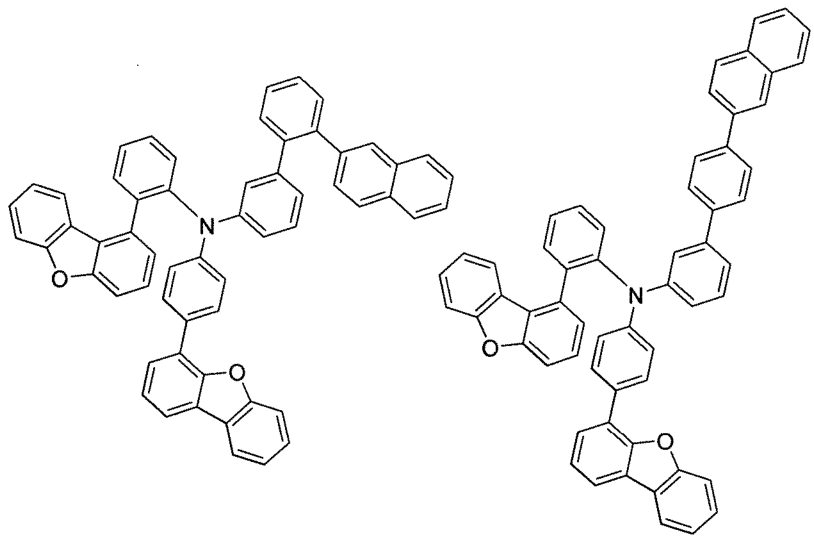

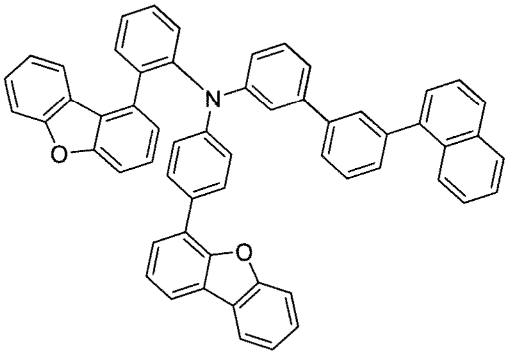

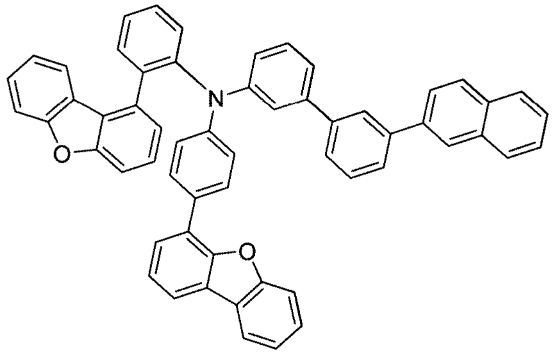

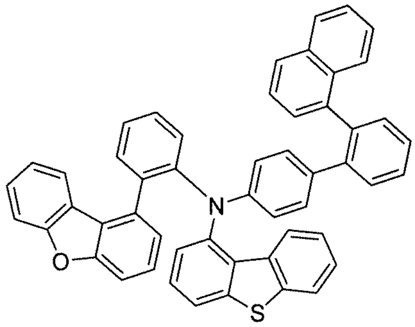

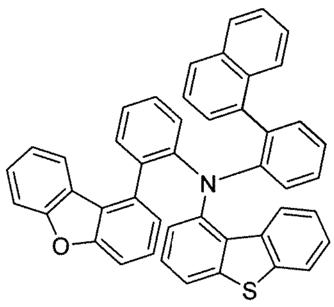

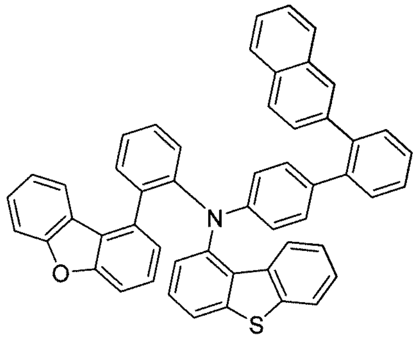

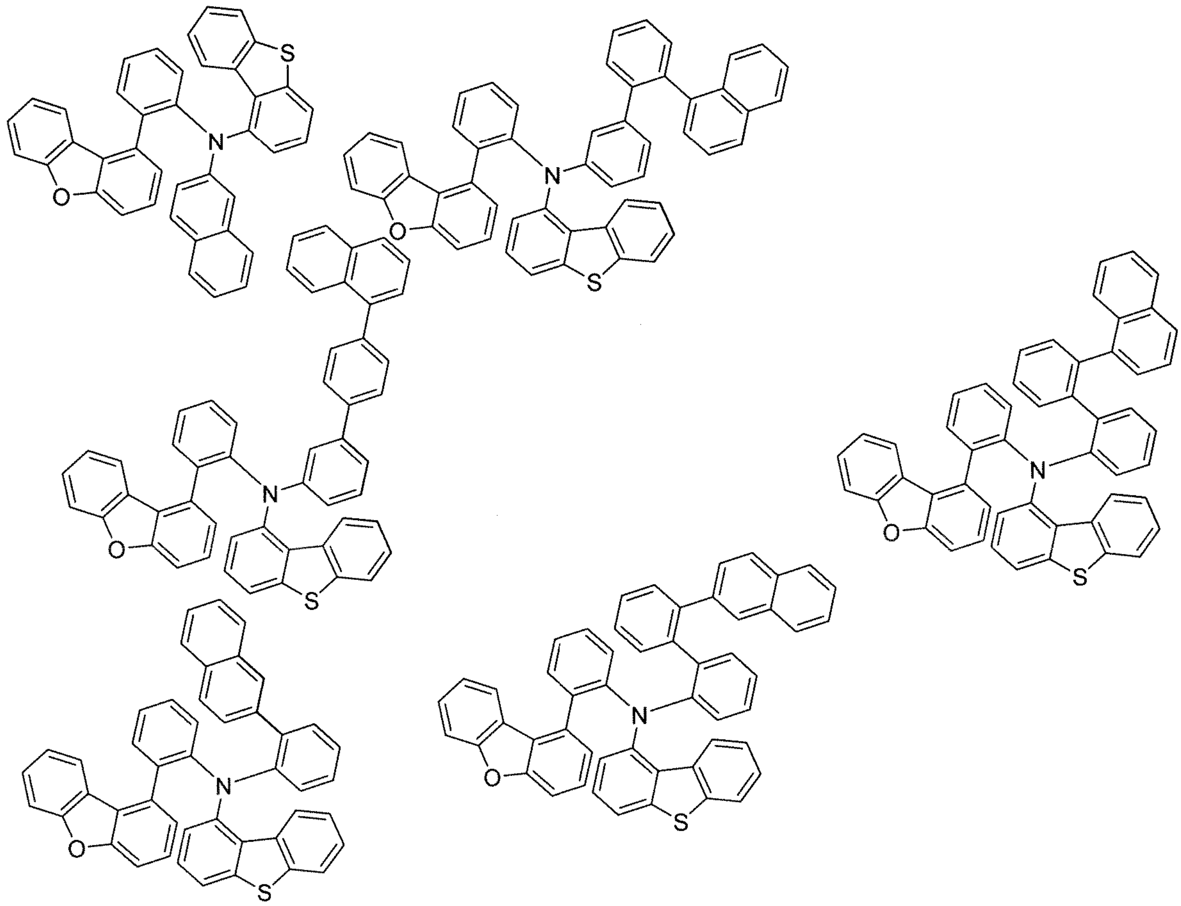

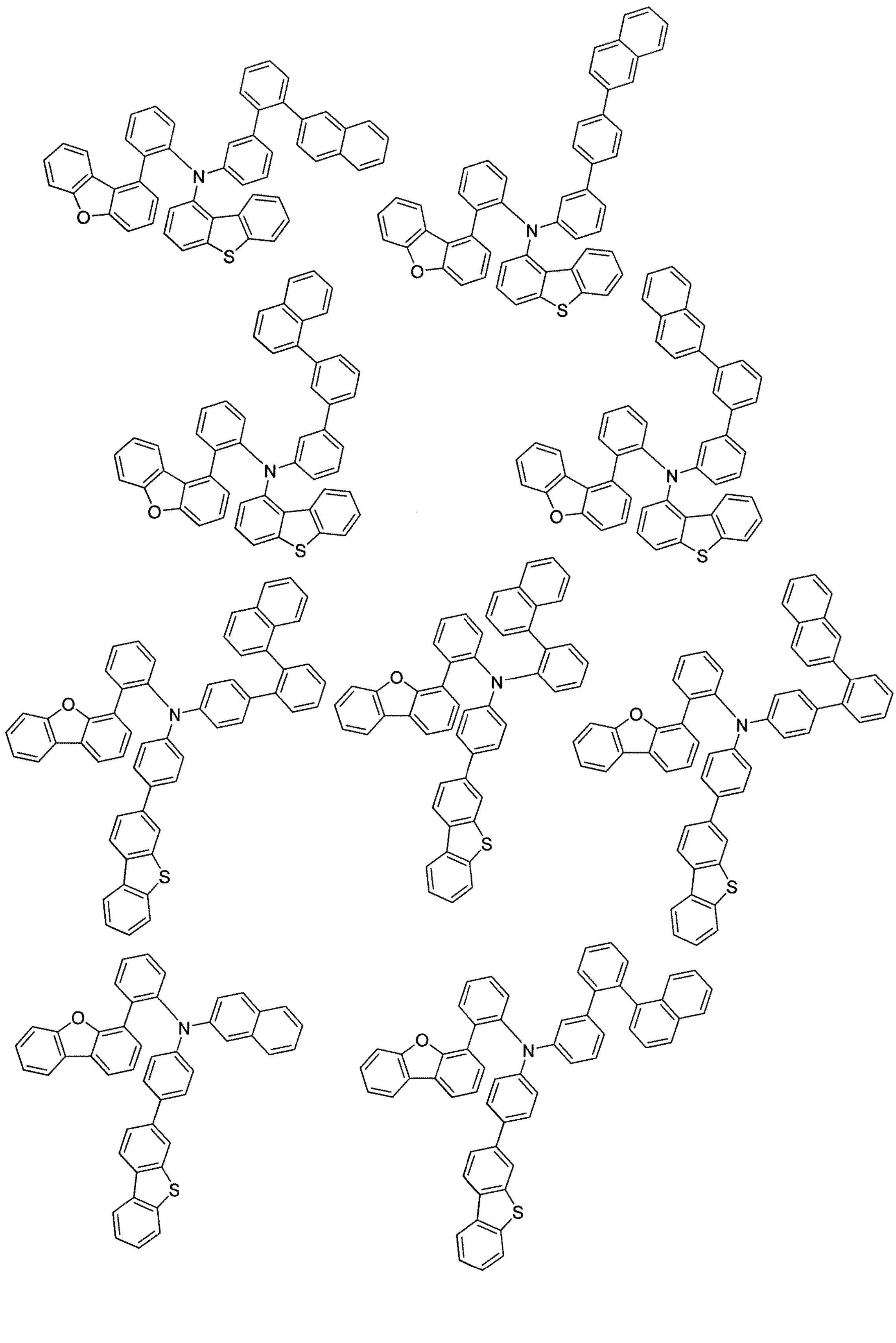

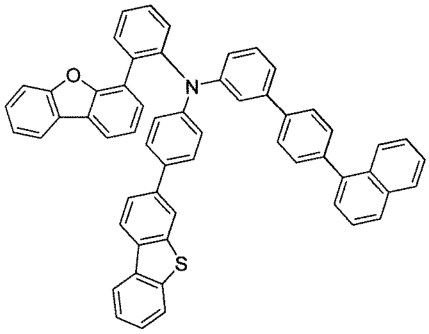
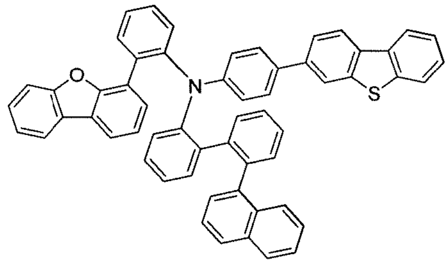

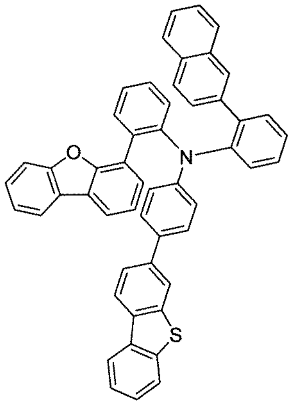
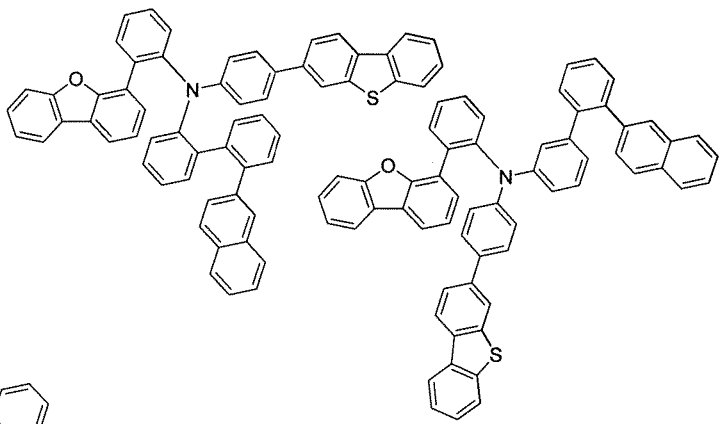

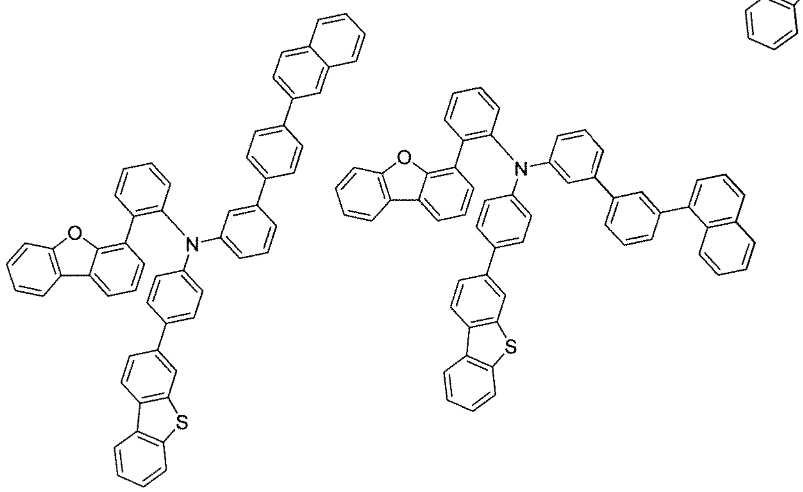

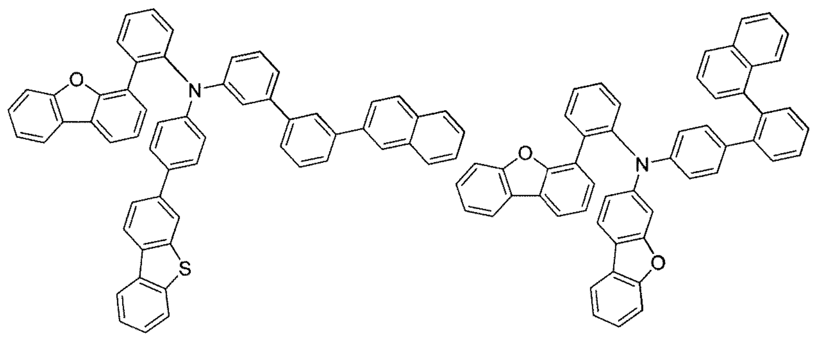

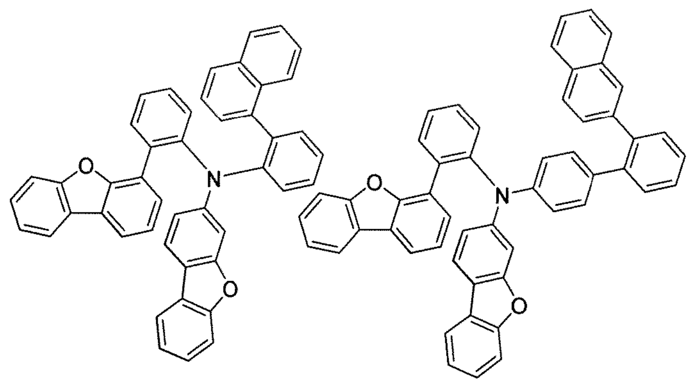

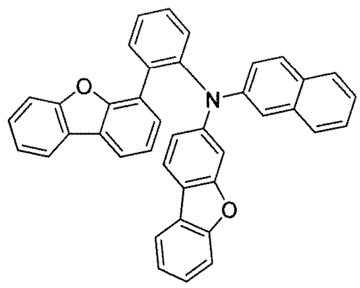

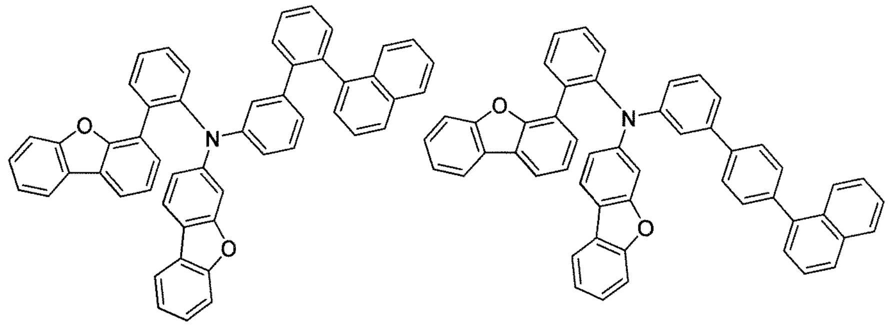

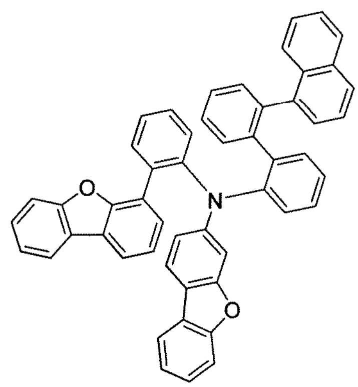

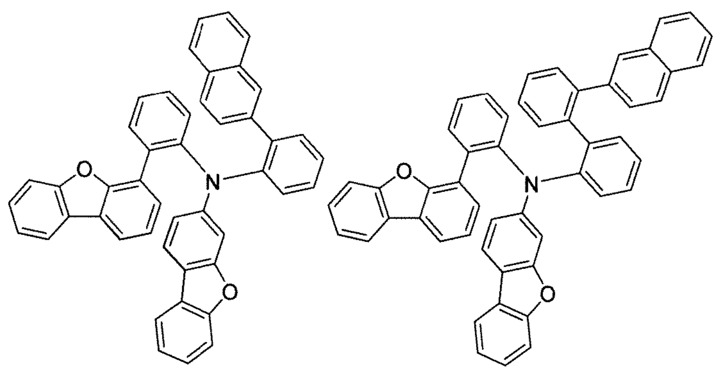

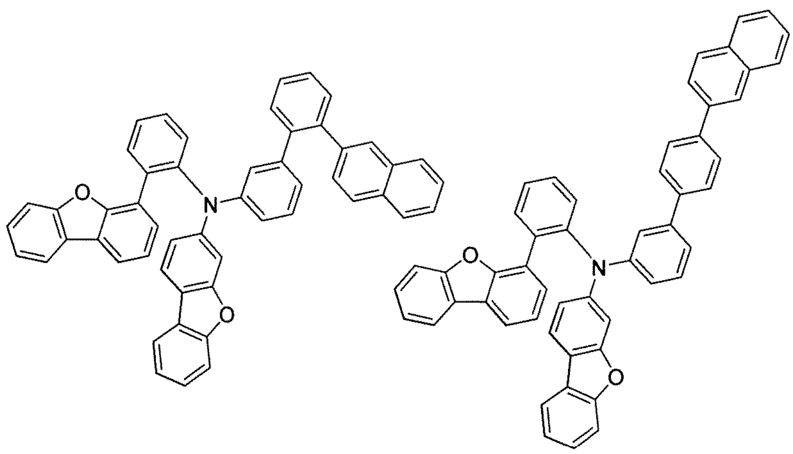

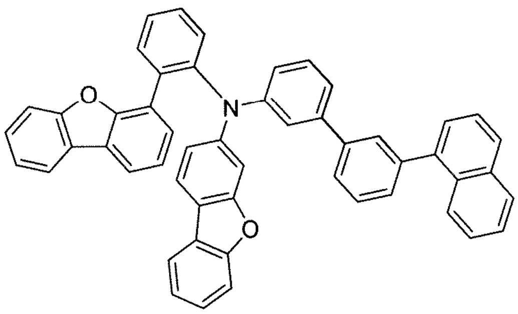
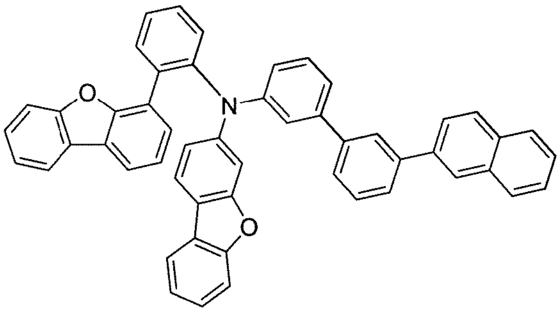

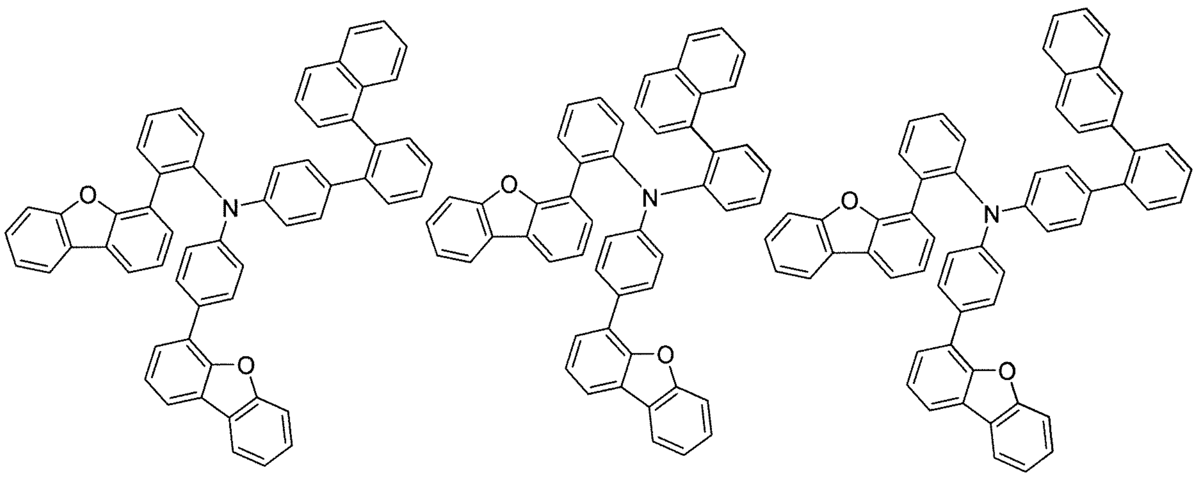

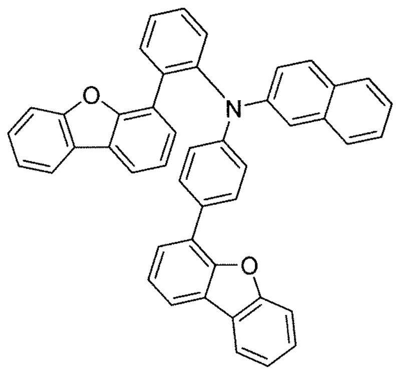
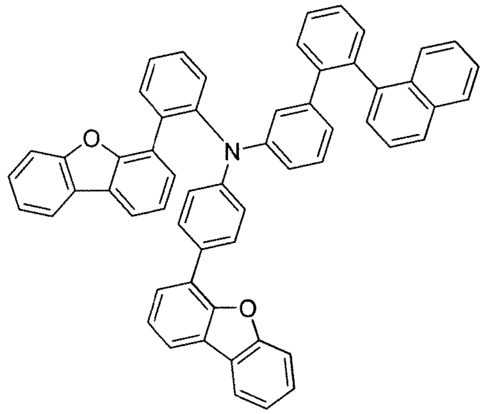

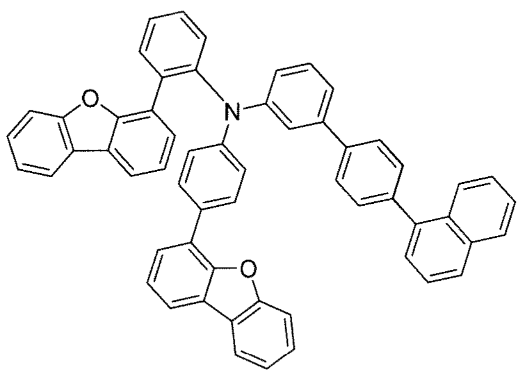
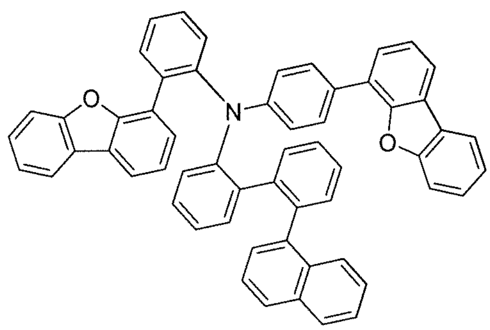

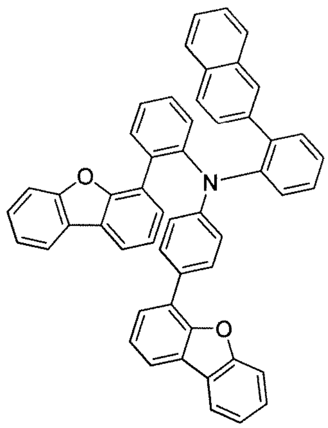

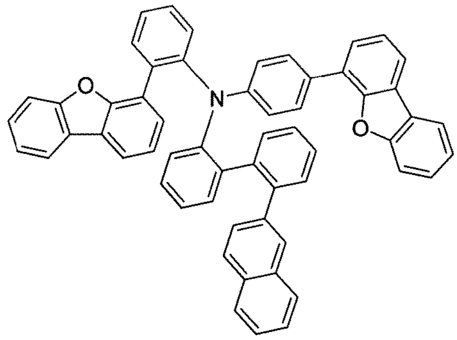

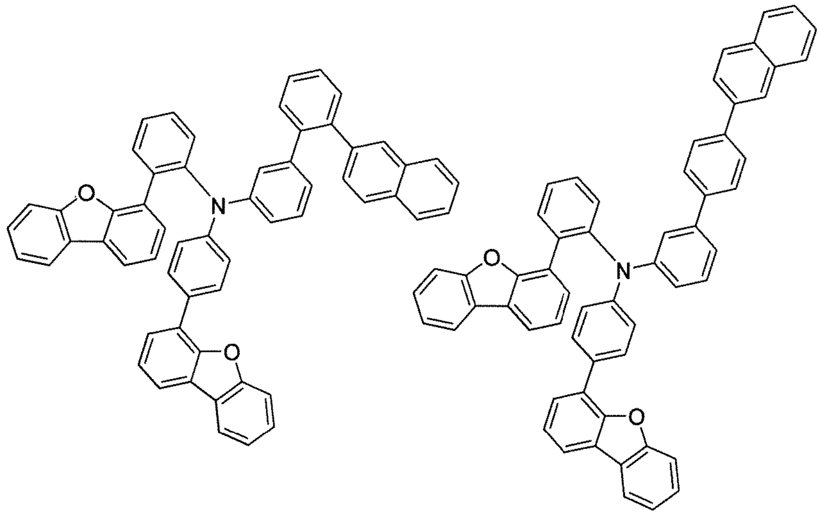

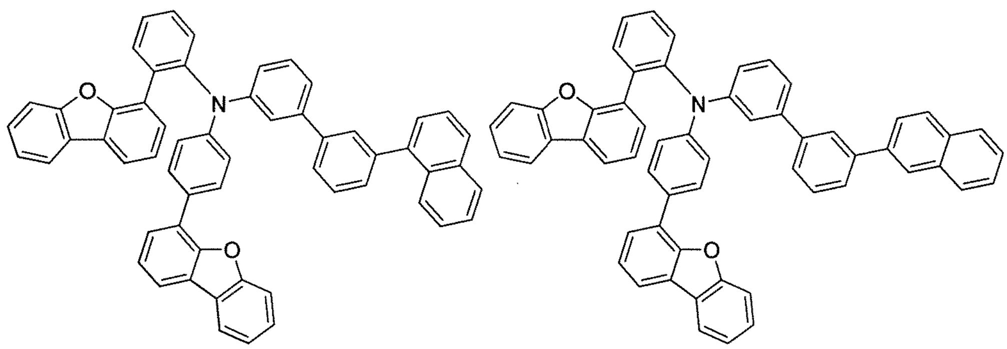

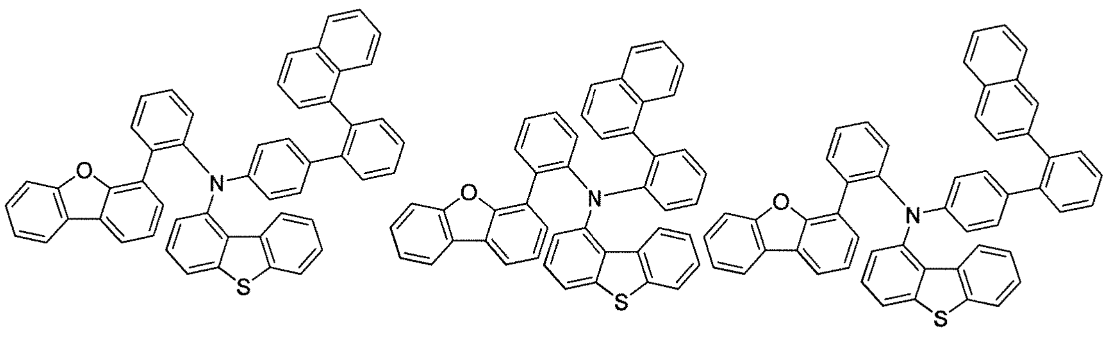

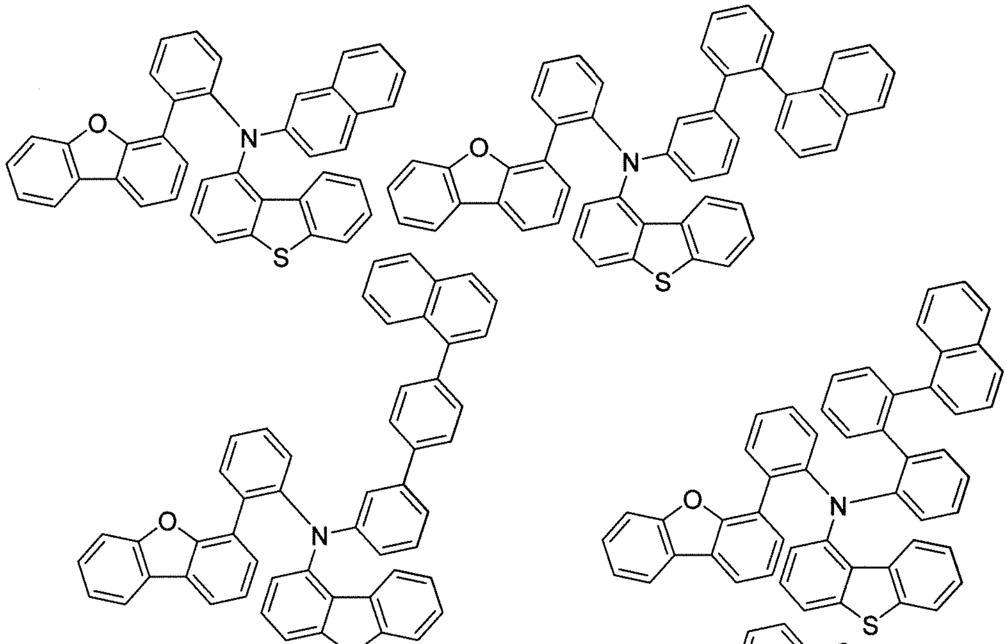

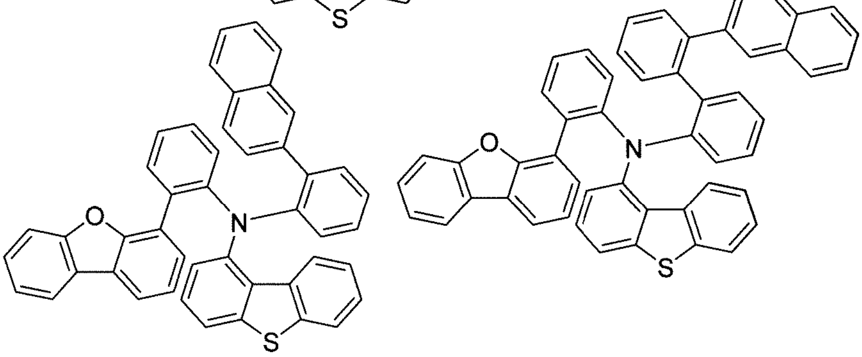

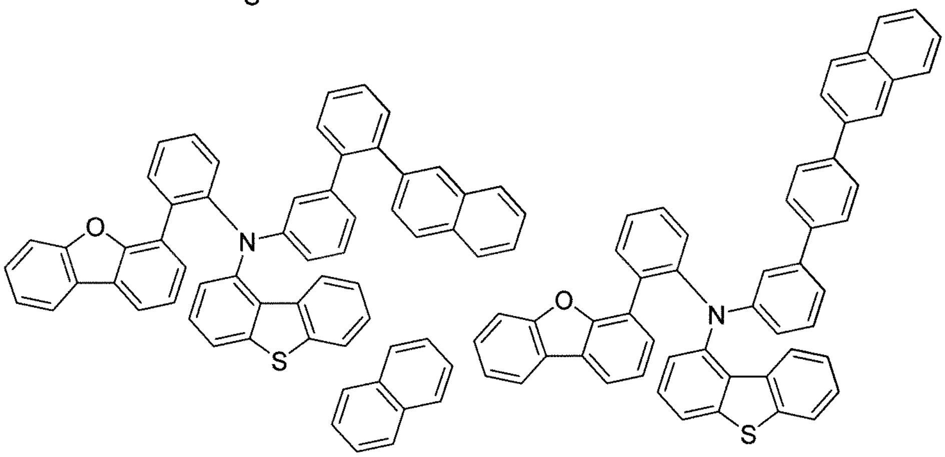

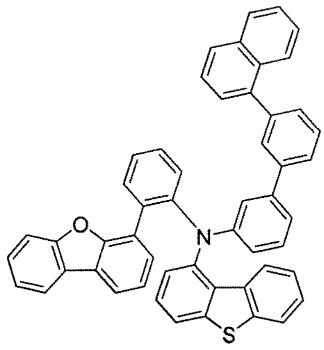

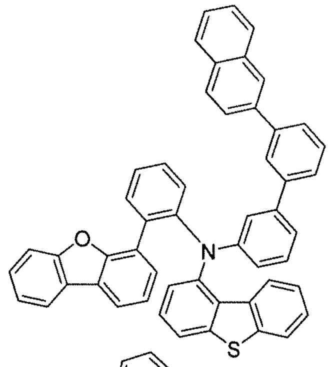

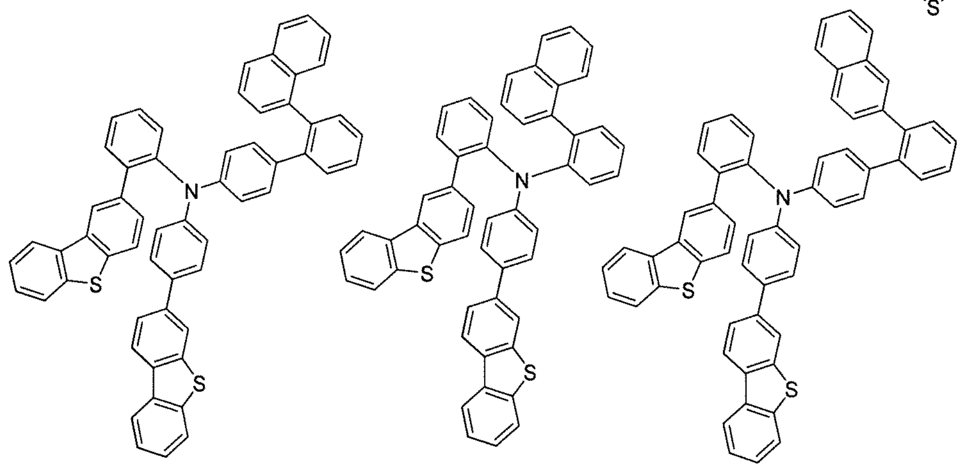

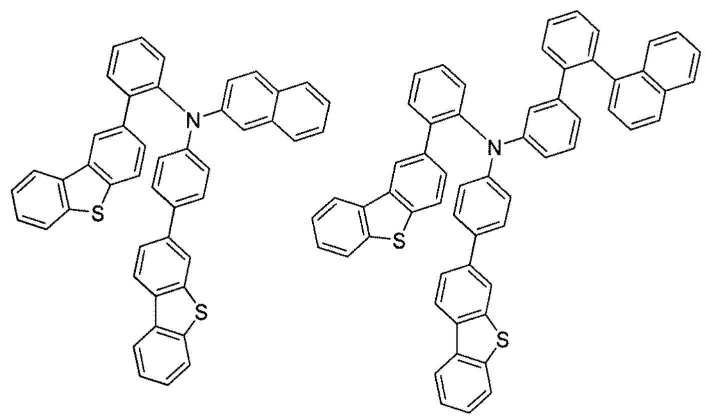

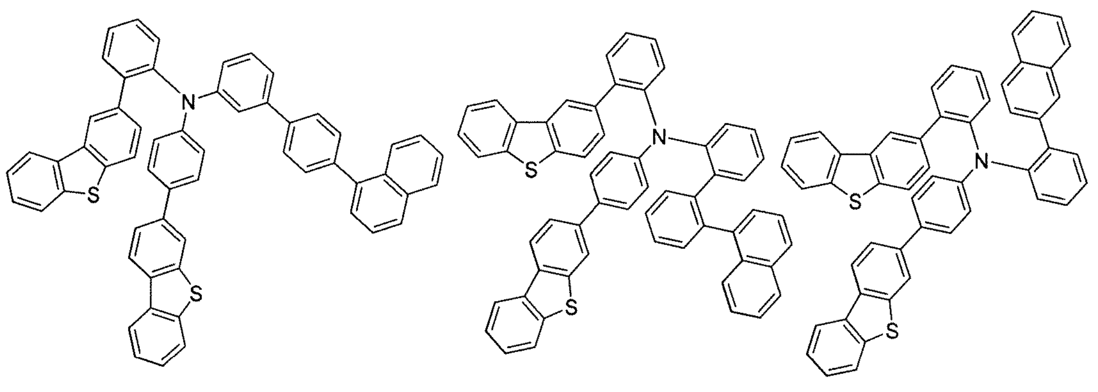

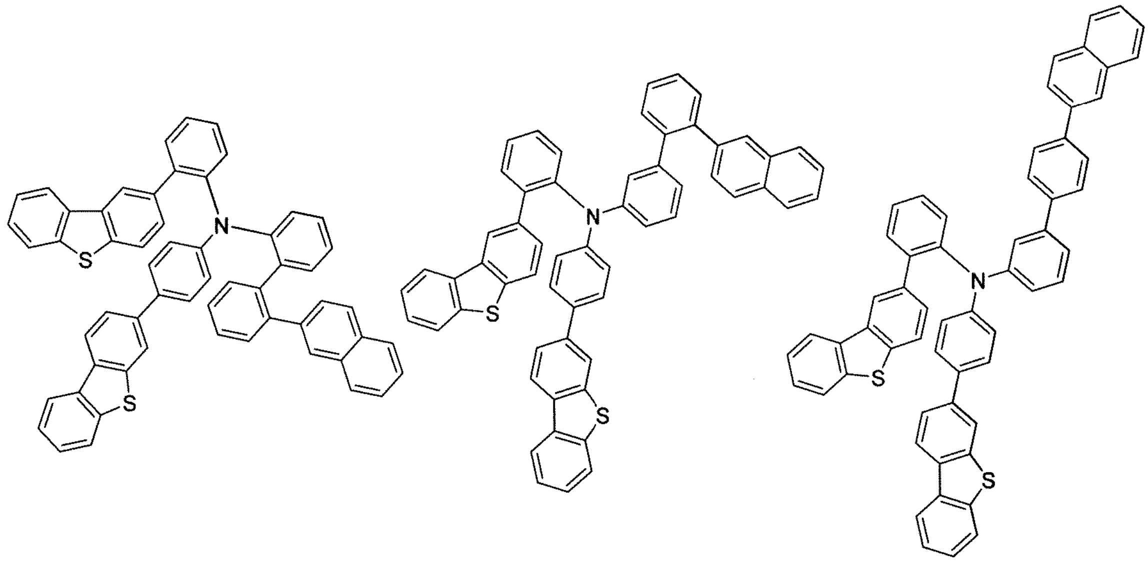

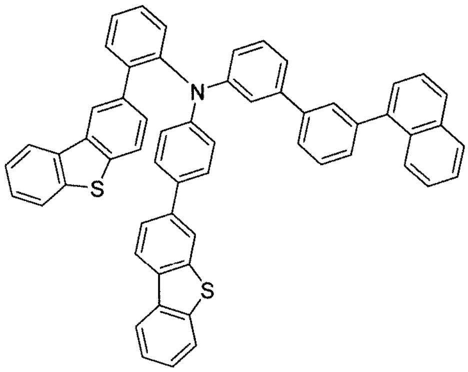

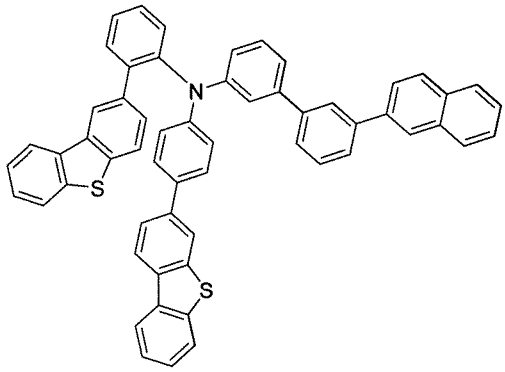

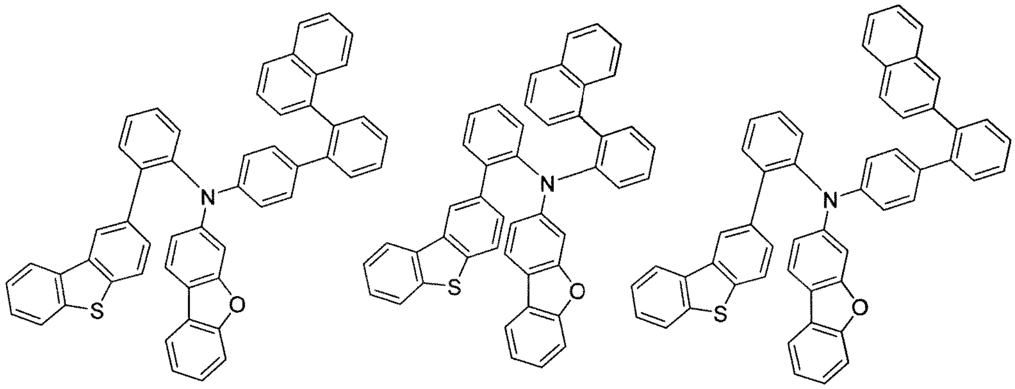

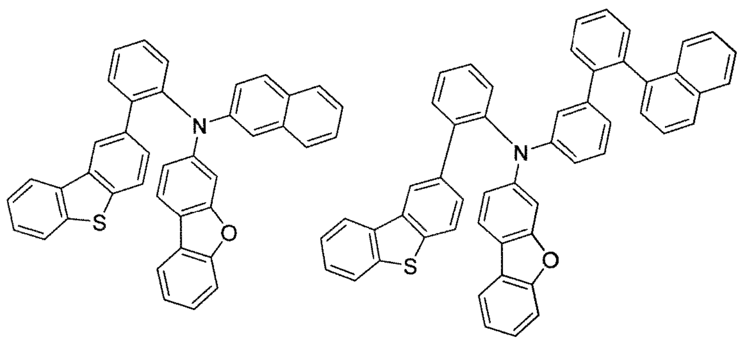

[0184]

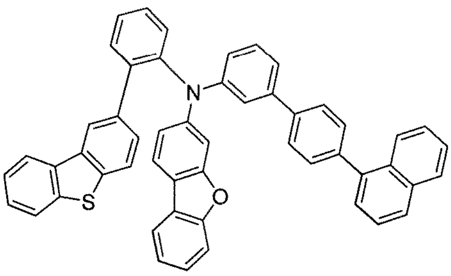
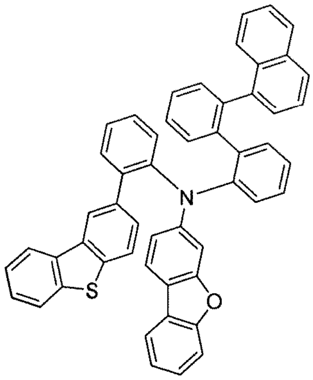
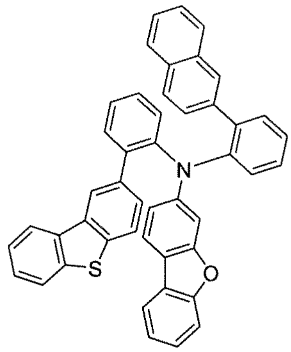

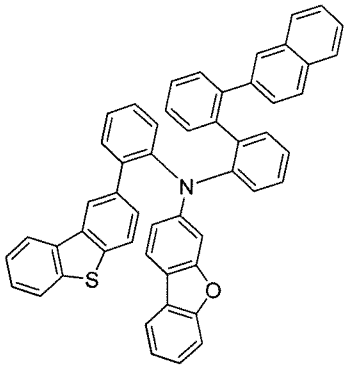
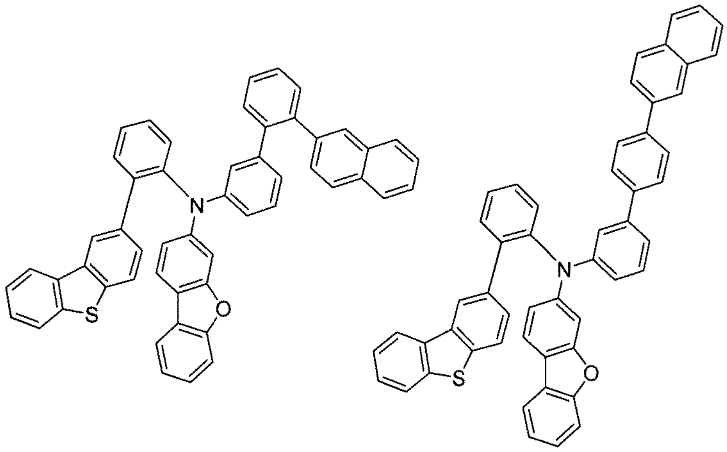

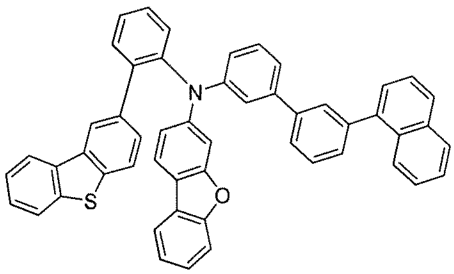
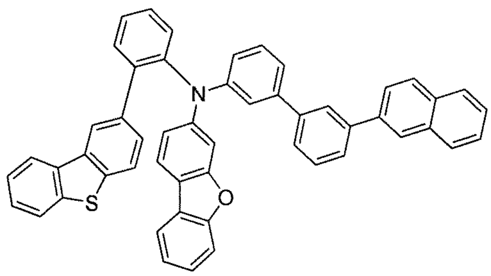

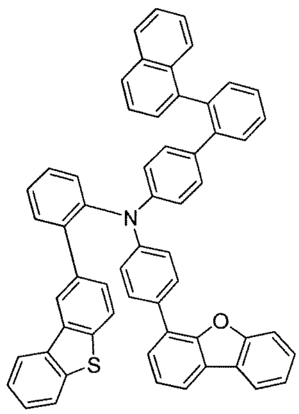
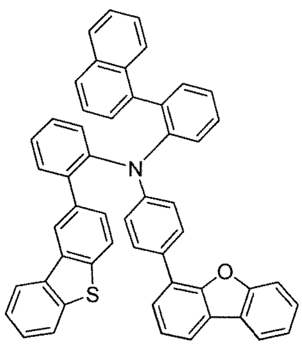
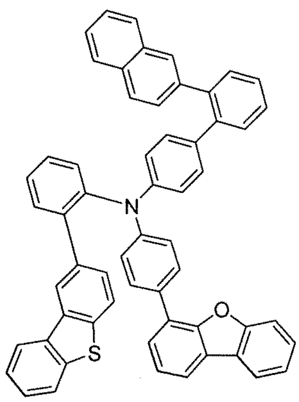

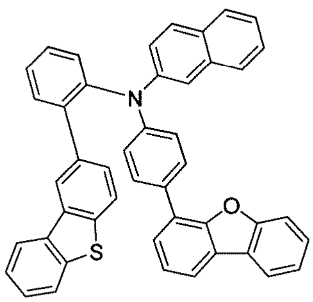

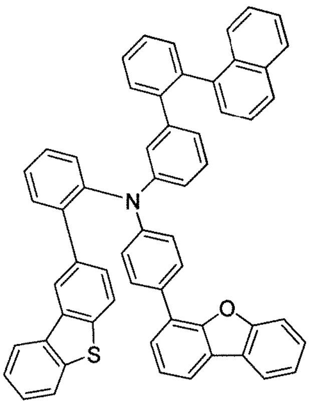

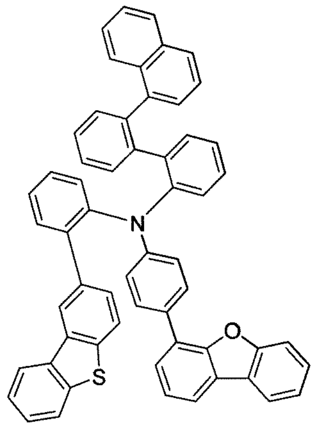

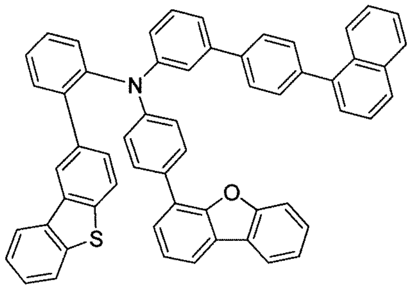

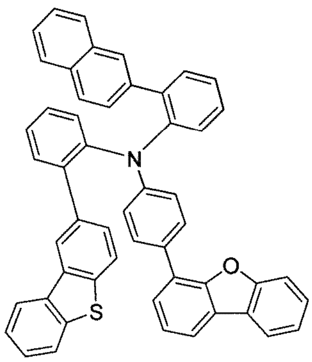

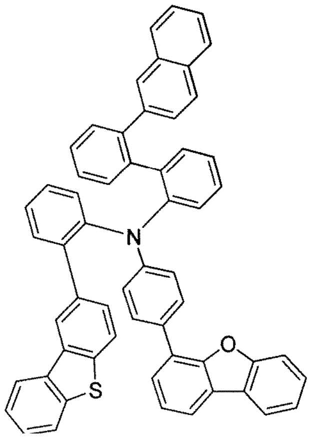

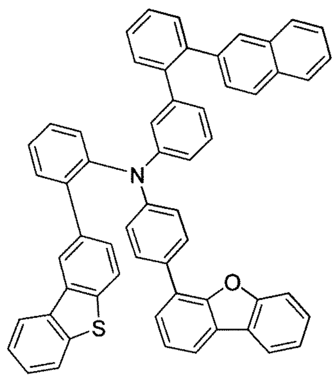

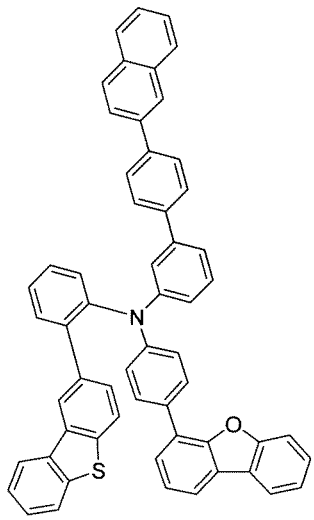

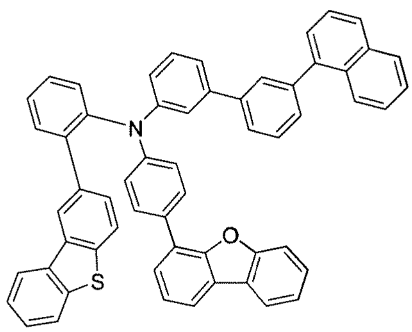

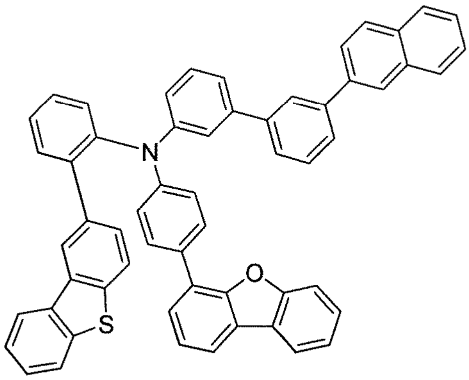

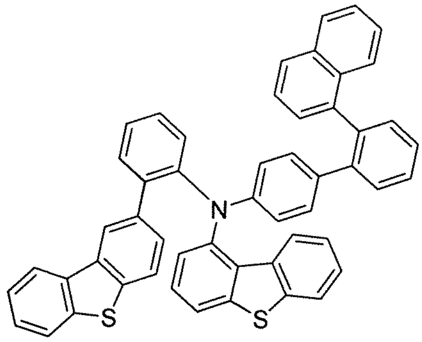

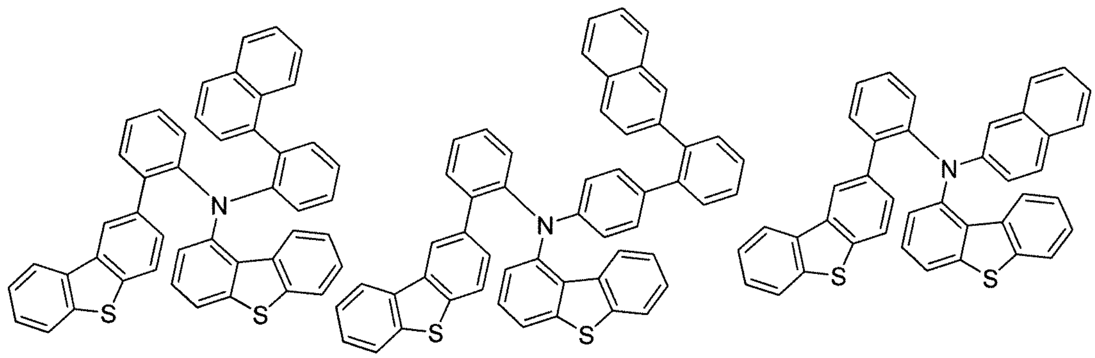

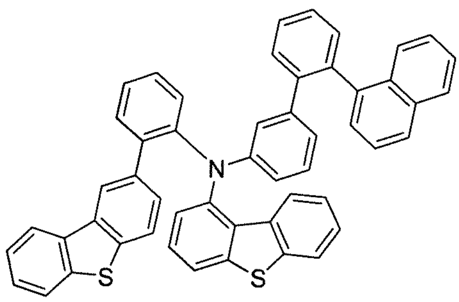

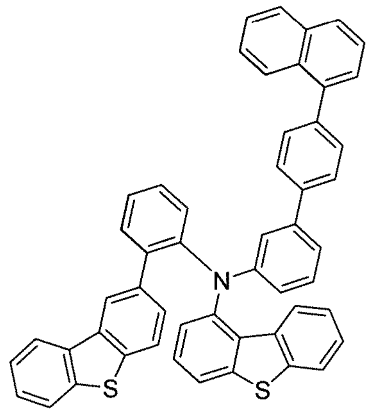

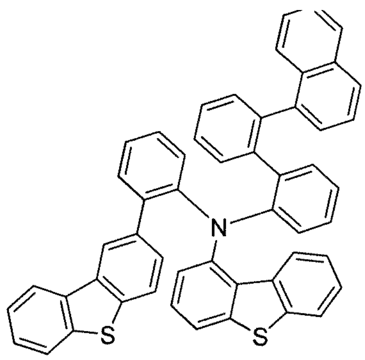

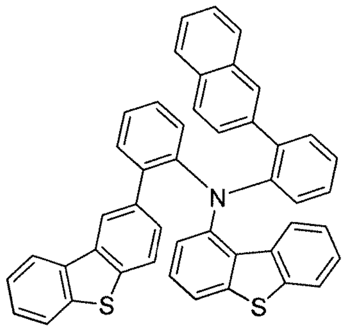

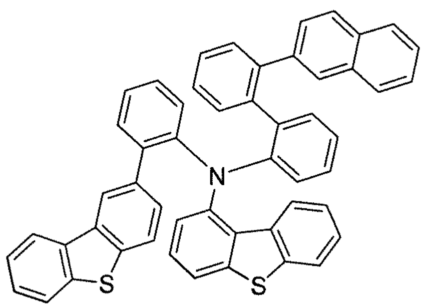

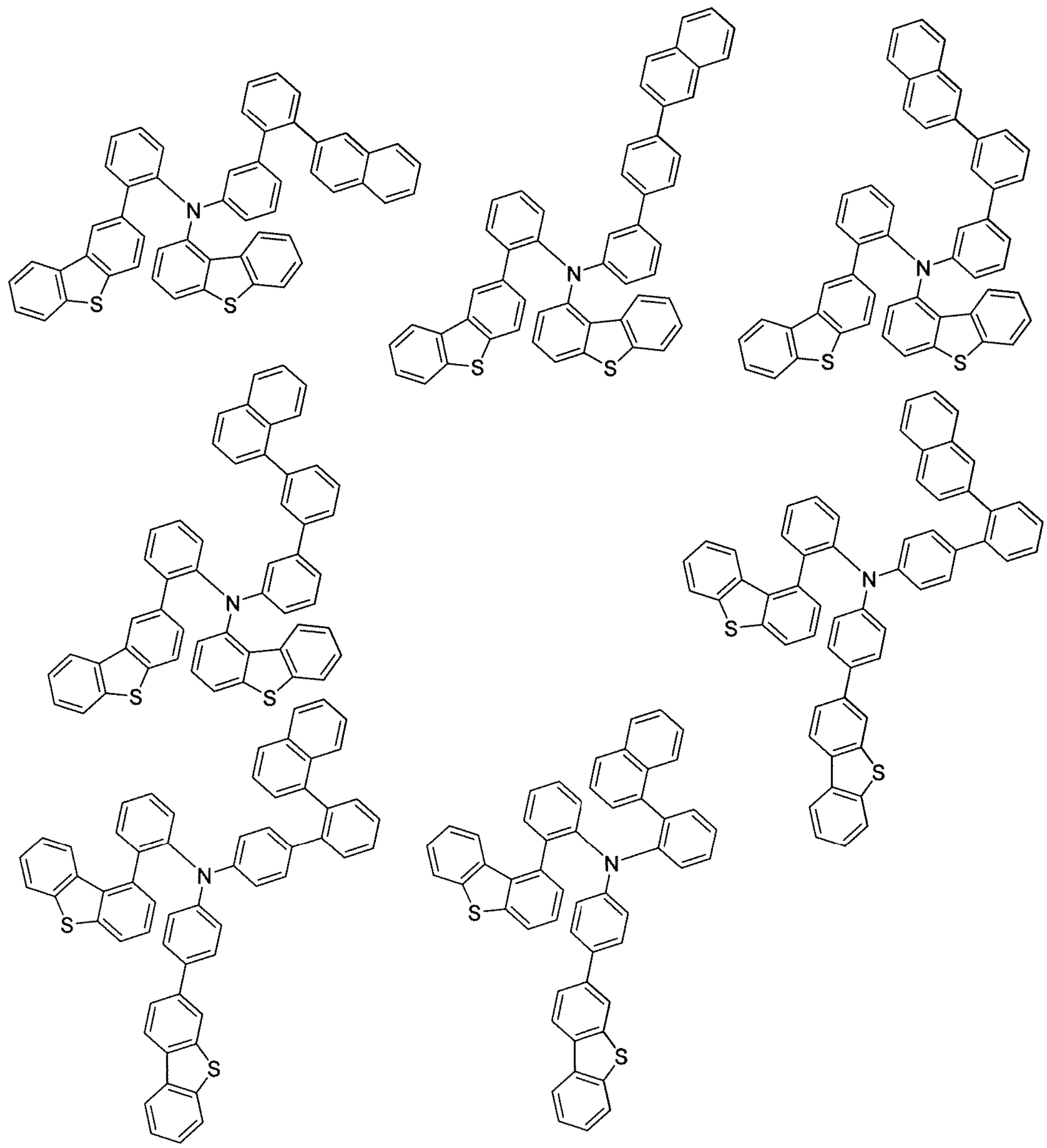

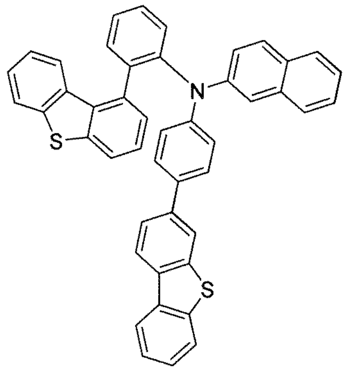

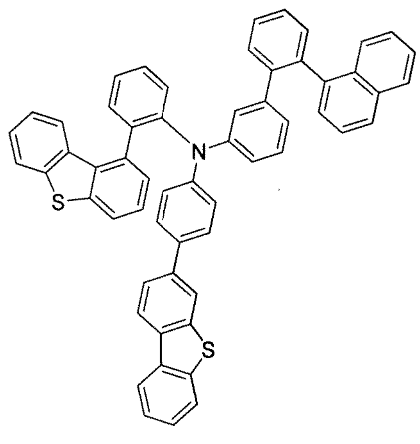

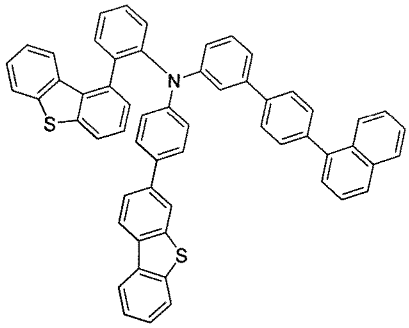

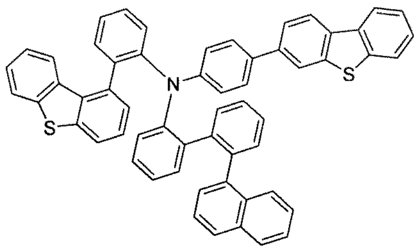

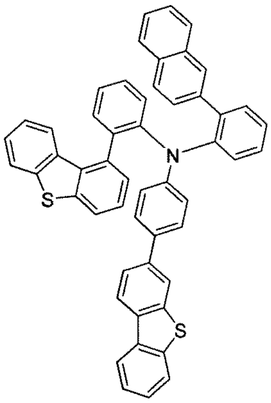

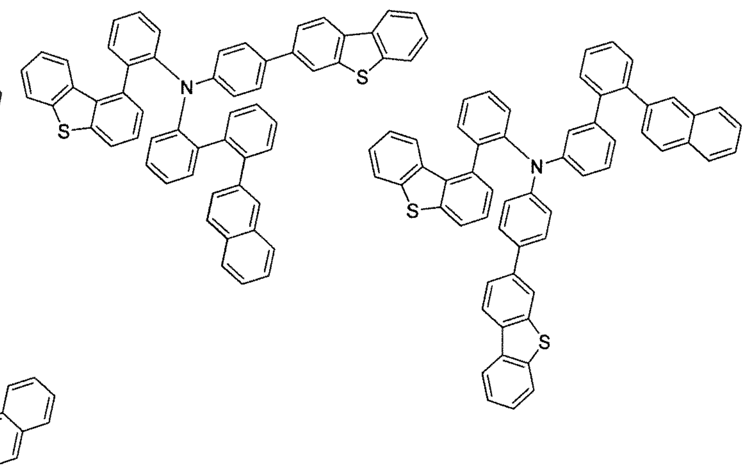

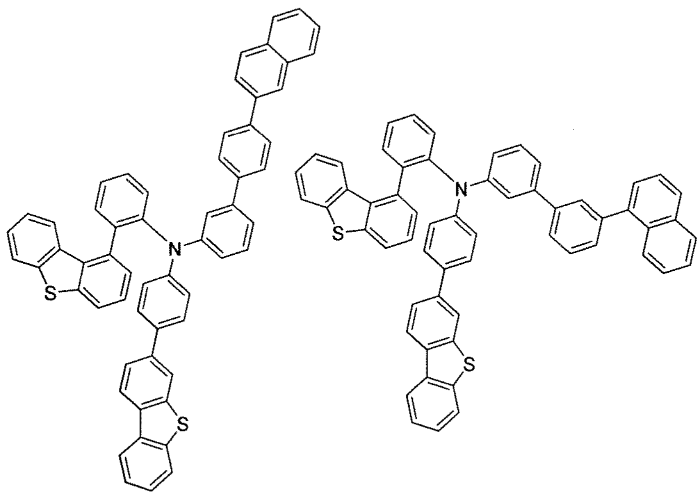

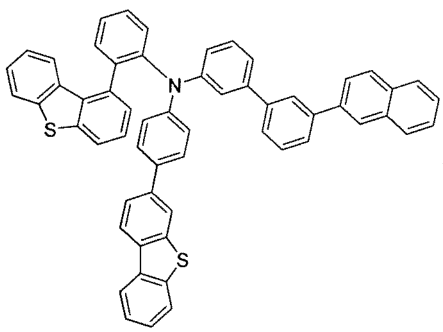

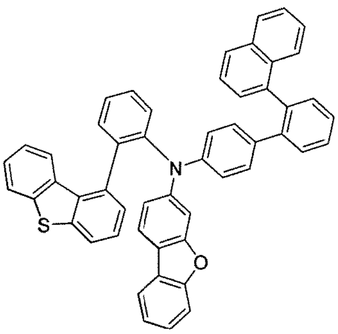

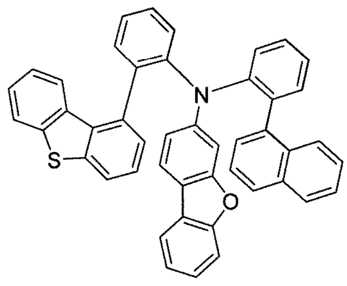

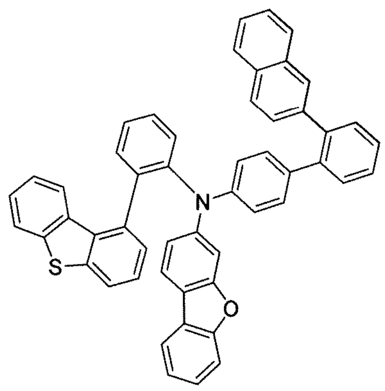

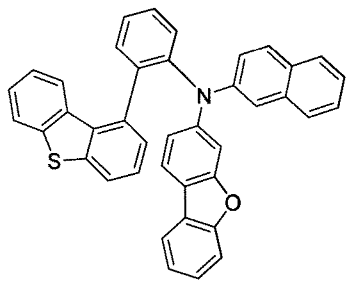

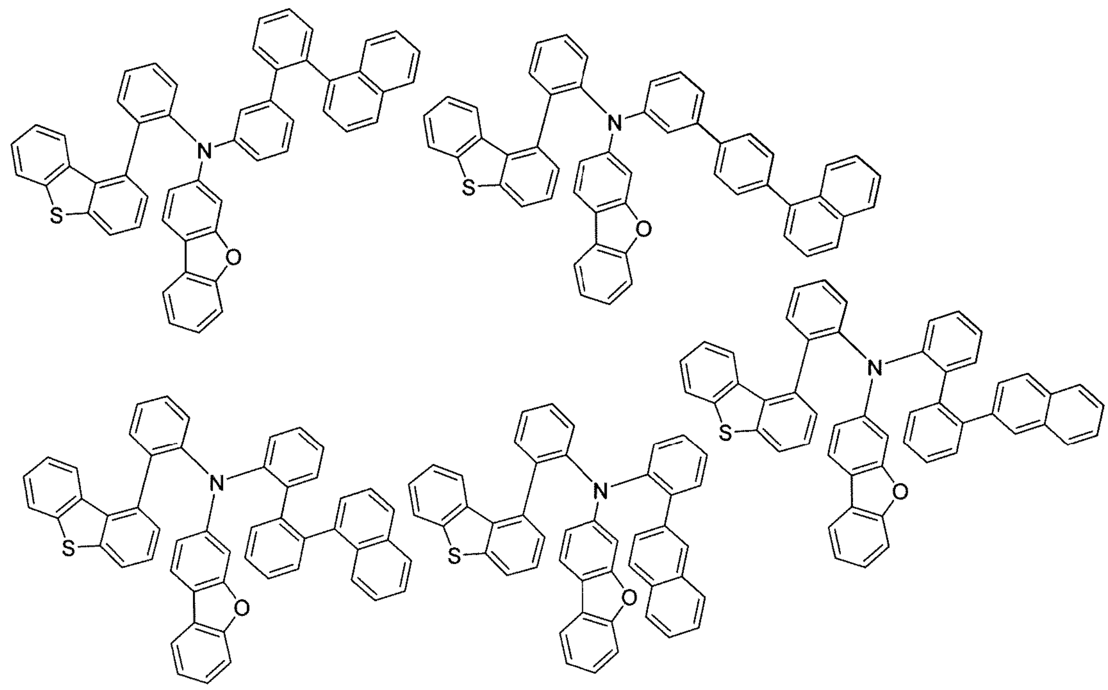

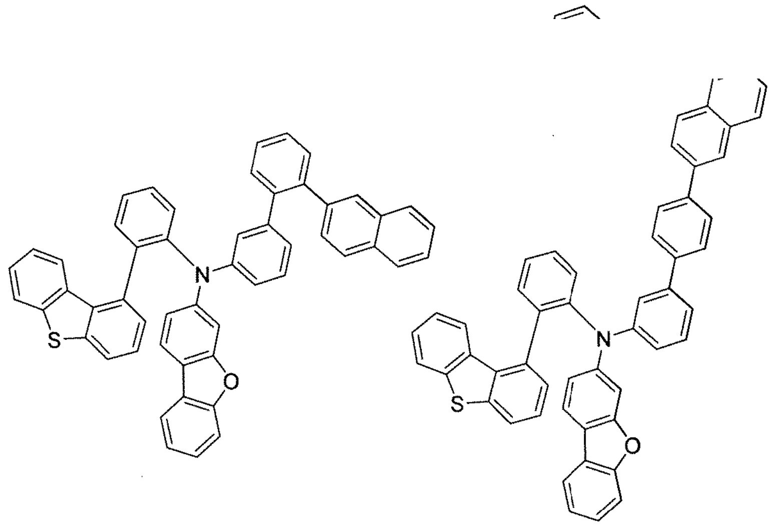

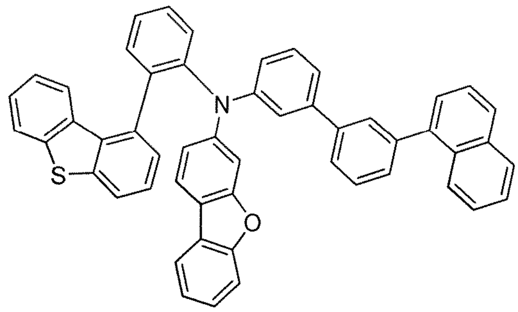

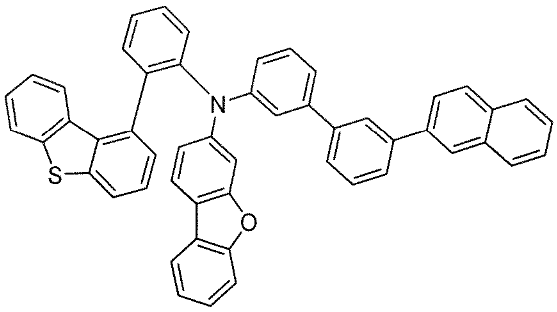

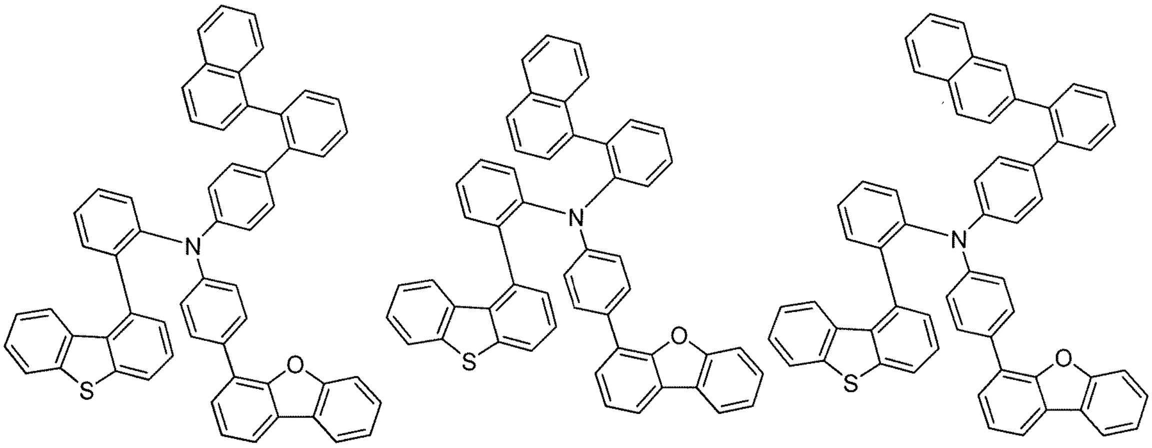

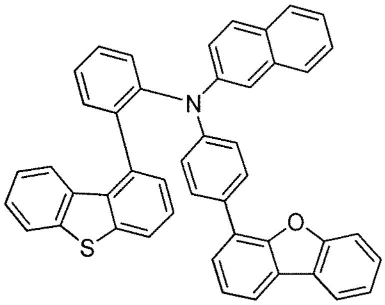

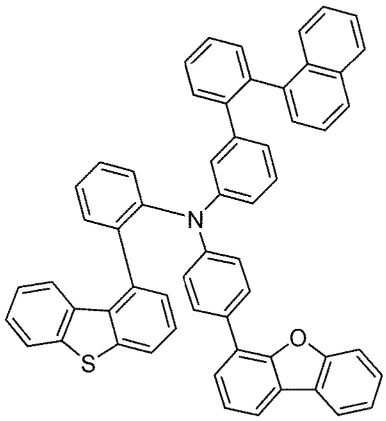

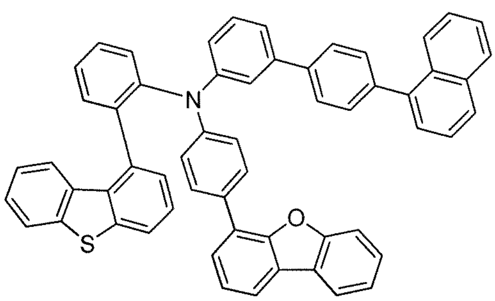

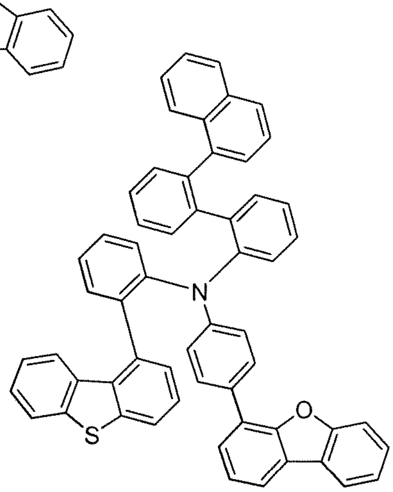

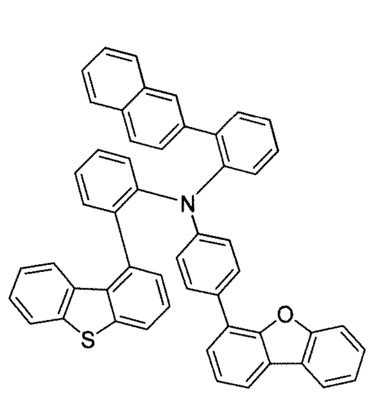

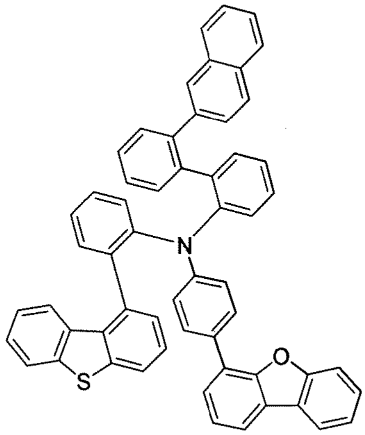

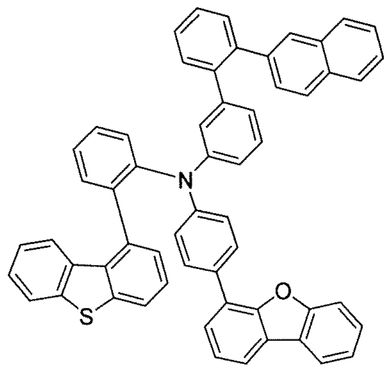

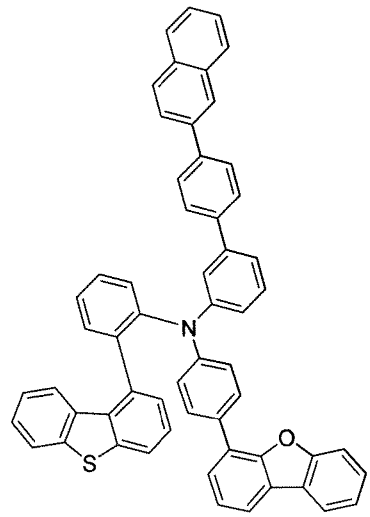

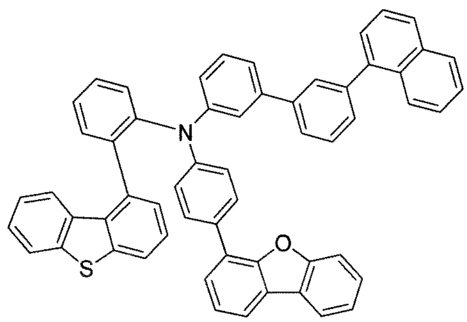

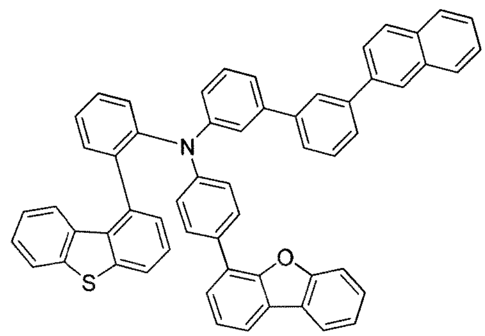

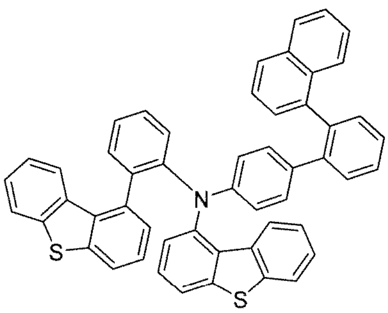

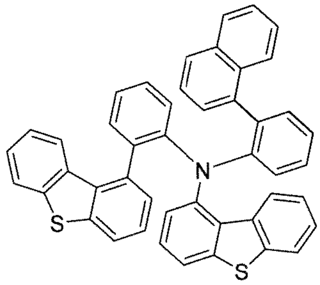

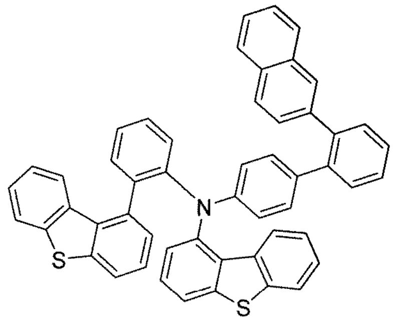

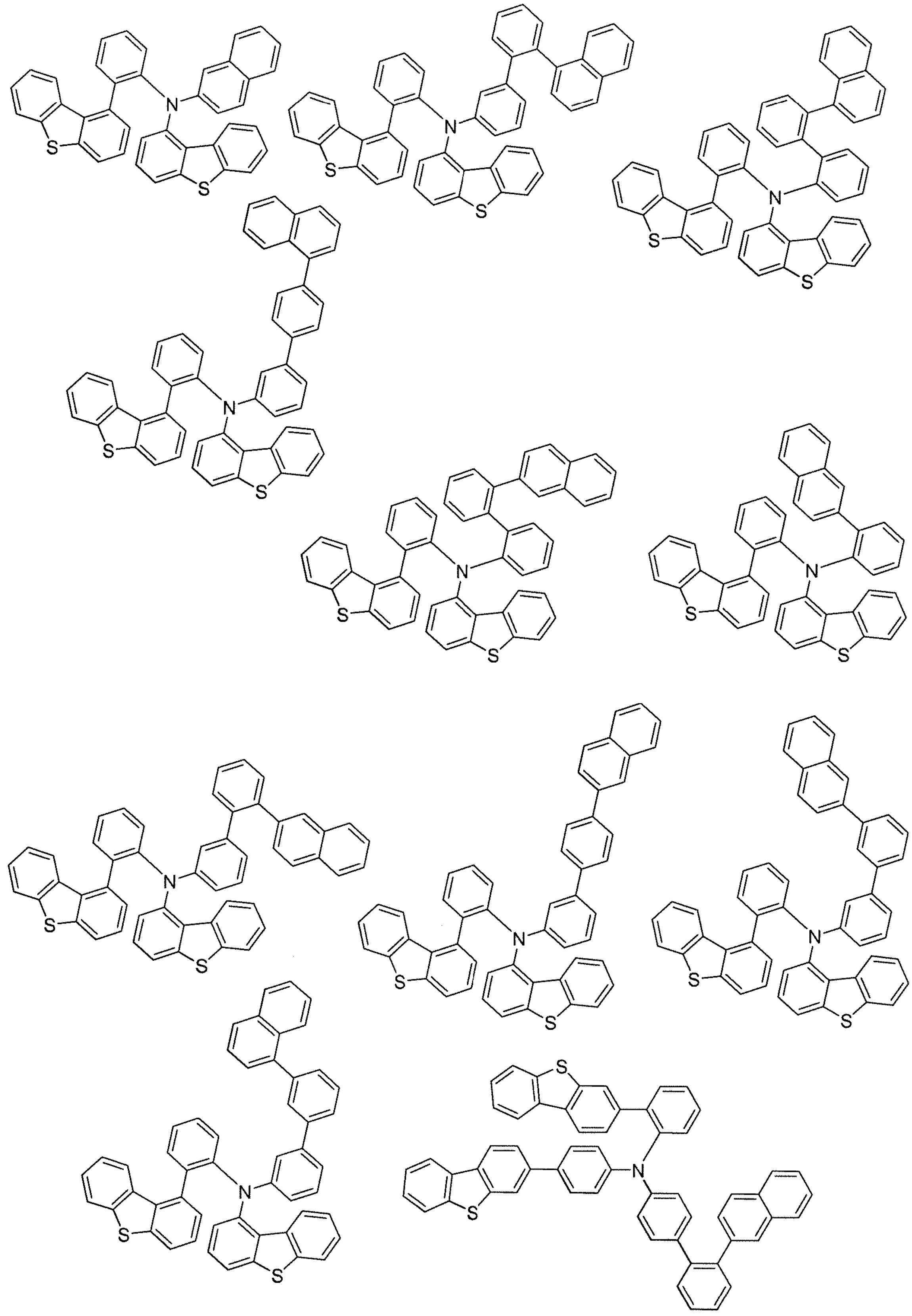

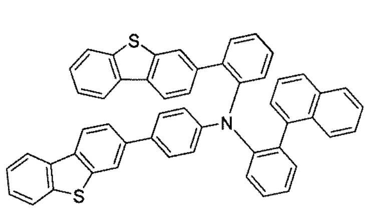
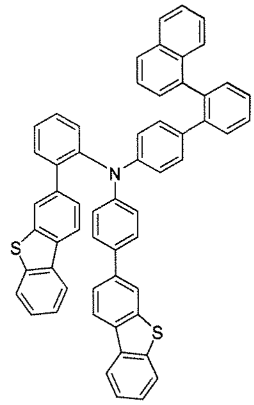
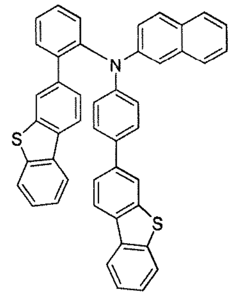

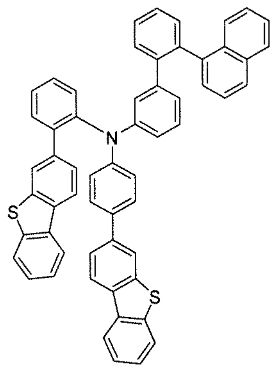
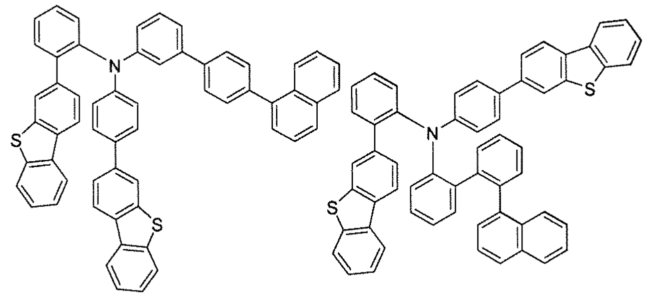

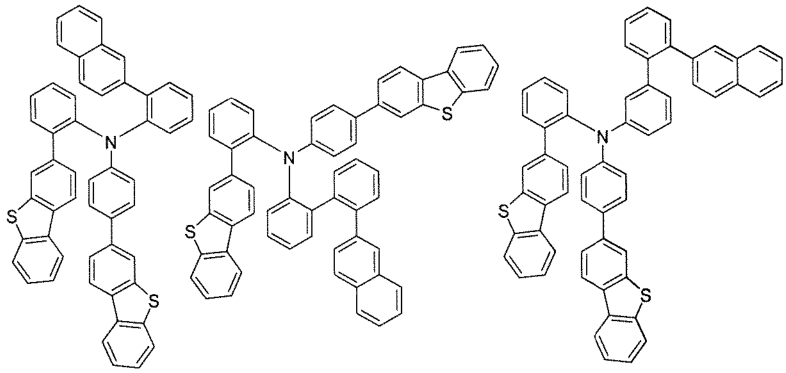

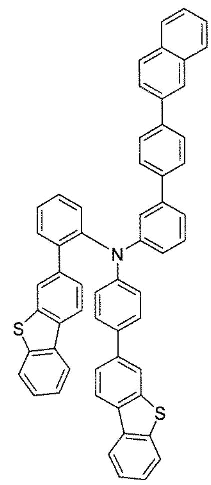
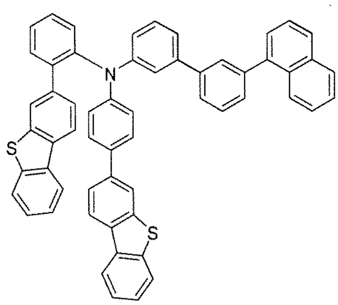
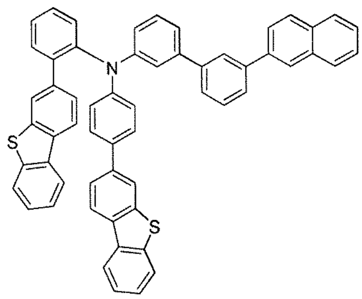

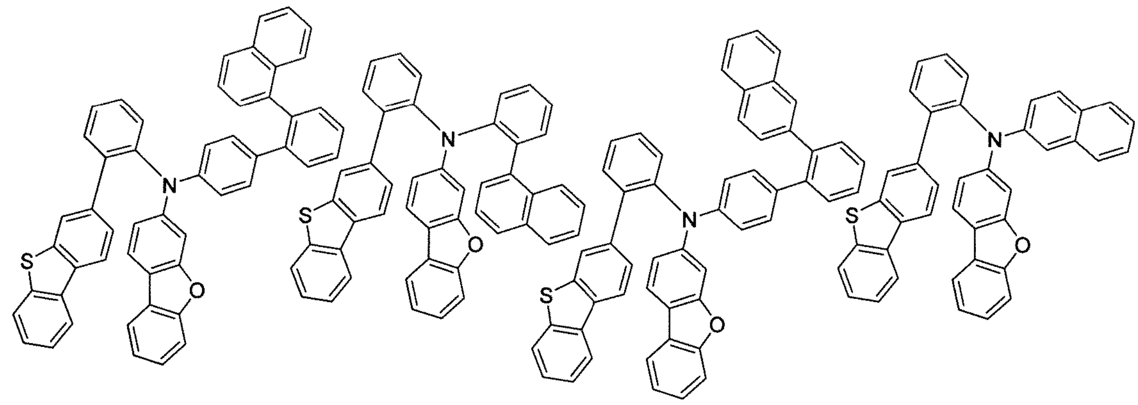

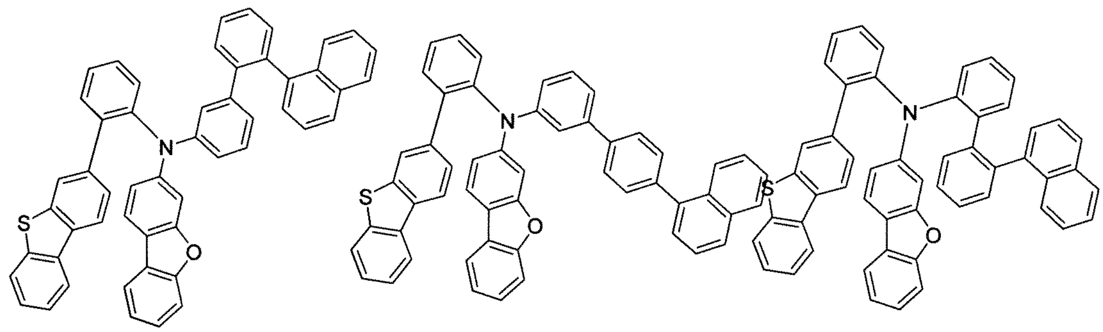

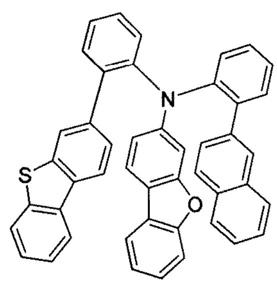

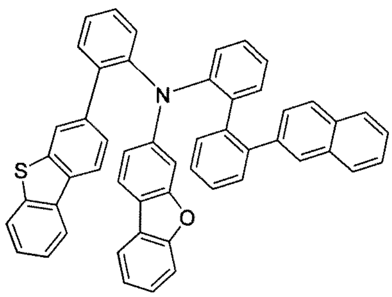

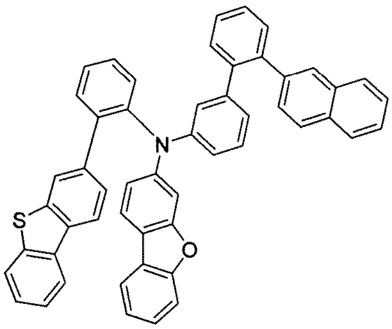

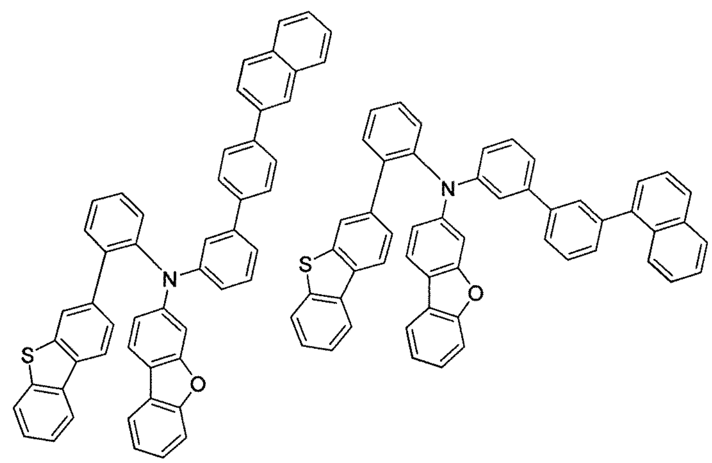

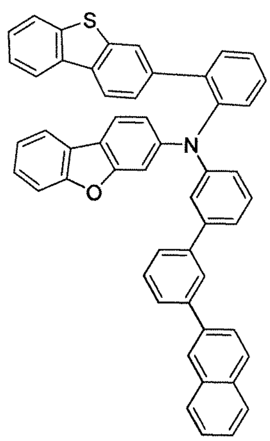

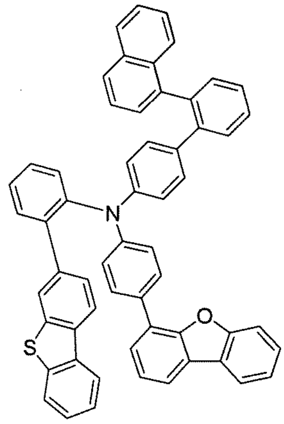
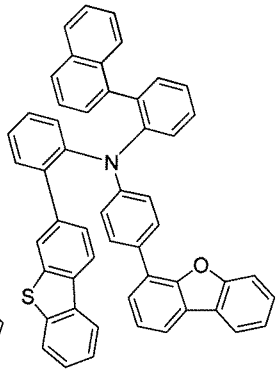
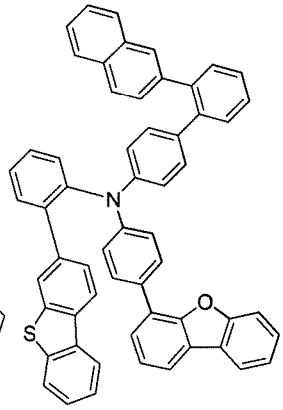

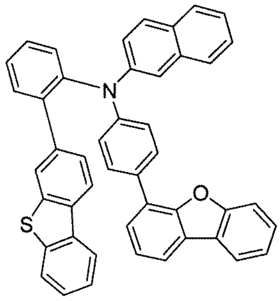
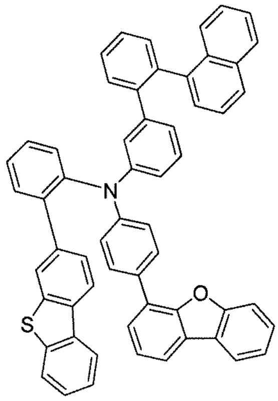
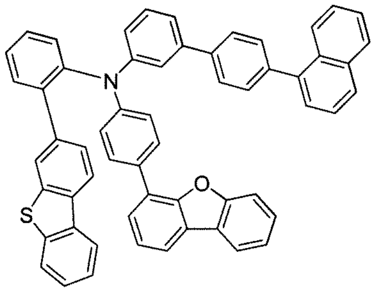

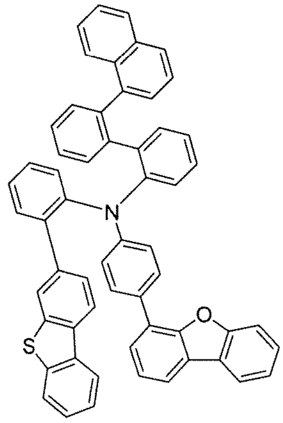
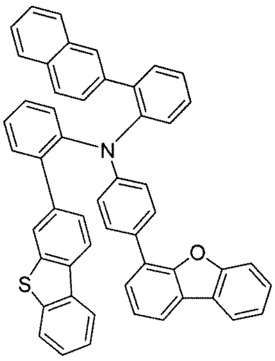
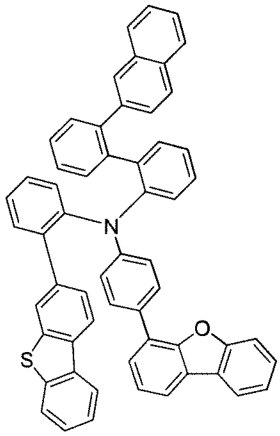

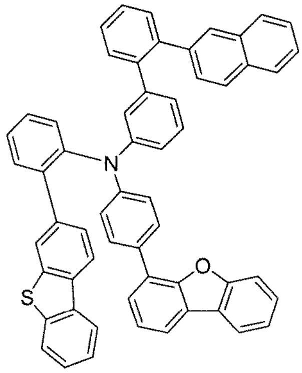

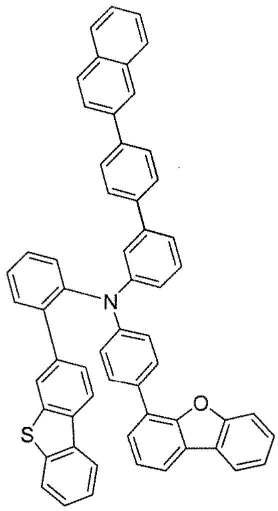

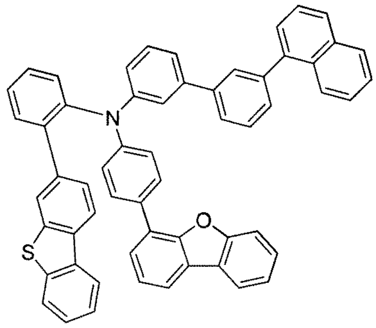

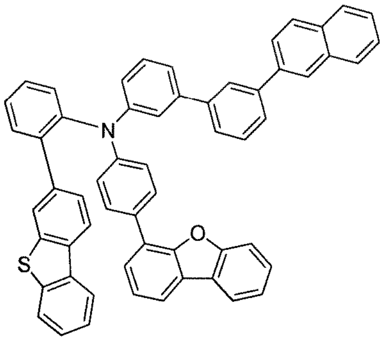

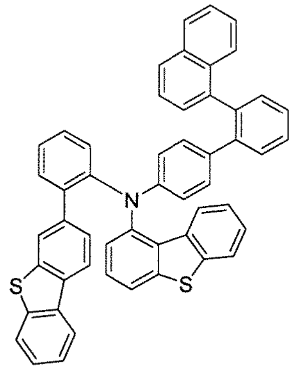

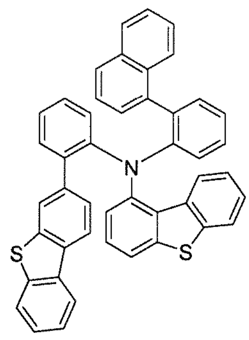

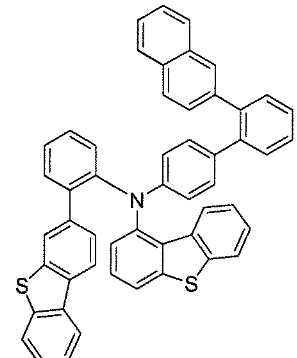

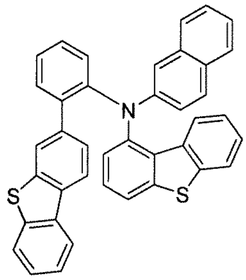

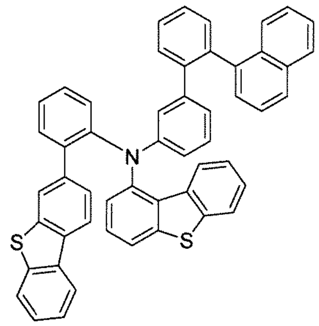

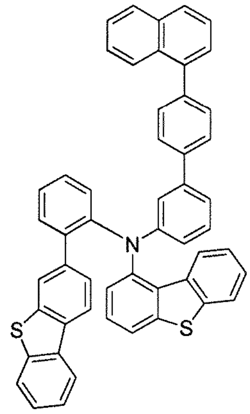

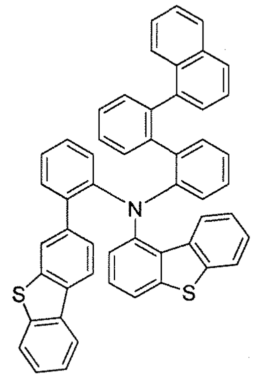

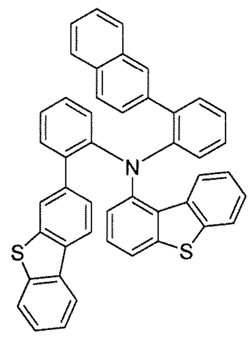

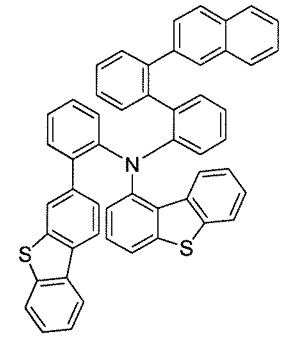
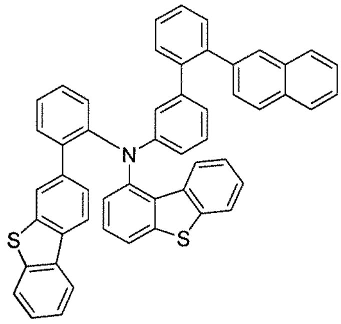
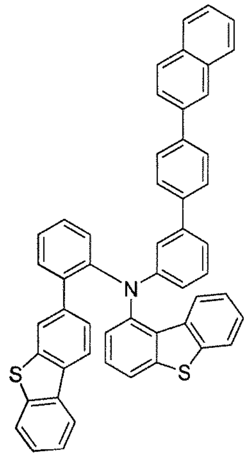

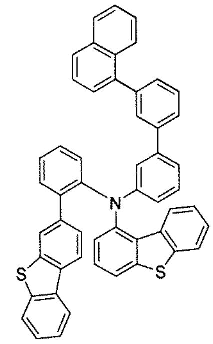
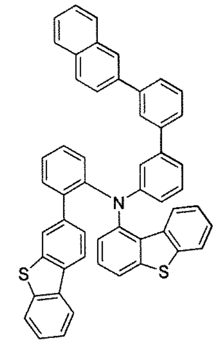

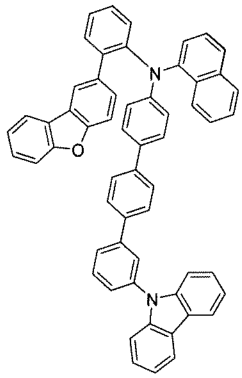
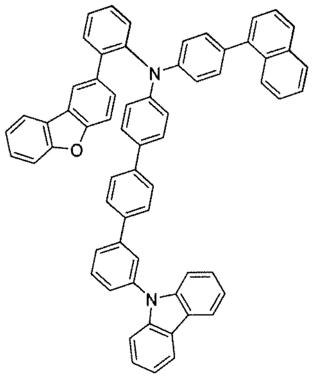
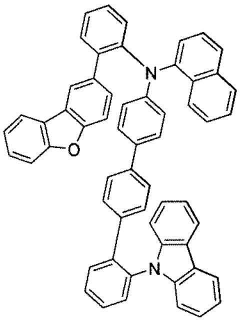

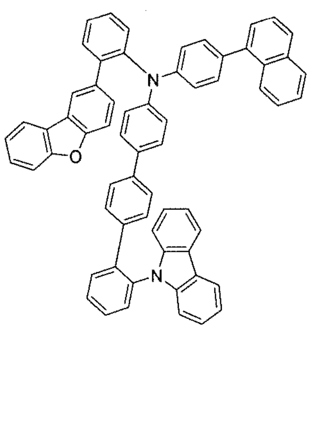
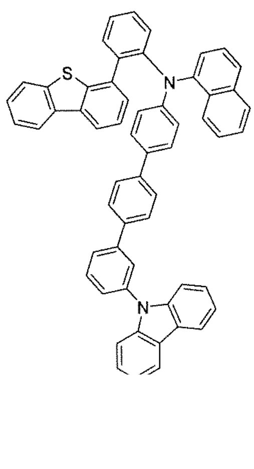
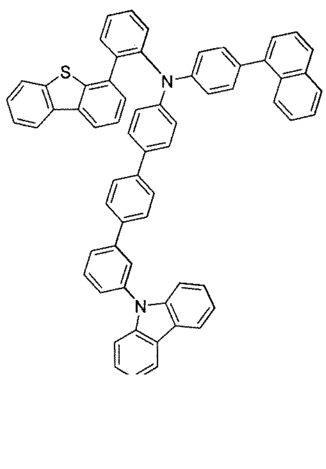

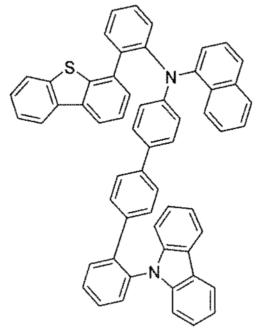
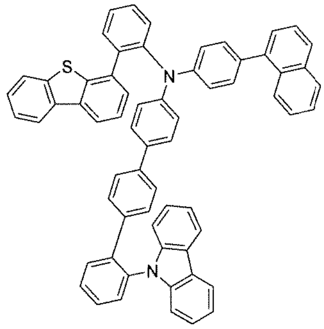
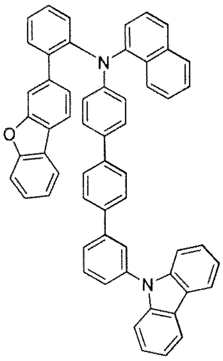

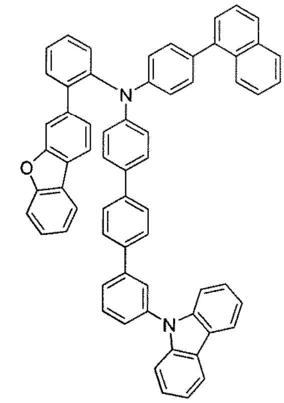
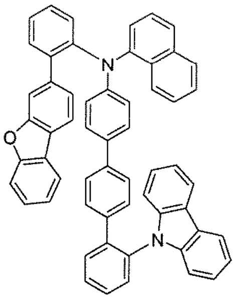
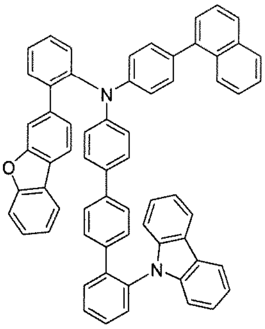

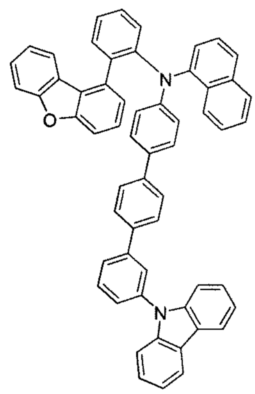
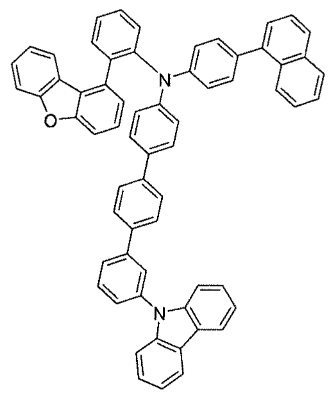
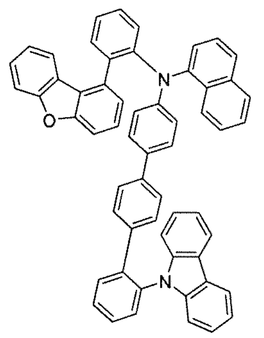

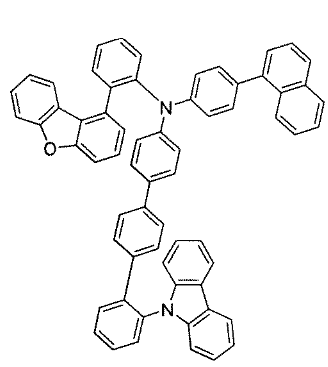
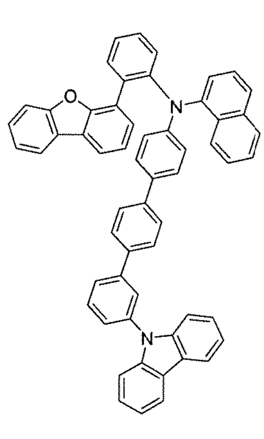

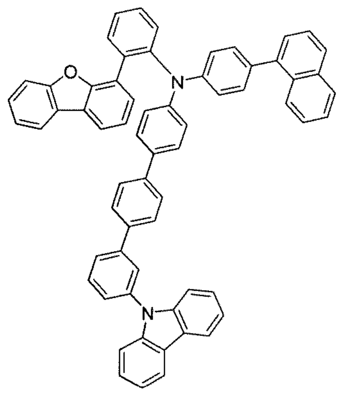

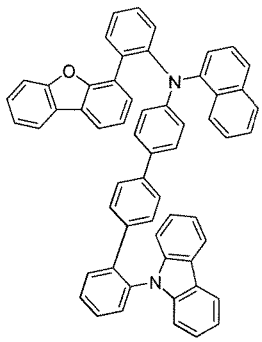

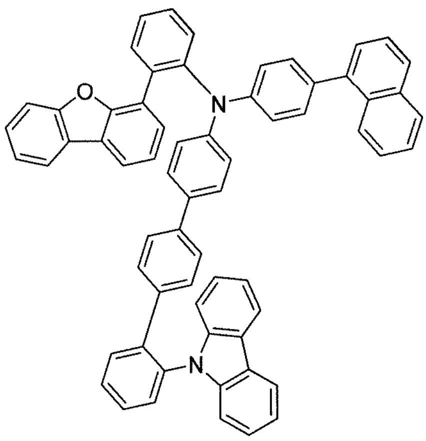

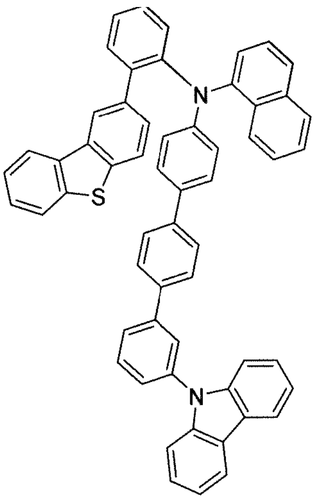

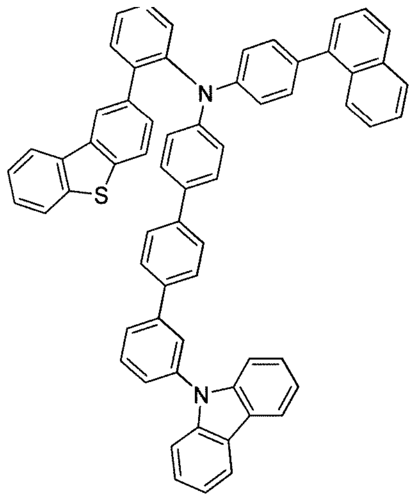

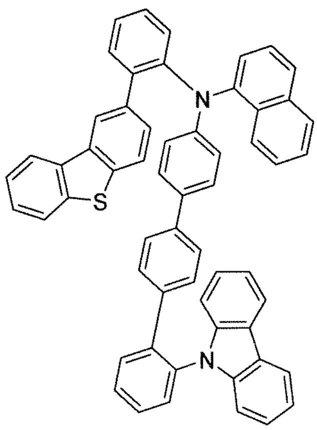

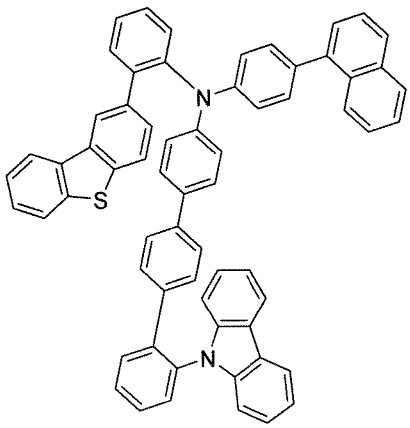

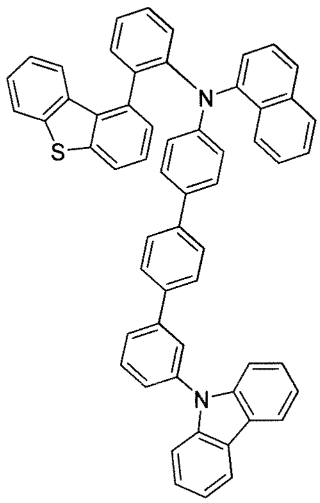

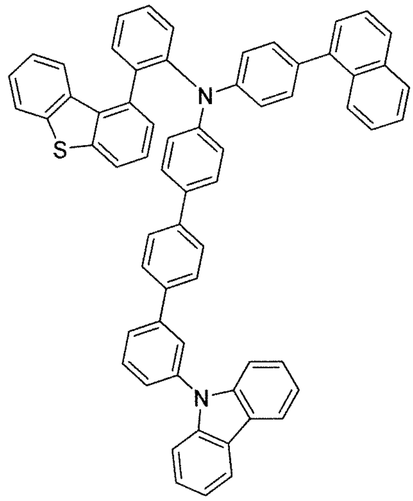

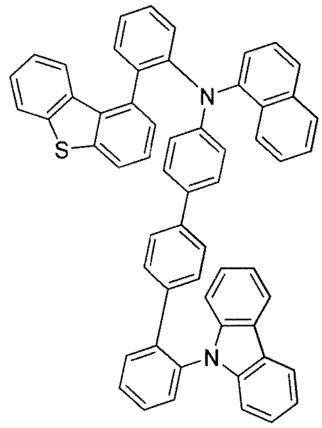

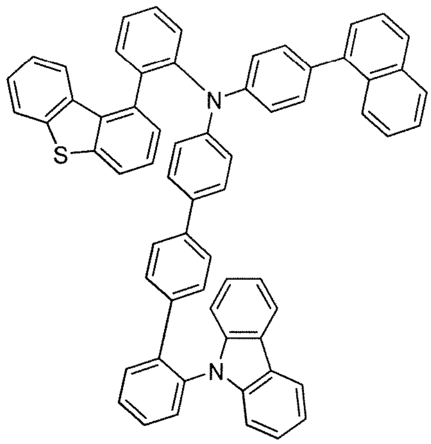

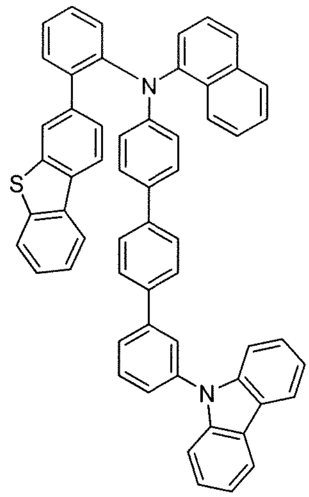

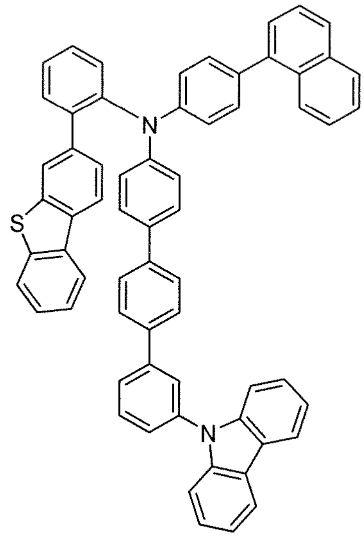

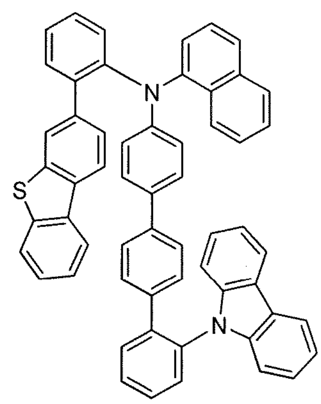

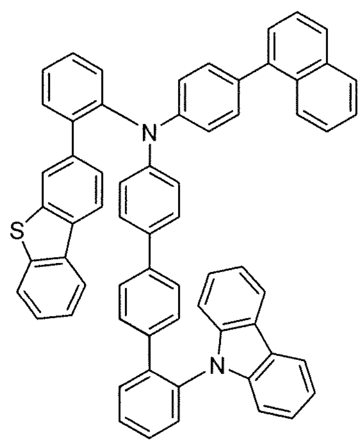

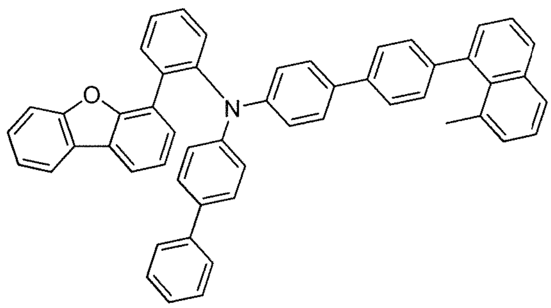

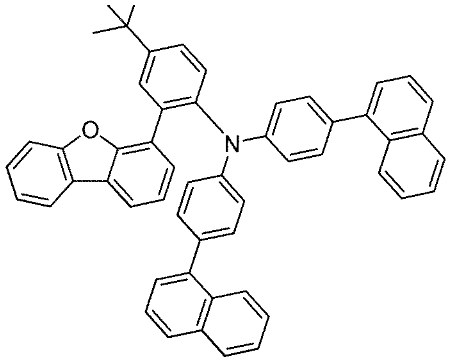

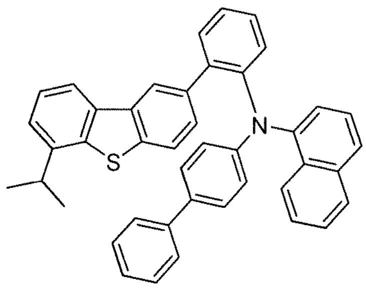

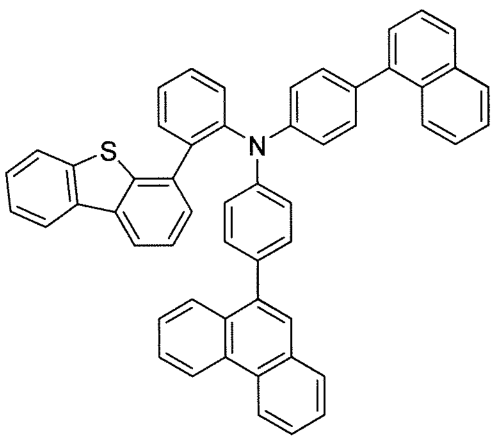

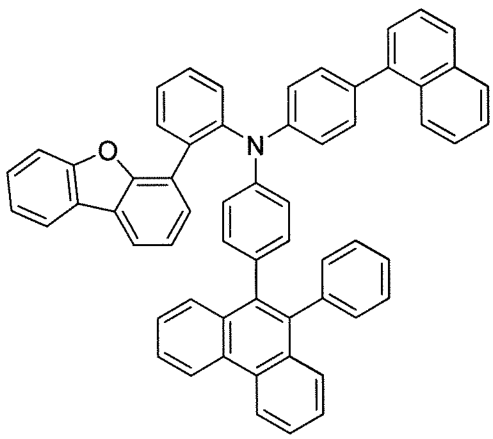

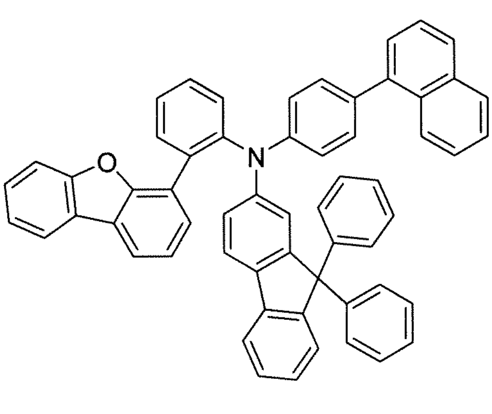

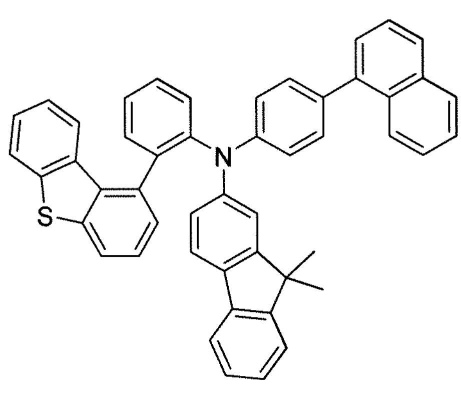

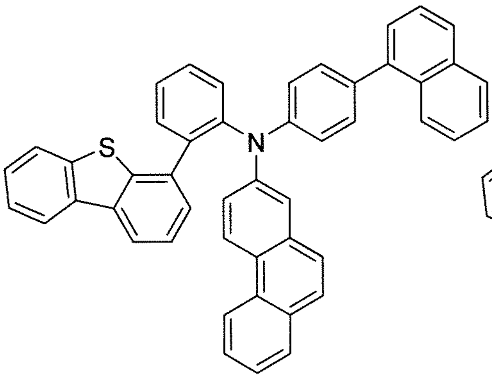

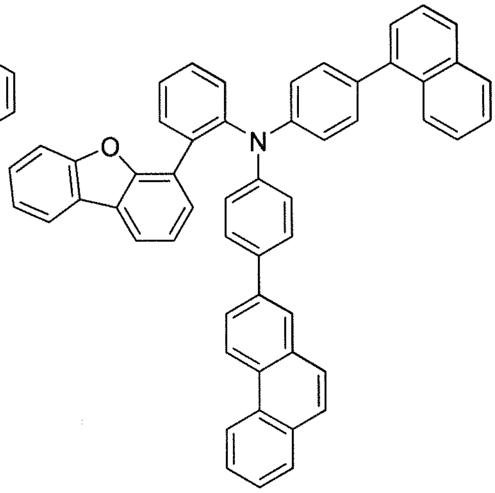

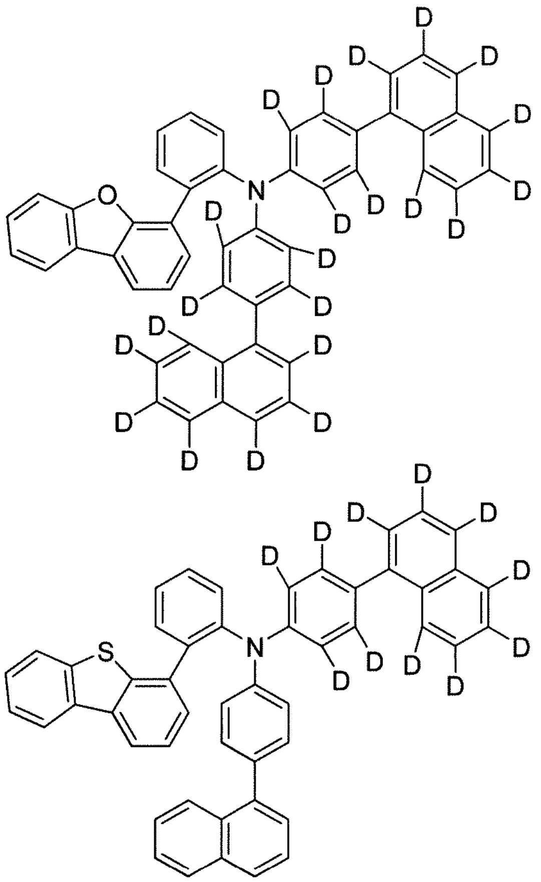

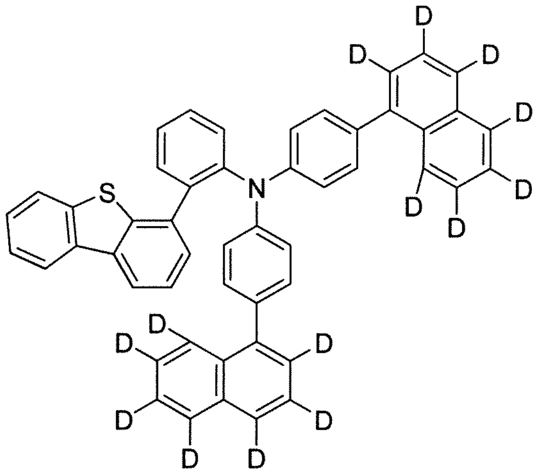

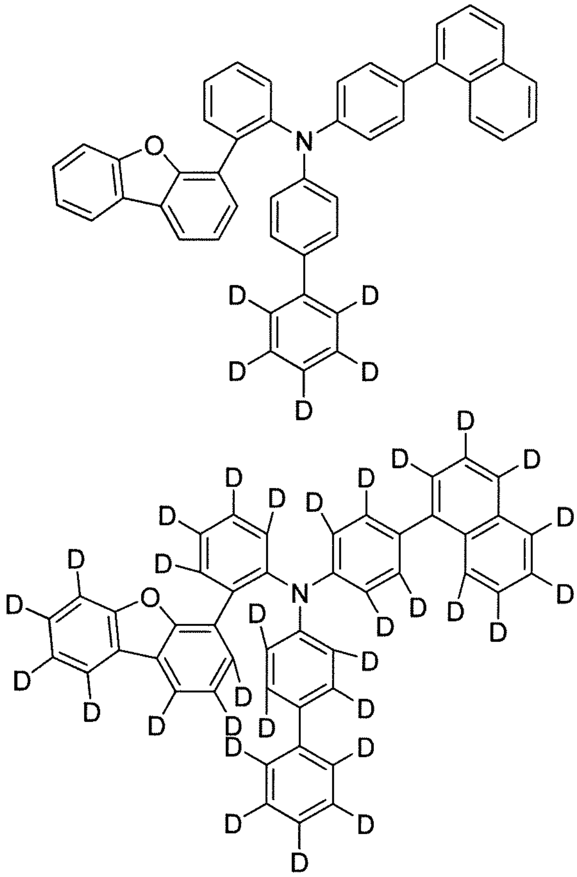

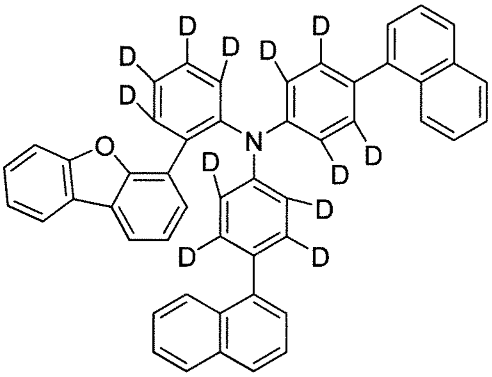

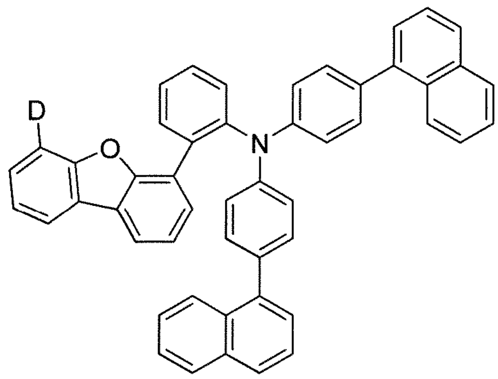

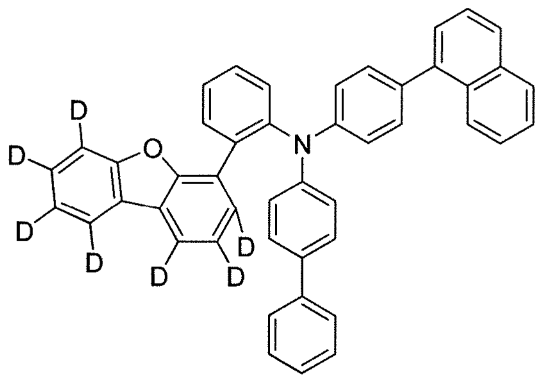

Material for Organic EL devices

The material for organic EL devices of the present invention contains the inventive compound. The content of the inventive compound in the material for organic EL devices may be 1% by mass or more (including 100%), and is preferably 10% by mass or more (including 100%), more preferably 50% by mass or more (including 100%), further preferably 80% by mass or more (including 100%), still further preferably 90% by mass or more (including 100%). The material for organic EL devices of the present invention is useful for the production of an organic EL device.

Organic EL Device

The organic EL device of the present invention includes an anode, a cathode, and organic layers intervening between the anode and the cathode. The organic layers include a light emitting layer, and at least one layer of the organic layers contains the inventive compound.

Examples of the organic layer containing the inventive compound include a hole transporting zone (such as a hole injecting layer, a hole transporting layer, an electron blocking layer, and an exciton blocking layer) intervening between the anode and the light emitting layer, the light emitting layer, a space layer, and an electron transporting zone (such as an electron injecting layer, an electron transporting layer, and a hole blocking layer) intervening between the cathode and the light emitting layer, but are not limited thereto. The inventive compound is preferably used as a material for the electron transporting zone or the light emitting layer in a fluorescent or phosphorescent EL device, more preferably as a material for the hole transporting zone, further preferably as a material for the hole injecting layer, the hole transporting layer, the electron blocking layer, or the exciton blocking layer, and particularly preferably as a material for the hole injecting layer or the hole transporting layer.

The organic EL device of the present invention may be a fluorescent or phosphorescent light emission-type monochromatic light emitting device or a fluorescent/phosphorescent hybrid-type white light emitting device, and may be a simple type having a single light emitting unit or a tandem type having a plurality of light emitting units. Above all, the fluorescent light emission-type device is preferred. The "light emitting unit" referred to herein refers to a minimum unit that emits light through recombination of injected holes and electrons, which includes organic layers among which at least one layer is a light emitting layer.

For example, as a representative device configuration of the simple type organic EL device, the following device configuration may be exemplified.

(1) Anode/Light Emitting Unit/Cathode

The light emitting unit may be a multilayer type having a plurality of phosphorescent light emitting layers or fluorescent light emitting layers. In this case, a space layer may intervene between the light emitting layers for the purpose of preventing excitons generated in the phosphorescent light emitting layer from diffusing into the fluorescent light emitting layer. Representative layer configurations of the simple type light emitting unit are described below. Layers in parentheses are optional.

(a) (hole injecting layer/) hole transporting layer/fluorescent light emitting layer/electron transporting layer (/electron injecting layer)
(b) (hole injecting layer/) hole transporting layer/phosphorescent light emitting layer/electron transporting layer (/electron injecting layer)
(c) (hole injecting layer/) hole transporting layer/first fluorescent light emitting layer/second fluorescent light emitting

layer/electron transporting layer (/electron injecting layer)

(d) (hole injecting layer/) hole transporting layer/first phosphorescent light emitting layer/second phosphorescent light emitting layer/electron transporting layer (/electron injecting layer)

(e) (hole injecting layer/) hole transporting layer/phosphorescent light emitting layer/space layer/fluorescent light emitting layer/electron transporting layer (/electron injecting layer)

(f) (hole injecting layer/) hole transporting layer/first phosphorescent light emitting layer/second phosphorescent light emitting layer/space layer/fluorescent light emitting layer/electron transporting layer (/electron injecting layer)

(g) (hole injecting layer/) hole transporting layer/first phosphorescent light emitting layer/space layer/second phosphorescent light emitting layer/space layer/fluorescent light emitting layer/electron transporting layer (/electron injecting layer)

(h) (hole injecting layer/) hole transporting layer/phosphorescent light emitting layer/space layer/first fluorescent light emitting layer/second fluorescent light emitting layer/electron transporting layer (/electron injecting layer)

(i) (hole injecting layer/) hole transporting layer/electron blocking layer/fluorescent light emitting layer/electron transporting layer (/electron injecting layer)

(j) (hole injecting layer/) hole transporting layer/electron blocking layer/phosphorescent light emitting layer/electron transporting layer (/electron injecting layer)

(k) (hole injecting layer/) hole transporting layer/exciton blocking layer/fluorescent light emitting layer/electron transporting layer (/electron injecting layer)

(l) (hole injecting layer/) hole transporting layer/exciton blocking layer/phosphorescent light emitting layer/electron transporting layer (/electron injecting layer)

(m) (hole injecting layer/) first hole transporting layer/second hole transporting layer/fluorescent light emitting layer/electron transporting layer (/electron injecting layer)

(n) (hole injecting layer/) first hole transporting layer/second hole transporting layer/phosphorescent light emitting layer/electron transporting layer (/electron injecting layer)

(o) (hole injecting layer/) first hole transporting layer/second hole transporting layer/fluorescent light emitting layer/first electron transporting layer/second electron transporting layer (/electron injecting layer)

(p) (hole injecting layer/) first hole transporting layer/second hole transporting layer/phosphorescent light emitting layer/first electron transporting layer/second electron transporting layer (/electron injecting layer)

(q) (hole injecting layer/) hole transporting layer/fluorescent light emitting layer/hole blocking layer/electron transporting layer (/electron injecting layer)

(r) (hole injecting layer/) hole transporting layer/phosphorescent light emitting layer/hole blocking layer/electron transporting layer (/electron injecting layer)

(s) (hole injecting layer/) hole transporting layer/fluorescent light emitting layer/exciton blocking layer/electron transporting layer (/electron injecting layer)

(t) (hole injecting layer/) hole transporting layer/phosphorescent light emitting layer/exciton blocking layer/electron transporting layer (/electron injecting layer)

The phosphorescent and fluorescent light emitting layers may emit emission colors different from each other, respectively. Specifically, in the light emitting unit (f), a layer configuration, such as (hole injecting layer/) hole transporting layer/first phosphorescent light emitting layer (red light emission)/second phosphorescent light emitting layer (green light emission)/space layer/fluorescent light emitting layer (blue light emission)/electron transporting layer, may be exemplified.

An electron blocking layer may be properly provided between each light emitting layer and the hole transporting layer or the space layer. A hole blocking layer may be properly provided between each light emitting layer and the electron transporting layer. The employment of the electron blocking layer or the hole blocking layer allows to improve the emission efficiency by trapping electrons or holes within the light emitting layer and increasing the probability of charge recombination in the light emitting layer.

As a representative device configuration of the tandem type organic EL device, the following device configuration may be exemplified.

(2) Anode/First Light Emitting Unit/Intermediate Layer/Second Light Emitting Unit/Cathode

For example, each of the first light emitting unit and the second light emitting unit may be independently selected from the above-described light emitting units.

The intermediate layer is also generally referred to as an intermediate electrode, an intermediate conductive layer, a charge generation layer, an electron withdrawing layer, a connecting layer, or an intermediate insulating layer, and a known material configuration can be used, in which electrons are supplied to the first light emitting unit, and holes are supplied to the second light emitting unit.

Fig. 1 is a schematic illustration showing an example of the configuration of the organic EL device of the present

invention. The organic EL device 1 of this example includes a substrate 2, an anode 3, a cathode 4, and a light emitting unit 10 disposed between the anode 3 and the cathode 4. The light emitting unit 10 includes a light emitting layer 5. A hole transporting zone 6 (such as a hole injecting layer and a hole transporting layer) is provided between the light emitting layer 5 and the anode 3, and an electron transporting zone 7 (such as an electron injecting layer and an electron transporting layer) is provided between the light emitting layer 5 and the cathode 4. In addition, an electron blocking layer (which is not shown in the figure) may be provided on the side of the anode 3 of the light emitting layer 5, and a hole blocking layer (which is not shown in the figure) may be provided on the side of the cathode 4 of the light emitting layer 5. According to the configuration, electrons and holes are trapped in the light emitting layer 5, thereby enabling one to further increase the production efficiency of excitons in the light emitting layer 5.

Fig. 2 is a schematic illustration showing another configuration of the organic EL device of the present invention. An organic EL device 11 includes the substrate 2, the anode 3, the cathode 4, and a light emitting unit 20 disposed between the anode 3 and the cathode 4. The light emitting unit 20 includes the light emitting layer 5. A hole transporting zone disposed between the anode 3 and the light emitting layer 5 includes a hole injecting layer 6a, a first hole transporting layer 6b and a second hole transporting layer 6c. The electron transporting zone disposed between the light emitting layer 5 and the cathode 4 includes an electron transporting layer 7.

In the present invention, a host combined with a fluorescent dopant (a fluorescent emitting material) is referred to as a fluorescent host, and a host combined with a phosphorescent dopant is referred to as a phosphorescent host. The fluorescent host and the phosphorescent host are not distinguished from each other merely by the molecular structures thereof. Specifically, the phosphorescent host means a material that forms a phosphorescent light emitting layer containing a phosphorescent dopant, but does not mean unavailability as a material that forms a fluorescent light emitting layer. The same also applies to the fluorescent host.

Substrate

The substrate is used as a support of the organic EL device. Examples of the substrate include a plate of glass, quartz, and plastic. In addition, a flexible substrate may be used. Examples of the flexible substrate include a plastic substrate made of polycarbonate, polyarylate, polyether sulfone, polypropylene, polyester, polyvinyl fluoride, or polyvinyl chloride. In addition, an inorganic vapor deposition film can be used.

Anode

It is preferred that a metal, an alloy, an electrically conductive compound, or a mixture thereof which has a high work function (specifically 4.0 eV or more) is used for the anode formed on the substrate. Specific examples thereof include indium oxide-tin oxide (ITO: Indium Tin Oxide), indium oxide-tin oxide containing silicon or silicon oxide, indium oxide-zinc oxide, indium oxide containing tungsten oxide and zinc oxide, and graphene. Besides, examples thereof include gold (Au), platinum (Pt), nickel (Ni), tungsten (W), chromium (Cr), molybdenum (Mo), iron (Fe), cobalt (Co), copper (Cu), palladium (Pd), titanium (Ti), or nitrides of the metals (for example, titanium nitride).

These materials are usually deposited by a sputtering method. For example, through a sputtering method, it is possible to form indium oxide-zinc oxide by using a target in which 1 to 10 wt% of zinc oxide is added to indium oxide, and to form indium oxide containing tungsten oxide and zinc oxide by using a target containing 0.5 to 5 wt% of tungsten oxide and 0.1 to 1 wt% of zinc oxide with respect to indium oxide. Besides, the manufacturing may be performed by a vacuum vapor deposition method, a coating method, an inkjet method, a spin coating method, or the like.

The hole injecting layer formed in contact with the anode is formed by using a material that facilitates hole injection regardless of a work function of the anode, and thus, it is possible to use materials generally used as an electrode material (for example, metals, alloys, electrically conductive compounds, or mixtures thereof, elements belonging to Group 1 or 2 of the periodic table of the elements).

It is also possible to use elements belonging to Group 1 or 2 of the periodic table of the elements, which are materials having low work functions, that is, alkali metals, such as lithium (Li) and cesium (Cs), alkaline earth metals, such as magnesium (Mg), calcium (Ca), and strontium (Sr), and alloys containing these (such as MgAg and AlLi), and rare earth metals, such as europium (Eu), and ytterbium (Yb) and alloys containing these. When the anode is formed by using the alkali metals, the alkaline earth metals, and alloys containing these, a vacuum vapor deposition method or a sputtering method can be used. Further, when a silver paste or the like is used, a coating method, an inkjet method, or the like can be used.

Hole Injecting Layer

The hole injecting layer is a layer containing a material having a high hole injection capability (a hole injecting material) and is provided between the anode and the light emitting layer, or between the hole transporting layer, if exists,

and the anode. The inventive compound can be used in the hole injecting layer.

As the hole injecting material except the inventive compound, molybdenum oxide, titanium oxide, vanadium oxide, rhenium oxide, ruthenium oxide, chromium oxide, zirconium oxide, hafnium oxide, tantalum oxide, silver oxide, tungsten oxide and manganese oxide can be used.

Examples of the hole injecting layer material also include aromatic amine compounds as low-molecular weight organic compounds, such as 4,4',4"-tris(N,N-diphenylamino)triphenylamine (abbreviation: TDATA), 4,4', 4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1).

High-molecular weight compounds (such as oligomers, dendrimers, and polymers) may also be used. Examples thereof include high-molecular weight compounds, such as poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). In addition, high-molecular weight compounds to which an acid is added, such as poly(3,4-ethylenedioxythiophene)/poly (styrene sulfonic acid) (PEDOT/PSS), and polyaniline/poly (styrenesulfonic acid) (PAni/PSS), can also be used.

Furthermore, it is also preferred to use an acceptor material, such as a hexaazatriphenylene (HAT) compound represented by formula (K).

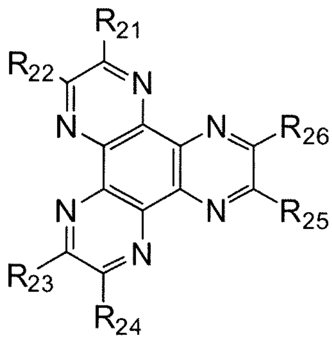

(K)

In the aforementioned formula, $R_{21}$ to $R_{26}$ each independently represent a cyano group, $-CONH_2$, a carboxy group, or $-COOR_{27}$ ($R_{27}$ represents an alkyl group having 1 to 20 carbon atoms or a cycloalkyl group having 3 to 20 carbon atoms). In addition, adjacent two selected from $R_{21}$ and $R_{22}$, $R_{23}$ and $R_{24}$, and $R_{25}$ and $R_{26}$ may be bonded to each other to form a group represented by -CO-O-CO-.

Examples of $R_{27}$ include a methyl group, an ethyl group, an n-propyl group, an isopropyl group, an n-butyl group, an isobutyl group, a t-butyl group, a cyclopentyl group, and a cyclohexyl group.

Hole Transporting Layer

The hole transporting layer is a layer containing a material having a high hole transporting capability (a hole transporting material) and is provided between the anode and the light emitting layer, or between the hole injecting layer, if exists, and the light emitting layer. Preferably, the inventive compound can be used as the hole transporting layer either singly or as combined with the compound mentioned below.

The hole transporting layer may have a single layer structure or a multilayer structure including two or more layers. For example, the hole transporting layer may have a two-layer structure including a first hole transporting layer (anode side) and a second hole transporting layer (cathode side). In one embodiment of the present invention, the hole transporting layer having a single layer structure is preferably disposed adjacent to the light emitting layer, and the hole transporting layer that is closest to the cathode in the multilayer structure, such as the second hole transporting layer in the two-layer structure, is preferably disposed adjacent to the light emitting layer. In another embodiment of the present invention, and an electron blocking layer described later may be disposed between the hole transporting layer having a single layer structure and the light emitting layer, or between the hole transporting layer that is closest to the light emitting layer in the multilayer structure and the light emitting layer.

In the hole transporting layer of a two-layer structure, the inventive compound may be contained in the first hole transporting layer and the second hole transporting layer, or may be contained in the two.

In one embodiment of the present invention, the inventive compound is preferably contained in the first hole transporting layer alone, and in another embodiment, the inventive compound is preferably contained in the second hole transporting layer alone, and in still another embodiment, the inventive compound is preferably contained in the first hole transporting layer and the second hole transporting layer.

As the hole transporting layer material except the inventive compound, for example, an aromatic amine compound, a carbazole derivative, and an anthracene derivative can be used.

Examples of the aromatic amine compound include 4,4'-bis[N-(1-naphthyl)-N-phenylamino]biphenyl (abbreviation: NPB) or N,N'-bis(3-methylphenyl)-N,N'-diphenyl-[1,1'-biphenyl]-4,4'-diamine (abbreviation: TPD), 4-phenyl-4'-(9-phenylfluoren-9-yl)triphenylamine (abbreviation: BAFLP), 4,4'-bis[N-(9,9-dimethylfluoren-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluoren-2-yl)-N-phenylamino]biphenyl (abbreviation: BSPB). The aforementioned compounds have a hole mobility of $10^{-6}$ $cm^2/Vs$ or more.

Examples of the carbazole derivative include 4,4'-di(9-carbazolyl)biphenyl (abbreviation: CBP), 9- [4-(9-carbazolyl)phenyl]-10-phenylanthracene (abbreviation: CzPA), and 9-phenyl-3-[4-(10-phenyl-9-anthryl)phenyl]-9H-carbazole (abbreviation: PCzPA).

Examples of the anthracene derivative include 2-t-butyl-9,10-di(2-naphthyl)anthracene (abbreviation: t-BuDNA), 9,10-di(2-naphthyl)anthracene (abbreviation: DNA), and 9,10-diphenylanthracene (abbreviation: DPAnth).

High-molecular weight compounds, such as poly(N-vinylcarbazole) (abbreviation: PVK) and poly(4-vinyltriphenylamine) (abbreviation: PVTPA), can also be used.

However, compounds other than those as mentioned above can also be used so long as they are compounds high in the hole transporting capability rather than in the electron transporting capability.

Dopant Material of Light Emitting Layer

The light emitting layer is a layer containing a material having a high light emitting property (a dopant material), and various materials can be used. For example, a fluorescent emitting material or a phosphorescent emitting material can be used as the dopant material. The fluorescent emitting material is a compound that emits light from a singlet excited state, and the phosphorescent emitting material is a compound that emits from a light triplet excited state.

Examples of a blue-based fluorescent emitting material that can be used for the light emitting layer include a pyrene derivative, a styrylamine derivative, a chrysene derivative, a fluoranthene derivative, a fluorene derivative, a diamine derivative, and a triarylamine derivative. Specific examples thereof include N,N'-bis[4-(9H-carbazole-9-yl)phenyl]-N,N'-diphenylstilbene-4,4'-diamine (abbreviation: YGA2S), 4-(9H-carbazole-9-yl)-4'-(10-phenyl-9-anthryl)triphenylamine (abbreviation: YGAPA), and 4-(10-phenyl-9-anthryl)-4'-(9-phenyl-9H-carbazole-3-yl)triphenylamine (abbreviation: PCBAPA).

Examples of a green-based fluorescent emitting material that can be used for the light emitting layer include an aromatic amine derivative. Specific examples thereof include N-(9,10-diphenyl-2-anthryl)-N,9-diphenyl-9H-carbazole-3-amine (abbreviation: 2PCAPA), N-[9,10-bis(1,1'-biphenyl-2-yl)-2-anthryl]-N,9-diphenyl-9H-carbazole-3-amine (abbreviation: 2PCABPhA), N-(9,10-diphenyl-2-anthryl)-N,N',N'-triphenyl-1,4-phenylenediamine (abbreviation: 2DPAPA), N-[9,10-bis(1,1'-biphenyl-2-yl)-2-anthryl]-N,N',N'-triphenyl-1,4-phenylenediamine (abbreviation: 2DPABPhA), N-[9,10-bis(1,1'-biphenyl-2-yl)]-N-[4-(9H-carbazole-9-yl)phenyl]-N-phenylanthracene-2-amine (abbreviation: 2YGABPhA), and N,N,9-triphenylanthracene-9-amine (abbreviation: DPhAPhA).

Examples of a red-based fluorescent emitting material that can be used for the light emitting layer include a tetracene derivative and a diamine derivative. Specific examples thereof include N,N,N',N'-tetrakis(4-methylphenyl)tetracene-5,11-diamine (abbreviation: p-mPhTD) and 7,14-diphenyl-N,N,N',N'-tetrakis(4-methylphenyl)acenaphtho[1,2-a]fluoranthene-3,10-diamine (abbreviation: p-mPhAFD).

Examples of a blue-based phosphorescent emitting material that can be used for the light emitting layer include a metal complex, such as an iridium complex, an osmium complex, and a platinum complex. Specific examples thereof include bis[2-(4',6'-difluorophenyl)pyridinato-N,C2']iridium(III)tetrakis(1-pyrazolyl)borate (abbreviation: FIr6), bis[2-(4',6'-difluorophenyl)pyridinato-N,C2']iridium(III)picolinate (abbreviation: FIrpic), bis[2-(3',5'bistrifluoromethylphenyl)pyridinato-N,C2']iridium(III)picolinate (abbreviation: Ir(CF3ppy)2(pic)), and bis[2-(4',6'-difluorophenyl)pyridinato-N,C2']iridium(III)acetylacetonate (abbreviation: FIracac).

Examples of a green-based phosphorescent emitting material that can be used for the light emitting layer include an iridium complex. Examples thereof include tris(2-phenylpyridinato-N,C2')iridium(III) (abbreviation: Ir(ppy)3), bis(2-phenylpyridinato-N,C2')iridium(III)acetylacetonate (abbreviation: Ir(ppy)2(acac)), bis(1,2-diphenyl-1H-benzimidazolato)iridium(III)acetylacetonate (abbreviation: Ir(pbi)2(acac)), and bis(benzo[h]quinolinato)iridium(III)acetylacetonate (abbreviation: Ir(bzq)2(acac)).

Examples of a red-based phosphorescent emitting material that can be used for the light emitting layer include a metal complex, such as an iridium complex, a platinum complex, a terbium complex, and a europium complex. Specific

examples thereof include organic metal complexes, such as bis[2-(2'-benzo[4,5-α]thienyl)pyridinato-N,C3']iridium(III)acetylacetonate (abbreviation: Ir(btp)2(acac)), bis(1-phenylisoquinolinato-N,C2')iridium(III)acetylacetonate (abbreviation: Ir(piq)2(acac)), (acetylacetonate)bis[2,3-bis(4-fluorophenyl)quinoxalinato]iridium(III) (abbreviation: Ir(Fdpq)2(acac)), and 2,3,7,8,12,13,17,18-octaethyl-21H,23H-porphyrinplatinum(II) (abbreviation: PtOEP).

Rare earth metal complexes, such as tris(acetylacetonate) (monophenanthroline)terbium(III) (abbreviation: Tb(acac)3(Phen)), tris(1,3-diphenyl-1,3- propanedionate)(monophenanthroline)europium(III) (abbreviation: Eu(DBM)3(Phen)), and tris[1-(2-thenoyl)-3,3,3-trifluoroacetonate](monophenanthroline)europium(III) (abbreviation: Eu(TTA)3(Phen)), emit light from rare earth metal ions (electron transition between different multiplicities), and thus may be used as the phosphorescent emitting material.

Host Material of Light Emitting Layer

The light emitting layer may have a configuration in which the aforementioned dopant material is dispersed in another material (a host material). The host material is preferably a material that has a higher lowest unoccupied orbital level (LUMO level) and a lower highest occupied orbital level (HOMO level) than the dopant material.

Examples of the host material include:

(1) a metal complex, such as an aluminum complex, a beryllium complex, and a zinc complex,
(2) a heterocyclic compound, such as an oxadiazole derivative, a benzimidazole derivative, and a phenanthroline derivative,
(3) a fused aromatic compound, such as a carbazole derivative, an anthracene derivative, a phenanthrene derivative, a pyrene derivative, and a chrysene derivative, or
(4) an aromatic amine compound, such as a triarylamine derivative and a fused polycyclic aromatic amine derivative.

For example,

metal complexes, such as tris(8-quinolinolato)aluminum(III) (abbreviation: Alq), tris(4-methyl-8-quinolinolato)aluminum(III) (abbreviation: Almq3), bis(10-hydroxybenzo[h]quinolinato)beryllium(II) (abbreviation: BeBq2), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ);

heterocyclic compounds, such as 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-biphenylyl)-4-phenyl-5-(4-tert-butylphenyl)-1,2,4-triazole (abbreviation: TAZ), 2,2',2"-(1,3,5-benzenetriyl)tris(1-phenyl-1H-benzimidazole) (abbreviation: TPBI), and bathophenanthroline (abbreviation: BPhen), bathocuproine (abbreviation: BCP);

fused aromatic compounds, such as 9-[4-(10-phenyl-9-anthryl)phenyl]-9H-carbazole (abbreviation: CzPA), 3,6-diphenyl-9-[4-(10-phenyl-9-anthryl)phenyl]-9H-carbazole (abbreviation: DPCzPA), 9,10bis(3,5-diphenylphenyl)anthracene (abbreviation: DPPA), 9,10-di(2-naphthyl)anthracene (abbreviation: DNA), 2-tert-butyl-9,10-di(2-naphthyl)anthracene (abbreviation: t-BuDNA), 9,9'-bianthryl(abbreviation: BANT), 9,9'-(stilbene-3,3'-diyl)diphenanthrene (abbreviation: DPNS), 9,9'-(stilbene-4,4'-diyl)diphenanthrene (abbreviation: DPNS2), 3,3',3"-(benzene-1,3,5-triyl)tripyrene (abbreviation: TPB3), 9,10-diphenylanthracene (abbreviation: DPAnth), and 6,12-dimethoxy-5,11-diphenylchrysene; and

aromatic amine compounds, such as N,N-diphenyl-9-[4-(10-phenyl-9-anthryl)phenyl]-9H-carbazole-3-amine (abbreviation: CzA1PA), 4-(10-phenyl-9-anthryl)triphenylamine (abbreviation: DPhPA), N,9-diphenyl-N-[4-(10-phenyl-9-anthryl)phenyl]-9H-carbazole-3-amine (abbreviation: PCAPA), N,9-diphenyl-N-{4-[4-(10-phenyl-9-anthryl)phenyl]phenyl}-9H-carbazole-3-amine (abbreviation: PCAPBA), N-(9,10-diphenyl-2-anthryl)-N,9-diphenyl-9H-carbazole-3-amine (abbreviation: 2PCAPA), 4,4'-bis[N-(1-naphthyl)-N-phenylamino]biphenyl (abbreviation: NPB or α-NPD), N,N'-bis(3-methylphenyl)-N,N'-diphenyl-[1,1'-biphenyl]-4,4'-diamine (abbreviation: TPD), 4,4'-bis[N-(9,9-dimethylfluoren-2-yl)-N-phenylamino]biphenyl (abbreviation: DFLDPBi), and 4,4'-bis[N-(spiro-9,9'-bifluoren-2-yl)-N-phenylamino]biphenyl (abbreviation: BSPB) can be used. A plurality of host materials may be used.

In particular, in the case of a blue fluorescent device, it is preferred to use the following anthracene compounds as the host material.

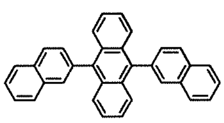 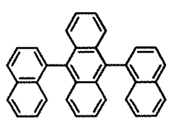 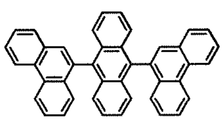 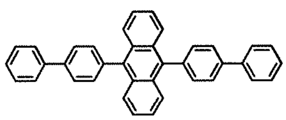

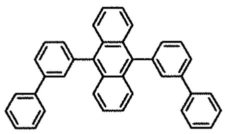 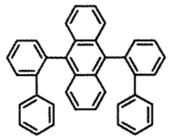 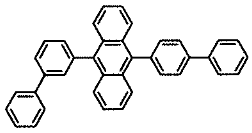 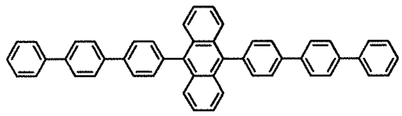

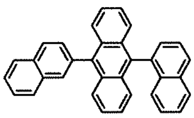 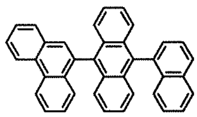 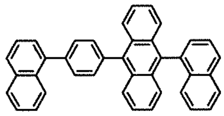 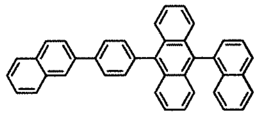

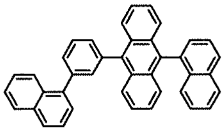 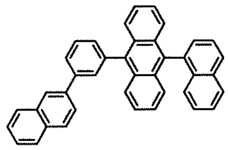 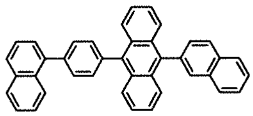 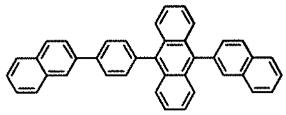

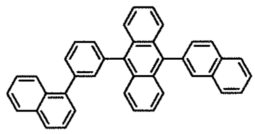 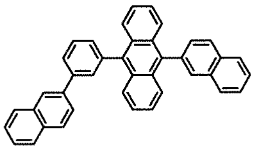 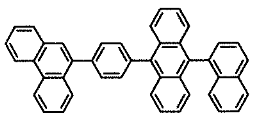 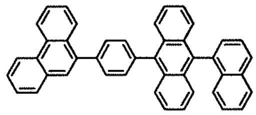

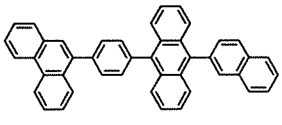 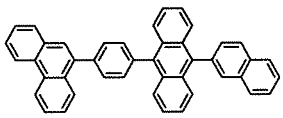 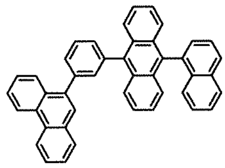 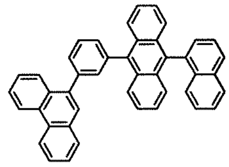

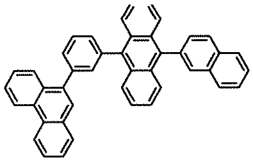 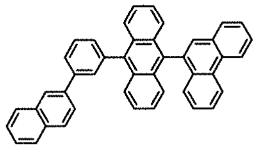 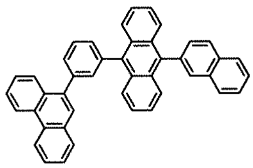 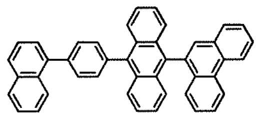

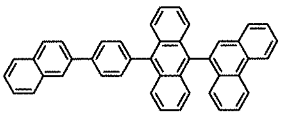 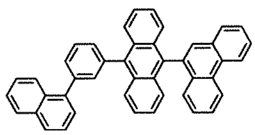 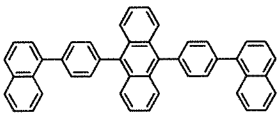 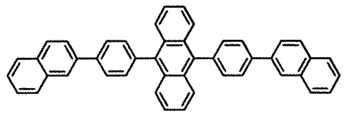

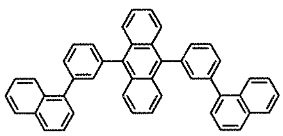 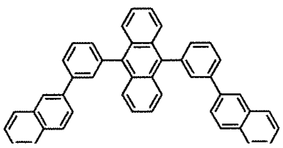 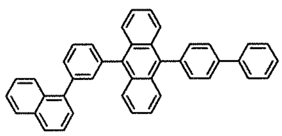 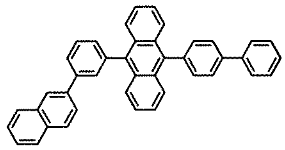

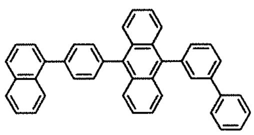 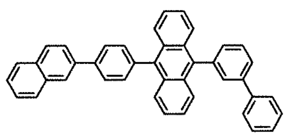 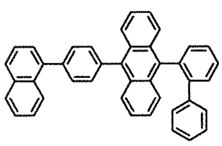 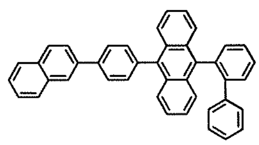

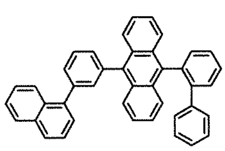 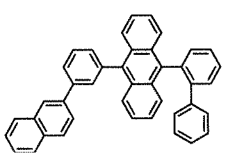 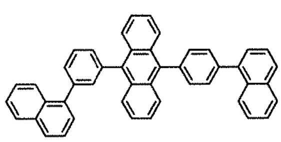 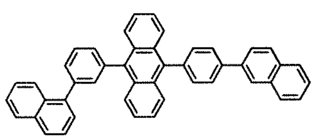

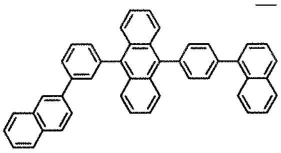
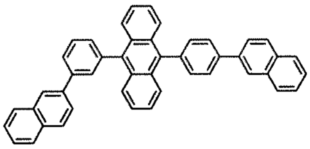
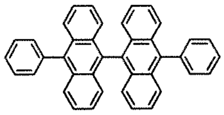
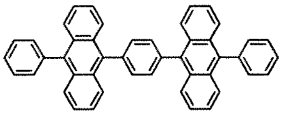
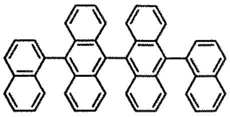
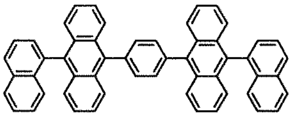
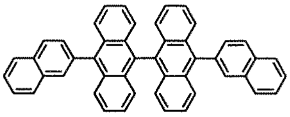
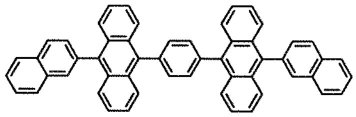
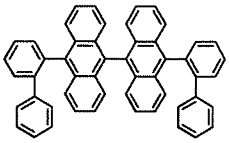
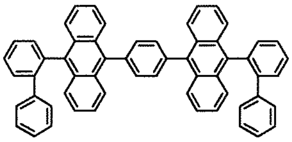
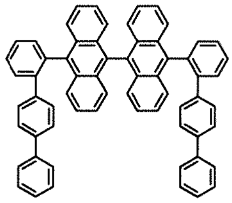
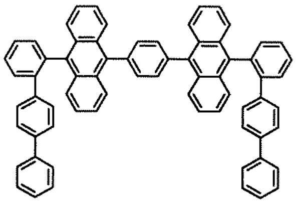
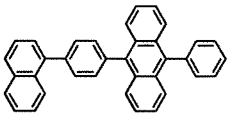
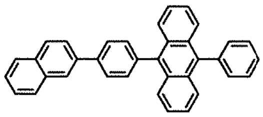
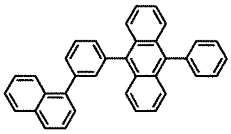
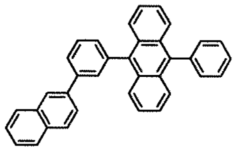
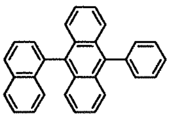
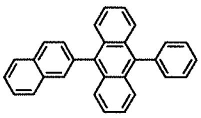
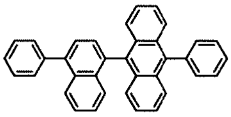
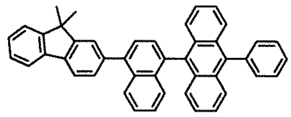
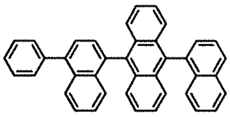
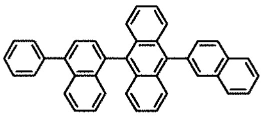
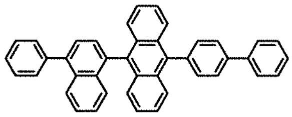
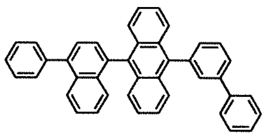
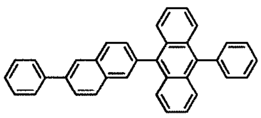
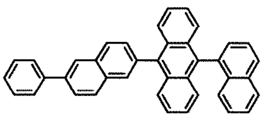
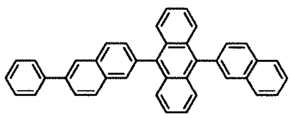
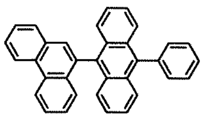
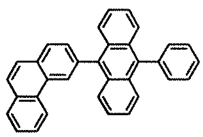
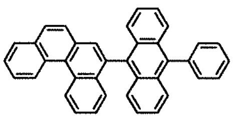
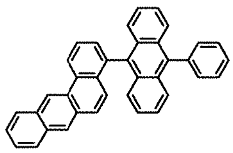
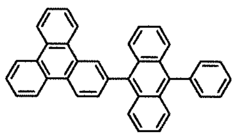
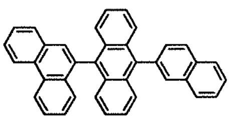
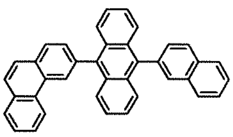
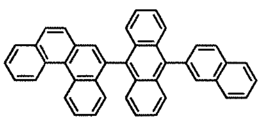
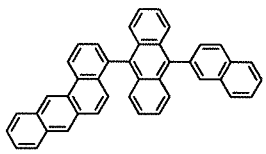

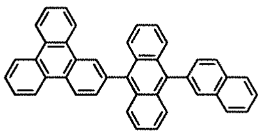
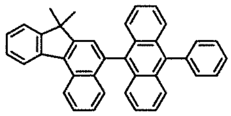
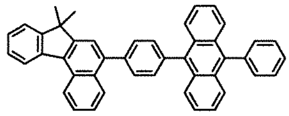
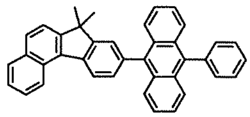

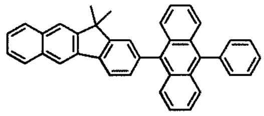
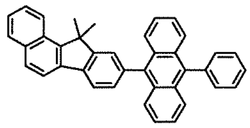
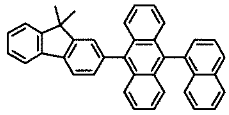
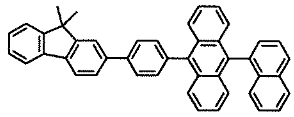

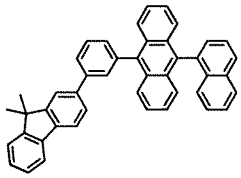
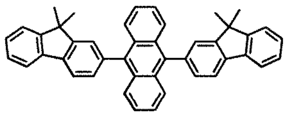
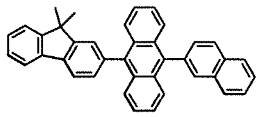
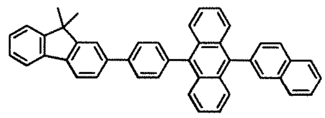

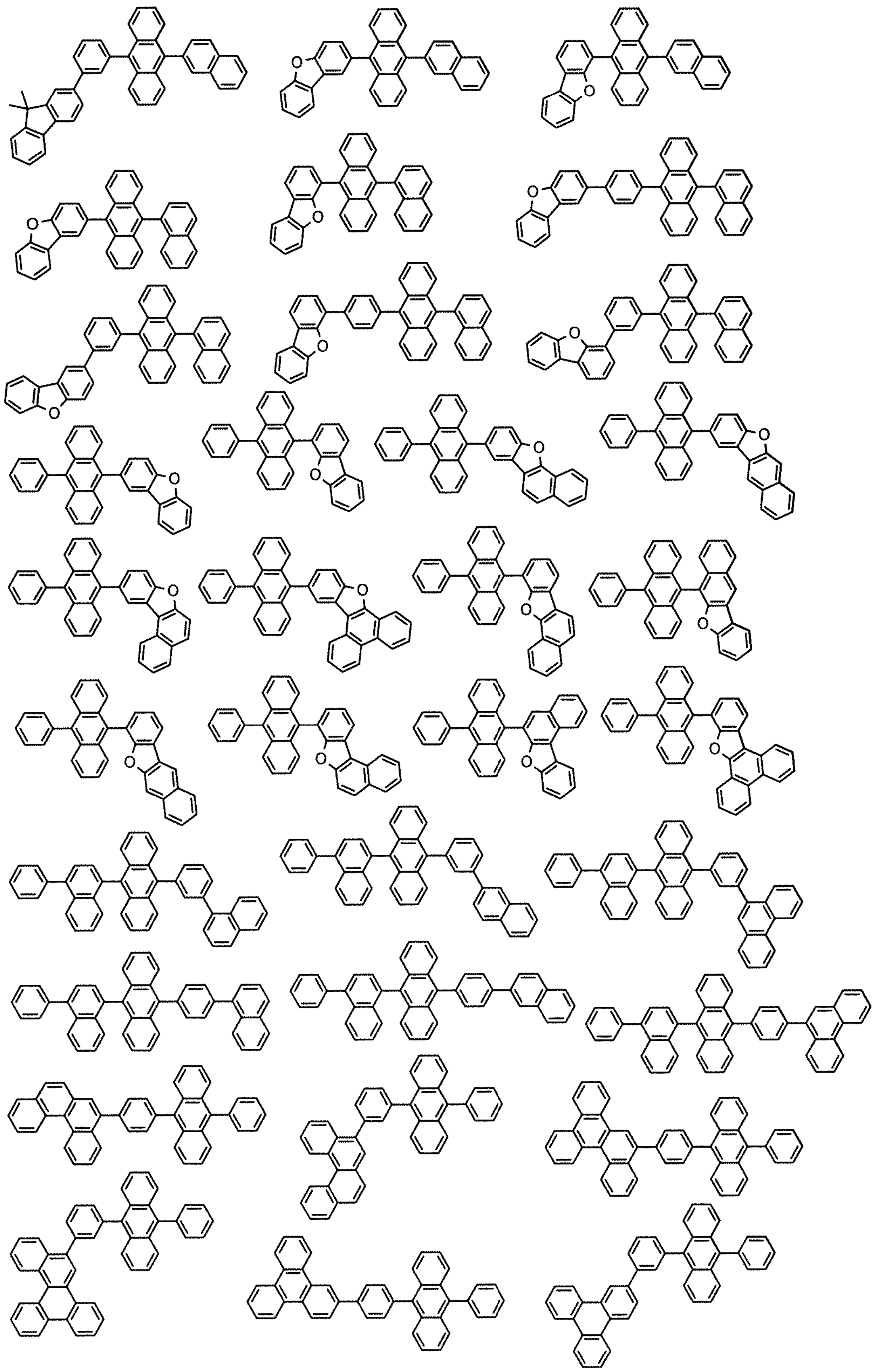

Electron Transporting Layer

The electron transporting layer is a layer containing a material having a high electron transporting capability (an electron transporting material) and is provided between the light emitting layer and the cathode, or between the electron injecting layer, if exists, and the light emitting layer.

The electron transporting layer may have a single layer structure or a multilayer structure including two or more layers. For example, the electron transporting layer may have a two-layer structure including a first electron transporting layer (anode side) and a second electron transporting layer (cathode side). In one embodiment of the present invention, the electron transporting layer having a single layer structure is preferably disposed adjacent to the light emitting layer, and the electron transporting layer that is closest to the anode in the multilayer structure, such as the first electron transporting layer in the two-layer structure, is preferably disposed adjacent to the light emitting layer. In another embodiment of the present invention, and a hole blocking layer described later may be disposed between the electron transporting layer having a single layer structure and the light emitting layer, or between the electron transporting layer that is closest to the light emitting layer in the multilayer structure and the light emitting layer.
As the electron transporting layer, for example,

(1) a metal complex, such as an aluminum complex, a beryllium complex, and a zinc complex;
(2) a heteroaromatic compound, such as an imidazole derivative, a benzimidazole derivative, an azine derivative, a carbazole derivative, and a phenanthroline derivative; and
(3) a high-molecular weight compound can be used.

Examples of the metal complex include tris(8-quinolinolato)aluminum(III) (abbreviation: Alq), tris(4-methyl-8-quinolinolato)aluminum (abbreviation: Almq3), bis(10-hydroxybenzo[h]quinolinato)beryllium (abbreviation: $BeBq_2$), bis(2-methyl-8-quinolinolato)(4-phenylphenolato)aluminum(III) (abbreviation: BAlq), bis(8-quinolinolato)zinc(II) (abbreviation: Znq), bis[2-(2-benzoxazolyl)phenolato]zinc(II) (abbreviation: ZnPBO), and bis[2-(2-benzothiazolyl)phenolato]zinc(II) (abbreviation: ZnBTZ).
Examples of the heteroaromatic compound include 2-(4-biphenylyl)-5-(4-tert-butylphenyl)-1,3,4-oxadiazole (abbreviation: PBD), 1,3-bis[5-(p-tert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazol-2-yl)stilbene (abbreviation: BzOs) .
Examples of the high-molecular weight compound include poly[(9,9-dihexylfluorene-2,7-diyl)-co-(pyridine-3,5-diyl)] (abbreviation: PF-Py), and poly[(9,9-dioctylfluorene-2,7-diyl)-co-(2,2'-bipyridine-6,6'-diyl)] (abbreviation: PF-BPy).
The materials are materials having an electron mobility of $10^{-6}$ $cm^2/Vs$ or more. Materials other than those as mentioned above may also be used in the electron transporting layer so long as they are materials high in the electron transporting capability rather than in the hole transporting capability.

Electron Injecting Layer

The electron injecting layer is a layer containing a material having a high electron injection capability. As the electron injecting layer, alkali metals, such as lithium (Li) and cesium (Cs), alkaline earth metals, such as magnesium (Mg), calcium (Ca), and strontium (Sr), rare earth metals, such as europium (Eu) and ytterbium (Yb), and compounds containing these metals can be used. Examples of the compounds include an alkali metal oxide, an alkali metal halide, an alkali metal-containing organic complex, an alkaline earth metal oxide, an alkaline earth metal halide, an alkaline earth metal-containing organic complex, a rare earth metal oxide, a rare earth metal halide, and a rare earth metal-containing organic complex. These compounds may be used as a mixture of a plurality thereof.
In addition, a material having an electron transporting capability, in which an alkali metal, an alkaline earth metal, or a compound thereof is contained, specifically Alq in which magnesium (Mg) is contained may be used. In this case, electron injection from the cathode can be more efficiently performed.
Otherwise, in the electron injecting layer, a composite material obtained by mixing an organic compound with an electron donor may be used. Such a composite material is excellent in the electron injection capability and the electron transporting capability because the organic compound receives electrons from the electron donor. In this case, the organic compound is preferably a material excellent in transporting received electrons, and specifically, examples thereof include a material constituting the aforementioned electron transporting layer (such as a metal complex and a heteroaromatic compound). As the electron donor, a material having an electron donation property for the organic compound may be used. Specifically, alkali metals, alkaline earth metals, and rare earth metals are preferred, and examples thereof include lithium, cesium, magnesium, calcium, erbium, and ytterbium. In addition, an alkali metal oxide or an alkaline earth metal oxide is preferred, and examples thereof include lithium oxide, calcium oxide, and barium oxide. In addition, a Lewis base, such as magnesium oxide, can also be used. In addition, an organic compound, such as tetrathiafulvalene (abbreviation: TTF), can also be used.

Cathode

It is preferred that a metal, an alloy, an electrically conductive compound, or a mixture thereof which has a low work function (specifically 3.8 eV or less) is used for the cathode. Specific examples of such a cathode material include elements belonging to group 1 or 2 of the periodic table of the elements, that is, alkali metals, such as lithium (Li) and cesium (Cs), alkaline earth metals, such as magnesium (Mg), calcium (Ca), and strontium (Sr), and alloys containing these (such as MgAg, and AlLi), and rare earth metals, such as europium (Eu), and ytterbium (Yb) and alloys containing these.

When the cathode is formed by using the alkali metals, the alkaline earth metals, and the alloys containing these, a vacuum vapor deposition method or a sputtering method can be adopted. In addition, when a silver paste or the like is used, a coating method, an inkjet method, or the like can be adopted.

By providing the electron injecting layer, the cathode can be formed using various conductive materials, such as Al, Ag, ITO, graphene, and indium oxide-tin oxide containing silicon or silicon oxide regardless of the magnitude of a work function. Such a conductive material can be deposited by using a sputtering method, an inkjet method, a spin coating method, or the like.

Insulating Layer

The organic EL device applies an electric field to an ultrathin film, and thus, pixel defects are likely to occur due to leaks or short-circuiting. In order to prevent this, an insulating layer formed of an insulating thin film layer may be inserted between a pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. A mixture or a laminate of these may also be used.

Space Layer

The space layer is, for example, a layer provided between a fluorescent light emitting layer and a phosphorescent light emitting layer for the purpose of preventing excitons generated in the phosphorescent light emitting layer from diffusing into the fluorescent light emitting layer, or adjusting a carrier balance, in the case where the fluorescent light emitting layers and the phosphorescent light emitting layers are stacked. The space layer can also be provided among the plurality of phosphorescent light emitting layers.

Since the space layer is provided between the light emitting layers, a material having both an electron transporting capability and a hole transporting capability is preferred. Also, one having a triplet energy of 2.6 eV or more is preferred in order to prevent triplet energy diffusion in the adjacent phosphorescent light emitting layer. Examples of the material used for the space layer include the same as those used for the hole transporting layer as described above.

Blocking Layer

The blocking layer such as the electron blocking layer, the hole blocking layer, or the exciton blocking layer may be provided adjacent to the light emitting layer. The electron blocking layer is a layer that prevents electrons from leaking from the light emitting layer to the hole transporting layer, and the hole blocking layer is a layer that prevents holes from leaking from the light emitting layer to the electron transporting layer. The exciton blocking layer has a function of preventing excitons generated in the light emitting layer from diffusing into the surrounding layers, and trapping the excitons within the light emitting layer.

Each layer of the organic EL device may be formed by a conventionally known vapor deposition method, a coating method, or the like. For example, formation can be performed by a known method using a vapor deposition method such as a vacuum vapor deposition method, or a molecular beam vapor deposition method (MBE method), or a coating method using a solution of a compound for forming a layer, such as a dipping method, a spin-coating method, a casting method, a bar-coating method, and a roll-coating method.

The film thickness of each layer is not particularly limited, but is typically 5 nm to 10 μm, and more preferably 10 nm to 0.2 μm because in general, when the film thickness is too small, defects such as pinholes are likely to occur, and conversely, when the film thickness is too large, a high driving voltage is required and the efficiency decreases.

The organic EL device can be used for electronic devices, such as display components of an organic EL panel module, display devices of a television, a mobile phone and a personal computer, and light emitting devices of lightings and vehicular lamps.

Examples

The present invention is hereunder described in more detail by reference to Examples, but it should be construed that the present invention is not limited to the following Examples.

<Compounds>

Inventive compounds of the formula (1) used in production of organic EL devices of Examples 1 to 8 are shown below.

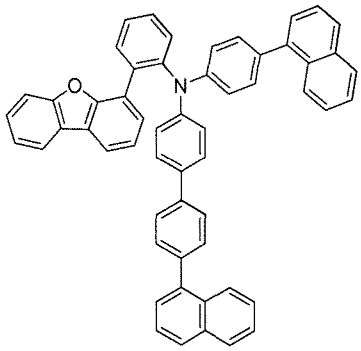

Compound 1

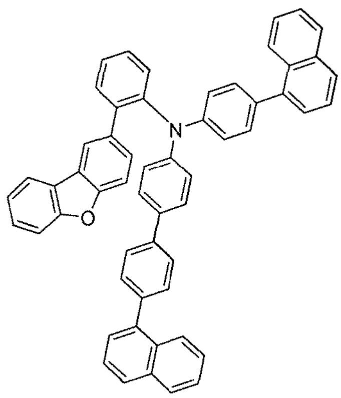

Compound 2

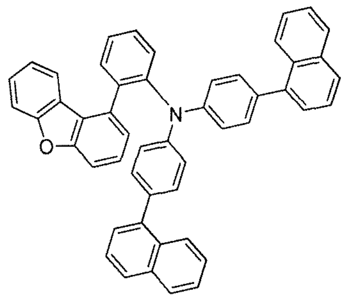

Compound 8

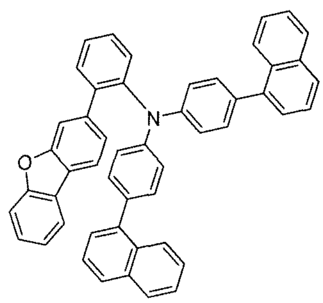

Compound 9

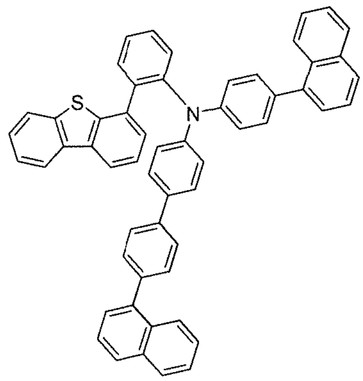

Compound 10

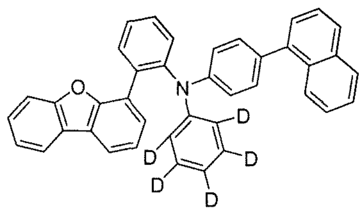

Compound 11

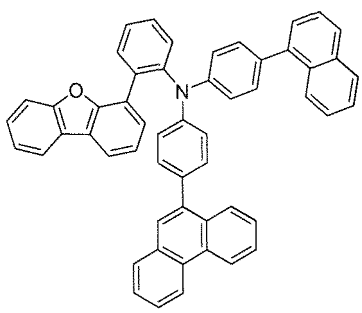

Compound 12

Structures of comparative compounds used in production of organic EL devices of Comparative Example 1 are shown below.

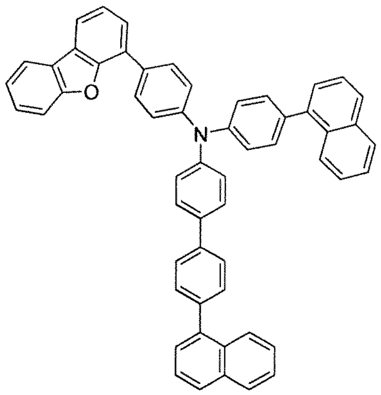

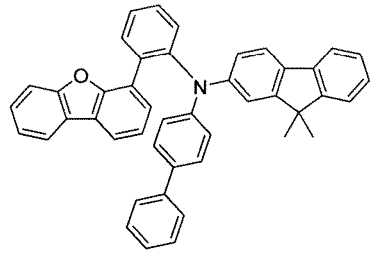

Comparative Compound 1 Comparative Compound 2

Structures of other compounds used in production of organic EL devices of Examples 1 to 2 and Comparative Example 1 are shown below.

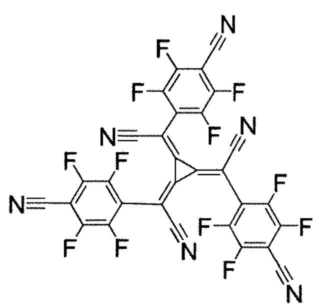

HA-1

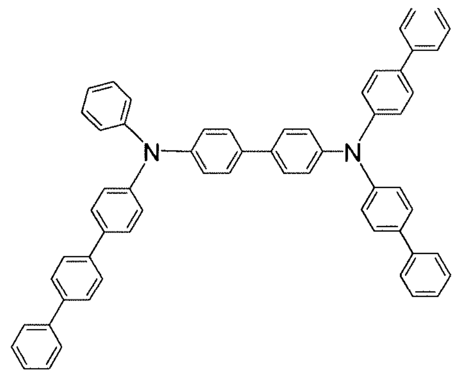

HT-1

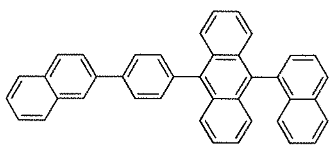

BH-1

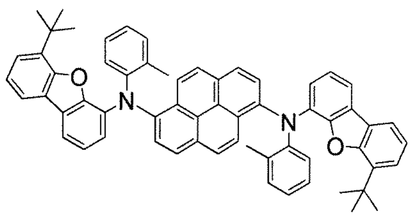

BD-1

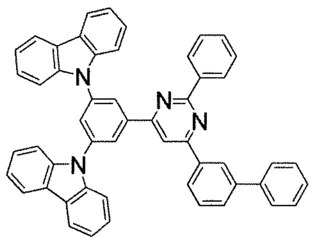

ET-1

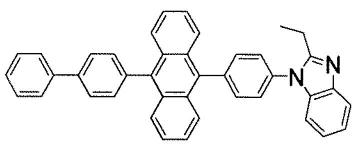

ET-2

Structures of other compounds used in production of organic EL devices of Examples 3 to 8 and Comparative Example 2 are shown below.

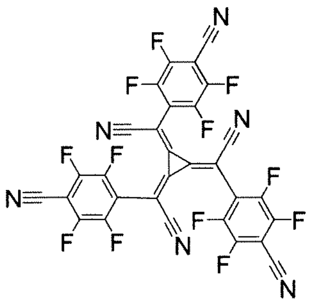

HA-1

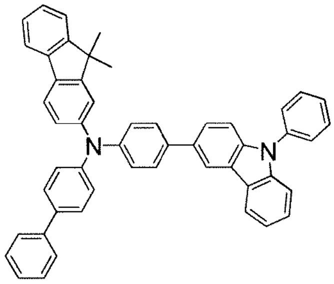

HT-12

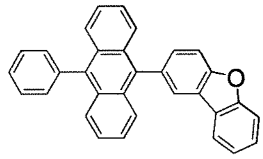

BH-2

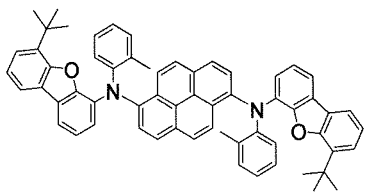

BD-1

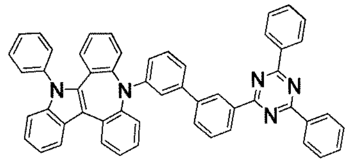

ET-3

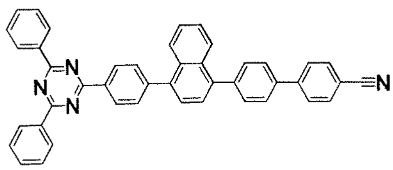

ET-4

Compounds 3 to 7 Synthesized in Synthesis Examples 3 to 7

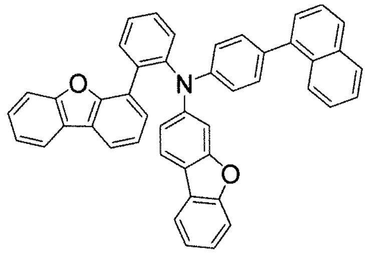

Compound 3

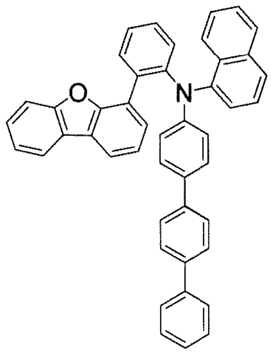

Compound 4

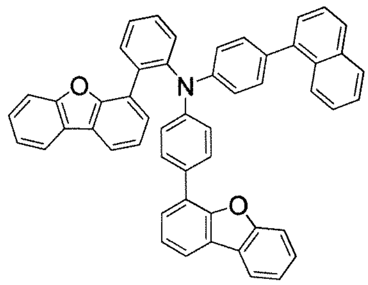

Compound 5

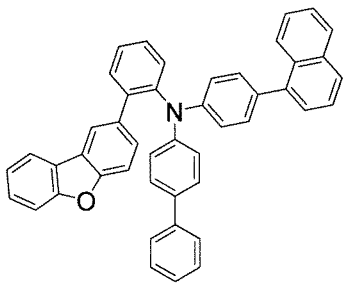

Compound 6

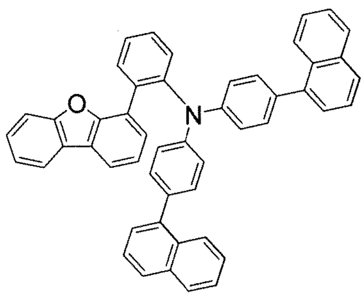

Compound 7

Organic EL devices were produced in the following manner, and the devices were evaluated for the EL device capability.

Production of Organic EL Device

Example 1

A glass substrate of 25 mm × 75 mm × 1.1 mm provided with an ITO transparent electrode (anode) (manufactured by GEOMATEC Co., Ltd.) was ultrasonically cleaned in isopropyl alcohol for 5 minutes and then subjected to UV ozone cleaning for 30 minutes. The film thickness of the ITO was 130 nm.

The cleaned glass substrate provided with the transparent electrode lines was mounted on a substrate holder of a vacuum vapor deposition apparatus, and firstly, Compound HT-1 and Compound HA-1 were vapor co-deposited on the surface having the transparent electrode lines formed thereon, so as to cover the transparent electrode, resulting in a hole injecting layer with a film thickness of 10 nm. The mass ratio of Compound HT-1 to Compound HA-1 (HT-1/HA-1) was 97/3.

Subsequently, on this hole injecting layer, Compound HT-1 was vapor deposited to form a first hole transporting layer with a film thickness of 80 nm.

Subsequently, on this first hole transporting layer, Compound HT-2 (Compound 1) was vapor deposited to form a second hole transporting layer with a film thickness of 10 nm.

Subsequently, on this second hole transporting layer, Compound BH-1 (host material) and Compound BD-1 (dopant material) were vapor co-deposited to form a light emitting layer with a film thickness of 25 nm. The mass ratio of Compound BH-1 to Compound BD-1 (BH-1/BD-1) was 96/4.

Subsequently, on this light emitting layer, Compound ET-1 and Compound ET-2 were vapor co-deposited to form an electron transporting layer with a film thickness of 20 nm. The mass ratio of Compound ET-1 to Compound ET-2 (ET-1/ET-2) was 50/50.

Subsequently, on this electron transporting layer, LiF was vapor deposited to form an electron injecting electrode (cathode) with a film thickness of 1 nm.

Then, on this electron injecting electrode, metal Al was vapor deposited to form a metal cathode with a film thickness of 50 nm.

The layer configuration (device configuration A) of the organic EL device of Example 1 thus obtained was as follows.

ITO (130)/HT-1/HA-1 = 97/3 (10)/HT-1 (80)/HT-2 (10)/BH-1/BD-1 = 96/4 (25)/ET-1/ET-2 = 50/50 (20)/LiF (1)/Al (50)

In the layer configuration, the numeral in parentheses indicates the film thickness (nm), and the ratio of HT-1 to HA-1, BH-1 to BD-1, and ET-1 to ET-2 is by mass.

Example 2

An organic EL device of Example 2 was produced in the same manner as in Example 1 except that in Example 1, Compound 2 was used in place of Compound 1 used in the second hole transporting layer.

Comparative Example 1

An organic EL device of Comparative Example 1 was produced in the same manner as in Example 1 except that in Example 1, Comparative Compound 1 was used in place of Compound 1 used in the second hole transporting layer.

Example 3

An organic EL device of Example 3 was produced according to the same method as in Example 1 except that the layer configuration (device configuration A) of the organic EL device was changed to a layer configuration (device configuration B) mentioned below and Compound HT-2 (Compound 10) was used in the second hole transporting layer.

ITO (130)/HT-12/HA-1 = 97:3 (10)/HT-12 (80)/HT-2 (10)/BH-2/BD-1 = 96/4 (25)/ET-3 (5)/ET-4/Liq = 50/50 (20)/LiF (1)/Al (50)

In the above layer configuration, the numeral in parentheses indicates the film thickness (nm), and the ratio of HT-12 to HA-1, BH-2 to BD-1, and ET-4 to Liq is by mass.

Example 4

An organic EL device of Example 4 was produced in the same manner as in Example 3 except that in Example 3, Compound 1 was used in place of Compound 10 used in the second hole transporting layer.

Example 5

An organic EL device of Example 5 was produced in the same manner as in Example 3 except that in Example 3, Compound 8 was used in place of Compound 10 used in the second hole transporting layer.

Example 6

An organic EL device of Example 6 was produced in the same manner as in Example 3 except that in Example 3, Compound 9 was used in place of Compound 10 used in the second hole transporting layer.

Example 7

An organic EL device of Example 7 was produced in the same manner as in Example 3 except that in Example 3, Compound 11 was used in place of Compound 10 used in the second hole transporting layer.

Example 8

An organic EL device of Example 8 was produced in the same manner as in Example 3 except that in Example 3, Compound 12 was used in place of Compound 10 used in the second hole transporting layer.

Comparative Example 2

An organic EL device of Comparative Example 2 was produced in the same manner as in Example 3 except that in Example 3, Comparative Compound 2 was used in place of Compound 10 used in the second hole transporting layer.

Evaluation of Organic EL Devices

The external quantum efficiency of the produced organic EL devices was evaluated. The evaluation results are shown in Table 1.

Measurement of External Quantum Efficiency (EQE)

The resulting organic EL device was driven at room temperature with DC direct current at a current density of 10 $mA/cm^2$. Using a luminance meter (spectral radiance meter CS-1000 by Konica Minolta, Inc.), the luminance of the device was measured, and from the found data, the external quantum efficiency (%) was derived.

Table 1

| | Compound HT-2 | EQE(%) @10$mA/cm^2$ | Device Configuration |
|---|---|---|---|
| Example 1 | Compound 1 | 10.1 | A |
| Example 2 | Compound 2 | 10.2 | A |
| Comparative Example 1 | Comparative Compound 1 | 9.5 | A |
| Example 3 | Compound 10 | 9.82 | B |
| Example 4 | Compound 1 | 9.34 | B |
| Example 5 | Compound 8 | 9.41 | B |
| Example 6 | Compound 9 | 8.96 | B |

(continued)

| | Compound HT-2 | EQE(%) @10mA/cm$^2$ | Device Configuration |
|---|---|---|---|
| Example 7 | Compound 11 | 9.55 | B |
| Example 8 | Compound 12 | 9.62 | B |
| Comparative Example 2 | Comparative Compound 2 | 8.54 | B |

As obvious from the results in Table 1, the compounds of the present invention having a configuration where a dibenzofuran or dibenzothiophene skeleton bonds to the central nitrogen atom via an orthophenylene linking group, a naphthalene skeleton bonds thereto via a single bond or via a phenylene group or a biphenyldiyl group and an aryl group or a heterocyclic group bonds thereto via a single bond or via a specific linking group exhibit an improved external quantum efficiency as compared with Comparative Compound 1 and Comparative Compound 2 not satisfying the configurational requirement in the present invention.

<Synthesis of Compounds>

Synthesis of Intermediate 1

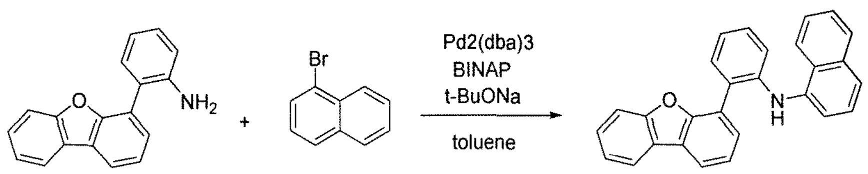


Intermediate 1

In an argon atmosphere, 3.67 g (17.74 mmol) of 1-bromonaphthalene, 4.6 g (17.74 mmol) of 2-(dibenzo[b,d]furan-4-yl)aniline, 325 mg (0.355 mmol) of tris(dibenzylideneacetone)dipalladium(0), 442 mg (0.71 mmol) of BINAP, 2.39 g (24.84 mmol) of sodium-t-butoxide and 89 mL of toluene were mixed and stirred under heat at 80°C for 3 hours. After left cooled, this was purified through column chromatography to give 8.1 g of Intermediate 1. The yield was 99%.

Synthesis of Intermediate 2

Intermediate 2 was synthesized according to the same synthesis method as that for Intermediate 1 but using 1-(4-bromophenyl)naphthalene in place of 1-bromonaphthalene.

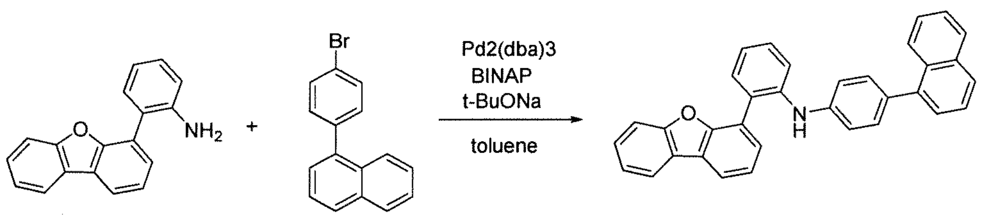


Intermediate 2

Synthesis of Intermediate 3

Intermediate 3 was synthesized according to the same synthesis method as that for Intermediate 1 but using 2-(dibenzo[b,d]furan-2-yl)aniline in place of 2-(dibenzo[b,d]furan-4-yl)aniline and using 1-(4-bromophenyl)naphthalene in place of 1-bromonaphthalene.

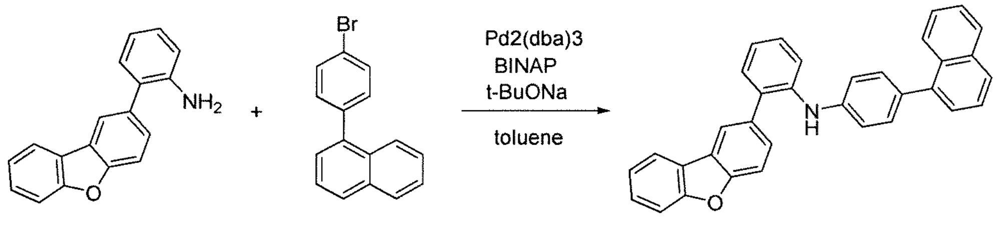


Intermediate 3

Synthesis Example 1: Synthesis of Compound 1

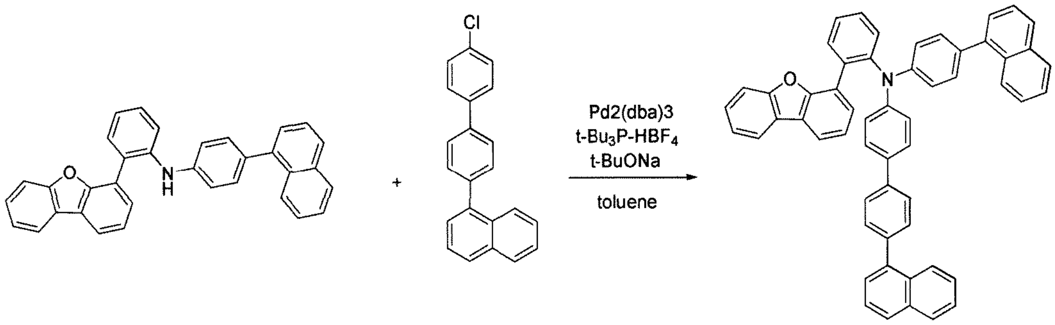


Intermediate 2 Compound 1

In an argon atmosphere, 2.68 g (17.33 mmol) of Intermediate 2, 19.07 mmol of 4-chloro-4'-(naphthalen-1-yl)biphenyl, 476 mg (0.52 mmol) of tris(dibenzylideneacetone)dipalladium(0), 302 mg (1.04 mmol) of tri-t-butylphosphonium tetrafluoroborate, 2.5 g (26 mmol) of sodium-t-butoxide and 87 mL of toluene were mixed and stirred under heat at 105°C for 3 hours. After cooled, the resulting residue was purified through column chromatography to give Compound 1 (5.3 g).

The resulting product was Compound 1 as a result of mass spectrometry (m/e = 739 relative to molecular weight 739.29). The yield was 62%.

Synthesis Example 2: Synthesis of Compound 2

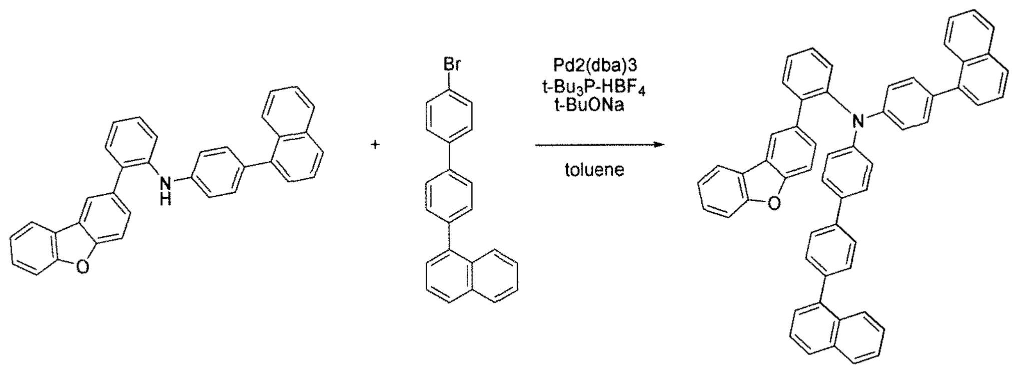


Intermediate 3 Compound 2

Compound 2 was produced in the same manner as that for synthesis of Compound 1 except that Intermediate 3 was used in place of Intermediate 2 and 4-bromo-4'-(naphthalen-1-yl)biphenyl was used in place of 4-chloro-4'-(naphthalen-1-yl)biphenyl.

The resulting product was Compound 2 as a result of mass spectrometry (m/e = 739 relative to molecular weight 739.29). The yield was 87%.

Synthesis Example 3: Synthesis of Compound 3

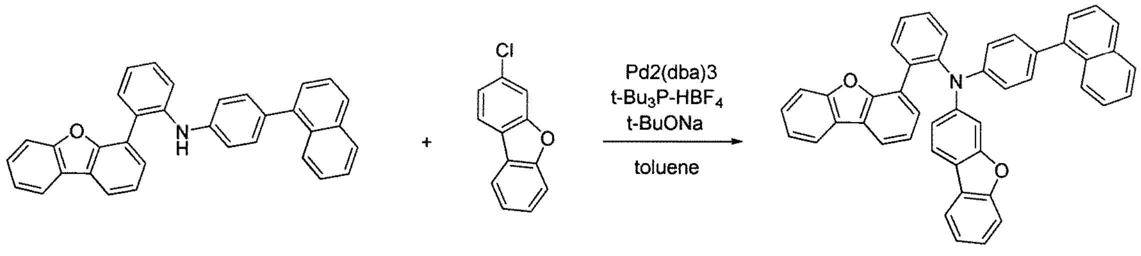


Intermediate 2 Compound 3

Compound 3 was produced in the same manner as that for synthesis of Compound 1 except that 3-chlorodibenzo[b,d]furan was used in place of 4-chloro-4'-(naphthalen-1-yl)biphenyl.

The resulting product was Compound 3 as a result of mass spectrometry (m/e = 627 relative to molecular weight 627.22). The yield was 69%.

Synthesis Example 4: Synthesis of Compound 4

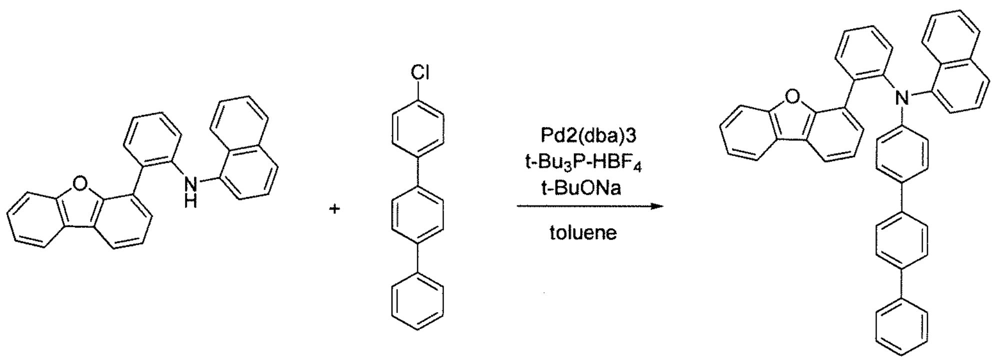


Intermediate 1 Compound 4

The same process as that for synthesis of Compound 1 was carried out, using Intermediate 1 in place of Intermediate 2 and using 4-chloro-1,1':4',1"-terphenyl in place of 4-chloro-4'-(naphthalen-1-yl)biphenyl.

The resulting product was Compound 4 as a result of mass spectrometry (m/e = 613 relative to molecular weight 613.24). The yield was 41%.

Synthesis Example 5: Synthesis of Compound 5

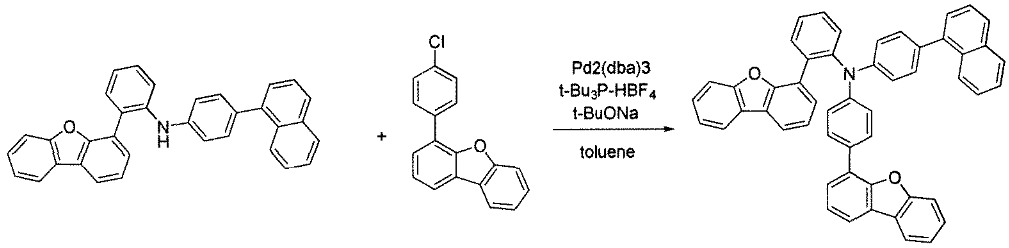


Intermediate 2 Compound 5

The same process as that for synthesis of Compound 3 was carried out to produce a compound, using 4-(4-chlorophenyl)dibenzo[b,d]furan in place of 3-chlorodibenzo[b,d]furan.

The resulting product was Compound 5 as a result of mass spectrometry (m/e = 703 relative to molecular weight 703.25). The yield was 78%.

Synthesis Example 6: Synthesis of Compound 6

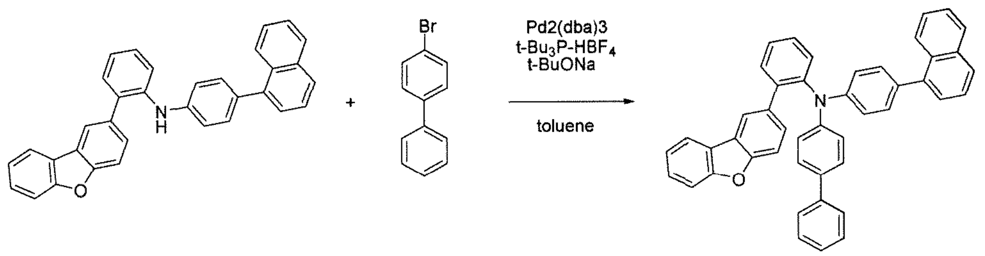


Intermediate 3 Compound 6

The same process as that for synthesis of Compound 4 was carried out to produce a compound, using Intermediate 3 in place of Intermediate 1 and using 4-bromobiphenyl in place of 4-chloro-1,1':4',1"-terphenyl.

The resulting product was Compound 6 as a result of mass spectrometry (m/e = 613 relative to molecular weight 613.24). The yield was 83%.

Synthesis Example 7: Synthesis of Compound 7

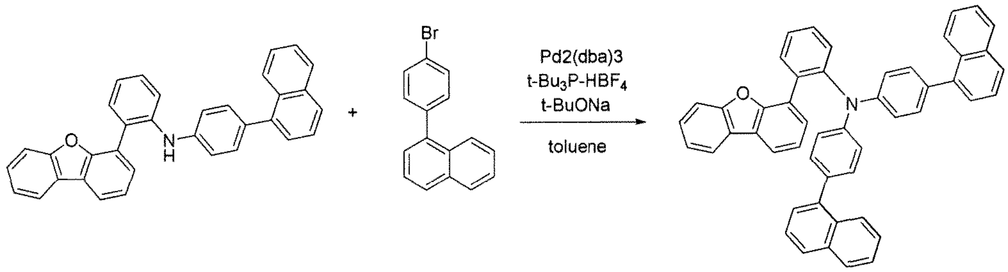


Intermediate 2 Compound 7

The same process as that for synthesis of Compound 1 was carried out to produce a compound, using 1-(4-bromophenyl)naphthalene in place of 4-chloro-4'-(naphthalen-1-yl)biphenyl.

The resulting product was Compound 7 as a result of mass spectrometry (m/e = 663 relative to molecular weight 663.26). The yield was 60%.

Synthesis Example 8: Synthesis of Compound 8

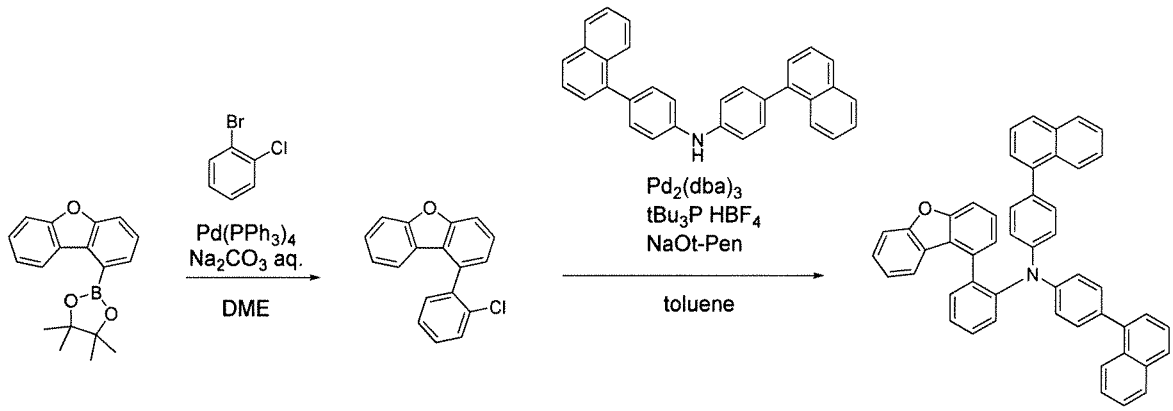


**Compound 8**

In an argon atmosphere, 8.82 g (30.00 mmol) of 2-(dibenzo[b,d]furan-1-yl)-4,4,5,5-tetramethyl-1,3,2-dioxaborane, 5.74 g (30.00 mmol) of 1-bromo-2-chlorobenzene, 693 mg (0.6 mmol) of tetrakis(triphenylphosphine)palladium(0), 30 mL (2M) of an aqueous solution of sodium carbonate, and 100 mL of 1,2-dimethoxyethane were mixed and stirred under heat at 80°C for 15 hours. After left cooled, this was subjected to liquid-liquid separation using ethyl acetate and water, and the resulting organic layer was concentrated and then purified through column chromatography to give 8.69 g of 1-(2-chlorophenyl)dibenzo[b,d]furan. The yield was 92%.

In an argon atmosphere, 2.93 g (10.51 mmol) of 1-(2-chlorophenyl)dibenzo[b,d]furan, 138 mg (0.151 mmol) of tris(dibenzylideneacetone)dipalladium(0), 180 mg (0.620 mmol) of tri-t-butylphosphonium tetrafluoroborate, 4.22 g (10.01 mmol) of 4-(naphthalen-1-yl)-N-[4-naphthalen-1-yl)phenyl]aniline, 33 ml of toluene, and 4.2 mL of a toluene solution of 40% sodium-t-pentoxide were mixed and stirred under heat at 130°C for 8 hours. Using a phase separator, this was filtered, and the residue was concentrated to give a solid. The solid was purified by recrystallization to give a compound (5.25 g). The resulting product was Compound 8 as a result of mass spectrometry (m/e = 663 relative to molecular weight 663.82). The yield was 79%.

Synthesis Example 9: Synthesis of Compound 9

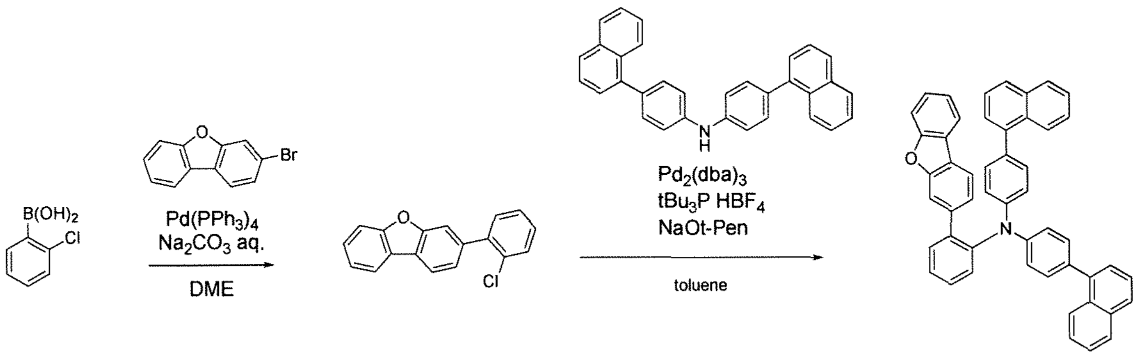


**Compound 9**

The same process as that for synthesis of Compound 8 was carried out to produce a compound, using 2-chlorophenylboronic acid in place of 2-(dibenzo[b,d]furan-1-yl)-4,4,5,5-tetramethyl-1,3,2-dioxaborane and using 3-bromodibenzofuran in place of 1-bromo-2-chlorobenzene. The resulting product was Compound 9 as a result of mass spectrometry (m/e = 663 relative to molecular weight 663.82). The yield was 73%.

Synthesis Example 10: Synthesis of Compound 10

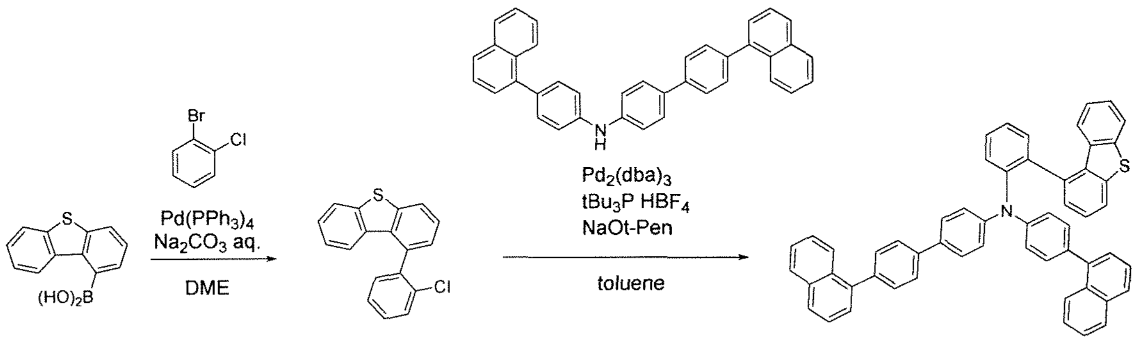


Compound 10

The same process as that for synthesis of Compound 8 was carried out to produce a compound, using dibenzo[b,d]thiophen-1-ylboronic acid in place of 2-(dibenzo[b,d]furan-1-yl)-4,4,5,5-tetramethyl-1,3,2-dioxaborane and using 4'-(naphthalen-1-yl)-N-[4-(naphthalen-1-yl)phenyl][1,1'-biphenyl]-4-amine in place of 4-(naphthalen-1-yl)-N-[4-(naphthalen-1-yl)phenyl]aniline. The resulting product was Compound 10 as a result of mass spectrometry (m/e = 755 relative to molecular weight 755.98). The yield was 54%.

Synthesis Example 11: Synthesis of Compound 11

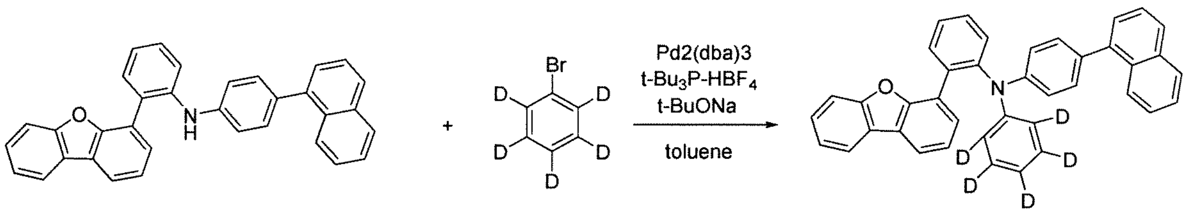


Intermediate 2

Compound 11

The same process as that for synthesis of Compound 2 was carried out to produce a compound, using bromobenzene-d5 in place of 1-(4-bromophenyl)naphthalene.

The resulting product was Compound 11 as a result of mass spectrometry (m/e = 542 relative to molecular weight 542.69). The yield was 88%.

Synthesis Example 12: Synthesis of Compound 12

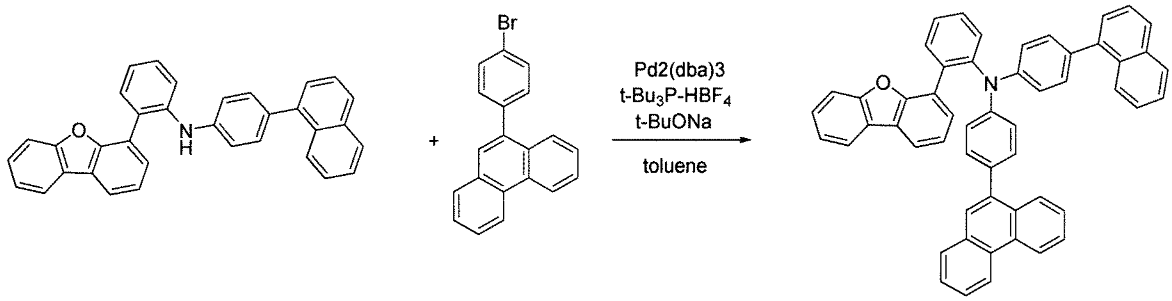


Intermediate 2

Compound 12

The same process as that for synthesis of Compound 2 was carried out to produce a compound, using 9-(4-bromophenyl)phenanthrene in place of 1-(4-bromophenyl)naphthalene.

The resulting product was Compound 12 as a result of mass spectrometry (m/e = 713 relative to molecular weight 713.88). The yield was 82%.

Reference Signs List

1, 11: Organic EL device
2: Substrate
3: Anode
4: Cathode
5: Light emitting layer
6: Hole transporting zone (e.g., hole injecting layer, hole transporting layer)
6a: Hole injecting layer
6b: First hole transporting layer
6c: Second hole transporting layer
7: Electron transporting zone (e.g., electron injecting layer, electron transporting layer)
10, 20: Light emitting unit

## Claims

**1.** A compound represented by the following formula (1):

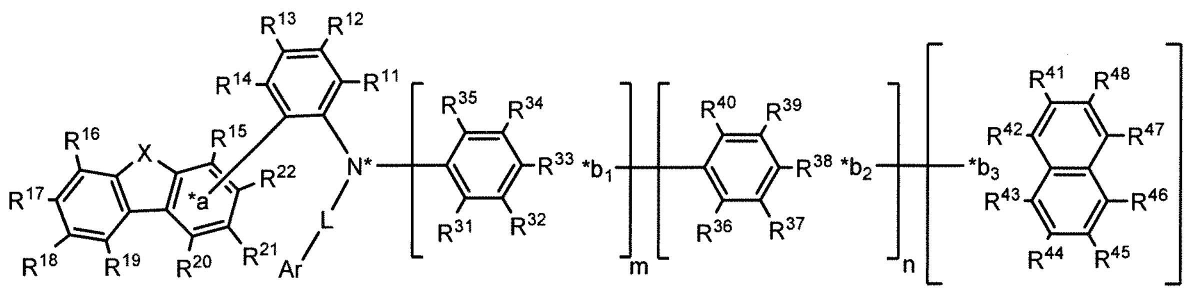

(1)

wherein

N* is a central nitrogen atom;
X represents an oxygen atom or a sulfur atom;
m represents 0 or 1, n represents 0 or 1:

Ar represents a substituted or unsubstituted aryl group having 6 to 50 ring carbon atoms, or a substituted or unsubstituted heterocyclic group having 5 to 50 ring atoms;
provided that when Ar has a substituent, the substituent is each independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group;
L is selected from a single bond, a substituted or unsubstituted arylene group having 6 to 50 ring carbon atoms, and a substituted or unsubstituted 2-valent heterocyclic group having 5 to 50 ring atoms;
$R^{11}$ to $R^{14}$ and $R^{41}$ to $R^{48}$ each are independently selected from a hydrogen atom, and a substituted or unsubstituted alkyl group having 1 to 50 carbon atoms,
provided that one selected from $R^{41}$ to $R^{48}$ is a single bond bonding to $*b_3$;
$R^{15}$ to $R^{16}$, and $R^{18}$ to $R^{22}$ each are independently selected from a hydrogen atom, a substituted or unsubstituted aryl group having 6 to 12 ring carbon atoms, and a substituted or unsubstituted alkyl group having 6 to 18 carbon atoms,
$R^{31}$ to $R^{40}$ each are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 ring carbon atoms, a group represented by $-Si(R_{901})(R_{902})(R_{903})$, a halogen atom, a substituted or unsubstituted haloalkyl group having 1 to 50 carbon atoms, a substituted or unsubstituted alkoxy group having 1 to 50 carbon atoms, a substituted or unsubstituted aryloxy group having 6 to 50 ring carbon atoms, a substituted or unsubstituted haloalkoxy group having 1 to 50 carbon atoms, a substituted or unsubstituted aralkyl group having 7 to 50 carbon atoms, a nitro group, and a cyano group,
$R^{17}$ is each independently selected from a hydrogen atom, an unsubstituted aryl group having 6 to 12 ring carbon atoms, and a substituted or unsubstituted alkyl group having 6 to 18 carbon atoms,
provided that at least one of $R^{16}$ to $R^{19}$ is a hydrogen atom,
one selected from $R^{15}$, and $R^{20}$ to $R^{22}$ is a single bond bonding to *a, one selected from $R^{31}$ to $R^{35}$ is a single bond bonding to $*b_1$, one selected from $R^{36}$ to $R^{40}$ is a single bond bonding to $*b_2$;
$R_{901}$, $R_{902}$, and $R_{903}$ each are independently selected from a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, and a substituted or unsubstituted cycloalkyl group having 3 to 50 ring carbon atoms; and
adjacent two selected from $R^{11}$ to $R^{14}$, $R^{15}$ to $R^{22}$, $R^{31}$ to $R^{40}$ and $R^{41}$ to $R^{48}$ do not bond to each other and do not form a ring.

2. The compound according to claim 1, represented by any of the following formulae (2) to (5):

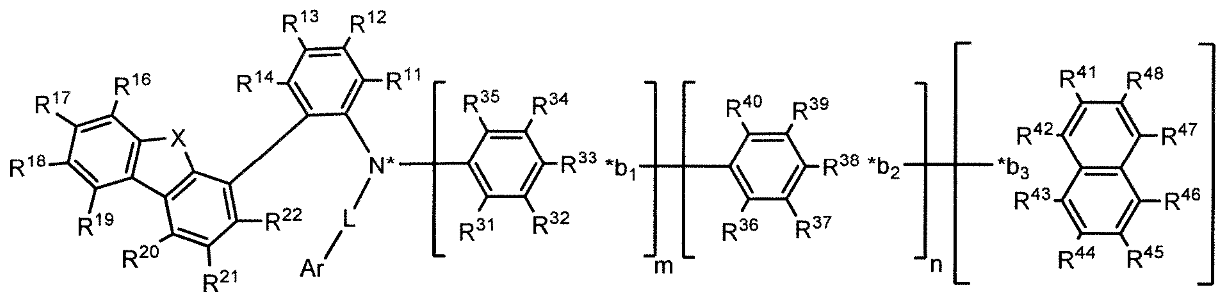

(2)

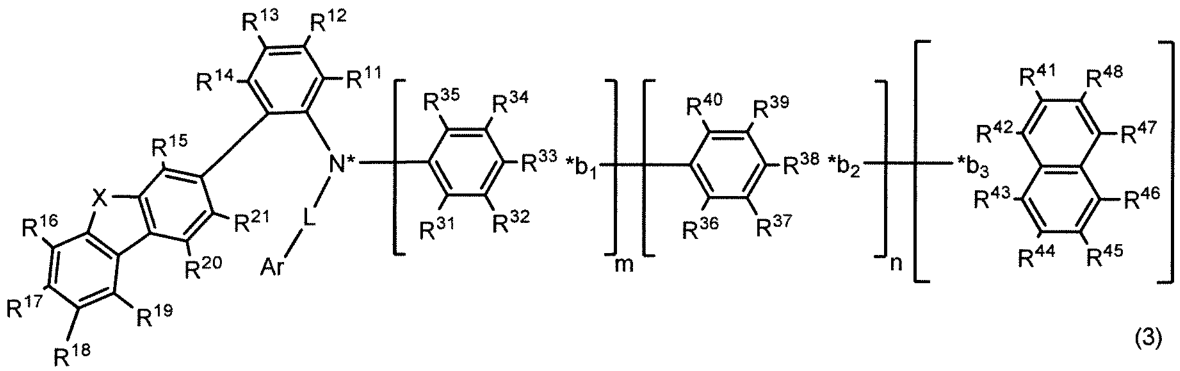

(3)

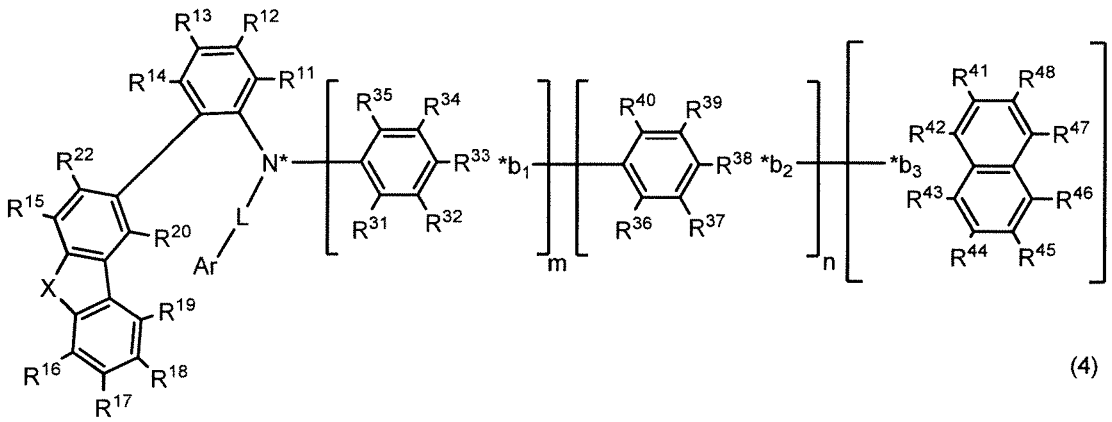

(4)

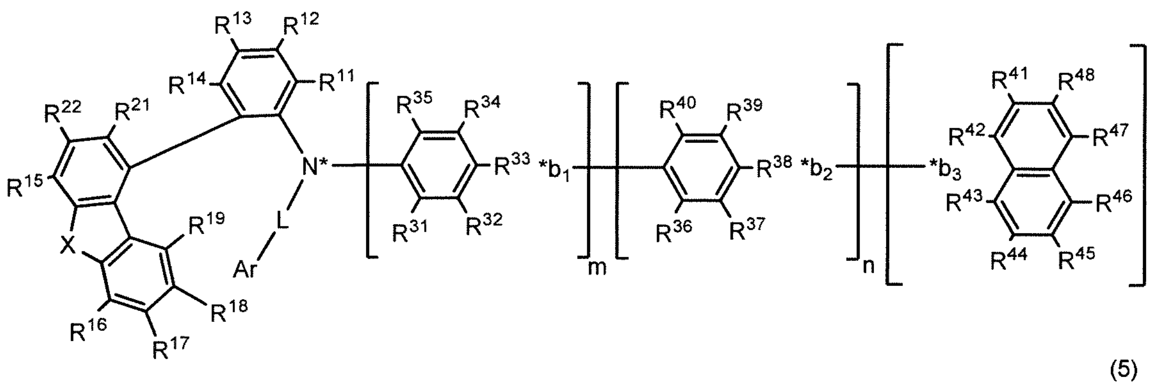

(5)

wherein N*, Ar, L, X, $*b_1$, $*b_2$, $*b_3$, m, n, $R^{11}$ to $R^{14}$, $R^{15}$ to $R^{22}$, $R^{31}$ to $R^{40}$, and $R^{41}$ to $R^{48}$ are as defined in the formula (1).

**3.** The compound according to claim 1 or 2, represented by any of the following formulae (6) to (9):

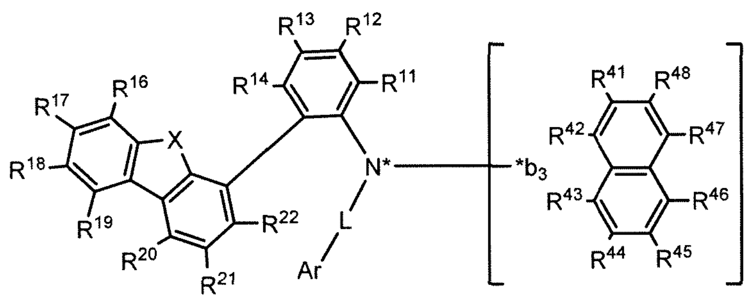

(6)

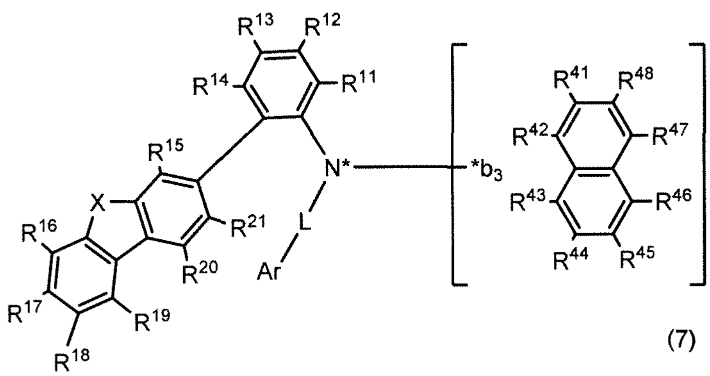

(7)

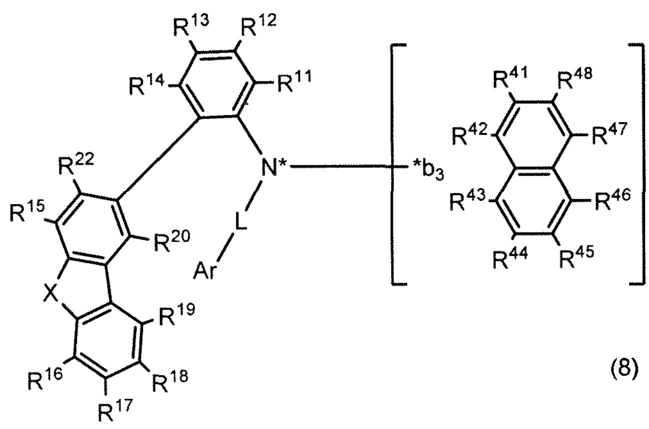

(8)

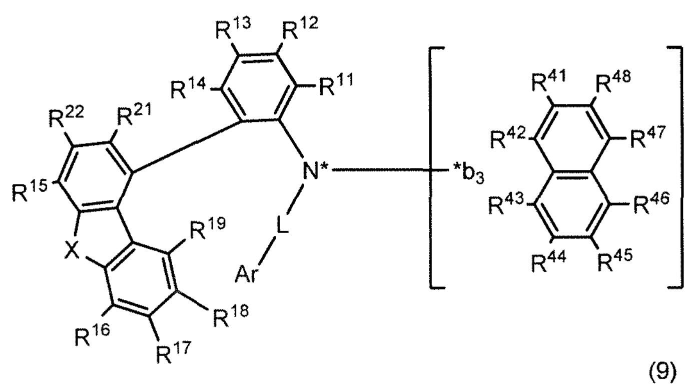

(9)

wherein N*, Ar, L, X, *$b_3$, $R^{11}$ to $R^{14}$, $R^{15}$ to $R^{22}$, and $R^{41}$ to $R^{48}$ are as defined in the formula (1).

**4.** The compound according to claim 1 or 2, represented by any of the following formulae (10) to (13).:

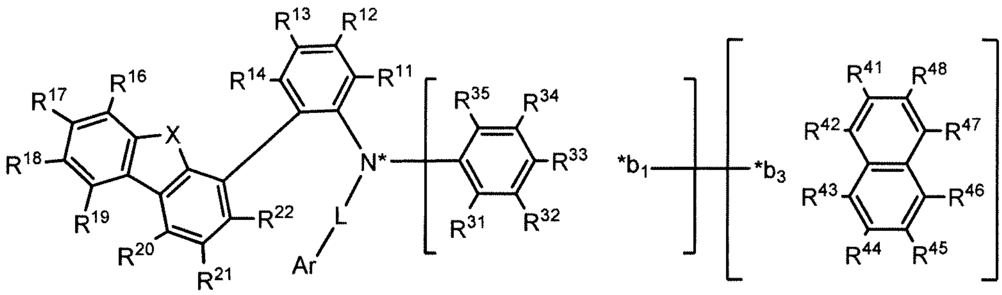

(10)

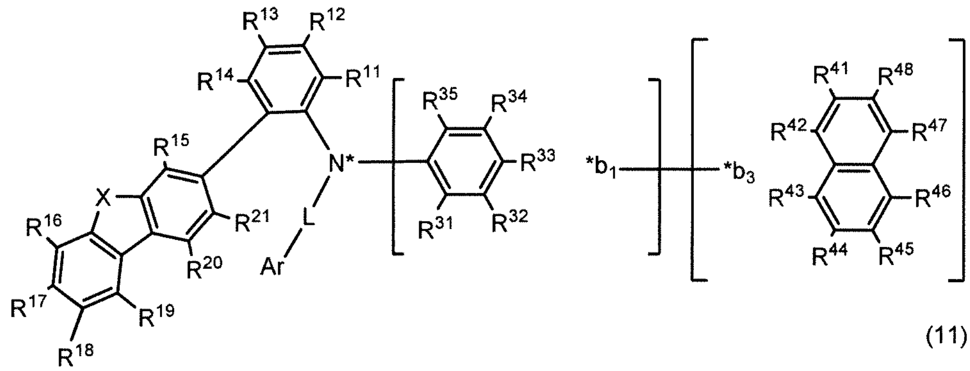

(11)

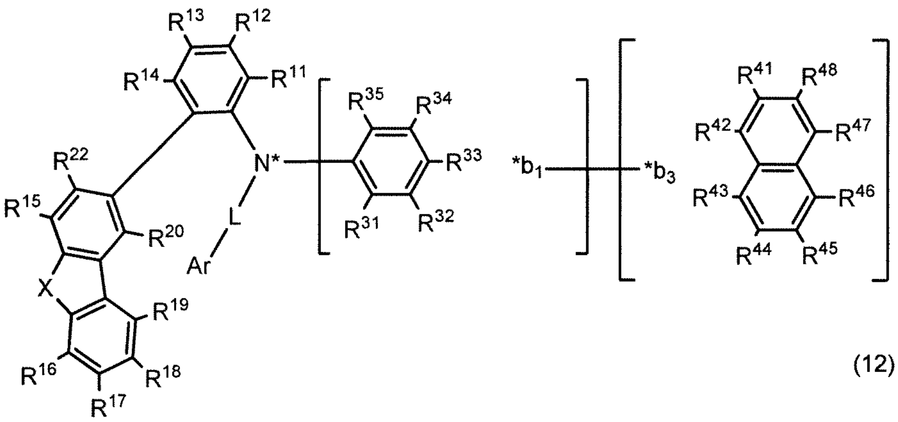

(12)

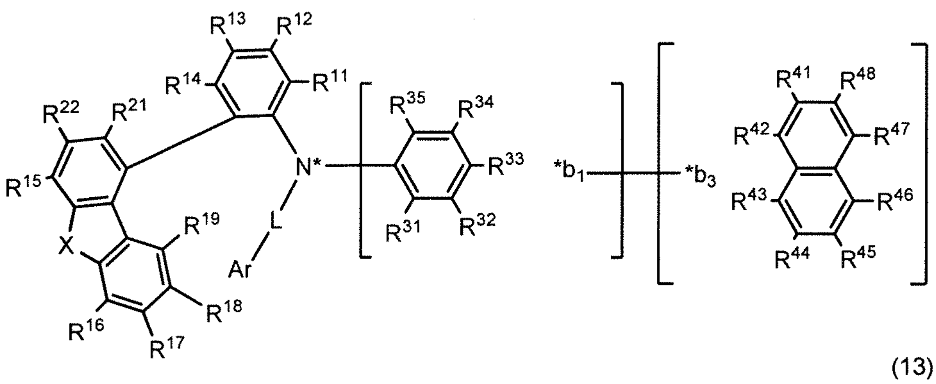

(13)

wherein N*, Ar, L, X, *$b_1$, *$b_3$, $R^{11}$ to $R^{14}$, $R^{15}$ to $R^{22}$, $R^{31}$ to $R^{35}$, and $R^{41}$ to $R^{48}$ are as defined in the formula (1).

**5.** The compound according to any one of claims 1 to 4, wherein the substituted or unsubstituted aryl group having 6

to 50 ring carbon atoms that Ar represents is selected from a phenyl group, a p-biphenyl group, an m-biphenyl group, an o-biphenyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group and a 9,9-diphenylfluorenyl group.

**6.** The compound according to any one of claims 1 to 4, wherein the substituted or unsubstituted heterocyclic group having 5 to 50 ring atoms that Ar represents is selected from a carbazolyl group, a benzocarbazolyl group, a dibenzofuranyl group, a naphthobenzofuranyl group, a dibenzothiophenyl group, a naphthobenzothiophenyl group, a (9-phenyl)carbazolyl group, a (9-biphenylyl)carbazolyl group, a (9-phenyl)phenylcarbazolyl group, a diphenylcarbazol-9-yl group, a phenylcarbazole-9-yl group, a phenyldibenzofuranyl group and a phenyldibenzothiophenyl group.

**7.** The compound according to any one of claims 1 to 4, wherein L is a single bond.

**8.** The compound according to any one of claims 1 to 4, wherein the substituted or unsubstituted arylene group having 6 to 50 ring carbon atoms that L represents is selected from a phenylene group, a biphenyldiyl group, a naphthylene group, a terphenylene group, a phenalenylene group and a fluorenylene group.

**9.** The compound according to any one of claims 1 to 4, wherein the substituted or unsubstituted divalent heterocyclic group having 5 to 50 ring atoms that L represents is selected from a divalent residue of an aromatic hetero ring of furan, thiophene, indole, carbazole, benzofuran, dibenzofuran, benzothiophene and dibenzothiophene.

**10.** The compound according to any one of claims 1 to 4, wherein the substituted or unsubstituted aryl group having 6 to 50 ring carbon atoms that $R^{31}$ to $R^{40}$, $R_{901}$, $R_{902}$, and $R_{903}$ represent is selected from a phenyl group, a p-biphenyl group, an m-biphenyl group, an o-biphenyl group, a p-terphenyl-4-yl group, a p-terphenyl-3-yl group, a p-terphenyl-2-yl group an m-terphenyl-4-yl group, an m-terphenyl-3-yl group, an m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, a 9,9-dimethylfluorenyl group and a 9,9-diphenylfluorenyl group.

**11.** The compound according to any one of claims 1 to 4, wherein the substituted or unsubstituted aryl group having 6 to 12 ring carbon atoms that $R^{15}$ to $R^{16}$, and $R^{18}$ to $R^{22}$ represent is selected from a phenyl group, a p-biphenyl group, an m-biphenyl group, an o-biphenyl group, a 1-naphthyl group and a 2-naphthyl group.

**12.** The compound according to any one of claims 1 to 4, wherein the unsubstituted aryl group having 6 to 12 ring carbon atoms that $R^{17}$ represents is selected from a phenyl group, a p-biphenyl group, an m-biphenyl group, an o- biphenyl group, a 1-naphthyl group and a 2-naphthyl group.

**13.** The compound according to any one of claims 1 to 4, wherein the substituted or unsubstituted heterocyclic group having 5 to 50 ring atoms that $R^{31}$ to $R^{40}$, $R_{901}$, $R_{902}$, and $R_{903}$ represent is selected from a carbazolyl group, a benzocarbazolyl group, a dibenzofuranyl group, a naphthobenzofuranyl group, a dibenzothiophenyl group, a naphthobenzothiophenyl group, a (9-phenyl)carbazolyl group, a (9-biphenyl)carbazolyl group, a (9-phenyl)phenylcarbazolyl group, a diphenylcarbazole-9-yl group, a phenylcarbazole-9-yl group, a phenyldibenzofuranyl group and a phenyldibenzothiophenyl group.

**14.** The compound according to any one of claims 1 to 4, wherein the substituted or unsubstituted alkyl group having 1 to 50 carbon atoms that $R^{11}$ to $R^{14}$, $R^{31}$ to $R^{40}$, $R^{41}$ to $R^{48}$, $R_{901}$, $R_{902}$, $R_{903}$, and $R^{41}$ to $R^{48}$ represent is selected from a methyl group, an ethyl group, an n-propyl group, an isopropyl group, an n-butyl group, an isobutyl group, an s-butyl group, a t-butyl group, a heptafluoropropyl group, a pentafluoroethyl group, a 2,2,2-trifluoroethyl group, and a trifluoromethyl group.

**15.** The compound according to any one of claims 1 to 4, wherein the substituted or unsubstituted alkyl group having 6 to 18 carbon atoms that $R^{15}$ to $R^{22}$ represent is selected from a methyl group, an ethyl group, an n-propyl group, an isopropyl group, an n-butyl group, an isobutyl group, an s-butyl group, a t-butyl group, a heptafluoropropyl group, a pentafluoroethyl group, a 2,2,2-trifluoroethyl group, and a trifluoromethyl group.

**16.** The compound according to any one of claims 1 to 4, wherein all $R^{15}$, $R^{20}$, $R^{21}$, $R^{22}$, and $R^{16}$ to $R^{19}$ that are not a

single bond bonding to *a are hydrogen atoms.

**17.** The compound according to any one of claims 1 to 4, wherein all $R^{11}$ to $R^{14}$ are hydrogen atoms.

**18.** The compound according to any one of claims 1, 2 and 4, wherein all $R^{31}$ to $R^{35}$ that are not a single bond bonding to $*b_1$ are hydrogen atoms.

**19.** The compound according to claim 1 or 2, wherein all $R^{36}$ to $R^{40}$ that are not a single bond bonding to $*b_2$ are hydrogen atoms.

**20.** The compound according to any one of claims 1 to 4, wherein all $R^{41}$ to $R^{48}$ that are not a single bond bonding to $*b_3$ are hydrogen atoms.

**21.** The compound according to any one of claims 1 to 4, wherein X is an oxygen atom.

**22.** The compound according to any one of claims 1 to 4, wherein X is a sulfur atom.

**23.** The compound according to any one of claims 1 to 22, wherein the compound contains at least one deuterium atom.

**24.** A material for an organic electroluminescent device containing the compound of any one of claims 1 to 23.

**25.** An organic electroluminescent device comprising an anode, a cathode, and organic layers intervening between the anode and the cathode, the organic layers including a light emitting layer, at least one layer of the organic layers containing the compound of any one of claims 1 to 23.

**26.** The organic electroluminescent device according to claim 25, wherein the organic layer includes a hole transporting zone between the anode and the light emitting layer, and the hole transporting zone contains the compound.

**27.** The organic electroluminescent device according to claim 26, wherein the hole transporting zone includes a first hole transporting layer on the anode side and a second hole transporting layer on the cathode side, and the first hole transporting layer or the second hole transporting layer or both the two contain the compound.

**28.** The organic electroluminescent device according to any one of claims 25 to 27, wherein the light emitting layer contains a fluorescent dopant material.

**29.** The organic electroluminescent device according to any one of claims 25 to 27, wherein the light emitting layer contains a phosphorescent dopant material.

**30.** An electronic device comprising the organic electroluminescent device of any one of claims 25 to 29.

Fig. 1

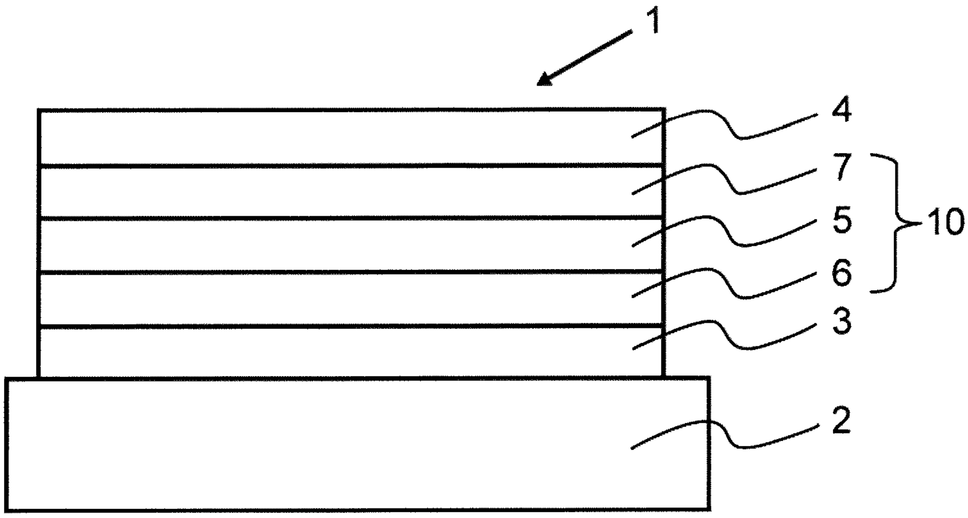


Fig. 2

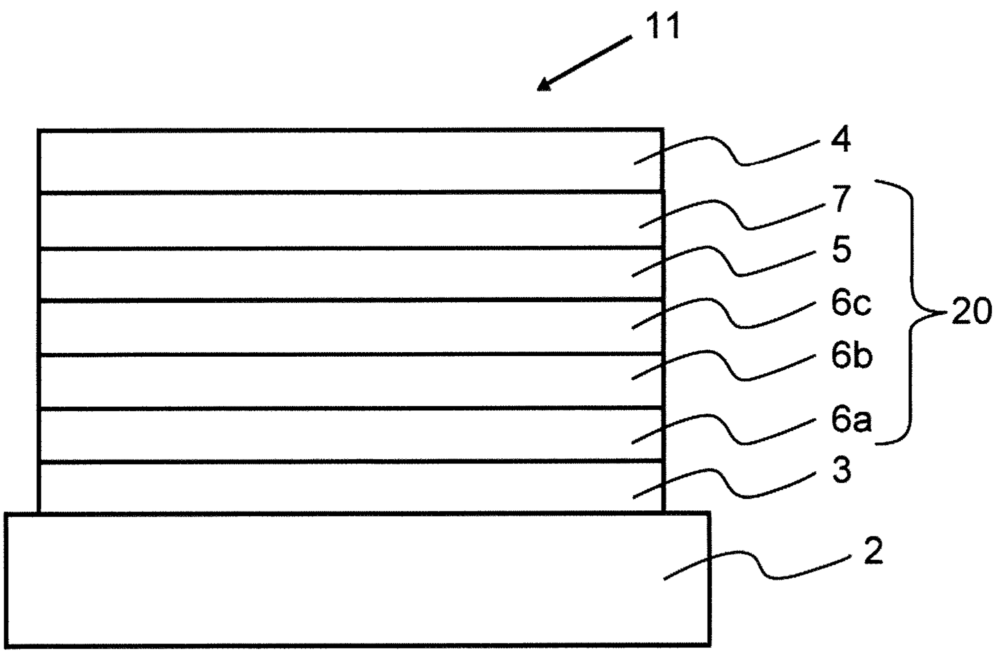

## INTERNATIONAL SEARCH REPORT

International application No. PCT/JP2020/038398

A. CLASSIFICATION OF SUBJECT MATTER
Int. Cl. G07D333/76(2006.01)i, C09K11/06(2006.01)i, C07D307/91(2006.01)i, H01L51/50(2006.01)i
FI: C07D307/91, C07D333/76 CSP, H05B33/22 D, C09K11/06 635, H05B33/14 A
According to International Patent Classification (IPC) or to both national classification and IPC

B. FIELDS SEARCHED

Minimum documentation searched (classification system followed by classification symbols)
Int. Cl. C07D333/76, C09K11/06, C07D307/91, H01L51/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 2019/108033 A1 (LG CHEM, LTD.) 06 June 2019, claims, page 102 | 1-3, 5, 7-30 |
| X | JP 2018-108939 A (IDEMITSU KOSAN CO., LTD.) 12 July 2018, claims, examples | 1-30 |
| X | WO 2018/164201 A1 (IDEMITSU KOSAN CO., LTD.) 13 September 2018, claims, examples | 1-30 |
| X | WO 2019/189033 A1 (IDEMITSU KOSAN CO., LTD.) 03 October 2019, claims, examples | 1-30 |
| X | WO 2016/068446 A1 (SAMSUNG SDI CO., LTD.) 06 May 2016, claims, examples | 1-30 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17.11.2020 | 01.12.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2020/038398

C (Continuation). DOCUMENTS CONSIDERED TO BE RELEVANT

| Category* | Citation of document, with indication, where appropriate, of the relevant passages | Relevant to claim No. |
|---|---|---|
| X | JP 2016-94408 A (E-RAY OPTOELECTRONICS TECH CO., LTD.) 26 May 2016, claims, tables 1-1 to 1-5 | 1-30 |
| X | JP 2016-108290 A (SAMSUNG DISPLAY CO., LTD.) 20 June 2016, claims, examples | 1-30 |
| X | WO 2016/178544 A2 (DONGJIN SEMICHEM CO., LTD.) 10 November 2016, claims, examples | 1-30 |
| X | WO 2016/208862 A1 (DUK SAN NEOLUX CO., LTD.) 29 December 2016, claims, examples | 1-30 |
| X | WO 2017/191976 A1 (DUK SAN NEOLUX CO., LTD.) 09 November 2017, claims, examples | 1-30 |
| X | WO 2018/139767 A1 (LG CHEM, LTD.) 02 August 2018, claims, examples | 1-30 |
| X | CN 110272421 A (BEIJING ETERNAL MATERIAL TECHNOLOGY CO., LTD.) 24 September 2019, claims, examples | 1-30 |
| A | KR 10-2019-0079181 A (DOOSAN CORPORATION) 05 July 2019, entire text | 1-30 |
| P, X | CN 110317139 A (BEIJING ETERNAL MATERIAL TECHNOLOGY CO., LTD.) 11 October 2019, claims, examples | 1-30 |

Form PCT/ISA/210 (continuation of second sheet) (January 2015)

**INTERNATIONAL SEARCH REPORT**
Information on patent family members

International application No.
PCT/JP2020/038398

| Patent Documents referred to in the Report | Publication Date | Patent Family | Publication Date |
|---|---|---|---|
| WO 2019/108033 A1 | 06.06.2019 | KR 10-2019-0063821 A<br>CN 110740998 A | |
| JP 2018-108939 A | 12.07.2018 | (Family: none) | |
| WO 2018/164201 A1 | 13.09.2018 | CN 110382489 A<br>KR 10-2019-0126790 A | |
| WO 2019/189033 A1 | 03.10.2019 | (Family: none) | |
| WO 2016/068446 A1 | 06.05.2016 | KR 10-2016-0049842 A<br>CN 107109211 A | |
| JP 2016-94408 A | 26.05.2016 | US 2016/0133848 A1<br>claims, pages 3-8<br>CN 105585555 A<br>KR 10-2016-0056767 A | |
| JP 2016-108290 A | 20.06.2016 | US 2016/0163993 A1<br>claims, examples<br>KR 10-2016-0070662 A | |
| WO 2016/178544 A2 | 10.11.2016 | KR 10-2016-0132344 A<br>CN 107667099 A | |
| WO 2016/208862 A1 | 29.12.2016 | US 2018/0226585 A1<br>claims, examples<br>KR 10-2017-0001830 A<br>CN 108112250 A | |
| WO 2017/191976 A1 | 09.11.2017 | US 2019/0097138 A1<br>claims, examples<br>KR 10-2017-0125555 A | |
| WO 2018/139767 A1 | 02.08.2018 | US 2019/0169176 A1<br>claims, examples<br>KR 10-2018-0088262 A<br>CN 109803966 A | |
| CN 110272421 A | 24.09.2019 | (Family: none) | |
| KR 10-2019-0079181 A | 05.07.2019 | (Family: none) | |
| CN 110317139 A | 11.10.2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 2018138333
- WO 2007125714 A1
- US 20190006590 A1
- US 20170018710 A1